# EUROPÄISCHE PATENTSCHRIFT

(11) **EP 2 401 256 B1**
(45) Veröffentlichungstag und Bekanntmachung des Hinweises auf die Patenterteilung: **17.04.2013**
(21) Anmeldenummer: 10711628.7
(22) Anmeldetag: 23.02.2010
(51) Int. Cl.: C07D 211/62, C07D 213/81, C07D 233/90, C07D 239/28, C07D 261/18, C07D 271/10, C07D 239/557, C07D 207/277, C07D 237/24, A61K 31/165, A61K 31/44, A61K 31/505, A61K 31/506, C07D 401/12, C07D 405/12

(54) **Verbindungen als Bradykinin-B1-Antagonisten**
Compounds as bradykinin B1 antagonists
Composés comme antagonistes de la bradykinine B1

(30) Priorität: 26.02.2009 EP 09153778
(43) Veröffentlichungstag der Anmeldung: 04.01.2012
(73) Patentinhaber: Boehringer Ingelheim International GmbH, 55216 Ingelheim am Rhein (DE)
(72) Erfinder: HAUEL, Norbert, 55216 Ingelheim am Rhein (DE); CECI, Angelo, 55216 Ingelheim am Rhein (DE); DOODS, Henri, 55216 Ingelheim am Rhein (DE); KONETZKI, Ingo, 55216 Ingelheim am Rhein (DE); MACK, Juergen, 55216 Ingelheim am Rhein (DE); PRIEPKE, Henning, 55216 Ingelheim am Rhein (DE); SCHULER-METZ, Annette, 55216 Ingelheim am Rhein (DE); WALTER, Rainer, 55216 Ingelheim am Rhein (DE); WIEDENMAYER, Dieter, 55216 Ingelheim am Rhein (DE)
(74) Vertreter: Simon, Elke Anna Maria
(86) Internationale Anmeldenummer: PCT/EP2010/052232
(87) Internationale Veröffentlichungsnummer: WO 2010/097372

(56) Entgegenhaltungen:
- WO-A1-03/066577
- WO-A1-2005/016886
- WO-A2-2004/019868
- WO-A2-2005/085198

## Beschreibung

Gegenstand der vorliegenden Erfindung sind die Verbindungen der allgemeinen Formel **I** 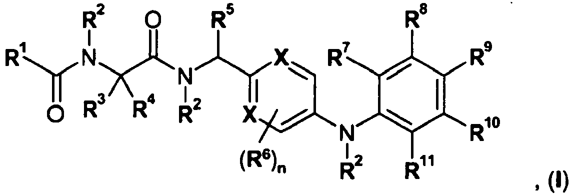 in der **n, R¹, R², R³, R⁴, R⁵, R⁶, R⁷, R⁸, R⁹, R¹⁰, R¹¹** und **X** wie nachstehend beschrieben definiert sind, deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung.

Ähnliche Bradykinin -B1- Antagonisten ohne den Bi(Hetero) aryl-Rest sind in WO-A-2005/085198, WO-A-03/066577, WO-A-2004/019868 und WO2005/016886 offenbart.

### DETAILLIERTE BESCHREIBUNG DER ERFINDUNG

In der obigen allgemeinen Formel I bedeuten in einer Ausführungsform 1
**n** eine der Ziffern 0, 1 oder 2,
**R¹** (a) eine gegebenenfalls mit einem Rest **R^{1.1}** substituierte C₁₋₆-Alkylgruppe,
(b) eine C₁₋₃₋Alkylgruppe in der jede Methylengruppe mit 1 oder 2 Fluoratomen und jede Methylgruppe mit 1, 2 oder 3 Fluoratomen substituiert sein kann,
(c) eine gegebenenfalls mit einem Rest **R^{1.2}** substituierte C₃₋₆-Cycloalkylgruppe, in der eine -CH₂-Einheit durch eine -C(O)-Gruppe ersetzt sein kann,
(d) eine gegebenenfalls mit 1, 2 oder 3 Resten **R^{1.3}** substituierte Aryl-C₀₋₂-alkylengruppe,
(e) ein gegebenenfalls mit 1, 2 oder 3 Resten **R^{1.4}** substituierter fünfgliedriger Heteroaryl-C₀₋₂-alkylen-Rest, der mindestens ein N-, O- oder S-Atom enthält und der gegebenenfalls zusätzlich ein, zwei oder drei weitere N-Atome enthält und der zusätzlich benzo-kondensiert sein kann,
(f) ein gegebenenfalls mit 1 oder 2 Resten **R^{1.4}** substituierter sechsgliedriger Heteroaryl-C_{0.2}-alkylen-Rest, der ein, zwei oder drei N-Atome enthält und der zusätzlich benzo-kondensiert sein kann,
(g) ein gegebenenfalls mit 1 oder 2 Resten **R^{1.4}** substituierter neun- oder zehngliedriger Heteroaryl-Rest, der ein, zwei oder drei N-Atome enthält,
(h) ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
(i) -O-**R^{1.1.1}**,
(j) -N**R^{1.1.3}R^{1.1.4}** oder
(k) -C(=N**R^{1.5}**)-CN,
**R^{1.1}** Halogen, -NO₂, -CN, C₃₋₆-Cycloalkyl, -O**R^{1.1.1}**, -S**R^{1.1.1}**, -C(O)**R^{1.1.1}**, -S(O)₂-**R^{1.1.2}**, -O-S(O)₂-R**^{1.1.1},** -CO₂**R^{1.1.1}**, -O-C(O)-**R^{1.1.1}**, -N**R^{1.1.3}R^{1.1.4}**, -N**R^{1.1.3}**-C(O)-**R^{1.1.1}**, -N**R^{1.1.3}**-C(O)-**R^{1.1.1}**, -N**R^{1.1.3}**-CO₂**R^{1.1.1}** oder -C(O)-N**R^{1.1.3}R^{1.1.4}**,
**R^{1.1.1}** (a) H,
(b) C₁₋₄-Alkyl,
(c) eine C₁₋₃-Alkylgruppe, in der jede Methylengruppe mit 1 oder 2 Fluoratomen und jede Methylgruppe mit 1, 2 oder 3 Fluoratomen substituiert sein kann,
(d) eine gegebenenfalls mit 1, 2 oder 3 Resten **R^{1.1.1.1}** substituierte Phenylgruppe,
(e) C₃₋₆-Cycloalkyl oder
(f) eine gegebenenfalls mit 1, 2 oder 3 Resten **R^{1.1.1.2}** substituierte Pyridylgruppe,
**R^{1.1.1.1}** unabhängig voneinander
(a) Halogen, -NO₂, -CN, -OH, -O-C₁₋₄-Alkyl, C₃₋₆-Cycloalkyl, C₁₋₄-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R^{1.1.1.2}** unabhängig voneinander Halogen oder C₁₋₄-Alkyl,
**R^{1.1.2}** (a) C₁₋₄-Alkyl,
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
(c) -O-C₁₋₄-Alkyl oder
(d) eine gegebenenfalls mit 1, 2 oder 3 Resten **R^{1.1.1.1}** substituierte Phenylgruppe,
**R^{1.1.3},**
**R^{1.1.4}** unabhängig voneinander
(a) H,
(b) eine gegebenenfalls mit 1, 2 oder 3 Resten **R^{1.1.4.1}** substituierte C₁₋₄-Alkylgruppe,
(c) eine gegebenenfalls mit 1, 2 oder 3 Resten **R^{1.1.1.1}** substituierte Phenylgruppe,
(d) C₃₋₆-Cycloalkyl, oder
**R^{1.1.3}** und **R^{1.1.4}** zusammen mit dem N-Atom, an das sie gebunden sind, einen 5- oder 6-gliedrigen heterocyclischen Ring bilden, der zusätzlich ein weiteres Heteroatom ausgewählt aus N, O und S enthalten kann, oder
**R^{1.1.3}** und **R^{1.1.4}** zusammen mit dem N-Atom, an das sie gebunden sind, ein cyclisches Imid bilden,
**R^{1.1.4.1}** unabhängig voneinander Halogen, -NH₂, -NH(C₁₋₄-Alkyl), -N(C₁₋₄-Alkyl)₂ oder -SO₂-**R^{1.1.2}**,
**R^{1.2}** Halogen, -NO₂, -CN, OH, -O-CH₃ oder Phenyl,
**R^{1.3}** (a) Halogen, -NO₂, -CN, **-**O**R^{1.1.1}, -**S**R^{1.1.1}, -**CO₂**R^{1.1.1},** C₁₋₆-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R^{1.4}** unabhängig voneinander
(a) Halogen, -NO₂, -CN, -O**R^{1.1.1}**, -S**R^{1.1.1},** -S(O)-**R^{1.1.2}**, -S(O)₂-**R^{1.1.2}**, -N**R^{1.1.3}R^{1.1.14}**, -N(**R^{1.4.1}**)-C(O)-C₁₋₄-Alkyl, C₁₋₆-Alkyl,
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, oder
(c) eine Oxo-Gruppe,
**R^{1.4.1}** H oder C₁₋₄-Alkyl,
**R^{1.5}** -OH oder -O-C₁₋₃-Alkyl,
**R²** (a) H,
(b) C₁₋₄-Alkyl,
(c) C₁₋₄-Alkyl-C(O)-,
**R³** und **R⁴** zusammen mit dem Kohlenstoffatom, an dem sie gebunden sind, eine gegebenenfalls durch einen Rest **R^{3·1}** substituiert C₃₋₆-Cycloakylengruppe, in der eine -CH₂-Einheit durch ein Heteroatom O, N, S oder durch eine Gruppe CO, SO oder SO₂ ersetzt sein kann,
**R^{3.1}** H, -OH,
**R⁵** (a) H,
(b) C₁₋₄-Alkyl,
(c) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R⁶** unabhängig voneinander
(a) H, Halogen, -CN, -OH, C₁₋₆-Alkyl, C₃₋₇-Cycloalkyl, -O-C₁₋₄-Alkyl, -O-CF₃, -O-C₃₋₆-Cycloalkyl, -N(C₁₋₃-Alkyl)₂, -C(O)-NH₂, -(SO₂)NH₂, -SO₂-C₁₋₃-Alkyl, oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R⁷** (a) H, Halogen, -CN, -OH,
(b) C₁₋₆-Alkyl,
(c) C₁-₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) -O-C₁₋₆-Alkyl,
(f) -O-C₃₋₇-Cycloalkyl,
(g) -NH₂, -NH(C₁₋₃-Alkyl), -N(C₁₋₃-Alkyl)₂,
(h) -C(O)-**R^{7.1}**,
(i) -S-C₁₋₄-Alkyl, -SO₂-**R^{7.2}**,
(j) eine gegebenenfalls mit einer oder zwei C₁₋₃-Alkylgruppen substituierte fünfgliedrige Heteroarylgruppe, die ausgewählt ist aus der Gruppe bestehend aus Pyrrolyl, Oxazolyl, Isoxazolyl, Oxadiazolyl, Thiazolyl, Isothiazolyl, Thiadiazolyl, Imidazolyl, Pyrazolyl, Triazolyl und Tetrazolyl, oder
(k) eine gegebenenfalls mit einer oder zwei C₁₋₃-Alkylgruppen substituierte sechsgliedrige Heteroarylgruppe, die ausgewählt ist aus der Gruppe bestehend aus Pyridyl, Pyrimidinyl, Pyrazinyl, Pyridazinyl und Triazinyl,
**R^{7.1}** -NH₂, -NH(C₁₋₆-Alkyl),-N(C₁₋₆-Alkyl)₂, *N*-Acetidinyl, *N*-Pyrrolidinyl, N-Piperidinyl, *N*-Morpholinyl, -OH, -O-C₁₋₈-Alkyl oder -O-C₃₋₇-Cycloalkyl,
**R^{7.2}** -NH₂, -NH(C₁₋₆-Alkyl),-N(C₁-₆-Alkyl)₂, *N*-Acetidinyl, *N*-Pyrrolidinyl, *N*-Piperidinyl oder *N*-Morpholinyl und
**R⁸** H, Halogen, C₁₋₄-Alkyl,
**R⁹** (a) H, Halogen, -CN, -OH,
(b) C₁₋₆-Alkyl,
(c) C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) C₂₋₄-Alkinyl,
(f) -O-C₁₋₆-Alkyl,
(g) -O-C₃₋₇-Cycloalkyl,
(h) -NH₂, -NH(C₁₋₃-Alkyl), -N(C₁₋₃-Alkyl)₂,
(i) -C(O)-**R^{9.1}**,
(j) -S-C₁₋₄-Alkyl, -SO-C₁₋₄-Alkyl, -SO₂-C₁₋₄-Alkyl,
**R^{9.1}** -NH₂, -NH(C₁₋₆-Alkyl),-N(C₁₋₆-Alkyl)₂, *N*-Acetidinyl, *N*-Pyrrolidinyl, *N*-Piperidinyl, *N*-Morpholinyl, -OH, -O-C₁₋₈-Alkyl oder -O-C₃₋₇-Cycloalkyl,
**R¹⁰** H, Halogen, C₁₋₄-Alkyl,
**R¹¹** (a) H, Halogen, -CN, -OH,

(b) C₁₋₆-Alkyl,
(c) C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) -O-C₁₋₆-Alkyl,
(f) -O-C₃₋₇-Cycloalkyl,
(g) -NH₂, -NH(C₁₋₃-Alkyl), -N(C₁₋₃-Alkyl)₂,
(h) -C(O)-**R^{11.1}**,
(i) -S-C₁₋₃-Alkyl, -SO₂-**R^{11.2}**,
(j) eine gegebenenfalls mit einer oder zwei C₁₋₃-Alkylgruppen substituierte fünfgliedrige Heteroarylgruppe, die ausgewählt ist aus der Gruppe bestehend aus Pyrrolyl, Oxazolyl, Isoxazolyl, Oxadiazolyl, Thiazolyl, Isothiazolyl, Thiadiazolyl, Imidazolyl, Pyrazolyl, Triazolyl und Tetrazolyl, oder
(k) eine gegebenenfalls mit einer oder zwei C₁₋₃-Alkylgruppen substituierte sechsgliedrige Heteroarylgruppe, die ausgewählt ist aus der Gruppe bestehend aus Pyridyl, Pyrimidinyl, Pyrazinyl, Pyridazinyl und Triazinyl,
**R^{11.1}** -NH₂, -NH(C₁₋₆-Alkyl),-N(C₁₋₆-Alkyl)₂, *N*-Acetidinyl, *N*-Pyrrolidinyl, *N*-Piperidinyl, *N*-Morpholinyl, -OH, -O-C₁₋₈-Alkyl oder -O-C₃₋₇-Cycloalkyl,
**R^{11.2}** -NH₂, -NH(C₁₋₆-Alkyl),-N(C₁₋₆-Alkyl)₂, *N*-Acetidinyl, *N*-Pyrrolidinyl, *N*-Piperidinyl oder *N*-Morpholinyl und
**X** unabhängig voneinander C-**R⁶** oder N, deren Enantiomere, deren Diastereomere, deren Gemische und deren Salze, insbesondere deren physiologisch verträgliche Salze mit organischen oder anorganischen Säuren oder Basen.

Eine Ausführungsform **2** der vorliegenden Erfindung besteht in den Verbindungen der obigen allgemeinen Formel **I**, in denen **R¹, R³, R⁴, R⁵, R⁶, R⁷, R⁸, R⁹, R¹⁰, R¹¹, n** und **X** wie voranstehend unter der Ausführungsform 1 erwähnt definiert sind und
**R¹** (a) eine gegebenenfalls mit einem Rest **R^{1.1}** substituierte C₁₋₆-Alkylgruppe,
(b) eine C₁₋₃-Alkylgruppe, in der jede Methylengruppe mit 1 oder 2 Fluoratomen und jede Methylgruppe mit 1,2 oder 3 Fluoratomen substituiert sein kann,
(c) eine gegebenenfalls mit einem Rest **R^{1.2}** substituierte C₃₋₆-Cycloalkylgruppe, in der eine -CH₂-Einheit durch eine -C(O)-Gruppe ersetzt sein kann,
(d) eine gegebenenfalls mit 1, 2 oder 3 Resten **R^{1.3}** substituierte Phenylgruppe,
(e) ein gegebenenfalls mit 1, 2 oder 3 Resten **R^{1.4}** substituierter fünfgliedriger Heteroaryl-Rest, der mindestens ein **N-,** O- oder S-Atom enthält und der gegebenenfalls zusätzlich ein, zwei oder drei weitere N-Atome enthält,
(f) ein gegebenenfalls mit 1 oder 2 Resten **R^{1.4}** substituierter sechsgliedriger Heteroaryl-Rest, der ein, zwei oder drei N-Atome enthält,
(g) ein gegebenenfalls mit 1 oder 2 Resten **R^{1.4}** substituierter neun- oder zehngliedriger Heteroaryl-Rest, der ein, zwei oder drei N-Atome enthält,
(h) ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
(i) -O-**R^{1.1.1}** oder
(j) -N**R^{1.1.3}R^{1.1.4}**,
**R^{1.1}** -CN, C₃₋₆-Cycloalkyl, -O**R^{1.1.1}**, -N**R^{1.1.3}R^{1.1.4}**,
**R^{1.1.1}** (a) H,
(b) C₁₋₄-Alkyl,
(c) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R^{1.1.3}**,
**R^{1.1.4}** unabhängig voneinander
(a) H,
(b) C₁₋₄-Alkyl,
(c) C₃₋₆-Cycloalkyl, oder
**R^{1.1.3}** und **R^{1.1.4}** zusammen mit dem N-Atom, an das sie gebunden sind, einen 5- oder 6-gliedrigen heterocyclischen Ring bilden, der zusätzlich ein weiteres Heteroatom ausgewählt aus N, O und S enthalten kann, oder
**R^{1.2}** Halogen, -NO₂, -CN, -OH, -O-CH₃ oder Phenyl,
**R^{1.3}** unabhängig voneinander
(a) Halogen, -NO₂, -CN, -O**R^{1.1.1}**, C₁₋₆-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R^{1.4}** unabhängig voneinander
(a) Halogen, -NO₂, -CN, -O**R^{1.1.1}**, -N**R^{1.1.3}R^{1.1.4}**, -N(**R^{1.4.1}**)-C(O)-C₁₋₄-Alkyl, C₁₋₆-Alkyl, oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4.1}** H oder C₁₋₄-Alkyl, bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **3** der vorliegenden Erfindung besteht in den Verbindungen der obigen allgemeinen Formel **I,** in denen **R², R³, R⁴, R⁵, R⁶, R⁷, R⁸, R⁹, R¹⁰, R¹¹,** n und **X** wie voranstehend unter der Ausführungsform **1** erwähnt definiert sind und
**R¹** (a) eine gegebenenfalls mit einem Rest **R^{1.1}** substituierte C₁₋₆-Alkylgruppe,
(b) eine gegebenenfalls mit 1, 2 oder 3 Resten **R^{1.3}** substituierte Phenylgruppe,
(c) ein gegebenenfalls mit 1, 2 oder 3 Resten **R^{1.4}** substituierter fünfgliedriger Heteroaryl-Rest, der mindestens ein **N**-, O- oder S-Atom enthält und der gegebenenfalls zusätzlich ein, zwei oder drei weitere N-Atome enthält,
(d) ein gegebenenfalls mit 1 oder 2 Resten **R^{1.4}** substituierter sechsgliedriger Heteroaryl-Rest, der ein, zwei oder drei N-Atome enthält,
(e) ein gegebenenfalls mit 1 oder 2 Resten **R^{1.4}** substituierter neun- oder zehngliedriger Heteroaryl-Rest, der ein, zwei oder drei N-Atome enthält,
(f) ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
**R^{1.1}** -CN, C₃₋₆-Cycloalkyl, -OH, -OCH₃, -NH₂, -NHCH₃, -N(CH₃)₂,
**R^{1.3}** unabhängig voneinander
(a) F, Cl, Br, -OH, -OCH₃, C₁₋₆-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
(a) F, Cl, Br, -OH, -OCH₃, -NH₂, -NHCH₃, -N(CH₃)₂, -NHC₂₋₃-Alkyl, -N(C₂₋₃-Alkyl)₂, -NH-C(O)-C₁₋₄-Alkyl, C₁₋₆-Alkyl, oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **4** der vorliegenden Erfindung besteht in den Verbindungen der obigen allgemeinen Formel **I,** in denen **R², R³, R⁴, R⁵, R⁶, R⁷, R⁸, R⁹, R¹⁰, R¹¹, n** und **X** wie voranstehend unter der Ausführungsform **1** erwähnt definiert sind und
**R¹** (a) eine gegebenenfalls mit einem Rest **R^{1.1}** substituierte C₁₋₆-Alkylgruppe,
(b) eine gegebenenfalls mit 1, 2 oder 3 Resten **R^{1.3}** substituierte Phenylgruppe,
(c) ein gegebenenfalls mit 1, 2 oder 3 Resten **R^{1.4}** substituierter fünfgliedriger Heteroaryl-Rest, der ausgewählt ist aus der Gruppe bestehend aus 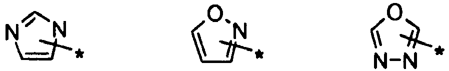 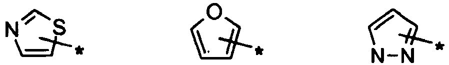 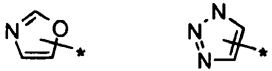 (d) ein gegebenenfalls mit 1 oder 2 Resten **R^{1.4}** substituierter sechsgliedriger Heteroaryl-Rest, der ausgewählt ist aus der Gruppe bestehend aus 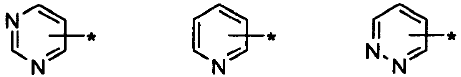 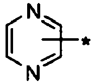 (e) ein gegebenenfalls mit 1 oder 2 Resten **R^{1.4}** substituierter neungliedriger Heteroaryl-Rest, der ausgewählt ist aus der Gruppe bestehend aus 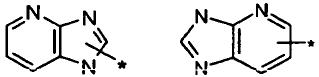 (f) ein gegebenenfalls mit 1 oder 2 Resten **R^{1.4}** substituierter 5- oder 6-gliedriger Heterocyclus, der ausgewählt ist aus der Gruppe bestehend aus 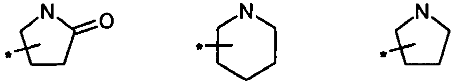 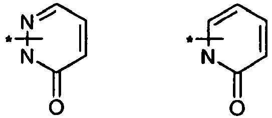 **R^{1.1}** -CN, Cyclopropyl, -OH, -OCH₃, -NH₂, -NHCH₃, -N(CH₃)₂,
**R^{1.3}** unabhängig voneinander
(a) F, Cl, Br, -OH, -OCH₃, -OCF₃, C₁₋₄-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
(a) F, Cl, Br, -OH, -OCH₃, -OCF₃, -NH₂, -NH-C₁₋₄-Alkyl, -N(C₁₋₄-Alkyl)₂, -NH-C(O)-C₁₋₄-Akyl, C₁₋₆-Alkyl, oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **5** der vorliegenden Erfindung besteht in den Verbindungen der obigen allgemeinen Formel **I**, in denen **R², R³, R⁴, R⁵, R⁶, R⁷, R⁸, R⁹, R¹⁰, R¹¹**, n und **X** wie voranstehend unter der Ausführungsform **1** erwähnt definiert sind und
**R¹** ausgewählt ist aus der Gruppe bestehend aus 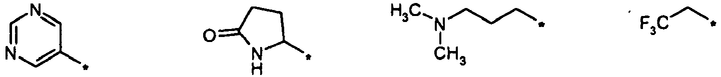 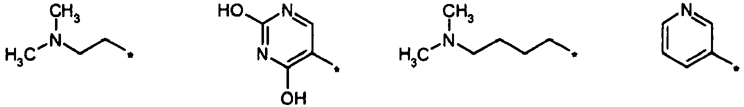 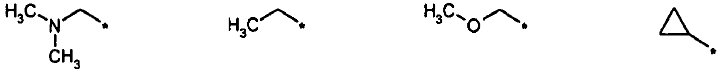 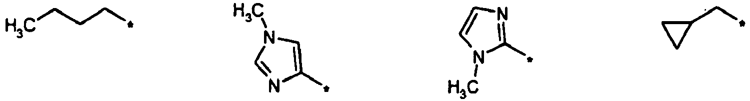 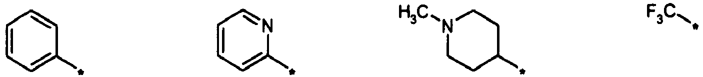 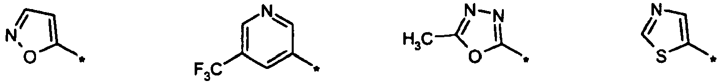 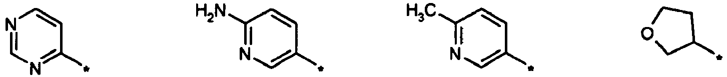 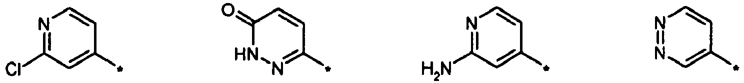 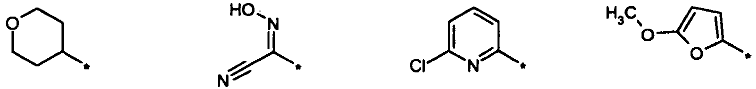 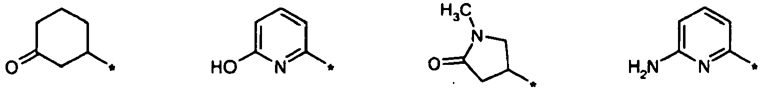 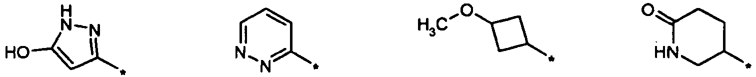 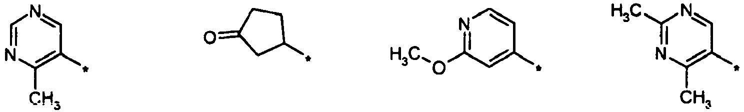 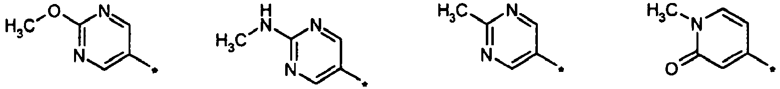 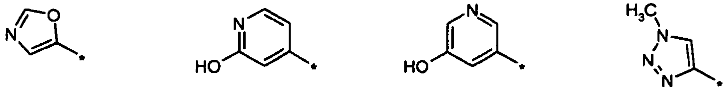 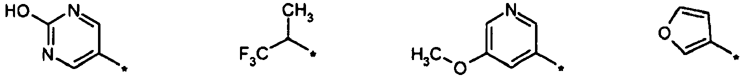 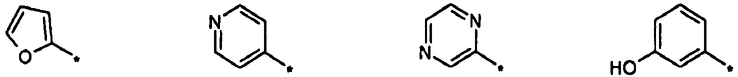 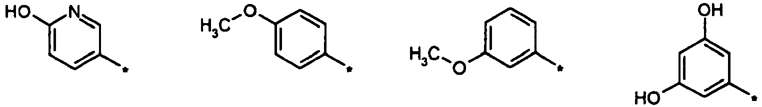 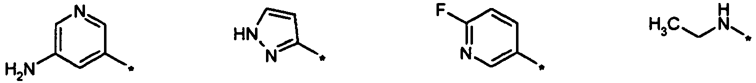 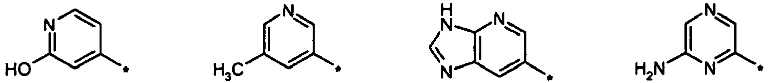 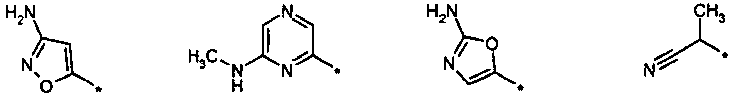 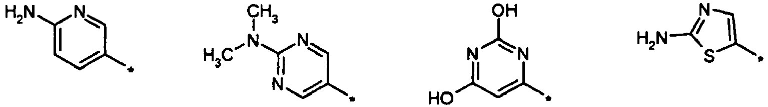 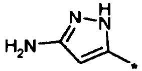 deren Enantiomere, deren Diastereomere, deren Gemische und deren Salze, insbesondere deren physiologisch verträgliche Salze mit organischen oder anorganischen Säuren oder Basen.

Eine Ausführungsform **6** der vorliegenden Erfindung besteht in den Verbindungen der obigen allgemeinen Formel **I**, in denen **R², R³, R⁴, R⁵, R⁶, R⁷, R⁸, R⁹, R¹⁰, R¹¹**, n und **X** wie voranstehend unter der Ausführungsform **1** erwähnt definiert sind und
**R¹** ausgewählt ist aus der Gruppe bestehend aus 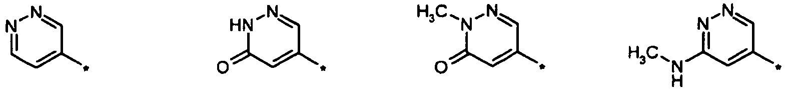 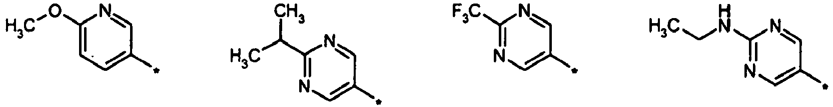 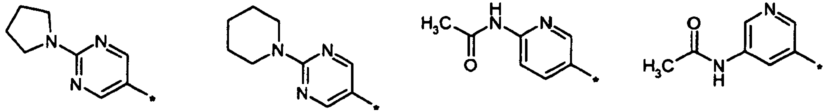 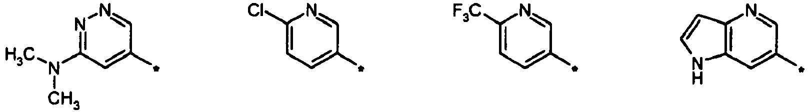 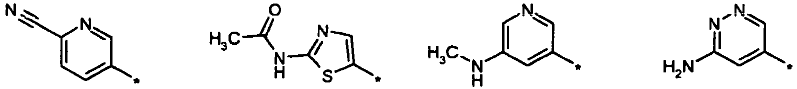 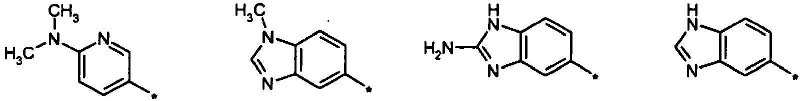 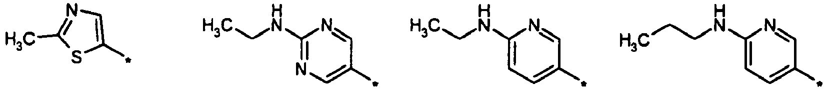 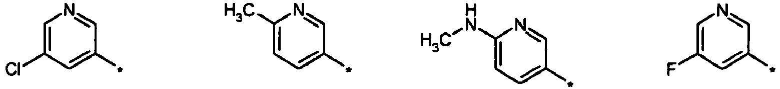 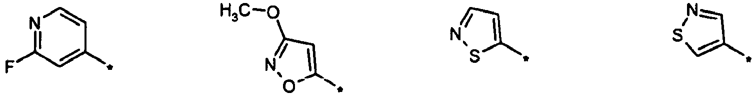 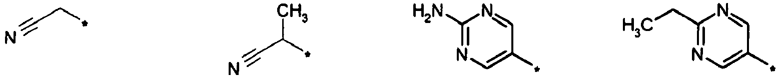 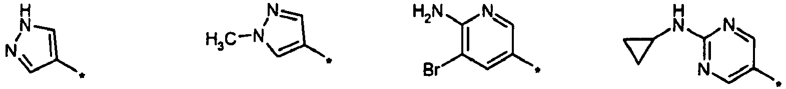 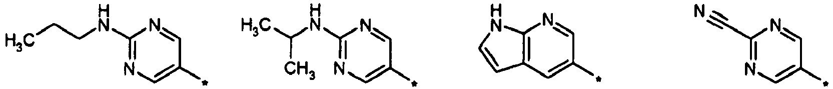 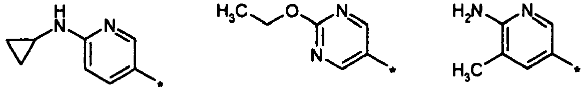 deren Enantiomere, deren Diastereomere, deren Gemische und deren Salze, insbesondere deren physiologisch verträgliche Salze mit organischen oder anorganischen Säuren oder Basen.

Eine Ausführungsform **7** der vorliegenden Erfindung besteht in den Verbindungen der obigen allgemeinen Formel **I**, in denen **R¹** wie voranstehend unter der Ausführungsform **1**, **2, 3, 4, 5** oder **6** erwähnt definiert ist und
**n** eine der Ziffern 0, 1 oder 2,
**R²** (a) H,
(b) C₁₋₄-Alkyl,
**R³** und **R⁴** zusammen mit dem Kohlenstoffatom, an dem sie gebunden sind, eine gegebenenfalls durch einen Rest **R^{3.1}** substituierte C₃₋₆-Cycloalkylengruppe, in der eine -CH₂-Einheit durch ein Heteroatom O, N, S oder durch eine Gruppe CO, SO oder SO₂ ersetzt sein kann,
**R^{3.1}** H, -OH,
**R⁵** (a) H,
(b) C₁₋₄-Alkyl,
(c) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R⁶** unabhängig voneinander
(a) H, Halogen, -CN, -OH, C₁₋₆-Alkyl, C₃₋₇-Cycloalkyl, -O-C₁₋₄-Alkyl, -O-CF₃, -O-C₃₋₆-Cycloalkyl, -N(C₁₋₃-Alkyl)₂, -C(O)-NH₂, -(SO₂)NH₂, -SO₂-C₁₋₃-Alkyl, oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R⁷** (a) H, Halogen, -CN, -OH,
(b) C₁₋₆-Alkyl,
(c) C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) -O-C₁₋₆-Alkyl,
(f) -O-C₃₋₇-Cycloalkyl,
(g) -NH₂, -NH(C₁₋₃-Alkyl), -N(C₁₋₃-Alkyl)₂,
(h) -C(O)-R^{7.1},
(i) -S-C₁₋₄-Alkyl,
**R^{7.1}** -NH₂, , -OH, -O-C₁₋₈-Alkyl,
**R⁸** H, Halogen, C₁₋₄-Alkyl,
**R⁹** (a) H, Halogen, -CN, -OH,
(b) C₁₋₆-Alkyl,
(c) C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) C₂₋₄-Alkinyl,
(f) -O-C₁₋₆-Alkyl,
(g) -O-C₃₋₇-Cycloalkyl,
(h) -NH₂, -NH(C₁₋₃-Alkyl), -N(C₁₋₃-Alkyl)₂,
(i) -C(O)-**R^{9.1}**,
(j) -S-C₁₋₄-Alkyl, -SO-C₁₋₄-Alkyl, -SO₂-C₁₋₄-Alkyl,
**R^{9.1}** -NH₂, -OH, -O-C₁₋₈-Alkyl,
**R¹⁰** H, Halogen, C₁₋₄-Alkyl,
**R¹¹** (a) H, Halogen, -CN, -OH,
(b) C₁₋₆-Alkyl,
(c) C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) -O-C₁₋₆-Alkyl,
(f) -O-C₃₋₇-Cycloalkyl,
(g) -NH₂, -NH(C₁₋₃-Alkyl), -N(C₁₋₃-Alkyl)₂,
(h) -C(O)-**R^{11.1}**,
(i) -S-C₁₋₃-Alkyl,
**R^{11,1}** -NH₂, , -OH, -O-C₁₋₈-Alkyl, und
**X** unabhängig voneinander C-**R⁶** oder N, bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **8** der vorliegenden Erfindung besteht in den Verbindungen der obigen allgemeinen Formel **I**, in denen **R¹, R³, R⁴, R⁵, R⁶, R⁷, R⁸, R⁹, R¹⁰, R¹¹**, **n** und **X** wie voranstehend unter der Ausführungsform **1, 2, 3, 4, 5, 6** oder **7** erwähnt definiert sind und
**R²** H oder CH₃ bedeutet,
deren Enantiomere, deren Diastereomere, deren Gemische und deren Salze, insbesondere deren physiologisch verträgliche Salze mit organischen oder anorganischen Säuren oder Basen.

Eine Ausführungsform **9** der vorliegenden Erfindung besteht in den Verbindungen der obigen allgemeinen Formel I, in denen **R¹, R³, R⁴, R⁵, R⁶, R⁷, R⁸, R⁹, R¹⁰, R¹¹**, n und **X** wie voranstehend unter der Ausführungsform **1 2, 3, 4, 5, 6** oder **7** erwähnt definiert sind und
**R²** H bedeutet,
deren Enantiomere, deren Diastereomere, deren Gemische und deren Salze, insbesondere deren physiologisch verträgliche Salze mit organischen oder anorganischen Säuren oder Basen.

Eine Ausführungsform **10** der vorliegenden Erfindung besteht in den Verbindungen der obigen allgemeinen Formel **I**, in denen **R¹, R², R⁵, R⁶, R⁷, R⁸, R⁹, R¹⁰, R¹¹**, **n** und **X** wie voranstehend unter der Ausführungsform **1, 2, 3, 4, 5, 6, 7,** **8** oder **9** erwähnt definiert sind und
**R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann, bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **11** der vorliegenden Erfindung besteht in den Verbindungen der obigen allgemeinen Formel **I**, in denen **R¹, R², R⁵, R⁶, R⁷, R⁸, R⁹, R¹⁰, R¹¹**, **n** und **X** wie voranstehend unter der Ausführungsform **1, 2, 3, 4, 5, 6, 7,** **8** oder **9** erwähnt definiert sind und
**R³** und **R⁴** zusammen mit dem Kohlenstoffatom, an dem sie gebunden sind, eine Gruppe ausgewählt aus 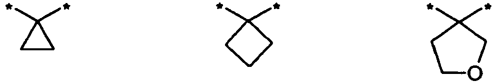 bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **12** der vorliegenden Erfindung besteht in den Verbindungen der obigen allgemeinen Formel **I**, in denen **R¹, R², R³, R⁴, R⁶, R⁷, R⁸, R⁹, R¹⁰, R¹¹**, **n** und **X** wie voranstehend unter der Ausführungsform **1, 2, 3, 4, 5, 6, 7, 8, 9, 10** oder **11** erwähnt definiert sind und
**R⁵** H oder CH₃ bedeutet,
deren Enantiomere, deren Diastereomere, deren Gemische und deren Salze, insbesondere deren physiologisch verträgliche Salze mit organischen oder anorganischen Säuren oder Basen.

Eine Ausführungsform **13** der vorliegenden Erfindung besteht in den Verbindungen der obigen allgemeinen Formel **I,** in denen **R¹, R², R³, R⁴, R⁵, R⁷, R⁸, R⁹, R¹⁰, R¹¹**, n und **X** wie voranstehend unter der Ausführungsform 1**, 2, 3, 4, 5, 6, 7, 8, 9, 10, 11** oder **12** erwähnt definiert sind und
**R⁶** H, F, Cl oder Methyl bedeutet,
deren Enantiomere, deren Diastereomere, deren Gemische und deren Salze, insbesondere deren physiologisch verträgliche Salze mit organischen oder anorganischen Säuren oder Basen.

Eine Ausführungsform **14** der vorliegenden Erfindung besteht in den Verbindungen der obigen allgemeinen Formel I, in denen **R¹, R², R³, R⁴, R⁵, R⁶,** n und **X** wie voranstehend unter der Ausführungsform **1, 2, 3, 4, 5, 6, 7, 8, 9, 10, 11,** **12** oder **13**erwähnt definiert sind und
**R⁷** H, F, Cl, Br, -CN, C₁₋₄-Alkyl, CF₃, CHF₂,
**R⁸** H,
**R**⁹ F, Cl, Br, C₁₋₄-Alkyl, -O-C₁₋₄-Alkyl, -S-C₁₋₄-Alkyl,
**R¹⁰** H und
**R¹¹** F, Cl, Br, -CN, C₁₋₄-Alkyl, CF₃, CHF₂, bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **15** der vorliegenden Erfindung besteht in den Verbindungen der allgemeinen Formel Ia 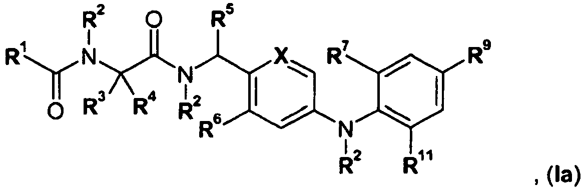 in der
**R¹** (a) eine gegebenenfalls mit einem Rest R^{1.1} substituierte C₁₋₆-Alkylgruppe,
(b) eine gegebenenfalls mit 1, 2 oder 3 Resten **R^{1.3}** substituierte Phenylgruppe,
(c) ein gegebenenfalls mit 1, 2 oder 3 Resten **R^{1.4}** substituierter fünfgliedriger Heteroaryl-Rest, der mindestens ein **N**-, O- oder S-Atom enthält und der gegebenenfalls zusätzlich ein, zwei oder drei weitere N-Atome enthält,
(d) ein gegebenenfalls mit 1 oder 2 Resten **R^{1.4}** substituierter sechsgliedriger Heteroaryl-Rest, der ein, zwei oder drei N-Atome enthält,
(e) ein gegebenenfalls mit 1 oder 2 Resten **R^{1.4}** substituierter neun- oder zehngliedriger Heteroaryl-Rest, der ein, zwei oder drei N-Atome enthält,
(f) ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
**R^{1.1}** -CN, C₃₋₆-Cycloalkyl, -OH, -OCH₃, -NH₂, -NHCH₃, -N(CH₃)₂,
**R^{1.3}** unabhängig voneinander
(a) F, Cl, Br, -OH, -OCH₃, C₁₋₆-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
(a) F, Cl, Br, -OH, -OCH₃, -NH₂, -NHCH₃, -N(CH₃)₂, -NH-C(O)-C₁₋₄-Alkyl, C₁₋₆-Alkyl, oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R²** H oder CH₃,
**R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann,
**R⁵** H oder C₁₋₄-Alkyl,
**R⁶** H, F, Cl, Br oder C₁₋₄-Alkyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.
Eine Ausführungsform **16** der vorliegenden Erfindung besteht in den Verbindungen der allgemeinen Formel Ia, in denen
**R¹** (a) eine gegebenenfalls mit einem Rest **R^{1.1}** substituierte C₁₋₆-Alkylgruppe,
(b) eine gegebenenfalls mit 1, 2 oder 3 Resten **R^{1.3}** substituierte Phenylgruppe,
(c) ein gegebenenfalls mit 1, 2 oder 3 Resten **R^{1.4}** substituierter fünfgliedriger Heteroaryl-Rest, der ausgewählt ist aus der Gruppe bestehend aus 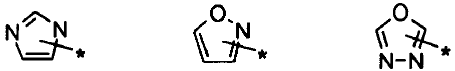 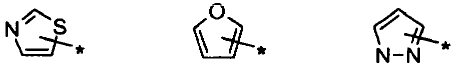 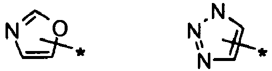 (d) ein gegebenenfalls mit **1** oder 2 Resten **R^{1.4}** substituierter sechsgliedriger Heteroaryl-Rest, der ausgewählt ist aus der Gruppe bestehend aus 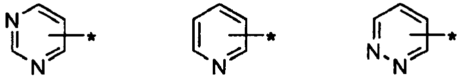 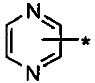 (e) ein gegebenenfalls mit **1** oder 2 Resten **R^{1.4}** substituierter neungliedriger Heteroaryl-Rest, der ausgewählt ist aus der Gruppe bestehend aus 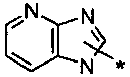 (f) ein gegebenenfalls mit 1 oder 2 Resten **R^{1.4}** substituierter 5- oder 6-gliedriger Heterocyclus, der ausgewählt ist aus der Gruppe bestehend aus 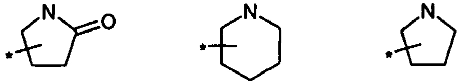 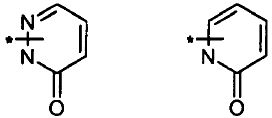 **R^{1.1}** -CN, Cyclopropyl, -OH, -OCH₃, -NH₂, -NHCH₃, -N(CH₃)₂,
**R^{1.3}** unabhängig voneinander
(a) F, Cl, Br, -OH, -OCH₃, -OCF₃, C₁₋₄-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
(a) F, Cl, Br, -OH, -OCH₃, -OCF₃, -NH₂, -NH-C₁₋₄-Alkyl, -N(C₁₋₄-Alkyl)₂, -NH-C(O)-C₁₋₄-Alkyl, C₁₋₆-Alkyl, oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R²** H oder CH₃,
**R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **17** der vorliegenden Erfindung besteht in den Verbindungen der allgemeinen Formel **Ia**, in denen
**R¹** eine Gruppe ausgewählt aus 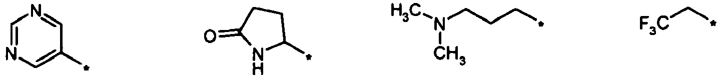 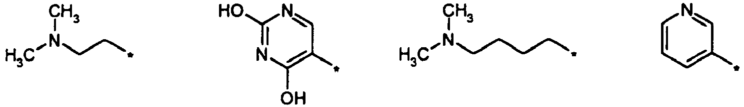 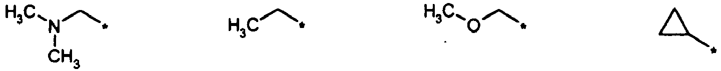 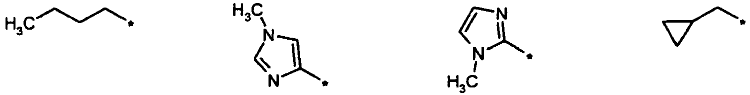 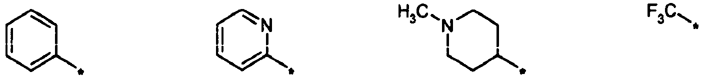 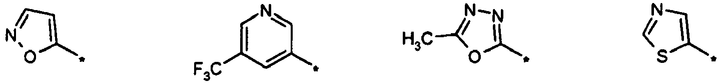 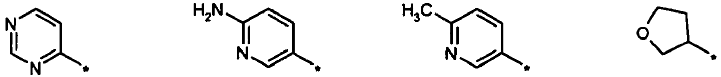 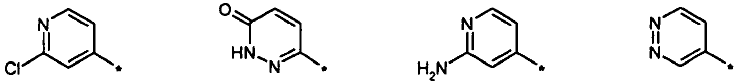 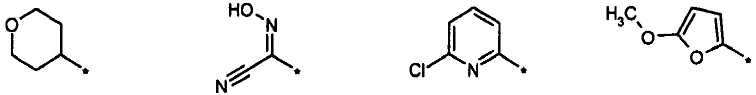 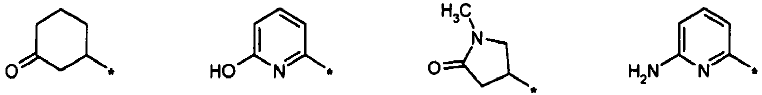 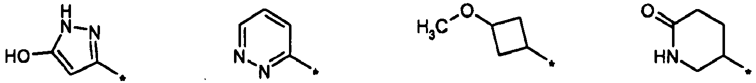 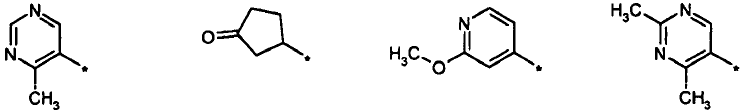 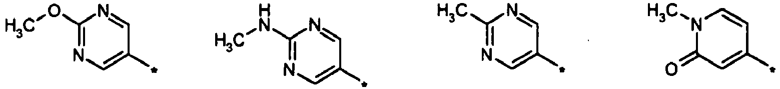 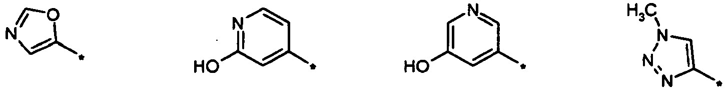 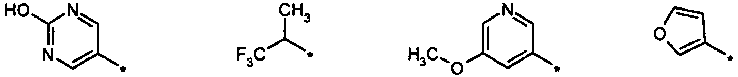 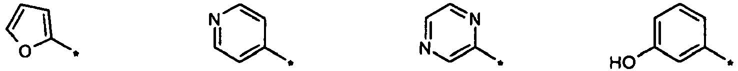 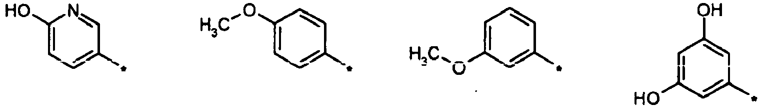 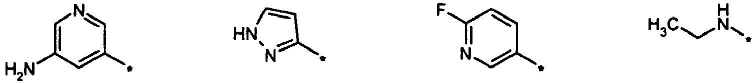 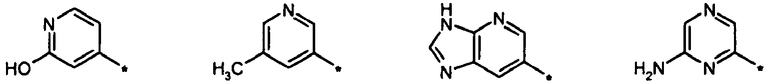 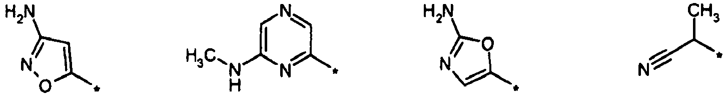 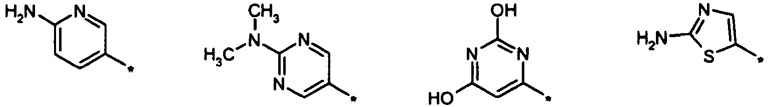 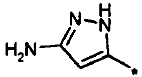 **R²** H oder CH₃,
**R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann, bedeutet,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **18** der vorliegenden Erfindung besteht in den Verbindungen der allgemeinen Formel **Ia**, in denen
**R¹** eine Gruppe ausgewählt aus 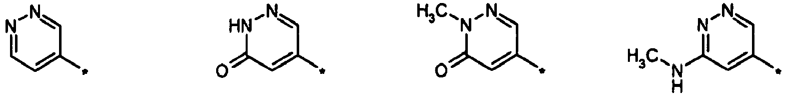 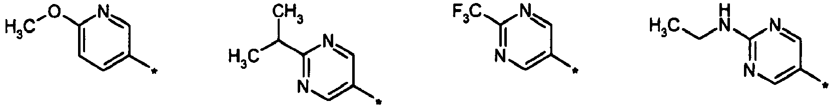 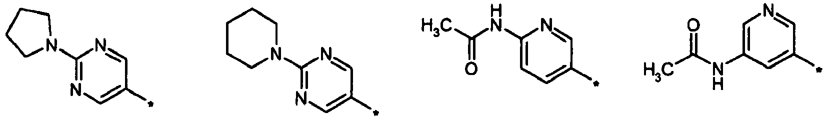 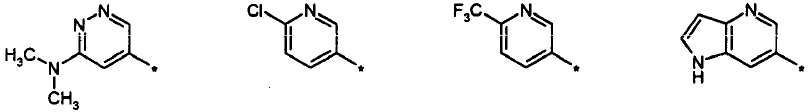 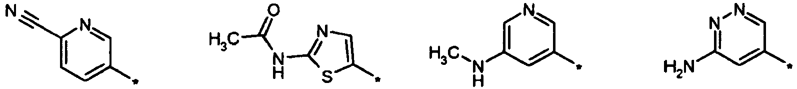 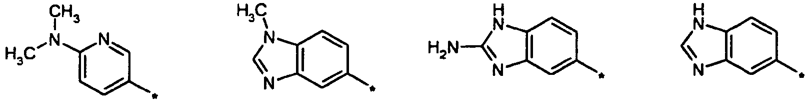 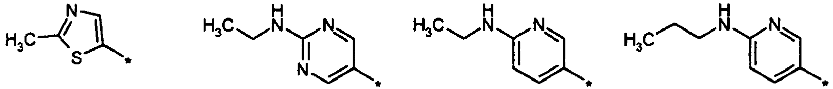 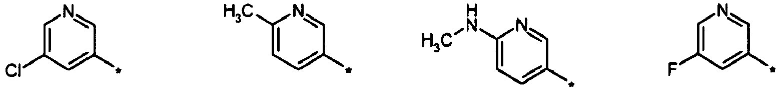 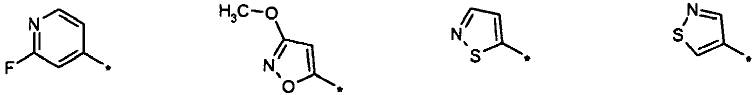 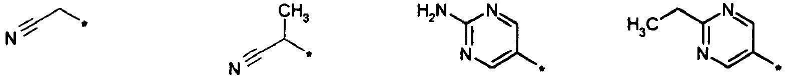 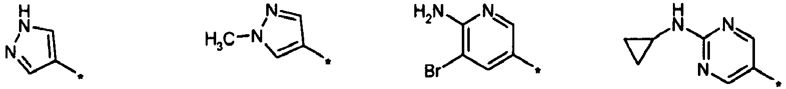 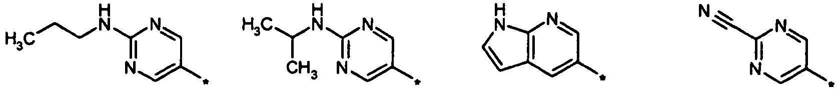 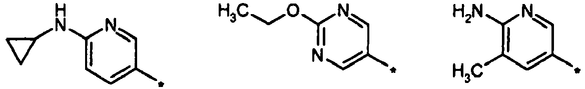 **R²** H oder CH₃,
**R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann, bedeutet,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **19** der vorliegenden Erfindung besteht in den Verbindungen der allgemeinen Formel **Ib** 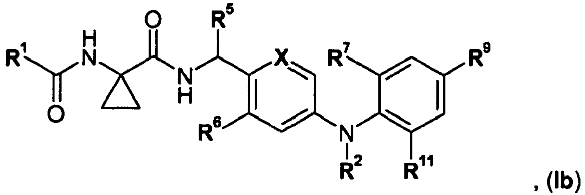 in der
**R¹** (a) eine gegebenenfalls mit einem Rest **R^{1.1}** substituierte C₁₋₆-Alkylgruppe,
(b) eine gegebenenfalls mit 1, 2 oder 3 Resten **R^{1.3}** substituierte Phenylgruppe,
(c) ein gegebenenfalls mit 1, 2 oder 3 Resten **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält,
(d) ein gegebenenfalls mit 1 oder 2 Resten **R^{1.4}** substituierter sechsgliedriger Heteroaryl-Rest, der ein, zwei oder drei N-Atome enthält,
(e) ein gegebenenfalls mit 1 oder 2 Resten **R^{1.4}** substituierter neun- oder zehngliedriger Heteroaryl-Rest, der ein, zwei oder drei N-Atome enthält,
(f) ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
**R^{1.1}** -CN, C₃₋₆-Cycloalkyl, -OH, -OCH₃, -NH₂, -NHCH₃, -N(CH₃)₂,
**R^{1.3}** unabhängig voneinander
(a) F, Cl, Br, -OH, -OCH₃, C₁₋₆-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
(a) F, Cl, Br, -OH, -OCH₃, -NH₂, -NHCH₃, -N(CH₃)₂, -NH-C(O)-C₁₋₄-Alkyl, C₁₋₆-Alkyl, **oder**
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R²** H oder CH₃,
**R⁵** H oder C₁₋₄-Alkyl,
**R⁶** H, F, Cl, Br oder C₁₋₄-Alkyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁-₄-Alkyl,
**R"** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **20** der vorliegenden Erfindung besteht in den Verbindungen der allgemeinen Formel Ib, in denen
**R¹** (a) eine gegebenenfalls mit einem Rest **R^{1.1}** substituierte C₁₋₆-Alkylgruppe,
(b) eine gegebenenfalls mit 1, 2 oder 3 Resten **R^{1.3}** substituierte Phenylgruppe,
(c) ein gegebenenfalls mit 1, 2 oder 3 Resten **R^{1.4}** substituierter fünfgliedriger Heteroaryl-Rest, der ausgewählt ist aus der Gruppe bestehend aus 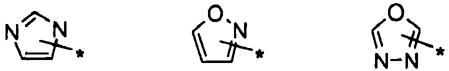 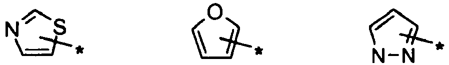 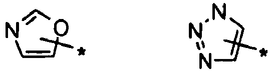 (d) ein gegebenenfalls mit 1 oder 2 Resten **R^{1.4}** substituierter sechsgliedriger Heteroaryl-Rest, der ausgewählt ist aus der Gruppe bestehend aus 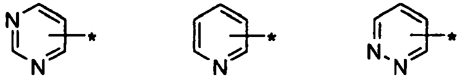 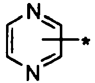 (e) ein gegebenenfalls mit 1 oder 2 Resten **R^{1.4}** substituierter neungliedriger Heteroaryl-Rest, der ausgewählt ist aus der Gruppe bestehend aus 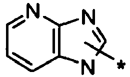 (f) ein gegebenenfalls mit 1 oder 2 Resten **R^{1.4}** substituierter 5- oder 6-gliedriger Heterocyclus, der ausgewählt ist aus der Gruppe bestehend aus 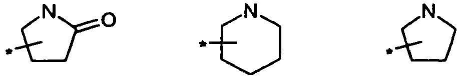 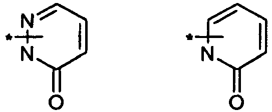 **R^{1.1}** -CN, Cyclopropyl, -OH, -OCH₃, -NH₂, -NHCH₃, -N(CH₃)₂,
**R^{1.3}** unabhängig voneinander
(a) F, Cl, Br, -OH, -OCH₃, -OCF₃, C₁₋₄-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, und
R^{1.4} unabhängig voneinander
(a) F, Cl, Br, -OH, -OCH₃, -OCF₃, -NH₂, -NH-C₁₋₄-Alkyl, -N(C₁₋₄-Alkyl)₂, -NH-C(O)-C₁₋₄-Alkyl, C₁₋₆-Alkyl, oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R²** H oder CH₃,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **21** der vorliegenden Erfindung besteht in den Verbindungen der allgemeinen Formel Ib, in denen
**R¹** eine Gruppe ausgewählt aus 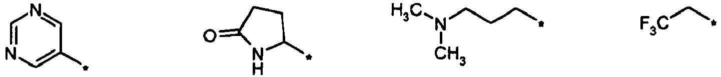 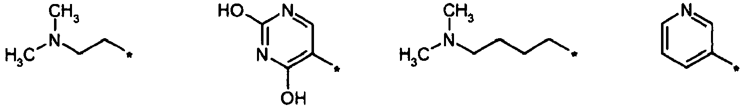 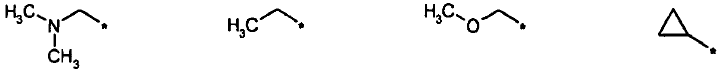 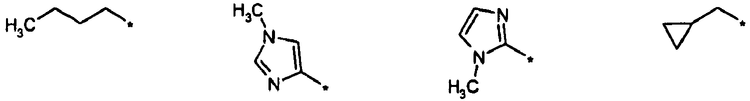 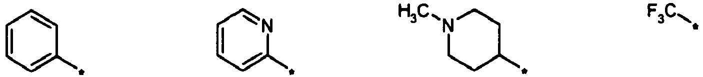 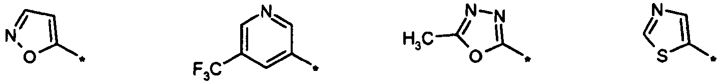 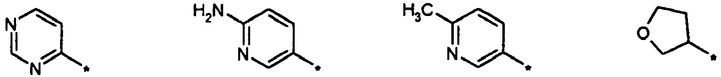 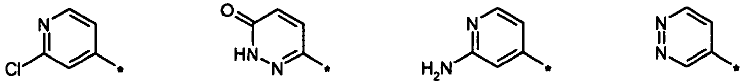 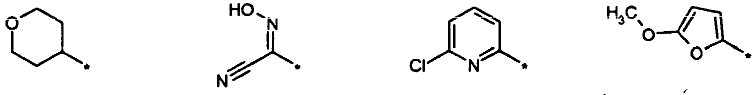 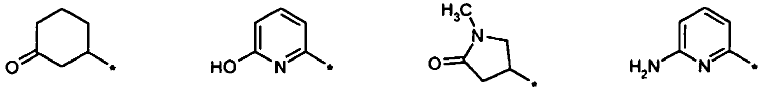 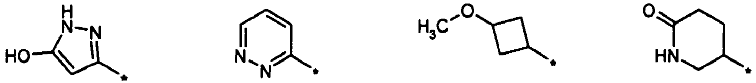 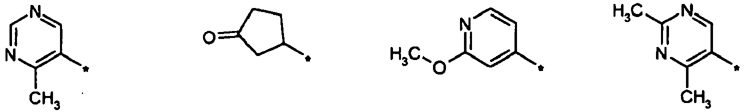 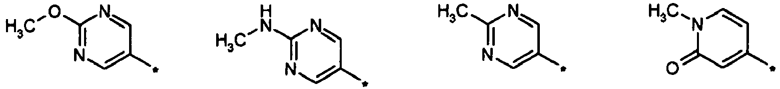 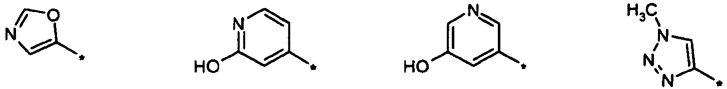 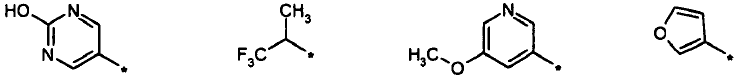 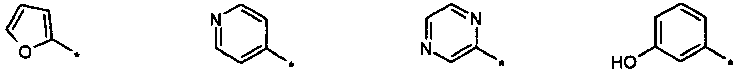 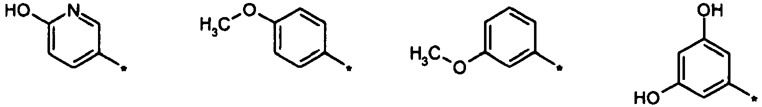 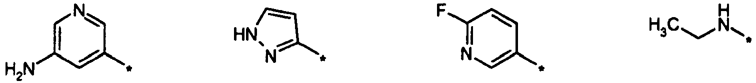 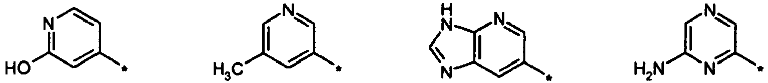 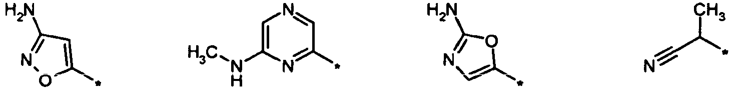 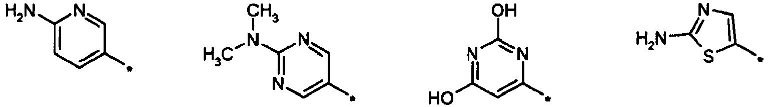 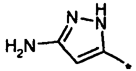 **R²** H oder CH₃,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten, deren Enantiomere, deren Diastereomere, deren Gemische und deren Salze, insbesondere deren physiologisch verträgliche Salze mit organischen oder anorganischen Säuren oder Basen.

Eine Ausführungsform **22** der vorliegenden Erfindung besteht in den Verbindungen der allgemeinen Formel Ib, in denen
**R¹** eine Gruppe ausgewählt aus 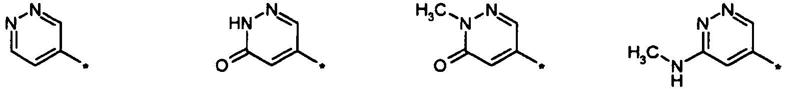 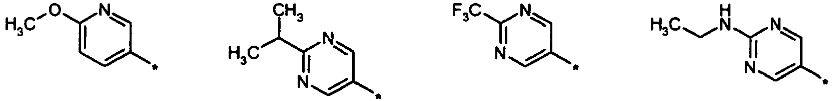 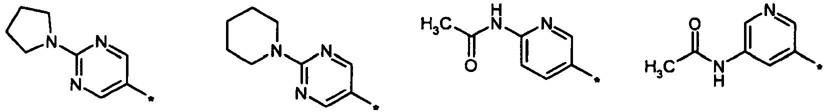 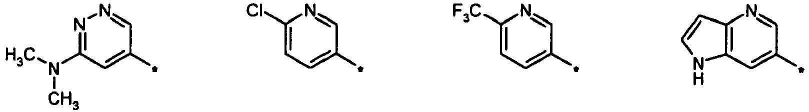 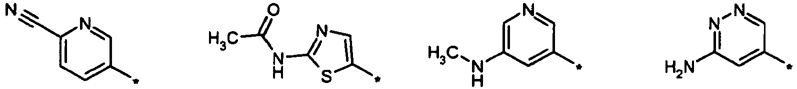 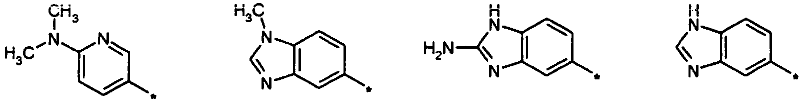 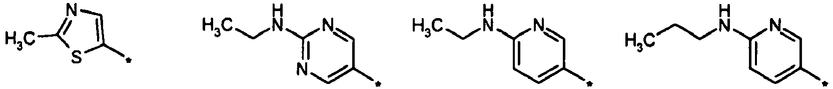 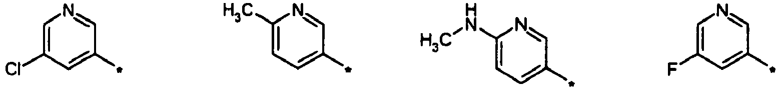 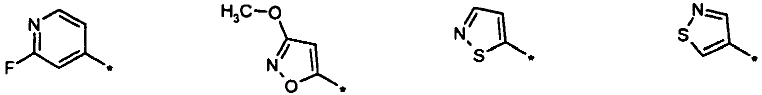 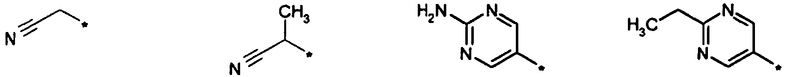 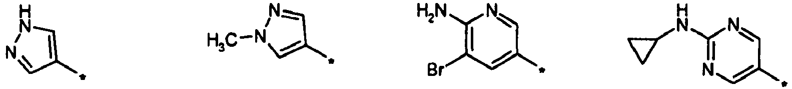 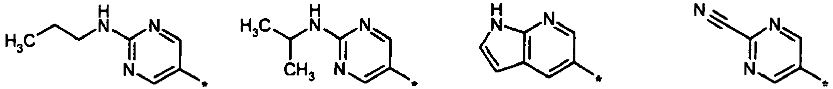 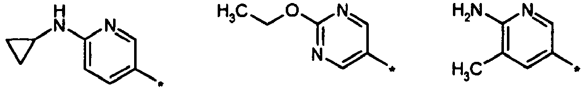 **R²** H,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **23** der vorliegenden Erfindung besteht in den Verbindungen der allgemeinen Formel **Ic** 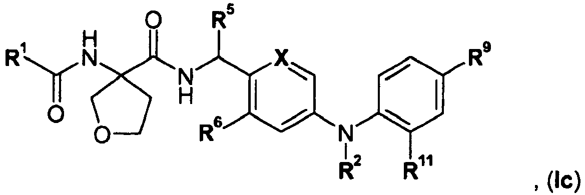 in der
**R¹** (a) eine gegebenenfalls mit einem Rest **R^{1.1}** substituierte C₁₋₆-Alkylgruppe,
(b) eine gegebenenfalls mit 1, 2 oder 3 Resten **R^{1,3}** substituierte Phenylgruppe,
(c) ein gegebenenfalls mit 1, 2 oder 3 Resten **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält,
(d) ein gegebenenfalls mit 1 oder 2 Resten **R^{1.4}** substituierter sechsgliedriger Heteroaryl-Rest, der ein, zwei oder drei N-Atome enthält,
(e) ein gegebenenfalls mit 1 oder 2 Resten **R^{1.4}** substituierter neun- oder zehngliedriger Heteroaryl-Rest, der ein, zwei oder drei N-Atome enthält,
(f) ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
**R^{1.1}** -CN, C₃₋₆-Cycloalkyl, -OH, -OCH₃, -NH₂, -NHCH₃, -N(CH₃)₂,
**R^{1.3}** unabhängig voneinander
(a) F, Cl, Br, -OH, -OCH₃, C₁₋₆-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
(a) F, Cl, Br, -OH, -OCH₃, -NH₂, -NHCH₃, -N(CH₃)₂, -NH-C(O)-C₁₋₄-Alkyl, C₁₋₆-Alkyl, oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R²** H oder CH₃,
**R⁵** H oder C₁₋₄-Alkyl,
**R⁶** H, F, Cl, Br oder C₁₋₄-Alkyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **24** der vorliegenden Erfindung besteht in den Verbindungen der allgemeinen Formel Ic, in denen
**R¹** (a) eine gegebenenfalls mit einem Rest **R^{1.1}** substituierte C₁₋₆-Alkylgruppe,
(b) eine gegebenenfalls mit 1, 2 oder 3 Resten **R^{1.3}** substituierte Phenylgruppe,
(c) ein gegebenenfalls mit 1, 2 oder 3 Resten **R^{1.4}** substituierter fünfgliedriger Heteroaryl-Rest, der ausgewählt ist aus der Gruppe bestehend aus 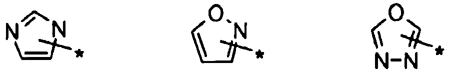 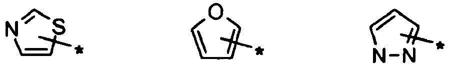 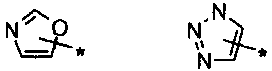 (d) ein gegebenenfalls mit 1 oder 2 Resten R^{1.4} substituierter sechsgliedriger Heteroaryl-Rest, der ausgewählt ist aus der Gruppe bestehend aus 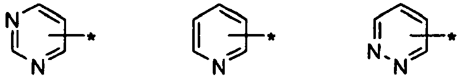 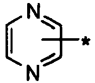 (e) ein gegebenenfalls mit 1 oder 2 Resten **R^{1.4}** substituierter neungliedriger Heteroaryl-Rest, der ausgewählt ist aus der Gruppe bestehend aus 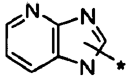 (f) ein gegebenenfalls mit 1 oder 2 Resten **R^{1.4}** substituierter 5- oder 6-gliedriger Heterocyclus, der ausgewählt ist aus der Gruppe bestehend aus 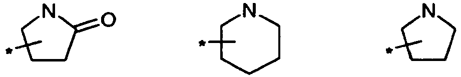 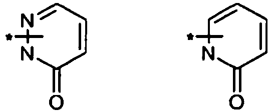 **R^{1.1}** -CN, Cyclopropyl, -OH, -OCH₃, -NH₂, -NHCH₃, -N(CH₃)₂,
**R^{1.3}** unabhängig voneinander
(a) F, Cl, Br, -OH, -OCH₃, -OCF₃, C₁₋₄-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
(a) F, Cl, Br, -OH, -OCH₃, -OCF₃, -NH₂, -NH-C₁₋₄-Alkyl, -N(C₁₋₄-Alkyl)₂, -NH-C(O)-C₁₋₄-Alkyl, C₁₋₆-Alkyl, oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R²** H oder CH₃,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **25** der vorliegenden Erfindung besteht in den Verbindungen der allgemeinen Formel Ic, in denen
**R¹** eine Gruppe ausgewählt aus 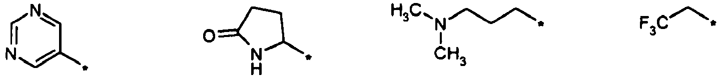 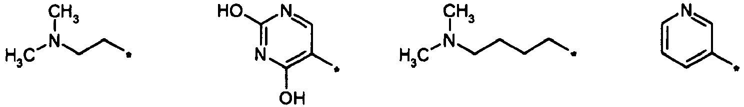 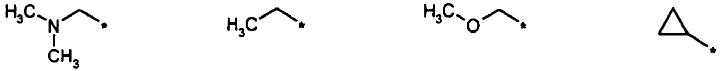 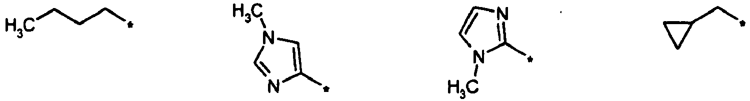 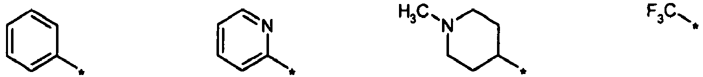 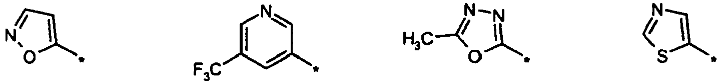 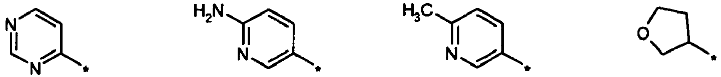 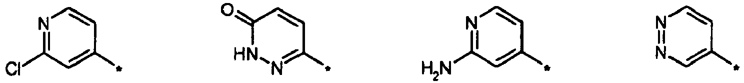 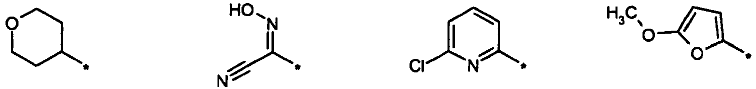 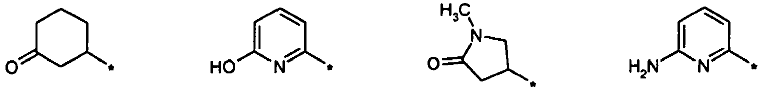 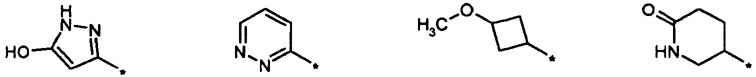 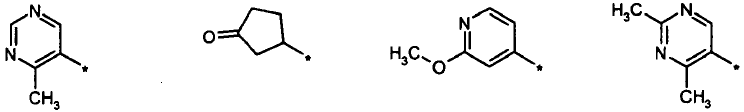 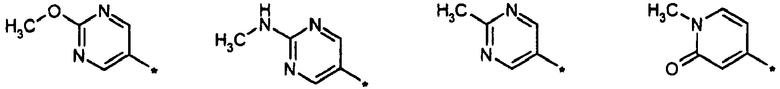 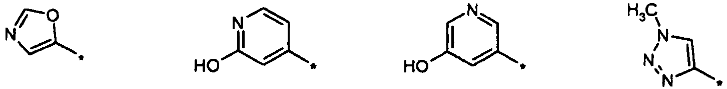 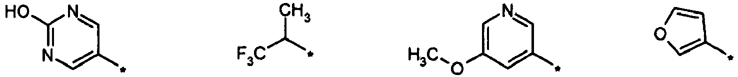 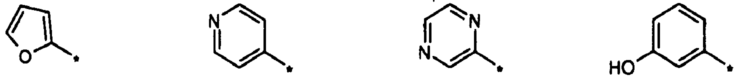 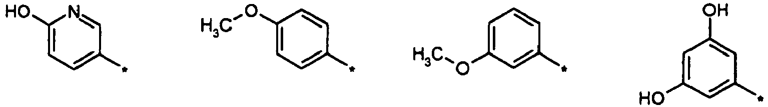 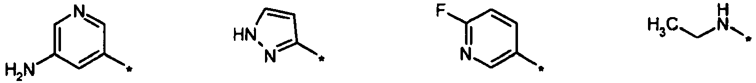 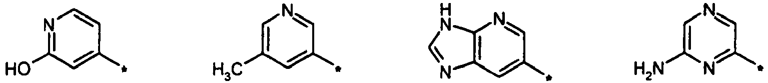 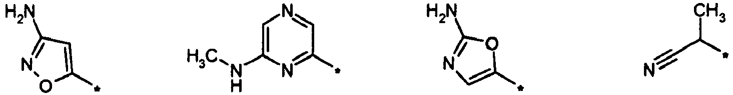 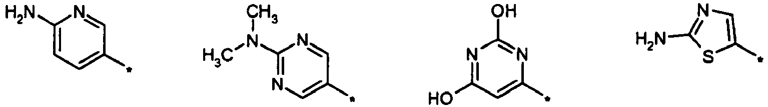 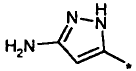 **R²** H oder CH₃,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten, deren Enantiomere, deren Diastereomere, deren Gemische und deren Salze, insbesondere deren physiologisch verträgliche Salze mit organischen oder anorganischen Säuren oder Basen.

Eine Ausführungsform **26** der vorliegenden Erfindung besteht in den Verbindungen der allgemeinen Formel Ic, in denen
**R¹** eine Gruppe ausgewählt aus 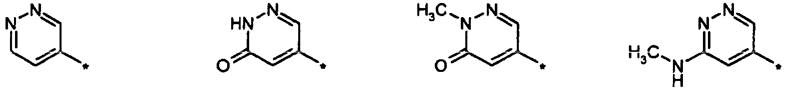 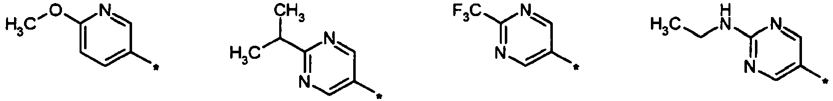 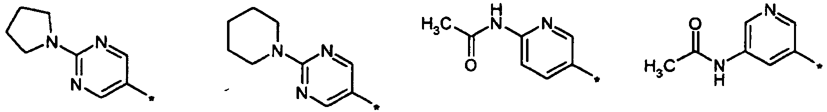 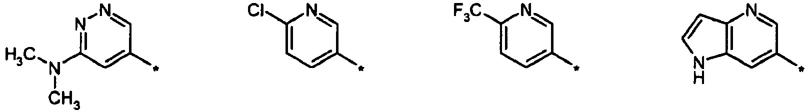 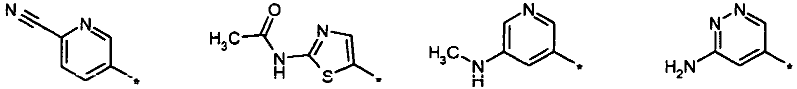 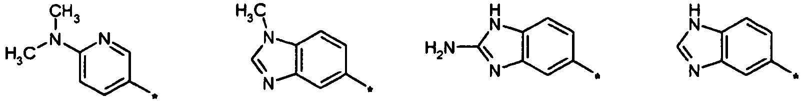 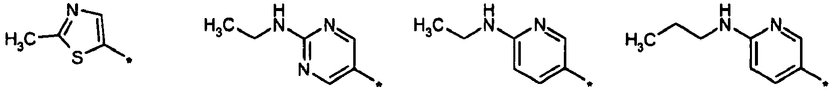 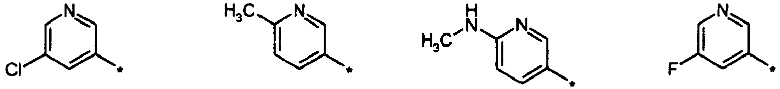 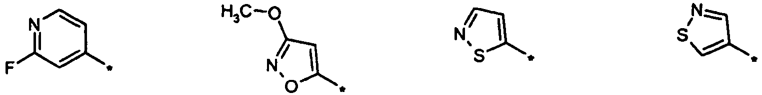 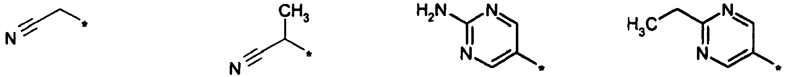 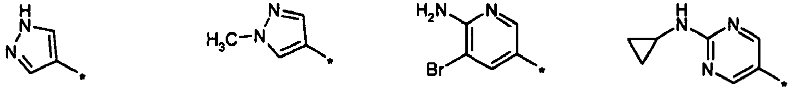 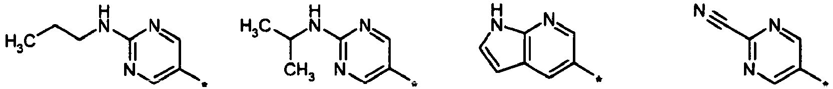 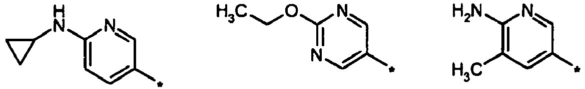 **R²** H oder CH₃,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **27** der vorliegenden Erfindung besteht in den Verbindungen der allgemeinen Formel Id 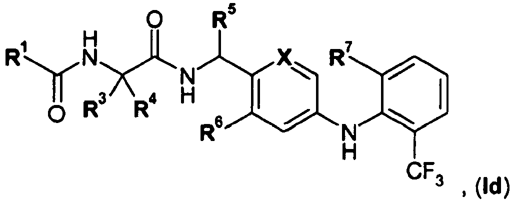 in der
**R¹** eine Gruppe ausgewählt aus 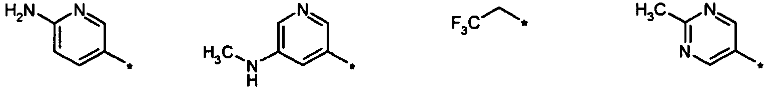 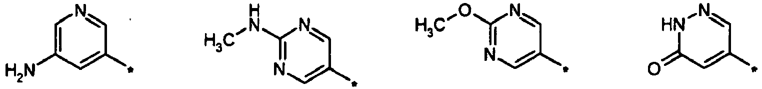 **R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann,
**R⁵** H oder CH₃,
**R⁶** Cl oder CH₃,
**R⁷** H oder F,
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **28** der vorliegenden Erfindung besteht in den Verbindungen der allgemeinen Formel **I, Ia,** Ib, **Ic** oder Id, in denen **n, R¹, R³, R⁴, R⁵, R⁶, R⁷, R⁸, R⁹, R¹⁰, R¹¹** und **X** wie voranstehend in Ausführungsform 1, 2, 3, 4, 5, 6, 7, 8, 9, 10, 11, 12, 13, 14, 15, 16, 17, 18, 19, 20, 21, 22, 23, 24, 25, 26 oder 27 beschrieben definiert sind und
**R²** H bedeutet,
deren Enantiomere, deren Diastereomere, deren Gemische und deren Salze, insbesondere deren physiologisch verträgliche Salze mit organischen oder anorganischen Säuren oder Basen.

Als ganz besonders bevorzugte Verbindungen der obigen allgemeinen Formel I seien beispielsweise folgende genannt:

| **Nr.** | **Struktur** |
|---|---|
| (1) | 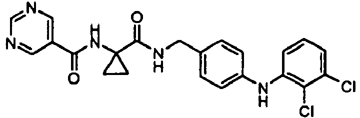 |
| (2) | 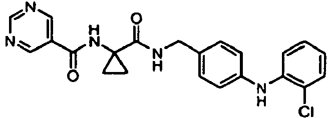 |
| (3) | 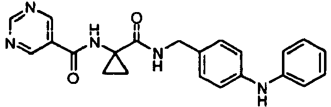 |
| (4) | 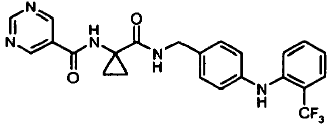 |
| (5) | 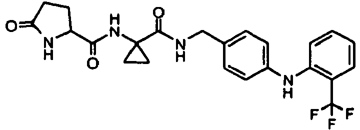 |
| (6) | 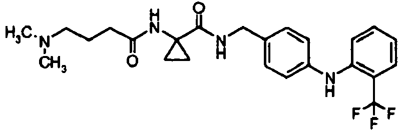 |
| (7) | 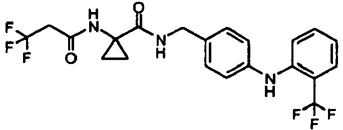 |
| (8) | 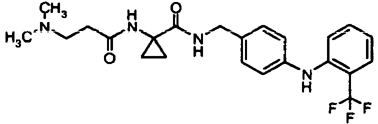 |
| (9) | 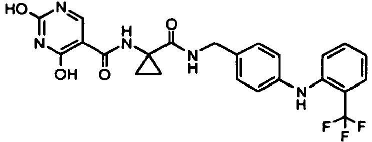 |
| (10) | 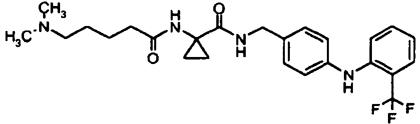 |
| (11) | 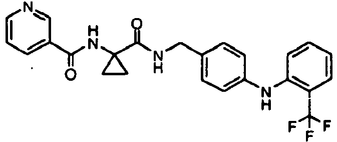 |
| (12) | 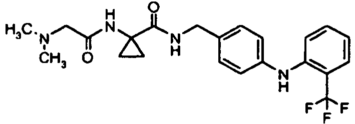 |
| (13) | 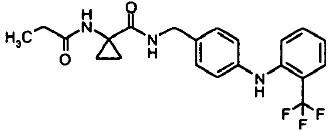 |
| (14) | 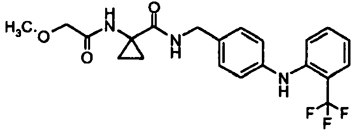 |
| (15) | 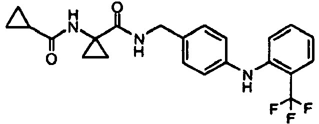 |
| (16) | 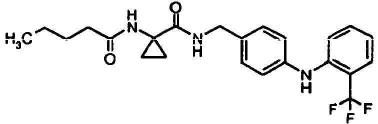 |
| (17) | 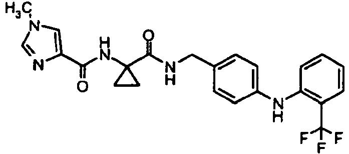 |
| (18) | 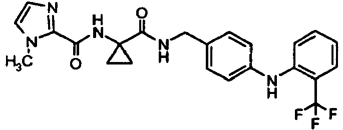 |
| (19) | 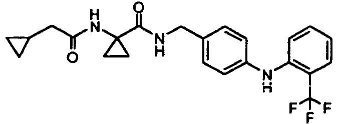 |
| (20) | 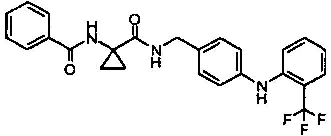 |
| (21) | 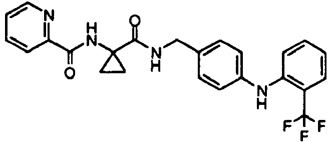 |
| (22) | 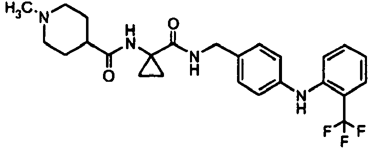 |
| (23) | 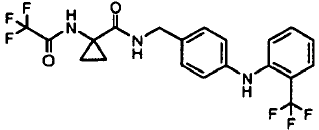 |
| (24) | 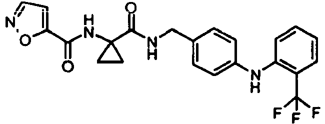 |
| (25) | 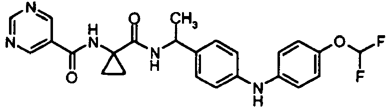 |
| (26) | 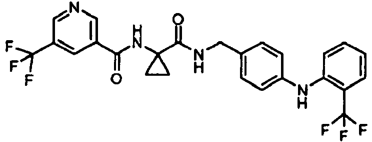 |
| (27) | 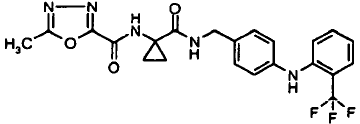 |
| (28) | 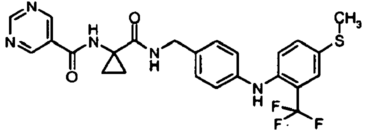 |
| (29) | 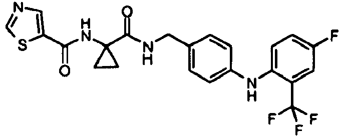 |
| (30) | 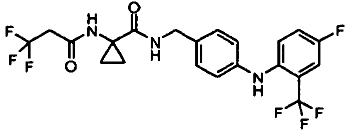 |
| (31) | 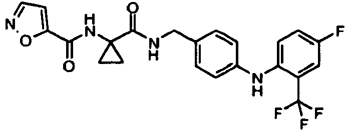 |
| (32) | 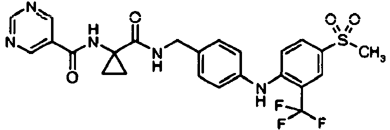 |
| (33) | 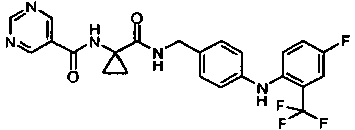 |
| (34) | 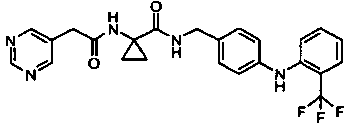 |
| (35) | 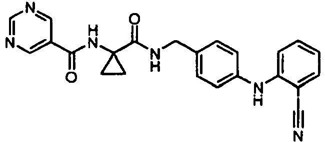 |
| (36) | 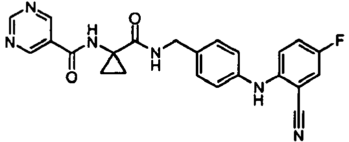 |
| (37) | 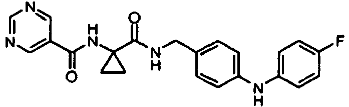 |
| (38) | 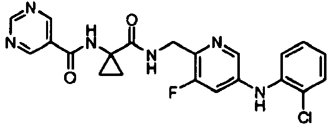 |
| (39) | 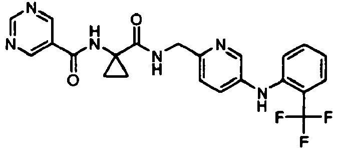 |
| (40) | 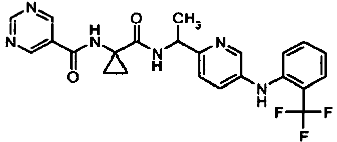 |
| (41) | 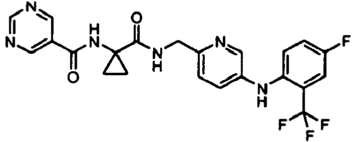 |
| (42) | 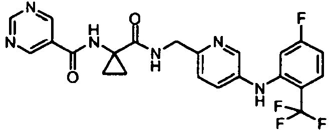 |
| (43) | 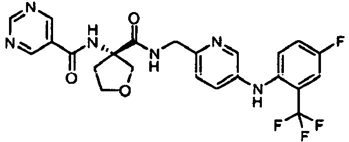 |
| (44) | 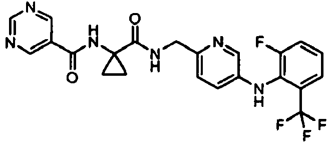 |
| (45) | 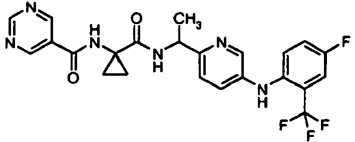 |
| (46) | 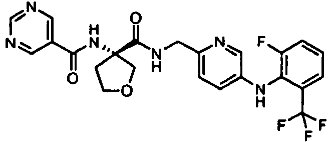 |
| (47) | 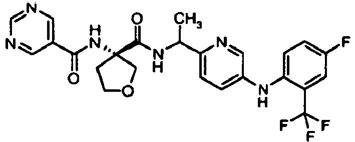 |
| (48) | 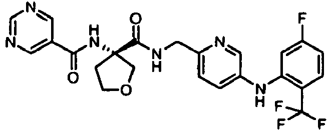 |
| (49) | 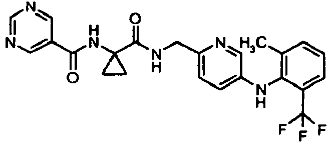 |
| (50) | 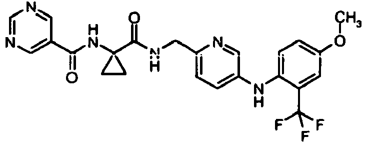 |
| (51) | 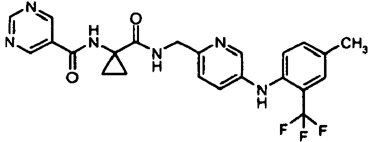 |
| (52) | 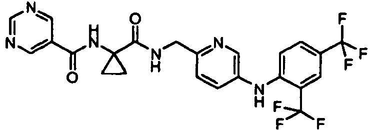 |
| (53) | 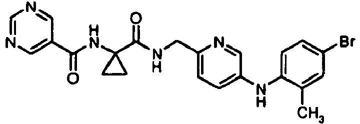 |
| (54) | 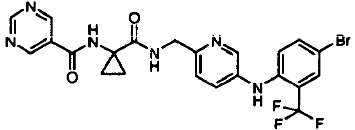 |
| (55) | 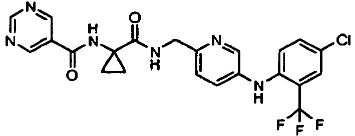 |
| (56) | 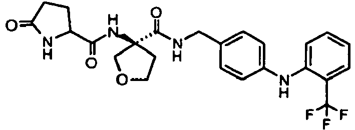 |
| (57) | 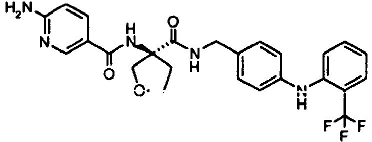 |
| (58) | 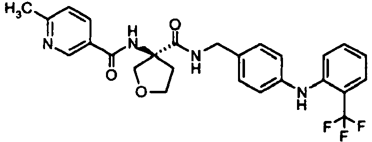 |
| (59) | 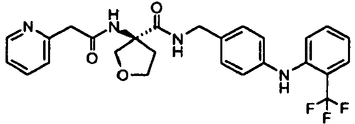 |
| (60) | 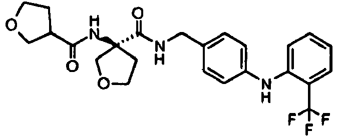 |
| (61) | 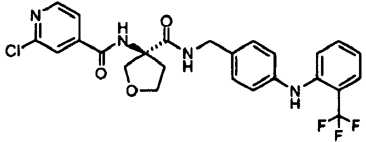 |
| (62) | 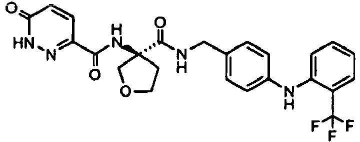 |
| (63) | 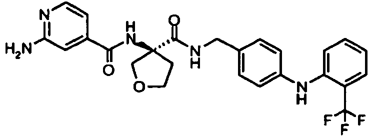 |
| (64) | 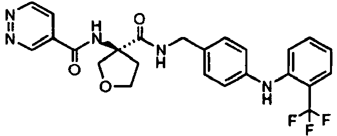 |
| (65) | 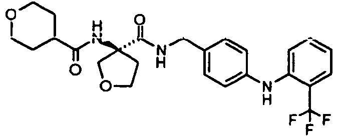 |
| (66) | 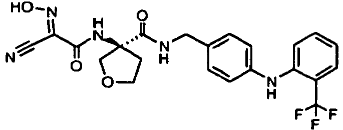 |
| (67) | 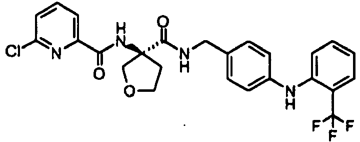 |
| (68) | 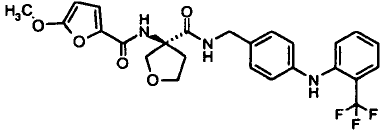 |
| (69) | 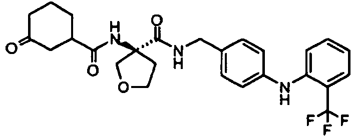 |
| (70) | 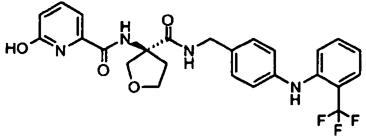 |
| (71) | 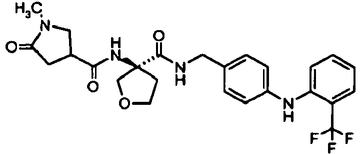 |
| (72) | 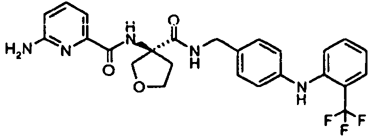 |
| (73) | 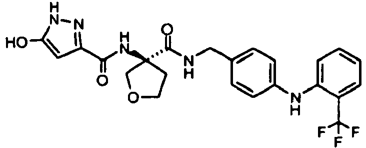 |
| (74) | 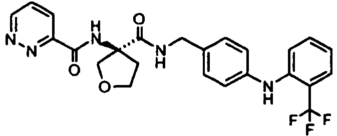 |
| (75) | 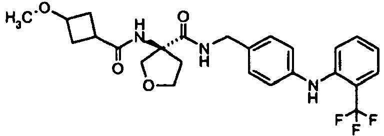 |
| (76) | 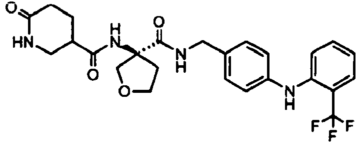 |
| (77) | 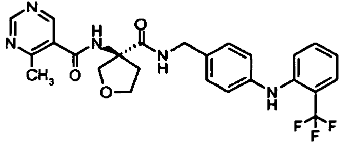 |
| (78) | 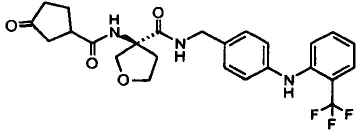 |
| (79) | 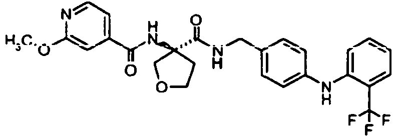 |
| (80) | 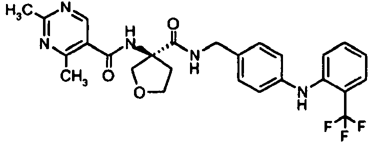 |
| (81) | 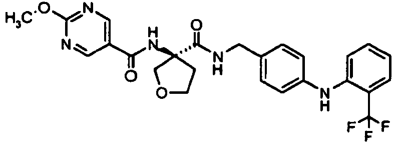 |
| (82) | 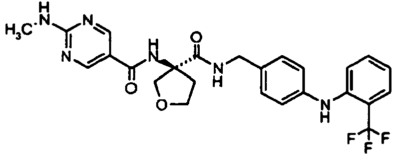 |
| (83) | 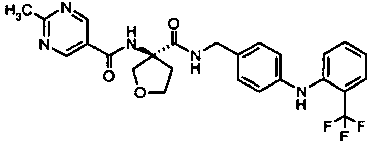 |
| (84) | 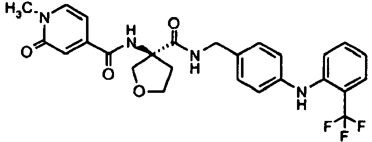 |
| (85) | 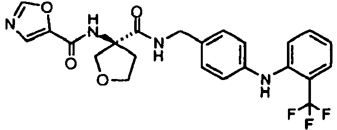 |
| (86) | 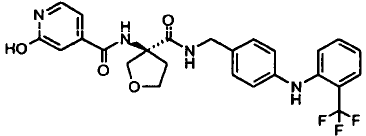 |
| (87) | 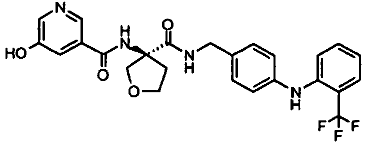 |
| (88) | 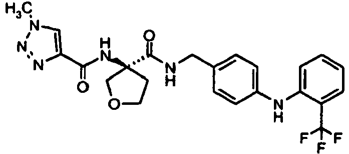 |
| (89) | 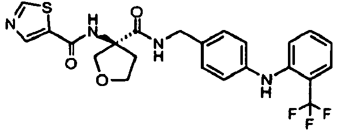 |
| (90) | 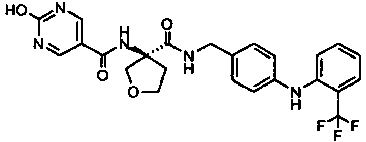 |
| (91) | 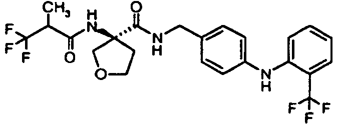 |
| (92) | 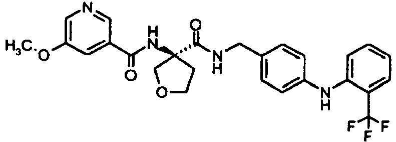 |
| (93) | 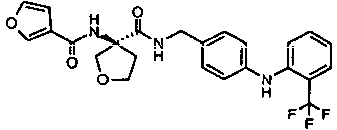 |
| (94) | 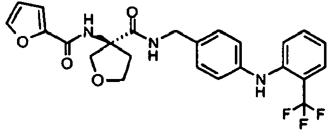 |
| (95) | 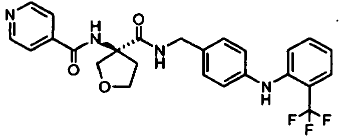 |
| (96) | 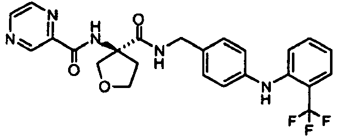 |
| (97) | 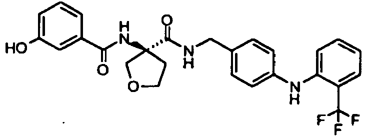 |
| (98) | 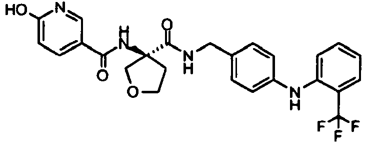 |
| (99) | 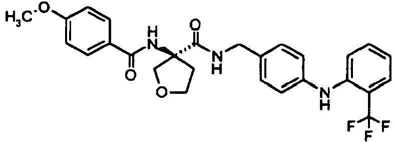 |
| (100) | 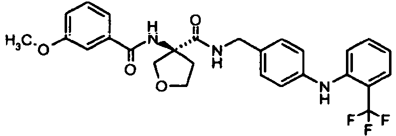 |
| (101) | 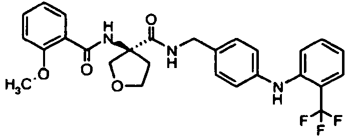 |
| (102) | 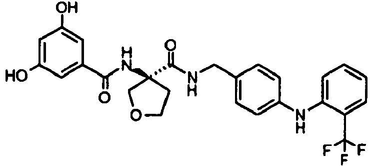 |
| (103) | 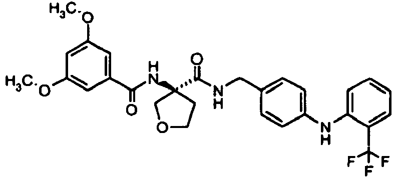 |
| (104) | 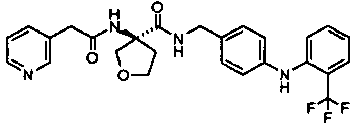 |
| (105) | 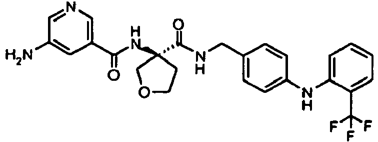 |
| (106) | 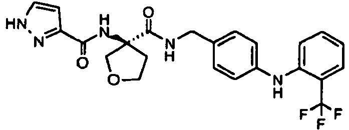 |
| (107) | 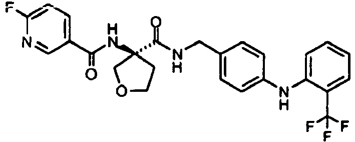 |
| (108) | 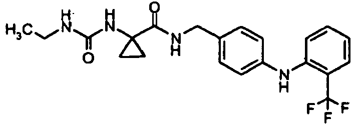 |
| (109) | 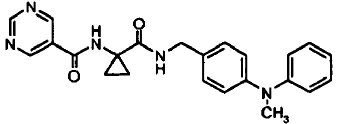 |
| (110) | 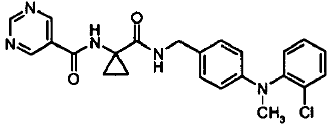 |
| (111) | 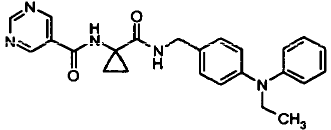 |
| (112) | 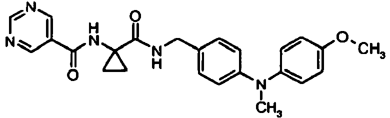 |
| (113) | 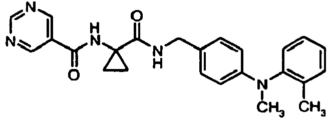 |
| (114) | 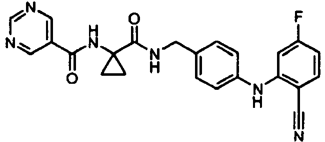 |
| (115) | 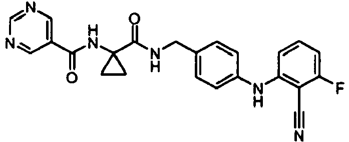 |
| (116) | 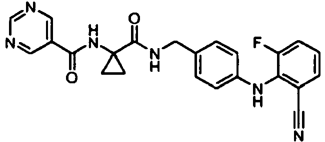 |
| (117) | 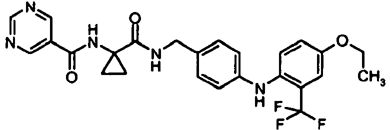 |
| (118) | 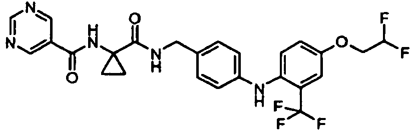 |
| (119) | 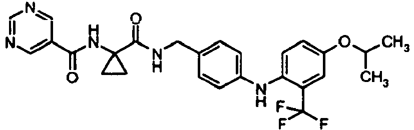 |
| (120) | 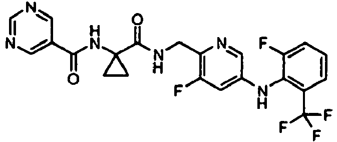 |
| (121) | 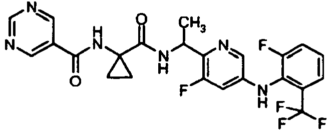 |
| (122) | 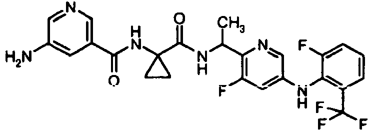 |
| (123) | 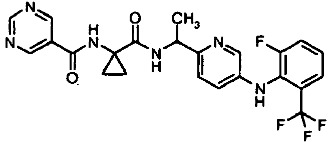 |
| (124) | 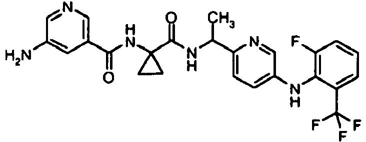 |
| (125) | 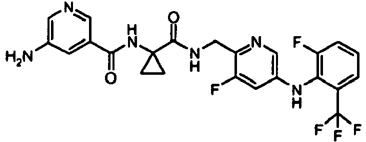 |
| (126) | 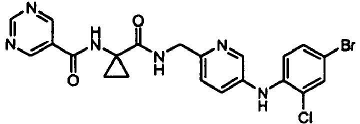 |
| (127) | 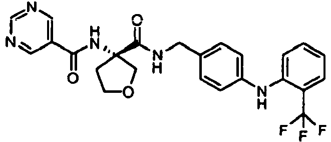 |
| (128) | 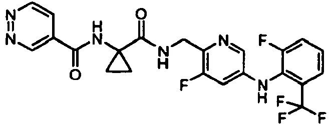 |
| (129) | 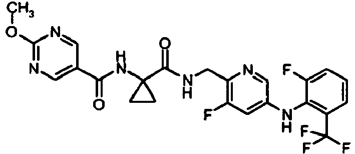 |
| (130) | 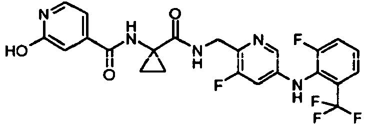 |
| (131) | 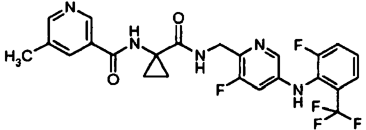 |
| (132) | 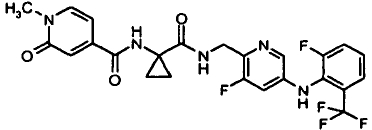 |
| (133) | 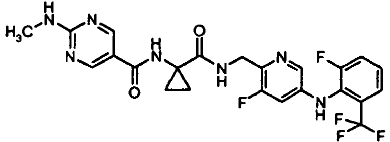 |
| (134) | 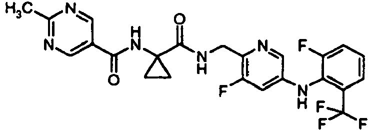 |
| (135) | 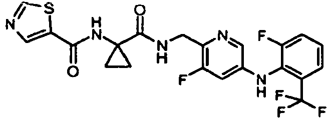 |
| (136) | 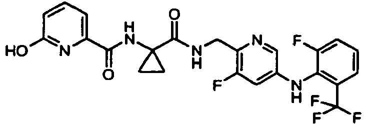 |
| (137) | 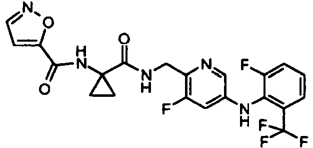 |
| (138) | 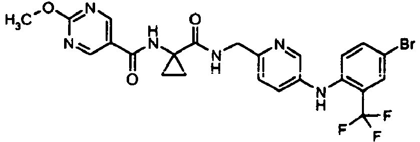 |
| (139) | 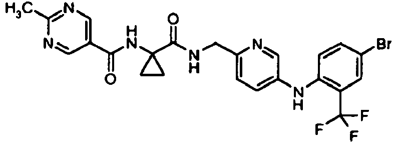 |
| (140) | 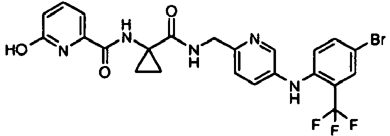 |
| (141) | 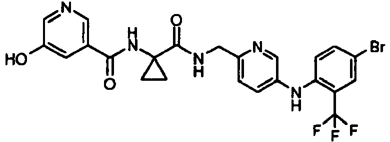 |
| (142) | 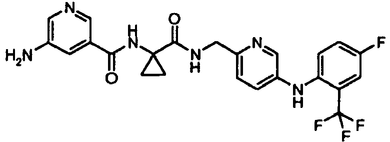 |
| (143) | 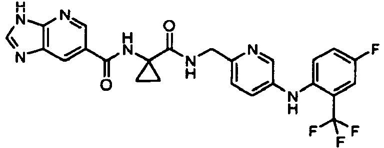 |
| (144) | 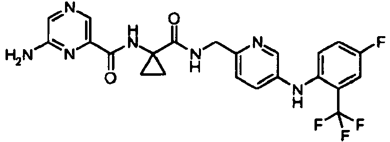 |
| (145) | 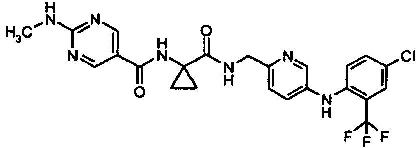 |
| (146) | 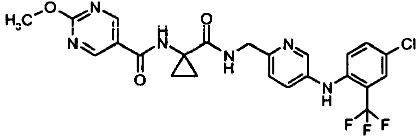 |
| (147) | 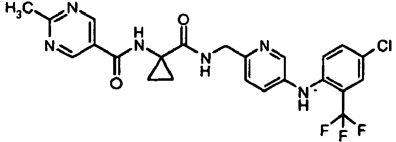 |
| (148) | 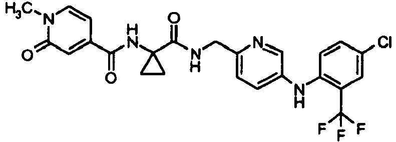 |
| (149) | 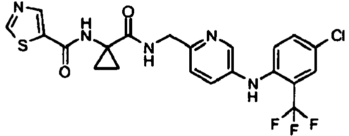 |
| (150) | 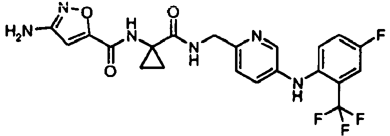 |
| (151) | 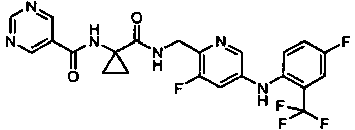 |
| (152) | 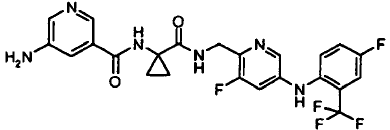 |
| (153) | 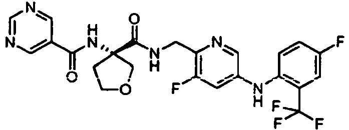 |
| (154) | 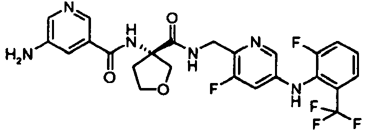 |
| (155) | 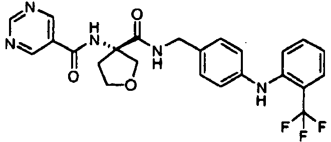 |
| (156) | 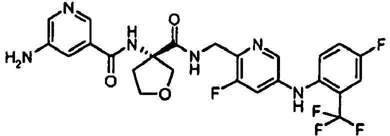 |
| (157) | 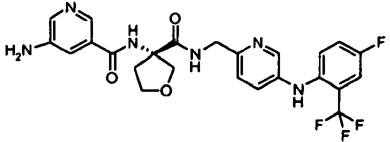 |
| (158) | 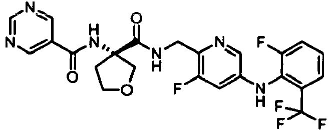 |
| (159) | 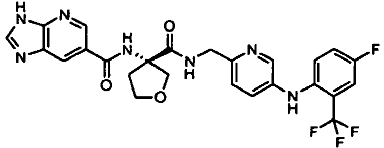 |
| (160) | 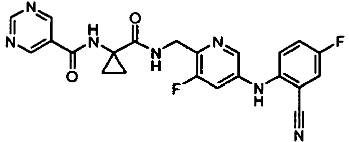 |
| (161) | 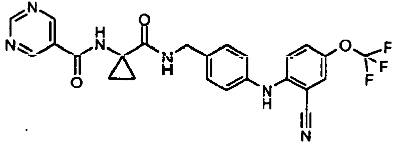 |
| (162) | 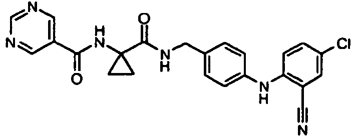 |
| (163) | 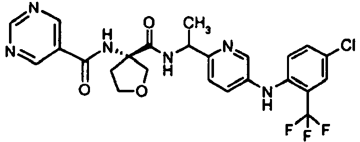 |
| (164) | 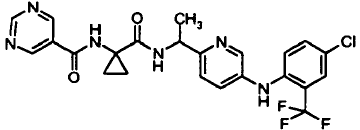 |
| (165) | 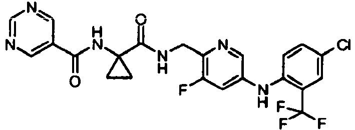 |
| (166) | 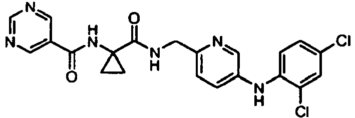 |
| (167) | 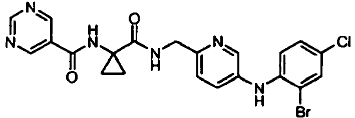 |
| (168) | 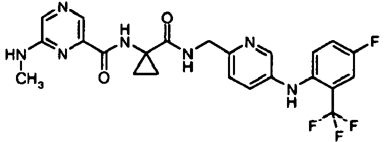 |
| (169) | 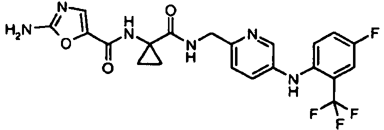 |
| (170) | 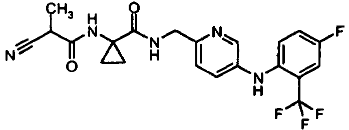 |
| (171) | 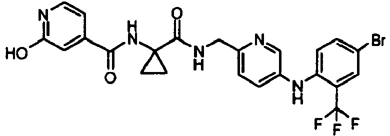 |
| (172) | 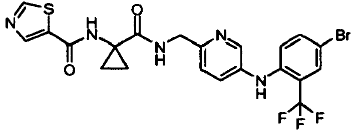 |
| (173) | 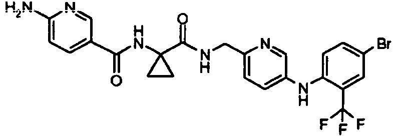 |
| (174) | 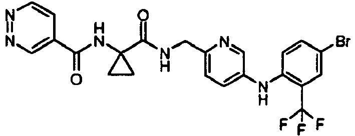 |
| (175) | 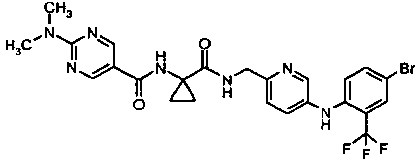 |
| (176) | 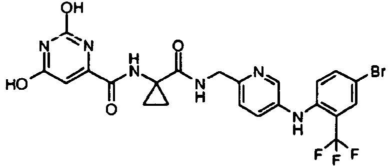 |
| (177) | 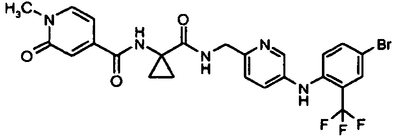 |
| (178) | 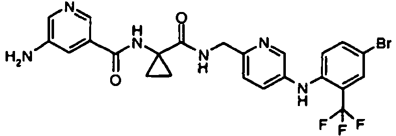 |
| (179) | 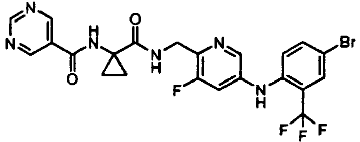 |
| (180) | 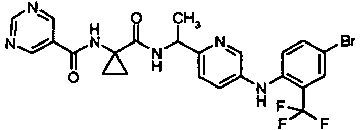 |
| (181) | 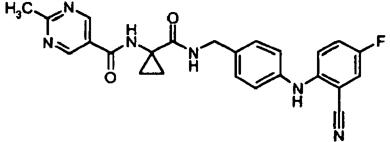 |
| (182) | 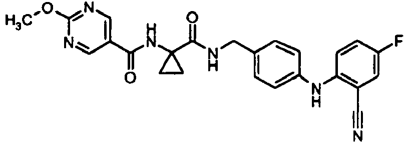 |
| (183) | 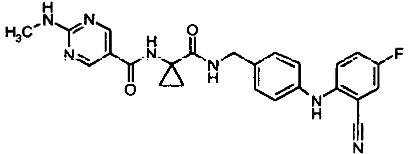 |
| (184) | 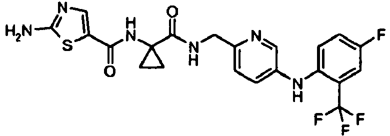 |
| (185) | 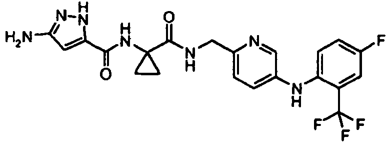 |
| (186) | 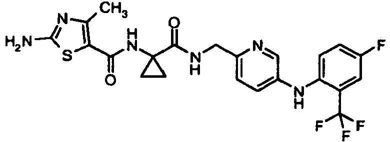 |
| (187) | 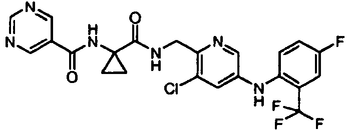 |
| (188) | 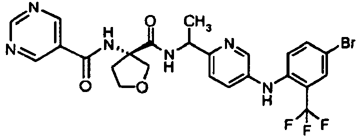 |
| (189) | 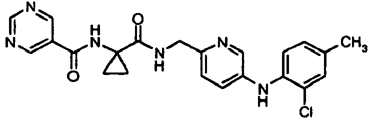 |
| (190) | 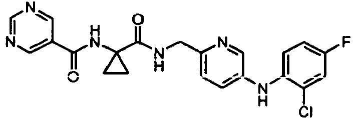 |
| (191) | 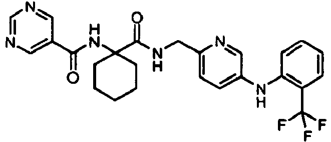 |
| (192) | 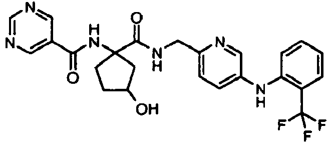 |
| (193) | 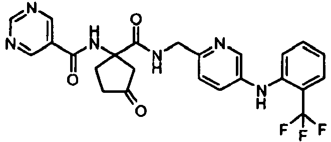 |
| (194) | 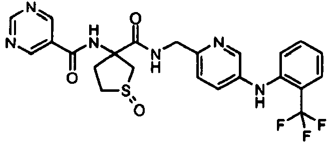 |
| (195) | 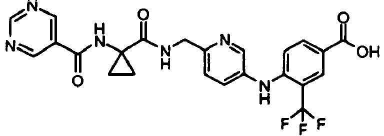 |
| (196) | 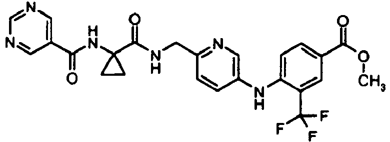 |
| (197) | 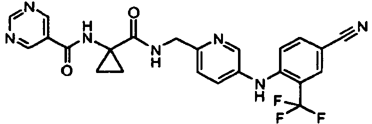 |
| (198) | 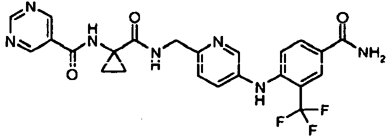 |
| (199) | 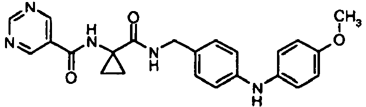 |
| (200) | 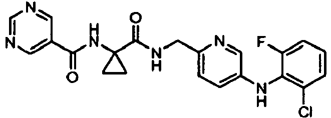 |
| (201) | 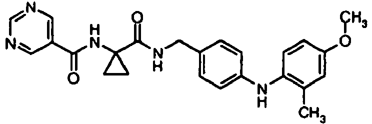 |
| (202) | 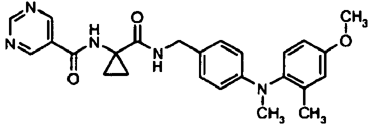 |
| (203) | 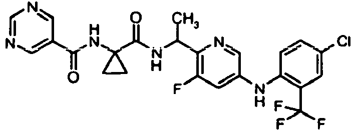 |
| (203a) | 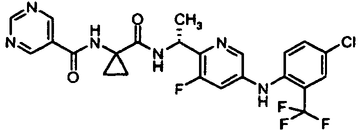 |
| (203b) | 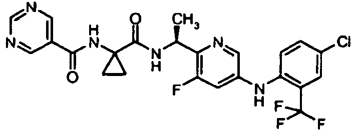 |
| (204) | 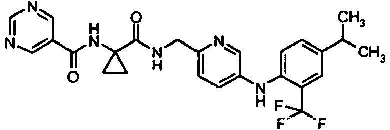 |
| (205) | 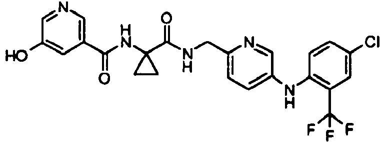 |
| (206) | 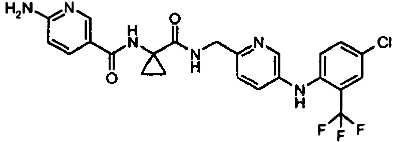 |
| (207) | 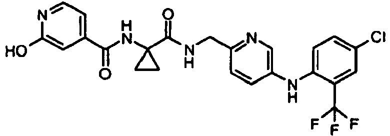 |
| (208) | 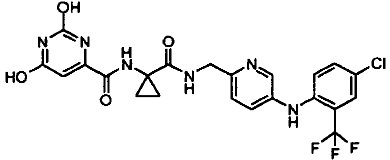 |
| (209) | 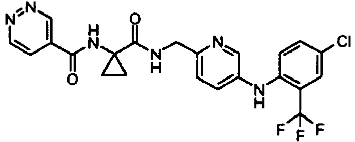 |
| (210) | 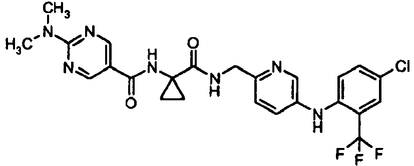 |
| (211) | 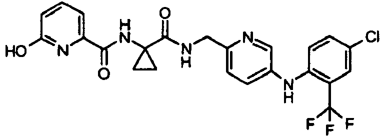 |
| (212) | 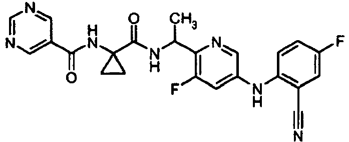 |
| (212a) | 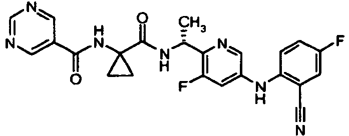 |
| (212b) | 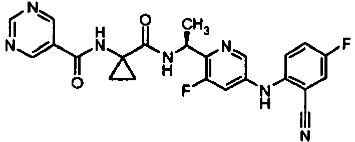 |
| (213) | 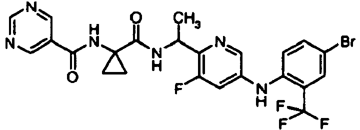 |
| (213a) | 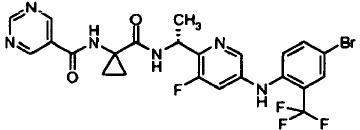 |
| (213b) | 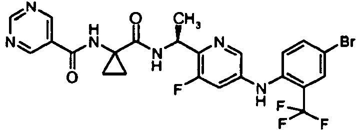 |
| (214) | 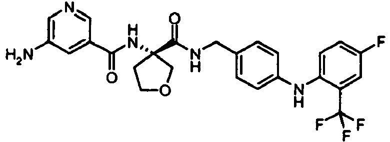 |
| (215) | 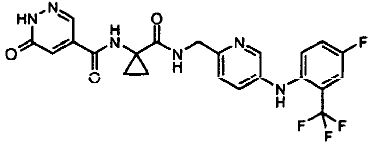 |
| (216) | 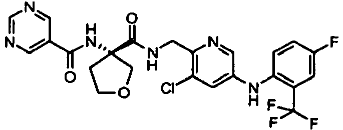 |
| (217) | 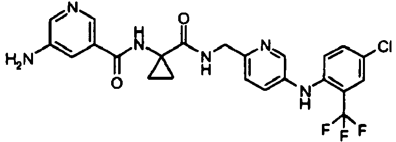 |
| (218) | 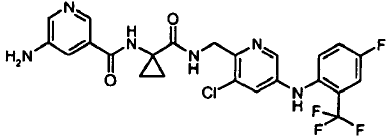 |
| (219) | 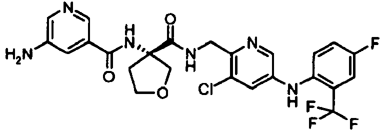 |
| (220) | 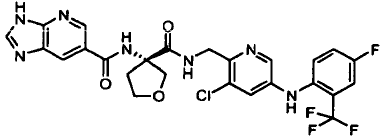 |
| (221) | 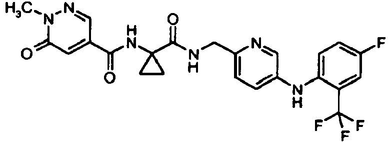 |
| (222) | 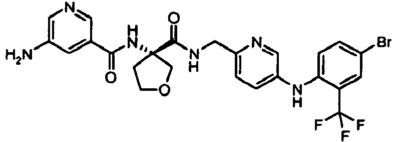 |
| (223) | 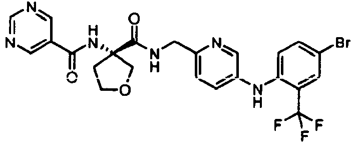 |
| (224) | 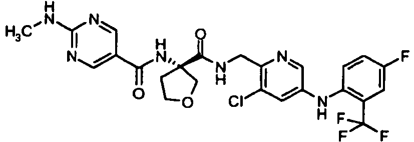 |
| (225) | 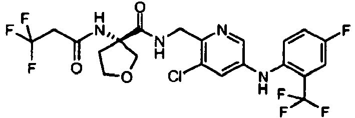 |
| (226) | 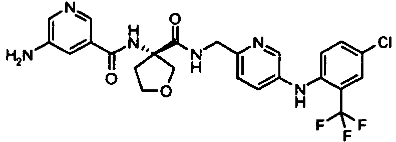 |
| (227) | 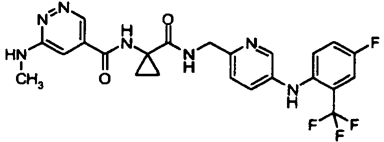 |
| (228) | 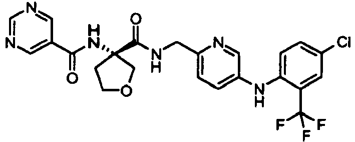 |
| (229) | 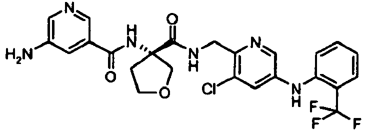 |
| (230) | 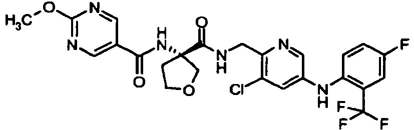 |
| (231) | 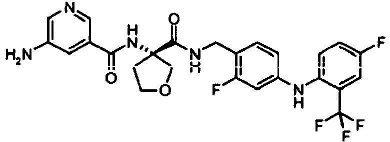 |
| (232) | 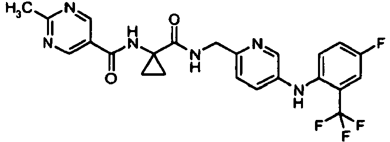 |
| (233) | 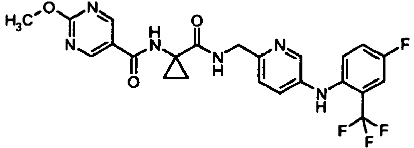 |
| (234) | 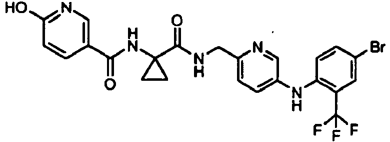 |
| (235) | 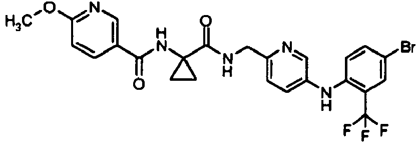 |
| (236) | 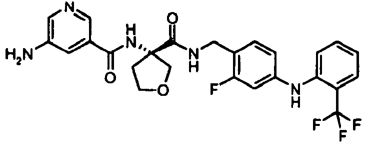 |
| (237) | 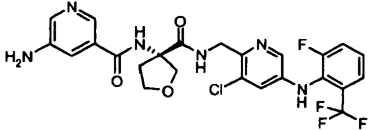 |
| (238) | 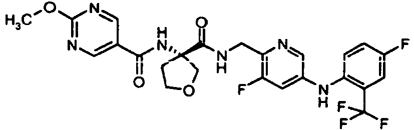 |
| (239) | 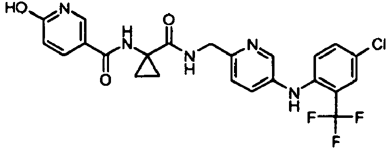 |
| (240) | 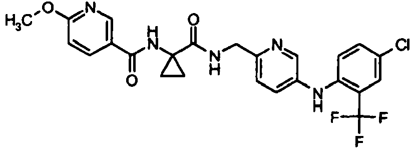 |
| (241) | 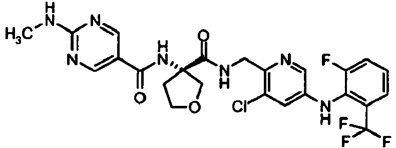 |
| (242) | 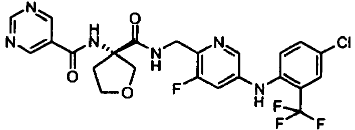 |
| (243) | 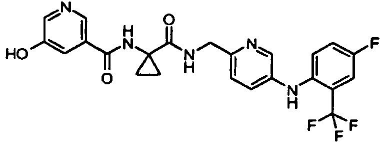 |
| (244) | 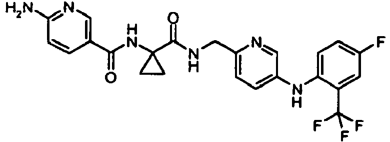 |
| (245) | 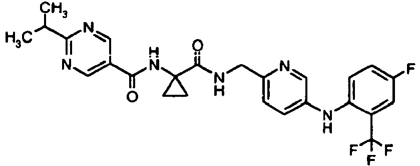 |
| (246) | 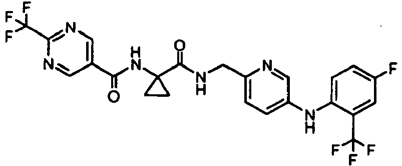 |
| (247) | 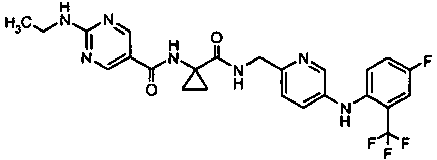 |
| (248) | 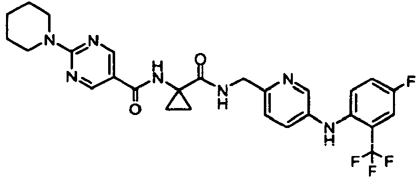 |
| (249) | 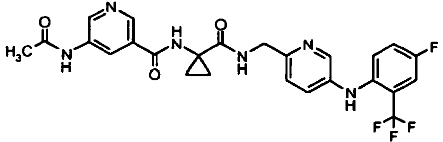 |
| (250) | 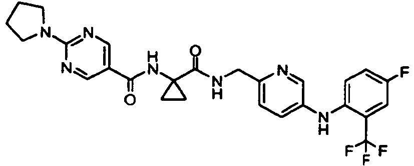 |
| (251) | 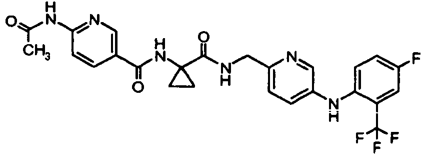 |
| (252) | 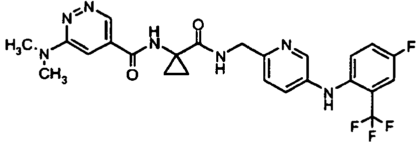 |
| (253) | 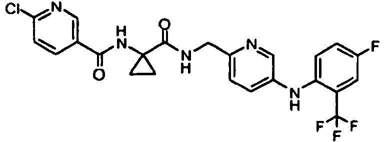 |
| (254) | 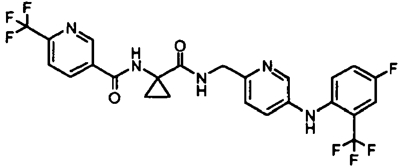 |
| (255) | 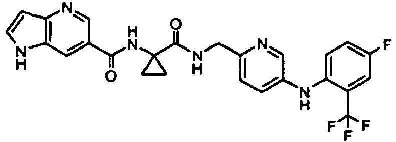 |
| (256) | 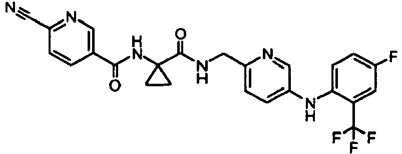 |
| (257) | 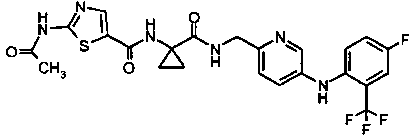 |
| (258) | 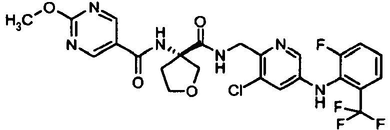 |
| (258) | 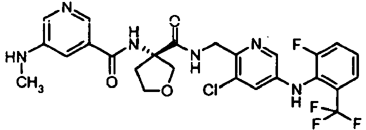 |
| (260) | 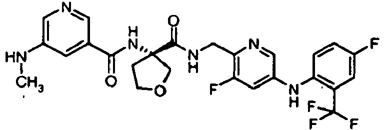 |
| (261) | 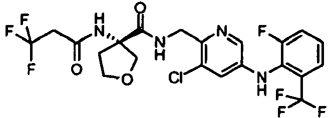 |
| (262) | 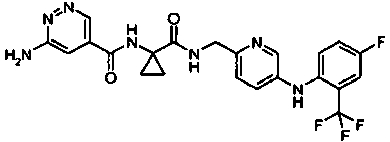 |
| (263) | 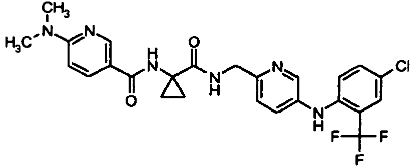 |
| (264) | 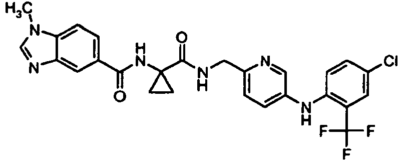 |
| (265) | 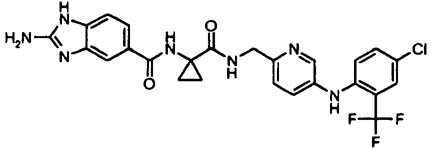 |
| (266) | 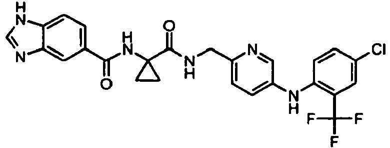 |
| (267) | 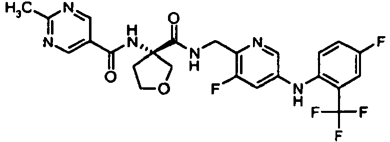 |
| (268) | 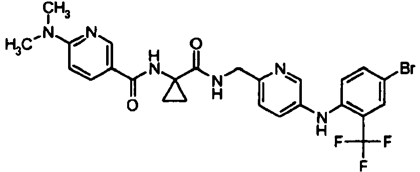 |
| (269) | 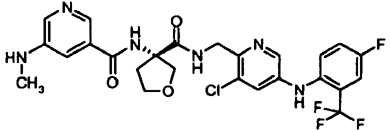 |
| (270) | 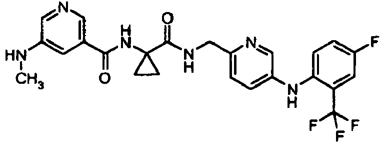 |
| (271) | 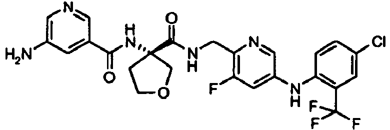 |
| (272) | 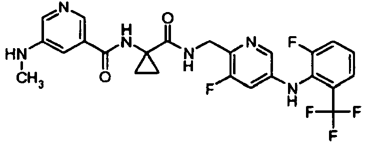 |
| (273) | 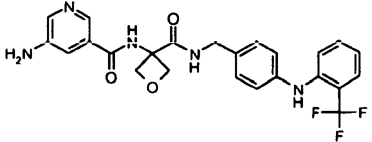 |
| (274) | 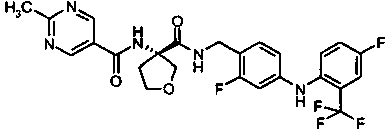 |
| (275) | 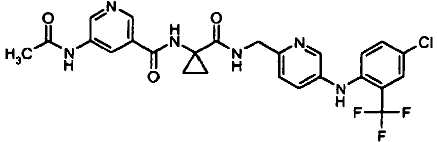 |
| (276) | 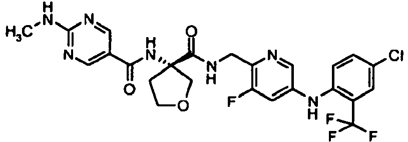 |
| (277) | 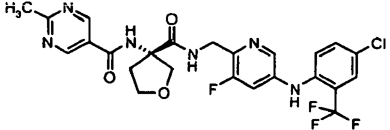 |
| (278) | 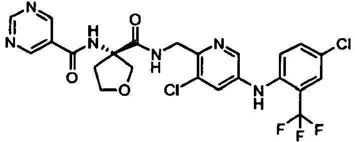 |
| (279) | 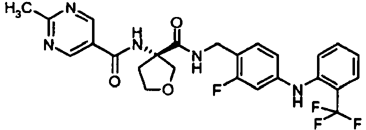 |
| (280) | 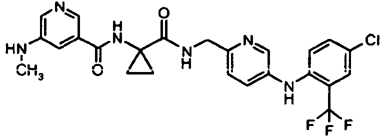 |
| (281) | 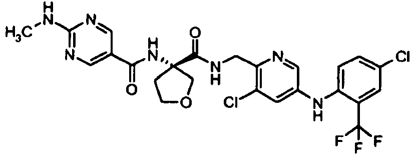 |
| (282) | 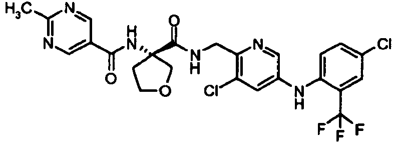 |
| (283) | 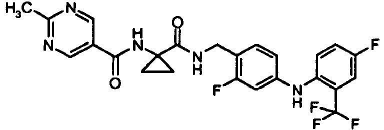 |
| (284) | 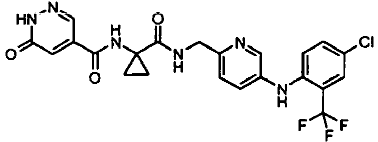 |
| (285) | 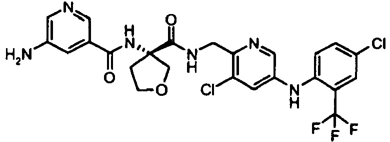 |
| (286) | 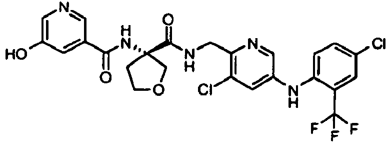 |
| (287) | 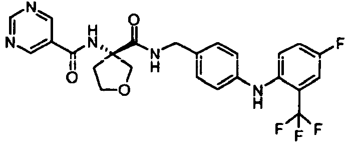 |
| (288) | 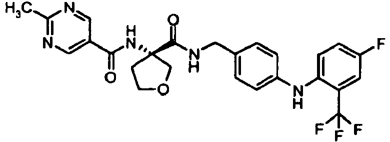 |
| (289) | 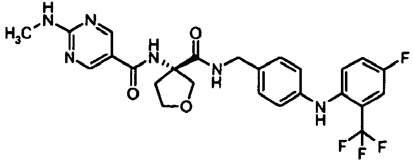 |
| (290) | 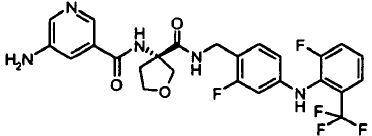 |
| (291) | 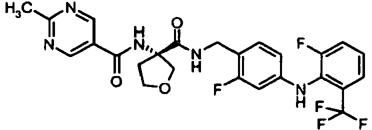 |
| (292) | 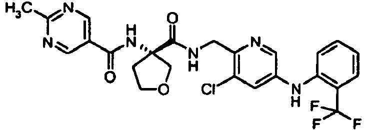 |
| (293) | 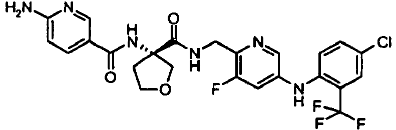 |
| (294) | 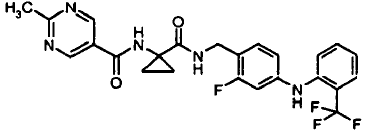 |
| (295) | 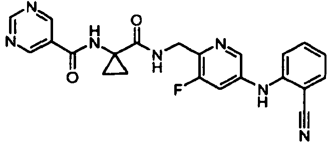 |
| (296) | 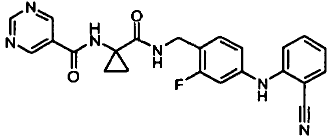 |
| (297) | 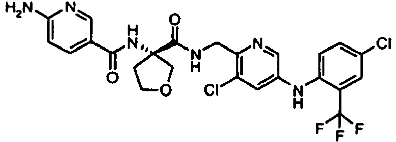 |
| (298) | 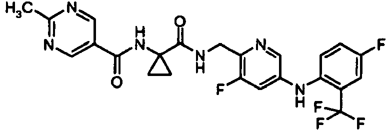 |
| (299) | 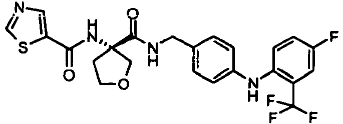 |
| (300) | 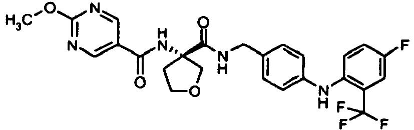 |
| (301) | 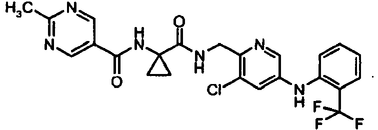 |
| (302) | 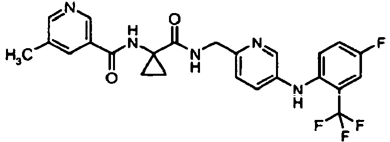 |
| (303) | 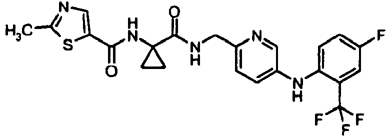 |
| (304) | 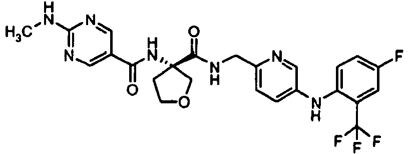 |
| (305) | 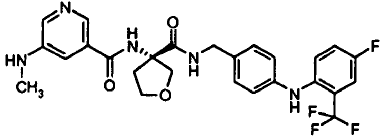 |
| (306) | 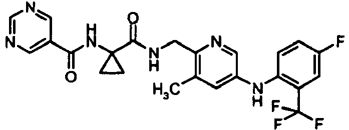 |
| (307) | 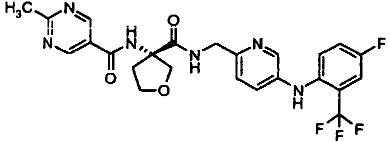 |
| (308) | 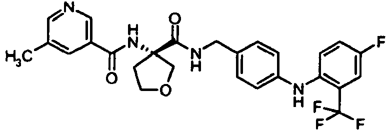 |
| (309) | 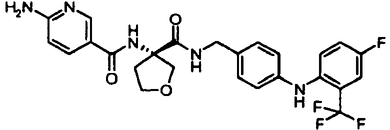 |
| (310) | 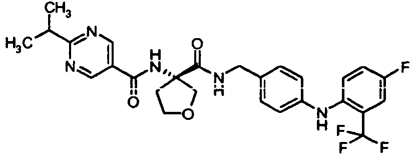 |
| (311) | 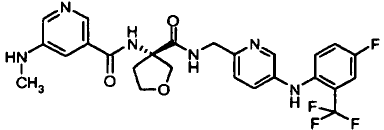 |
| (312) | 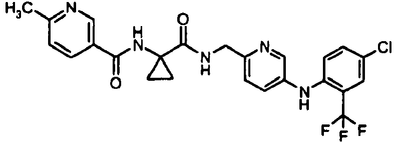 |
| (313) | 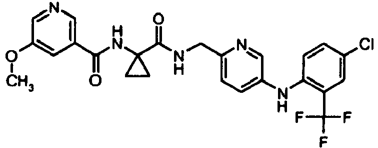 |
| (314) | 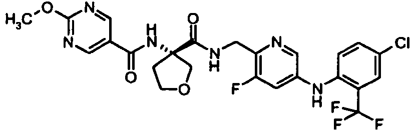 |
| (315) | 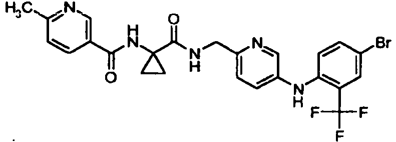 |
| (316) | 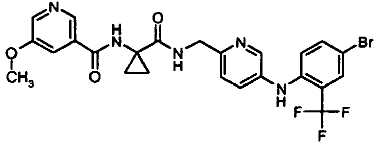 |
| (317) | 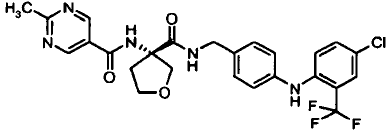 |
| (318) | 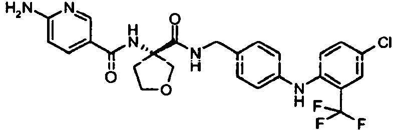 |
| (319) | 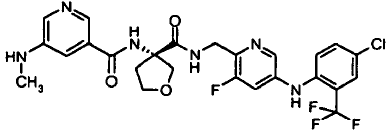 |
| (320) | 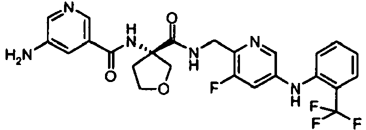 |
| (321) | 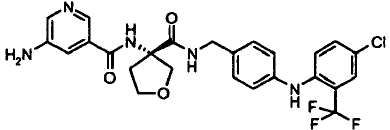 |
| (322) | 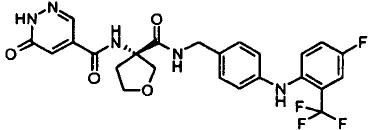 |
| (323) | 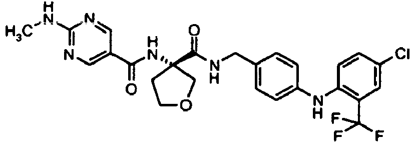 |
| (324) | 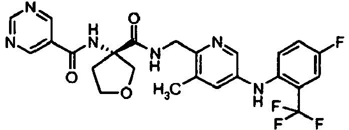 |
| (325) | 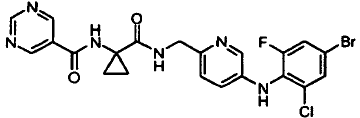 |
| (326) | 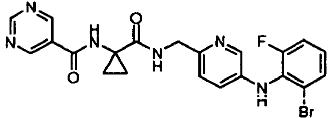 |
| (327) | 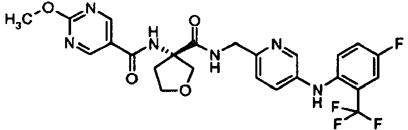 |
| (328) | 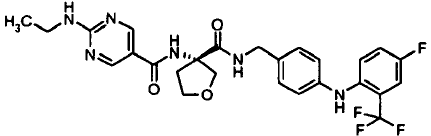 |
| (329) | 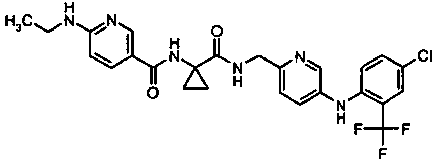 |
| (330) | 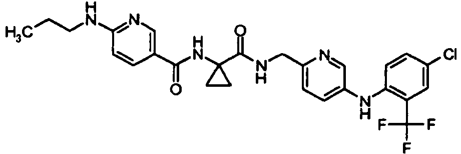 |
| (331) | 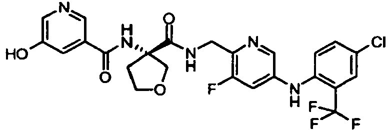 |
| (332) | 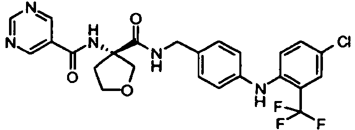 |
| (333) | 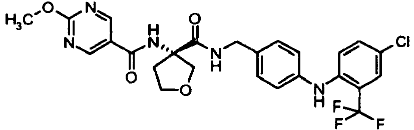 |
| (334) | 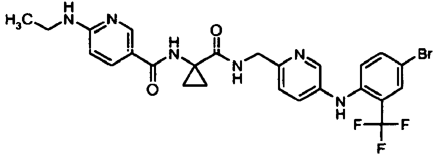 |
| (335) | 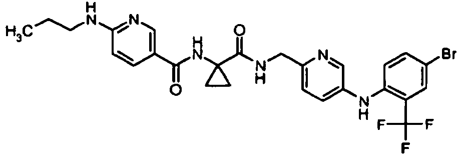 |
| (336) | 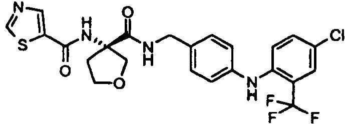 |
| (337) | 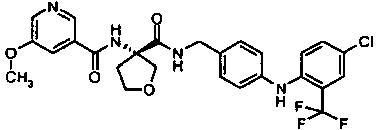 |
| (338) | 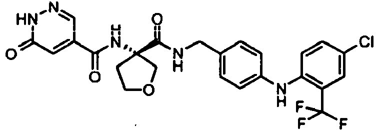 |
| (339) | 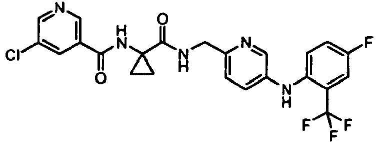 |
| (340) | 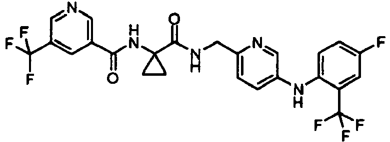 |
| (341) | 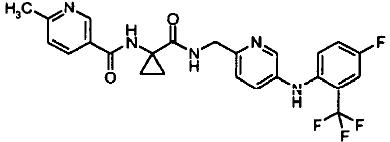 |
| (342) | 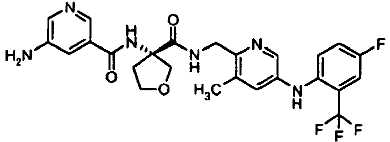 |
| (343) | 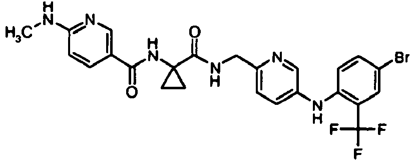 |
| (344) | 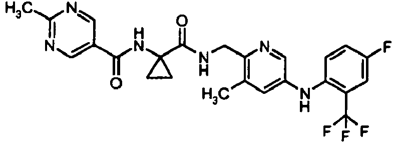 |
| (345) | 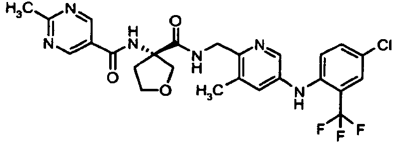 |
| (346) | 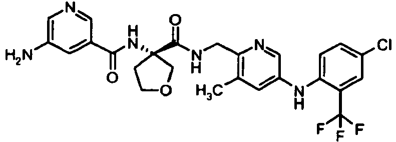 |
| (347) | 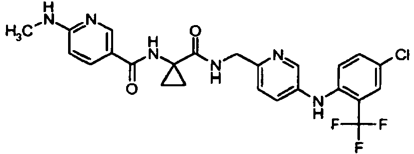 |
| (348) | 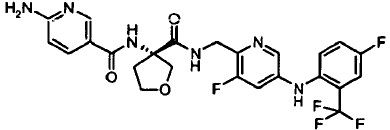 |
| (349) | 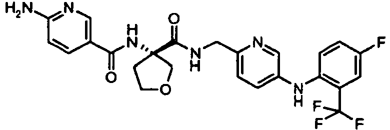 |
| (350) | 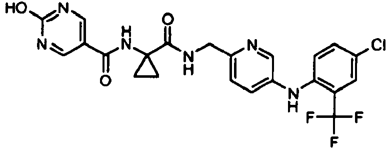 |
| (351) | 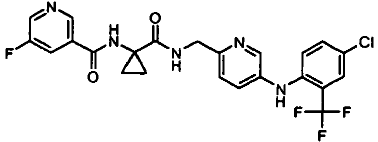 |
| (352) | 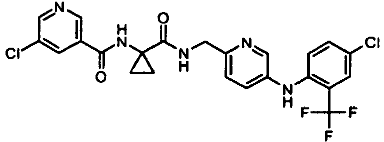 |
| (353) | 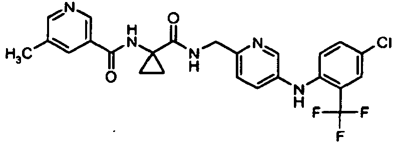 |
| (354) | 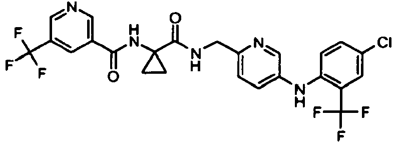 |
| (355) | 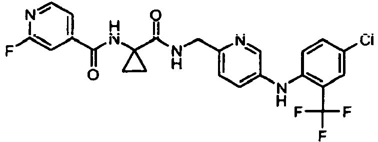 |
| (356) | 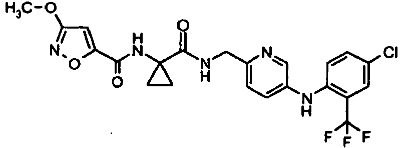 |
| (357) | 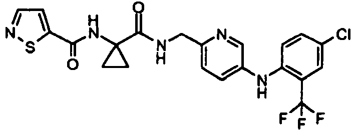 |
| (358) | 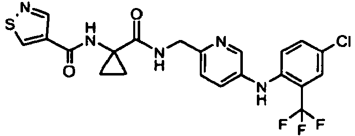 |
| (359) | 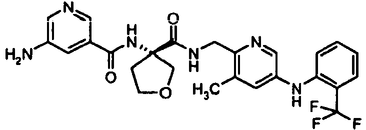 |
| (360) | 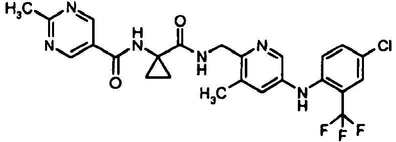 |
| (361) | 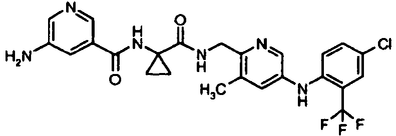 |
| (362) | 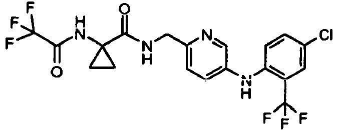 |
| (363) | 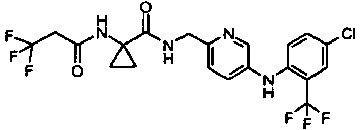 |
| (364) | 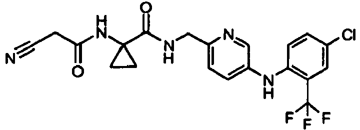 |
| (365) | 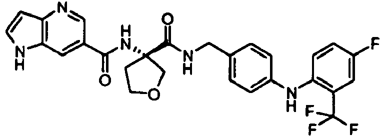 |
| (366) | 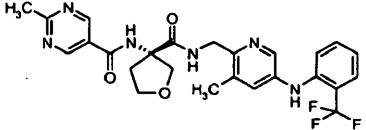 |
| (367) | 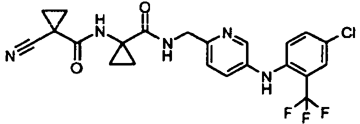 |
| (368) | 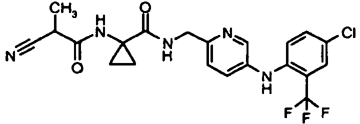 |
| (369) | 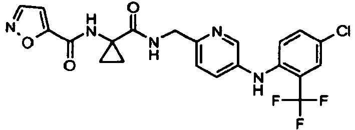 |
| (370) | 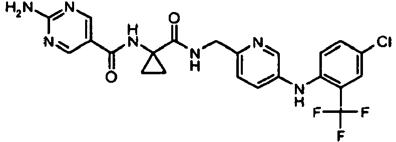 |
| (371) | 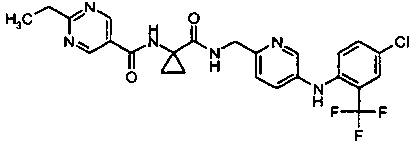 |
| (372) | 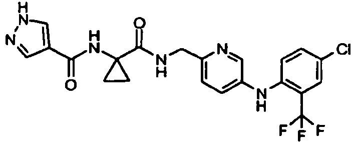 |
| (373) | 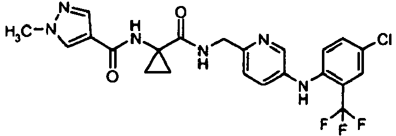 |
| (374) | 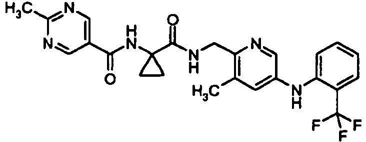 |
| (375) | 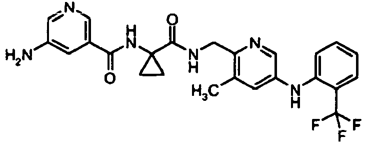 |
| (376) | 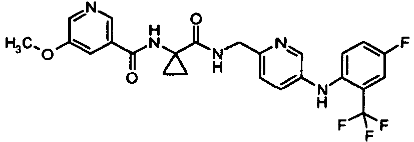 |
| (377) | 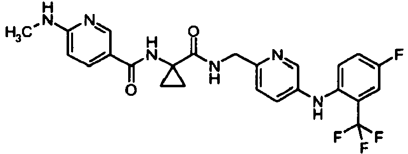 |
| (378) | 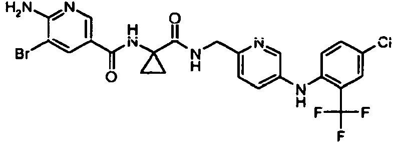 |
| (379) | 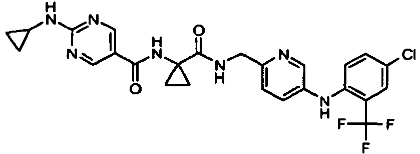 |
| (380) | 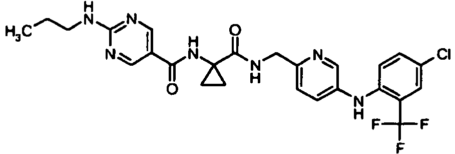 |
| (381) | 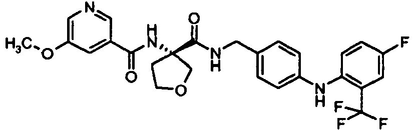 |
| (382) | 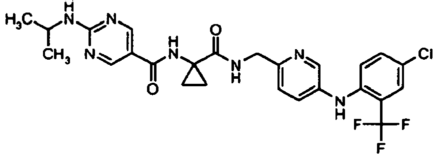 |
| (383) | 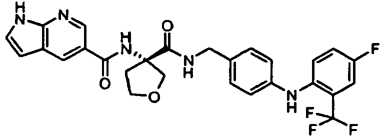 |
| (384) | 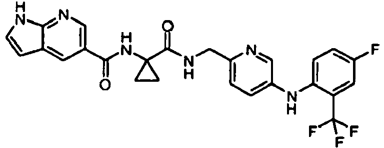 |
| (385) | 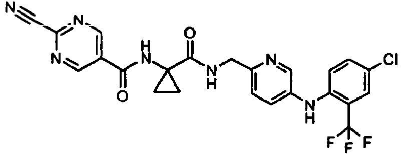 |
| (386) | 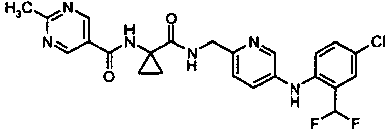 |
| (387) | 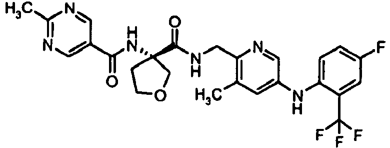 |
| (388) | 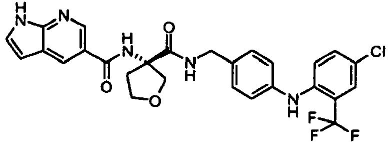 |
| (389) | 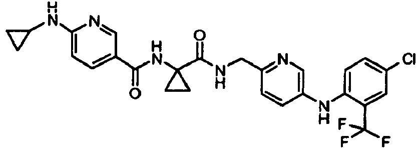 |
| (390) | 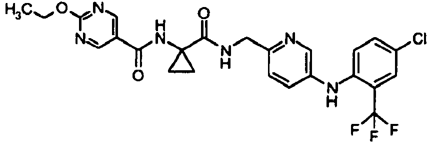 |
| (391) | 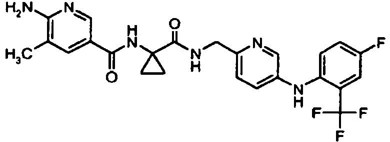 |
| (392) | 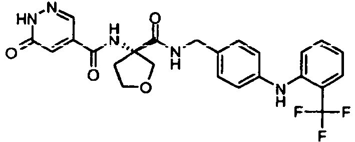 |
| (393) | 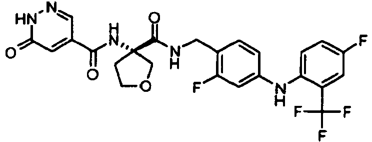 |
| (394) | 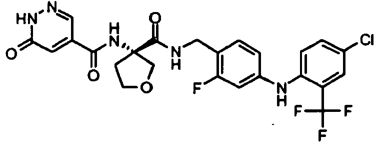 |
| (395) | 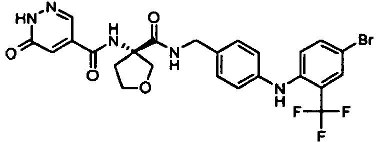 |
| (396) | 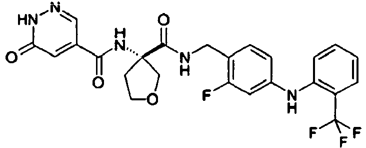 |

deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der allgemeinen Formel **II** 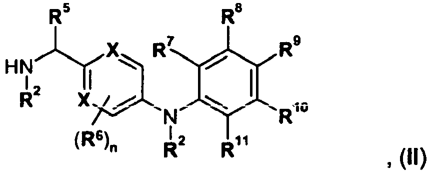 in der
**n** eine der Ziffern 0, 1 oder 2,
**R²** (a) H,

(b) C₁₋₄-Alkyl,
(c) C₁₋₄-Alkyl-C(O)-,
**R⁵** (a) H,

(b) C₁₋₄-Alkyl,
(c) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R⁶** unabhängig voneinander
(a) H, Halogen, -CN, -OH, C₁₋₆-Alkyl, C₃₋₇-Cycloalkyl, -O-C₁₋₄-Alkyl, -O-CF₃, -O-C₃₋₆-Cycloalkyl, -N(C₁₋₃-Alkyl)₂, -C(O)-NH₂, -(SO₂)NH₂, -SO₂-C₁₋₃-Alkyl, oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R⁷** (a) H, Halogen, -CN, -OH,
(b) C₁₋₆-Alkyl,
(c) C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) -O-C₁₋₆-Alkyl,
(f) -O-C₃₋₇-Cycloalkyl,
(g) -NH₂, -NH(C₁₋₃-Alkyl), -N(C₁₋₃-Alkyl)₂,
(h) -C(O)-**R^{7.1}**,
(i) -S-C₁₋₄-Alkyl, -SO₂-**R^{7.2}**,
(j) eine gegebenenfalls mit einer oder zwei C₁₋₃-Alkylgruppen substituierte fünfgliedrige Heteroarylgruppe, die ausgewählt ist aus der Gruppe bestehend aus Pyrrolyl, Oxazolyl, Isoxazolyl, Oxadiazolyl, Thiazolyl, Isothiazolyl, Thiadiazolyl, Imidazolyl, Pyrazolyl, Triazolyl und Tetrazolyl, oder
(k) eine gegebenenfalls mit einer oder zwei C₁₋₃-Alkylgruppen substituierte sechsgliedrige Heteroarylgruppe, die ausgewählt ist aus der Gruppe bestehend aus Pyridyl, Pyrimidinyl, Pyrazinyl, Pyridazinyl und Triazinyl,
**R^{7.1}** -NH₂, -NH(C₁₋₆-Alkyl),-N(C₁₋₆-Alkyl)₂, *N*-Acetidinyl, *N*-Pyrrolidinyl, *N*-Piperidinyl, *N*-Morpholinyl, -OH, -O-C₁₋₈-Alkyl oder -O-C₃₋₇-Cycloalkyl,
**R^{7.2}** -NH₂, -NH(C₁₋₆-Alkyl),-N(C₁-₆-Alkyl)₂, *N*-Acetidinyl, *N*-Pyrrolidinyl, *N*-Piperidinyl oder *N*-Morpholinyl und
**R⁸** H, Halogen, C₁₋₄-Alkyl,
**R⁹** (a) H, Halogen, -CN, -OH,

(b) C₁₋₆-Alkyl,
(c) C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) C₂₋₄-Alkinyl,
(f) -O-C₁₋₆-Alkyl,
(g) -O-C₃₋₇-Cycloalkyl,
(h) -NH₂, -NH(C₁₋₃-Alkyl), -N(C₁₋₃-Alkyl)₂,
(i) -C(O)-**R^{9.1}**,
(j) -S-C₁₋₄-Alkyl, -SO-C₁₋₄-Alkyl, -SO₂-C₁₋₄-Alkyl,
**R^{9.1}** -NH₂, -NH(C₁₋₆-Alkyl),-N(C₁₋₆-Alkyl)₂, *N*-Acetidinyl, *N*-Pyrrolidinyl, *N*-Piperidinyl, *N*-Morpholinyl, -OH, -O-C₁₋₈-Alkyl oder -O-C₃₋₇-Cycloalkyl,
**R¹⁰** H, Halogen, C₁₋₄-Alkyl,
**R¹¹** (a) H, Halogen, -CN, -OH,
(b) C₁₋₆-Alkyl,
(c) C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) -O-C₁₋₆-Alkyl,
(f) -O-C₃₋₇-Cycloalkyl,
(g) -NH₂, -NH(C₁₋₃-Alkyl), -N(C₁₋₃-Alkyl)₂,
(h) -C(O)-**R^{11.1}**,
(i) -S-C₁₋₃-Alkyl, -SO₂-**R^{11.2}**,
(j) eine gegebenenfalls mit einer oder zwei C₁₋₃-Alkylgruppen substituierte fünfgliedrige Heteroarylgruppe, die ausgewählt ist aus der Gruppe bestehend aus Pyrrolyl, Oxazolyl, Isoxazolyl, Oxadiazolyl, Thiazolyl, Isothiazolyl, Thiadiazolyl, Imidazolyl, Pyrazolyl, Triazolyl und Tetrazolyl, oder
(k) eine gegebenenfalls mit einer oder zwei C₁₋₃-Alkylgruppen substituierte sechsgliedrige Heteroarylgruppe, die ausgewählt ist aus der Gruppe bestehend aus Pyridyl, Pyrimidinyl, Pyrazinyl, Pyridazinyl und Triazinyl,
**R^{11.1}** -NH₂, -NH(C₁₋₆-Alkyl),-N(C₁₋₆-Alkyl)₂, *N*-Acetidinyl, *N*-Pyrrolidinyl, *N*-Piperidinyl, *N*-Morpholinyl, -OH, -O-C₁₋₈-Alkyl oder -O-C₃₋₇-Cycloalkyl.
**R^{11.2}** -NH₂, -NH(C₁₋₆-Alkyl),-N(C₁₋₆-Alkyl)₂, *N*-Acetidinyl, *N*-Pyrrolidinyl, *N*-Piperidinyl oder *N*-Morpholinyl und
X unabhängig voneinander C-R⁶ oder N,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **II,** in der
**n** eine der Ziffern 0, 1 oder 2,
**R²** H oder CH₃,
**R⁵** H oder CH₃,
**R⁶** H, F, Cl oder Methyl,
**R⁷** H, F, Cl, Br, -CN, C₁₋₄-Alkyl, CF₃, CHF₂,
**R⁸** H,
**R⁹** F, Cl, Br, C₁₋₄-Alkyl, -O-C₁₋₄-Alkyl, -S-C₁₋₄-Alkyl,
**R¹⁰** H,
**R¹¹** F, Cl, Br, -CN, C₁₋₄-Alkyl, CF₃, CHF₂, und
**X** unabhängig voneinander C-**R⁶** oder N bedeuten,
deren Enantiomere, deren Diastereomere, deren Gemische und deren Salze, insbesondere deren physiologisch verträgliche Salze mit organischen oder anorganischen Säuren oder Basen.

Als ganz besonders bevorzugte Verbindungen der obigen allgemeinen Formel **II** seien beispielsweise folgende Verbindungen genannt:

| **Nr.** | **Struktur** |
|---|---|
| (1.1) | 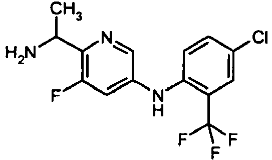 |
| (1.2) | 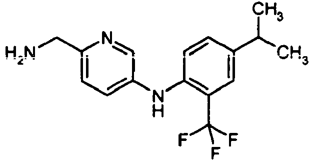 |
| (1.3) | 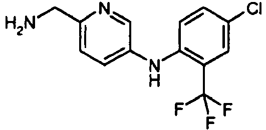 |
| (1.4) | 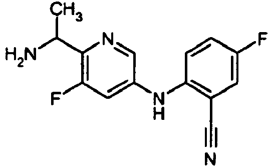 |
| (1.5) | 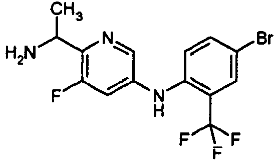 |
| (1.6) | 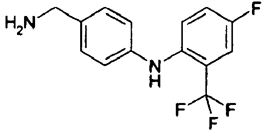 |
| (1.7) | 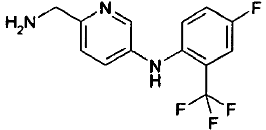 |
| (1.8) | 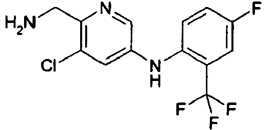 |
| (1.9) | 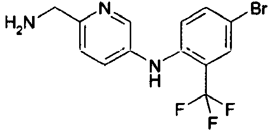 |
| (1.10) | 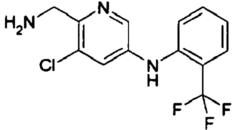 |
| (1.11) | 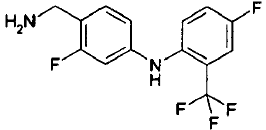 |
| (1.12) | 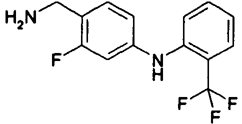 |
| (1.13) | 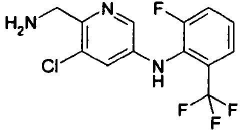 |
| (1.14) | 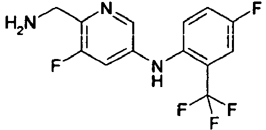 |
| (1.15) | 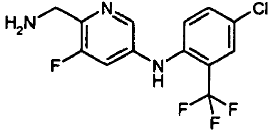 |
| (1.16) | 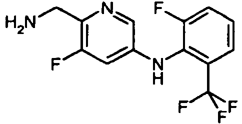 |
| (1.17) | 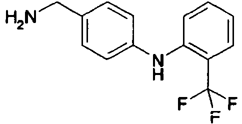 |
| (1.18) | 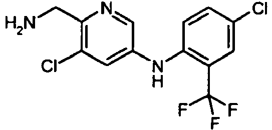 |
| (1.19) | 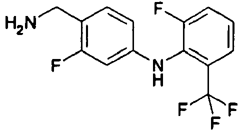 |
| (1.20) | 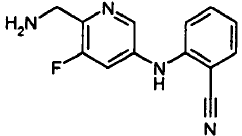 |
| (1.21 | 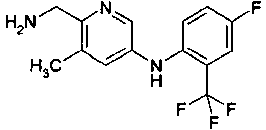 |
| (1.22) | 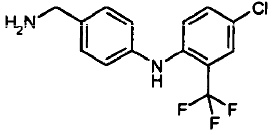 |
| (1.23) | 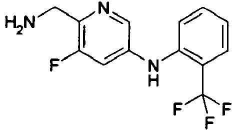 |
| (1.24) | 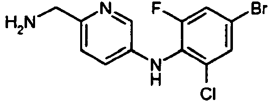 |
| (1.25) | 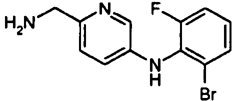 |
| (1.26) | 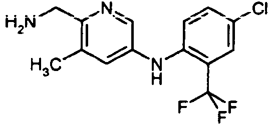 |
| (1.27) | 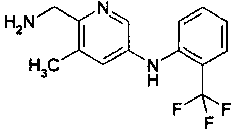 |
| (1.28) | 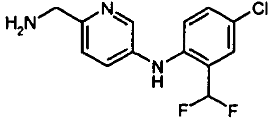 |
| (1.29) | 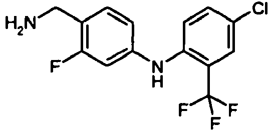 |
| (1.30) | 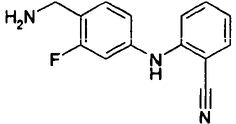 |
| (1.31) | 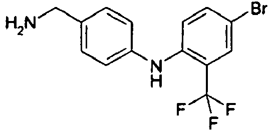 |

deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Anmeldung betrifft die Verwendung der Verbindungen der allgemeinen Formel **II**, in der **R²**, **R⁵**, **R⁶**, **R⁷**, **R⁸**, **R⁹**, **R**^{**1**0} und **R¹¹** wie eingangs erwähnt definiert sind, deren Diastereomere, deren Enantiomere und deren Salze, insbesondere deren physiologisch verträgliche Salze mit anorganischen oder organischen Säuren oder Basen zur Herstellung von Verbindungen der allgemeinen Formel **I,** welche B1-antagonistische Eigenschaften besitzen.

Eine weitere Ausführungsform der vorliegenden Erfindung besteht in den Verbindungen der allgemeinen Formel **III** 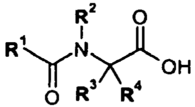 in der
**R¹** (a) eine gegebenenfalls mit einem Rest **R^{1.1}** substituierte C₁₋₆-Alkylgruppe,
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
(c) eine gegebenenfalls mit einem Rest **R^{1.2}** substituierte C₃₋₆-Cycloalkylgruppe, in der eine -CH₂-Einheit durch eine -C(O)-Gruppe ersetzt sein kann,
(d) eine gegebenenfalls mit 1, 2 oder 3 Resten **R^{1.3}** substituierte Aryl-C₀₋₂-alkylengruppe,
(e) ein gegebenenfalls mit 1, 2 oder 3 Resten **R^{1.4}** substituierter fünfgliedriger Heteroaryl-C₀₋₂-alkylen-Rest, der mindestens ein N-, O- oder S-Atom enthält und der gegebenenfalls zusätzlich ein, zwei oder drei weitere N-Atome enthält und der zusätzlich benzo-kondensiert sein kann,
(f) ein gegebenenfalls mit 1 oder 2 Resten **R^{1.4}** substituierter sechsgliedriger Heteroaryl-C₀₋₂-alkylen-Rest, der ein, zwei oder drei N-Atome enthält und der zusätzlich benzo-kondensiert sein kann,
(g) ein gegebenenfalls mit 1 oder 2 Resten **R^{1.4}** substituierter neun- oder zehngliedriger Heteroaryl-Rest, der ein, zwei oder drei N-Atome enthält,
(h) ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
(i) -O-**R^{1.1.1}**,
(j) -N**R^{1.1.3}R^{1.1.4}** oder
(k) -C(=NR^{1.5})-CN,
**R^{1.1}** Halogen, -NO₂, -CN, C₃₋₆-Cycloalkyl, -O**R^{1.1.1}**, -S**R^{1.1.1}**, -C(O)**R^{1.1.1}**, -S(O)₂-**R^{1.1.2}**, -O-S(O)₂-**R^{1.1.1}**, -CO₂**R^{1.1.1}**, -O-C(O)-**R^{1.1.1}**, -N**R^{1.1.3}R^{1.1.4}**, -N**R^{1.1.3}**-C(O)-**R^{1.1.1}**, -N**R^{1.1.3}**-C(O)-**R^{1.1.1}**, -N**R^{1.1.3}**-CO₂-**R^{1.1.1}** oder -C(O)-N**R^{1.1.3}R^{1.1.4}**,
**R^{1.1.1}** (a) H,
(b) C₁₋₄-Alkyl,
(c) eine C₁₋₃-Alkylgruppe, in der jede Methylengruppe mit 1 oder 2 Fluoratomen und jede Methylgruppe mit 1, 2 oder 3 Fluoratomen substituiert sein kann,
(d) eine gegebenenfalls mit 1, 2 oder 3 Resten **R^{1.1.1.1}** substituierte Phenylgruppe,
(e) C₃₋₆-Cycloalkyl oder
(f) eine gegebenenfalls mit 1, 2 oder 3 Resten **R^{1.1.1.2}** substituierte Pyridylgruppe,
**R^{1.1.1.1}** unabhängig voneinander
(a) Halogen, -NO₂, -CN, -OH, -O-C₁₋₄-Alkyl, C₃₋₆-Cycloalkyl, C₁₋₄-Alkyl oder
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R^{1.1.1.2}** unabhängig voneinander Halogen oder C₁₋₄-Alkyl,
**R^{1.1.2}** (a) C₁₋₄-Alkyl,
(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
(c) -O-C₁₋₄-Alkyl oder
(d) eine gegebenenfalls mit 1, 2 oder 3 Resten **R^{1.1.1.1}** substituierte Phenylgruppe,
**R^{1.1.3}**,
**R^{1.1.4}** unabhängig voneinander
(a) H,
(b) eine gegebenenfalls mit 1, 2 oder 3 Resten **R^{1.1.4.1}** substituierte C₁₋₄-Alkylgruppe,
(c) eine gegebenenfalls mit 1, 2 oder 3 Resten **R^{1.1.1.1}** substituierte Phenylgruppe,
(d) C₃₋₆-Cycloalkyl, oder
**R^{1.1.3}** und **R^{1.1.4}** zusammen mit dem N-Atom, an das sie gebunden sind, einen 5- oder 6-gliedrigen heterocyclischen Ring bilden, der zusätzlich ein weiteres Heteroatom ausgewählt aus N, O und S enthalten kann, oder
**R^{1.1.3}** und **R^{1.1.4}** zusammen mit dem N-Atom, an das sie gebunden sind, ein cyclisches Imid bilden,
**R^{1.1.4.1}** unabhängig voneinander Halogen, -NH₂, -NH(C₁₋₄-Alkyl), -N(C₁₋₄-Alkyl)₂ oder -SO₂-**R^{1.1.2}**,
**R^{1.2}** Halogen, -NO₂, -CN, OH, -O-CH₃ oder Phenyl,
**R^{1.3}** (a) Halogen, -NO₂, -CN, -O**R^{1.1.1}**, -S**R^{1.1.1}**, -CO₂**R^{1.1.1}**, C₁₋₆-Alkyl oder

(b) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R^{1.4}** unabhängig voneinander
(a) Halogen, -NO₂, -CN, -O**R^{1.1.1}**, -S**R^{1.1.1}**, -S(O)-**R^{1.1.2}**, -S(O)₂-**R^{1.1.2}**, -N**R^{1.1.3}R^{1.1.4}**, -N(**R^{1.4.1}**)-C(O)-C₁₋₄-Alkyl, C₁₋₆-Alkyl,
(b) eine C₁₋₃-Alkylgruppe, in der jede Methylengruppe mit 1 oder 2 Fluoratomen und jede Methylgruppe mit in oder 3 Fluoratomen substituiert sein kann, oder
(c) eine Oxo-Gruppe,
**R^{1.4.1}** H oder C₁₋₄-Alkyl,
**R^{1.5}** -OH oder -O-C₁₋₃-Alkyl,
**R²** (a) H,
(b) C₁₋₄-Alkyl,
(c) C₁₋₄-Alkyl-C(O)-,
**R³** und **R⁴** zusammen mit dem Kohlenstoffatom, an dem sie gebunden sind, eine gegebenenfalls durch einen Rest **R^{3.1}** substituierte C₃₋₆-Cycloalkylengruppe, in der eine -CH₂-Einheit durch ein Heteroatom O, N, S oder durch eine Gruppe CO, SO oder SO₂ ersetzt sein kann, und
**R^{3.1}** H, -OH bedeuten,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **III**, in der
**R¹** ausgewählt ist aus der Gruppe bestehend aus 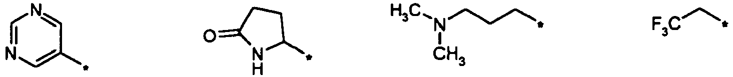 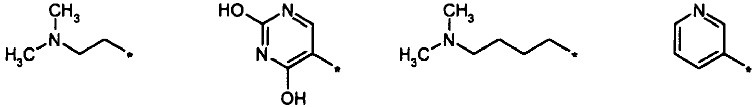 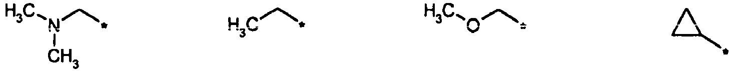 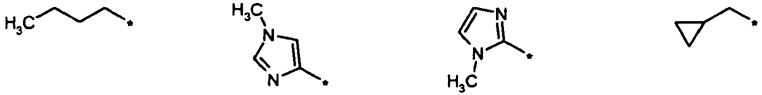 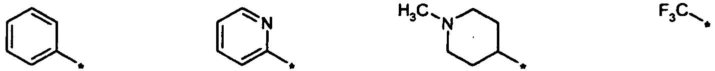 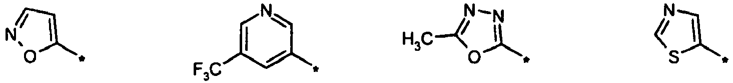 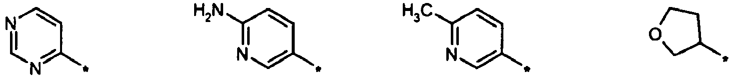 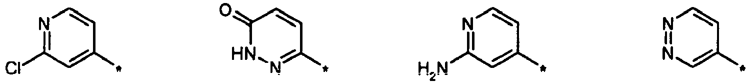 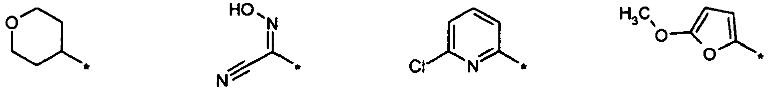 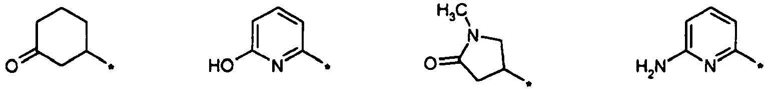 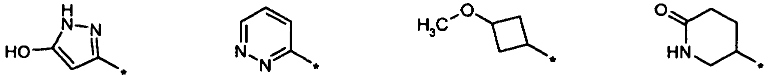 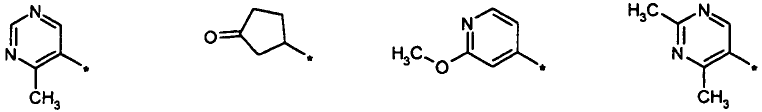 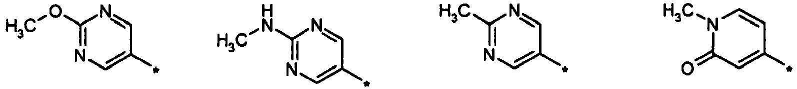 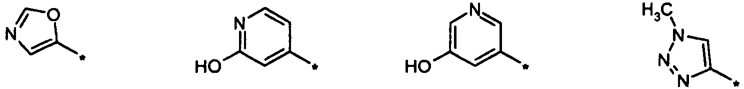 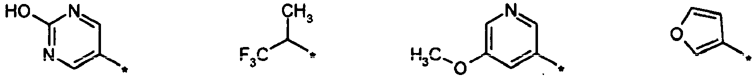 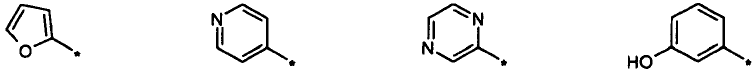 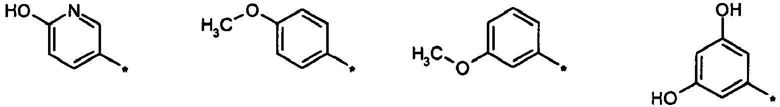 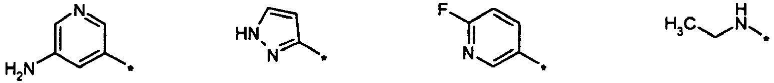 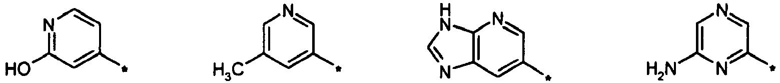 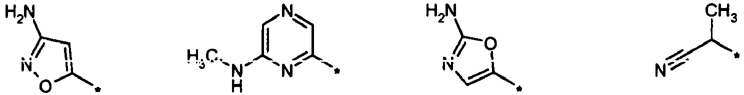 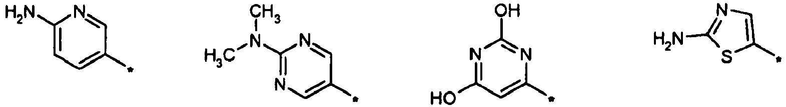 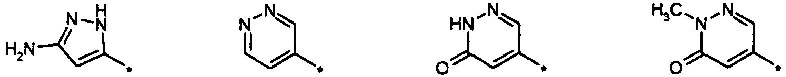 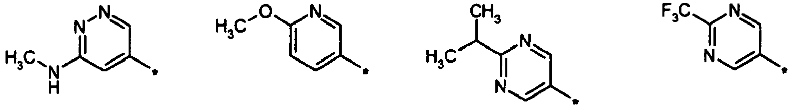 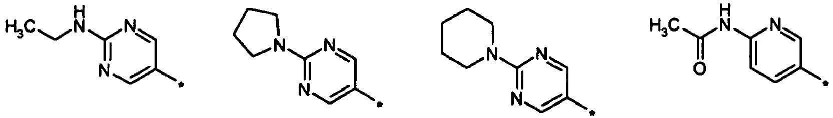 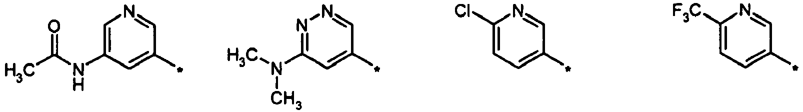 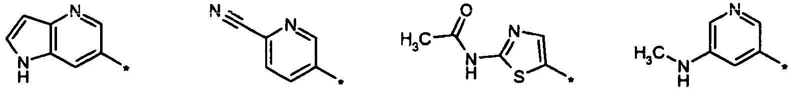 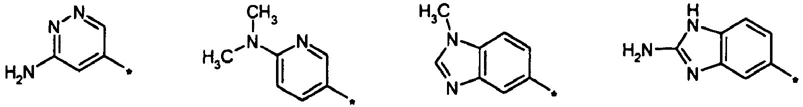 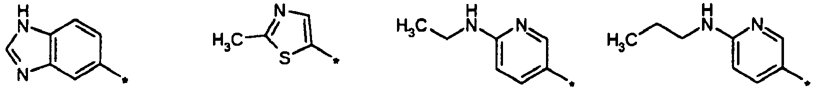 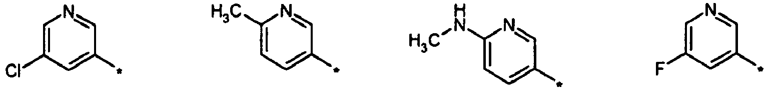 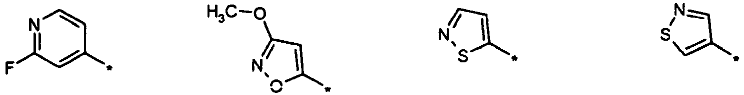 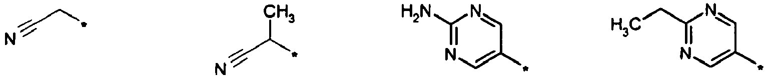 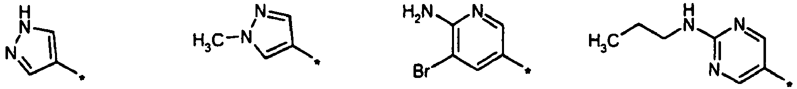 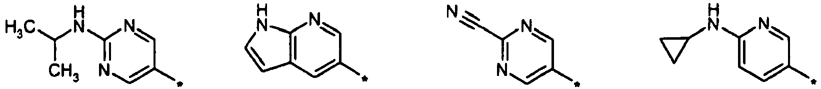 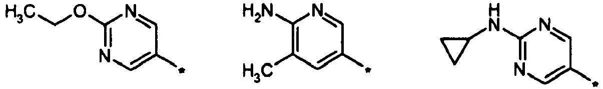 **R²** H oder CH₃,
**R³** und **R⁴** zusammen mit dem Kohlenstoffatom, an dem sie gebunden sind, eine gegebenenfalls durch einen Rest **R^{3.1}** substituierte C₃₋₆-Cycloalkylengruppe, in der eine -CH₂-Einheit durch ein Heteroatom O, N, S oder durch eine Gruppe CO, SO oder SO₂ ersetzt sein kann, und
**R^{3.1}** H, -OH bedeuten,
deren Enantiomere, deren Diastereomere, deren Gemische und deren Salze, insbesondere deren physiologisch verträgliche Salze mit organischen oder anorganischen Säuren oder Basen.

Als ganz besonders bevorzugte Verbindungen der obigen allgemeinen Formel III seien beispielsweise folgende Verbindungen genannt:

| **Nr.** | **Struktur** |
|---|---|
| (2.1) | 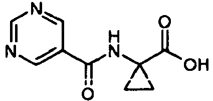 |
| (2.2) | 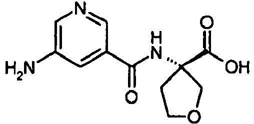 |
| (2.3) | 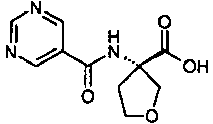 |
| (2.4) | 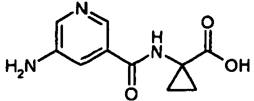 |
| (2.5) | 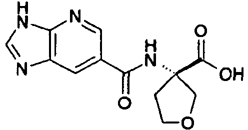 |
| (2.6) | 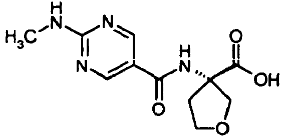 |
| (2.7) | 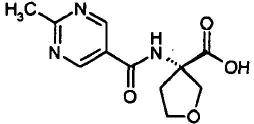 |
| (2.8) | 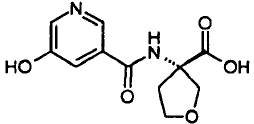 |
| (2.9) | 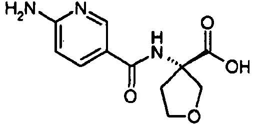 |
| (2.10) | 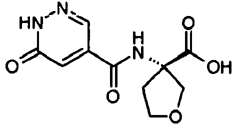 |
| (2.11) | 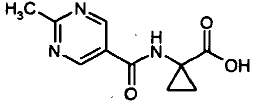 |

deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Anmeldung betrifft die Verwendung der Verbindungen der allgemeinen Formel **III**, in der **R²**, **R³**, und **R⁴** wie eingangs erwähnt definiert sind, deren Diastereomere, deren Enantiomere und deren Salze, insbesondere deren physiologisch verträgliche Salze mit anorganischen oder organischen Säuren oder Basen zur Herstellung von Verbindungen der allgemeinen Formel **I**, welche B1-antagonistische Eigenschaften besitzen.

Eine weitere Ausführungsform der vorliegenden Erfindung besteht in den Verbindungen der allgemeinen Formel **IV** 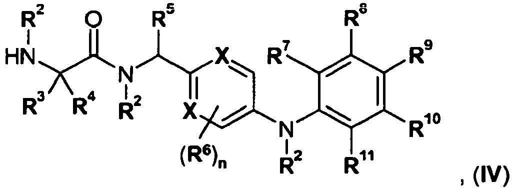 in der
**n** eine der Ziffern 0, 1 oder 2,
**R²** (a) H,
(b) C₁₋₄-Alkyl,
(c) C₁₋₄-Alkyl-C(O)-,
**R³** und **R⁴** zusammen mit dem Kohlenstoffatom, an dem sie gebunden sind, eine gegebenenfalls durch einen Rest **R^{3.1}** substituierte C₃₋₆-Cycloalkylengruppe, in der eine -CH₂-Einheit durch ein Heteroatom O, N, S oder durch eine Gruppe CO, SO oder SO₂ ersetzt sein kann,
**R^{3.1}** H, -OH,
**R⁵** (a) H,
(b) C₁₋₄-Alkyl,
(c) eine C₁₋₃-Alkylgruppe, in der jede Methylengruppe mit 1 oder 2 Fluoratomen und jede Methylgruppe mit 1, 2 oder 3 Fluoratomen substituiert sein kann,
**R⁶** unabhängig voneinander
(a) H, Halogen, -CN, -OH, C₁₋₆-Alkyl, C₃₋₇-Cycloalkyl, -O-C₁₋₄-Alkyl, -O-CF₃, -O-C₃₋₆-Cycloalkyl, -N(C₁₋₃-Alkyl)₂, -C(O)-NH₂, -(SO₂)NH₂, -SO₂-C₁₋₃-Alkyl, oder (b) eine C₁₋₃-Alkylgruppe, in der jede Methylengruppe mit 1 oder 2 Fluoratomen
und jede Methylgruppe mit 1, 2 oder 3 Fluoratomen substituiert sein kann,
**R⁷** (a) H, Halogen, -CN, -OH,
(b) C₁₋₆-Alkyl,
(c) C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) -O-C₁₋₆-Alkyl,
(f) -O-C₃₋₇-Cycloalkyl,
(g) -NH₂, -NH(C₁₋₃-Alkyl), -N(C₁₋₃-Alkyl)₂,
(h) -C(O)-**R^{7.1}**,
(i) -S-C₁₋₄-Alkyl, -SO₂-**R^{7.2}**,
(j) eine gegebenenfalls mit einer oder zwei C₁₋₃-Alkylgruppen substituierte fünfgliedrige Heteroarylgruppe, die ausgewählt ist aus der Gruppe bestehend aus Pyrrolyl, Oxazolyl, Isoxazolyl, Oxadiazolyl, Thiazolyl, Isothiazolyl, Thiadiazolyl, Imidazolyl, Pyrazolyl, Triazolyl und Tetrazolyl, oder
(k) eine gegebenenfalls mit einer oder zwei C₁₋₃-Alkylgruppen substituierte sechsgliedrige Heteroarylgruppe, die ausgewählt ist aus der Gruppe bestehend aus Pyridyl, Pyrimidinyl, Pyrazinyl, Pyridazinyl und Triazinyl,
**R^{7.1}** -NH₂, -NH(C₁₋₆-Alkyl),-N(C₁₋₆-Alkyl)₂, *N*-Acetidinyl, *N*-Pyrrolidinyl, *N*-Piperidinyl, *N*-Morpholinyl, -OH, -O-C₁₋₈-Alkyl oder -O-C₃₋₇-Cycloalkyl,
**R^{7.2}** -NH₂, -NH(C₁₋₆-Alkyl),-N(C₁₋₆-Alkyl)₂, *N*-Acetidinyl, *N*-Pyrrolidinyl, *N*-Piperidinyl oder *N*-Morpholinyl und
**R⁸** H, Halogen, C₁₋₄-Alkyl,
**R⁹** (a) H, Halogen, -CN, -OH,
(b) C₁₋₆-Alkyl,
(c) C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) C₂₋₄-Alkinyl,
(f) -O-C₁₋₆-Alkyl,
(g) -O-C₃₋₇Cycloalkyl,
(h) -NH₂, -NH(C₁₋₃-Alkyl), -N(C₁₋₃-Alkyl)₂,
(i) -C(O)-**R^{9.1}**,
(j) -S-C₁₋₄-Alkyl, -SO-C₁₋₄-Alkyl, -SO₂-C₁₋₄-Alkyl,
**R^{9.1}** -NH₂, -NH(C₁₋₆-Alkyl),-N(C₁₋₆-Alkyl)₂, *N*-Acetidinyl, *N*-Pyrrolidinyl, *N*-Piperidinyl, *N*-Morpholinyl, -OH, -O-C₁₋₈-Alkyl oder -O-C₃₋₇-Cycloalkyl,
**R¹⁰** H, Halogen, C₁₋₄-Alkyl,
**R¹¹** (a) H, Halogen, -CN, -OH,
(b) C₁₋₆-Alkyl,
(c) C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
(d) C₃₋₇-Cycloalkyl,
(e) -O-C₁₋₆-Alkyl,
(f) -O-C₃₋₇-Cycloalkyl,
(g) -NH₂, -NH(C₁₋₃-Alkyl), -N(C₁₋₃-Alkyl)₂,
(h) -C(O)-**R^{11.1}**,
(i) -S-C₁₋₃-Alkyl, -SO₂-**R^{11.2}**,
(j) eine gegebenenfalls mit einer oder zwei C₁₋₃-Alkylgruppen substituierte fünfgliedrige Heteroarylgruppe, die ausgewählt ist aus der Gruppe bestehend aus Pyrrolyl, Oxazolyl, Isoxazolyl, Oxadiazolyl, Thiazolyl, Isothiazolyl, Thiadiazolyl, Imidazolyl, Pyrazolyl, Triazolyl und Tetrazolyl, oder
(k) eine gegebenenfalls mit einer oder zwei C₁₋₃-Alkylgruppen substituierte sechsgliedrige Heteroarylgruppe, die ausgewählt ist aus der Gruppe bestehend aus Pyridyl, Pyrimidinyl, Pyrazinyl, Pyridazinyl und Triazinyl,
**R^{11.1}** -NH₂, -NH(C₁₋₆-Alkyl),-N(C₁₋₆-Alkyl)₂, *N*-Acetidinyl, *N*-Pyrrolidinyl, *N*-Piperidinyl, *N*-Morpholinyl, -OH, -O-C₁₋₈-Alkyl oder -O-C₃₋₇-Cycloalkyl,
**R^{11.2}** -NH₂, -NH(C₁₋₆-Alkyl),-N(C₁₋₆-Alkyl)₂, *N*-Acetidinyl, *N*-Pyrrolidinyl, *N*-Piperidinyl oder *N*-Morpholinyl und
**X** unabhängig voneinander C-**R⁶** oder N bedeuten,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **IV**, in der
**n** eine der Ziffern 0, 1 oder 2,
**R²** H oder CH₃,
**R³** und **R⁴** zusammen mit dem Kohlenstoffatom, an dem sie gebunden sind, eine gegebenenfalls durch einen Rest **R^{3.1}** substituierte C₃₋₆-Cycloalkylengruppe, in der eine -CH₂-Einheit durch ein Heteroatom O, N, S oder durch eine Gruppe CO, SO oder SO₂ ersetzt sein kann,
**R^{3.1}** H, -OH,
**R⁵** H oder CH₃,
**R⁶** H, F, Cl oder Methyl,
**R⁷** H, F, Cl, Br, -CN, C₁₋₄-Alkyl, CF₃, CHF₂,
**R⁸** H,
**R⁹** F, Cl, Br, C₁₋₄-Alkyl, -O-C₁₋₄-Alkyl, -S-C₁₋₄-Alkyl,
**R¹⁰** H,
**R¹¹** F, Cl, Br, -CN, C₁₋₄-Alkyl, CF₃, CHF₂, und
X unabhängig voneinander C-**R⁶** oder N bedeuten,
deren Enantiomere, deren Diastereomere, deren Gemische und deren Salze, insbesondere deren physiologisch verträgliche Salze mit organischen oder anorganischen Säuren oder Basen.

Als ganz besonders bevorzugte Verbindungen der obigen allgemeinen Formel **IV** seien beispielsweise folgende Verbindungen genannt:

| **Nr.** | **Struktur** |
|---|---|
| (3.1) | 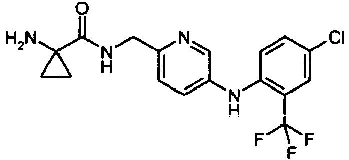 |
| (3.2) | 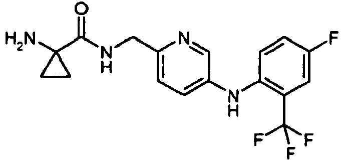 |
| (3.3) | 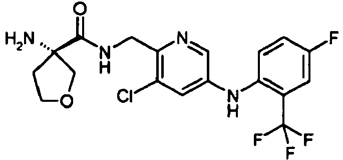 |
| (3.4) | 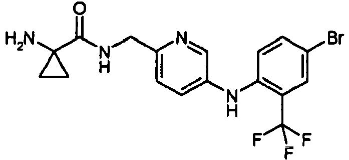 |
| (3.5) | 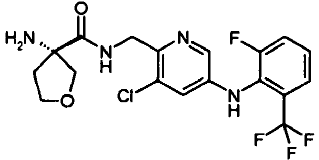 |
| (3.6) | 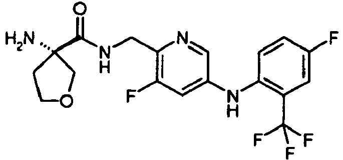 |
| (3.7) | 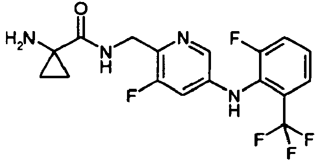 |
| (3.8) | 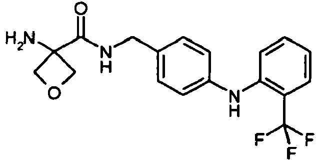 |
| (3.9) | 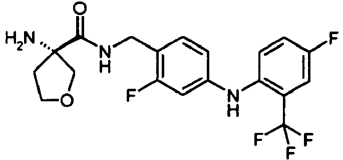 |
| (3.10) | 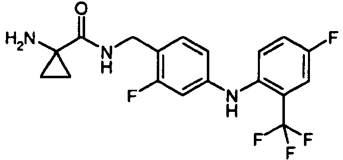 |
| (3.11) | 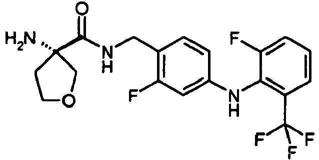 |
| (3.12) | 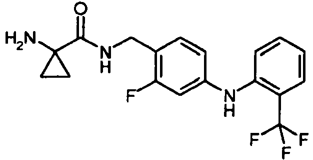 |
| (3.13) | 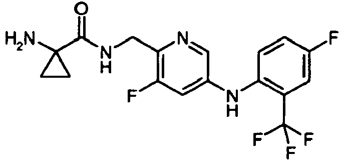 |
| (3.14) | 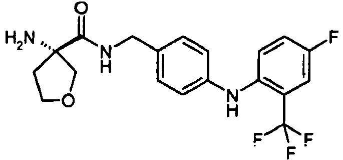 |
| (3.15) | 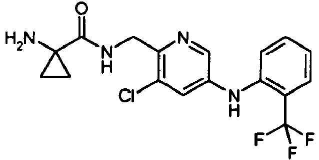 |
| (3.16) | 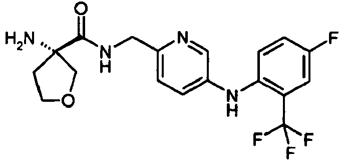 |
| (3.17) | 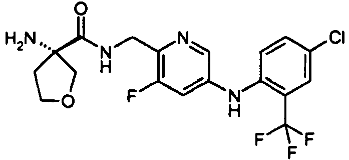 |
| (3.18) | 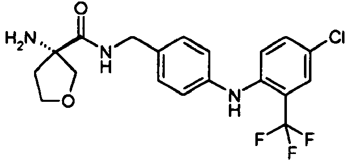 |
| (3.19) | 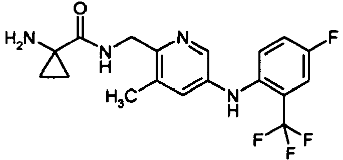 |
| (3.20) | 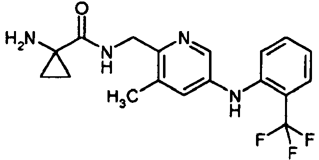 |
| (3.21 | 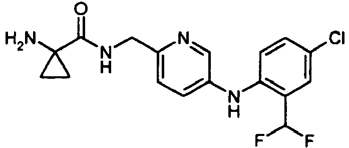 |
| (3.22) | 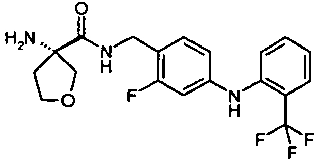 |

deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Anmeldung betrifft die Verwendung der Verbindungen der allgemeinen Formel **IV**, in der **R²**, **R⁵**, **R⁶**, **R⁷**, **R⁸**, **R⁹**, **R¹⁰** und **R¹¹** wie eingangs erwähnt definiert sind, deren Diastereomere, deren Enantiomere und deren Salze, insbesondere deren physiologisch verträgliche Salze mit anorganischen oder organischen Säuren oder Basen zur Herstellung von Verbindungen der allgemeinen Formel **I**, welche B1-antagonistische Eigenschaften besitzen.

### VERWENDETE BEGRIFFE UND DEFINITIONEN

Soweit nicht anders angegeben, sind alle Substituenten voneinander unabhängig. Sollten an einer Gruppe z.B. mehrere C₁₋₆-Alkylgruppen als Substituenten sein, so könnte im Fall von drei Substituenten C₁₋₆-Alkyl unabhängig voneinander einmal Methyl, einmal n-Propyl und einmal tert-Butyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden Alkylgruppen mit 1 bis 3 Kohlenstoffatomen, unter dem Begriff "C₁₋₄-Alkyl" verzweigte und unverzweigte Alkylgruppen mit 1 bis 4 Kohlenstoffatomen, unter dem Begriff "C₁₋₆-Alkyl" verzweigte und unverzweigte Alkylgruppen mit 1 bis 6 Kohlenstoffatomen und unter dem Begriff "C₁₋₈-Alkyl" verzweigte und unverzweigte Alkylgruppen mit 1 bis 8 Kohlenstoffatomen verstanden. Beispielsweise werden hierfür genannt: Methyl, Ethyl, *n-*Propyl, *iso*-Propyl, *n*-Butyl, *iso*-Butyl, *sec*-Butyl, *tert*-Butyl, *n*-Pentyl, Neopentyl, *n*-Hexyl, *n*-Heptyl und *n*-Octyl. Gegebenenfalls werden für die vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl.
Weiterhin umfassen die voranstehend genannten Begriffe auch solche Reste, in denen jede Methylengruppe mit bis zu zwei und jede Methylgruppe mit bis zu drei Fluoratomen substituiert sein kann.

Unter dem Begriff "C₀₋₂-Alkylen" werden verzweigte und unverzweigte Alkylengruppen mit 0 bis 2 Kohlenstoffatomen verstanden, wobei eine C₀-Alkylengruppe eine Bindung darstellt. Beispielsweise werden hierfür genannt: Methylen, Ethylen und Ethan-1,1-diyl. Weiterhin umfassen die voranstehend genannten Begriffe auch solche Reste, in denen jede Methylengruppe mit bis zu zwei Fluoratomen substituiert sein kann.

Unter dem Begriff "C₃₋₇-Cycloalkyl" (auch soweit sie Bestandteil anderer Reste sind) werden cyclische Alkylgruppen mit 3 bis 7 Kohlenstoffatomen und unter dem Begriff "C₃₋₆-Cycloalkyl" cyclische Alkylgruppen mit 3 bis 6 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "C₃₋₆-Cycloalkylen" (auch soweit sie Bestandteil anderer Reste sind) werden cyclische Alkylengruppen mit 3 bis 6 Kohlenstoffatomen verstanden. Beispielsweise werden hierfür genannt: Cyclopropylen, Cyclobutylen, Cyclopentylen oder Cyclohexylen. Soweit nicht anders beschrieben, können die cyclischen Alkylengruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "C₂₋₄-Alkinyl" (auch soweit sie Bestandteil anderer Reste sind) werden verzweigte und unverzweigte Alkinylgruppen mit 2 bis 4 Kohlenstoffatomen verstanden, soweit sie mindestens eine Dreifachbindung aufweisen. Beispielsweise werden hierfür genannt: Ethinyl, Propinyl oder Butinyl. Sofern nicht anders beschrieben, umfassen die Definitionen Propinyl und Butinyl alle denkbaren isomeren Formen der jeweiligen Reste. So umfasst beispielsweise Propinyl 1-Propinyl und 2-Propinyl, Butinyl umfasst 1-Butinyl, 2-Butinyl und 3-Butinyl etc.

"Halogen" steht im Rahmen der vorliegenden Erfindung für Fluor, Chlor, Brom oder Jod. Sofern nicht gegenteilig angegeben, gelten Fluor, Chlor und Brom als bevorzugte Halogene.

Unter dem Begriff "heterocyclische Ringe" oder "Hetercyclus" werden stabile 5- oder 6-gliedrige monocyclische Ringsysteme verstanden, die sowohl gesättigt als auch einfach oder zweifach ungesättigt sein können und neben Kohlenstoffatomen ein oder zwei Heteroatome tragen können, die ausgewählt sind aus der Gruppe bestehend aus Stickstoff, Sauerstoff und Schwefel. Dabei können sowohl Stickstoff- als auch Schwefelheteroatome optional oxidiert sein. Die voranstehend genannten Heterocyclen können über ein Kohlenstoffatom oder über ein Stickstoffatom mit dem restlichen Molekül verknüpft sein. Als Beispiele seien folgende Verbindungen genannt:

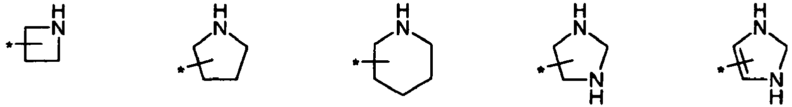

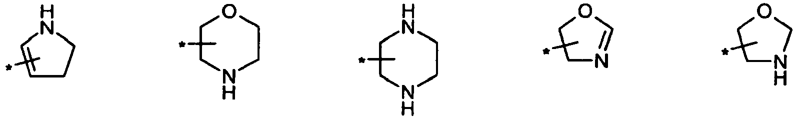

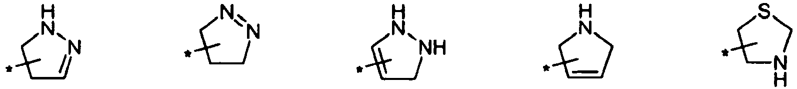

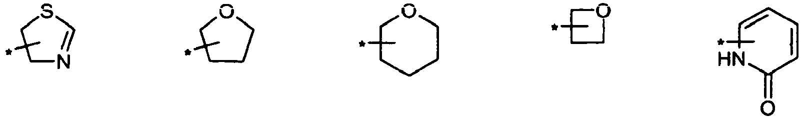

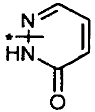

"Cyclische Imide" umfassen beispielsweise Succinimid, Maleimid und Phthalimid.

Unter dem Begriff "Aryl" (auch soweit sie Bestandteil anderer Reste sind) werden aromatische Ringsysteme mit 6 oder 10 Kohlenstoffatomen verstanden. Beispielsweise werden hierfür Phenyl, 1-Naphthyl oder 2-Naphthyl genannt; bevorzugter Arylrest ist Phenyl. Soweit nicht anders beschrieben, können die Aromaten substituiert sein mit einem oder mehreren Resten ausgewählt aus der Gruppe bestehend aus Methyl, Ethyl, n-Propyl, iso-Propyl, tert-Butyl, Hydroxy, Methoxy, Trifluormethoxy, Fluor, Chlor, Brom und Jod, wobei die Reste gleich oder verschieden sein können.

Unter dem Begriff "Heteroaryl" werden fünf- oder sechsgliedrige heterocyclische Aromaten verstanden, die ein, zwei, drei oder vier Heteroatome, ausgewählt aus der Gruppe Sauerstoff, Schwefel und Stickstoff, enthalten können und zusätzlich so viele konjugierte Doppelbindungen enthalten, dass ein aromatisches System gebildet wird. Diese Heteroaryle können zusätzlich mit einem Phenylring benzo-kondensiert sein, so dass neun- oder zehngliedrige bicyclische Heteroaryle gebildet werden.

Als Beispiele für fünf- oder sechsgliedrige Heteroaromaten seien genannt:

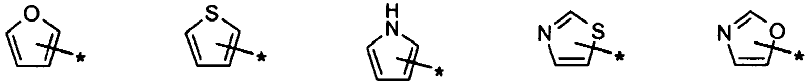

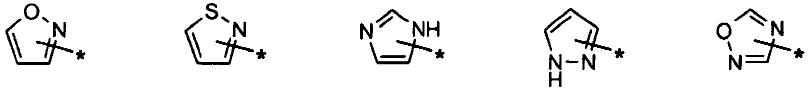

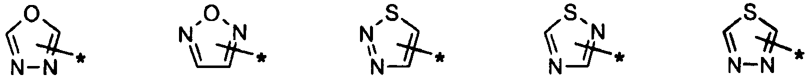

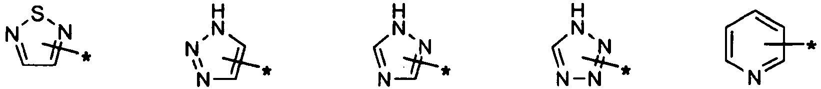

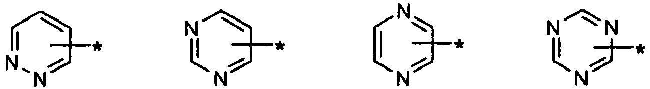

Als Beispiele für neun- oder zehngliedrige heterocyclische Aromaten seien genannt:

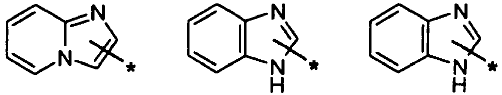

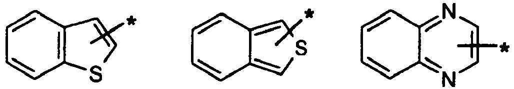

Soweit nicht anders beschrieben, können die voranstehend genannten Heteroaryle substituiert sein mit einem oder mehreren Resten ausgewählt aus der Gruppe bestehend aus Methyl, Ethyl, *n*-Propyl, *iso*-Propyl, *tert*-Butyl, Hydroxy, Methoxy, Trifluormethoxy, Fluor, Chlor, Brom und Jod, wobei die Reste gleich oder verschieden sein können. Weiterhin kann ein im Heteroarylrest vorhandenes Stickstoffatom oxidiert sein, wodurch ein N-Oxid entsteht.

Unter dem Begriff "Oxo-Gruppe" wird ein Sauerstoff-Substituent an einem Kohlenstoffatom verstanden, was zur Ausbildung einer Carbonylgruppe -C(O)- führt. Das Einführen einer Oxo-Gruppe als Substituent an einem nichtaromatischen Kohlenstoffatom führt zu einer Umwandlung einer -CH₂-Gruppe in eine -C(O)-Gruppe. Das Einführen einer Oxo-Gruppe an einem aromatischen Kohlenstoffatom führt zur Umwandlung einer-CH-Gruppe in eine -C(O)-Gruppe und kann den Verlust der Aromatizität zur Folge haben.

Verbindungen der allgemeinen Formel I können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden. Als anorganische Säuren kommen hierfür beispielsweise Bromwasserstoffsäure, Phosphorsäure, Salpetersäure, Salzsäure, Schwefelsäure, Methansulfonsäure, Ethansulfonsäure, Benzolsulfonsäure oder *p*-Toluolsulfonsäure in Frage, als organische Säuren kommen beispielsweise Äpfelsäure, Bernsteinsäure, Essigsäure, Fumarsäure, Maleinsäure, Mandelsäure, Milchsäure, Weinsäure oder Zitronensäure in Betracht.

Weiterhin lassen sich die Verbindungen der allgemeinen Formel **I**, sofern sie geeignete Carbonsäurefunktionen enthalten, insbesondere für pharmazeutische Anwendungen in ihre physiologisch verträglichen Salze mit anorganischen oder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dicyclohexylamin in Betracht.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enantiomeren, aus denen sich die erwähnten Racemate zusammensetzen.

Verbindungen mit einer Kohlenstoff-Doppelbindung können sowohl in der E- als auch Z-Form vorliegen.

Falls eine Verbindung in verschiedenen tautomeren Formen vorliegen kann, so ist die dargestellte Verbindung nicht auf eine tautomere Form beschränkt, sondern umfasst alle tautomeren Formen. Dies gilt insbesondere auch bei stickstoffhaltigen Heteroarylen:

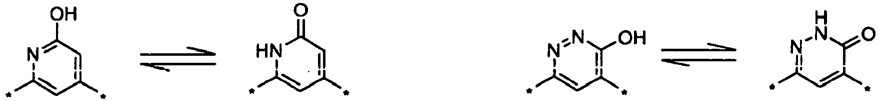

### HERSTELLVERFAHREN

Erfindungsgemäß erhält man die Verbindungen der allgemeinen Formel I nach an sich bekannten Verfahren, beispielsweise nach folgenden Verfahren:

### (A) Amid-Kupplung:

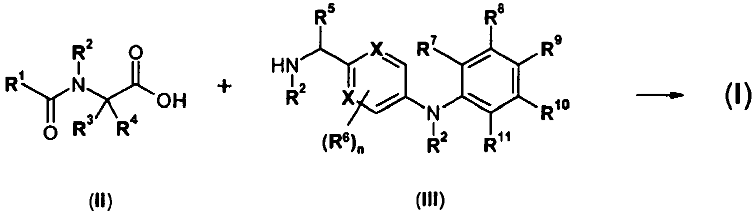

Die dargestellte Verknüpfung von Carbonsäuren der allgemeinen Formel **II**, in der alle Reste wie vorstehend erwähnt definiert sind, mit Aminen der allgemeinen Formel **III**, in der alle Reste wie vorstehend erwähnt definiert sind, unter Bildung von Carbonsäureamiden der allgemeinen Formel **I**, in der alle Reste wie vorstehend erwähnt definiert sind, kann mit herkömmlichen Methoden zur Amidbildung durchgeführt werden.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z.B. Dicyclohexylcarbodiimid (DCC), Diisopropylcarbodiimid (DIC) oder Ethyl-(3-dimethylamino-propyl)-carbodiimid, *O*-(1*H*-Benzotriazol-1-yl)-*N,N-N',N*'-tetramethyluronium-hexafluorphosphat (HBTU) oder -tetrafluorborat (TBTU) oder 1*H*-Benzotriazol-1-yl-oxy-tris-(dimethylamino)-phosphoniumhexafluorphosphat (BOP) eingesetzt werden. Durch Zugabe von 1-Hydroxybenzotriazol (HOBt) oder von 3-Hydroxy-4-oxo-3,4-di-hydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösungsmitteln wie Dichlormethan, Tetrahydrofuran (THF), Acetonitril, Dimethylformamid (DMF), Dimethylacetamid (DMA), *N*-Methylpyrrolidon (NMP) oder Gemischen aus diesen durchgeführt. Sofern erforderlich wird zusätzlich eine Hilfsbase wie beispielsweise Diisopropylethylamin (DIPEA, Hünig-Base) eingesetzt.

### (B) Amid-Kupplung:

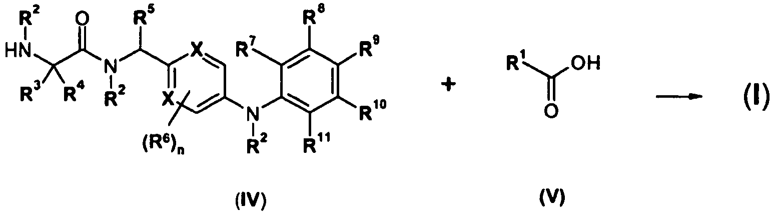

Eine alternative Methode zur Darstellung von Verbindungen der allgemeinen Formel **I** besteht in der Verknüpfung von Carbonsäuren der allgemeinen Formel **V**, in der alle Reste wie vorstehend erwähnt definiert sind, mit Aminen der allgemeinen Formel **IV**, in der alle Reste wie vorstehend erwähnt definiert sind.

Die Verbindungen der allgemeinen Formel **V** sind entweder käuflich erhältlich oder können nach literaturbekannten Methoden hergestellt werden.

Weiter ist es möglich, die Carbonsäuren der allgemeinen Formel **V** in Carbonsäurechloride zu überführen und diese anschließend mit Aminen der allgemeinen Formel **IV** umzusetzen. Die Synthese von Carbonsäurechloriden erfolgt nach literaturbekannten Verfahren (siehe z. B. Houben-Weyl, Methoden der Organischen Chemie, Bd. E5/1).

### (C) Reduktion der Nitrilgruppe:

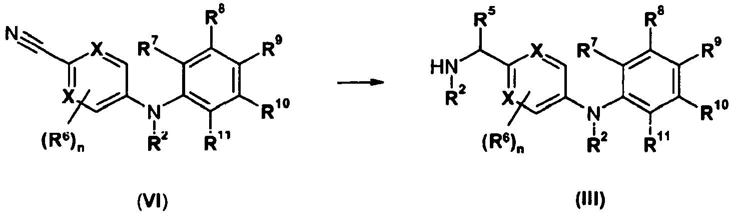

Die Reduktion eines Nitrils der allgemeinen Formel **VI** zu einem Amin der allgemeinen Formel **III,** in der der Rest **R²** am aminischen Stickstoff Wasserstoff bedeutet und alle übrigen Reste wie vorstehend erwähnt definiert sind, kann unter Standard-Bedingungen der katalytischen Hydrogenolyse mit einem Katalysator wie beispielsweise Raney-Nickel in einem Lösungsmittel wie ammoniakalischem Methanol oder Ethanol oder mit einem Reduktionsmittel wie Lithiumaluminiumhydrid oder Natriumborhydrid in einem Lösungsmittel wie Tetrahydrofuran, gegebenenfalls in Gegenwart einer Lewissäure wie Aluminiumchlorid, durchgeführt werden.
Verbindungen der allgemeinen Formel **III,** in der der Rest **R²** am aminischen Stickstoff nicht Wasserstoff sondern beispielsweise ein Alkylrest bedeutet, lassen sich ebenfalls aus Verbindungen der allgemeinen Formel **VI** herstellen. So werden beispielsweise aus der Umsetzung eines Nitrils der allgemeinen Formel **VI** mit einem Alkylgrignard-Reagenz Ketone erhalten, die mittels reduktiver Aminierung in die Verbindungen der allgemeinen Formel **III** überführt werden können. Die reduktive Aminierung erfolgt nach bekannten Verfahren, beispielsweise mit einem Reduktionsmittel wie Natriumtriacetoxyborhydrid, Natriumborhydrid oder Natriumcyanoborhydrid zweckmäßigerweise in einem Lösungsmittel wie Tetrahydrofuran oder Dichlorethan gegebenenfalls unter Zusatz von Essigsäure.
Alternativ können die erhaltenen Ketone auch in Oxime überführt werden. Die anschliessende Reduktion der Oxime liefert dann Verbindungen der allgemeinen Formel **III**.

### (D) nucleophile aromatische Substitution oder Übergangsmetall-katalysierte Kupplung:

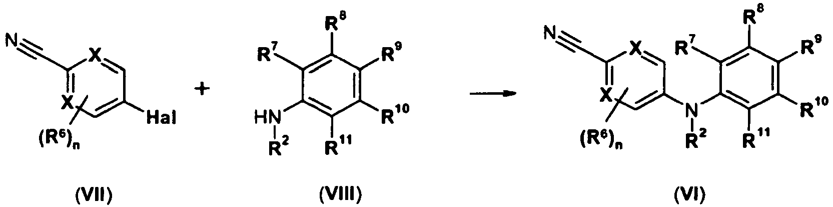

Die Reaktion eines Anilins der allgemeinen Formel **VIII**, in der alle Reste wie vorstehend erwähnt definiert sind, mit einem Nitril der allgemeinen Formen **VII**, in der **X**, **R₆** und **n** wie vorstehend erwähnt definiert sind, und **Hal** ein Fluor-, Chlor- oder Bromatom bedeutet, erfolgt nach bekannten Verfahren, beispielsweise in einem Lösungsmittel wie Tetrahydrofuran, Dimethylformamid oder Dimethylsulfoxid und zweckmäßigerweise in Gegenwart einer Base wie Triethylamin, Natronlauge oder Kaliumcarbonat bei einer Temperatur von 20°C bis 160°C. Ist das Anilin der allgemeinen Formel **VIII** flüssig, kann die Reaktion auch ohne Lösungsmittel und zusätzlicher Base durchgeführt werden.

Eine alternative Methode zur Darstellung von Verbindungen der allgemeinen Formel **VI** stellt die Palladium katalysierte Reaktion eines Nitrils der allgemeinen Formel **VII**, in dem **Hal** Brom oder Chlor bedeutet, mit einem Anilin der allgemeinen Formel **VIII** dar. Reaktionsbedingungen für diese auch als Buchwald-Hartwig Reaktion bekannten Umsetzung sind aus der Literatur bekannt.

### Methodenbeschreibung zur cynoBK1-Rezeptorbindung

CHO -Zellen, die den Cynomolgus B1-Rezeptor exprimieren, werden in "HAM'S F-12 Medium" kultiviert. Von konfluenten Kulturen wird das Medium entfernt, die Zellen werden mit PBS-Puffer gewaschen, abgeschabt oder mit Versene abgelöst und durch Zentrifugieren isoliert. Anschließend werden die Zellen in Suspension homogenisiert, das Homogenat zentrifugiert und resuspendiert. Nach Bestimmung des Proteingehalts werden 200 µl des Homogenats (50 bis 250 µg Protein/Assay) für 60-180 Minuten bei Raumtemperatur mit 0.5 bis 5.0 nM Kallidin (DesArg10,Leu9), [3,4-Prolyl-3,43H(N)] und ansteigenden Konzentrationen der Testsubstanz in einem Gesamtvolumen von 250 µl inkubiert. Die Inkubation wird durch rasche Filtration durch GF/B-Glasfaserfilter, die mit Polyethyleneimine (0.3%) vorbehandelt wurden, beendet. Die an das Protein gebundene Radioaktivität wird mit einem TopCount NXT gemessen. Als nichtspezifische Bindung wird die gebundene Radioaktivität in Gegenwart von 1.0 µM (DesArg10) Kallidin definiert. Die Analyse der Konzentrations-Bindungskurve kann mit Hilfe einer computergestützten nichtlinearen Kurvenanpassung erfolgen, um für die Testsubstanz die entsprechenden Kᵢ-Wert zu ermittelt.

**Testergebnisse vom cynoBK1-Rezeptorbindungsassay:**

| **Beispiel Nr.** | **%Inhibierung bei 10 µmol/L** | **Kᵢ [nM]** |
|---|---|---|
| (1) | 54 | |
| (2) | 100 | 25 |
| (3) | 65 | |
| (4) | 109 | |
| (5) | 88 | |
| (6) | 59 | |
| (7) | 100 | |
| (8) | 75 | |
| (9) | 78 | |
| (10) | 58 | |
| (11) | 99 | |
| (12) | 78 | |
| (13) | 101 | |
| (14) | 84 | |
| (15) | 99 | |
| (16) | 91 | |
| (17) | 75 | |
| (18) | 67 | |
| (19) | 96 | |
| (20) | 81 | |
| (21) | 68 | |
| (22) | 60 | |
| (23) | 110 | |
| (24) | 107 | |
| (25) | 86 | |
| (26) | 109 | |
| (27) | 104 | |
| (28) | 101 | 0.6 |
| (29) | 113 | 6.2 |
| (30) | 111 | 13 |
| (31) | 112 | 13 |
| (32) | 93 | 200 |
| (33) | 110 | 1.9 |
| (34) | 94 | >500 |
| (35) | 111 | 70 |
| (36) | 113 | 21 |
| (37) | 77 | >500 |
| (38) | | |
| (39) | 110 | 5.5 |
| (40) | 110 | 2.9 |
| (41) | 108 | 2.6 |
| (42) | 89 | |
| (43) | 94 | 36 |
| (44) | 109 | 7.0 |
| (45) | 104 | 2 |
| (46) | 88 | 290 |
| (47) | 94 | 39 |
| (48) | 71 | |
| (49) | 103 | 365 |
| (50) | 115 | 0.7 |
| (51) | 112 | 38 |
| (52) | 63 | 312 |
| (53) | 101 | 197 |
| (54) | 112 | 3.8 |
| (55) | 108 | 3.3 |
| (56) | 99 | |
| (57) | 111 | 77 |
| (58) | 109 | |
| (59) | 22 | |
| (60) | 84 | |
| (61) | 97 | >500 |
| (62) | 60 | |
| (63) | 108 | 118 |
| (64) | 109 | 98 |
| (65) | 76 | |
| (66) | 81 | |
| (67) | 63 | |
| (68) | 78 | |
| (69) | 42 | |
| (70) | 107 | |
| (71) | 80 | |
| (72) | 89 | 416 |
| (74) | 56 | |
| (75) | 71 | |
| (76) | 80 | |
| (77) | 96 | |
| (78) | 73 | |
| (79) | 104 | 292 |
| (80) | 88 | |
| (81) | 113 | |
| (82) | 107 | |
| (83) | 113 | 9 |
| (84) | 104 | 90 |
| (85) | 93 | |
| (86) | 108 | 100 |
| (87) | 112 | |
| (88) | 80 | |
| (89) | 96 | |
| (90) | 109 | 135 |
| (91) | 100 | |
| (92) | 110 | 62 |
| (93) | 84 | |
| (94) | 57 | |
| (95) | 86 | |
| (96) | 78 | |
| (97) | | 132 |
| (98) | 103 | 417 |
| (99) | 96 | |
| (100) | 103 | |
| (102) | 105 | 87 |
| (103) | 87 | |
| (104) | 54 | |
| (105) | 113 | 20 |
| (106) | 69 | |
| (107) | 95 | |
| (108) | 101 | 135 |
| (109) | 27 | |
| (110) | 33 | |
| (111) | 20 | |
| (112) | 45 | |
| (113) | 7 | |
| (114) | 77 | >500 |
| (115) | 99 | >500 |
| (116) | 83 | >500 |
| (117) | 111 | 2.8 |
| (118) | 109 | 64 |
| (119) | 82 | 375 |
| (120) | 113 | 2.4 |
| (121) | 113 | 2 |
| (122) | 113 | 6.3 |
| (123) | 110 | 8.3 |
| (124) | 112 | 33 |
| (125) | 108 | 12 |
| (126) | 103 | 46 |
| (127) | 108 | 17 |
| (128) | 103 | 84 |
| (129) | 107 | 11 |
| (130) | 101 | 101 |
| (131) | 105 | 33 |
| (132) | 98 | 180 |
| (133) | 101 | |
| (134) | 101 | 4.7 |
| (135) | 106 | 15 |
| (136) | 104 | 42 |
| (138) | 107 | 6.7 |
| (139) | 107 | 2.8 |
| (140) | 106 | 14 |
| (141) | 100 | 7.6 |
| (142) | 106 | 12 |
| (143) | 106 | 12 |
| (144) | 97 | 126 |
| (145) | 99 | 2.8 |
| (146) | 107 | 7.6 |
| (147) | 107 | 4.4 |
| (148) | 96 | |
| (149) | 100 | |
| (150) | 105 | 37 |
| (151) | 107 | 1.8 |
| (152) | 105 | 3.2 |
| (153) | 106 | 9.7 |
| (154) | 101 | 89 |
| (155) | 101 | 143 |
| (156) | 107 | 8.5 |
| (157) | 96 | 56 |
| (158) | 101 | 115 |
| (159) | 91 | 97 |
| (160) | 106 | 17 |
| (161) | 69 | >500 |
| (162) | 104 | 17 |
| (163) | 87 | 288 |
| (164) | 106 | 3.4 |
| (165) | 101 | |
| (166) | 104 | 45 |
| (167) | 104 | 19 |
| (168) | 90 | 235 |
| (169) | 90 | 292 |
| (170) | 104 | 35 |
| (171) | 97 | |
| (172) | 100 | 9.8 |
| (173) | 105 | 2.7 |
| (174) | 99 | |
| (175) | 96 | 63 |
| (176) | 100 | |
| (177) | 99 | |
| (178) | 107 | 11 |
| (179) | 101 | |
| (180) | 106 | 1.6 |
| (181) | 99 | 19 |
| (182) | 99 | 20 |
| (183) | 100 | 21 |
| (184) | 99 | |
| (185) | 87 | |
| (186) | 82 | |
| (187) | 100 | |
| (188) | 92 | 43 |
| (189) | 96 | >500 |
| (190) | 91 | |
| (191) | 56 | |
| (192) | 95 | |
| (193) | 101 | |
| (194) | 95 | |
| (195) | 42 | >500 |
| (196) | 82 | >500 |
| (197) | 81 | >500 |
| (198) | 64 | >500 |
| (200) | 92 | |
| (201) | | |
| (202) | | |
| (203) | | 1.9 |
| (204) | | 402 |
| (205) | | 17 |
| (206) | | 5.1 |
| (207) | | 84 |
| (208) | | 6.8 |
| (209) | | 73 |
| (210) | | 187 |
| (211) | | 25 |
| (212) | | 8 |
| (213) | | 1.9 |
| (214) | | 13 |
| (215) | | 3.5 |
| (216) | | 7.8 |
| (217) | | 12 |
| (218) | | 3.7 |
| (219) | | 8.9 |
| (220) | | 15 |
| (221) | | 13 |
| (222) | | 68 |
| (223) | | 75 |
| (224) | | 6.3 |
| (225) | | 68 |
| (226) | | 157 |
| (227) | | 97 |
| (228) | | 78 |
| (229) | | 27 |
| (230) | | 6.1 |
| (231) | | 4.2 |
| (232) | | 5.6 |
| (233) | | 10 |
| (234) | | 105 |
| (235) | | 489 |
| (236) | | 8.2 |
| (237) | | 151 |
| (238) | | 6.6 |
| (239) | | 182 |
| (240) | | 506 |
| (241) | | 68 |
| (242) | | 15 |
| (243) | | 28 |
| (244) | | 8.4 |
| (245) | | 18 |
| (246) | | 21 |
| (247) | | 3.9 |
| (248) | | 276 |
| (249) | | 518 |
| (250) | | 175 |
| (251) | | 14 |
| (252) | | 120 |
| (253) | | 49 |
| (254) | | 257 |
| (255) | | 15 |
| (256) | | 72 |
| (257) | | 17 |
| (258) | | 37 |
| (259) | | 394 |
| (260) | | 43 |
| (261) | | 456 |
| (262) | | 37 |
| (263) | | 485 |
| (264) | | 60 |
| (265) | | 75 |
| (266) | | 78 |
| (267) | | 5.5 |
| (268) | | 281 |
| (269) | | 47 |
| (270) | | 51 |
| (271) | | 17 |
| (272) | | 35 |
| (273) | | 141 |
| (274) | | 4.0 |
| (275) | | 405 |
| (276) | | 17 |
| (277) | | 10 |
| (278) | | 16 |
| (279) | | 5.5 |
| (280) | | 25 |
| (281) | | 21 |
| (282) | | 7.9 |
| (283) | | 1.3 |
| (284) | | 5.1 |
| (285) | | 27 |
| (286) | | 36 |
| (287) | | 11 |
| (288) | | 9.1 |
| (289) | | 21 |
| (290) | | 46 |
| (291) | | 40 |
| (292) | | 7.2 |
| (293) | | 65 |
| (294) | | 1.2 |
| (295) | | 31 |
| (296) | | 28 |
| (297) | | 59 |
| (298) | | 0.7 |
| (299) | | 222 |
| (300) | | 45 |
| (301) | | 1.1 |
| (302) | | 42 |
| (303) | | 82 |
| (304) | | 135 |
| (305) | | 76 |
| (306) | | 2.0 |
| (307) | | 54 |
| (308) | | 427 |
| (309) | | 184 |
| (310) | | 39 |
| (311) | | 349 |
| (312) | | 57 |
| (313) | | 95 |
| (314) | | 15 |
| (315) | | 40 |
| (316) | | 57 |
| (317) | | 30 |
| (318) | | 316 |
| (319) | | 58 |
| (320) | | 19 |
| (321) | | 57 |
| (322) | | 12 |
| (323) | | 69 |
| (324) | | 22 |
| (325) | | 374 |
| (326) | | 504 |
| (327) | | 89 |
| (328) | | 33 |
| (329) | | 24 |
| (330) | | 35 |
| (331) | | 66 |
| (332) | | 41 |
| (333) | | 89 |
| (334) | | 20 |
| (335) | | 28 |
| (336) | | 567 |
| (337) | | 312 |
| (338) | | 27 |
| (339) | | 49 |
| (340) | | 28 |
| (341) | | 69 |
| (342) | | 50 |
| (343) | | 7.2 |
| (344) | | 1.9 |
| (345) | | 17 |
| (346) | | 48 |
| (347) | | 12 |
| (348) | | 31 |
| (349) | | 648 |
| (350) | | 43 |
| (351) | | 24 |
| (352) | | 59 |
| (353) | | 52 |
| (354) | | 27 |
| (355) | | 433 |
| (356) | | 22 |
| (357) | | 44 |
| (358) | | 37 |
| (359) | | 125 |
| (360) | | 2.5 |
| (361) | | 10 |
| (362) | | 101 |
| (363) | | 45 |
| (364) | | 77 |
| (365) | | 376 |
| (366) | | 33 |
| (367) | | 508 |
| (368) | | 42 |
| (369) | | 37 |
| (370) | | 1.4 |
| (371) | | 6.1 |
| (372) | | 24 |
| (373) | | 95 |
| (374) | | 5.5 |
| (375) | | 48 |
| (376) | | 61 |
| (377) | | 45 |
| (378) | | 4.6 |
| (379) | | 5.5 |
| (380) | | 6.5 |
| (381) | | 93 |
| (382) | | 6.8 |
| (383) | | 225 |
| (384) | | 21 |
| (385) | | 9.4 |
| (386) | | 15 |
| (387) | | 10 |
| (388) | | 386 |
| (389) | | 15 |
| (390) | | 7.3 |
| (391) | | 9.3 |
| (392) | | 11 |
| (393) | | |
| (394) | | |
| (395) | | 37 |
| (396) | | 6.6 |

### INDIKATIONEN

Aufgrund ihrer pharmakologischen Eigenschaften eignen sich die neuen Verbindungen und deren physiologisch verträgliche Salze zur Behandlung von Krankheiten und Krankheitssymptomen, die wenigstens teilweise durch die Stimulierung von Bradykinin-B1-Rezeptoren hervorgerufen werden, oder in denen eine Antagonisierung des Bradykinin-1 Rezeptors eine Symptomverbesserung bewirken kann.

Ein weiterer Gegenstand der vorliegenden Erfindung umfasst die erfindungsgemäßen Verbindungen der eingangs erwähnten allgemeinen Formel I zur Verwendung als Arzneimittel.

Aufgrund ihrer pharmakologischen Wirkung eignen sich die Substanzen zur Behandlung
(a) von **akuten Schmerzen,** wie beispielsweise Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;
(b) von **Eingeweideschmerzen,** wie beispielsweise chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, Cholecystitis, Prostatitis, bei Angina pectoris, Schmerzen bei Kolon irritabile, bei nichtulzeröser Dyspepsie und bei Gastritis, Prostatitis, nicht-kardialen Thoraxschmerzen und Schmerzen bei myokardialer Ischämie und Herzinfarkt;
(c) von **neuropathischen Schmerzen,** wie beispielsweise schmerzhaften Polyneuropathien, Schmerzen bei diabetischer Neuropathie, AIDS-assoziierten neuropathischen Schmerzen, bei nicht-herpes-assoziierter Neuralgie, bei Post-zoster Neuralgie, bei Nervenverletzungen, bei Schädel-Hirn-Trauma, Schmerzen bei Nervenschädigungen infolge von Toxinen oder Chemotherapie, Phantomschmerzen, Schmerzen bei multipler Sklerose, bei Nervenwurzelabriß und schmerzhaften traumatisch bedingten Einzeinervenschädigungen, sowie zentralem Schmerz wie z.B. nach Schlaganfall, Rückenmarksverletzungen oder Tumoren;
(d) von **entzündlichen** / **Schmerzrezeptor-vermittelten Schmerzen** im Zusammenhang mit Erkrankungen wie beispielsweise Osteoarthritis, rheumatoider Arthritis, rheumatischem Fieber, Tendo-Synovitis, Bursitis, Sehnenentzündungen, Gicht und Gicht-Arthritis, traumatische Arthritiden, Vulvodynie, Schädigungen und Erkrankungen der Muskeln und Faszien, juveniler Arthritis, Spondylitis, Psoriasis-Arthritis, Myositiden, Zahnerkrankungen, Influenza und anderen Virusinfektionen wie Erkältungen, systemischer Lupus erythematodes oder Schmerzen bei Verbrennungen;
(e) von **Tumorschmerzen** im Zusammenhang mit Krebserkrankungen, wie beispielsweise lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;
(f) von **Kopfschmerz-Erkrankungen** unterschiedlicher Ursache, wie beispielsweise Cluster-Kopfschmerz, Migräne (mit oder ohne Aura) und Spannungskopfschmerz;
(g) von Schmerzzuständen gemischter Ursache, wie beispielsweise chronische Rückenschmerzen, einschließlich Lumbago, oder Fibromyalgie.

Weiterhin eigenen sich die Verbindungen zur Behandlung
(h) von entzündlichen Erkrankungen oder Entzündungserscheinungen bei Sonnenbrand und Verbrennungen, von Zahnfleischentzündungen, Ödemen nach Traumata durch Verbrennungen, Hirnödemen und Angioödemen, Darmerkrankungen einschließlich Morbus Crohn und Colitis ulzerosa, Reizdarmsyndrom, Pankreatitis, Nephritis, Zystitis (interstitielle Zystitis), Uveitis; entzündlichen Erkrankungen der Haut (wie z.B. Psoriasis und Ekzeme), vaskulären Bindegewebserkrankungen, Zerrungen und Frakturen, sowie muskuloskeletale Erkrankungen mit Entzündungserscheinungen wie akutes rheumatisches Fieber, Polymyalgia Rheumatica, reaktive Arthritiden, Rheumatiode Arthritis, Spondylarthritiden, aber auch Oseoarthritis, sowie entzündliche Bindegewebserkrankungen anderer Genese, und Kollagenosen jeglicher Genese wie systemischer Lupus Erythematodes, Sklerodermie, Polymyositis, dermatomyositis, Sjögren Syndrom, Morbus Still oder Felty Syndrom;
(i) von entzündlichen Veränderungen im Zusammenhang mit Erkrankungen der Atemwege wie Asthma bronchiale, einschließlich allergischem Asthma (atopisch und nicht-atopisch) sowie Bronchospasmen bei Anstrengung, beruflich-bedingtem Asthma, viraler oder bakterieller Exazerbation einer bestehenden Asthma-Erkrankung und anderen nicht-allergisch bedingten asthmatischen Erkrankungen;
(j) von chronischer Bronchitis sowie chronisch obstruktiver Lungen-Erkrankung (COPD) einschließlich Lungenemphysem, virale oder bakterielle Exazerbation von chronischer Bronchitis oder chronisch obstruktiver Bronchitis, akutem Atemnotsyndrom des Erwachsenen (ARDS), Bronchitis, Lungenentzündung, allergischer Rhinitis (saisonal und ganzjährig), vasomotorischer Rhinitis und Staublungen-Erkrankungen wie Aluminose, Anthrakose, Asbestose, Chalikose, Siderose, Silikose, Tabakose, Byssinose, exogen allergische Alveolitiden, Lungenfibrose, Bronchiektasien, Lungenerkrankungen bei alpha1-Antritrypsinmangel und Husten;
(k) von Diabetes Mellitus und dessen Folgen (wie beispielsweise diabetische Vaskulopathie, diabetische Neuropathie, diabetische Retinopathie) und von diabetischen Symptomen bei Insulitis (beispielsweise Hyperglycämie, Diurese, Proteinurie und erhöhte renale Nitrit- und Kallikrein-Exkretion);
(l) von Sepsis und septischem Schock nach bakteriellen Infektionen oder nach Trauma;
(m) von Juckreiz (Pruritus) verursachenden Syndromen und allergischen Hautreaktionen;
(n) von Verletzungen des Zentralnervensystems;
(o) von Wunden und Gewebeschädigungen;
(p) von benigner Prostata-Hyperplasie und hyperaktiver Blase;
(q) von vaskulären Erkrankungen wie Panarteriitis nodosa, Polyarthritis nodosa, Periarteriitis nodosa, Arteriitis temporalis, Wegnersche Granulomatose, Riesenzellartriitis, Arteriosklerose, Erythema nodosum;
(r) von Störungen der Motilität oder Spasmen von respiratorischen, genito-urinalen, gastro-intestinalen inclusive biliärer, oder vaskulären Strukturen und Organen;
(s) von post-operativem Fieber;
(t) zur Behandlung und Vorbeugung von cardiovasculären Erkrankungen, wie beispielsweise Bluthochdruck und verwandte Erkrankungen;
(u) zur Behandlung und Vorbeugung von Krebs und verwandten Erkrankungen;
(v) zur Behandlung und Vorbeugung von psychatrischen Erkrankungen, wie beispielsweise Depressionen;
(w) zur Behandlung und Vorbeugung von Harninkontinenz und verwandten Erkrankungen;
(x) zur Behandlung und Vorbeugung von krankhaftem Übergewicht und verwandten Erkrankungen;
(y) zur Behandlung und Vorbeugung von Artheriosklerose und verwandten Erkrankungen; und
(z) zur Behandlung und Vorbeugung von Epilepsie.

Die Substanzen eignen sich zur kausalen Behandlung im Sinne einer Verlangsamung oder Unterbrechung des Voranschreitens chronisch progredienter Erkrankungen, insbesondere von Osteoarthritis, Rheumatoider Arthritis und Spondylarthritiden.

Ein weiterer Gegenstand der vorliegenden Erfindung umfasst die Verwendung der erfindungsgemäßen Verbindungen der eingangs erwähnten allgemeinen Formel I zur Herstellung eines Arzneimittels für eine therapeutische Anwendung in den voranstehend genannten Indikationen.

Bevorzugt werden die erfindungsgemäßen Verbindungen der allgemeinen Formel I zur Behandlung von Osteoarthritis, Rheumatioder Arthritis oder COPD eingesetzt.

Unter dem Ausdruck "Behandlung" oder "Therapie" ist eine therapeutische Behandlung von Patienten mit manifester, akuter oder chronischer Indikation zu verstehen, wobei einerseits die symptomatische (palliative) Behandlung zur Linderung der Krankheitssymptome und andererseits die kausale oder kurative Behandlung der Indikation mit dem Ziel, den pathologischen Zustand zu beenden, den Schweregrad des pathologischen Zustands zu vermindern oder die Progression des pathologischen Zustands zu verzögern, abhängig von der Art oder Schwere der Indikation, eingeschlossen sind.

Ein weiterer Gegenstand der vorliegenden Erfindung ist die Verwendung einer Verbindung der allgemeinen Formel I zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen, Kopfschmerz-Erkrankungen und Schmerzzuständen gemischter Ursache sowie weiterer der voranstehend genannten Erkrankungen. Dabei ist die Verwendung dadurch gekennzeichnet, dass sie die Verabreichung einer wirksamen Menge einer Verbindung der allgemeinen Formel I oder eines physiologisch verträglichen Salzes davon an einen Patienten beinhaltet, der einer solchen Behandlung bedarf.

Unter dem Begriff "Patient" wird vorzugsweise ein Mensch verstanden.

Neben der Eignung als Humantherapeutika, sind diese Substanzen auch nützlich in der veterinärmedizinischen Therapie von Haustieren, exotischen Tieren und Nutztieren.

### KOMBINATIONEN

Zur Behandlung von Schmerzen kann es vorteilhaft sein, die erfindungsgemäßen Verbindungen mit belebenden Stoffen wie Koffein oder anderen schmerzlindernden Wirkstoffen zu kombinieren. Stehen zur Behandlung der Ursache der Schmerzen geeignete Wirkstoffe zur Verfügung, so können diese mit den erfindungsgemäßen Verbindungen kombiniert werden.

Für eine Kombinationstherapie kommen beispielsweise die folgenden Verbindungen in Frage:
Nicht-steroidale Antirheumatika (NSAR), wie beispielsweise Propionsäure-Derivate, die ausgewählt sein können aus der Gruppe bestehend aus Alminoprofen, Bucloxinsäure, Carprofen, Fenoprofen, Ibuprofen, Ketoprofen, Naproxen, Oxaprozin, Pirprofen, Pranoprofen und Tiaprofensäure; Essigsäure-Derivate, die ausgewählt sein können aus der Gruppe bestehend aus Indomethacin, Acemetacin, Alcofenac, Isoxepac, Sulindac und Tolmetin; Fenaminsäure-Derivate, die ausgewählt sein können aus der Gruppe bestehend aus Meclofenaminsäure, Mefenaminsäure und Tolfenaminsäure; Biphenyl-Carboxylsäure-Derivate; Oxicame, die ausgewählt sein können aus der Gruppe bestehend aus Meloxicam, Piroxicam und Tenoxicam; Salicylsäure-Derivate, die ausgewählt sein können aus der Gruppe bestehend aus Acetylsalicylsäure und Sulfasalazin; Pyrazolone, die ausgewählt sein können aus der Gruppe bestehend aus Apazon und Feprazon); Coxibe, die ausgewählt sein können aus der Gruppe bestehend aus Celecoxib und Etoricoxib.

Opiat Rezeptor Agonisten, die beispielsweise ausgewählt sein können aus der Gruppe bestehend aus wie Morphin, Darvon, Tramadol und Buprenorphin.

Cannabinoid Agonisten, wie beispielsweise GW-1000.

Natriumkanalblocker, die beispielsweise ausgewählt sein können aus der Gruppe bestehend aus Carbamazepin, Mexiletin, Pregabalin, Tectin und Ralfinamide.

N-Typ Calciumkanalblocker, wie beispielsweise Ziconotid.

Serotonerge und noradrenerge Modulatoren, die beispielsweise ausgewählt sein können aus der Gruppe bestehend aus Duloxetin und Amitriptylin.

Corticosteroide, die beispielsweise ausgewählt sein können aus der Gruppe bestehend aus Betamethason, Budesonid, Cortison, Dexamethason, Hydrocortison, Methylprednisolon, Prednisolon, Prednison und Triamcinolon.

Histamin H1-Rezeptorantagonisten, die beispielsweise ausgewählt sein können aus der Gruppe bestehend aus Brompheniramin, Chlorpheniramin, Dexchlorpheniramin, Triprolidin, Clemastin, Diphenhydramin, Diphenylpyralin, Tripelennamin, Hydroxyzin, Promethazin, Trimeprazin, Azatadin, Cyproheptadin, Antazolin, Pheniramin, Pyrilamin, Loratadin, Cetirizin, Desloratadin, Fexofenadin und Levocetirizin.

Leukotrien Antagonisten und 5-Lipoxygenasehemmer, die beispielsweise ausgewählt sein können aus der Gruppe bestehend aus Zafirlukast, Montelukast, Pranlukast und Zileuton. Lokalanästhetika, die beispielsweise ausgewählt sein können aus der Gruppe bestehend aus Ambroxol und Lidocain.

TRPV1 Antagonisten, die beispielsweise ausgewählt sein können aus der Gruppe bestehend aus AZD-1386, JTS-653 und PHE-377.

Nikotinrezeptor Agonisten, wie beispielsweise A-366833.

P2X3-Rezeptor Antagonisten, wie beispielsweise A-317491.

anti-NGF Antikörper und NGF Antagonisten, die beispielsweise ausgewählt sein können aus der Gruppe bestehend aus JNJ-42160443 und PPH 207.

NK1 und NK2 Antagonisten, wie beispielsweise CP-728663.

NMDA Antagonisten, die beispielsweise ausgewählt sein können aus der Gruppe bestehend aus CNS-5161, AZ-756 und V-3381.

Kaliumkanal Modulatoren, wie beispielsweise CL-888.

GABA Modulatoren, wie beispielsweise Baclofen.

Anti-Migräne Therapeutika, die beispielsweise ausgewählt sein können aus der Gruppe bestehend aus Sumatriptan, Zolmitriptan, Naratriptan und Eletriptan.

Zur Behandlung von einer oder mehrerer der voranstehend genannten Atemwegserkrankungen kann es vorteilhaft sein, die erfindungsgemäßen Verbindungen der allgemeinen Formel **I** mit anderen Wirkstoffen zur Behandlung von Atemwegserkrankungen zu kombinieren. Stehen zur Behandlung der Ursache der Atemwegserkrankungen geeignete Wirkstoffe zur Verfügung, so können diese mit den erfindungsgemäßen Verbindungen kombiniert werden.

Gegebenenfalls können die Verbindungen der allgemeinen Formel **I** auch in Kombination mit weiteren pharmakologisch aktiven Wirkstoffen eingesetzt werden. Bevorzugt gelangen hierbei solche Wirkstoffe zur Anwendung, die beispielsweise ausgewählt sind aus der Gruppe bestehend aus Betamimetika, Anticholinergika, Corticosteroiden, weiteren PDE4-Inhibitoren, LTD4-Rezeptor (CysLT1, CysLT2, CysLT3) Antagonisten, Inhibitoren von MAP Kinasen wie zum Beispiel p38, ERK1, ERK2, JNK1, JNK2, JNK3 oder SAP, LTB4-Rezeptor (BLT1, BLT2) Antagonisten, EGFR-Hemmern, H1-Rezeptor Antagonisten, Antihistaminika, H4-Rezeptor Antagonisten, PAF-Antagonisten und PI3-Kinase Inhibitoren CXCR1 und/ oder CXCR2 Rezeptor Antagonisten und Substanzen gegen Husten.

Die Verbindungen der allgemeinen Formel I können auch in Form von Zwei- oder Dreifachkombinationen davon eingesetzt werden, wie beispielsweise Kombinationen von Verbindungen der Formel I mit ein oder zwei Verbindungen aus der Gruppe bestehend aus
- Betamimetika, Corticosteroiden, PDE4-Inhibitoren, EGFR-Hemmer und LTD4-Antagonisten,
- Anticholinergika, Betamimetika, Corticosteroiden, PDE4-Inhibitoren, EGFR-Hemmern und LTD4-Antagonisten,
- PDE4-Inhibitoren, Corticosteroiden, EGFR-Hemmern und LTD4-Antagonisten,
- EGFR-Hemmern, PDE4-inhibitoren und LTD4-Antagonisten,
- EGFR-Hemmern und LTD4-Antagonisten,
- CCR3-Inhibitoren, iNOS-Inhibitoren (inducible nitric oxide synthase-Inhibitoren), (6R)-L-erythro-5,6,7,8-tetrahydrobiopterin (im folgenden "BH4" genannt) und dessen Derivate, die in der WO 2006/120176 genannt sind, und SYK-Inhibitoren (spleen tyrosine kinase-Inhibitoren),
- Anticholinergika, Betamimetika, Corticosteroiden, PDE4-Inhibitoren und MRP4-Inhibitoren.

Auch die Kombinationen dreier Wirkstoffe je einer der oben genannten Verbindungsklassen ist Bestandteil der Erfindung.

Als **Betamimetika** gelangen erfindungsgemäß vorzugsweise Verbindungen zur Anwendung, die ausgewählt sind aus der Gruppe bestehend aus Arformoterol, Carmoterol, Formoterol, Indacaterol, Salmeterol, Albuterole, Bambuterol, Bitolterol, Broxaterol, Carbuterol, Clenbuterol, Fenoterol, Hexoprenalin, Ibuterol, Isoetharin, Isoprenalin, Levosalbutamol, Mabuterol, Meluadrin, Metaproterenol, Milveterol, Orciprenalin, Pirbuterol, Procaterol, Reproterol, Rimiterol, Ritodrin, Salmefamol, Soterenol, Sulphonterol, Terbutalin, Tiaramid, Tolubuterol und Zinterol oder
- 6-Hydroxy-8-{1-hydroxy-2-[2-(4-methoxy-phenyl)-1,1-dimethyl-ethylamino]-ethyl}-4H-benzo[1,4]oxazin-3-on,
- 8-{2-[2-(2,4-Difluor-phenyl)-1,1-dimethyl-ethylamino]-1-hydroxy-ethyl}-6-hydroxy-4H-benzo[1,4]oxazin-3-on,
- 8-{2-[2-(3,5-Difluor-phenyl)-1,1-dimethyl-ethylamino]-1-hydroxy-ethyl}-6-hydroxy-4H-benzo[1,4]oxazin-3-on,
- 8-{2-[2-(4-Ethoxy-phenyl)-1,1-dimethyl-ethylamino]-1-hydroxy-ethyl}-6-hydroxy-4H-benzo[1,4]oxazin-3-on,
- 8-{2-[2-(4-Fluor-phenyl)-1,1-dimethyl-ethylamino]-1-hydroxy-ethyl}-6-hydroxy-4H-benzo[1,4]oxazin-3-on,
- N-(5-{2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid,
- N-(5-{2-[3-(4,4-Diethyl-6-fluoro-2-oxo-4H-benzo[d][1,3]oxazin-1-yl)-1,1-dimethylpropylamino]-1-hydroxy-ethyl}-2-hydroxy-phenyl)-methansulfonamid,
- N-(5-{2-[3-(4,4-Diethyl-6-methoxy-2-oxo-4H-benzo[d][1,3]oxazin-1-yl)-1,1-dimethylpropylamino]-1-hydroxy-ethy}-2-hydroxy-phenyl)-methansulfonamid,
- N-(5-{2-[1,1-Dimethyl-3-(2-oxo-4,4-dipropyl-4H-benzo[d][1,3]oxazin-1-yl)-propylamino]-1-hydroxy-ethyl}-2-hydroxy-phenyl)-methansulfonamid,
- 8-{2-[1,1-Dimethyl-3-(2-oxo-2,3-dihydro-benzoimidazol-1-yl)-propylamino]-1-hydroxyethyl}-6-hydroxy-4H-benzo[1,4]oxazin-3-on,
- 8-{2-[1,1-Dimethyl-3-(6-methyl-2-oxo-2,3-dihydro-benzoimidazol-1-yl)-propylamino]-1-hydroxy-ethyl}-6-hydroxy-4H-benzo[1,4]oxazin-3-on,
- 8-{2-[1,1-Dimethyl-3-(2-oxo-5-trifluormethyl-2,3-dihydro-benzoimidazol-1-yl)-propylamino]-1-hydroxy-ethyl}-6-hydroxy-4H-benzo[1,4]oxazin-3-on,
- 8-{2-[1,1-Dimethyl-3-(3-methyl-2-oxo-2,3-dihydro-benzoimidazol-1-yl)-propylamino]-1-hydroxy-ethyl}-6-hydroxy-4H-benzo[1,4]oxazin-3-on,
- N-[2-Hydroxy-5-((1R)-1-hydroxy-2-{2-[4-(2-hydroxy-2-phenyl-ethylamino)-phenyl]-ethylamino}-ethyl)-phenyl]-formamid
- 8-Hydroxy-5-((1R)-1-hydroxy-2-{2-[4-(6-methoxy-biphenyl-3-ylamino)-phenyl]-ethylamino}-ethyl)-1H-chinolin-2-on,
- 8-Hydroxy-5-[(1R)-1-hydroxy-2-(6-phenethylamino-hexylamino)-ethyl]-1H-chinolin-2-on,
- 5-[(1R)-2-(2-{4-[4-(2-Amino-2-methyl-propoxy)-phenylamino]-phenyl}-ethylamino)-1-hydroxy-ethyl]-8-hydroxy-1 H-chinolin-2-on,
- [3-(4-{6-[(2R)-2-Hydroxy-2-(4-hydroxy-3-hydroxymethyl-phenyl)-ethylamino]-hexyloxy}-butyl)-5-methyl-phenyl]-harnstoff,
- 4-((1R)-2-{6-[2-(2,6-Dichlor-benzyloxy)-ethoxy]-hexylamino}-1-hydroxy-ethyl)-2-hydroxymethyl-phenol,
- 3-(4-{6-[(2R)-2-Hydroxy-2-(4-hydroxy-3-hydroxymethyl-phenyl)-ethylamino]-hexyloxy}-butyl)-benzenesulfonamid,
- 3-(3-{7-[(2R)-2-Hydroxy-2-(4-hydroxy-3-hydroxymethyl-phenyl)-ethylamino]-heptyloxy}propyl)-benzenesulfonamid,
- 4-((1R)-2-{6-[4-(3-Cyclopentanesulfonyl-phenyl)-butoxy]-hexylamino}-1-hydroxy-ethyl)-2-hydroxymethyl-phenol,
- *N*-1-Adamantanyl-2-{3-[(2R)-2-({(2R)-2-hydroxy-2-[4-hydroxy-3-(hydroxymethyl)-phenyl]ethyl}amino)propyl]phenyl}acetamid,
- (1R)-5-{2-[6-(2,2-Difluor-2-phenyl-ethoxy)-hexylamino]-1-hydroxy-ethyl}-8-hydroxy-1 H-chinolin-2-on
- (R,S)-4-(2-{[6-(2,2-Difluor-4-phenylbutoxy)hexyl]amino}-1-hydroxy-ethyl)-2-(hydroxylmethyl)phenol,
- (R,S)-4-(2-{[6-(2,2-Difluor-2-phenylethoxy)hexyl]amino}-1-hydroxy-ethyl)-2-(hydroxylmethyl)phenol,
- (R,S)-4-(2-{[4,4-Difluor-6-(4-phenylbutoxy)hexyl]amino}-1-hydroxy-ethyl)-2-(hydroxylmethyl)phenol,
- (R,S)-4-(2-{[6-(4,4-Difluor-4-phenylbutoxy)hexyl]amino}-1-hydroxy-ethyl)-2-(hydroxylmethyl)phenol,
- (R,S)-5-(2-{[6-(2,2-Difluor-2-phenylethoxy)hexyl]amino}-1-hydroxy-ethyl)-8-hydroxychinolin-2(1H)-on,
- (R,S)-[2-({6-[2,2-Difluor-2-(3-methylphenyl)ethoxy]hexyl}amino)-1-hydroxyethyl]-2-(hydroxymethyl)phenol,
- 4-(1R)-2-{[6-(2,2-Difluor-2-phenylethoxy)hexyl]amino}-1-hydroxyethyl)-2-(hydroxylmethyl)phenol,
- (R,S)-2-(Hydroxymethyl)-4-(1-hydroxy-2-{[4,4,5l5-tetrafluor-6-(3-phenylpropoxy)-hexyl]amino}ethyl)phenol,
- (R,S)-[5-(2-{[6-(2,2-Difluor-2-phenylethoxy)hexyl]amino}-1-hydroxy-ethyl)-2-hydroxyphenyl]formamid,
- (R,S)-4-[2-({6-[2-(3-Bromophenyl)-2,2-difluoroethoxy]hexyl}amino)-1-hydroxyethyl]-2-(hydroxymethyl)phenol,
- (R, S)-N-[3-(1,1-Difluor-2-{[6-({2-hydroxy-2-[4-hydroxy-3-(hydroxymethyl)phenyl]-ethyl}amino)hexyl]oxy}ethyl)phenyl]-hamstoff,
- 3-[3-(1,1-Difluor-2-{[6-({2-hydroxy-2-[4-hydroxy-3-(hydroxymethyl) phenyl]ethyl}-amino)hexyl]oxy}ethyl)phenyl]imidazolidin-2,4-dion,
- (R,S)-4-[2-({6-[2,2-Difluor-2-(3-methoxyphenyl)ethoxy]hexyl}amino)-1-hydroxyethyl]-2-(hydroxymethyl)phenol,
- 5-((1R)-2-{[6-(2,2-Difluor-2-phenylethoxy)hexyl]amino}-1-hydroxyethyl)-8-hydroxychinolin-2(1 H)-on,
- 4-((1R)-2-{[4,4-Difluor-6-(4-phenylbutoxy)hexyl]amino}-1-hydroxy-ethyl)-2-(hydroxylmethyl)phenol,
- (R,S)-4-(2-{[6-(3,3-Difluor-3-phenylpropoxy)hexyl]amino}-1-hydroxy-ethyl)-2-(hydroxylmethyl)phenol,
- (R,S)-(2-{[6-(2,2-Difluor-2-phenylethoxy)-4,4-difluorohexyl]amino}-1-hydroxyethyl)-2-(hydroxymethyl)phenol,
- (R,S)-4-(2-{[6-(2,2-Difluor-3-phenylpropoxy)hexyl]amino}-1-hydroxyethyl)-2-(hydroxylmethyl)phenol,
- 3-[2-(3-Chlor-phenyl)-ethoxy]-N-(2-diethylamino-ethyl)-N-{2-[2-(4-hydroxy-2-oxo-2,3-dihydro-benzothiazol-7-yl)-ethylamino]-ethyl}propionamid,
- N-(2-Diethylamino-ethyl)-N-{2-[2-(4-hydroxy-2-oxo-2,3-dihydro-benzothiazol-7-yl)-ethylamino]-ethyl}-3-(2-naphthalen-1-yl-ethoxy)-propionamid,
- 7-[2-(2-{3-[2-(2-Chlor-phenyl)-ethylamino]-propylsulfanyl}-ethylamino)-hydroxyethyl]-4-hydroxy-3H-benzothiazol-2-on,

gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **Anticholinergika** gelangen erfindungsgemäß vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Aclidiniumsalze, bevorzugt das Bromidsalz, Glycopyrroniumsalzen, bevorzugt das Bromidsalz, Trospiumsalzen, bevorzugt das Chloridsalz, Tolterodin, (3R)-1-Phenethyl-3-(9H-xanthen-9-carbonyloxy)-1-azoniabicyclo[2.2.2]octan-Salze. In den vorstehend genannten Salzen stellen die Kationen die pharmakologisch aktiven Bestandteile dar. Als Anionen X⁻ können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Weitere Anticholinergika können ausgewählt sein aus der Gruppe bestehend aus
- 2,2-Diphenylpropionsäuretropenolester-Methobromid,
- 2,2-Diphenylpropionsäurescopinester-Methobromid,
- 2-Fluor-2,2-Diphenylessigsäurescopinester-Methobromid,
- 2-Fluor-2,2-Diphenylessigsäuretropenolester-Methobromid,
- 3,3',4,4'-Tetrafluorbenzilsäuretropenolester-Methobromid,
- 3,3',4,4'-Tetrafluorbenzilsäurescopinester-Methobromid,
- 4,4'-Difluorbenzilsäuretropenolester-Methobromid,
- 4,4'-Difluorbenzilsäurescopinester-Methobromid,
- 3,3'-Difluorbenzilsäuretropenolester-Methobromid,
- 3,3'-Difluorbenzilsäurescopinester-Methobromid,
- 9-Hydroxy-fluoren-9-carbonsäuretropenolester-Methobromid,
- 9-Fluor-fluoren-9-carbonsäuretropenolester-Methobromid,
- 9-Hydroxy-fluoren-9-carbonsäurescopinester-Methobromid,
- 9-Fluor-fluoren-9-carbonsäurescopinester-Methobromid,
- 9-Methyl-fluoren-9-carbonsäuretropenolester-Methobromid,
- 9-Methyl-fluoren-9-carbonsäurescopinester-Methobromid,
- Benzilsäurecyclopropyltropinester-Methobromid,
- 2,2-Diphenylpropionsäurecyclopropyltropinester-Methobromid,
- 9-Hydroxy-xanthen-9-carbonsäurecyclopropyltropinester-Methobromid,
- 9-Methyl-fluoren-9-carbonsäurecyclopropyltropinester-Methobromid,
- 9-Methyl-xanthen-9-carbonsäurecyclopropyltropinester-Methobromid,
- 9-Hydroxy-fluoren-9-carbonsäurecyclopropyltropinester-Methobromid,
- 4,4'-Difluorbenzilsäuremethylestercyclopropyltropinester-Methobromid,
- 9-Hydroxy-xanthen-9-carbonsäuretropenolester-Methobromid,
- 9-Hydroxy-xanthen-9-carbonsäurescopinester-Methobromid,
- 9-Methyl-xanthen-9-carbonsäuretropenolester-Methobromid,
- 9-Methyl-xanthen-9-carbonsäurescopinester-Methobromid,
- 9-Ethyl-xanthen-9-carbonsäuretropenolester-Methobromid,
- 9-Difluormethyl-xanthen-9-carbonsäuretropenolester-Methobromid, und
- 9-Hydroxymethyl-xanthen-9-carbonsäurescopinester-Methobromid.

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als **Corticosteroide** gelangen erfindungsgemäß vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisoion, Prednison, Rofleponid, Triamcinolon und Tipredane oderPregna-1,4-dien-3,20-dion, 6-Fluor-11-hydroxy-16,17-[(1-methylethyliden)-bis(oxy)]-21-[[4-[(nitrooxy)methyl]benzoyl]oxy]-, (6-alpha,11-beta,16-alpha)-(9Cl) (NCX-1024)
- 16,17-Butylidendioxy-6,9-difluor-11-hydroxy-17-(methylthio)androst-4-en-3-on (RPR-106541),
- 6,9-Difluor-17-[(2-furanylcarbonyl)oxy]-11-hydroxy-16-methyl-3-oxo-androsta-1,4-dien-17-carbothionsäure-(S)-fluormethylester,
- 6,9-Difluor-11-hydroxy-16-methyl-3-oxo-17-propionyloxy-androsta-1,4-dien-17-carbothionsäure (S)-(2-oxo-tetrahydrofuran-3S-yl)ester, und
- 6-alpha,9-alpha-Difluor-11-beta-hydroxy-16alpha-methyl-3-oxo-17alpha-(2,2,3,3-tetramethylcyclopropylcarbonyl)oxy-androsta-1,4-dien-17beta-carbonsäure-cyanomethylester,
   gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als **PDE4-Inhibitoren** gelangen erfindungsgemäß vorzugsweise Verbindungen zur Anwendung, die ausgewählt sind aus der Gruppe bestehend aus Enprofyllin, Theophyllin, Roflumilast, Ariflo (Cilomilast), TofimilastPumafentrin, Lirimilast, Apremilast, Arofyllin, Atizoram, Oglemilast und Tetomilast oder
• 5-[(N-(2,5-Dichlor-3-pyridinyl)-carboxamid]-8-methoxy-chinolin (D-4418),
• N-(3,5-Dichlor-1-oxido-4-pyridinyl)-carboxamid]-8-methoxy-2-(trifluormethyl)-chinolin (D-4396 (Sch-351591)),N-(3,5-Dichlorpyrid-4-yl)-[1-(4-fluorbenzyl)-5-hydroxy-indol-3-yl]glyoxylsäureamid (AWD-12-281 (GW-842470)), 9-[(2-Fluorphenyl)methyl]-N-methyl-2-(trifluormethyl)-9H-purin-6-amin (NCS-613),
• 4-[(2R)-2-[3-(Cyclopentyloxy)-4-methoxyphenyl]-2-phenylethyl]-pyridin (CDP-840),
• N-[(3R)-3,4,6,7-Tetrahydro-9-methyl-4-oxo-1-phenylpyrrolo[3,2,1-jk][1,4]benzodiazepin-3-yl]-4-pyridincarboxamid (PD-168787),
• 4-[6,7-Diethoxy-2,3-bis(hydroxymethyl)-1-naphthalenyl]-1-(2-methoxyethyl)-2(1H)-pyridinon (T-440),
• 2-[4-[6,7-Diethoxy-2,3-bis(hydroxymethyl)-1-naphthalenyl]-2-pyridinyl]-4-(3-pyridinyl)-1(2H)-phthalazinon (T-2585),
• (3-(3-Cyclopenyloxy-4-methoxybenzyl)-6-ethylamino-8-isopropyl-3H-purin (V-11294A),
• beta-[3-(Cyclopentyloxy)-4-methoxyphenyl]-1,3-dihydro-1,3-dioxo-2H-isoindol-2-propanamid (CDC-801),
• Imidazo[1,5-a]pyrido[3,2-e]pyrazin-6(5H)-on, 9-ethyl-2-methoxy-7-methyl-5-propyl-(D-22888)
• 5-[3-(cyclopentyloxy)-4-methoxyphenyl]-3-[(3-methylphenyl)methyl]-, (3S,5S)-2-Piperidinon (HT-0712),
• 4-[1-[3,4-bis(difluoromethoxy)phenyl]-2-(3-methyl-1-oxido-4-pyridinyl)ethyl]-alpha,alpha-bis(trifluoromethyl)-Benzenemethanol (L-826141),
• N-(3,5-Dichlor-1-oxo-pyridin-4-yl)-4-difluormethoxy-3-cyclopropylmethoxybenzamid,
• (-)p-[(4aR*,10*b*S*)-9-Ethoxy-1,2,3,4,4a,10b-hexahydro-8-methoxy-2-methylbenzo[s]-[1,6]naphthyridin-6-yl]-N,N-diisopropylbenzamid,
• (R)-(+)-1-(4-Brombenzyl)-4-[(3-cyclopentyloxy)-4-methoxyphenyl]-2-pyrrolidon,
• 3-(Cyclopentyloxy-4-methoxyphenyl)-1-(4-N'-[N-2-cyano-S-methyl-isothioureido]-benzyl)-2-pyrrolidon,
• cis[4-Cyano-4-(3-cyclopentyloxy-4-methoxyphenyl)cyclohexan-1-carbonsäure],
• 2-Carbomethoxy-4-cyano-4-(3-cyclopropylmethoxy-4-difluoromethoxyphenyl)-cyclohexan-1-on,
• cis[4-Cyano-4-(3-cyclopropylmethoxy-4-difluormethoxyphenyl)cyclohexan-1-ol],
• (R)-(+)-Ethyl[4-(3-cyclopentyloxy-4-methoxyphenyl)pyrrolidin-2-yliden]acetate,
• (S)-(-)-Ethyl[4-(3-cyclopentyloxy-4-methoxyphenyl)pyrrolidin-2-yliden]acetate,
• 9-Cyclopentyl-5,6-dihydro-7-ethyl-3-(2-thienyl)-9*H*-pyrazolo[3,4-c]-1,2,4-triazolo-[4,3-a]pyridin und
• 9-Cyclopentyl-5,6-dihydro-7-ethyl-3-(tert-butyl)-9*H*-pyrazolo[3,4-c]-1,2,4-triazolo-[4,3-a]pyridin,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **EGFR-Hemmer** gelangen erfindungsgemäß vorzugsweise Verbindungen zur Anwendung, die ausgewählt sind aus der Gruppe bestehend ausCetuximab, Trastuzumab, Panitumumab (= ABX-EGF), Mab ICR-62, Gefitinib, Canertinib und Erlotinib oder
- 4-[(3-Chlor-4-fluorphenyl)amino]-6-{[4-(morpholin-4-yl)-1-oxo-2-buten-1-yl]amino}-7-cyclopropylmethoxy-chinazolin,
- 4-[(3-Chlor-4-fluorphenyl)amino]-6-{[4-(N,N-diethylamino)-1-oxo-2-buten-1-yl]amino}-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopropylmethoxy-chinazolin,
- 4-[(R)-(1-Phenyl-ethyl)amino]-6-{[4-(morpholin-4-yl)-1-oxo-2-buten-1-yl]amino}-7-cyclopentyloxy-chinazolin,
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin,
- g-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin,
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin,
- 4-[(3-Chlor-4-fluor-phenyl)amino]-6-[2-((S)-6-methyl-2-oxo-morpholin-4-yl)-ethoxy]-7-methoxy-chinazolin,
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopentyloxy-chinazolin,
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin,
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin,
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin,
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin,
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin,
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin,
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin,
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin,
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin,
- 4-[(3-Ethinyl-phenyl)amino]-6,7-bis-(2-methoxy-ethoxy)-chinazolin,
- 4-[(3-Chlor-4-fluorphenyl)amino]-7-[3-(morpholin-4-yl)-propyloxy]-6-[(vinylcarbonyl)-amino]-chinazolin,
- 4-[(R)-(1-Phenyl-ethyl)amino]-6-(4-hydroxy-phenyl)-7H-pyrrolo[2,3-d]pyrimidin,
- 3-Cyano-4-[(3-chlor-4-fluorphenyl)amino]-6-{[4-(N,N-dimethylamino)-1-oxo-2-buten-1-yl]amino}-7-ethoxy-chinolin,
- 4-{[3-Chlor-4-(3-fluor-benzyloxy)-phenyl]amino}-6-(5-{[(2-methansulfonyl-ethyl)amino]-methyl}-furan-2-yl)chinazolin,
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin,
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin,
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin,
- 4-[(3-Ethinyl-phenyl)amino]-6-{[4-(5,5-dimethyl-2-oxo-morpholin-4-yl)-1-oxo-2-buten-1-yl]amino}-chinazolin,
- 4-[(3-Chlor-4-fluor-phenyl)amino]-6-[2-(2,2-dimethyl-6-oxo-morpholin-4-yl)-ethoxy]-7-methoxy-chinazolin,
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin,
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin,
- 4-[(3-Chlor-4-fluor-phenyl)amino]-6-{2-[4-(2-oxo-morpholin-4-yl)-piperidin-1-yl]-ethoxy}-7-methoxy-chinazolin,
- 4-[(3-Chlor-4-fluor-phenyl)amino]-6-[1-*(tert*-butyloxycarbonyl)-piperidin-4-yloxy]-7-methoxy-chinazolin,
- 4-[(3-Chlor-4-fluor-phenyl)amino]-6-(trans-4-amino-cyclohexan-1-yloxy)-7-methoxychinazolin,
- 4-[(3-Chlor-4-fluor-phenyl)amino]-6-(trans-4-methansulfonylamino-cyclohexan-1-yloxy)-7-methoxy-chinazolin,
- 4-[(3-Chlor-4-fluor-phenyl)amino]-6-(tetrahydropyran-3-yloxy)-7-methoxy-chinazolin,
- 4-[(3-Chlor-4-fluor-phenyl)amino]-6-(1-methyl-piperidin-4-yloxy)-7-methoxy-chinazolin,
- 4-[(3-Chlor-4-fluor-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(methoxymethyl)carbonyl]-piperidin-4-yloxy}-7-methoxy-chinazolin,
- 4-[(3-Chlor-4-fluor-phenyl)amino]-6-(piperidin-3-yloxy)-7-methoxy-chinazolin,
- 4-[(3-Chlor-4-fluor-phenyl)amino]-6-[1-(2-acetylamino-ethyl)-piperidin-4-yloxy]-7-methoxy-chinazolin,
- 4-[(3-Chlor-4-fluor-phenyl)amino]-6-(tetrahydropyran-4-yloxy)-7-ethoxy-chinazolin,
- 4-[(3-Chlor-4-fluor-phenyl)amino]-6-((S)-tetrahydrofuran-3-yloxy)-7-hydroxy-chinazolin,
- 4-[(3-Chlor-4-fluor-phenyl)amino]-6-(tetrahydropyran-4-yloxy)-7-(2-methoxy-ethoxy)-chinazolin,
- 4-[(3-Chlor-4-fluor-phenyl)amino]-6-{trans-4-[(dimethylamino)sulfonylamino]-cyclohexan-1-yloxy}-7-methoxy-chinazolin,
- 4-[(3-Chlor-4-fluor-phenyl)amino]-6-{trans-4-[(morpholin-4-yl)carbonylamino]-cyclohexan-1-yloxy}-7-methoxy-chinazolin,
- 4-[(3-Chlor-4-fluor-phenyl)amino]-6-{trans-4-[(morpholin-4-yl)sulfonylamino]-cyclohexan-1-yloxy)-7-methoxy-chinazolin,
- 4-[(3-Chlor-4-fluor-phenyl)amino]-6-(tetrahydropyran-4-yloxy)-7-(2-acetylaminoethoxy)-chinazolin,
- 4-[(3-Chlor-4-fluor-phenyl)amino]-6-(tetrahydropyran-4-yloxy)-7-(2-methansulfonylamino-ethoxy)-chinazolin,
- 4-[(3-Chlor-4-fluor-phenyl)amino]-6-{1-[(piperidin-1-yl)carbonyl]-piperidin-4-yloxy}-7-methoxy-chinazolin,
- 4-[(3-Chlor-4-fluor-phenyl)amino]-6-(1-aminocarbonylmethyl-piperidin-4-yloxy)-7-methoxy-chinazolin,
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(morpholin-4-yl)carbonyl]-N-methylamino}-cyclohexan-1-yloxy)-7-methoxy-chinazolin,
- 4-[(3-Chlor-4-fluor-phenyl)amino]-6-(cis-4-{N-[(morpholin-4-yl)sulfonyl]-N-methylamino}-cyclohexan-1-yloxy)-7-methoxy-chinazolin,
- 4-[(3-Chlor-4-fluor-phenyl)amino]-6-(trans-4-ethansulfonylamino-cyclohexan-1-yloxy)-7-methoxy-chinazolin,
- 4-[(3-Chlor-4-fluor-phenyl)amino]-6-(1-methansulfonyl-piperidin-4-yloxy)-7-ethoxychinazolin,
- 4-[(3-Chlor-4-fluor-phenyl)amino]-6-(1-methansulfonyl-piperidin-4-yloxy)-7-(2-methoxyethoxy)-chinazolin,
- 4-[(3-Chlor-4-fluor-phenyl)amino]-6-[1-(2-methoxy-acetyl)-piperidin-4-yloxy]-7-(2-methoxy-ethoxy)-chinazolin,
- 4-[(3-Chlor-4-fluor-phenyl)amino]-6-(cis-4-acetylamino-cyclohexan-1-yloxy)-7-methoxychinazolin,
- 4-[(3-Ethinyl-phenyl)amino]-6-[1-(*tert*-butyloxycarbonyl)-piperidin-4-yloxy]-7-methoxychinazolin,
- 4-[(3-Ethinyl-phenyl)amino]-6-(tetrahydropyran-4-yloxy)-7-methoxy-chinazolin,
- 4-[(3-Chlor-4-fluor-phenyl)amino]-6-(cis-4-{N-[(piperidin-1-yl)carbonyl]-N-methylamino}-cyclohexan-1-yloxy)-7-methoxy-chinazolin,
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin,
- 4-[(3-Chlor-4-fluor-phenyl)amino]-6-{cis-4-[(morpholin-4-yl)carbonylamino]-cyclohexan-1-yloxy}-7-methoxy-chinazolin,
- 4-[(3-Chlor-4-fluor-phenyl)amino]-6-{1-[2-(2-oxopyrrolidin-1-yl)ethyl]-piperidin-4-yloxy}-7-methoxy-chinazolin,
- 4-[(3-Chlor-4-fluor-phenyl)amino]-6-{1-[(morpholin-4-yl)carbonyl]-piperidin-4-yloxy}-7-(2-methoxy-ethoxy)-chinazolin,
- 4-[(3-Ethinyl-phenyl)amino]-6-(1-acetyl-piperidin-4-yloxy)-7-methoxy-chinazolin,
- 4-[(3-Ethinyl-phenyl)amino]-6-(1-methyl-piperidin-4-yloxy)-7-methoxy-chinazolin,
- 4-[(3-Ethinyl-phenyl)amino]-6-(1-methansulfonyl-piperidin-4-yloxy)-7-methoxychinazolin,
- 4-[(3-Chlor-4-fluor-phenyl)amino]-6-(1-methyl-piperidin-4-yloxy)-7-(2-methoxy-ethoxy)-chinazolin,
- 4-[(3-Chlor-4-fluor-phenyl)amino]-6-(1-isopropyloxycarbonyl-piperidin-4-yloxy)-7-methoxy-chinazolin,
- 4-[(3-Chlor-4-fluor-phenyl)amino]-6-(cis-4-methylamino-cyclohexan-1-yloxy)-7-methoxy-chinazolin,
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin,
- 4-[(3-Ethinyl-phenyl)amino]-6-(piperidin-4-yloxy)-7-methoxy-chinazolin,
- 4-[(3-Ethinyl-phenyl)amino]-6-[1-(2-methoxy-acetyl)-piperidin-4-yloxy]-7-methoxychinazolin,
- 4-[(3-Ethinyl-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin,
- 4-[(3-Chlor-4-fluor-phenyl)amino]-6-{1-[(2-methyl-morpholin-4-yl)carbonyl]-piperidin-4-yloxy}-7-methoxy-chinazolin,
- 4-[(3-Chlor-4-fluor-phenyl)amino]-6-{1-[(S,S)-(2-oxa-5-aza-bicyclo[2.2.1]hept-5-yl)-carbonyl]-piperidin-4-yloxy}-7-methoxy-chinazolin,
- 4-[(3-Chlor-4-fluor-phenyl)amino]-6-{1-[(N-methyl-N-2-methoxyethyl-amino)carbonyl]-piperidin-4-yloxy}-7-methoxy-chinazolin,
- 4-[(3-Chlor-4-fluor-phenyl)amino]-6-(1-ethyl-piperidin-4-yloxy)-7-methoxy-chinazolin,
- 4-[(3-Chlor-4-fluor-phenyl)amino]-6-{1-[(2-methoxyethyl)carbonyl]-piperidin-4-yloxy}-7-methoxy-chinazolin,
- 4-[(3-Chlor-4-fluor-phenyl)amino]-6-{1-[(3-methoxypropyl-amino)-carbonyl]-piperidin-4-yloxy}-7-methoxy-chinazolin,
- 4-[(3-Chlor-4-fluor-phenyl)amino]-6-[cis-4-(N-methansulfonyl-N-methyl-amino)-cyclohexan-1-yloxy]-7-methoxy-chinazolin,
- 4-[(3-Chlor-4-fluor-phenyl)amino]-6-[cis-4-(N-acetyl-N-methyl-amino)-cyclohexan-1-yloxy]-7-methoxy-chinazolin,
- 4-[(3-Chlor-4-fluor-phenyl)amino]-6-(trans-4-methylamino-cyclohexan-1-yloxy)-7-methoxy-chinazolin,
- 4-[(3-Chlor-4-fluor-phenyl)amino]-6-[trans-4-(N-methansulfonyl-N-methyl-amino)-cyclohexan-1-yloxy]-7-methoxy-chinazolin,
- 4-[(3-Chlor-4-fluor-phenyl)amino]-6-(trans-4-dimethylamino-cyclohexan-1-yloxy)-7-methoxy-chinazolin,
- 4-[(3-Chlor-4-fluor-phenyl)amino]-6-(trans-4-{N-[(morpholin-4-yl)carbonyl]-N-methylamino}-cyclohexan-1-yloxy)-7-methoxy-chinazolin,
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin,
- 4-[(3-Chlor-4-fluor-phenyl)amino]-6-(1-methansulfonyl-piperidin-4-yloxy)-7-methoxychinazolin,
- 4-[(3-Chlor-4-fluor-phenyl)amino]-6-(1-cyano-piperidin-4-yloxy)-7-methoxy-chinazolin,
- 3-Cyano-4-[(3-chlor-4-fluorphenyl)amino]-6-{[4-(N,N-dimethylamino)-1-oxo-2-buten-1-yl]amino}-7-ethoxy-chinolin ;
- [4-[(3-Chlor-4-fluorphenyl)amino]-6-{[4-(homomorpholin-4-yl)-1-oxo-2-buten-1-yl]-amino}-7-[(S)-(tetrahydrofuran-3-yl)oxy]-chinazolin,
- 4-[(3-Chlor-4-fluorphenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin,
- 4-[(3-Chlor-4-fluorphenyl)amino]-7-[2-((S)-6-methyl-2-oxo-morpholin-4-yl)-ethoxy]-6-[(vinylcarbonyl)amino]-chinazolin,
- 4-[(3-Chlor-4-fluorphenyl)amino]-7-[4-((R)-6-methyl-2-oxo-morpholin-4-yl)-butyloxy]-6-[(vinylcarbonyl)amino]-chinazolin,
- 4-[(3-Chlor-4-fluorphenyl)amino]-7-[4-((S)-6-methyl-2-oxo-morpholin-4-yl)-butyloxy]-6-[(vinylcarbonyl)amino]-chinazolin, und
- 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin,
   gegebenenfalls in Form ihrer Racemate, Enantiomere oder Diastereomere, gegebenenfalls in Form ihrer pharmakologisch verträglichen Säureadditionssalze, ihrer Solvate und/oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **LTD4-Rezeptor Antagonisten** gelangen erfindungsgemäß vorzugsweise Verbindungen zur Anwendung, die ausgewählt sind aus der Gruppe bestehend aus Montelukast, Pranlukast und Zafirlukast, oder(E)-8-[2-[4-[4-(4-Fluorphenyl)butoxy]phenyl]ethenyl]-2-(1H-tetrazol-5-yl)-4H-1-benzopyran-4-on (MEN-91507),
• 4-[6-Acetyl-3-[3-(4-acetyl-3-hydroxy-2-propylphenylthio)propoxy]-2-propylphenoxy]-buttersäure (MN-001),
• 1-(((R)-(3-(2-(6,7-Difluor-2-chinolinyl)ethenyl)phenyl)-3-(2-(2-hydroxy-2-propyl)phenyl)-thio)methylcyclopropan-essigsäure,
• 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure und
• [2-[[2-(4-tert-Butyl-2-thiazolyl)-5-benzofuranyl]oxymethyl]phenyl]essigsäure,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Rezeptor Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als **Histamin H1 Rezeptor Antagonisten** gelangen erfindungsgemäß vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Olopatadine, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **Histamin H4 Rezeptor Antagonisten** gelangen erfindungsgemäß vorzugsweise Verbindungen zur Anwendung wie beispielsweise (5-Chlor-1 H-indol-2-yl)-(4-methyl-1-piperazinyl)-Methanon (JNJ-7777120), gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als **MAP Kinase Inhibitoren** gelangen erfindungsgemäß vorzugsweise Verbindungen zur Anwendung, die ausgewählt sind aus der Gruppe bestehend aus:
- Bentamapimod (AS-602801)
- Doramapimod,
- 5-Carbamoylindole (SD-169),
- 6-[(Aminocarbonyl)(2,6-difluorphenyl)amino]-2-(2,4-difluorphenyl)-3-pyridincarboxamid (VX-702),
- alpha-[2-[[2-(3-Pyridinyl)ethyl]amino]-4-pyrimidinyl]-2-benzothiazoleacetonitril (AS-601245),
- 9,12-Epoxy-1H-diindolo[1,2,3-fg:3',2',1'-kl]pyrrolo[3,4-i][1,6]benzodiazocin-10-Carboxylsäure (CEP-1347), und
- 4-[3-(4-Chlorphenyl)-5-(1-methyl-4-piperidinyl)-1 H-pyrazol-4-yl]-pyrimidin (SC-409),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als **Neurokinin (NK1 oder NK2) Antagonisten** gelangen erfindungsgemäß vorzugsweise Verbindungen zur Anwendung, die ausgewählt sind aus der Gruppe bestehend aus: Saredutant, Nepadutant und Figopitant, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als **Substanzen gegen Husten** gelangen erfindungsgemäß vorzugsweise Verbindungen zur Anwendung, die ausgewählt sind aus der Gruppe bestehend aus Hydrocodone, Caramiphen, Carbetapentane und Dextramethorphan, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter **CXCR1 oder CXCR2 Antagonisten** gelangen erfindungsgemäß vorzugsweise Verbindungen zur Anwendung, wie z.B. 3-[[3-[(Dimethylamino)-carbonyl]-2-hydroxyphenyl]amino]-4-[[(R)-1-(5-methylfuran-2-yl)propyl]amino]cyclobut-3-en-1,2-dion (SCH-527123), gegebenenfalls in Form seiner Racemate, Enantiomere, Diastereomere und gegebenenfalls in Form seiner pharmakologisch verträglichen Säureadditionssalze, Prodrugs, Solvate oder Hydrate.

Die zur Erzielung einer schmerzstillenden Wirkung erforderliche Dosierung beträgt bei intravenöser Gabe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils 1 bis 3 x täglich. Die erfindungsgemäß hergestellten Verbindungen können intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

### EXPERIMENTELLER TEIL

Für die hergestellten Verbindungen liegen in der Regel Massenspektren und/oder ¹H-NMR-Spektren vor. Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser. Soweit nicht anders vermerkt sind die bei den Aufarbeitungen der Reaktionslösungen verwendeten Säure-, Basen- und Salzlösungen wässrige Systeme der angegebenen Konzentrationen.

Zu chromatographischen Reinigungen wird Kieselgel der Firma Millipore (MATREX^{™}, 35 bis 70 µm) oder Alox (E. Merck, Darmstadt, Aluminiumoxid 90 standardisiert, 63 bis 200 µm, Artikel-Nr: 1.01097.9050) verwendet.

In den Versuchsbeschreibungen werden die folgenden Abkürzungen verwendet:

| | |
|---|---|
| DC | Dünnschichtchromatogramm |
| DIPEA | Diisopropylethylamin |
| DMA | *N,N*-Dimethylacetamid |
| DMAP | 4-Dimethylaminopyridin |
| DMF | *N,N*-Dimethylformamid |
| DMSO | Dimethylsulfoxid |
| HATU | *O*-(7-Azabenzotriazol-1-yl)-*N,N,N',N'*-tetramethyluroniumhexafluorphosphat |
| RP | Reverse Phase |
| Rₜ | Retentionszeit |
| *tert* | tertiär |
| TBTU | 2-(1*H*-Benzotriazol-1-yl)-1,1,3,3-tetramethyluronium-tetrafluorborat |
| TEA | Triethylamin |
| THF | Tetrahydrofuran |

Folgende analytische HPLC-Methoden wurden verwendet:
Methode 1: Säule: Interchim Strategy C18, 5 µM, 4.6 x 50 mm
Detektion: 220 - 320 nm
Fließmittel A: Wasser / 0.1 % Essigsäure
Fließmittel B: Acetonitril

**Gradient:**

| Zeit in min | %A | %B | Flussrate in mL/min |
|---|---|---|---|
| 0.0 | 95.0 | 5.0 | 3.0 |
| 0.3 | 95.0 | 5.0 | 3.0 |
| 2.0 | 2.0 | 98.0 | 3.0 |
| 2.4 | 2.0 | 98.0 | 3.0 |
| 2.45 | 95.0 | 5.0 | 3.0 |
| 2.8 | 95.0 | 5.0 | 3.0 |

Methode 2: Säule: Merck Cromolith Flash RP18e, 4.6 x 25 mm
Fließmittel A: Wasser / 0.1% Ameisensäure
Fließmittel B: Acetonitril / 0.1% Ameisensäure

**Gradient:**

| Zeit in min | %A | %B | Flussrate in mL/min |
|---|---|---|---|
| 0.0 | 90.0 | 10.0 | 1.6 |
| 2.7 | 10.0 | 90.0 | 1.6 |
| 3.0 | 10.0 | 90.0 | 1.6 |
| 3.3 | 90.0 | 10.0 | 1.6 |

Methode 3: Säule: YMC-Pack ODS-AQ, 3 µM, 4.6 x 75 mm
Fließmittel A: Wasser / 0.15% Ameisensäure
Fließmittel B: Acetonitril

**Gradient:**

| Zeit in min | %A | %B | Flussrate in mUmin |
|---|---|---|---|
| 0.0 | 95.0 | 5.0 | 1.6 |
| 2.0 | 10.0 | 90.0 | 1.6 |
| 5.0 | 10.0 | 90.0 | 1.6 |
| 5.5 | 90.0 | 10.0 | 1.6 |

Methode 4: Säule: Zorbax Stable Bond C18, 1.8 µM, 3 x 30 mm
Fließmittel A: Wasser / 0.15% Ameisensäure
Fließmittel B: Acetonitril

**Gradient:**

| Zeit in min | %A | %B | Flussrate in mL/min |
|---|---|---|---|
| 0.0 | 95.0 | 5.0 | 1.6 |
| 1.0 | 10.0 | 90.0 | 1.6 |
| 2.5 | 10.0 | 90.0 | 1.6 |
| 2.75 | 95.0 | 5.0 | 1.6 |

Methode 5: Säule: Sunfire C18, 3.5 µM, 4.6 x 50 mm
Detektion: 180 - 820 nm
Fließmittel A: Wasser / 0.1% Trifluoressigsäure
Fließmittel B: Acetonitril / 0.1% Trifluoressigsäure
Temperatur: 40°C

**Gradient:**

| Zeit in min | %A | %B | Flussrate in mL/min |
|---|---|---|---|
| 0.0 | 95.0 | 5.0 | 1.5 |
| 2.0 | 0.0 | 100.0 | 1.5 |
| 2.5 | 0.0 | 100.0 | 1.5 |
| 2.6 | 95.0 | 5.0 | 1.5 |

Methode 6: Säule: Sunfire C18, 3.5 µM, 4.6 x 50 mm
Detektion: 180 - 820 nm
Fließmittel A: Wasser / 0.1 % Trifluoressigsäure
Fließmittel B: Acetonitril / 0.1% Trifluoressigsäure
Temperatur: 40°C

**Gradient:**

| Zeit in min | %A | %B | Flussrate in mL/min |
|---|---|---|---|
| 0.0 | 95.0 | 5.0 | 1.5 |
| 2.0 | 0.0 | 100.0 | 1.5 |
| 3.0 | 0.0 | 100.0 | 1.5 |
| 3.4 | 95.0 | 5.0 | 1.5 |

Methode 7: Säule: YMC-Pack ODS-AQ, 3 µM, 4.6 x 75 mm
Fließmittel A: Wasser / 0.15% Ameisensäure
Fließmittel B: Acetonitril

**Gradient:**

| Zeit in min | %A | %B | Flussrate in mL/min |
|---|---|---|---|
| 0.0 | 95.0 | 5.0 | 1.6 |
| 4.5 | 10.0 | 90.0 | 1.6 |
| 5.0 | 10.0 | 90.0 | 1.6 |
| 5.50 | 90.0 | 10.0 | 1.6 |

Methode 8: Säule: Zorbax Stable Bond C18, 1.8 µM, 3 x 30 mm
Fließmittel A: Wasser / 0.15% Ameisensäure
Fließmittel B: Acetonitril

**Gradient:**

| Zeit in min | %A | %B | Flussrate in mL/min |
|---|---|---|---|
| 0.0 | 95.0 | 5.0 | 1.6 |
| 2.00 | 50.0 | 50.0 | 1.6 |
| 2.25 | 10.0 | 90.0 | 1.6 |
| 2.50 | 10.0 | 90.0 | 1.6 |
| 2.75 | 95.0 | 5.0 | 1.6 |

Methode 9: Säule: Zorbax Stable Bond C18, 1.8 µM, 3 x 30 mm
Fließmittel A: Wasser / 0.15% Ameisensäure
Fließmittel B: Acetonitril

**Gradient:**

| Zeit in min | %A | %B | Flussrate in mUmin |
|---|---|---|---|
| 0.0 | 95.0 | 5.0 | 1.6 |
| 2.25 | 10.0 | 90.0 | 1.6 |
| 2.50 | 10.0 | 90.0 | 1.6 |
| 2.75 | 95.0 | 5.0 | 1.6 |

Methode 10: Säule: Zorbax Stable Bond C18, 3.5 µM, 4.6 x 75 mm
Fließmittel A: Wasser / 0.15% Ameisensäure
Fließmittel B: Acetonitril

**Gradient:**

| Zeit in min | %A | %B | Flussrate in mL/min |
|---|---|---|---|
| 0.0 | 95.0 | 5.0 | 1.6 |
| 4.5 | 10.0 | 90.0 | 1.6 |
| 5.0 | 10.0 | 90.0 | 1.6 |
| 5.50 | 90.0 | 10.0 | 1.6 |

Methode 11: Säule: X Terra C18, 3.5 µM, 4.6 x 50 mm
Detektion: 180 - 820 nm
Fließmittel A: Wasser / 0.1 % Trifluoressigsäure
Fließmittel B: Acetonitril / 0.1% Trifluoressigsäure
Temperatur: 40°C

**Gradient:**

| Zeit in min | %A | %B | Flussrate in mL/min |
|---|---|---|---|
| 0.0 | 95.0 | 5.0 | 1.5 |
| 2.0 | 0.0 | 100.0 | 1.5 |
| 3.0 | 0.0 | 100.0 | 1.5 |
| 3.4 | 95.0 | 5.0 | 1.5 |

Methode 12: Säule: Merck Cromolith Flash RP18e, 4.6 x 25 mm
Fließmittel A: Wasser / 0.1 % Ameisensäure
Fließmittel B: Acetonitril / 0.1% Ameisensäure

**Gradient:**

| Zeit in min | %A | %B | Flussrate in mL/min |
|---|---|---|---|
| 0.0 | 90.0 | 10.0 | 1.6 |
| 2.7 | 10.0 | 90.0 | 1.6 |
| 3.0 | 10.0 | 90.0 | 1.6 |
| 3.3 | 95.0 | 5.0 | 1.6 |

Methode 13: Säule: Merck Cromolith SpeedROD RP-18e, 4.6 x 50 mm
Fließmittel A: Wasser / 0.1 % Ameisensäure
Fließmittel B: Acetonitril / 0.1% Ameisensäure

**Gradient:**

| Zeit in min | %A | %B | Flussrate in mL/min |
|---|---|---|---|
| 0.0 | 90.0 | 10.0 | 1.5 |
| 4.5 | 10.0 | 90.0 | 1.5 |
| 5.0 | 10.0 | 90.0 | 1.5 |
| 5.5 | 95.0 | 5.0 | 1.5 |

Methode 14: Säule: Zorbax Stable Bond C18, 3.5 µM, 4.6 x 75 mm
Fließmittel A: Wasser / 0.15% Ameisensäure
Fließmittel B: Acetonitril

**Gradient:**

| Zeit in min | %A | %B | Flussrate in mL/min |
|---|---|---|---|
| 0.0 | 95.0 | 5.0 | 1.6 |
| 2.0 | 10.0 | 90.0 | 1.6 |
| 5.0 | 10.0 | 90.0 | 1.6 |
| 5.5 | 90.0 | 10.0 | 1.6 |

Folgende präparative Methoden wurden für die Umkehrphasen-Chromatographie verwendet:
Methode 1: Säule: Atlantis C18, 5 µM, 100 x 30 mm
Detektion: 210 - 500 nm
Fließmittel A: Wasser / 0.1% Trifluoressigsäure
Fließmittel B: Acetonitril

**Gradient:**

| Zeit in min | %A | %B | Flussrate in mL/min |
|---|---|---|---|
| 0.0 | 95.0 | 5.0 | 5 |
| 0.5 | 95.0 | 5.0 | 50 |
| 8.0 | 5.0 | 95.0 | 50 |
| 9.0 | 5.0 | 95.0 | 50 |
| 9.5 | 95.0 | 5.0 | 50 |
| 10.0 | 95.0 | 5.0 | 50 |
| 10.1 | 95.0 | 5.0 | 5 |

Methode 2: Säule: Varian Pursuit 5 µM, 50 x 200 mm
Fließmittel A: Wasser / 0.1% Trifluoressigsäure
Fließmittel B: Acetonitril / 0.1% Trifluoressigsäure

**Gradient:**

| Zeit in min | %A | % B | Flussrate in mL/min |
|---|---|---|---|
| 0.0 | 95.0 | 5.0 | 180 |
| 1.15 | 95.0 | 5.0 | 180 |
| 12.4 | 2.0 | 98.0 | 180 |
| 14.0 | 2.0 | 98.0 | 180 |
| 15.3 | 95.0 | 5.0 | 180 |
| 15.3 | 95.0 | 5.5 | 180 |

Methode 3: Säule: YMC-Pack ODS-AQ 5 µM, 30 x 100 mm
Fließmittel A: Wasser / 0.15% Ameisensäure
Fließmittel B: Acetonitril

**Gradient:**

| Zeit in min | %A | %B | Flussrate in mL/min |
|---|---|---|---|
| 0.0 | 95.0 | 5.0 | 50 |
| 2.0 | 95.0 | 5.0 | 50 |
| 6.0 | 10.0 | 90.0 | 50 |
| 12.0 | 10.0 | 90.0 | 50 |
| 13 | 90.0 | 10.0 | 50 |

### Herstellung der Ausgangsverbindungen:

Die Verbindungen der allgemeinen Formel I können aus den folgenden Intermediaten **A**, **B** und **C** hergestellt werden:

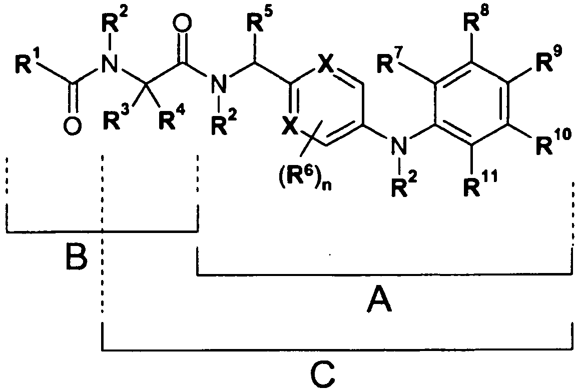

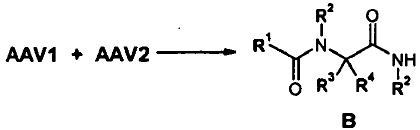

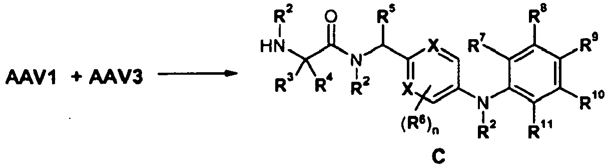

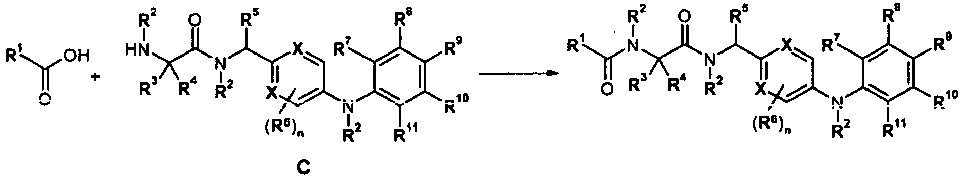

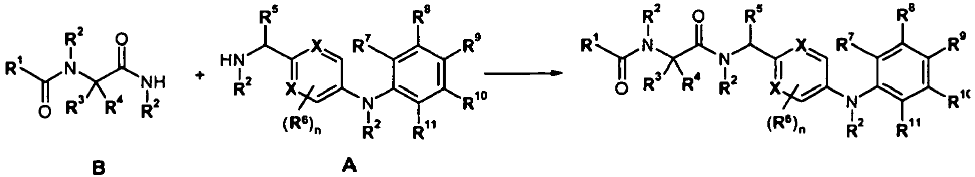

### AAV 1:Amid-Kupplung

Eine Lösung der Carbonsäure-Komponente (1 Mol-Äquivalent), Triethylamin (2.5 Mol-Äquivatente) und TBTU (1.1 Mol-Äquivalente) in THF wurde 30 Minuten bei Raumtemperatur gerührt. Dann wurde die Amin-Komponente (1.1 Mol-Äquivalent als Hydrochlorid) hinzugegeben und über Nacht weiter gerührt. Danach wurde das Gemisch eingedampft, mit Wasser versetzt, mit verdünnter Kaliumcarbonat-Lösung alkalisch gestellt und mit Essigsäureethylester extrahiert. Mittels Säulenchromatographie (entweder Kieselgel oder Reversed Phase Chromatographie) wurde das Produkt isoliert und gereinigt.

### AAV 2: Ester-Hydrolyse

Zu einer Lösung des Esters (1 Mol-Äquivalent) in Methanol wurde 2N Natronlauge (2 Mol-Äquivalente) gegeben und das Gemisch 1 bis 5 Stunden bei Raumtemperatur gerührt. Danach wurde mit Essigsäure angesäuert und das Gemisch im Vakuum bis zur Trockne eingedampft. Das so erhaltene Rohprodukt wurde üblicherweise durch Säulenchromatographie an Kieselgel gereinigt.

### AAV 3:Abspaltung der tert-Butyloxycarbonyl-Schutzgruppe

Eine Lösung der *tert*-Butoxycarbonyl-Aminoverbindung (1 Mol-Äquivalent) in Dichlormethan wurde mit Trifluoressigsäure (3 bis 10 Mol-Äquivalente) versetzt und bei Raumtemperatur gerührt, bis die Schutzgruppe vollständig abgespalten war. Das Reaktionsgemisch wurde dann bis zur Trockne eingedampft und das so erhaltene Rohprodukt chromatographisch gereinigt.

### AAV 4:Herstellung der Intermediate A

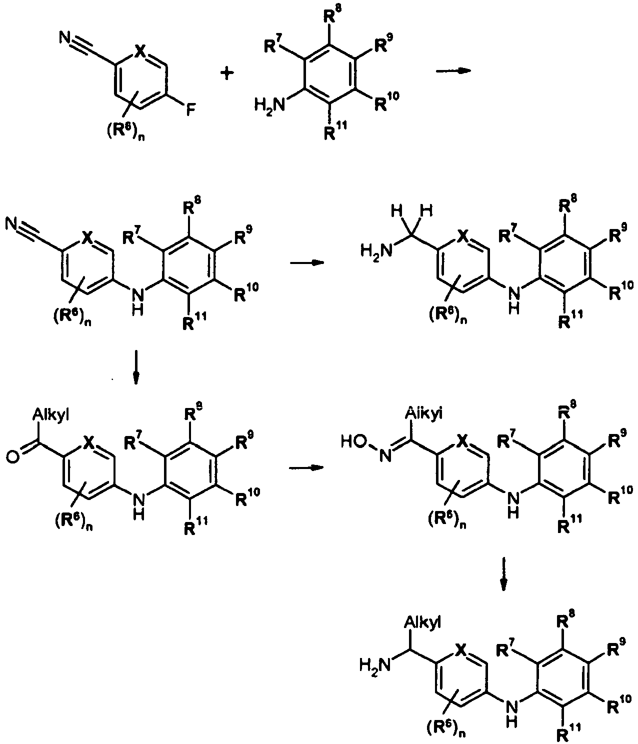

Eine Lösung der Anilin-Komponente (1 Mol-Äquivalent) und einer starken Base wie z.B. Kalium-*tert*-butylat (1 Mol-Äquivalent) in DMSO wurde eine Stunde bei Raumtemperatur gerührt, dann mit der 4-Fluor-benzonitril-Komponente (1 Mol-Äquivalent) versetzt und weiter über Nacht bei ca. 80°C gerührt. Zur Aufarbeitung wurde das Gemisch über Alox filtriert und im Vakuum zur Trockne eingedampft.

Die Nitrilgruppe des so erhaltenen Diphenylamin-Zwischenprodukts wurde anschließend unter Zusatz von Raney-Nickel bei 55°C und 3 bar Wasserstoffüberdruck zur Aminomethylgruppe reduziert und das erhaltene Produkt chromatographisch gereinigt. Zur Herstellung der Intermediate **A** mit alpha-Alkylbenzylgruppe (z.B. A1, A4, A5) wurde das Nitril-Derivat (1 Mol-Äquivalent) in Diethylether gelöst und bei 0 bis 5°C unter Rühren zu einer Lösung von Alkylmagnesiumbromid (4 Mol-Äquivalente) in Diethylether getropft und anschließend noch ca. 30 Minuten nachgerührt. Das Reaktionsgemisch wurde dann bei -5°C in 1 M Salzsäure eingerührt und das so erhaltene Alkylketon auf übliche Weise chromatographisch isoliert und gereingt.

Eine Lösung des so erhaltenen Ketons (1 Mol-Äquivalent) in Acetonitril wurde mit Triethylamin (2 Mol-Äquivalente) und Hydrxylamin-Hydrochlorid (1.3 Mol-Äquivalente) versetzt und 4 Stunden unter Rückfluß erhitzt. Dann wurde Wasser hinzugefügt und mit Dichlormethan extrahiert. Aus der organischen Phase wurde nach üblichem Verfahren das enstandene Oxim isoliert und gereinigt.

Eine Lösung des Oxims (1 Mol-Äquivalent) in Methanol wurde mit methanolischer Salzsäure (6.6 Mol-Äquivalente) versetzt. Nach Zugabe von Zinkstaub (1.4 Mol-Äquivalente) wurde 3 Stunden unter Rühren zum Rückfluß erhitzt. Nach dem Abkühlen wurde das Gemisch mit Wasser versetzt und mit Dichlormethan extrahiert. Wenn notwendig, wurde das so erhaltene Amin chromatographisch gereinigt.

Eine weitere Möglichkeit, das Oxim zum entsprechenden Amin zu reduzieren, besteht in der katalytischen Hydrierung. Dazu wurde das Oxim in methanolischer Ammoniak-Lösung nach Zusatz von Raney-Nickel bei 50°C und einem Wasserstoffüberdruck von 50 psi bis zur vollständigen Wasserstoff-Aufnahme hydriert. Wenn notwendig, wurde das so erhaltene Amin chromatographisch gereinigt.

### Herstellung der Intermediate A

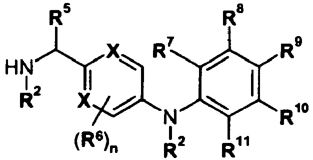

Die folgenden Intermediate A1 bis A31 wurden nach der allgemeinen Arbeitsvorschrift AAV4 hergestellt:
Intermediat A1: [6-(1-Amino-ethyl)-5-fluor-pyridin-3-yl]-(4-chlor-2-trifluormethylphenyl)-amin 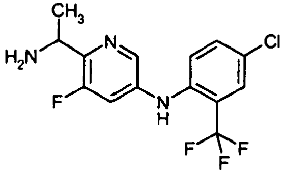 HPLC: Rₜ = 1.98 Minuten (Methode 2)
   Massenspektrum (ESI): [M+H]+ = 334
Intermediat A2: (6-Aminomethyl-pyridin-3-yl)-(4-isopropyl-2-trifluormethyl-phenyl)-amin 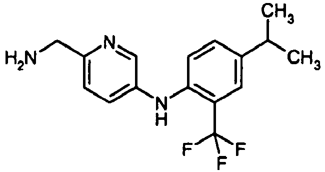 HPLC: Rₜ = 1.95 Minuten (Methode 2)
   Massenspektrum (ESI): [M+H]+ = 310
Intermediat A3: (6-Aminomethyl-pyridin-3-yl)-(4-chlor-2-trifluormethyl-phenyl)-amin 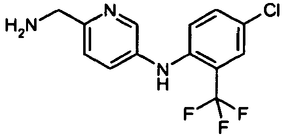 HPLC: Rₜ = 1.74 Minuten (Methode 13)
   Massenspektrum (ESI): [M+H]+ = 302
Intermediat A4: 2-[6-(1-Amino-ethyl)-5-fluor-pyridin-3-ylamino]-5-fluor-benzonitril 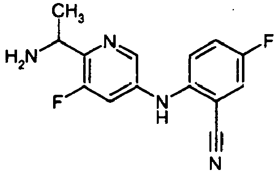 HPLC: Rₜ = 1.39 Minuten (Methode 2)
   Massenspektrum (ESI): [M+H]+ = 275; [M-H]- = 273
Intermediat A5: [6-(1-Amino-ethyl)-5-fluor-pyridin-3-yl]-(4-brom-2-trifluormethylphenyl)-amin 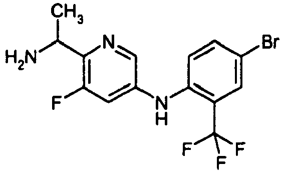 HPLC: Rₜ = 1.92 Minuten (Methode 2)
Intermediat A6: (4-Aminomethyl-phenyl)-(4-fluor-2-trifluormethyl-phenyl)-amin 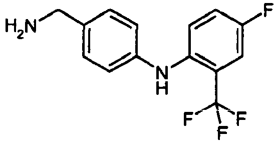 Massenspektrum (ESI): [M+H]+ = 285
   Dünnschicht-Chromatogramm (Kieselgel, CH₂Cl₂/Ethanol 19:1): R_{f} = 0.16
Intermediat A7: (6-Aminomethyl-pyridin-3-yl)-(4-fluor-2-trifluormethyl-phenyl)-amin 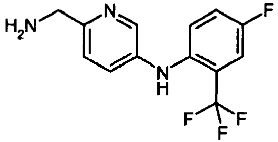 HPLC: Rₜ = 2.06 Minuten (Methode 3)
   Massenspektrum (ESI): [M+H]+ = 286; [M-H]- = 284
Intermediat A8: (6-Aminomethyl-5-chlor-pyridin-3-yl)-(4-fluor-2-trifluormethylphenyl)-amin 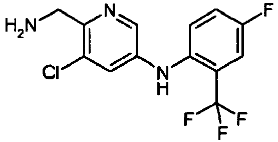 Massenspektrum (ESI): [M+H]+ = 320; [M-H]- = 320
Intermediat A9: (6-Aminomethyl-pyridin-3-yl)-(4-brom-2-trifluormethyl-phenyl)-amin 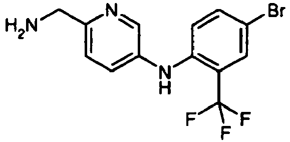 HPLC: Rₜ = 1.97 Minuten (Methode 2)
   Massenspektrum (ESI): [M+H]+ = 346
Intermediat A10: (6-Aminomethyl-5-chlor-pyridin-3-yl)-(2-trifluormethyl-phenyl)-amin 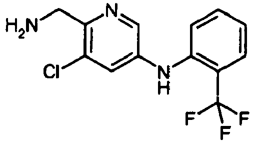 Dünnschicht-Chromatogramm (Kieselgel, CH₂Cl₂/Ethanol/NH₄OH 9:1:0.1): R_{f} = 0.52
Intermediat A11: (4-Aminomethyl-3-fluor-phenyl)-(4-fluor-2-trifluormethyl-phenyl)-amin 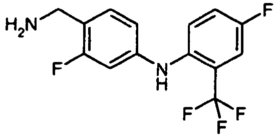 Massenspektrum (ESI): [M+H]+ = 303
   Dünnschicht-Chromatogramm (Kieselgel, CH₂Cl₂/Ethanol 19:1): R_{f} = 0.08
Intermediat A12: (4-Aminomethyl-3-fluor-phenyl)-(2-trifluormethyl-phenyl)-amin 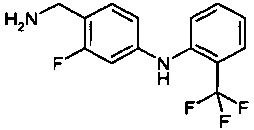 Massenspektrum (ESI): [M-H]- = 283
   Dünnschicht-Chromatogramm (Kieselgel, CH₂Cl₂/Ethanol 19:1): R_{f} = 0.09
Intermediat A13: (6-Aminomethyl-5-chlor-pyridin-3-yl)-(2-fluor-6-trifluormethylphenyl)-amin 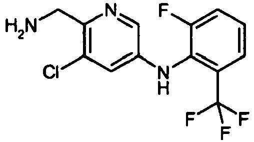 Massenspektrum (ESI): [M+H]+ = 320
   Dünnschicht-Chromatogramm (Kieselgel, CH₂Cl₂/Ethanol/NH₄OH 9:1:0.1): R_{f} = 0.58
Intermediat A14: (6-Aminomethyl-5-fluor-pyridin-3-yl)-(4-fluor-2-trifluormethyl-phenyl)-amin 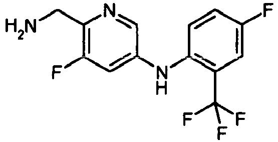 Massenspektrum (ESI): [M+H]+ = 304
   Dünnschicht-Chromatogramm (Kieselgel, CH₂Cl₂/Ethanol/NH₄OH 9:1:0.1): R_{f} = 0.56
Intermediat A15: (6-Aminomethyl-5-fluor-pyridin-3-yl)-(4-chlor-2-trifluormethylphenyl)-amin 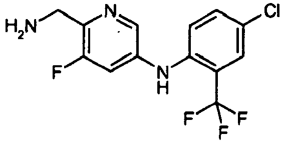 Massenspektrum (ESI): [M+H]+ = 320; [M-H]- = 318
Intermediat A16: (6-Aminomethyl-5-fluor-pyridin-3-yl)-(2-fluor-6-trifluormethyl-phenyl)-amin 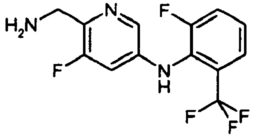 HPLC: Rₜ = 1.44 Minuten (Methode 2)
Intermediat A17: (4-Aminomethyl-phenyl)-(2-trifluormethyl-phenyl)-amin 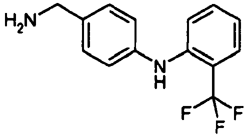 HPLC: Rₜ = 1.36 Minuten (Methode 1)
   Massenspektrum (ESI): [M+H-NH₃]+ = 250
Intermediat A18: (6-Aminomethyl-5-chlor-pyridin-3-yl)-(4-chlor-2-trifluormethylphenyl)-amin 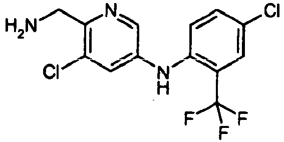 HPLC: Rₜ = 2.05 Minuten (Methode 2)
Intermediat A19: (4-Aminomethyl-3-fluor-phenyl)-(6-fluor-2-trifluormethyl-phenyl)-amin 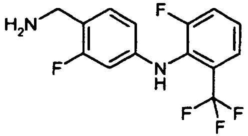 Dünnschicht-Chromatogramm (Kieselgel, CH₂Cl₂/Ethanol 9:1): R_{f} = 0.18
Intermediat A20: 2-(6-Aminomethyl-5-fluor-pyridin-3-ylamino)-benzonitril 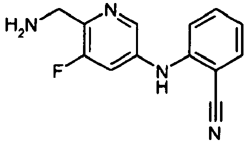 HPLC: Rₜ = 1.14 Minuten (Methode 2)
   Massenspektrum (ESI): [M+H]+ = 243
Intermediat A21: (6-Aminomethyl-5-methyl-pyridin-3-yl)-(4-fluor-2-trifluormethylphenyl)-amin 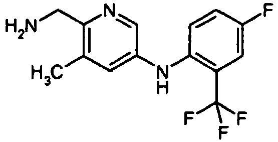 HPLC: Rₜ = 1.79 Minuten (Methode 2)
   Massenspektrum (ESI): [M+H]+ = 300
Intermediat A22: (4-Aminomethyl-phenyl)-(4-chlor-2-trifluormethyl-phenyl)-amin 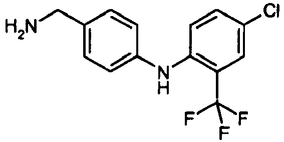 Massenspektrum (ESI): [M+H-NH₃]+ = 284/286
Intermediat A23: (6-Aminomethyl-5-fluor-pyridin-3-yl)-(2-trifluormethyl-phenyl)-amin 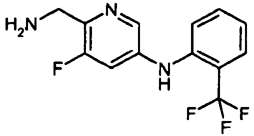 Massenspektrum (ESI): [M+H]+ = 286
Intermediat A24: (6-Aminomethyl-pyridin-3-yl)-(4-brom-2-chlor-6-fluorphenyl)-amin 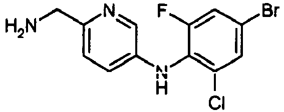 HPLC: Rₜ = 1.87 Minuten (Methode 2)
   Massenspektrum (ESI): [M+H]+ = 330
Intermediat A25: (6-Aminomethyl-pyridin-3-yl)-(2-brom-6-fluor-phenyl)-amin 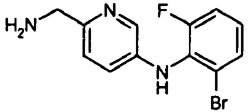 HPLC: Rₜ = 2.18 Minuten (Methode 2)
   Massenspektrum (ESI): [M+H]+ = 296
Intermediat A26: (6-Aminomethyl-5-methyl-pyridin-3-yl)-(4-chlor-2-trifluormethylphenyl)-amin 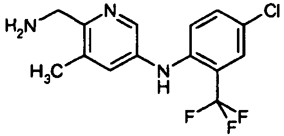 HPLC: Rₜ = 2.33 Minuten (Methode 2)
intermediat A27: (6-Aminomethyl-5-methyl-pyridin-3-yl)-(2-trifluormethyl-phenyl)-amin 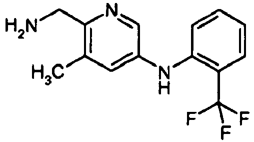 Massenspektrum (ESI): [M+H]+ = 282
Intermediat A28: (6-Aminomethyl-pyridin-3-yl)-(4-chlor-2-difluormethyl-phenyl)-amin 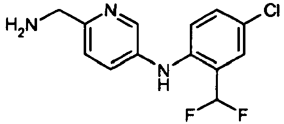 HPLC: Rₜ = 1.66 Minuten (Methode 2)
   Massenspektrum (ESI): [M+H]+ = 284
Intermediat A29: (4-Aminomethyl-3-fluor-phenyl)-(4-chlor-2-trifluormethyl-phenyl)-amin 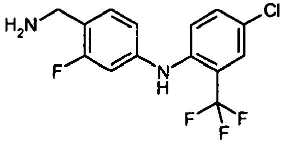 HPLC: Rₜ = 1.83 Minuten (Methode 2)
Intermediat A30: 2-(4-Aminomethyl-3-fluor-phenylamino)-benzonitril 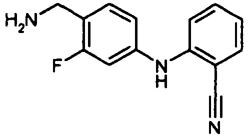 HPLC: Rₜ = 1.38 Minuten (Methode 2)
Intermediat A31: (4-Aminomethyl-phenyl)-(4-brom-2-trifluormethyl-phenyl)-amin 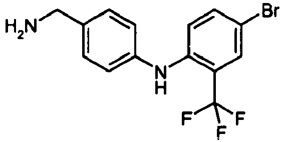 HPLC: Rₜ = 1.81 Minuten (Methode 2)

### Herstellung der Intermediate B

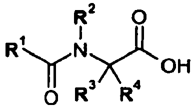

Die folgenden Intermediate B1 bis B11 wurden durch Amid-Kupplung nach der allgemeinen Arbeitsvorschrift AAV1 und anschließende Ester-Verseifung nach der allgemeinen Arbeitsvorschrift AAV2 hergestellt:
Intermediat B1: 1-[(Pyrimidin-5-carbonyl)-amino]-cyclopropancarbonsäure 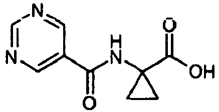 Massenspektrum (ESI): [M+H]+ = 208; [M-H]- = 206
Intermediat B2: (*S*)-3-[(5-Amino-pyridin-3-carbonyl)-amino]-tetrahydrofuran-3-carbonsäure 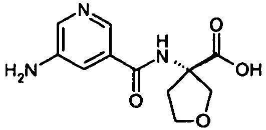 HPLC: Rₜ = 0.85 Minuten (Methode 7)
   Massenspektrum (ESI): [M+H]+ = 252
Intermediat B3: (*S*)-3-[(Pyrimidin-5-carbonyl)-amino]-tetrahydrofuran-3-carbonsäure 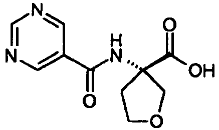 Massenspektrum (ESI): [M+H]+ = 238; [M-H]- = 236
Intermediat B4: 1-[(5-Amino-pyridin-3-carbonyl)-amino]-cyclopropancarbonsäure 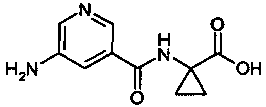 Massenspektrum (ESI): [M+H]+ = 222
Intermediat B5: (*S*)-3-[(3H-Imidazo[4,5-b]pyridin-6-carbonyl)-amino]-tetrahydrofuran-3-carbonsäure 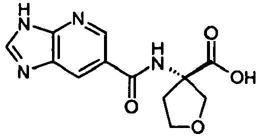 HPLC: Rₜ = 1.49 Minuten (Methode 3)
   Massenspektrum (ESI): [M-H]- = 275
Intermediat B6: (*S*)-3-[(2-Methylamino-pyrimidin-5-carbonyl)-amino]-tetrahydrofuran-3-carbonsäure 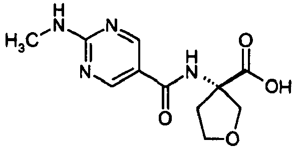 HPLC: Rₜ = 0.47 Minuten (Methode 2)
   Massenspektrum (ESI): [M+H]+ = 267
Intermediat B7: (*S*)-3-[(2-Methyl-pyrimidin-5-carbonyl)-amino]-tetrahydrofuran-3-carbonsäure 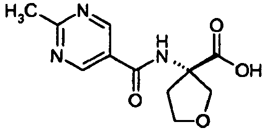 HPLC: Rₜ = 0.43 Minuten (Methode 2)
   Massenspektrum (ESI): [M+H]+ = 252; [M-H]- = 250
Intermediat B8: (*S*)-3-[(5-Hydroxy-pyridin-3-carbonyl)-amino]-tetrahydrofuran-3-carbonsäure 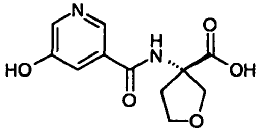 HPLC: Rₜ = 0.48 Minuten (Methode 2)
   Massenspektrum (ESI): [M+H]+ = 253
Intermediat B9: (*S*)-3-[(6-Amino-pyridin-3-carbonyl)-amino]-tetrahydrofuran-3-carbonsäure 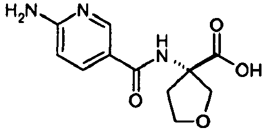 HPLC: Rₜ = 0.33 Minuten (Methode 2)
   Massenspektrum (ESI): [M+H]+ = 252; [M-H]- = 250
Intermediat B10: (*S*)-3-[(6-Oxo-1,6-dihydro-pyridazin-4-carbonyl)-amino]-tetrahydrofuran-3-carbonsäure 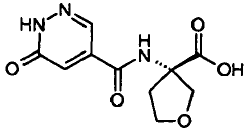 HPLC: Rₜ = 0.33 Minuten (Methode 2)
   Massenspektrum (ESI): [M+H]+ = 254
Intermediat B11: 1-[(2-Methyl-pyrimidin-5-carbonyl)-amino]-cyclopropancarbonsäure 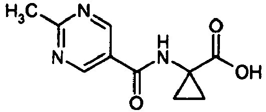 Massenspektrum (ESI): [M+H]+ = 222; [M-H]- = 220

Analog können die folgenden Intermediate B12 bis B15 hergestellt werden:
Internmediat B12: 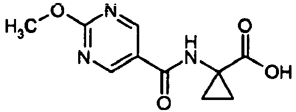
Intermediat B13: 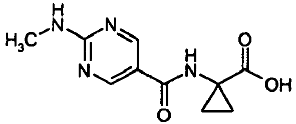
Intermediat B14: 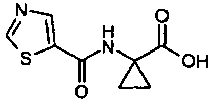
Intermediat B15:

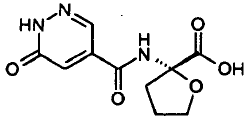

### Herstellung der Intermediate C

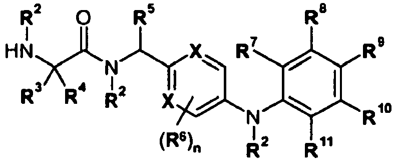

Die folgenden Intermediate C1 bis C22 wurden durch Amid-Kupplung nach der allgemeinen Arbeitsvorschrift AAV1 und anschließender Abspaltung der tert-Butyloxycarbonyl-Schutzgruppe nach der allgemeinen Arbeitsvorschrift AAV3 hergestellt:
Intermediat C1: 1-Amino-cyclopropancarbonsäure-[5-(4-chlor-2-trifluormethylphenylamino)-pyridin-2-ylmethyl]-amid 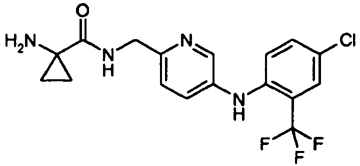 HPLC: Rₜ = 1.55 Minuten (Methode 13)
   Massenspektrum (ESI): [M-H]- = 383
Intermediat C2: 1-Amino-cyclopropancarbonsäure-[5-(4-fluor-2-trifluormethylphenylamino)-pyridin-2-ylmethyl]-amid 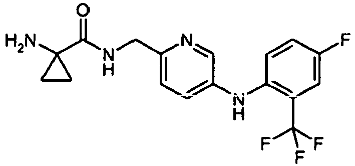 HPLC: Rₜ = 2.33 Minuten (Methode 7)
   Massenspektrum (ESI): [M+H]+ = 369; [M-H]- = 367
Intermediat C3: (*S*)-3-Amino-tetrahydrofuran-3-carbonsäure-[3-chlor-5-(4-fluor-2-trifluormethyl-phenylamino)-pyridin-2-ylmethyl]-amid 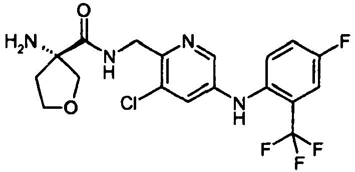 Massenspektrum (ESI): [M+H]+ = 433
Intermediat C4: 1-Amino-cyclopropancarbonsäure-[5-(4-brom-2-trifluormethylphenylamino)-pyridin-2-ylmethyl]-amid 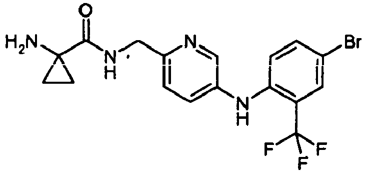 HPLC: Rₜ = 1.65 Minuten (Methode 2)
   Massenspektrum (ESI): [M+H]+ = 429
Intermediat C5: (*S*)-3-Amino-tetrahydrofuran-3-carbonsäure-[3-chlor-5-(2-fluor-6-trifluormethyl-phenylamino)-pyridin-2-ylmethyl]-amid 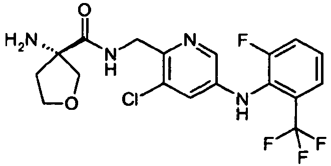 Massenspektrum (ESI): [M+H]+ = 433
Intermediat C6: (*S*)-3-Amino-tetrahydrofuran-3-carbonsäure-[3-fluor-5-(4-fluor-2-trifluormethyl-phenylamino)-pyridin-2-ylmethyl]-amid 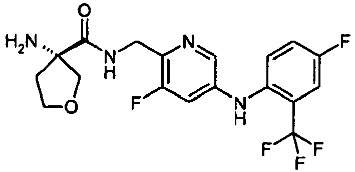 Massenspektrum (ESI): [M+H]+ = 417
Intermediat C7: 1-Amino-cyclopropancarbonsäure-[3-fluor-5-(2-fluor-6-trifluormethylphenylamino)-pyridin-2-ylmethyl]-amid 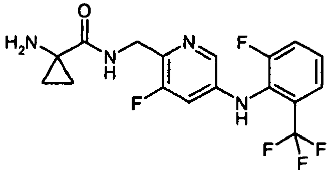 HPLC: Rₜ = 1.50 Minuten (Methode 2)
Intermediat C8: 3-Amino-oxetan-3-carbonsäure-4-(2-trifluormethyl-phenylamino)-benzylamid

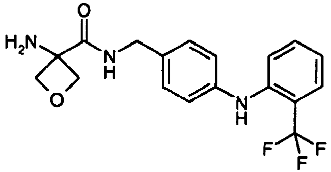

### (a) 3-Dibenzylamino-oxetan-3-carbonitril

Eine Lösung von 3-Oxetanon (908 mg, 12.6 mMol), Dibenzylamin (6.08 mL, 31.6 mMol) und Trimethylsilylcyanid (2.00 mL, 15.8 mMol) in 20 mL konzentrierter Esigsäure wurde über Nacht bei 60°C gerührt. Nach dem Abkühlen wurde mit konzentriertem Ammoniak auf pH 10 eingestellt und die Lösung mit Chloroform extrahiert. Nach dem Eindampfen erhielt man das Rohprodukt, das durch Chromatographie über Kieselgel gereinigt wurde.
C₁₈H₁₈N₂O (278.35)
Massenspektrum (ESI): [M+H]+ = 279

### (b) 3-Dibenzylamino-oxetan-3-carbonsäure

Eine Lösung von 3-Dibenzylamino-oxetan-3-carbonitril (370 mg, 1.33 mmol) und 5 mL 4M Natronlauge in 20 mL Ethanol wurde über Nacht zum Rückfluß erhitzt, anschließend mit 1 M Salzsäure neutralisiert und zur Trockne eingedampft. Das so erhaltene Rohprodukt wurde chromatographisch gereinigt.
C₁₈H₁₉NO₃ (297.35)
Massenspektrum (ESI): [M+H]+ = 298

### (c) 3-Dibenzylamino-oxetan-3-carbonsäure-4-(2-trifluormethyl-phenylamino)-benzylamid

Hergestellt aus 3-Dibenzylamino-oxetan-3-carbonsäure und 4-(2-Trifluormethyl-phenylamino)-benzylamin durch Amidkupplung nach der allgemeinnen Arbeitsvorschrift AAV1.
C₃₂H₃₀F₃N₃O₂ (545.59)
Massenspektrum (ESI): [M+H]+ = 546

### (d) 3-Amino-oxetan-3-carbonsäure-4-(2-trifluormethyl-phenylamino)-benzylamid

3-Dibenzylamino-oxetan-3-carbonsäure-4-(2-trifluormethyl-phenylamino)-benzylamid (32.0 mg, 0.059 mmol) wurde in 10 mL Methanol gelöst, mit 20 mg Pd/Kohle (10%) versetzt und bei Raumtemperatur und 3 bar Wasserstoffdruck debenzyliert.
C₁₈H₁₈F₃N₃O₂ (365.35)
HPLC: Rₜ = 1.93 Minuten (Methode 5)
Massenspektrum (ESI): [M+H]+ = 366
Intermediat C9: (*S*)-3-Amino-tetrahydrofuran-3-carbosäure-2-fluor-4-(4-fluor-2-trifluormethyl-phenylamino)-benzylamid 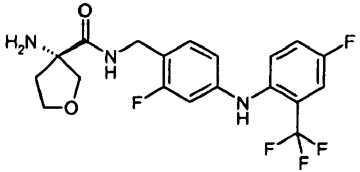 Massenspektrum (ESI): [M+H]+ = 416
   Intermediat C10: 1-Amino-cyclopropancarbonsäure-2-fluor-4-(4-fluor-2-trifluormethylphenylamino)-benzylamid 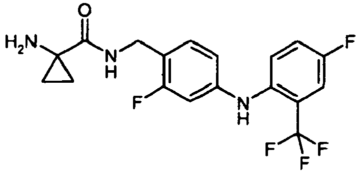 Massenspektrum (ESI): [M+H]+ = 386
Intermediat C11: (*S*)-3-Amino-tetrahydrofuran-3-carbonsäure-2-fluor-4-(2-fluor-6-trifluormethyl-phenylamino)-benzylamid 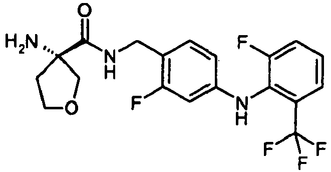 Massenspektrum (ESI): [M+H]+ = 416
Intermediat C12: 1-Amino-cyclopropancarbonsäure-2-fluor-4-(2-trifluormethylphenylamino)benzylamid 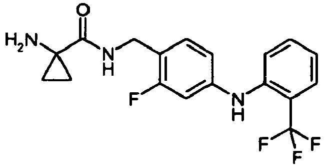 Massenspektrum (ESI): [M+H]+ = 368
Intermediat C13: 1-Amino-cyclopropancarbonsäure-[3-fluor-5-(4-fluor-2-trifluormethylphenylamino)-pyridin-2-ylmethyl]-amid 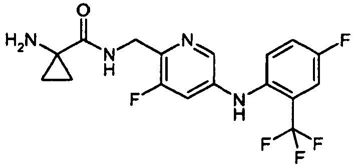 Massenspektrum (ESI): [M+H]+ = 387
Intermediat C14: (*S*)-3-Amino-tetrahydrofuran-3-carbonsäure-4-(4-fluor-2-trifluormethyl-phenylamino)-benzylamid 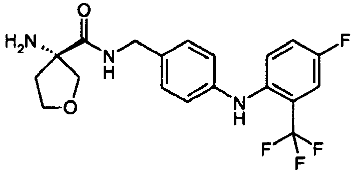 HPLC: Rₜ = 1.99 Minuten (Methode 2)
   Massenspektrum (ESI): [M+H]+ = 398
Intermediat C15: 1-Amino-cyclopropancarbonsäure-[3-chlor-5-(2-trifluormethylphenylamino)-pyridin-2-ylmethyl]-amid 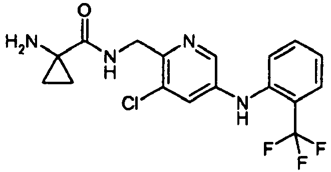 Massenspektrum (ESI): [M+H]+ = 385
Intermediat C16: (*S*)-3-Amino-tetrahydrofuran-3-carbonsäure-[5-(4-fluor-2-trifluormethyl-phenylamino)-pyridin-2-ylmethyl]-amid 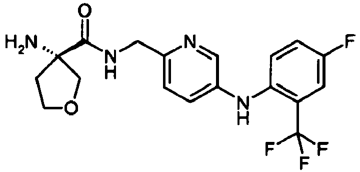 HPLC: Rₜ = 1.34 Minuten (Methode 2)
   Massenspektrum (ESI): [M+H]+ = 399
Intermediat C17: (*S*)-3-Amino-tetrahydrofuran-3-carbonsäure-[5-(4-chlor-2-trifluormethyl-phenylamino)-3-fluor-pyridin-2-ylmethyl]-amid 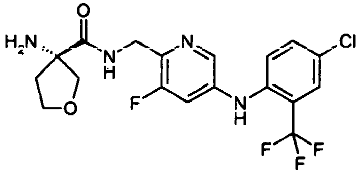 HPLC: Rₜ = 2.35 Minuten (Methode 2)
Intermediat C18: (*S*)-3-Amino-tetrahydrofuran-3-carbonsäure-4-(4-chlor-2-trifluormethyl-phenylamino)-benzylamid 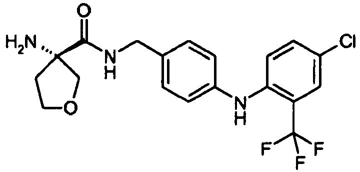 HPLC: Rₜ = 2.41 Minuten (Methode 2)
Intermediat C19: 1-Amino-cyclopropancarbonsäure-[5-(4-fluor-2-trifluormethylphenylamino)-3-methyl-pyridin-2-ylmethyl]-amid 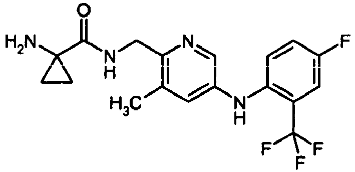 HPLC: Rₜ = 1.24 Minuten (Methode 2)
   Massenspektrum (ESI): [M+H]+ = 383
Intermediat C20: 1-Amino-cyclopropancarbonsäure-[3-methyl-5-(2-trifluormethylphenylamino)-pyridin-2-ylmethyl]-amid 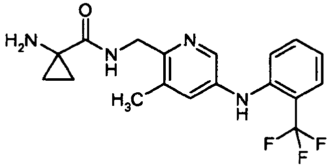 HPLC: Rₜ = 1.30 Minuten (Methode 2)
   Massenspektrum (ESI): [M+H]+ = 365
Intermediat C21: 1-Amino-cyclopropancarbonsäure-[5-(4-chlor-2-difluormethylphenylamino)-pyridin-2-ylmethyl]-amid 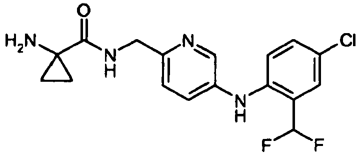 HPLC: Rₜ = 1.48 Minuten (Methode 2)
   Massenspektrum (ESI): [M+H]+ = 367
Intermediat C22: *(*S)-3-Amino-tetrahydrofuran-3-carbonsäure 2-fluor-4-(2-trifluormethyl-phenylamino)-benzylamid 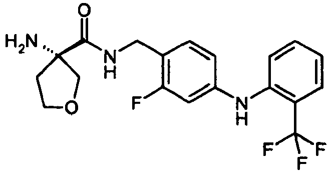 Massenspektrum (ESI): [M+H]+ = 398

Analog können die folgenden Intermediate C23 bis C25 hergestellt werden:
Intermediat C23: 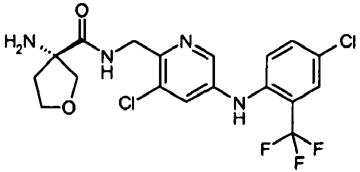
Intermediat C24: 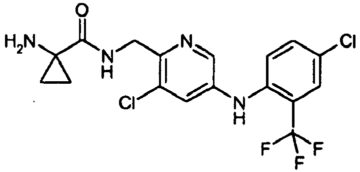
Intermediat C25:

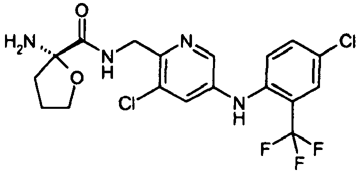

### Herstellung der Endverbindungen:

### Beispiel 1: Pyrimidin-5-carbonsäure N-(1-(4-(2,3-dichlorphenylamino)benzylcarbamoyl)cyclopropyl)amid

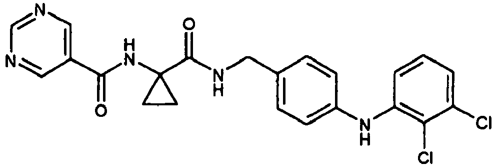

### 1a) 1-[(Pyrimidin-5-carbonyl)-amino]-cyclopropancarbonsäureethylester

Eine Lösung von 15.74 g (126.9 mmol) Pyrimidin-5-carbonsäure, 43.57 mL (312.6 mmol) Triethylamin und 44.61 g (138.9 mmol) TBTU in 460 mL THF wurde 30 Minuten bei Raumtemperatur gerührt. Dann wurden 9.11 g (127.3 mmol) 1-Amino-cyclopropancarbonsäureethylester-Hydrochlorid hinzugefügt und es wurde über Nacht weiter gerührt. Anschließend wurde das Gemisch eingedampft und der Rückstand mit 200 mL Wasser versetzt, mit verdünnter Kaliumcarbonat-Lösung alkalisch gestellt und mit Essigsäureethylester extrahiert. Mittels Säulenchromatographie (Kieselgel, Dichlormethan + 0-4% Methanol) wurde das Zwischenprodukt gereinigt.
Ausbeute: 95% der Theorie
C₁₁H₁₃N₃O₃ (235.24)
Rₜ= 1.23 min. Methode 1

### 1b) 1-[(Pyrimidin-5-carbonyl)-amino]-cyclopropancarbonsäure

Zu einer Lösung von 13.36 g (56.79 mmol) 1-[(Pyrimidin-5-carbonyl)-amino]-cyclopropancarbonsäureethylester in 240 mL Methanol wurden 28.39 mL einer 2N Natronlauge gegeben und das Gemisch wurde eine Stunde bei Raumtemperatur gerührt. Dann wurde mit konzentrierter Essigsäure angesäuert und im Vakuum bis zur Trockne eingedampft. Das so erhaltene Rohprodukt wurde durch Säulenchromatographie (Kieselgel, Dichlormethan + 5-30% 10%ige Essigsäure in Methanol) gereinigt.
Ausbeute: 100% der Theorie
C₉H₉N₃O₃ (207.19)
Rₜ= 1.23 min. Methode 1

### 1c) N-(4-Aminomethyl)phenyl)-2,3-dichloranilin-Trifluoracetat

Eine Lösung von 32 mg (0.2 mmol) 2,3-Dichloroanilin und 22 mg (0.2 mmol) Kalium-*tert-*butylat in 9 mL DMSO wurde eine Stunde bei Raumtemperatur gerührt, dann mit 24 mg (0.2 mmol) 4-Fluorbenzonitril versetzt und weiter über Nacht bei 80°C gerührt. Zur Aufarbeitung wurde das Reaktionsgemisch über Alox B filtriert, mit DMF gewaschen und im Vakuum bis zur Trockne eingedampft. Den Rückstand hydrierte man in 100 µL methanolischer Ammoniak-Lösung mit 20 mg Raney-Nickel als Katalysator bei 55°C und einem Wasserstoffdruck von 3 bar über 5 Stunden. Anschließend wurde der Katalysator abfiltriert, das Filtrat vom Lösungsmittel befreit und das Rohprodukt mittels HPLC (Methode 1) gereinigt.
Ausbeute: 47% der Theorie
C₁₃H₁₂Cl₂N₂ (267.15)

### 1d) Pyrimidin-5-carbonsäure N-(1-(4-(2,3-dichlorphenylamino)benzylcarbamoyl)-cyclopropyl)amid

Zu einer Lösung von 250 mg (1.2 mmol) 1-[(Pyrimidin-5-carbonyl)-amino]-cyclopropancarbonsäure (aus 1b) in 15 mL Tetrahydrofuran wurden 0.5 mL (3.6 mmol) Triethylamin, 433 mg (1.35 mmol) TBTU und 326 mg (1.2 mmol) N-(4-Aminomethyl)phenyl-2,3-Dichloroanilin-Trifluoracetat (aus 1c) gegeben. Das Gemisch wurde über Nacht bei Raumtemperatur gerührt, anschließend bis zur Trockne eingeengt und mittels HPLC (Methode 1) gereinigt.
Ausbeute: 16% der Theorie
C₂₂H₁₉Cl₂N₅O₃ (456.32)
Rₜ= 2.1 min. Methode 5

### Beispiel 2: Pyrimidin-5-carbonsäure-N-(1-(4-(2-chlorphenylamino)benzylcarbamoyl)-cyclopropyl)amid

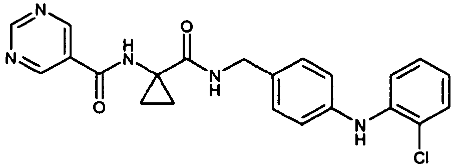

### 2a) N-(4-(Aminomethyl)phenyl)-2-chloroanilin

Analog Vorschrift (1c) wurde ausgehend von 2-Chloranilin, 4-Fluorbenzonitril und Raney-Nickel die Titelverbindung hergestellt.
C₁₃H₉ClN₂ (228.68)

### 2b) Pyrimidin-5-carbonsäure-N-(1-(4-(2-chlorphenylamino)benzylcarbamoyl)cyclopropyl)amid

Analog Vorschrift (1 d) wurde ausgehend von N-(4-(Aminomethyl)phenyl)-2-chloranilin (aus 2a) und 1-[(Pyrimidin-5-carbonyl)-amino]-cyclopropancarbonsäure (aus 1b) die Titelverbindung hergestellt.
C₂₂H₂₀ClN₅O₂ (421.88)
Rₜ= 2.13 min. Methode 6

### Beispiele 3: Pyrimidin-5-carbonsäure- N-(1-(4-(phenylamino)benzylcarbamoyl)cyclopropyl)amid

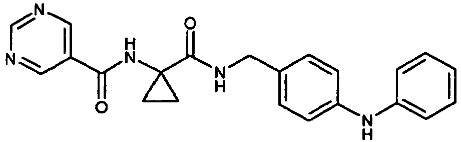

### 3a) 4-(Aminomethyl)-N-phenylanilin

Analog Vorschrift (1 c) wurde ausgehend von Anilin, 4-Fluorbenzonitril und Raney-Nickel die Titelverbindung hergestellt.
C₁₃H₁₄N₂ (199.26)

### 3b) Pyrimidin-5-carbonsäure- N-(1-(4-(phenylamino)benzylcarbamoyl)cyclopropyl)-amid

Analog Vorschrift (1d) wurde ausgehend von 4-(Aminomethyl)-N-phenylanilin (aus 3a) und 1-[(Pyrimidin-5-carbonyl)-amino]-cyclopropancarbonsäure (aus 1b) die Titelverbindung hergestellt.
C₂₂H₂₀ClN₅O₂ (421.88)
Rₜ= 1.82 min. Methode 5

### Beispiel 4: Pyrimidin-5-carbonsäure- N-(1-(4-(2-(trifluormethyl)phenylamino)benzylcarbamoyl)cyclopropyl)amid

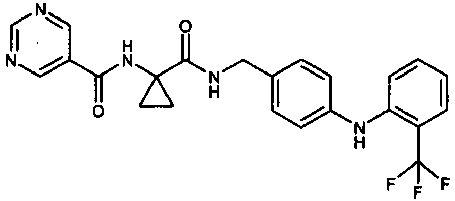

### 4a) N-(4-(Aminomethyl)phenyl)-2-(trifluormethyl)anilin

Analog Vorschrift (1 c) wurde ausgehend von 2-(Trifluormethyl)anilin, 4-Fluorbenzonitril und Raney-Nickel die Titelverbindung hergestellt.
C₁₄H₁₃F₃N₂ (266.26)

### 4b) Pyrimidin-5-carbonsäure- N-(1-(4-(2-(trifluormethyl)phenylamino)benzylcarbamoyl)cyclopropyl)amid

Analog Vorschrift (1d) wurde ausgehend von N-(4-(Aminomethyl)phenyl)-2-(trifluormethyl)anilin (aus 4a) und 1-[(Pyrimidin-5-carbonyl)-amino]-cyclopropancarbonsäure (aus 1b) die Titelverbindung hergestellt.
C₂₃H₂₀F₃N₅O₂ (455.43)
Rₜ= 2.27 min. Methode 6

### Beispiel 5: 5-Oxo-pyrrolidin-2-carbonsäure-{1-[4-(2-trifluormethyl-phenylamino)-benzylcarbamoyl]-cyclopropyl}-amid

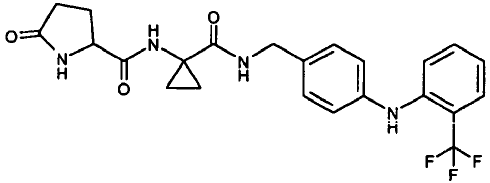

### 5a) 1-Amino-N-(4-(2-(trifluormethyl)phenylamino)benzyl)cyclopropancarboxamid

Eine Lösung von 376 mg (1.87 mmol) 1-(tert-Butoxycarbonylamino)cyclopropancarbonsäure in 20 mL DMF wurde mit 0.4 mL (2.85 mmol) Triethylamin und 600 mg (1.87 mmol) TBTU versetzt und 5 Minuten bei Raumtemperatur gerührt. Dann wurden 500 mg N-(4-(Aminomethyl)phenyl)-2-(trifluormethyl)anilin (aus 4a) zugegeben und man ließ über das Wochenende bei Raumtemperatur rühren. Das Gemisch wurde anschließend über Alox B filtriert, mit DMF:Methanol = 9:1 gewaschen und im Vakuum bis zur Trockne eingedampft. Der Rückstand wurde mit einem 1:1 Gemisch aus Dichlormethan und Trifluoressigsäure versetzt und eine Stunde bei Raumtemperatur gerührt. Das Reaktionsgemisch wurde im Vakuum bis zur Trockne eingedampft und das Rohprodukt mittels Säulenchromatographie (Kieselgel, Dichlormethan + 2-8% Methanol:Ammoniak = 9:1) gereinigt.
Ausbeute: 16% der Theorie
C₂₂H₁₉Cl₂N₅O₃ (456.32)

### 5b) 5-Oxo-pyrrolidin-2-carbonsäure-{1-[4-(2-trifluormethyl-phenylamino)-benzylcarbamoyl]-cyclopropyl}-amid

Analog Vorschrift (1d) wurde ausgehend von 1-Amino-N-(4-(2-(trifluormethyl)phenylamino)benzyl)cyclopropancarboxamid (aus 5a) und 5-Oxopyrrolidin-2-carbonsäure die Titelverbindung hergestellt
C₂₃H₂₃F₃N₄O₃ (460.46)
Rₜ = 1.89 min. Methode 5

Die nachfolgend aufgeführten Beispiele 6 bis 22 wurden analog zu Vorschrift (1 d) aus 1-Amino-N-(4-(2-(trifluormethyl)phenylamino)benzyl)cyclopropancarboxamid und den entsprechenden Säuren hergestellt:

### Beispiel 6: 1-(4-Dimethylamino-butyrylamino)-cyclopropancarbonsäure-4-(2-trifluormethyl-phenylamino)-benzylamid

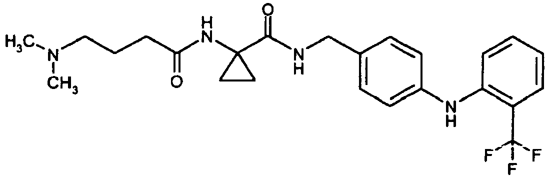

C₂₄H₂₉F₃N₄O₂ (462.5)
Rₜ = 1.67 min. Methode 5

### Beispiel 7: 1-(3,3,3-Trifluor-propionylamino)-cyclopropancarbonsäure-4-(2-trifluormethyl-phenylamino)-benzylamid

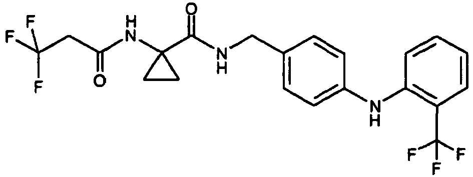

C₂₁H₁₉F₆N₃O₂ (459.4)
Rₜ= 2.21 min. Methode 5

### Beispiel 8: 1-(3-Dimethylamino-propionylamino)-cyclopropancarbonsäure-4-(2-trifluormethyl-phenylamino)-benzylamid

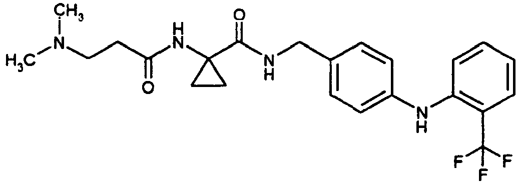

C₂₃H₂₇F₃N₄O₂ (448.5)
Rₜ = 1.67 min. Methode 5

### Beispiel 9: 2,4-Dihydroxy-pyrimidin-5-carbonsäure-{1-[4-(2-trifluormethyl-phenylamino)-benzylcarbamoyl]-cyclopropyl}-amid

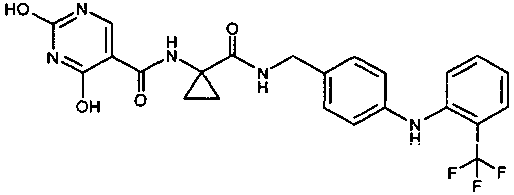

C₂₃H₂₀F₃N₅O₄ (487.4)
Rₜ = 1.93 min. Methode 5

### Beispiel 10: 1-(5-Dimethylamino-pentanoylamino)-cyclopropancarbonsäure-4-(2-trifluormethyl-phenylamino)-benzylamid

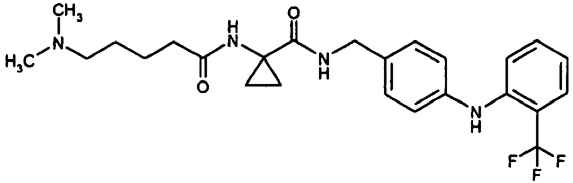

C₂₅H₃₁F₃N₄O₂ (476.5)
Rₜ= 1.69 min. Methode 5

### Beispiel 11: N-{1-[4-(2-Trifluormethyl-phenylamino)-benzylcarbamoyl]-cyclopropyl}-nikotinamid

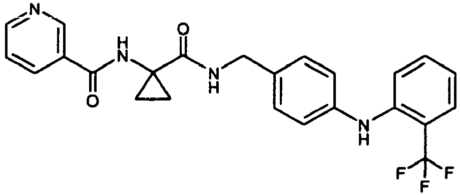

C₂₄H₂₁F₃N₄O₂ (454.4)
Rₜ= 1.79 min. Methode 5

### Beispiel 12: 1-(2-Dimethylamino-acetylamino)-cyclopropancarbonsäure-4-(2-trifluormethyl-phenylamino)-benzylamid

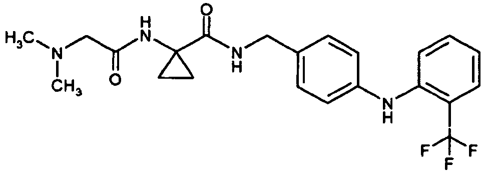

C₂₂H₂₅F₃N₄O₂ (434.5)
Rₜ = 1.68 min. Methode 5

### Beispiel 13: 1-Propionylamino-cyclopropancarbonsäure-4-(2-trifluormethyl-phenylamino)-benzylamid

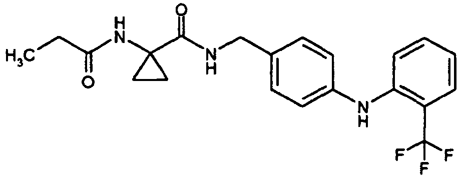

C₂₁H₂₂F₃N₃O₂ (405.4)
Rₜ = 2.09 min. Methode 5

### Beispiel 14: 1-(2-Methoxy-acetylamino)-cyclopropancarbonsäure-4-(2-trifluormethylphenylamino)-benzylamid

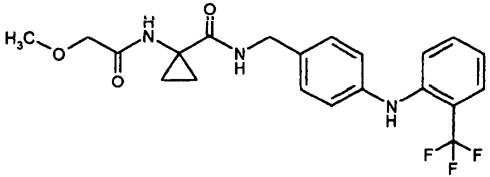

C₂₁H₂₂F₃N₃O₃ (421.4)
Rₜ = 2.07 min. Methode 5

### Beispiel 15: 1-(Cyclopropancarbonyl-amino)-cyclopropancarbonsäure-4-(2-trifluormethyl-phenylamino)-benzylamid

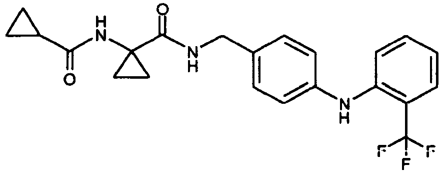

C₂₂H₂₂F₃N₃O₂ (417.4)
Rₜ = 2.13 min. Methode 5

### Beispiel 16: 1-Pentanoylamino-cyclopropancarbonsäure-4-(2-trifluormethyl-phenylamino)-benzylamid

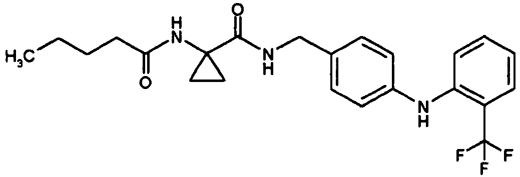

C₂₃H₂₆F₃N₃O₂ (433.5)
Rₜ = 2.24 min. Methode 5

### Beispiel 17: 1-Methyl-1H-imidazol-4-carbonsäure-{1-[4-(2-trifluormethyl-phenylamino)-benzylcarbamoyl]-cyclopropyl}-amid

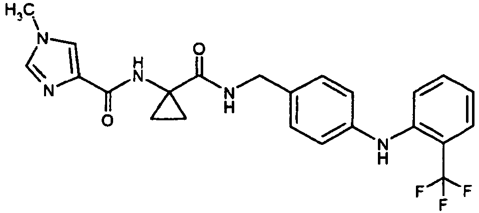

C₂₃H₂₂F₃N₅O₂ (457.5)
Rₜ = 1.73 min. Methode 5

### Beispiel 18: 1-Methyl-4H-imidazol-2-carbonsäure-{1-[4-(2-trifluormethyl-phenylamino)-benzylcarbamoyl]-cyclopropyl}-amid

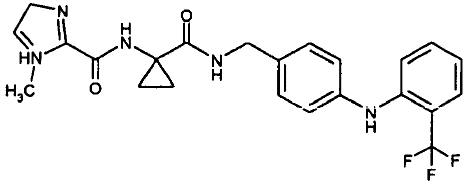

C₂₃H₂₂F₃N₅O₂ (457.5)
Rₜ = 1.88 min. Methode 5

### Beispiel 19: 1-(2-Cyclopropyl-acetylamino)-cyclopropancarbonsäure-4-(2-trifluormethylphenylamino)-benzylamid

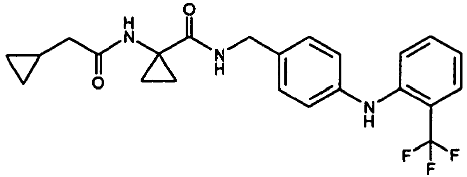

C₂₃H₂₄F₃N₃O₂ (431.5)
Rₜ = 2.18 min. Methode 5

### Beispiel 20: N-{1-[4-(2-Trifluormethyl-phenylamino)-benzylcarbamoyl]-cyclopropyl}-benzamid

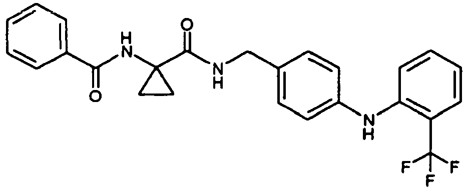

C₂₅H₂₂F₃N₃O₂ (453.5)
Rₜ = 2.26 min. Methode 5

### Beispiel 21: Pyridin-2-carbonsäure {1-[4-(2-trifluormethyl-phenylamino)-benzylcarbamoyl]-cyclopropyl}-amid

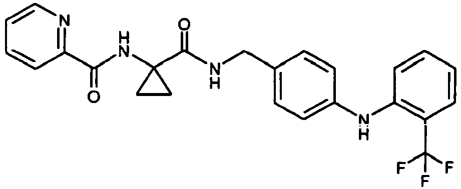

C₂₄H₂₁F₃N₄O₂ (454.4)
Rₜ = 2.20 min. Methode 5

### Beispiel 22: 1-Methyl-piperidin-4-carbonsäure {1-[4-(2-trifluormethyl-phenylamino)-benzylcarbamoyl]-cyclopropyl}-amid

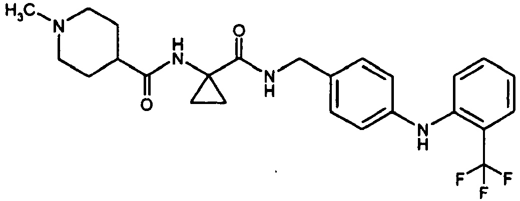

C₂₅H₂₉F₃N₄O₂ (474.5)
Rₜ = 1.68 min. Methode 5

### Beispiel 23: 1-(2,2,2-Trifluoracetamido)-N-(4-(2-(trifluormethyl)phenylamino)benzyl)-cyclopropancarboxamid

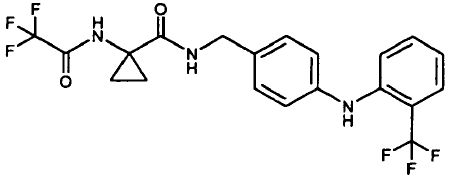

Analog Vorschrift (1d) wurde ausgehend von 1-Amino-N-(4-(2-(trifluormethyl)phenylamino)benzyl)cyclopropancarboxamid (aus 5a) und Trifluoressigsäure die Titelverbindung hergestellt.
C₂₀H₁₇F₆N₃O₂ (445.37)
Rₜ= 2.27 min. Methode 5

### Beispiel 24: N-(1-(4-(2-(Trifluormethyl)phenylamino)benzylcarbamoyl)cyclopropyl)-isoxazol-5-carboxamid

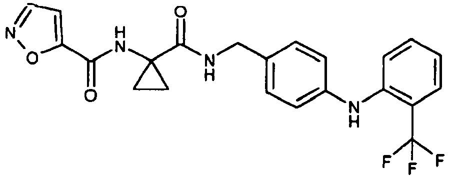

Zu einer Lösung von 35 mg (0.1 mmol) 1-Amino-N-(4-(2-(trifluormethyl)phenylamino)-benzyl)cyclopropancarboxamid (aus 5a) und 70 µL (0.50 mmol) Triethylamin in 1 mL DMF wurden 20 mg (0.15 mmol) Isoxazol-5-carbonylchlorid gegeben und es wurde bei Raumtemperatur über Nacht gerührt. Das Reaktionsgemisch wurde über eine präparative RP-HPLC-MS mit Fließmittelgradient (Wasser:Acetonitril+0.1%Trifluoressigsäure = 95:5 bis 5:95) gereinigt.
Ausbeute: 29% der Theorie
C₂₂H₁₉F₃N₄O₃ (444.41)
Rₜ= 2.42 min. Methode 6

### Beispiel 25: Pyrimidin-5-carbonsäure N-(1-(1-(4-(4-(difluormethoxy)phenylamino)-phenyl)ethylcarbamoyl)cyclopropyl)amid

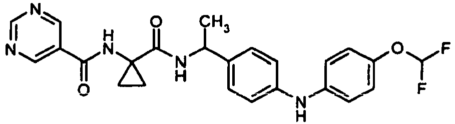

### 25a) 1-(4-(4-(Difluormethoxy)phenylamino)phenyl)ethanon

Die Reaktion findet unter Schutzgas (Argon) statt. Ein Gemisch aus 2.39 g (12 mmol) 1-(4-Bromphenyl)ethanon, 0.99 mL (8 mmol) 4-(Difluormethoxy)anilin, 2.21 g (16 mmol) Kaliumcarbonat, 150 mg (0.8 mmol) Kupferiodid und 180 mg (1.6 mmol) L-Prolin in 12 mL DMSO wurde 72 Stunden bei 95°C gerührt. Das Reaktionsgemisch wurde auf Wasser gegeben, mit etwas Ammoniak versetzt und zweimal mit *tert*-Butyl-methylether extrahiert. Die vereinten organischen Phasen wurden über Natriumsulfat getrocknet und im Vakuum bis zur Trockne eingedampft. Der Rückstand wurde mittels Säulenchromatographie (Kieselgel, Petrolether + 30% Essigsäureethylester) gereinigt. Das Produkt wurde direkt weiter umgesetzt.
Ausbeute: 33% der Theorie
C₁₅H₁₃F₂NO₂ (277.27)
Rₜ=1.98 min. Methode 1

### 25b) (Z)-1-(4-(4-(Difluormethoxy)phenylamino)phenyl)ethanon-oxim

Ein Gemisch aus 1.08 g (3.9 mmol) 1-(4-(4-(Difluormethoxy)phenylamino)phenyl)ethanon und 0.92 mL (15.58 mmol) wässriger 50%iger Hydroxylamin-Lösung in 10 mL Ethanol wurde 3 Stunden bei 100°C gerührt. Das Reaktionsgemisch wurde im Vakuum bis zur Trockne eingedampft und der Rückstand mittels präparativer HPLC (Methode 2) gereinigt.
Ausbeute: 19% der Theorie
C₁₅H₁₄F₂N₂O₂ (292.28)
Rₜ= 1.96 min. Methode 1

### 25c) 4-(1-Aminoethyl)-N-(4-(difluormethoxy)phenyl)anilin

0.22 g (0.75 mmol) (Z)-1-(4-(4-(Difluormethoxy)phenylamino)phenyl)ethanon-oxim in 20 mL methanolischer Ammoniak-Lösung wurden unter Zusatz von 50 mg Raney-Nickel bei 50°C und einem Wasserstoffdruck von 50 psi 5 Stunden hydriert. Anschließend wurde der Katalysator abfiltriert und das Filtrat zur Trockne eingedampft. Das so erhaltene Rohprodukt wurde direkt weiter umgesetzt.
C₁₅H₁₆F₂N₂O (278.3)
Rₜ= 1.37 min. Methode 1

### 25d) Pyrimidin-5-carbonsäure N-(1-(1-(4-(4-(difluormethoxy)phenylamino)phenyl)-ethylcarbamoyl)cyclopropyl) amid

Analog Vorschrift (1 d) wurde ausgehend von 4-(1-Aminoethyl)-N-(4-(difluormethoxy)-phenyl)anilin (aus 25c) und 1-[(Pyrimidin-5-carbonyl)-amino]-cyclopropancarbonsäure (aus 1 b) die Titelverbindung hergestellt.
C₂₄H₂₃F₂N₅O₃ (467.47)
Rₜ= 1.78 min. Methode 1

### Beispiel 26: 5-(Trifluormethyl)-N-(1-(4-(2-(trifluormethyl)phenylamino)benzylcarbamoyl)-cyclopropyl)nikotinamid

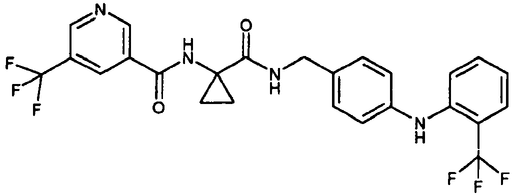

### 26a) 5-(Trifluormethyl)nikotinsäure

Zu einem Gemisch aus 9.96 mL (15.9 mmol) 1.6 molarer Butyllithium-Lösung in Hexan und 3.98 mL (8 mmol) 2 molarer Butylmagnesiumchlorid-Lösung in Diethylether und 10 mL THF wurde bei -75°C eine Lösung von 1.5 g 3-Brom-5-(trifluormethyl)pyridin in 50 ml Toluol zugetropft. Nach 20 Minuten wurden 20 g (454 mmol) Trockeneis zugegeben und es wurde nochmals 20 Minuten bei -75°C und 3 Stunden bei RT gerührt. Das Reaktionsgemisch wurde mit 50 mL 1 molarer Natronlauge versetzt und zweimal mit Diethylether extrahiert. Die wässrige Phase wurde mit 4 molarer Salzsäure sauer gestellt und dreimal mit Diethylether extrahiert. Die vereinten organischen Phasen wurden über Natriumsulfat getrocknet und im Vakuum bis zur Trockne eingedampft. Der Rückstand wurde mit Dichlormethan versetzt und der ausfallende Niederschlag abgesaugt und im Umlufttrockenschrank bei 55°C getrocknet.
Ausbeute: 9% der Theorie
C₇H₄F₃NO₂ (191.11)

### 26b) 5-(Trifluormethyl)-N-1-(4-(2-(trifluormethyl)phenylamino)benzylcarbamoyl)-cyclopropyl)nikotinamid

Analog Vorschrift (1d) wurde ausgehend von 1-Amin-N-(4-(2-(trifluormethyl)phenylamino)-benzyl)cyclopropancarboxamid (aus 5a) und 5-(Trifluormethyl)nicotinsäure (aus 26a) die Titelverbindung hergestellt.
C₂₂H₂₀F₃N₅O₃ (459.42)
Rₜ= 2.41 min. Methode 6

### Beispiel 27: 5-Methyl-N-(1-(4-(2-(trifluormethyl)phenylamino)benzylcarbamoyl)-cyclopropyl)-1,3,4-oxadiazol-2-carboxamid

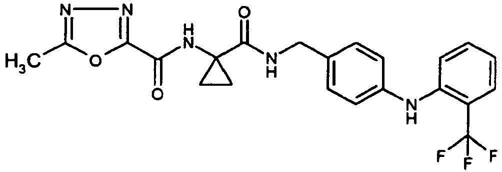

### 27a) 5-Methyl-N-(1-(4-(2-(trifluormethyl)phenylamino)benzylcarbamoyl)cyclopropyl)-1,3,4-oxadiazol-2-carboxamid

Analog Vorschrift (1d) wurde ausgehend von 1-Amin-N-(4-(2-(trifluormethyl)phenylamino)-benzyl)cyclopropancarboxamid (aus 5a) und 5-Methyl-1,3,4-oxadiazol-2-carbonsäure die Titelverbindung hergestellt.
C₂₂H₂₀F₃N₅O₃ (459.42)
Rₜ= 1.66 min. Methode 6

### Beispiel28: Pyrimidin-5-carbonsäure-N-(1-(4-(4-(methylthio)-2-(trifluormethyl)phenylamino)benzylcarbamoyl)cyclopropyl)amid

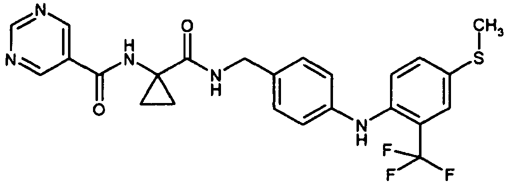

### 28a) N-(4-(Aminomethyl)phenyl)-4-(methylthio)-2-(trifluormethyl)anilin

Analog Vorschrift (1 c) wurde ausgehend von 4-(Methylthio)-2-(trifluormethyl)anilin und 4-Fluorbenzonitril die Titelverbindung hergestellt.
C₁₅H₁₅F₃N₂S (312.35)
Rₜ= 1.88 min. Methode 2

### 28b) Pyrimidin-5-carbonsäure-N-(1-(4-(4-(methylthio)-2-(trifluormethyl)phenylamino)-benzylcarbamoyl)cyclopropyl)amid

Analog Vorschrift (1 d) wurde ausgehend von N-(4-(Aminomethyl)phenyl)-4-(methylthio)-2-(trifluormethyl)anilin (aus 28a) und 1-[(Pyrimidin-5-carbonyl)-amino]-cyclopropancarbonsäure (aus 1 b) die Titelverbindung hergestellt.
C₂₄H₂₂F₃N₅O₂S (501.53)
Rₜ= 2.33 min. Methode 2

### Beispiel 29: N-(1-(4-(4-Fluor-2-(trifluormethyl)phenylamino)benzylcarbamoyl)-cyclopropyl)thiazol-5-carboxamid

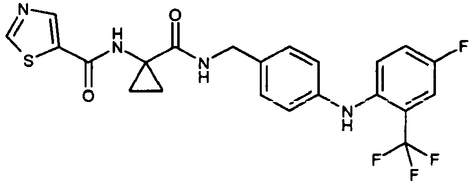

### 29a) N-(4-(Aminomethyl)phenyl)-2-(trifluormethyl)anilin

Analog Vorschrift (1c) wurde ausgehend von 2-Trifluormethyl-4-fluoranilin und 4-Fluorbenzonitril die Titelverbindung hergestellt.
C₁₄H₈F₄N₂ (280.22)
Rₜ= 0.38 min. Methode 4

### 29b) tert-Butyl 1-(4-(4-fluor-2-(trifluormethyl)phenylamino)benzylcarbamoyl)-cyclopropylcarbamat

Zu einer Lösung von 710 mg (3.52 mmol) 1-(*tert*-Butoxycarbonylamino)cyclopropancarbonsäure in 60 mL DMF wurden 0.98 mL (7.04 mmol) Triethylamin und 1.24 g (3.87 mmol) TBTU gegeben und es wurde 30 Minuten bei Raumtemperatur gerührt. Dann wurde 1 g N-(4-(Aminomethyl)phenyl)-2-(trifluormethyl)anilin zugegeben und 1 Stunde bei Raumtemperatur gerührt. Das Reaktionsgemisch wurde im Vakuum bis zur Trockne eingedampft. Der Rückstand wurde in Essigsäureethylester aufgenommen und zweimal mit 5%iger Natriumhydrogencarbonat-Lösung gewaschen. Die organische Phase wurde über Natriumsulfat getrocknet und im Vakuum bis zur Trockne eingedampft.
Ausbeute: 98% der Theorie
C₂₃H₂₅F₄N₃O₃ (467.46)
Rₜ= 1.50 min. Methode 4

### 29c) 1-Amino-N-(4-(4-fluor-2-(trifluormethyl)phenylamino)benzyl)cyclopropancarboxamid

1.57 g (3.36 mmol) tert-Butyl 1-(4-(4-fluor-2-(trifluormethyl)phenylamino)benzylcarbamoyl)cyclopropylcarbamat in 10 mL Diethylether wurden mit 20 mL 4 molarem Chlorwasserstoff in Dioxan versetzt und 10 Minuten bei Raumtemperatur gerührt. Das Reaktionsgemisch wurde mit Essigsäureethylester versetzt und mit gesättigter Kaliumcarbonat-Lösung alkalisch gestellt. Die organische Phase wurde über Natriumsulfat getrocknet und im Vakuum bis zur Trockne eingedampft.
Ausbeute: 101 % der Theorie
C₁₈H₁₇F₄N₃O (367.34)
Rₜ= 1.33 min. Methode 4

### 29d) N-(1-(4-(4-Fluor-2-(trifluormethyl)phenylamino)benzylcarbamoyl)cyclopropyl)-thiazol-5-carboxamid

Analog Vorschrift (1 d) wurde ausgehend von 1-Amino-N-(4-(4-fluor-2-(trifluormethyl)-phenylamino)benzyl)cyclopropancarboxamid (aus 29c) und Thiazol-5-carbonsäure die Titelverbindung hergestellt.
C₂₂H₁₈F₄N₄O₃S (478.46)
Rₜ= 2.76 min. Methode 3

### Beispiel 30: N-(4-(4-Fluor-2-(trifluormethyl)phenylamin)benzyl)-1-(3,3,3-trifluorpropanamido)cyclopropancarboxamid

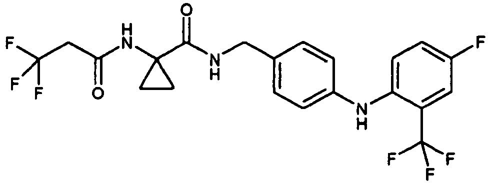

### 30a) N-(4-(4-Fluor-2-(trifluormethyl)phenylamin)benzyl)-1-(3,3,3-trifluorpropanamido)-cyclopropancarboxamid

Zu einer Lösung von 110.2 mg (0.3 mmol) 1-Amino-N-(4-(4-fluor-2-(trifluormethyl)phenylamino)benzyl)cyclopropancarboxamid (aus 29c) und 80 µL (0.8 mmol) Triethylamin in 10 mL Dichlormethan wurden 44.0 mg (0.15 mmol) 3,3,3-Trifluorpropionylchlorid, gelöst in 5 mL Dichlormethan, zugetropft. Anschließend ließ man bei Raumtemperatur über das Wochenende rühren und reinigte das Reaktionsgemisch dann über eine präparative RP-HPLC-MS (Methode 3). Das Eluat wurde mit konz. Ammoniak alkalisch gestellt und das Acetonitril abdestilliert. Das wässrige Gemisch wurde mit Essigsäureethylester extrahiert und die organische Phase über Natriumsulfat getrocknet und im Vakuum bis zur Trockne eingedampft.
Ausbeute: 44% der Theorie
C₂₁H₁₈F₇N₃O₂ (477.38)
Rₜ= 2.85 min. Methode 3

### Beispiel 31: N-(1-(4-(4-Fluor-2-(trifluormethyl)phenylamin)benzylcarbamoyl)-cyclopropyl)isoxazol-5-carboxamid

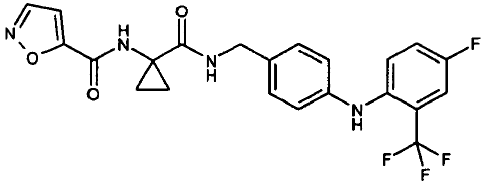

Analog Vorschrift (30a) wurde ausgehend von 1-Amino-N-(4-(4-fluor-2-(trifluormethyl)-phenylamino)benzyl)cyclopropancarboxamid (aus 29c) und Isoxazol-5-carbonylchlorid die Titelverbindung hergestellt.
C₂₂H₁₈F₄N₄O₃ (462.4)
Rₜ= 2.79 min. Methode 3

### Beispiel 32: Pyrimidin-5-carbonsäure-N-(1-(4-(4-(methylsulfonyl)-2-(trifluormethyl)-phenylamin)benzylcarbamoyl)cyclopropyl)amid

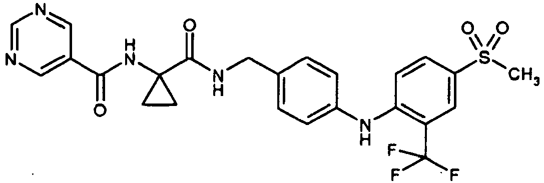

Zu 66 mg (0.13 mmol) Pyrimidin-5-carbonsäure-N-(1-(4-(4-(methylthio)-2-(trifluormethyl)-phenylamino)benzylcarbamoyl)cyclopropyl)amid (aus 28b), gelöst in 5 mL Dichlormethan, wurden 34 mg (0.2 mmol) 3-Chlorperoxybenzoesäure gegeben und man ließ über Nacht bei Raumtemperatur rühren. Anschließend wurde der Ansatz auf gesättigte Natriumhydrogencarbonat-Lösung gegeben und mit Dichlormethan ausgeschüttelt. Die organische Phase wurde über eine Phasentrennkartusche getrocknet und das Filtrat im Vakuum bis zur Trockne eingedampft.
Ausbeute: 40% der Theorie
C₂₄H₂₂F₃N₅O₄S (533.52)
Rₜ= 1.84 min. Methode 2

### Beispiel 33: Pyrimidin-5-carbonsäure-N-(1-(4-(4-fluor-2-(trifluormethyl)phenylamin)-benzylcarbamoyl)cyclopropyl)amid

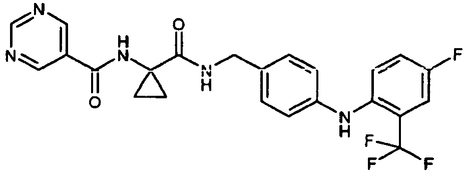

Analog Vorschrift (1d) wurde ausgehend von 1-Amino-N-(4-(4-fluor-2-(trifluormethyl)-phenylamino)benzyl)cyclopropancarboxamid (aus 29c) und Pyrimidin-5-carbonsäure die Titelverbindung hergestellt.
C₂₃H₁₉F₄N₅O₂ (473.42)
Rₜ= 2.09 min. Methode 2

### Beispiel 34: 1-(2-(Pyrimidin-5-yl)acetamido)-N-(4-(2-(trifluormethyl)phenylamino)-benzyl)cyclopropancarboxamid

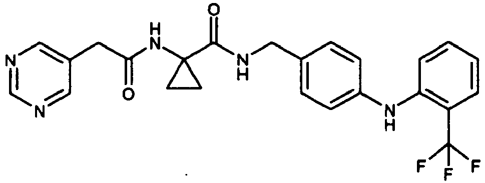

Analog Vorschrift (1d) wurde ausgehend von 1-Amino-N-(4-(4-fluor-2-(trifluormethyl)-phenylamino)benzyl)cyclopropancarboxamid (aus 29c) und Pyrimidin-5-carbonsäure die Titelverbindung hergestellt.
C₂₄H₂₂F₃N₅O₂ (469.46)
Rₜ= 2.21 min. Methode 6

### Beispiel 35: Pyrimidin-5-carbonsäure-N-(1-(4-(2-cyanphenylamin)benzylcarbamoyl)-cyclopropyl)amid

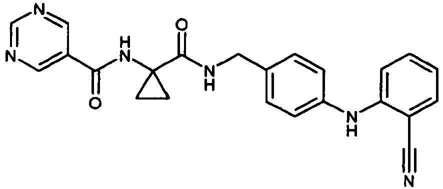

### 35a) tert-Butyl-4-aminobenzylcarbamat

Zu 61.85 g (424.4 mmol) 4-Aminomethyl-anilin gelöst in 850 mL Chloroform wurden 92.65 g (424.5 mmol) Di-*tert*-butyl-dicarbonat gegeben und es wurde bei Raumtemperatur gerührt bis kein Edukt mehr vorhanden war. Der Ansatz wurde im Vakuum bis zur Trockne eingedampft und der Rückstand aus Essigsäureethylester/Hexan (ca. 3 mUg) umkristallisiert.
Ausbeute: 66% der Theorie
C₁₂H₁₈N₂O₂ (222.28)
Rₜ= 0.49 Hexan : Essigsäureethylester (1/1)

### 35b) tert-Butyl-4-(2-cyanphenylamino)benzylcarbamat

Die Reaktion fand unter Schutzgas statt (Stickstoff). Zu 100 mg (0.45 mmol) tert-Butyl-4-aminobenzylcarbamat, 138 mg (0.63 mmol) Kaliumsulfat und 98 mg (0.54 mmol) 2-Brombenzonitril in 5 mL Toluol wurden 8 mg (0.01 mmol) Tris(dibenzylidenaceton)-dipalladium und 17 mg (0.04 mmol) Xantphos gegeben. Man ließ über Nacht bei 110°C rühren und filtrierte anschließend die anorganischen Salze ab. Das Filtrat wurde im Vakuum bis zur Trockne eingedampft und der Rückstand über eine RP-Säule mit Lösungsmittelgradient (Wasser/Acetonitril + 0.1%Trifluoressigsäure) gereinigt.
Ausbeute: 82% der Theorie
C₁₉H₂₁N₃O₂ (323.39)
Rₜ= 2.57 min. Methode 2

### 35c) 2-(4-(Aminomethyl)phenylamino)benzonitril 2,2,2-Trifluoracetat

119 mg (0.37 mmol) *tert*-Butyl 4-(2-cyanphenylamino)benzylcarbamat wurden in 5 mL Dichlormethan gelöst und mit 1 mL (13.06 mmol) Trifluoressigsäure versetzt. Die Reaktion wurde über Nacht bei Raumtemperatur gerührt und anschließend im Vakuum bis zur Trockne eingedampft.
Ausbeute: 99% der Theorie
C₁₄H₁₃N₃*C₂HF₃O₂ (337.3)
Rₜ= 1.30 min. Methode 2

### 35d) Pyrimidin-5-carbonsäure-N-(1-(4-(2-cyanphenylamin)benzylcarbamoyl)cyclopropyl)amid

Analog Vorschrift (1d) wurde ausgehend von 2-(4-(Aminomethyl)phenylamino)benzonitril 2,2,2-Trifluoracetat (aus 35c) und 1-[(Pyrimidin-5-carbonyl)-amino]-cyclopropancarbonsäure (aus 1 b) die Titelverbindung hergestellt.
C₂₃H₂₀N₆O₂ (412.44)
Rₜ= 1.84 min. Methode 2

### Beispiel 36: Pyrimidin-5-carbonsäure-N-(1-(4-(2-cyan-4-fluorphenylamin)benzylcarbamoyl)cyclopropyl)amid

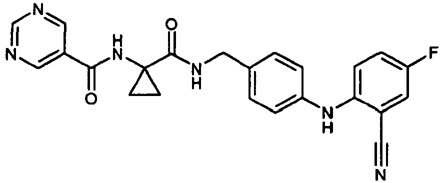

### 36a) Tert-butyl-4-(2-cyan-4-fluorphenylamino)benzylcarbamat

Analog Vorschrift (35b) wurde ausgehend von tert-Butyl-4-aminobenzylcarbamat (aus 35a), Kaliumsulfat, 2-Brom-5-fluorbenzonitril, Tris(dibenzylidenaceton)dipalladium und Xantphos die Titelverbindung hergestellt.
C₁₉H₂₀FN₆O₂ (341.38)
Rₜ= 2.61 min. Methode 2

### 36b) 2-(4-(Aminomethyl)phenylamino)-5-fluorbenzonitril 2,2,2-Trifluoracetat

Analog Vorschrift (35c) wurde ausgehend von *tert*-Butyl-4-(2-cyan-4-fluorphenylamino)-benzylcarbamat und Trifluoressigsäure die Titelverbindung hergestellt.
C₁₄H₁₂FN₃*C₂HF₃O₂ (355.29)
Rₜ= 1.39 min. Methode 2

### 36c) Pyrimidin-5-carbonsäure-N-(1-(4-(2-cyan-4-fluorphenylamin)benzylcarbamoyl)-cyclopropyl)amid

Analog Vorschrift (1d) wurde aus 2-(4-(Aminomethyl)phenylamino)-5-fluorbenzonitril 2,2,2-Trifluoracetat (aus 36b) und 1-[(Pyrimidin-5-carbonyl)-amino]-cyclopropancarbonsäure (aus 1 b) die Titelverbindung hergestellt.
C₂₃H₁₉FN₆O₂ (430.43)
Rₜ= 1.91 min. Methode 2

### Beispiel 37: Pyrimidin-5-carbonsäure-N-(1-(4-(4-fluorphenylamino)benzylcarbamoyl)-cyclopropyl)amid

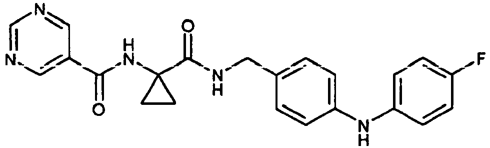

### 37a) 4-(Aminomethyl)-N-(4-fluorphenyl)anilin

Analog Vorschrift (1 c) wurde ausgehend von 2-Brom-4-fluor-anilin, 4-Fluorbenzonitril und Raney-Nickel die Titelverbindung hergestellt.
C₁₃H₁₃FN₂ (216.25)

### 37b) Pyrimidin-5-carbonsäure-N-(1-(4-(4-fluorphenylamino)benzylcarbamoyl)cyclopropyl)amid

Analog Vorschrift (1 d) wurde aus 4-(Aminomethyl)-N-(4-fluorphenyl)anilin (aus 37a) und 1-[(Pyrimidin-5-carbonyl)-amino]-cyclopropancarbonsäure (aus 1b) die Titelverbindung hergestellt.
C₂₂H₂₀FN₅O₂ (405.43)
Massenspektroskopie [M+H]⁺ = 406

### Beispiel 38: Pyrimidin-5-carbonsäure-N-(1-((5-(2-chlorphenylamino)-3-fluorpyridin-2-yl)methylcarbamoyl)cyclopropyl)amid

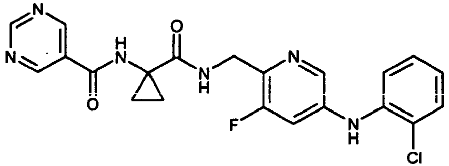

### 38a) 6-(Aminomethyl)-N-(2-chlorphenyl)-5-fluorpyridin-3-amin

Analog Vorschrift (1 c) wurde ausgehend von 2-Chlor-anilin, 2-Cyan-3,5-difluorpyridin und Raney-Nickel die Titelverbindung hergestellt.
C₁₂H₁₁FN₃ (251.69)
Rₜ= 1.295 min. Methode 1

### 38b) Pyrimidin-5-carbonsäure- N-(1-((5-(2-chlorphenylamino)-3-fluorpyridin-2-yl)-methylcarbamoyl)cyclopropyl)amid

Analog Vorschrift (1d) wurde aus 6-(Aminomethyl)-N-(2-chlorphenyl)-5-fluorpyridin-3-amin (aus 38a) und 1-[(Pyrimidin-5-carbonyl)-amino]-cyclopropancarbonsäure (aus 1b) die Titelverbindung hergestellt.
C₂₁H₁₈FN₆O₂ (440.86)
Rₜ= 1.73 min. Methode 1

### Beispiel 39: Pyrimidin-5-carbonsäure-N-(1-((5-(2-(trifluormethyl)phenylamino)pyridin-2-yl)methylcarbamoyl)cyclopropyl)amid

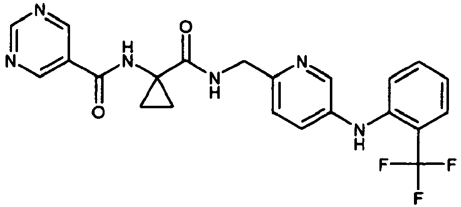

### 39a) 6-(Aminomethyl)-N-(2-(trifluormethyl)phenyl)pyridin-3-amin

Analog Vorschrift (1 c) wurde ausgehend von 2-(Trifluormethyl)anilin, 5-Fluor-picolinsäurenitril und Raney-Nickel die Titelverbindung hergestellt.
C₁₃H₁₂F₃N₃ (267.25)
Rₜ= 1.29 min. Methode 1

### 39b) Pyrimidin-5-carbonsäure-N-(1-((5-(2-(trifluormethyl)phenylamino)pyridin-2-yl)-methylcarbamoyl)cyclopropyl)amid

Analog Vorschrift (1d) wurde aus 6-(Aminomethyl)-N-(2-(trifluormethyl)phenyl)pyridin-3-amin (aus 39a) und 1-[(Pyrimidin-5-carbonyl)-amino]-cyclopropancarbonsäure (aus 1 b) die Titelverbindung hergestellt.
C₂₂H₁₉F₃N₆O₂ (456.42)
Rₜ= 1.39 min. Methode 1

### Beispiel 40: Pyrimidin-5-carbonsäure-N-(1-(1-(5-(2-(trifluormethyl)phenylamino)pyridin-2-yl)ethylcarbamoyl)cyclopropyl)amid

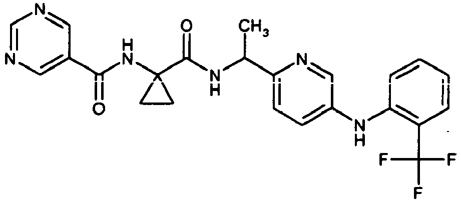

### 40a) 5-(2-(Trifluormethyl)phenylamino)picolinsäurenitril

820 mg (6.72 mmol) 5-Fluorpicolinsäurenitril und 0.84 mL (6.72 mmol) 2-(Trifluormethyl)-anilin in 10 mL DMSO wurden mit 1.51 g (13.43 mmol) Kalium-tert-butylat versetzt und 2 Stunden bei Raumtemperatur gerührt. Das Gemisch wurde auf eine wässrige Natriumchlorid-Lösung gegossen und mit tert-Butylmethyether extrahiert. Die organische Phase wurde im Vakuum bis zur Trockne eingedampft und das so erhaltene Rohprodukt mittels HPLC (Methode 2) gereinigt.
Ausbeute: 54% der Theorie
C₁₃H₈F₃N₃ (263.22)

### 40b) 1-(5-(2-(Trifluormethyl)phenylamino)pyridin-2-yl)ethanon

Die Reaktion fand unter Schutzgas statt (Stickstoff). Zu 9.34 mL (13.07 mmol) einer 1.4 molaren Lösung von Methylmagnesiumbromid in Toluol/THF (3:1) wurden 860 mg (3.27 mmol) 5-(2-(Trifluormethyl)phenylamino)picolinsäurenitril in 5 mL Diethylether bei -10°C zugetropft und man ließ 15 Minuten bei dieser Temperatur rühren. Die Reaktionsmischung wurde mit gesättigter Ammoniumchlorid-Lösung versetzt, mit 1 molarer wässriger Salzsäure bei -5°C neutralisiert und mit tert-Butylmethylether extrahiert. Die organische Phase wurde im Vakuum bis zur Trockne eingedampft.
Ausbeute: 96% der Theorie
C₁₄H₁₁F₃N₂O (280.25)
Rₜ= 1.97 min. Methode 1

### 40c) (Z)-1-(5-(2-(Trifluormethyl)phenylamino)pyridin-2-yl)ethanon-oxim

Zu 870 mg (3.1 mmol) 1-(5-(2-(Trifluormethyl)phenylamino)pyridin-2-yl)ethanon in 5 mL Ethanol wurden 0.73 mL (12.42 mmol) einer 50%igen wässrigen Hyroxylamin-Lösung gegeben. Man ließ 2 Stunden bei 100°C rühren und destillierte dann die Lösungsmittel ab.
Ausbeute: 98% der Theorie
C₁₄H₁₂F₃N₃O (295.26)
Rₜ= 1.75 min. Methode 1

### 40d) 6-(1-Aminoethyl)-N-(2-(trifluormethyl)phenyl)pyridin-3-amin

900 mg (3.05 mmol) (Z)-1-(5-(2-(Trifluormethyl)phenylamino)pyridin-2-yl)ethanon-oxim und 100 mg Raney-Nickel in 25 mL methanolischem Ammoniak wurden 1.5 Tage bei Raumtemperatur und 50 psi Wasserstoffdruck hydriert. Die Reaktionsmischung wurde filtriert, bis zur Trockne eingedampft und anschließend direkt weiter umgesetzt.
Ausbeute: 96% der Theorie
C₁₄H₁₄F₃N₃ (281.28)
Rₜ= 1.33 min. Methode 1

### 40e) Pyrimidin-5-carbonsäure-N-(1-(1-(5-(2-(trifluormethyl)phenylamino)pyridin-2-yl)-ethylcarbamoyl)cyclopropyl)amid

Analog Vorschrift (1 d) wurde aus 6-(1-Aminoethyl)-N-(2-(trifluormethyl)phenyl)pyridin-3-amin (aus 40d) und 1-[(Pyrimidin-5-carbonyl)-amino]-cyclopropancarbonsäure (aus 1b) die Titelverbindung hergestellt.
C₂₃H₂₁F₃N₆O₂ (470.45)
Rₜ= 1.46 min. Methode 1

### Beispiel 41: Pyrimidin-5-carbonsäure- N-(1-((5-(4-fluor-2-(trifluormethyl)phenylamino)-pyridin-2-yl)methylcarbamoyl)cyclopropyl)amid

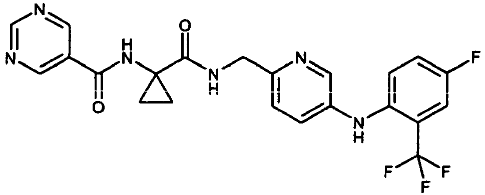

### 41a) 6-(Aminomethyl)-N-(4-fluor-2-(trifluormethyl)phenyl)pyridin-3-amin

Analog Vorschrift (1c) wurde ausgehend von 2-Trifluormethyl-4-fluor-anilin, 2-Cyan-5-fluorpyridin und Raney-Nickel die Titelverbindung hergestellt.
C₁₃H₁₁F₄N₃ (285.24)
Rₜ= 1.50 min. Methode 9

### 41 b) Pyrimidin-5-carbonsäure- N-(1-((5-(4-fluor-2-(trifluormethyl)phenylamino)pyridin-2-yl)methylcarbamoyl)cyclopropyl)amid

Analog Beispiel (1 d) wurde aus 6-(Aminomethyl)-N-(4-fluor-2-(trifluormethyl)phenyl)-pyridin-3-amin (aus 41a) und 1-[(Pyrimidin-5-carbonyl)-amino]-cyclopropancarbonsäure (aus 1 b) die Titelverbindung hergestellt.
C₂₂H₁₈F₄N₆O₂ (474.41)
Rₜ= 2.96 min. Methode 7

### Beispiel 42: Pyrimidin-5-carbonsäure-N-(1-((5-(5-fluor-2-(trifluormethyl)phenylamino)-pyridin-2-yl)methylcarbamoyl)cyclopropyl)amid

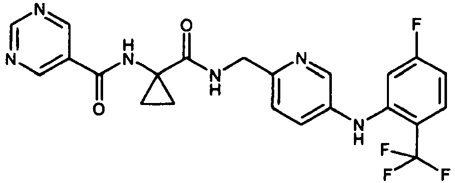

### 42a) 6-(Aminomethyl)-N-(5-fluor-2-(trifluormethyl)phenyl)pyridin-3-amin

Analog Vorschrift (1 c) wurde ausgehend von 2-Fluor-6-(trifluormethyl)anilin, 2-Cyan-5-fluorpyridin und Raney-Nickel die Titelverbindung hergestellt.
C₁₃H₁₁F₄N₃ (281.24)
Rₜ= 1.95 min. Methode 8

### 42b) Pyrimidin-5-carbonsäure-N-(1-((5-(4-fluor-2-(trifluormethyl)phenylamino)pyridin-2-yl)methylcarbamoyl)cyclopropyl)amid

Analog Vorschrift (1d) wurde ausgehend von 6-(Aminomethyl)-N-(4-fluor-2-(trifluormethyl)-phenyl)pyridin-3-amin (aus 41a) und 1-[(Pyrimidin-5-carbonyl)-amino]-cyclopropancarbonsäure (aus 1 b) die Titelverbindung hergestellt.
C₂₂H₁₈F₄N₆O₂ (474.41)
Rₜ= 3.10 min. Methode 7

### Beispiel 43: (S)-Pyrimidin-5-carbonsäure-N-(3-((5-(4-fluor-2-(trifluormethyl)phenylamino)pyridin-2-yl)methylcarbamoyl)tetrahydrofuran-3-yl)amid

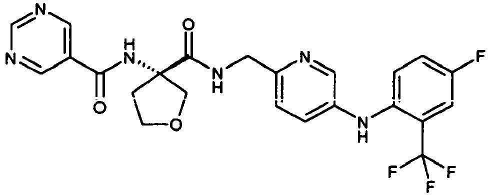

### 43a) (S)-Phenethyl-3-aminotetrahydrofuran-3-carboxylat

19.37 g (50 mmol) (S)-Phenethyl-3-aminotetrahydrofuran-3-carboxylate (S)-2-hydroxy-2-phenylacetat wurden in 75 mL THF und 75 mL Wasser suspendiert, mit 6.3 g (75 mmol) Natriumhydrogencarbonat versetzt und 3 Stunden bei Raumtemperatur gerührt. Das Gemisch wurde zweimal mit Dichlormethan extrahiert. Die organischen Phasen wurden mit 14%iger Natriumchlorid-Lösung gewaschen, über Natriumsulfat getrocknet und im Vakuum bis zur Trockne eingedampft. Das so erhaltene Rohprodukt wurde direkt weiter umgesetzt.
Ausbeute: 85% der Theorie
C₁₃H₁₇NO₃ (235.28)
Rₜ= 1.19 min. Methode 1

### 43b) (S)-Phenethyl-3-(pyrimidin-5-carboxamido)tetrahydrofuran-3-carboxylat

Zu einer Lösung von 2 g (16.1 mmol) Pyrimidin-5-carbonsäure in 50 mL DMF wurden 4.43 mL (40.3 mmol) N-Methylmorpholin und 5.69 g (17.7 mmol) TBTU gegeben. Man ließ 30 Minuten bei Raumtemperatur rühren und versetzte dann mit 3.8 g (16.16 mmol) (S)-Phenethyl-3-aminotetrahydrofuran-3-carboxylat. Das Gemisch wurde über Nacht bei Raumtemperatur gerührt und anschließend bis zur Trockne eingeengt. Das so erhaltene Rohprodukt wurde mittels HPLC (Methode 2) gereinigt.
Ausbeute: 93% der Theorie
C₁₈H₁₉N₃O₄ (341.36)
Rₜ= 1.60 min. Methode 1

### 43c) (S)-3-(Pyrimidin-5-carboxamido)tetrahydrofuran-3-carbonsäure

Zu einer Lösung von 5.14 g (15.1 mmol) (S)-Phenethyl 3-(pyrimidin-5-carboxamido)tetrahydrofuran-3-carboxylat in 97 mL Ethanol wurden 60.24 mL (60.24 mmol) einer 1 molaren Natronlauge gegeben. Es wurde 1 Stunde bei Raumtemperatur gerührt und dann mit 4 molarer Salzsäure sauer gestellt. Die Reinigung erfolgte mittels HPLC (Methode 2).
Ausbeute: 93% der Theorie
C₁₀H₁₁N₃O₄ (237.21)
Rₜ= 0.87 min. Methode 1

### 43d) Pyrimidin-5-carbonsäure-(S)-N-(3-((5-(4-fluor-2-(trifluormethyl)phenylamino)-pyridin-2-yl)methylcarbamoyl)tetrahydrofuran-3-yl)amid

Analog Vorschrift (1 d) wurde aus 6-(Aminomethyl)-N-(4-fluor-2-(trifluormethyl)phenyl)-pyridin-3-amin (aus 41 a) und (S)-3-(Pyrimidin-5-carboxamido)tetrahydrofuran-3-carbonsäure (aus 43c) die Titelverbindung hergestellt.
C₂₃H₂₀F₄N₆O₃ (504.44)
Rₜ= 2.86 min. Methode 7

### Beispiel 44: Pyrimidin-5-carbonsäure-N-(1-((5-(2-fluor-6-(trifluormethyl)phenylamino)-pyridin-2-yl)methylcarbamoy)cyclopropyl)amid

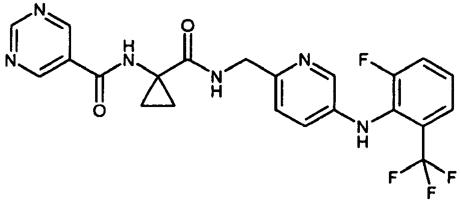

### 44a) 6-(Aminomethyl)-N-(2-fluor-6-(trifluormethyl)phenyl)pyridin-3-amin

Analog Vorschrift (1 c) wurde ausgehend von 2-Trifluormethyl-5-fluor-anilin, 2-Cyan-5-fluorpyridin und Raney-Nickel die Titelverbindung hergestellt.
C₁₃H₁₁F₄N₃ (285.24)
Rₜ= 1.95 min. Methode 8

### 44b) Pyrimidin-5-carbonsäure-N-(1-((5-(2-fluor-6-(trifluormethyl)phenylamino)pyridin-2-yl)methylcarbamoyl)cyclopropyl)amid

Analog Vorschrift (1d) wurde ausgehend von 6-(Aminomethyl)-N-(2-fluor-6-(trifluormethyl)phenyl)pyridin-3-amin (aus 44a) und 1-[(Pyrimidin-5-carbonyl)-amino]-cyclopropancarbonsäure (aus 1b) die Titelverbindung hergestellt.
C₂₂H₁₈F₄N₆O₂ (474.41)
Rₜ=2.71 min. Methode 7

### Beispiel 45: Pyrimidin-5-carbonsäure-N-(1-(1-(5-(4-fluor-2-(trifluormethyl)phenylamino)-pyridin-2-yl)ethylcarbamoyl)cyclopropyl)amid

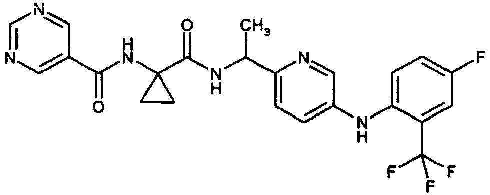

### 45a) 5-(4-Fluor-2-(trifluormethyl)phenylamino)picolinsäurenitril

Analog Vorschrift (40a) wurde ausgehend von 5-Fluor-picolinsäurenitril, 4-Fluor-2-(trifluormethyl)anilin und Kalium-tert-butylat in DMSO die Titelverbindung hergestellt.
C₁₃H₈F₃N₃ (281.21)
Rₜ= 1.40 min. Methode 4

### 45b) 1-(5-(4-Fluor-2-(trifluormethyl)phenylamino)pyridin-2-yl)ethanon

Analog Vorschrift (40b) wurde aus Methylmagnesiumbromid und 5-(4-Fluor-2-(trifluormethyl)phenylamino)picolinsäurenitril die Titelverbindung hergestellt.
C₁₄H₁₁F₄N₂O (298.24)
Rₜ= 1.43 min. Methode 4

### 45c) (E)-1-(5-(4-Fluor-2-(trifluormethyl)phenylamino)pyridin-2-yl)ethanon-oxim

Analog Vorschrift (40c) wurde ausgehend von 1-(5-(2-(Trifluormethyl)phenylamino)pyridin-2-yl)ethanon und einer 50%igen wässrigen Hyroxylamin-Lösung die Titelverbindung hergestellt.
C₁₄H₁₁F₄N₃O (313.25)
Rₜ= 1.31 min. Methode 4

### 45d) 6-(1-Aminoethyl)-N-(4-fluor-2-(trifluormethyl)phenyl)pyridin-3-amin

Analog Vorschrift (40d) wurde ausgehend von (E)-1-(5-(4-Fluor-2-(trifluormethyl)phenylamino)pyridin-2-yl)ethanon-oxim und Raney-Nickel die Titelverbindung hergestellt.
C₁₄H₁₄F₄N₃ (299.27)
Rₜ= 1.65 min. Methode 9

### 45e) Pyrimidin-5-carbonsäure-N-(1-(1-(5-(4-fluor-2-(trifluormethyl)phenylamino)pyridin-2-yl)ethylcarbamoyl)cyclopropyl)amid

Analog Vorschrift (1 d) wurden 6-(1-Aminoethyl)-N-(4-fluor-2-(trifluormethyl)phenyl)pyridin-3-amin (aus 45d) und 1-[(Pyrimidin-5-carbonyl)-amino]-cyclopropancarbonsäure (aus 1b) zur Titelverbindung umgesetzt.
C₂₃H₂₀F₄N₆O₂ (488.44)
Rₜ= 3.01 min. Methode 7

### Beispiel 46: (S)-Pyrimidin-5-carbonsäure-N-(3-((5-(2-fluor-6-(trifluormethyl)pheny)-amino)pyridin-2-yl)methylcarbamoyl)tetrahydrofuran-3-yl)amid

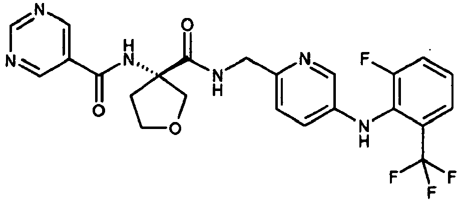

Analog Vorschrift (1 d) wurde aus 6-(Aminomethyl)-N-(4-fluor-2-(trifluormethyl)phenyl)-pyridin-3-amin (aus 41a) und (S)-3-(Pyrimidin-5-carboxamido)tetrahydrofuran-3-carbonsäure (aus 43c) die Titelverbindung erhalten.
C₂₃H₂₀F₄N₆O₃ (504.44)
Rₜ= 2.74 min. Methode 7

### Beispiel 47: (S)-Pyrimidin-5-carbonsäure-N-(-3-(1-(5-(4-fluor-2-(trifluormethyl)phenylamino)pyridin-2-yl)ethylcarbamoyl)tetrahydrofuran-3-yl)amid

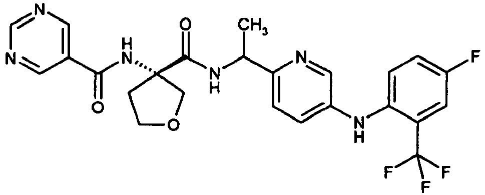

Analog Vorschrift (1 d) wurde aus 6-(1-Aminoethyl)-N-(4-fluor-2-(trifluormethyl)phenyl)-pyridin-3-amin (aus 45d) und (S)-3-(Pyrimidin-5-carboxamido)tetrahydrofuran-3-carbonsäure (aus 43c) die Titelverbindung hergestellt.
C₂₄H₂₂F₄N₆O₃ (518.46)
Rₜ= 3.00 min. Methode 7

### Beispiel 48: (S)-Pyrimidin-5-carbonsäure-N-(3-((5-(5-fluor-2-(trifluormethyl)phenylamino)pyridin-2-yl)methylcarbamoyl)tetrahydrofuran-3-yl)amid

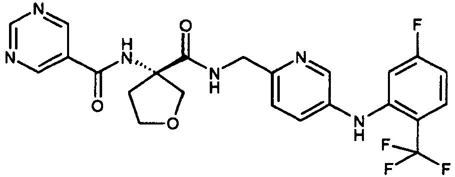

Analog Vorschrift (1d) wurde aus 6-(Aminomethyl)-N-(5-fluor-2-(trifluormethyl)phenyl)-pyridin-3-amin (aus 42a) und (S)-3-(Pyrimidin-5-carboxamido)tetrahydrofuran-3-carbonsäure (aus 43c) die Titelverbindung erhalten.
C₂₃H₂₀F₄N₆O₃ (504.44)
Rₜ= 3.15min. Methode 7

### Beispiel 49: Pyrimidin-5-carbonsäure-N-(1-((5-(2-methyl-6-(trifluormethyl)phenylamino)-pyridin-2-yl)methylcarbamoyl)cyclopropyl)amid

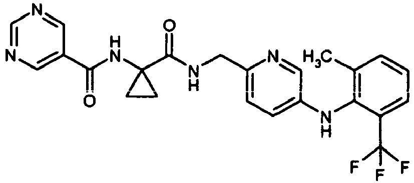

### 49a) 6-(Aminomethyl)-N-(2-methyl-6-(trifluormethyl)phenyl)pyridin-3-amin

Analog Vorschrift (1 c) wurde aus 2-Methyl-6-(trifluormethyl)-anilin und 2-Cyan-5-fluorpyridin mit Raney-Nickel als Katalysator die Titelverbindung hergestellt.
C₁₄H₁₄F₃N₃ (281.28)
Rₜ= 1.52 min. Methode 2

### 49b) Pyrimidin-5-carbonsäure-N-(1-((5-(2-methyl-6-(trifluormethyl)phenylamino)pyridin-2-yl)methylcarbamoyl)cyclopropyl)amid

Analog Vorschrift (1d) wurde aus 6-(Aminomethyl)-N-(2-methyl-6-(trifluormethyl)pheny)-pyridin-3-amin (aus 49a) und 1-[(Pyrimidin-5-carbonyl)-amino]-cyclopropancarbonsäure (aus 1 b) die Titelverbindung hergestellt.
C₂₂H₁₈F₄N₆O₂ (470.45)
Rₜ=1.57 min. Methode 2

### Beispiel 50: Pyrimidin-5-carbonsäure-N-(1-((5-(4-methoxy-2-(trifluormethyl)phenylamino)pyridin-2-yl)methylcarbamoyl)cyclopropyl)amid

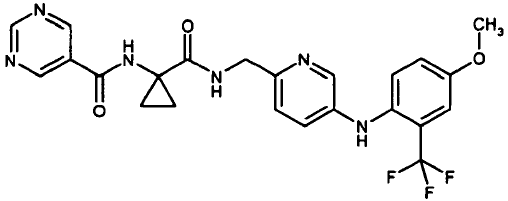

### 50a) 6-(Aminomethyl)-N-(4-methoxy-2-(trifluormethyl)phenyl)pyridin-3-amin

Analog Vorschrift (1c) wurde ausgehend von 2-Amino-5-methoxybenzotrifluorid und 2-Cyan-5-fluorpyridin mit Raney-Nickel als Katalysator die Titelverbindung hergestellt.
C₁₄H₁₄F₃N₃O (297.28)
R_{f}= 0.21 Essigsäureethylester / Methanol / Ammoniak = 9:1:0.1

### 50b) Pyrimidin-5-carbonsäure-N-(1-((5-(4-methoxy-2-(trifluormethyl)phenylamino)-pyridin-2-yl)methylcarbamoyl)cyclopropyl)amid

Analog Vorschrift (1d) wurde aus 6-(Aminomethyl)-N-(4-methoxy-2-(trifluormethyl)phenyl)-pyridin-3-amin (aus 50a) und 1-[(Pyrimidin-5-carbonyl)-amino]-cyclopropancarbonsäure (aus 1 b) die Titelverbindung hergestellt.
C₂₃H₂₁F₃N₆O₃ (486.45)
Rₜ=2.82 min. Methode 7

### Beispiel 51: Pyrimidin-5-carbonsäure-N-(1-((5-(4-methyl-2-(trifluormethyl)phenylamino)pyridin-2-yl)methylcarbamoyl)cyclopropyl)amid

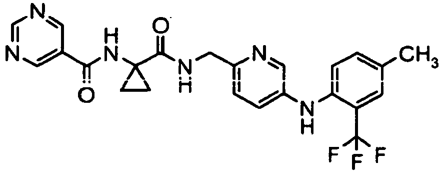

### 51 a) 6-(Aminomethyl)-N-(4-methyl-2-(trifluormethyl)phenyl)pyridin-3-amin

Analog Vorschrift (1 c) wurde aus 2-Amino-5-methylbenzotrifluorid und 2-Cyan-5-fluorpyridin unter Verwendung von Raney-Nickel die Titelverbindung hergestellt.
C₁₄H₁₄F₃N₃ (281.28)
Rₜ= 1.63 min. Methode 2

### 51b) Pyrimidin-5-carbonsäure-N-(1-((5-(4-methyl-2-(trifluormethyl)phenylamino)pyridin-2-yl)methylcarbamoyl)cyclopropyl)amid

Zu einer Lösung von 50 mg (0.24 mmol) 1-[(Pyrimidin-5-carbonyl)-amino]-cyclopropancarbonsäure (aus 1b) in 2 mL THF wurden 0.1 mL (0.56 mmol) DIPEA und 87 mg (0.27 mmol) O-[(Ethoxycarbonyl)cyanomethylenamino]-N,N,N',N'-tetramethyluroniumtetrafluoroborat, gelöst in 0.5 mL DMF, gegeben. Man ließ 15 Minuten bei Raumtemperatur rühren und gab dann 82 mg (0.29 mmol) 6-(Aminomethyl)-N-(4-methyl-2-(trifluormethyl)-phenyl)pyridin-3-amin (aus 51a) in 0.5 mL DMF zu. Das Gemisch wurde über Nacht bei Raumtemperatur gerührt und anschließend mittels HPLC (Microsorb C18; 41.4 x 250 mm mit Acetonitril/Wasser/Trifluoressigsäure = 10/90/0.1 => 100/0/0.1) gereinigt.
Ausbeute: 33% der Theorie
C₂₃H₂₁F₃N₆O₂ (470.45)
Rₜ= 1.63 min. Methode 2

### Beispiel 52: Pyrimidin-5-carbonsäure-N-(1-((5-(2,4-bis(trifluormethyl)phenylamino)-pyridin-2-yl)methylcarbamoyl)cyclopropyl)amid

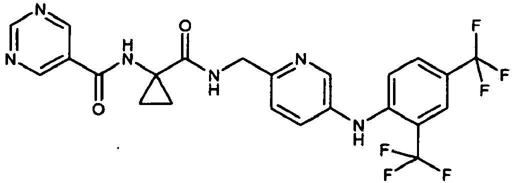

### 52a) 6-(Aminomethyl)-N-(2,4-bis(trifluormethyl)phenyl)pyridin-3-amin

Analog Vorschrift (1c) wurde aus 2,4-Bis(trifluormethyl)anilin und 2-Cyano-5-fluorpyridin unter Verwendung von Raney-Nickel die Titelverbindung hergestellt.
C₁₄H₁₁F₆N₃ (335.25)

### 52b) Pyrimidin-5-carbonsäure-N-(1-((5-(2,4-bis(trifluormethyl)phenylamino)pyridin-2-yl)-methylcarbamoyl)cyclopropyl)amid

Analog Vorschrift (1 d) wurde aus 6-(Aminomethyl)-N-(2,4-bis(trifluormethyl)pheny)pyridin-3-amin (aus 52a) und 1-[(Pyrimidin-5-carbonyl)-amino]-cyclopropancarbonsäure (aus 1b) die Titelverbindung erhalten.
C₂₃H₁₈F₆N₆O₂ (524.42)
Rₜ= 3.63 min. Methode 10

### Beispiel 53: Pyrimidin-5-carbonsäure-N-(1-((5-(4-brom-2-methylphenylamino)pyridin-2-yl)methylcarbamoyl)cyclopropyl)amid

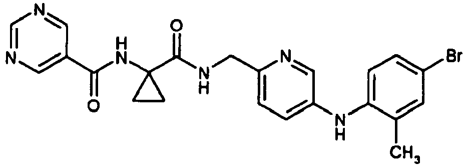

### 53a) 5-(4-Brom-2-(trifluormethyl)phenylamino)picolinonitril

Analog Vorschrift (40a) wurde ausgehend von 2-Cyan-5-fluorpyridin, 2-Amino-5-brombenzotrifluorid und Kalium-*tert*-butylat mit DMSO als Lösungsmittel die Titelverbindung hergestellt.
C₁₃H₇BrF₃N₃ (342.11)
Rₜ=2.50 min. Methode 2

### 53b-1) 6-(Aminomethyl)-N-(4-brom-2-methylphenyl)pyridin-3-amin 2,2,2-Trifluoracetat

Zu einer Lösung von 564 mg (1.65 mmol) 5-(4-Brom-2-(trifluormethyl)phenylamino)picolinsäurenitril in 5 mL THF wurden 1.65 mL (3.3 mmol) 2 molare Lithiumaluminiumhydrid-Lösung in THF gegeben. Die Reaktionsmischung wurde 30 Minuten bei Raumtemperatur gerührt und dann mit Wasser versetzt. Die Salze wurden abgesaugt und das Filtrat im Vakuum eingeengt. Der Rückstand wurde mittels HPLC (mit Lösungsmittelgradient, Acetonitril und Wasser mit 0.1% Trifluoressigsäure) gereinigt. Es entstanden 2 Produkte.
Ausbeute: 65% der Theorie
C₁₃H₁₄BrN₃*C₂HF₃O₂ (406.2)
Rₜ=1.63 min. Methode 2

### 53b-2) 6-(Aminomethyl)-N-(4-brom-2-trifluormethyl)phenyl)pyridin-3-amin

Ausbeute: 11 % der Theorie
C₁₃H₁₁Br_{F}F₃N₃ (346.15)
Rₜ=1.72 min. Methode 2

### 53c) Pyrimidin-5-carbonsäure-N-(1-((5-(4-brom-2-methylphenylamino)pyridin-2-yl)-methylcarbamoyl)cyclopropyl)amid

Analog Vorschrift (1d) wurde ausgehend von 6-(Aminomethyl)-N-(4-brom-2-methylphenyl)pyridin-3-amin 2,2,2-Trifluoracetat (aus 53b-1) und 1-[(Pyrimidin-5-carbonyl)-amino]-cyclopropancarbonsäure (aus 1b) die Titelverbindung hergestellt.
C₂₂H₂₁BrN₆O₂ (481.35)
Rₜ = 1.61 min. Methode 2

### Beispiel 54: Pyrimidin-5-carbonsäure-N-(1-((5-(4-brom-2-(trifluormethyl)phenylamino)-pyridin-2-yl)methylcarbamoyl)cyclopropyl)amid

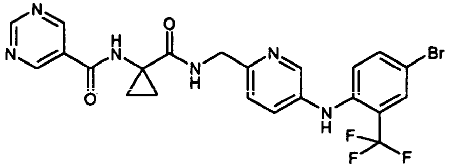

Analog Vorschrift (1d) wurde aus 6-(Aminomethyl)-N-(4-brom-2-(trifluormethyl)phenyl)-pyridin-3-amin (aus 53b-2) und 1-[(Pyrimidin-5-carbonyl)-amino]-cyclopropancarbonsäure (aus 1 b) die Titelverbindung hergestellt.
C₂₂H₁₈BrF₃N₆O₂ (535.32)
Rₜ = 1.82 min. Methode 2

### Beispiel 55: Pyrimidin-5-carbonsäure-N-(1-((5-(4-chlor-2-(trifluormethyl)phenylamino)-pyridin-2-yl)methylcarbamoyl)cyclopropyl)amid

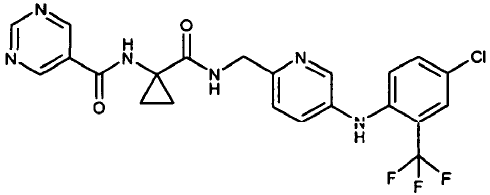

### 55a) 5-(4-Chlor-2-(trifluormethyl)phenylamino)picolinnitril

Die Reaktion fand unter Schutzgas (Stickstoff) statt. Zu einer Lösung von 100 mg (0.55 mmol) 5-Brom-2-cyanpyridin, 93 µL (0.66 mmol) 2-Amino-5-chlorbenzotrifluorid und 167 mg (0.77 mmol) Kaliumphosphat in 5 mL Toluol wurden 21 mg (0.04 mmol) Xantphos und 10 mg (0.01 mmol) Tris(dibenzylidenaceton)dipalladium gegeben. Man ließ über Nacht bei 110 °C rühren, filtrierte die Salze ab und engte das Filtrat im Vakuum zur Trockne ein. Der Rückstand wurde mittels HPLC (mit Fliessmittelgradient, Acetonitril und Wasser mit 0.1% Trifluoressigsäure) gereinigt.
Ausbeute: 68% der Theorie
C₁₃H₇ClF₃N₃ (297.66)
Rₜ = 2.53 min. Methode 2

### 55b) 6-(Aminomethyl)-N-(4-chlor-2-(trifluormethyl)phenyl)pyridin-3-amin

Analog Vorschrift (53b) wurde ausgehend von 5-(4-Chlor-2-(trifluormethyl)phenylamino)-picolinsäurenitril und 2 molarer Lithiumaluminiumhydrid-Lösung die Titelverbindung hergestellt.
C₁₃H₁₁ClF₃N₃ (301.69)
Rₜ = 1.72 min. Methode 2

### 55c) Pyrimidin-5-carbonsäure-N-(1-((5-(4-chlor-2-(trifluormethyl)phenylamino)pyridin-2-yl)methylcarbamoyl)cyclopropyl)amid

Analog Vorschrift (1d) wurde aus 6-(Aminomethyl)-N-(4-chlor-2-(trifluormethyl)phenyl)-pyridin-3-amin (aus 55b) und 1-[(Pyrimidin-5-carbonyl)-amino]-cyclopropancarbonsäure (aus 1 b) die Titelverbindung erhalten.
C₂₂H₁₈ClF₃N₆O₂ (490.87)
Rₜ = 1.76 min. Methode 2

### Beispiel 56: 5-Oxo-N-((S)-3-(4-(2-(trifluormethyl)phenylamino)benzylcarbamoyl)-tetrahydrofuran-3-yl)pyrrolidin-2-carboxamid

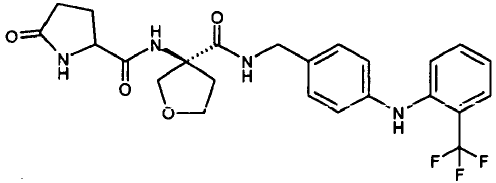

### 56a) (S)-Phenethyl-3-(tert-butoxycarbonylamino)tetrahydrofuran-3-carboxylat

Zu einer Lösung von 1.8 g (7.65 mmol) (S)-Phenethyl 3-aminotetrahydrofuran-3-carboxylat (aus 43a) in 30 mL Dichlormethan wurden 2 g (9.18 mmol) Di-*tert*-butyl-dicarbonat und 11.29 mL (9.18 mmol) TEA gegeben. Man ließ über Nacht bei Raumtemperatur rühren und gab dann nochmals Di-*tert*-butyldicarbonat und 50 mg Dimethylaminopyridin zu. Das Reaktionsgemisch wurde im Vakuum zur Trockne eingeengt und der Rückstand in 50 mL Dioxan aufgenommen und 6 Stunden bei 60°C gerührt. Das Lösungsmittel wurde abdestilliert und der Rückstand zwischen Essigsäureethylester und 0.5 molarer Kaliumhydrogensulfat-Lösung verteilt. Die organische Phase wurde mit Natriumhydrogensulfat-Lösung gewaschen, über Natriumsulfat getrocknet und im Vakuum zur Trockne eingeengt. Der Rückstand wurde über Kieselgel mit Petrolether/Essigsäureethylester im Verhältnis 4:1 gereinigt.
Ausbeute: 63% der Theorie
C₁₈H₂₅NO₅ (335.39)
Rₜ = 2.05 min. Methode 1

### 56b) (S)-3-tert-Butoxycarbonylamino)tetrahydrofuran-3-carbonsäure

Zu einer Lösung von 1.5 g (4.47 mmol) (S)-Phenethyl-3-(*tert*-butoxycarbonylamino)-tetrahydrofuran-3-carboxylat in 20 mL Ethanol wurden 8.94 mL (17.89 mmol) 2 molare Natronlauge gegeben. Man ließ 2 Stunden bei Raumtemperatur rühren und gab dann 8.94 mL (17.89 mmol) 2 molare Salzsäure zu. Der Ansatz wurde eingeengt, der Rückstand in Ethanol suspendiert und die Salze abgesaugt. Das Filtrat wurde vom Lösungsmittel befreit und roh weiter umgesetzt.
Ausbeute: 100% der Theorie
C₁₀H₁₇NO₅ (231.25)
Rₜ = 1.53 min. Methode 1

### 56c) (S)-tert-butyl-3-(4-(2-(trifluormethyl)phenylamino)benzylcarbamoyl)tetrahydrofuran-3-ylcarbamat

Analog Vorschrift (1d) wurde aus N-(4-(Aminomethyl)phenyl)-2-(trifluormethyl)anilin (aus 4a) und 1-[(Pyrimidin-5-carbonyl)-amino]-cyclopropancarbonsäure (aus 56b) die Titelverbindung erhalten.
C₂₄H₂₈F₃N₃O₄ (479.49)

### 56d) (S)-3-Amino-N-(4-(2-(trifluormethyl)phenylamino)benzyl)tetrahydrofuran-3-carboxamid

2 g (4.17 mmol) (S)-*tert*-Butyl-3-(4-(2-(trifluormethyl)phenylamino)benzylcarbamoyl)-tetrahydrofuran-3-ylcarbamat wurden in 15 mL eines 1:1 Gemisches aus Dichlormethan und Trifluoressigsäure 30 Minuten bei Raumtemperatur gerührt. Nach dem Einengen der Reaktionsmischung wurde der Rückstand in Dichlormethan gelöst, mit 4 molarer Natronlauge basisch gestellt und auf eine Phasentrennkartusche gegeben. Das Filtrat wurde vom Lösungsmittel befreit und das Rohprodukt über Kieselgel mit Cyclohexan / Essigsäureethylester im Verhältnis 1:1 und dann ein zweites Mal mit Dichlormethan / Methanol im Verhältnis 9:1 chromatographiert.
Ausbeute: 77% der Theorie
C₁₉H₂₀F₃N₃O₂ (379.38)
Rₜ = 1.97 min. Methode 6

### 56e) (S)-5-Oxo-N-(-3-(4-(2-(trifluormethyl)phenylamino)benzyl)carbamoyl)tetrahydrofuran-3-yl)pyrrolidin-2-carboxamid

Analog Vorschrift (1 d) wurde ausgehend von (S)-3-Amino-N-(4-(2-(trifluormethyl)phenylamino)benzyl)tetrahydrofuran-3-carboxamid (aus 56d) und 5-Oxopyrrolidin-2-carbonsäure die Titelverbindung hergestellt.
C₂₄H₂₅F₃N₄O₄ (490.49)
Rₜ = 1.84 min. Methode 5

Die nachfolgend aufgeführten Beispiele 57 bis 107 wurden analog der Durchführungsvorschrift (1d) aus (S)-3-Amino-N-(4-(2-(trifluormethyl)phenylamino)benzyl)tetrahydrofuran-3-carboxamid und den entsprechenden Säuren erhalten.

### Beispiel 57: (S)-6-Amino-N-{3-[4-(2-trifluormethyl-phenylamino)-benzylcarbamoyl]-tetrahydrofuran-3-yl}-nikotinamid

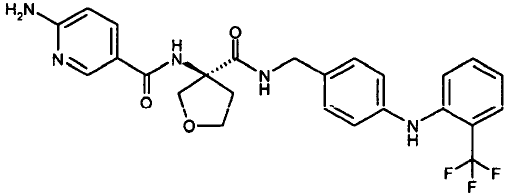

C₂₅H₂₄F₃N₅O₃ (499.5)
Rₜ = 1.66 min. Methode 5

### Beispiel 58: (S)-6-Methyl-N-{3-[4-(2-trifluormethyl-phenylamino)-benzylcarbamoyl]-tetrahydrofuran-3-yl}-nikotinamid

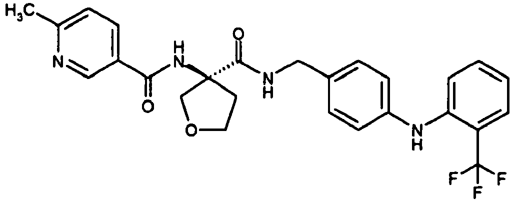

C₂₆H₂₅F₃N₄O₃ (498.5)
Rₜ = 1.69 min. Methode 5

### Beispiel 59: 3-(2-Pyridin-2-yl-acetylamino)-tetrahydrofuran-3-carbonsäure 4-(2-trifluormethyl-phenylamino)-benzylamid

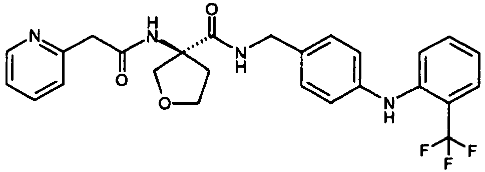

C₂₆H₂₅F₃N₄O₃ (498.5)
Rₜ = 1.64 min. Methode 5

### Beispiel 60: (S)-3-[(Tetrahydrofuran-3-carbonyl)-amino]-tetrahydrofuran-3-carbonsäure-4-(2-trifluormethyl-phenylamino)-benzylamid

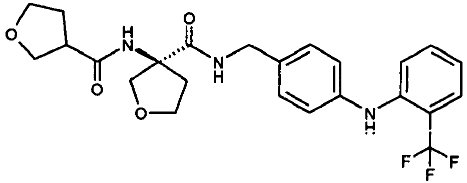

C₂₄H₂₆F₃N₃O₄ (477.5)
Rₜ = 1.96 min. Methode 5

### Beispiel 61: (S)-2-Chlor-N-{3-[4-(2-trifluormethyl-phenylamino)-benzylcarbamoyl]-tetrahydrofuran-3-yl}-isonikotinamid

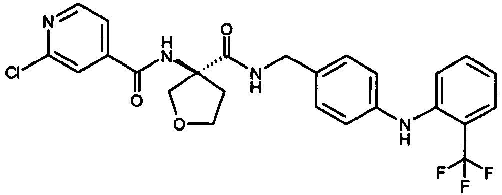

C₂₅H₂₂ClF₃N₄O₃ (518.9)
Rₜ = 2.15 min. Methode 5

### Beispiel 62: (S)-6-Oxo-1,6-dihydro-pyridazin-3-carbonsäure {3-[4-(2-trifluormethylphenylamino)-benzylcarbamoyl]-tetrahydrofuran-3-yl}-amid

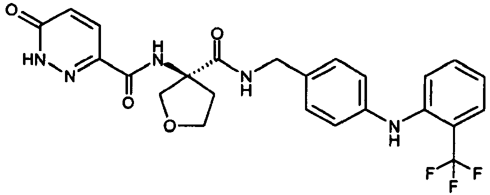

C₂₄H₂₂F₃N₅O₄ (501.5)
Rₜ = 1.91 min. Methode 5

### Beispiel 63: (S)-2-Amino-N-{3-[4-(2-trifluormethyl-phenylamino)-benzylcarbamoyl]-tetrahydrofuran-3-yl}-isonikotinamid

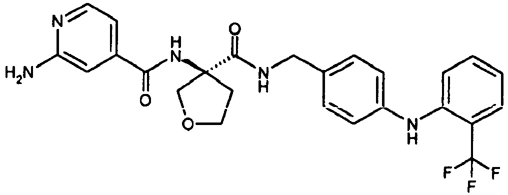

C₂₅H₂₄F₃N₅O₃ (499.5)
Rₜ = 1.64 min. Methode 5

### Beispiel 64: (S)-Pyridazin-4-carbonsäure {3-[4-(2-trifluormethyl-phenylamino)-benzylcarbamoyl]-tetrahydrofuran-3-yl}-amid

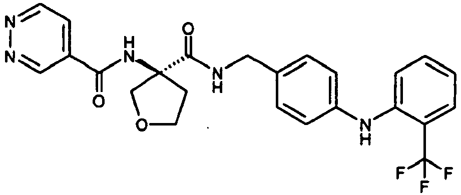

C₂₄H₂₂F₃N₅O₃ (485.5)
Rₜ = 1.92 min. Methode 5

### Beispiel 65: (S)-Tetrahydropyran-4-carbonsäure {3-[4-(2-trifluormethyl-phenylamino)-benzylcarbamoyl]-tetrahydrofuran-3-yl}-amid

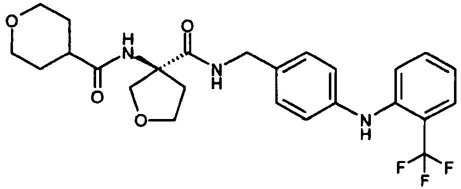

C₂₅H₂₈F₃N₃O₄ (491.5)
Rₜ = 1.97 min. Methode 5

### Beispiel 66: (S)-3-(2-Cyan-2-hydroxyimino-acetylamino)-tetrahydrofuran-3-carbonsäure-4-(2-trifluormethyl-phenylamino)-benzylamid

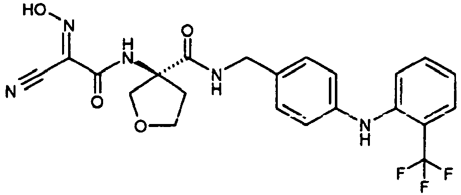

C₂₂H₂₀F₃N₅O₄ (475.4)
Rₜ = 2.07 min. Methode 5

### Beispiel 67: (S)-6-Chlor-pyridin-2-carbonsäure-{3-[4-(2-trifluormethyl-phenylamino)-benzylcarbamoyl]-tetrahydrofuran-3-yl}-amid

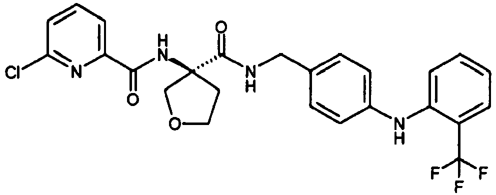

C₂₅H₂₂ClF₃N₄O₃ (518.9)
Rₜ = 2.25 min. Methode 5

### Beispiel 68: (S)-5-Methoxy-furan-2-carbonsäure-{3-[4-(2-trifluormethyl-phenylamino)-benzylcarbamoyl]-tetrahydrofuran-3-yl}-amid

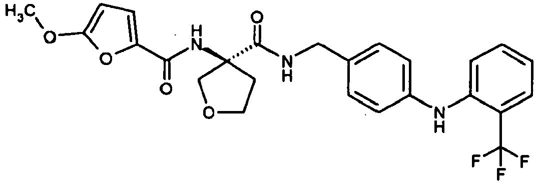

C₂₅H₂₄F₃N₃O₅ (503.5)
Rₜ = 2.12 min. Methode 5

### Beispiel 69: (S)-3-[(3-Oxo-cyclohexancarbonyl)-amino]-tetrahydrofuran-3-carbonsäure-4-(2-trifluormethyl-phenylamino)-benzylamid

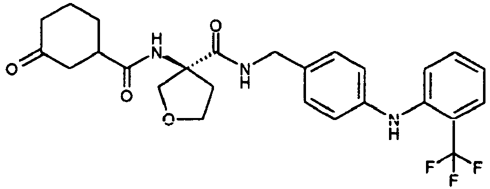

C₂₆H₂₈F₃N₃O₄ (503.5)
Rₜ = 2.00 min. Methode 5

### Beispiel 70: (S)-6-Hydroxy-pyridin-2-carbonsäure-{3-[4-(2-trifluormethyl-phenylamino)-benzylcarbamoyl]-tetrahydrofuran-3-yl}-amid

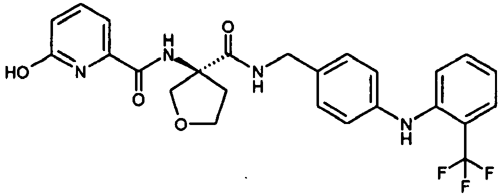

C₂₅H₂₃F₃N₄O₄ (500.5)
Rₜ = 1.94 min. Methode 5

### Beispiel 71: (S)-1-Methyl-5-oxo-pyrrolidin-3-carbonsäure {3-[4-(2-trifluormethyl-phenylamino)benzylcarbamoyl]-tetrahydrofuran-3-yl}-amid

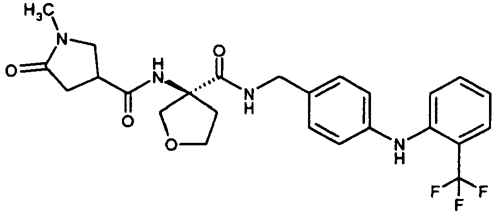

C₂₅H₂₇F₃N₄O₄ (504.5)
Rₜ = 1.85 min. Methode 5

### Beispiel 72: (S)-6-Amino-pyridin-2-carbonsäure {3-[4-(2-trifluormethyl-phenylamino)-benzylcarbamoyl]-tetrahydrofuran-3-yl}-amid

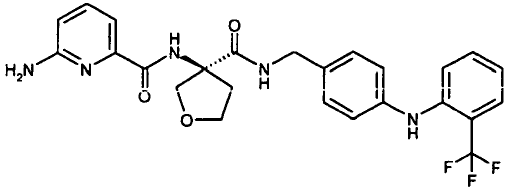

C₂₅H₂₄F₃N₅O₃ (499.5)
Rₜ = 1.72 min. Methode 5

### Beispiel 73: (S)-5-Hydroxy-1H-pyrazol-3-carbonsäure {3-[4-(2-trifluormethyl-phenylamino)-benzylcarbamoyl]-tetrahydrofuran-3-yl}-amid

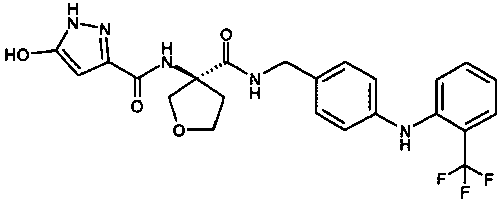

C₂₃H₂₂F₃N₅O₄ (489.5)
Rₜ = 1.89 min. Methode 5

### Beispiel 74: (S)-Pyridazin-3-carbonsäure {3-[4-(2-trifluormethyl-phenylamino)-benzylcarbamoyl]-tetrahydrofuran-3-ylyamid

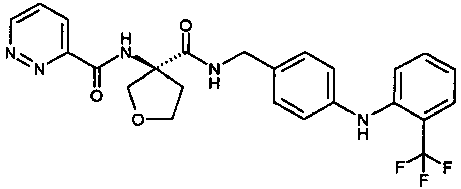

C₂₄H₂₂F₃N₅O₃ (485.5)
Rₜ = 2.02 min. Methode 5

### Beispiel 75: (S)-3-[(3-Methoxy-cyclobutancarbonyl)-amino]-tetrahydrofuran-3-carbonsäure-4-(2-trifluormethyl-phenylamino)-benzylamid

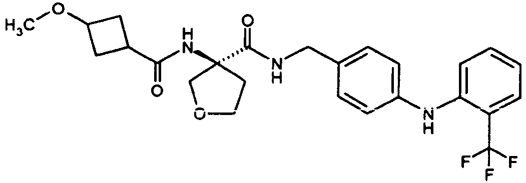

C₂₅H₂₈F₃N₃O₄ (491.5)
Rₜ = 2.02 min. Methode 5

### Beispiel 76: (S)-6-Oxo-piperidin-3-carbonsäure-{3-[4-(2-trifluormethyl-phenylamino)-benzylcarbamoyl]-tetrahydrofuran-3-yl}-amid

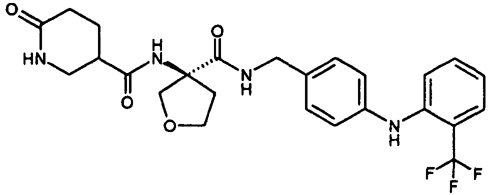

C₂₅H₂₇F₃N₄O₄ (504.5)
Rₜ = 1.81 min. Methode 5

### Beispiel 77: (S)-4-Methyl-pyrimidin-5-carbonsäure-{3-[4-(2-trifluormethyl-phenylamino)-benzylcarbamoyl]-tetrahydrofuran-3-yl}-amid

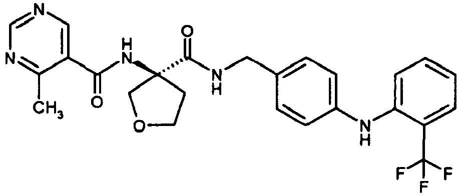

C₂₅H₂₄F₃N₅O₃ (499.5)
Rₜ = 1.96 min. Methode 5

### Beispiel 78: (S)-3-[(3-Oxo-cyclopentancarbonyl)-amino]-tetrahydrofuran-3-carbonsäure-4-(2-trifluormethyl-phenylamino)-benzylamid

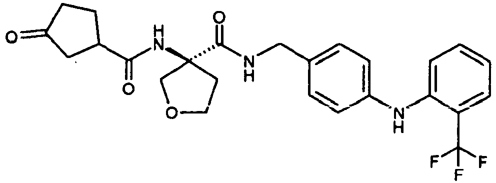

C₂₅H₂₆F₃N₃O₄ (489.5)
Rₜ = 1.97 min. Methode 5

### Beispiel 79: (S)-2-Methoxy-N-{3-[4-(2-trifluormethyl-phenylamino)-benzylcarbamoyl]-tetrahydrofuran-3-yl}-isonikotinamid

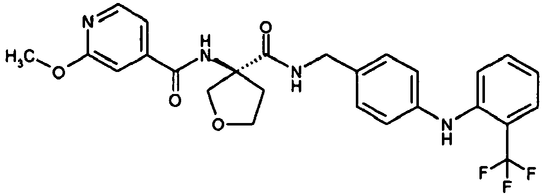

C₂₆H₂₅F₃N₄O₄ (514.5)
Rₜ = 2.11 min. Methode 5

### Beispiel 80: (S)-2,4-Dimethyl-pyrimidin-5-carbonsäure-{3-[4-(2-trifluormethyl-phenylamino)-benzylcarbamoyl]-tetrahydrofuran-3-yl}-amid

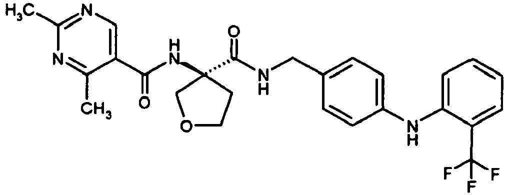

C₂₆H₂₆F₃N₅O₃ (513.5)
Rₜ = 1.95 min. Methode 5

### Beispiel 81: (S)-2-Methoxy-pyrimidin-5-carbonsäure-{3-[4-(2-trifluormethyl-phenylamino)-benzylcarbamoyl]-tetrahydrofuran-3-yl}-amid

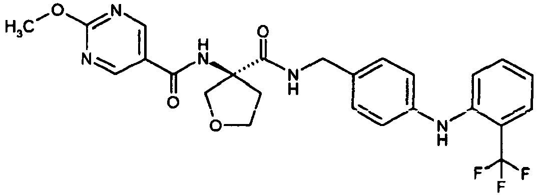

C₂₅H₂₄F₃N₅O₄ (515.5)
Rₜ = 2.04 min. Methode 5

### Beispiel 82: (S)-2-Methylamino-pyrimidin-5-carbonsäure-{3-[4-(2-trifluormethyl-phenylamino)-benzylcarbamoyl]-tetrahydrofuran-3-yl}-amid

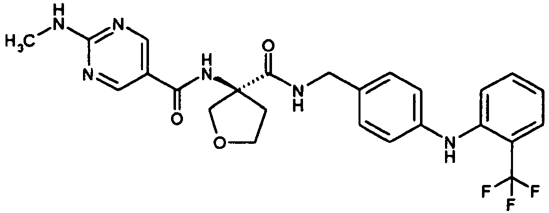

C₂₅H₂₅F₃N₆O₃ (514.5)
Rₜ = 1.91 min. Methode 5

### Beispiel 83: (S)-2-Methyl-pyrimidin-5-carbonsäure-{3-[4-(2-trifluormethyl-phenylamino)-benzylcarbamoyl]-tetrahydrofuran-3-yl}-amid

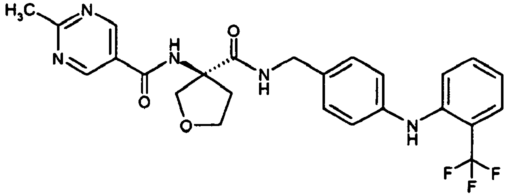

C₂₅H₂₄F₃N₅O₃ (499.5)
Rₜ = 1.97 min. Methode 5

### Beispiel 84: (S)-1-Methyl-2-oxo-1,2-dihydro-pyridin-4-carbonsäure-{3-[4-(2-trifluormethyl-phenylamino)-benzylcarbamoyl]-tetrahydrofuran-3-yl}-amid

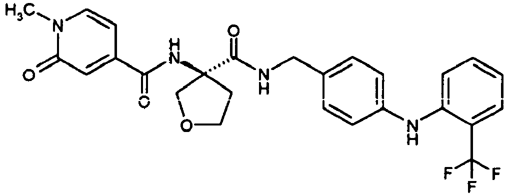

C₂₆H₂₅F₃N₄O₄ (514.5)
Rₜ = 1.90 min. Methode 5

### Beispiel 85: (S)-Oxazol-5-carbonsäure-{3-[4-(2-trifluormethyl-phenylamino)-benzylcarbamoyl]-tetrahydrofuran-3-yl}-amid

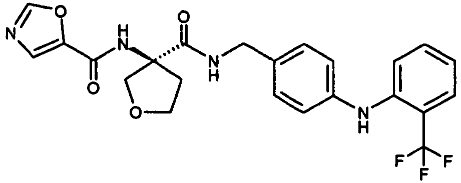

C₂₃H₂₁F₃N₄O₄ (474.4)
Rₜ = 1.97 min. Methode 5

### Beispiel 86: (S)-2-Hydroxy-N-{3-[4-(2-trifluormethyl-phenylamino)-benzylcarbamoyl]-tetrahydrofuran-3-yl}-isonikotinamid

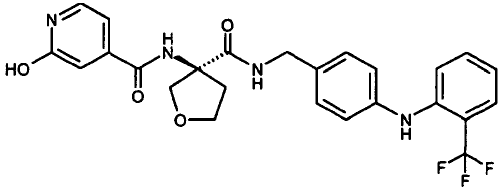

C₂₅H₂₃F₃N₄O₄ (500.5)
Rₜ = 1.85 min. Methode 5

### Beispiel 87: (S)-5-Hydroxy-N-{3-[4-(2-trifluormethyl-phenylamino)-benzylcarbamoyl]-tetrahydrofuran-3-yl}-nikotinamid

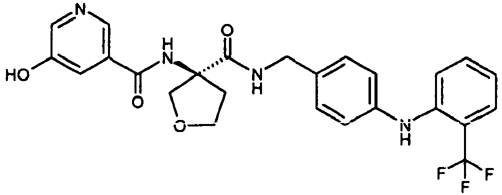

C₂₅H₂₃F₃N₄O₄ (500.5)
Rₜ = 1.72 min. Methode 5

### Beispiel 88: (S)-1-Methyl-1H-[1,2,3]triazol-4-carbonsäure {3-[4-(2-trifluormethyl-phenylamino)-benzylcarbamoyl]-tetrahydrofuran-3-yl}-amid

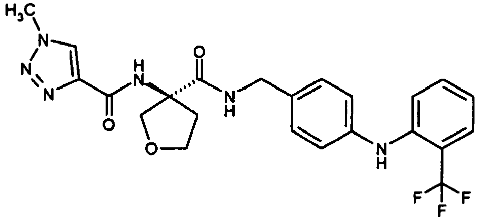

C₂₃H₂₃F₃N₆O₃ (488.5)
Rₜ = 1.99 min. Methode 5

### Beispiel 89: (S)-Thiazol-5-carbonsäure-{3-[4-(2-trifluormethyl-phenylamino)-benzyl-carbamoyl]-tetrahydrofuran-3-yl}-amid

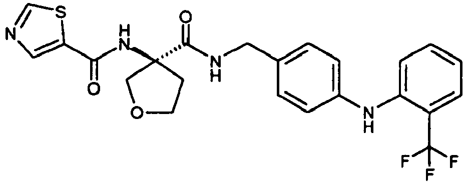

C₂₃H₂₁F₃N₄O₃S (490.5)
Rₜ = 2.01 min. Methode 5

### Beispiel 90: (S)-2-Hydroxy-pyrimidin-5-carbonsäure-{3-[4-(2-trifluormethyl-phenyl-amino)-benzylcarbamoyl]-tetrahydrofuran-3-yl}-amid

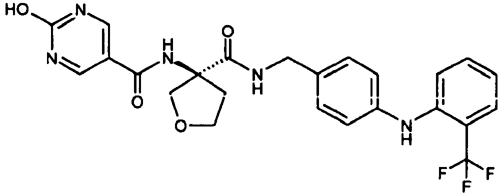

C₂₄H₂₂F₃N₅O₄ (501.5)
Rₜ = 1.79 min. Methode 5

### Beispiel 91: (S)-3-(3,3,3-Trifluor-2-methyl-propionylamino)-tetrahydrofuran-3-carbonsäure-4-(2-trifluormethyl-phenylamino)-benzylamid

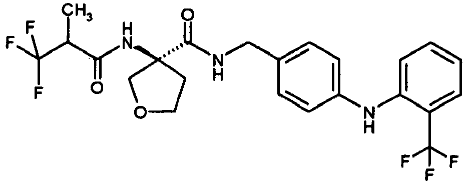

C₂₃H₂₃F₆N₃O₃ (503.4)
Rₜ = 2.18 min. Methode 5

### Beispiel 92: (S)-5-Methoxy-N-{3-[4-(2-trifluormethyl-phenylamino)-benzylcarbamoyl]-tetrahydrofuran-3-yl}-nikotinamid

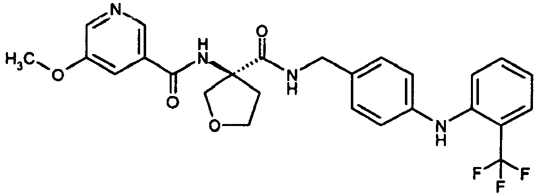

C₂₆H₂₅F₃N₄O₄ (514.5)
Rₜ = 1.85 min. Methode 5

### Beispiel 93: (S)-Furan-3-carbonsäure {3-[4-(2-trifluormethyl-phenylamino)-benzyl-carbamoyl]-tetrahydrofuran-3-yl}-amid

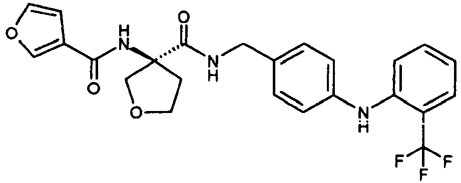

C₂₄H₂₂F₃N₃O₄ (473.4)
Rₜ = 2.09 min. Methode 5

### Beispiel 94: (S)-Furan-2-carbonsäure {3-[4-(2-trifluormethyl-phenylamino)-benzyl-carbamoyl]-tetrahydrofuran-3-yl}-amid

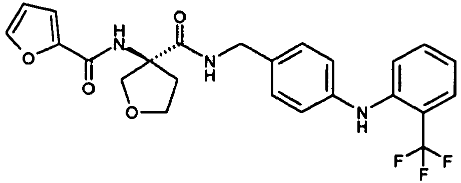

C₂₄H₂₂F₃N₃O₄ (473.4)
Rₜ = 2.08 min. Methode 5

### Beispiel 95: (S)-N-{3-[4-(2-Trifluormethyl-phenylamino)-benzylcarbamoyl]-tetrahydrofuran-3-yl}-isonikotinamid

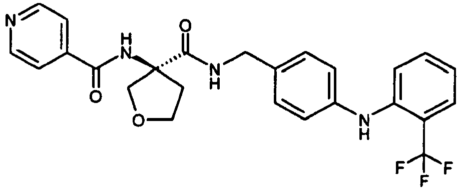

C₂₅H₂₃F₃N₄O₃ (484.5)
Rₜ = 1.71 min. Methode 5

### Beispiel 96: (S)-Pyrazin-2-carbonsäure-{3-[4-(2-trifluormethyl-phenylamino)-benzylcarbamoyl]-tetrahydrofuran-3-yl}-amid

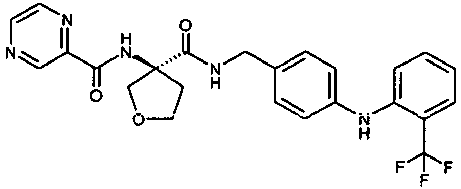

C₂₄H₂₂F₃N₅O₃ (485.5)
Rₜ = 2.06 min. Methode 5

### Beispiel 97: (S)-3-(3-Hydroxy-benzoylamino)-tetrahydrofuran-3-carbonsäure-4-(2-trifluormethyl-phenylamino)-benzylamid

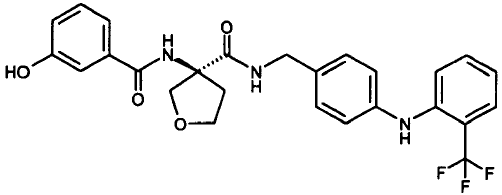

C₂₆H₂₄F₃N₃O₄ (499.5)
Rₜ = 2.03 min. Methode 5

### Beispiel 98: (S)-6-Hydroxy-N-{3-[4-(2-trifluormethyl-phenylamino)-benzylcarbamoyl]-tetrahydrofuran-3-yl}-nikotinamid

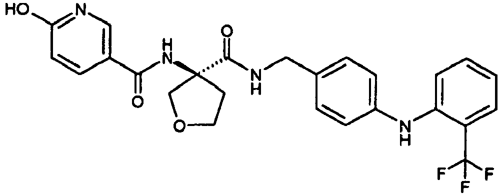

C₂₅H₂₃F₃N₄O₄ (500.5)
Rₜ = 1.84 min. Methode 5

### Beispiel 99: (S)-3-(4-Methoxy-benzoylamino)-tetrahydrofuran-3-carbonsäure-4-(2-trifluormethyl-phenylamino)-benzylamid

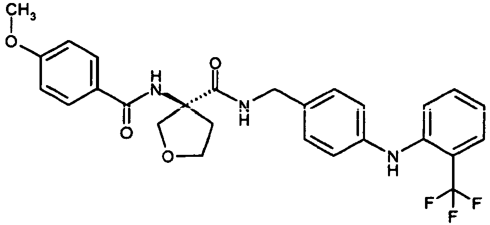

C₂₇H₂₆F₃N₃O₄ (513.5)
Rₜ = 2.16 min. Methode 5

### Beispiel 100: (S)-3-(3-Methoxy-benzoylamino)-tetrahydrofuran-3-carbonsäure-4-(2-trifluormethyl-phenylamino)-benzylamid

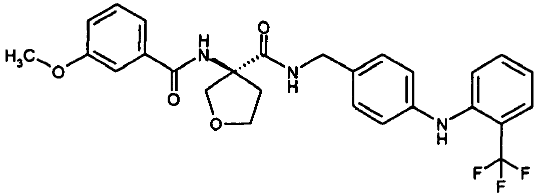

C₂₇H₂₆F₃N₃O₄ (513.5)
Rₜ = 2.18 min. Methode 5

### Beispiel 101: (S)-3-(2-Methoxy-benzoylamino)-tetrahydrofuran-3-carbonsäure-4-(2-trifluormethyl-phenylamino)-benzylamid

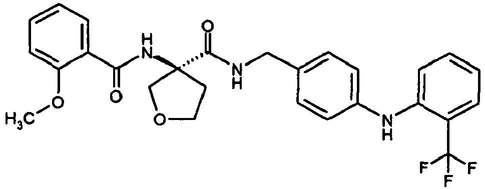

C₂₇H₂₆F₃N₃O₄ (513.5)
Rₜ = 2.22 min. Methode 5

### Beispiel 102: (S)-3-(3,5-Dihydroxy-benzoylamino)-tetrahydrofuran-3-carbonsäure-4-(2-trifluormethyl-phenylamino)-benzylamid

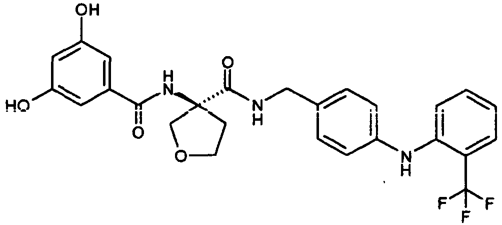

C₂₆H₂₄F₃N₃O₅ (515.5)
Rₜ = 1.93 min. Methode 5

### Beispiel 103: (S)-3-(3,5-Dimethoxy-benzoylamino)-tetrahydrofuran-3-carbonsäure-4-(2-trifluormethyl-phenylamino)-benzylamid

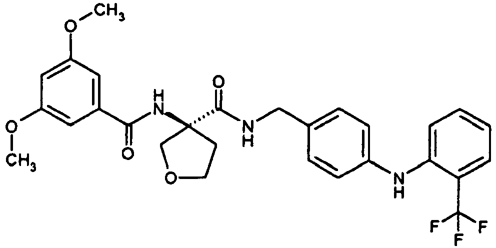

C₂₈H₂₈F₃N₃O₅ (543.5)
Rₜ = 2.20 min. Methode 5

### Beispiel 104: (S)-3-(2-Pyridin-3-yl-acetylamino)-tetrahydrofuran-3-carbonsäure-4-(2-trifluormethyl-phenylamino)-benzylamid

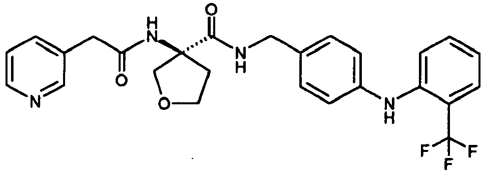

C_{26H25}F₃N₄O₃ (498.5)
Rₜ = 1.64 min. Methode 5

### Beispiel 105: (S)-5-Amino-N-{3-[4-(2-trifluormethyl-phenylamino)-benzylcarbamoyl]-tetrahydrofuran-3-yl}-nikotinamid

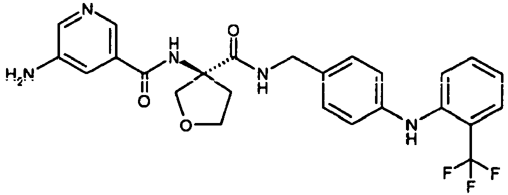

C₂₅H₂₄F₃N₅O₃ (499.5)
Rₜ = 1.65 min. Methode 5

### Beispiel 106: (S)-1H-Pyrazol-3-carbonsäure {3-[4-(2-trifluormethyl-phenylamino)-benzyl-carbamoyl]-tetrahydrofuran-3-yl}-amid

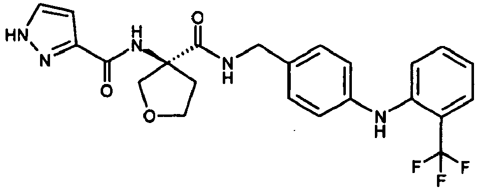

C₂₃H₂₂F₃N₅O₃ (473.5)
Rₜ = 1.96 min. Methode 5

### Beispiel 107: (S)-6-Fluor-N-{3-[4-(2-trifluormethyl-phenylamino)-benzylcarbamoyl]-tetrahydrofuran-3-yl}-nikotinamid

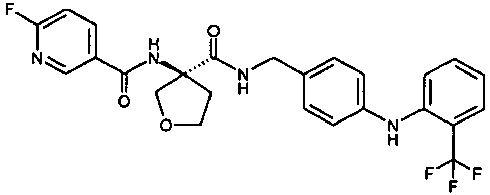

C₂₅H₂₂F₄N₄O₃ (502.5)
Rₜ = 2.10min. Methode 5

### Beispiel 108: 1-(3-Ethylureido)-N-(4-(2-(trifluormethyl)phenylamino)benzyl)cyclopropan-carboxamid

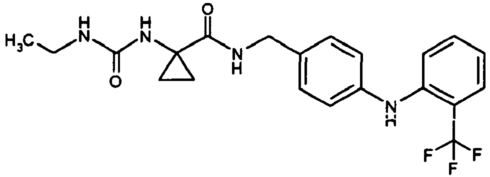

Eine Lösung von 55 mg (0.16 mmol) 1-Amin-N-(4-(2-(trifluormethyl)phenylamino)benzyl)-cyclopropancarboxamid (aus 5a) in 2 mL Dichlormethan wurde mit 68 µL (0.49 mmol) TEA und 16 µL (0.2 mmol) Ethylisocyanat versetzt und über Nacht bei Raumtemperatur gerührt. Es wurde noch dreimal Ethylisocyanat zugegeben und weiter bei Raumtemperatur bzw. bei 60°C gerührt. Anschließend wurde das Reaktionsgemisch im Vakuum zur Trockne eingeengt und über eine RP-HPLC-MS mit Fließmittel-Gradient (Wasser/Acetonitril = 1:1 bis 1:20 + 0.1% Trifluoressigsäure) gereinigt.
Ausbeute: 79% der Theorie
C₂₁H₂₃ F₃N₄O₂ (420.43)
Rₜ = 2.27 min. Methode 5

### Beispiel 109: Pyrimidin-5-carbonsäure-N-(1-(4-(methyl(phenyl)amino)benzylcarbamoyl)-cyclopropyl)amid

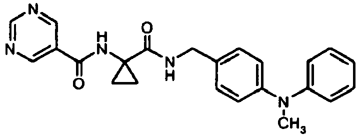

### 109a) 4-(Aminomethyl)-N-methyl)-N-phenylanilin

Analog Vorschrift (1c) wurde aus N-Methylanilin und 4-Fluorbenzonitril die Titelverbindung hergestellt.
C₁₄H₁₆N₂ (212.3)

### 109b) Pyrimidin-5-carbonsäure-N-(1-(4-(methyl(phenyl)amino)benzylcarbamoyl)-cyclopropyl)amid

Analog Vorschrift (1d) wurde ausgehend von 4-(Aminomethyl)-N-methyl-N-phenylanilin (aus 109a) und 1-[(Pyrimidin-5-carbonyl)-amino]-cyclopropan-carbonsäure (aus 1b) die Titelverbindung hergestellt.
C₂₃H₂₃N₅O₂ (401.47)
R ₜ= 2.87 min. Methode 5

### Beispiel 110: Pyrimidin-5-carbonsäure- N-(1-(4-((2-chlorphenyl)(methyl)amino)-benzylcarbamoyl)cyclopropyl)amid

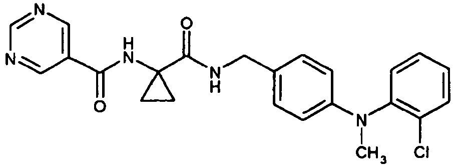

### 110a) N-(4-(Aminomethyl)phenyl)-2-chlor-N-methylanilin

Analog Beispiel (1 c) wurde ausgehend von 2-Chlor-N-methylanilin und 4-Fluorbenzonitril die Titelverbindung hergestellt.
C₁₄H₁₅ClN₂ (246.74)

### 110b) Pyrimidin-5-carbonsäure-N-(1-(4-((2-chlorphenyl)(methyl)amino)benzylcarbamoyl)-cyclopropyl)amid

Analog Beispiel (1d) wurde ausgehend von N-(4-(Aminomethyl)phenyl)2-chlor-N-methylanilin (aus 110a) und 1-[(Pyrimidin-5-carbonyl)-amino]-cyclopropan-carbonsäure (aus 1b) die Titelverbindung hergestellt.
C₂₃H₂₂ClN₅O₂ (435.91)
Rₜ = 2.23 min. Methode 11

### Beispiel 111: Pyrimidin-5-carbonsäure-N-(1-(4-(ethyl(phenyl)amino)benzylcarbamoyl)-cyclopropyl)amid

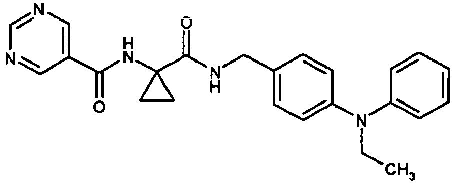

### 111a) 4-(Aminomethyl)-N-ethy)-N-phenylanilin

Analog Vorschrift (1 c) wurde ausgehend von N-Ethylanilin und 4-Fluorbenzonitril die Titelverbindung hergestellt.
C₁₅H₁₈N₂ (226.32)

### 111 b) Pyrimidin-5-carbonsäure-N-(1-(4-(ethyl(phenyl)amino)benzylcarbamoyl)-cyclopropyl)amid

Analog Vorschrift (1d) wurde aus 4-(Aminomethyl)-N-ethyl-N-phenylanitin (aus 111a) und 1-[(Pyrimidin-5-carbonyl)-amino]-cyclopropan-carbonsäure (aus 1b) die Titelverbindung hergestellt.
C₂₄H₂₅N₅O₂ (415.49)
Rₜ = 2.26 min. Methode 11

### Beispiel 112: Pyrimidin-5-carbonsäure-N-(1-(4-((4-methoxyphenyl)(methyl)amino)benzyl-carbamoyl)cyclopropyl)amid

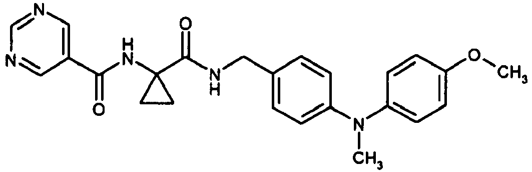

### 111a) 4-(Aminomethyl)-N-(4-methoxyphenyl)-N-methylanilin

Analog Vorschrift (1 c) wurde ausgehend von 4-Methoxy-N-methylanilin und 4-Fluor-benzonitril unter Verwendung von Raney-Nickel die Titelverbindung hergestellt.
C₁₅H₁₈N₂O (242.32)

### 112b) Pyrimidin-5-carbonsäure-N-(1-(4-((4-methoxyphenyl)(methyl)amino)benzyl-carbamoyl)cyclopropyl)amid

Analog Vorschrift (1 d) wurde ausgehend von 4-(Aminomethyl)-N-(4-methoxyphenyl)-N-methylanilin (aus 112a) und 1-[(Pyrimidin-5-carbonyl)-amino]-cyclopropan-carbonsäure (aus 1b) die Titelverbindung erhalten.
C₂₄H₂₅N₅O₃ (431.49)
Rₜ = 2.13min. Methode 11

### Beispiel 113: Pyrimidin-5-carbonsäure-N-(1-(4-(methyl(o-tolyl)amino)benzylcarbamoyl)-cyclopropyl)amid

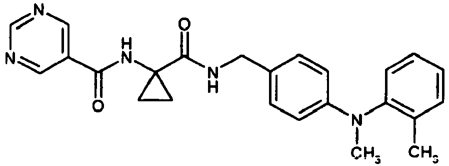

### 113a) N-(4-(Aminomethyl)phenyl)-2-chlor-N-methylanilin

Analog Vorschrift (1c) wurden N-Methyl-o-toluidin und 4-Fluorbenzonitril unter Verwendung Raney-Nickel zur Titelverbindung umgesetzt.
C₁₅H₁₈N₂ (226.32)

### 113b) Pyrimidin-5-carbonsäure-N-(1-(4-(methyl(o-tolyl)amino)benzylcarbamoyl)-cyclopropyl)amid

Analog Vorschrift (1 d) wurde aus N-(4-(Aminomethyl)phenyl)-2-chlor-N-methylanilin (aus 113a) und 1-[(Pyrimidin-5-carbonyl)-amino]-cyclopropan-carbonsäure (aus 1b) die Titelverbindung hergestellt.
C₂₄H₂₅N₅O₂ (415.49)
Rₜ = 2.26 min. Methode 11

### Beispiel 114: Pyrimidin-5-carbonsäure-{1-[4-(2-cyan-5-fluor-phenylamino)-benzyl-carbamoyl]-cyclopropyl}-amid

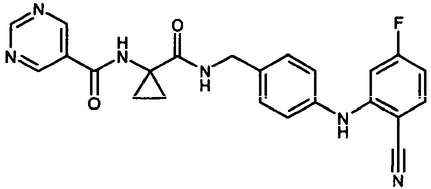

### 114a) [4-(2-Cyan-5-fluor-phenylamino)-benzyl]-carbaminsäure-tert-butylester

Herstellung in Analogie zur Vorschrift (55a) aus 2-Brom-4-fluor-benzonitril und (4-Amino-benzyl)-carbaminsäure-tert-butylester.
Ausbeute: 60% der Theorie
C₁₉H₂₀FN₃O₂ (341.38)
Rₜ = 2.65 min. Methode 12

### 114b) 2-(4-Aminomethyl-phenylamino)-4-fluor-benzonitril Di-trifluoracetat

92 mg (0.27 mmol) [4-(2-Cyan-5-fluor-phenylamino)benzyl]-carbaminsäure-*tert*-butylester wurden in 1 mL Trifluoressigsäure und 5 mL Dichlormethan 1 Stunde bei Raumtemperatur gerührt. Anschließend wurde die Reaktionsmischung im Vakuum bis zur Trockne eingedampft.
Ausbeute: 96% der Theorie
C₁₄H₁₂FN₃ * 2 C₂HF₃O₂ (469.31)
Rₜ = 1.41 min. Methode 12

### 114c) Pyrimidin-5-carbonsäure {1-[4-(2-cyan-5-fluor-phenylamino)-benzylcarbamoyl]-cyclopropyl}-amid

Zu einer Lösung von 54 mg (0.26 mmol) 1-[(Pyrimidin-5-carbonyl)-amino]-cyclopropancarbonsäure in 5 mL DMF wurden 83 mg (0.21 mmol) TBTU, 146 µL (1.0 mmol) Triethylamin und 122 mg (0.21 mmol) 2-(4-Aminomethyl-phenylamino)-4-fluor-benzonitril Di-trifluoracetat gegeben. Man ließ über Nacht bei Raumtemperatur rühren und destillierte dann die Lösungsmittel im Vakuum ab. Der Rückstand wurde chromatographisch gereinigt (RP mit Fließmittel-Gradient, Acetonitril und Wasser mit 0.1% Trifluoressigsäure).
Ausbeute: 69% der Theorie
C₂₃H₁₉FN₆O₂ (430.44)
Rₜ = 1.91 min. Methode 12

### Beispiel 115: Pyrimidin-5-carbonsäure {1-[4-(2-cyan-3-fluor-phenylamino)-benzyl-carbamoyl]-cyclopropyl}-amid

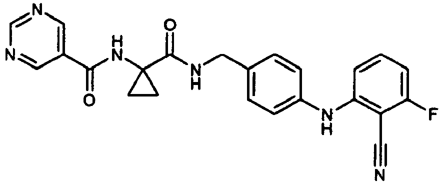

### 115a) 4-(2-Cyan-3-fluor-phenylamino)-benzyl]-carbaminsäure-tert-butylester

Die Titelverbindung wurde aus 2-Brom-6-fluor-benzonitril und (4-Amino-benzyl)-carbaminsäure-*tert*-butylester analog zu Vorschrift (55a) erhalten.
C₁₉H₂₀FN₃O₂ (341.38)
Rₜ = 2.65 min. Methode 12

### 115b) 2-(4-Aminomethyl-phenylamino)-6-fluor-benzonitril Di-trifluoracetat

Aus [4-(2-Cyan-3-fluor-phenylamino)-benzyl]-carbaminsäure-*tert*-butylester wurde die Titelverbindung analog zu Vorschrift (114b) hergestellt.
C₁₄H₁₂FN₃ * 2 C₂HF₃O₂ (469.31)
Rₜ = 1.46 min. Methode 12

### 115c) Pyrimidin-5-carbonsäure-{1-[4-(2-cyan-3-fluor-phenylamino)-benzylcarbamoyl]-cyclopropyl}-amid

Die Titelverbindung wurde analog zu Vorschrift (114c) aus 2-(4-Aminomethyl-phenyl-amino)-6-fluor-benzonitril Di-trifluoracetat und 1-[(Pyrimidin-5-carbonyl)-amino]-cyclopropancarbonsäure erhalten.
Ausbeute: 44% der Theorie
C₂₃H₁₉FN₆O₂ (430.44)
Rₜ = 1.94 min. Methode 12

### Beispiel 116: Pyrimidin-5-carbonsäure-{1-[4-(2-cyan-6-fluorphenylamino)-benzyl-carbamoyl]-cyclopropyl}-amid

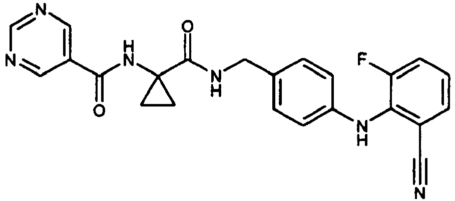

### 116a) [4-(2-Cyan-6-fluor-phenylamino)-benzyl]-carbamidsäure-tert-butylester

Die Titelverbindung wurde aus 2-Brom-3-fluor-benzonitril und (4-Amino-benzyl)-carbaminsäure-tert-butylester nach Vorschrift (55a) hergestellt.
C₁₉H₂₀FN₃O₂ (341.38)
Rₜ = 2.50 min. Methode 12

### 116b) 2-(4-Aminomethyl-phenylamino)-3-fluor-benzonitril Di-trifluoracetat

Herstellung der Titelverbindung aus [4-(2-Cyan-6-fluor-phenylamino)-benzyl]-carbaminsäure-tert-butylester analog zu Vorschrift (114b).
C₁₄H₁₂FN₃ * 2 C₂HF₃O₂ (469.31)
Rₜ = 1.27 min. Methode 12

### 116c) Pyrimidin-5-carbonsäure{1-[4-(2-cyan-6-fluor-phenylamino)-benzylcarbamoyl]-cyclopropyl}-amid

Die Titelverbindung wurde analog zu Vorschrift (114c) aus 2-(4-Aminomethyl-phenyl-amino)-3-fluor-benzonitril Di-trifluoracetat und 1-[(Pyrimidin-5-carbonyl)-amino]-cyclopropancarbonsäure hergestellt.
C₂₃H₁₉FN₆O₂ (430.44)
Rₜ = 1.78 min. Methode 12

### Beispiel 117: Pyrimidin-5-carbonsäure {1-[4-(4-ethoxy-2-trifluormethyl-phenylamino)-benzylcarbamoyl]-cyclopropyl}-amid

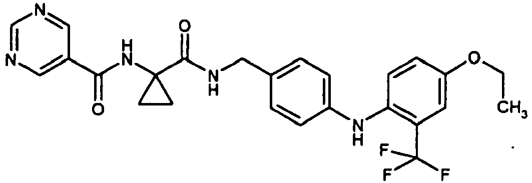

### 117a) 4-(4-Ethoxy-2-trifluormethyl-phenylamino)-benzonitril

276 mg (2.28 mmol) 4-Fluor-benzonitril und 550 mg (2.28 mmol) 4-Ethoxy-2-trifluormethyl-phenylamin-Hydrochlorid wurden in 10 mL DMSO gelöst und unter Eiskühlung mit 639 mg (5.69 mmol) Kalium-tert-butylat versetzt. Die Reaktionsmischung wurde über Nacht bei Raumtemperatur gerührt, mit Wasser verdünnt und mit Diethylether extrahiert. Die organische Phase wurde mit Natriumsulfat getrocknet und eingeengt. Der Rückstand wurde chromatographisch über Kieselgel gereinigt (Petrolether/Essigsäureethylester = 9:1).
Ausbeute: 20% der Theorie
C₁₆H₁₃F₃N₂O (306.28)
Massenspektroskopie [M+H]⁺ = 307

### 117b) (4-Aminomethyl-phenyl)-(4-ethoxy-2-trifluormethyl-phenyl)-amin

140 mg (0.46 mmol) 4-(4-Ethoxy-2-trifluormethyl-phenylamino)-benzonitril in 10 mL methanolischem Ammoniak wurden mit Raney-Nickel als Katalysator bei 50 psi Wasserstoffdruck hydriert. Der Katalysator wurde abfiltriert und das Filtrat vom Lösungsmittel befreit.
C₁₆H₁₇F₃N₂O (310.31)

### 117c) Pyrimidin-5-carbonsäure {1-[4-(4-ethoxy-2-trifluormethyl-phenylamino)-benzyl-carbamoyl]-cyclopropyl}-amid

1-[(Pyrimidin-5-carbonyl)-amino]-cyclopropancarbonsäure und (4-Aminomethyl-phenyl)-(4-ethoxy-2-trifluormethyl-phenyl)-amin wurden analog Vorschrift (1d) umgesetzt. Nach Reaktionsende wurde das Lösungsmittel abdestilliert und der Rückstand mit Essigsäureethylester versetzt, mit Natriumhydrogencarbonat-Lösung extrahiert und mit Natriumsulfat getrocknet. Die Lösung wurde im Vakuum eingeengt und der Rückstand über eine Kieselgelsäule gereinigt (Dichlormethan/Ethanol = 19:1)
C₂₅H₂₄F₃N₅O₃ (499.49)
Massenspektroskopie [M+H]⁺ = 500

### Beispiel 118: Pyrimidin-5-carbonsäure (1-{4-[4-(2,2-difluor-ethoxy)-2-trifluormethylphenylamino]-benzylcarbamoyl}-cyclopropyl)-amid Hydrochlorid

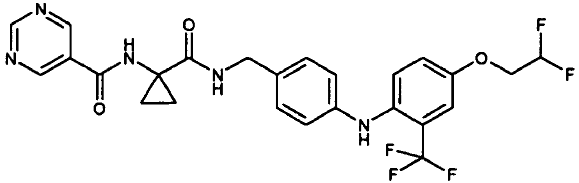

### 118a) 4-[4-(2,2-Difluor-ethoxy)-2-trifluormethyl-phenylamino]-benzonitril

Die Titelverbindung wurde in Analogie zu Vorschrift (117a) aus 4-Fluor-benzonitril und 4-(2,2-Difluor-ethoxy)-2-trifluormethyl-phenylamin erhalten.
Ausbeute: 32% der Theorie
C₁₆H₁₁F₅N₂O (342.26)
Massenspektroskopie [M+H]⁺ = 343

### 118b) (4-Aminomethyl-phenyl)-[4-(2,2-difluor-ethoxy)-2-trifluormethyl-phenyl]-amin

Die Titelverbindung wurde analog Vorschrift (117b) aus 4-[4-(2,2-Difluor-ethoxy)-2-trifluormethyl-phenylamino]-benzonitril hergestellt.
C₁₆H₁₅F₅N₂O (346.30)
Massenspektroskopie [M+H-NH₃]⁺ = 330

### 118c) Pyrimidin-5-carbonsäure (1-{4-[4-(2,2-difluor-ethoxy)-2-trifluormethylphenylamino]-benzylcarbamoyl}-cyclopropyl)-amid Hydrochlorid

(4-Aminomethyl-phenyl)-[4-(2,2-difluor-ethoxy)-2-trifluormethyl-phenyl]-amin und 1-[(Pyrimidin-5-carbonyl)-amino]-cylopropancarbonsäure wurden analog Vorschrift (1d) umgesetzt. Das Reaktionsgemisch wurde anschließend chromatographisch gereinigt (RP, Acetonitril/Wasser + 0.15% Ameisensäure). Die produkthaltigen Fraktionen wurden eingeengt, mit wässriger Ammoniak-Lösung alkalisch gestellt und mit Essigsäureethylester extrahiert. Anschließend engte man die organischen Phasen ein, löste den Rückstand in Essigsäureethylester, stellte mit Salzsäure einen sauren pH-Wert ein und destillierte das Lösungsmittel ab.
Ausbeute: 56% der Theorie
C₂₅H₂₂F₅N₅O₃ * HCl (571.93)
Rₜ = 3.96 min Methode 10

### Beispiel 119: Pyrimidin-5-carbonsäure-{1-[4-(4-isopropoxy-2-trifluormethyl-phenylamino)-benzylcarbamoyl]-cyclopropyl}-amid

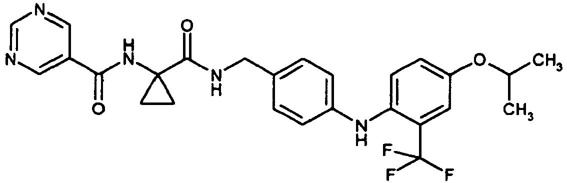

### 119a) 4-(4-Isopropoxy-2-trifluormethyl-phenylamino)-benzonitril

Herstellung analog Vorschrift (117a) aus 4-Fluor-benzonitril und 4-Isopropoxy-2-trifluormethyl-phenylamin.
Ausbeute: 37% der Theorie
C₁₇H₁₅F₃N₂O (320.31)
Massenspektroskopie [M+H]⁺ = 321

### 119b) (4-Aminomethyl-phenyl)-(4-isopropoxy-2-trifluormethyl-phenyl)-amin

Die Titelverbindung wurde nach Vorschrift (117b) aus 4-(4-Isopropoxy-2-trifluormethylphenylamino)-benzonitril erhalten.
C₁₇H₁₉F₃N₂O (324.34)
Massenspektroskopie [M+H-NH₃]⁺ = 308

### 119c) Pyrimidin-5-carbonsäure {1-[4-(4-isopropoxy-2-trifluormethyl-phenylamino)-benzyl-carbamoyl]-cyclopropyl}-amid

(4-Aminomethyl-phenyl)-(4-isopropoxy-2-trifluormethyl-phenyl)-amin und 1-[(Pyrimidin-5-carbonyl)-amino]-cyclopropancarbonsäure wurden analog Vorschrift (1 d) umgesetzt. Nach der chromatographischen Reinigung (RP, Acetonitril/Wasser + 0.15% Ameisensäure) des Reaktionsgemisches wurde das Hydrochlorid wie in Vorschrift (118c) beschrieben hergestellt.
Ausbeute: 28% der Theorie
C₂₆H₂₆F₃N₅O₃ * HCl (549.97)
Rₜ = 2.72 min Methode 14

### Beispiel 120: Pyrimidin-5-carbonsäure-(1-{[3-fluor-5-(2-fluor-6-trifluormethyl-phenylamino)-pyridin-2-ylmethyl]-carbamoyl}-cyclopropyl)-amid

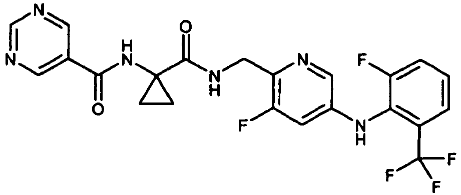

### 120a) 3-Fluor-5-(2-fluor-6-trifluormethyl-phenylamino)-pyridin-2-carbonitril

Zu einer Lösung von 6.00 g (42.8 mmol) 3,5-Difluor-pyridin-2-carbonitril und 7.67 g (42.8 mmol) 2-Fluor-6-trifluormethyl-phenylamin in 240 mL DMSO wurden unter Eiskühlung 7.21 g (64.2 mmol) Kalium-tert-butylat zugegeben. Die Reaktionsmischung wurde 2 Stunden bei Raumtemperatur gerührt, mit Wasser versetzt und mit Diethylether extrahiert. Die organischen Phasen wurden mit Natriumsulfat getrocknet und eingeengt. Der Rückstand wurde chromatographisch gereinigt (Kieselgel, Petrolether mit 0-15% Essigsäureethylester).

### 120b) (6-Aminomethyl-5-fluor-pyridin-3-yl)-(2-fluor-6-trifluormethyl-phenyl)-amin

3-Fluor-5-(2-fluor-6-trifluormethyl-phenylamino)-pyridin-2-carbonitril wurde analog Vorschrift (1c) hydriert. Das erhaltene Produkt wurde direkt weiter umgesetzt.

### 120c) Pyrimidin-5-carbonsäure (1-{[3-fluor-5-(2-fluor-6-trifluormethyl-phenylamino)-Pyridin-2-ylmethyl]-carbamoyl}-cyclopropyl)-amid

(6-Aminomethyl-5-fluor-pyridin-3-yl)-(2-fluor-6-trifluormethyl-phenyl)-amin und 1-[(Pyrimidin-5-carbonyl)-amino]-cyclopropancarbonsäure wurden analog Vorschrift (1d) umgesetzt. Zur Aufarbeitung destillierte man das Lösungsmittel ab. Anschließend wurde der Rückstand mit Wasser versetzt, mit Kaliumcarbonat-Lösung alkalisch gestellt und mit Essigsäureethylester extrahiert. Die organischen Phasen wurden mit Wasser gewaschen, mit Natriumsulfat getrocknet und eingeengt. Das zurückbleibende Rohprodukt wurde chromatographisch gereinigt.
C₂₂H₁₇F₅N₆O₂ (492.40)
Massenspektroskopie [M+H]⁺ = 493

### Beispiel 121: Pyrimidin-5-carbonsäure-(1-{1-[3-fluor-5-(2-fluor-6-trifluormethyl-phenylamino)-pyridin-2-yl]-ethylcarbamoyl}-cyclopropyl)-amid

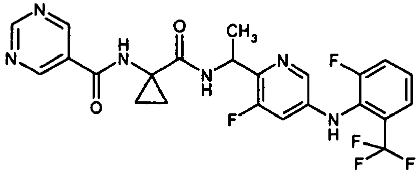

### 121a) 1-[3-Fluor-5-(2-fluor-6-trifluormethyl-phenylamino)-pyridin-2-yl]-ethanon

Zu 12 mL einer 3 molaren Methylmagnesiumbromid-Lösung in 50 mL Diethylether wurde bei -25°C eine Lösung von 2.70 g (9.02 mmol) (6-Aminomethyl-5-fluor-pyridin-3-yl)-(2-fluor-6-trifluormethyl-phenyl)-amin in 50 mL Diethylether getropft. Die Reaktionsmischung wurde auf 5°C erwärmt und dann unter Kühlung mit 1 molarer wässriger Salzsäure versetzt. Anschließend wurde die organische Phase abgetrennt, mit Natriumsulfat getrocknet und eingeengt. Der Rückstand wurde ohne weitere Reinigung in der nächsten Reaktion eingesetzt.
C₁₄H₉F₅N₂O (316.23)
Massenspektroskopie [M+H]⁺ = 317

### 121b) 1-[3-Fluor-5-(2-fluor 6-trifluormethyl-phenylamino)-pyridin-2-yl]-ethanon-oxim

1-[3-Fluor-5-(2-fluor-6-trifluormethyl-phenylamino)-pyridin-2-yl]-ethanon wurde analog Vorschrift (40c) umgesetzt. Zur Aufarbeitung wurde die Reaktionsmischung eingeengt, mit Wasser versetzt und mit Essigsäureethylester extrahiert. Die organischen Phasen wurden mit Wasser und Natriumchlorid-Lösung gewaschen und mit Natriumsulfat getrocknet. Nach dem Abdestillieren des Lösungsmittels wurde der Rückstand chromatographisch gereinigt (Kieselgel, Dichlormethan mit 2-6% Methanol).
C₁₄H₁₀F₅N₃O (331.24)
Massenspektroskopie [M+H]⁺ = 332

### 121c) 6-(1-Amino-ethyl)-5-fluor-pyridin-3-yl]-(2-fluor-6-trifluormethyl-phenyl)-amin

Die Umsetzung von 1-[3-Fluor-5-(2-fluor 6-trifluormethyl-phenylamino)-pyridin-2-yl]-ethanon-oxim erfolgte analog zu Vorschrift (40d). Das Rohprodukt wurde chromatographisch gereinigt (Kieselgel, Essigsäureethylester mit 0-10% Methanol/Ammoniak = 9:1).
C₁₄H₁₂F₅N₃ (317.26)
Massenspektroskopie [M+H]⁺ = 318

### 121d) Pyrimidin-5-carbonsäure-(1-{1-[3-fluor-5-(2-fluor-6-trifluormethyl-phenylamino)-pyridin-2-yl]-ethylcarbamoyl}-cyclopropyl)-amid

6-(1-Amino-ethyl)-5-fluor-pyridin-3-yl]-(2-fluor-6-trifluormethyl-phenyl)-amin und 1-[(Pyrimidin-5-carbonyl)-amino]-cyclopropancarbonsäure wurden analog Vorschrift (1d) umgesetzt. Zur Aufarbeitung wurde die Reaktionsmischung eingeengt und mit Kaliumcarbonat-Lösung alkalisch gestellt. Der Feststoff wurde abfiltriert, mit Wasser gewaschen und getrocknet. Anschließend wurde der Rückstand mittels Chromatographie an einer Kieselgelsäule gereinigt und die produkthaltigen Fraktionen wurden vom Lösungsmittel befreit. Das Hydrochlorid wurde erhalten, in dem man den Rückstand in etwas Essigsäureethylester löste und mit etherischer Salzsäure versetzte.
C₂₃H₁₉F₅N₆O₂ * 2 HCl (579.35)
Massenspektroskopie [M+H]⁺ = 507

Das (R)- und (S)-Enantiomer von Beispiel 121 wurde mitttels chiraler HPLC (SFC) aus der racemischen Verbindung erhalten (Säule: Daicel AD-H, 250 x 20 mm, Fließmittel: 80% überkritisches Kohlendioxid und 20% Isopropanol mit 0.2% Diethylamin, Flussrate: 70 mL/min).

### Beispiel 122: 5-Amino-N-(1-{1-[3-fluor-5-(2-fluor-6-trifluormethyl-phenylamino)-pyridin-2-yl]-ethylcarbamoyl}-cyclopropyl)-nikotinamid

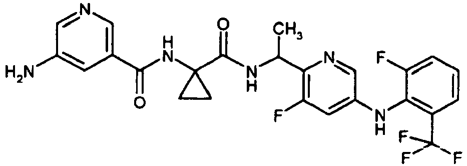

### 122a) (1-{1-[3-Fluor-5-(2-fluor-6-trifluormethyl-phenylamino)-pyridin-2-yl]-ethylcarbamoyl}-cyclopropyl)-carbaminsäure-tert-butylester

1-tert-Butoxycarbonylamino-cyclopropancarbonsäure und 6-(1-Amino-ethyl)-5-fluorpyridin-3-yl]-(2-fluor-6-trifluormethyl-phenyl)-amin wurden wie in Vorschrift (121d) beschrieben umgesetzt und aufgearbeitet. Bei der abschließenden chromatographischen Reinigung wurde eine Kieselgelsäule (Petrolether mit 30-50% Essigsäureethylester) verwendet.

### 122b) 1-Amino-cyclopropancarbonsäure-{1-[3-fluor-5-(2-fluor-6-trifluormethyl-phenyl-amino)-pyridin-2-yl]-ethyl}-amid

400 mg (0.80 mmol) (1-{1-[3-Fluor-5-(2-fluor-6-trifluormethyl-phenylamino)-pyridin-2-yl]-ethylcarbamoyl}-cyclopropyl)-carbaminsäure-tert-butylester in 10 mL Dichlormethan wurden mit 3 mL 4 molarer Salzsäure in Dioxan versetzt und zwei Stunden bei Raumtemperatur gerührt. Anschließend destillierte man die Lösungsmittel ab. Der Rückstand wurde direkt weiter umgesetzt.

### 122c) 5-Amino-N-(1-{1-[3-fluor-5-(2-fluor-6-trifluormethyl-phenylamino)-pyridin-2-yl]-ethylcarbamoyl}-cyclopropyl)-nikotinamid

1-Amino-cyclopropancarbonsäure {1-[3-fluor-5-(2-fluor-6-trifluormethyl-phenylamino)-pyridin-2-yl]-ethyl}-amid Dihydrochlorid und 5-Amino-nikotinsäure wurden wie in Vorschrift (121 d) beschrieben umgesetzt und aufgearbeitet. Bei der chromatographischen Reinigung über Kieselgel wurde Dichlormethan mit 0-15% Methanol zum Eluieren verwendet.
C₂₄H₂₁F₅N₆O₂ * 2 HCl (593.38)
Massenspektroskopie [M+H]⁺ = 521

### Beispiel 123: Pyrimidin-5-carbonsäure-(1-{1-[5-(2-fluor-6-trifluormethyl-phenylamino)-pyridin-2-yl]-ethylcarbamoyl}-cyclopropyl)-amid

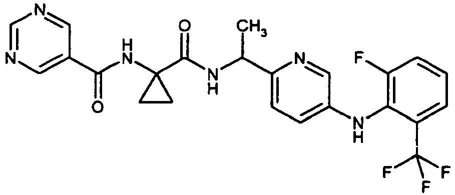

### 123a) 1-[5-(2-Fluor-6-trifluormethyl-phenylamino)-pyridin-2-yl]-ethanon

39.7 mL einer 1.4 molaren Methylmagnesiumbromid-Lösung in Toluol/THF (3:1) und 200 mL Diethylether wurden vorgelegt und auf -30°C runtergekühlt. Anschließend tropfte man 3.90 g (13.9 mmol) 5-(2-Fluor-6-trifluormethyl-phenylamino)-pyridin-2-carbonitril in 100 mL Diethylether zu und ließ die Reaktionsmischung unter Erwärmung auf Raumtemperatur über Nacht rühren. Die Reaktionsmischung wurde mit 1 molarer wässriger Salzsäure versetzt und einige Zeit gerührt. Die organische Phase wurde abgetrennt, mit Natriumsulfat getrocknet und eingeengt. Das Rohprodukt wurde ohne weitere Aufreinigung in der nachstehenden Reaktion eingesetzt.
C₁₄H₁₀F₄N₂O₂ (298.24)
Massenspektroskopie [M+H]⁺ = 299

### 123b) 1-[5-(2-Fluor-6-trifluormethyl-phenylamino)-pyridin-2-yl]-ethanon-oxim

1-[5-(2-Fluor-6-trifluormethyl-phenylamino)-pyridin-2-yl]-ethanon wurde analog Vorschrift (121 b) umgesetzt und aufgearbeitet. Bei der anschließenden Säulenchromatographie über Kieselgel wurde mit Dichlormethan/Ethanol 50:1 eluiert.
C₁₄H₁₁F₄N₃O (313.25)

### 123c) [6-(1-Amino-ethyl)-pyridin-3-yl]-(2-fluor-6-trifluormethyl-phenyl)-amin

1-[5-(2-Fluor-6-trifluormethyl-phenylamino)-pyridin-2-yl]-ethanon-oxim wird analog Vorschrift (40d) hydriert. Das Rohprodukt wurde chromatographisch gereinigt (Kieselgel, Dichlormethan mit 2 bis 5% Methanol/Ammoniak 10:1).
C₁₄H₁₃F₄N₃ (299.27))
Rₜ = 2.76 min. Methode 7

### 123d) Pyrimidin-5-carbonsäure (1-{1-[5-(2-fluor-6-trifluormethyl-phenylamino)-pyridin-2-yl]-ethylcarbamoyl}-cyclopropyl)-amid

[6-(1-Amino-ethyl)-pyridin-3-yl]-(2-fluor-6-trifluormethyl-phenyl)-amin und 1-[(Pyrimidin-5-carbonyl)-amino]-cyclopropancarbonsäure wurden analog Vorschrift (1d) umgesetzt. Zur Aufarbeitung wurde die Reaktionsmischung eingeengt, mit Essigsäureethylester versetzt und mit Natriumhydrogencarbonat-Lösung gewaschen. Die organische Phase wurde mit Natriumsulfat getrocknet und das Lösungsmittel abdestilliert. Der Rückstand wurde mittels Chromatographie gereinigt (Kieselgel, Dichlormethan/Ethanol = 50:1).
C₂₃H₂₀F₄N₆O₂ (488.44)
Massenspektroskopie [M+H]⁺ = 489

### Beispiel 124: 5-Amino-N-(1-{1-[5-(2-fluor-6-trifluormethyl-phenylamino)-pyridin-2-yl]-ethylcarbamoyl}-cyclopropyl)-nikotinamid

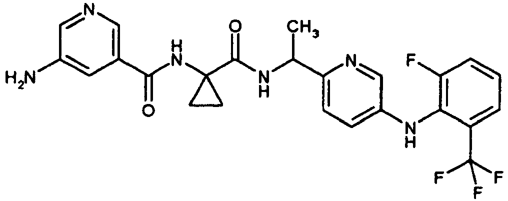

### 124a) (1-{1-[5-(2-Fluor-6-trifluormethyl-phenylamino)-pyridin-2-yl]-ethylcarbamoyl}-cyclopropyl)-carbaminsäure-tert-butylester

1-tert-Butoxycarbonylamino-cyclopropancarbonsäure und [6-(1-Amino-ethyl)-pyridin-3-yl]-(2-fluor-6-trifluormethyl-phenyl)-amin wurden wie in Vorschrift (123d) beschrieben umgesetzt und aufgearbeitet. Das Rohprodukt wurde ohne säulenchromatographische Reinigung in der nachstehenden Reaktion eingesetzt.
C₂₃H₂₆F₄N₄O₃ (482.47)
Massenspektroskopie [M+H]⁺ = 483

### 124b) 1-Amino-cyclopropancarbonsäure {1-[5-(2-fluor-6-trifluormethyl-phenylamino)-pyridin-2-yl]-ethyl}-amid Hydrochlorid

Umsetzung von (1-{1-[5-(2-Fluor-6-trifluormethyl-phenylamino)-pyridin-2-yl]-ethylcarbamoyl}-cyclopropyl)-carbaminsäure-*tert*-butylester analog Vorschrift (122b).
C₁₈H₁₈F₄N₄O * HCl (418.82)
Massenspektroskopie [M+H]⁺ = 383

### 124c) 5-Amino-N-(1-{1-[5-(2-fluor-6-trifluormethyl-phenylamino)-pyridin-2-yl]-ethylcarbamoyl}-cyclopropyl)-nikotinamid

1-Amino-cyclopropancarbonsäure {1-[5-(2-fluor-6-trifluormethyl-phenylamino)-pyridin-2-yl]-ethyl}-amid Hydrochlorid und 5-Aminonikotinsäure wurden analog Vorschrift (1d) umgesetzt. Zur Aufarbeitung wurde die Reaktionsmischung eingeengt, mit Essigsäureethylester versetzt und mit Natriumhydrogencarbonat-Lösung gewaschen. Die organische Phase wurde mit Natriumsulfat getrocknet und das Lösungsmittel abdestilliert. Der Rückstand wurde mittels Chromatographie gereinigt (Kieselgel, Dichlormethan/Ethanol = 9:1).
C₂₄H₂₂F₄N₆O₂ (502.46)
Massenspektroskopie [M+H]⁺ = 503

### Beispiel 125: 5-Amino-N-(1-{[3-fluor-5-(2-fluor-6-trifluormethyl-phenylamino)-pyridin-2-ylmethyl]-carbamoyl}-cyclopropyl)-nikotinamid

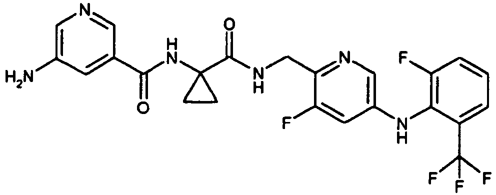

### 125a) (1-{[3-Fluor-5-(2-fluor-6-trifluormethyl-phenylamino)-pyridin-2-ylmethyl]-carbamoyl}-cyclopropyl)-carbaminsäure-tert-butylester

Umsetzung von (6-Aminomethyl-5-fluor-pyridin-3-yl)-(2-fluor-6-trifluormethyl-phenyl)-amin und 1-*tert*-Butoxycarbonylamino-cyclopropancarbonsäure analog Vorschrift (1 d). Nach Reaktionsende wurde die Lösung eingeengt und mit Kaliumcarbonat-Lösung alkalisch gestellt. Der Niederschlag wurde abfiltriert, mit Wasser gewaschen und getrocknet.
C₂₂H₂₃F₅N₄O₃ (486.44)

### 125b) 1-Amino-cyclopropancarbonsäure-{1-[5-(2-fluor-6-trifluormethyl-phenylamino)-pyridin-2-yl]-ethyl}-amid

(1-{[3-Fluor-5-(2-fluor-6-trifluormethyl-phenylamino)-pyridin-2-ylmethyl]-carbamoyl}-cyclopropyl)-carbaminsäure-tert-butylester wurde analog Vorschrift (122b) umgesetzt. C₁₇H₁₅F₅N₄O * 2 HCl (459.24)

125c) 5-Amino-N-(1-{[3-fluor-5-(2-fluor-6-trifluormethyl-phenylamino)-pyridin-2-ylmethyl]-carbamoyl}-cyclopropyl)-nikotinamid

5-Amino-nikotinsäure und 1-Amino-cyclopropancarbonsäure {1-[5-(2-fluor-6-trifluormethylphenylamino)-pyridin-2-yl]-ethyl}-amid wurden wie in Vorschrift (121 d) beschrieben umgesetzt. Bei der chromatographischen Reinigung über Kieselgel wurde mit Dichlormethan und 0 bis 15% Methanol eluiert.
C₂₃H₁₉F₅N₆O₂ * 2 HCl (579.35)
Massenspektroskopie [M+H]⁺ = 507

### Beispiel 126: Pyrimidin-5-carbonsäure-(1-{[5-(4-brom-2-chlor-phenylamino)-pyridin-2-yl-methyl]-carbamoyl}-cyclopropyl)-amid

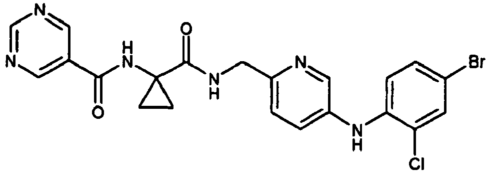

### 126a) 5-(4-Brom-2-chlor-phenylamino)-pyridin-2-carbonitril

Zu einer Lösung von 423 mg (2.0 mmol) 4-Brom-2-chloranilin und 459 mg (4.0 mmol) Kalium-*tert*-butylat in 4 moL DMSO wurden bei Raumtemperatur 250 mg (2.0 mmol) 5-Fluor-pyridin-2-carbonitril gegeben. Die Reaktionsmischung wurde über Nacht gerührt, dann mit Natriumchlorid-Lösung versetzt und mit *tert*-Butyl-methylether extrahiert. Die organischen Phasen wurden mit Natriumsulfat getrocknet und eingeengt.
C₁₂H₇BrClN₃ (308.56)
Massenspektroskopie [M+H]⁺ = 308

### 126b) (6-Aminomethyl-pyridin-3-yl)-(4-brom-2-chlor-phenyl)-amin

Zu 0.8 mL einer 2 molaren Lösung von Lithiumaluminiumhydrid in THF wurde bei Raumtemperatur eine Lösung von 250 mg 5-(4-Brom-2-chlor-phenylamino)-pyridin-2-carbonitril in 4 mL THF getropft. Anschließend ließ man die Reaktionsmischung 20 Minuten refluxieren. Es wurde vorsichtig mit Wasser hydrolysiert und mit THF extrahiert. Die organischen Phasen wurden mit Natriumchlorid-Lösung gewaschen, mit Natriumsulfat getrocknet und eingeengt. Der Rückstand wurde chromatographisch gereinigt (RP, Fließmittel: Acetonitril und Wasser mit 0.1 % Trifluoressigsäure).
C₁₂H₁₁BrClN₃ (312.59)
Massenspektroskopie [M+H]⁺ = 311

### 126c) Pyrimidin-5-carbonsäure-(1-{[5-(4-brom-2-chlor-phenylamino)-pyridin-2-ylmethyl]-carbamoyl}-cyclocropyl)-amid

Herstellung analog Vorschrift (51 b) aus (6-Aminomethyl-pyridin-3-yl)-(4-brom-2-chlorphenyl)-amin und 1-[(Pyrimidin-5-carbonyl)-amino]-cyclopropancarbonsäure.
C₂₁H₁₈BrClN₆O₂ (501.76)
Massenspektroskopie [M+H]⁺ = 501

### Beispiel 127: (S)-Pyrimidin-5-carbonsäure-{3-[4-(2-trifluormethyl-phenylamino)-benzylcarbamoyl]-tetrahydrofuran-3-yl}-amid

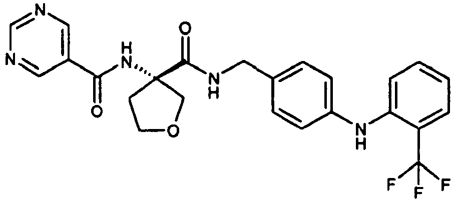

(S)-3-(Pyrimidin-5-carboxamido)tetrahydrofuran-3-carbonsäure und N-(4-(Aminomethyl)-phenyl)-2-(trifluormethyl)anilin wurden analog Vorschrift (51 b) umgesetzt. Die abschließende Reinigung erfolgte mittels Chromatographie (RP, Fließmittel: Acetonitril und Wasser mit 0.2% Trifluoressigsäure).
C₂₄H₂₂F₃N₅O₃ (485.46)
Massenspektroskopie (ESI): [M+H]⁺ = 486

### Beispiel 128: Pyridazin-4-carbonsäure-(1-{[3-fluor-5-(2-fluor-6-trifluormethyl-phenylamino)-pyridin-2-ylmethyl]-carbamoyl}-cyclopropyl)-amid

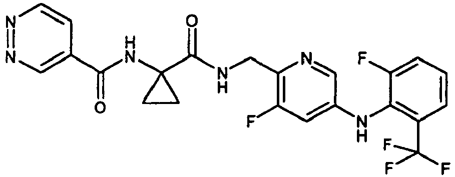

### 128a) (1-{[3-Fluor-5-(2-fluor-6-trifluormethyl-phenylamino)-pyridin-2-ylmethyl]-carbamoyl}-cyclopropyl)-carbaminsäure-tert-butylester

Umsetzung von (1-Carbamoyl-cyclopropyl)-carbaminsäure-*tert-*butylester und (6-Aminomethyl-5-fluor-pyridin-3-yl)-(2-fluor-6-trifluormethyl-phenyl)-amin analog Vorschrift (1d). Zur Aufarbeitung wurde die Reaktionsmischung eingeengt und mit Kaliumcarbonat-Lösung alkalisch gestellt. Das ausfallende Produkt wurde abfiltriert, mit Wasser gewaschen und getrocknet.
C₂₂H₂₃F₅N₄O₃ (486.44)
Rₜ = 2.30 min. Methode 12

### 128b) 1-Amino-cyclopropancarbonsäure [3-fluor-5-(2-fluor-6-trifluormethyl-phenylamino)-pyridin-2-ylmethyl]-amid Dihydrochlorid

(1-{[3-Fluor-5-(2-fluor-6-trifluormethyl-phenylamino)-pyridin-2-ylmethyl]-carbamoyl}-cyclopropyl)-carbaminsäure-*tert-*butylester wurde wie in Vorschrift (122b) beschrieben umgesetzt.
C₁₇H₁₅F₅N₄O*2 HCl (459.24)
Rₜ = 1.50 min. Methode 12

### 128c) Pyridazin-4-carbonsäure-(1-{[3-fluor-5-(2-fluor-6-trifluormethyl-phenylamino)-pyridin-2-ylmethyl]-carbamoyl}-cyclopropyl)-amid

1-Amino-cyclopropancarbonsäure [3-fluor-5-(2-fluor-6-trifluormethyl-phenylamino)-pyridin-2-ylmethyl]-amid Dihydrochlorid und Pyridazin-4-carbonsäure wurden analog Vorschrift (1d) umgesetzt und dann mittels Chromatographie (RP, Flieβmittel: Acetonitril und Wasser mit 0.1 % Trifluoressigsäure) gereinigt.
Ausbeute: 47% der Theorie
C₂₂H₁₇F₅N₆O₂ (492.40)
Massenspektroskopie (ESI): [M+H]⁺ = 493

Die nachfolgenden Beispiele 129 bis 137 wurden analog Vorschrift (128c) aus 1-Amino-cyclopropancarbonsäure [3-fluor-5-(2-fluor-6-trifluormethyl-phenylamino)-pyridin-2-yl-methyl]-amid Dihydrochlorid und der entsprechenden Carbonsäure erhalten.

### Beispiel 129: 2-Methoxy-pyrimidin-5-carbonsäure-(1-{[3-fluor-5-(2-fluor-6-trifluormethylphenylamino)-pyridin-2-ylmethyl]-carbamoyl}-cyclopropyl)-amid

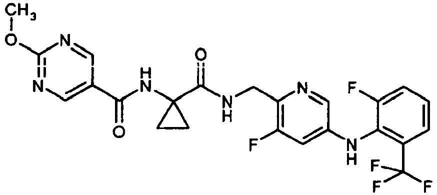

C₂₃H₁₉F₅N₆O₃* C₂HF₃O₂ (636.45)
Massenspektroskopie (ESI): [M+H]⁺ = 523

### Beispiel 130: N-(1-{[3-Fluor-5-(2-fluor-6-trifluormethyl-phenylamino)-pyridin-2-ylmethyl]-carbamoyl}-cyclopropyl)-2-hydroxy-isonikotinamid

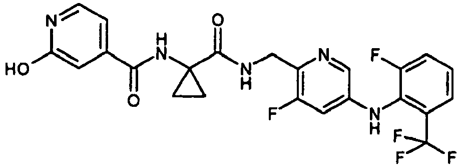

C₂₃H₁₈F₅N₅O₃ (507.41)
Massenspektroskopie (ESI): [M+H]⁺ = 508

### Beispiel 131: N-(1-{[3-Fluor-5-(2-fluor-6-trifluormethyl-phenylamino)-pyridin-2-ylmethyl]-carbamoyl}-cyclopropyl)-5-methyl-nikotinamid

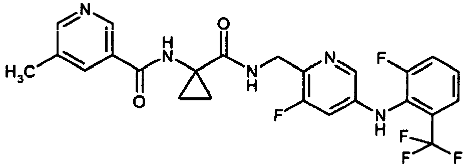

C₂₄H₂₀F₅N₅O₂ (505.44)
Massenspektroskopie (ESI): [M+H]⁺ = 506

### Beispiel 132: 1-Methyl-2-oxo-1,2-dihydro-pyridin-4-carbonsäure-(1-{[3-fluor-5-(2-fluor-6-trifluormethyl-phenylamino)-pyridin-2-ylmethyl]-carbamoyl}cyclopropyl)-amid

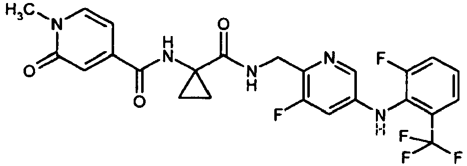

C₂₄H₂₀F₅N₅O₃ (521.44)
Massenspektroskopie (ESI): [M+H]⁺ = 522

### Beispiel 133: 2-Methylamino-pyrimidin-5-carbonsäure-(1-{[3-fluor-5-(2-fluor-6-trifluormethyl-phenylamino)-pyridin-2-ylmethyl]-carbamoyl}-cyclopropyl)-amid

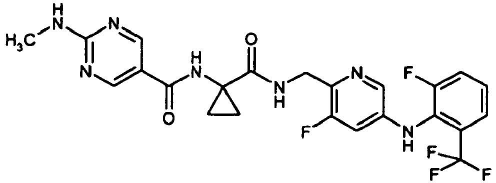

C₂₃H₂₀F₅N₇O₂ (521.44)
Massenspektroskopie (ESI): [M+H]⁺ = 522

### Beispiel 134: 2-Methyl-pyrimidin-5-carbonsäure-(1-{[3-fluor-5-(2-fluor-6-trifluormethylphenylamino)-pyridin-2-ylmethyl]-carbamoyl}cyclopropyl)-amid

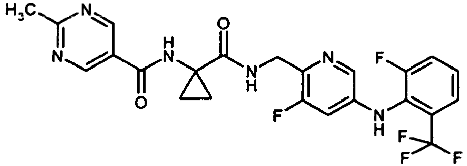

C₂₃H₁₉F₅N₆O₂ (506.43)
Massenspektroskopie (ESI): [M+H]⁺ = 507

### Beispiel 135: Thiazol-5-carbonsäure-(1-{[3-fluor-5-(2-fluor-6-trifluormethyl-phenylamino)-pyridin-2-ylmethyl]-carbamoyl}-cyclopropyl)-amid

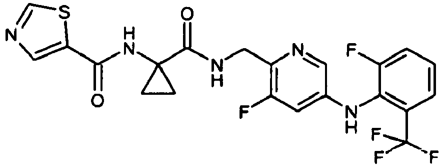

C₂₁H₁₆F₅N₅O₂S (497.44)
Massenspektroskopie (ESI): [M+H]⁺ = 498

### Beispiel 136: 6-Hydroxy-pyridin-2-carbonsäure-(1-{[3-fluor-5-(2-fluor-6-trifluormethylphenylamino)-pyridin-2-ylmethyl]-carbamoyl}-cyclopropyl)-amid

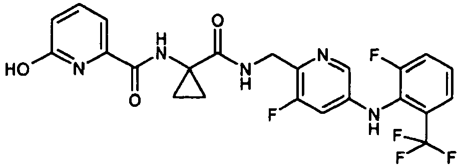

C₂₃H₁₈F₅N₅O₃ (507.41)
Massenspektroskopie (ESI): [M+H]⁺ = 508

### Beispiel 137: Isoxazol-5-carbonsäure-(1-{[3-fluor-5-(2-fluor-6-trifluormethyl-phenylamino)-pyridin-2-ylmethyl]-carbamoyl}cyclopropyl)-amid

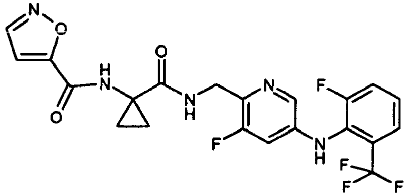

C₂₁H₁₆F₅N₅O₃ (481.38)
Massenspektroskopie (ESI): [M+H]⁺ = 482

### Beispiel 138: 2-Methoxy-pyrimidin-5-carbonsäure (1-{[5-(4-brom-2-trifluormethyl-phenylamino)-pyridin-2-ylmethyl]-carbamoyl}-cyclopropyl)-amid

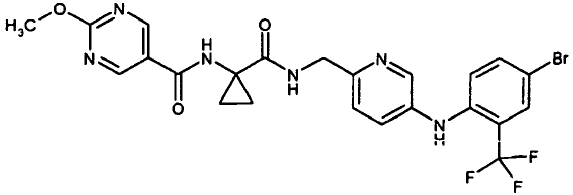

### 138a) (1-{[5-(4-Brom-2-trifluormethyl-phenylamino)-pyridin-2-ylmethyl]-carbamoyl}-cyclopropyl)-carbaminsäure-tert-butylester

Umsetzung von (6-Aminomethyl-pyridin-3-yl)-(4-brom-2-trifluormethyl-phenyl)-amin und 1-*tert-*Butoxycarbonylamino-cyclopropancarbonsäure analog Vorschrift (1d). Anschließend wurde die Reaktionsmischung eingeengt und der Rückstand chromatographiert (RP, Fließmittel: Acetonitril und Wasser mit 0.1% Trifluoressigsäure)
C₂₂H₂₄BrF₃N₄O₃ (529.35)
Massenspektroskopie (ESI): [M+H]⁺ = 529

### 138b) 1-Amino-cyclopropancarbonsäure [5-(4-brom-2-trifluormethyl-phenylamino)-pyridin-2-ylmethyl]-amid Trifluoracetat

150 mg (0.28 mmol) (1-{[5-(4-Brom-2-trifluormethyl-phenylamino)-pyridin-2-ylmethyl]-carbamoyl}-cyclopropyl)-carbaminsäure-*tert-*butylester in 2.5 mL Dichlormethan wurden mit 1 mL Trifluoressigsäure versetzt und eine Stunde bei Raumtemperatur gerührt. Das Lösungsmittel wurde abdestilliert und der Rückstand direkt weiter umgesetzt.
Ausbeute: 100% der Theorie
C₁₇H₁₆BrF₃N₄O* C₂HF₃O₂ (543.26)
Rₜ = 1.59 min. Methode 12

### 138c) 2-Methoxy-pyrimidin-5-carbonsäure-(1-{[5-(4-brom-2-trifluormethyl-phenylamino)-pyridin-2-ylmethyl]-carbamoyl}-cyclopropyl)-amid

Das Produkt wurde nach Vorschrift (1d) aus 1-Amino-cyclopropancarbonsäure [5-(4-brom-2-trifluormethyl-phenylamino)-pyridin-2-ylmethyl]-amid Trifluoracetat und 2-Methoxypyrimidin-5-carbonsäure hergestellt.
C₂₃H₂₀BrF₃N₆O₃ (565.34)
Massenspektroskopie (ESI): [M+H]⁺ = 565

Die nachfolgenden Beispiele 139 bis 141 wurden aus 1-Amino-cyclopropancarbonsäure [5-(4-brom-2-trifluormethyl-phenylamino)-pyridin-2-ylmethyl]-amid Trifluoracetat und der entsprechenden Carbonsäure nach Vorschrift (1d) hergestellt.

### Beispiel 139: 2-Methyl-pyrimidin-5-carbonsäure-(1-{[5-(4-brom-2-trifluormethyl-phenylamino)-pyridin-2-ylmethyl]-carbamoyl}-cyclopropyl)-amid

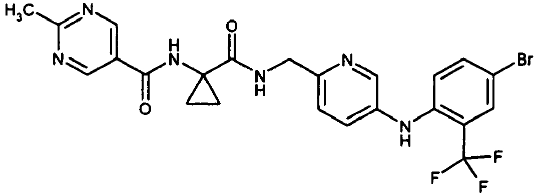

C₂₃H₂₀BrF₃N₆O₂ (549.34)
Massenspektroskopie (ESI): [M+H]⁺ = 549

### Beispiel 140: 6-Hydroxy-pyridin-2-carbonsäure-(1-{[5-(4-brom-2-trifluormethyl-phenylamino)-pyridin-2-ylmethyl]-carbamoyl}-cyclopropyl)-amid

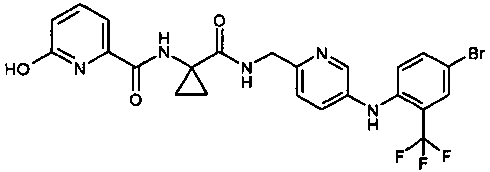

C₂₃H₁₉BrF₃N₅O₃ (550.33)
Massenspektroskopie (ESI): [M+H]⁺ = 550

### Beispiel 141: N-(1-{[5-(4-Brom-2-trifluormethyl-phenylamino)-pyridin-2-ylmethyl]-carbamoyl}-cyclopropyl)-5-hydroxy-nikotinamid

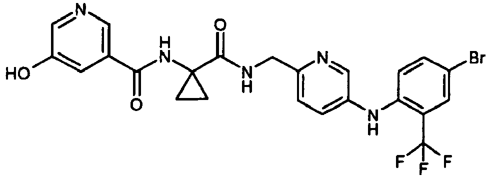

C₂₃H₁₉BrF₃N₅O₃ (550.33)
Massenspektroskopie (ESI): [M+H]⁺ = 550

### Beispiel 142: 5-Amino-N-(1-{[5-(4-fluor-2-trifluormethyl-phenylamino)-pyridin-2-ylmethyl]-carbamoyl}-cyclopropyl)-nikotinamid

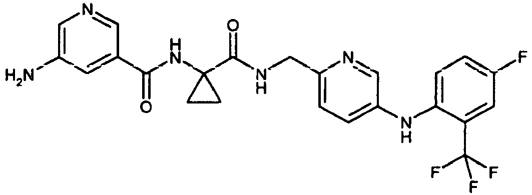

### 142a) 1-[(5-Amino-pyridin-3-carbonyl)-amino]-cyclopropancarbonsäure-benzylester

5-Amino-nikotinsäure und 1-Amino-cyclopropancarbonsäure-benzylester Hydrochlorid wurden nach Vorschrift (1 d) gekuppelt. Zur Aufarbeitung wurde die Reaktionsmischung eingeengt, mit Kaliumcarbonat-Lösung versetzt und mit Essigsäureethylester extrahiert. Die organischen Phasen wurden mit Wasser gewaschen, mit Natriumsulfat getrocknet und im Vakuum vom Lösungsmittel befreit. Der Rückstand wurde an einer Kieselgelsäule chromatographiert (Laufmittel: Dichlormethan mit 0-10% Methanol).

### 142b) 1-[(5-Amino-pyridin-3-carbonyl)-amino]-cyclopropancarbonsäure

1.90 g (6.1 mmol) 1-[(5-Amino-pyridin-3-carbonyl)-amino]-cyclopropancarbonsäure-benzylester wurden in 70 mL Methanol gelöst und mit Palladium auf Kohle (10%ig) als Katalysator bei 3 bar Wasserstoffdruck hydriert. Der Katalysator wurde abfiltriert und das Filtrat eingeengt. Zur Reinigung wurde der Rückstand mit Diethylether verrührt, filtriert und getrocknet.
Ausbeute: 85% der Theorie
C₁₀H₁₁N₃O₃ (221.21)
Massenspektroskopie (ESI): [M+H]⁺ = 222

### 142c) 5-Amino-N-(1-{[5-(4-fluor-2-trifluormethyl-phenylamino)-pyridin-2-ylmethyl]-carbamoyl}-cyclopropyl)-nikotinamid

1-[(5-Amino-pyridin-3-carbonyl)-amino]-cyclopropancarbonsäure und (6-Aminomethylpyridin-3-yl)-(4-fluor-2-trifluormethyl-phenyl)-amin wurden analog Vorschrift (1d) umgesetzt. Im Anschluss an die chromatographische Reinigung (RP, Fließmittel: Acetonitril und Wasser mit 0.15% Ameisensäure) wurden die produkthaltigen Fraktionen mit Kaliumcarbonat-Lösung alkalisch gestellt. Dann destillierte man das Acetonitril ab und extrahierte mit Essigsäureethylester. Die organischen Phasen wurden mit Natriumsulfat getrocknet, vom Lösungsmittel befreit und mit Diisopropylether verrieben.
C₂₃H₂₀F₄N₆O₂ (488.44)
Massenspektroskopie (ESI): [M+H]⁺ = 489

### Beispiel 143: 3H-Imidazo[4,5-b]pyridin-6-carbonsäure-(1-{[5-(4-fluor-2-trifluormethylphenylamino)-pyridin-2-ylmethyl]-carbamoyl}cyclopropyl)-amid

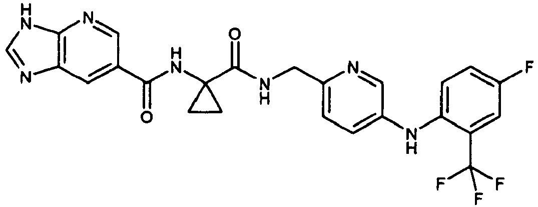

### 143a) 1-[(3H-Imidazo[4,5-b]pyridin-6-carbonyl)-amino]-cyclopropancarbonsäure-benzylester

Herstellung aus 3H-Imidazo[4,5-b]pyridin-6-carbonsäure und 1-Amino-cyclopropancarbonsäure-benzylester Hydrochlorid analog Vorschrift (142a).
C₁₈H₁₆N₄O₃ (336.35)
Massenspektroskopie (ESI): [M+H]⁺ = 337

### 143b) 1-[(3H-Imidazo[4,5-b]pyridin-6-carbonyl)-amino]-cyclopropancarbonsäure

Erhalten aus der Umsetzung von 1-[(3H-Imidazo[4,5-b]pyridin-6-carbonyl)-amino]-cyclopropancarbonsäure-benzylester nach Vorschrift (142b).
C₁₁H₁₀N₄O₃ (246.22)
Rₜ = 1.64 min. Methode 10

### 143c) 3H-Imidazo[4,5-b]pyridin-6-carbonsäure (1-{[5-(4-fluor-2-trifluormethyl-phenylamino)-pyridin-2-ylmethyl]-carbamoyl}-cyclopropyl)-amid

Die Verbindung wurde aus der Umsetzung von 1-[(3H-Imidazo[4,5-b]pyridin-6-carbonyl)-amino]-cyclopropancarbonsäure und (6-Aminomethyl-pyridin-3-yl)-(4 fluor-2-trifluormethylphenyl)-amin analog (142c) erhalten.
C₂₄H₁₉F₄N₇O₂ (513.45)
Massenspektroskopie (ESI): [M+H]⁺ = 514

### Beispiel 144: 6-Amino-pyrazin-2-carbonsäure-(1-{[5-(4-fluor-2-trifluormethyl-phenylamino)-pyridin-2-ylmethyl]-carbamoyl}-cyclopropyl)-amid

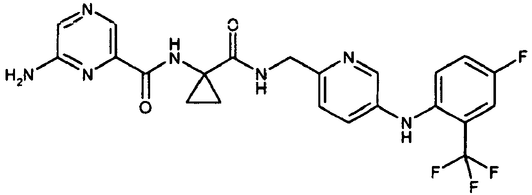

### 144a) (1-{[5-(4-Fluor-2-trifluormethyl-phenylamino)-pyridin-2-ylmethyl]-carbamoyl}-cyclopropyl)-carbaminsäure-tert-butylester

1-*tert-*Butoxycarbonylamino-cyclopropancarbonsäure und (6-Aminomethyl-pyridin-3-yl)-(4-fluor-2-trifluormethyl-phenyl)-amin wurden analog Vorschrift (1d) mit TBTU gekuppelt. Das Reaktionsgemisch wurde eingeengt, mit Essigsäureethylester versetzt und mit Natriumhydrogencarbonat-Lösung gewaschen. Dann wurde die organische Phase mit Natriumsulfat getrocknet und vom Lösungsmittel befreit. Den Rückstand reinigte man über eine Kieselgesäule mit Dichlormethan/Ethanol als Fließmittel im Verhältnis 1:50 bis 1:20.
C₂₂H₂₄F₄N₄O₃ (468.45)
Massenspektroskopie (ESI): [M+H]⁺ = 469

### 144b) 1-Amino-cyclopropancarbonsäure [5-(4-fluor-2-trifluormethyl-phenylamino)-pyridin-2-ylmethyl]-amid Hydrochlorid

1.00 g (2.14 mmol) (1-{[5-(4-Fluor-2-trifluormethyl-phenylamino)-pyridin-2-ylmethyl]-carbamoyl}-cyclopropyl)-carbaminsäure-*tert-*butylester wurden in 30 mL Dioxan gelöst und nach Zugabe von 3.2 mL 4 molare Hydrochlorid-Lösung in Dioxan über Nacht bei Raumtemperatur gerührt. Das Lösungsmittel wurde im Vakuum abdestilliert und der Rückstand direkt weiter umgesetzt.
C₁₇H₁₆F₄N₄O· HCl (404.79)
Massenspektroskopie (ESI): [M+H]⁺ = 369

### 144c) 6-Amino-pyrazin-2-carbonsäure (1-{[5-(4-fluor-2-trifluormethyl-phenylamino)-pyridin-2-ylmethyl]-carbamoyl}-cyclopropyl)-amid

Erhalten analog Vorschrift (142c) aus 1-Amino-cyclopropancarbonsäure [5-(4-fluor-2-trifluormethyl-phenylamino)-pyridin-2-ylmethyl]-amid Hydrochlorid und 6-Amino-pyrazin-2-carbonsäure.
C₂₂H₁₉F₄N₇O₂ (489.43)
Massenspektroskopie (ESI): [M+H]⁺ = 490

### Beispiel 145: 2-Methylamino-pyrimidin-5-carbonsäure-(1-{[5-(4-chlor-2-trifluormethylphenylamino)-pyridin-2-ylmethyl]-carbamoyl}-cyclopropyl)-amid

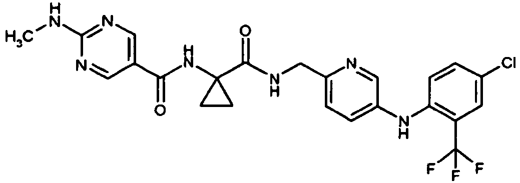

### 145a) (1-{[5-(4-Chlor-2-trifluormethyl-phenylamino)-pyridin-2-ylmethyl]-carbamoyl}-cyclopropyl)-carbaminäure-tert-butylester

1-(*tert-*Butoxycarbonylamino)-cyclopropancarbonsäure und (6-Aminomethyl-pyridin-3-yl)-(4-chlor-2-trifluormethyl-phenyl)-amin wurden wie in Vorschrift (1 d) beschrieben gekuppelt. Zur Aufarbeitung wurde das Reaktionsgemisch mit Wasser versetzt und mit Dichlormethan extrahiert. Die organischen Phasen wurden mit Natriumsulfat getrocknet und eingeengt.
C₂₂H₂₄ClF₃N₄O₃ (484.90)
Rₜ = 2.23 min. Methode 12

### 145b) 1-Amino-cyclopropancarbonsäure-[5-(4-chlor-2-trifluormethyl-phenylamino)-pyridin-2-ylmethyl]-amid Trifluoracetat

906 mg (1.40 mmol) (1-{[5-(4-Chlor-2-trifluormethyl-phenylamino)-pyridin-2-ylmethyl]-carbamoyl}-cyclopropyl)-carbaminsäure-*tert-*butylester in 5 mL Dichlormethan wurden mit 2 mL Trifluoressigsäure versetzt und eine Stunde bei Raumtemperatur gerührt. Anschließend wurde das Lösungsmittel im Vakuum abdestilliert und der Rückstand ohne weitere Reinigung umgesetzt.
C₁₇H₁₆ClF₃N₄O* C₂HF₃O₂ (498.81)
Rₜ= 1.54 min. Methode 12

### 145c) 2-Methylamino-pyrimidin-5-carbonsäure-(1-{[5-(4-chlor-2-trifluormethyl-phenylamino)-pyridin-2-ylmethyl]-carbamoyl}-cyclopropyl)-amid

1-Amino-cyclopropancarbonsäure [5-(4-chlor-2-trifluormethyl-phenylamino)-pyridin-2-yl-methyl]-amid Trifluoracetat und 2-Methylamino-pyrimidin-5-carbonsäure wurden analog Vorschrift (1d) umgesetzt. Anschließend wurde das Reaktionsgemisch chromatographisch gereinigt (RP, Fließmittel: Acetonitril und Wasser mit 0.1% Trifluoressigsäure).
C₂₃H₂₁ClF₃N₇O₂ (519.91)
Massenspektroskopie (ESI): [M+H]⁺ = 520

Die Beispiele 146 bis 149 wurden analog aus 1-Amino-cyclopropancarbonsäure [5-(4-chlor-2-trifluormethyl-phenylamino)-pyridin-2-ylmethyl]-amid und der entsprechenden Carbonsäure erhalten.

### Beispiel 146: 2-Methoxy-pyrimidin-5-carbonsäure-(1-{[5-(4-chlor-2-trifluormethyl-phenylamino)-pyridin-2-ylmethyl]-carbamoyl}-cyclopropyl)-amid

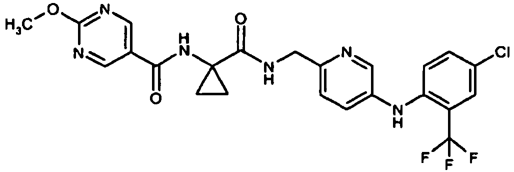

C₂₃H₂₀ClF₃N₆O₃ (520.89)
Massenspektroskopie (ESI): [M+H]⁺ = 521

### Beispiel 147: 2-Methyl-pyrimidin-5-carbonsäure (1-{[5-(4-chlor-2-trifluormethyl-phenylamino)-pyridin-2-ylmethyl]-carbamoyl}-cyclopropyl)-amid

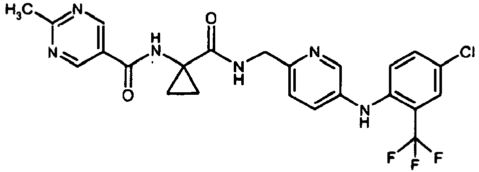

C₂₃H₂₀ClF₃N₆O₂ (504.89)
Massenspektroskopie (ESI): [M+H]⁺ = 505

### Beispiel 148: 1-Methyl-2-oxo-1,2-dihydro-pyridin-4-carbonsäure (1-{[5-(4-chlor-2-trifluormethyl-phenylamino)-pyridin-2-ylmethyl]-carbamoyl}-cyclopropyl)-amid

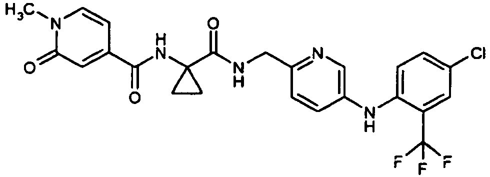

C₂₄H₂₁ClF₃N₅O₃ (519.90)
Massenspektroskopie (ESI): [M+H]⁺ = 520
Rₜ = 1.74 min. Methode 13

### Beispiel 149: Thiazol-5-carbonsäure (1-{[5-(4-chlor-2-trifluormethyl-phenylamino)-pyridin-2-ylmethyl]-carbamoyl}-cyclopropyl)-amid

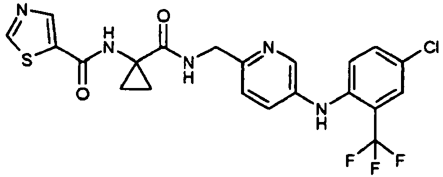

C₂₁H₁₇ClF₃N₅O₂S (495.91)
Massenspektroskopie (ESI): [M+H]⁺ = 496
Rₜ = 1.84 min. Methode 13

### Beispiel 150: 3-Amino-isoxazol-5-carbonsäure (1-{[5-(4-fluor-2-trifluormethyl-phenylamino)-pyridin-2-ylmethyl]-carbamoyl}-cyclopropyl)-amid

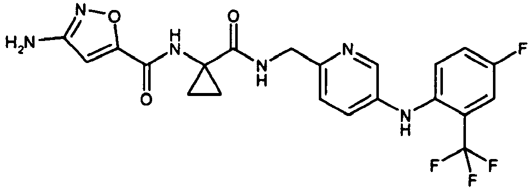

Nach Vorschrift (142c) aus 1-Amino-cyclopropancarbonsäure [5-(4-fluor-2-trifluormethylphenylamino)-pyridin-2-ylmethyl]-amid Hydrochlorid und 3-Amino-isoxazol-5-carbonsäure hergestellt.
C₂₁H₁₈F₄N₆O₃ (478.40)
Massenspektroskopie (ESI): [M+H]⁺ = 479

### Beispiel 151: Pyrimidin-5-carbonsäure (1-{[3-fluor-5-(4-fluor-2-trifluormethyl-phenylamino)-pyridin-2-ylmethyl]-carbamoyl}-cyclopropyl)-amid Dihydrochlorid

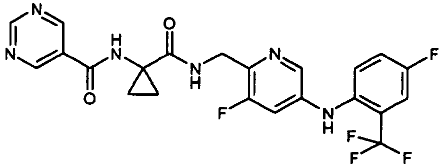

1-[(Pyrimidin-5-carbonyl)-amino]-cyclopropancarbonsäure und (6-Aminomethyl-5-fluorpyridin-3-yl)-(4-fluor-2-trifluormethyl-phenyl)-amin wurden analog Vorschrift (1d) umgesetzt. Die Reaktionsmischung wurde eingeengt, mit Kaliumcarbonat-Lösung versetzt und mit Essigsäureethylester extrahiert. Die organischen Phasen wurden mit Wasser und Natriumchlorid-Lösung gewaschen, mit Natriumsulfat getrocknet und vom Lösungsmittel befreit. Nach der chromatographischen Reinigung des Rückstands (RP, Fließmittel: Wasser und Acetonitril mit Ameisensäure) stellte man die produkthaltigen Fraktionen mit Ammoniak alkalisch, destillierte das Acetonitril ab und extrahierte mit Essigsäureethylester. Aus der organischen Lösung wurde nach Trocknung mit Natriumsulfat mit etherischer Hydrochlorid-Lösung das Produkt ausgefällt.
C₂₂H₁₇F₅N₆O₂ *2 HCl (565.32)
Massenspektroskopie (ESI): [M+H]⁺ = 493

### Beispiel 152: 5-Amino-N-(1-{[3-fluor-5-(4-fluor-2-trifluormethyl-phenylamino)-pyridin-2-yl-methyl]-carbamoyl}-cyclopropyl)-nikotinamid Dihydrochlorid

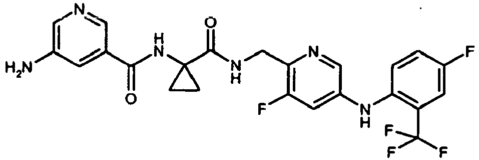

Erhalten analog zur Vorschrift für Beispiel 147 aus 5-Amino-nikotinsäure und 1-Amino-cyclopropancarbonsäure-[3-fluor-5-(4-fluor-2-trifluormethyl-phenylamino)-pyridin-2-yl-methyl]-amid. Abweichend wurde zur chromatographischen Reinigung eine Kieselgelsäule verwendet (Fließmittel: Dichlormethan mit 5 bis 12% Methanol).
C₂₃H₁₉F₅N₆O₂ * 2 HCl (579.35)
Massenspektroskopie (ESI): [M+H]⁺ = 507

### Beispiel 153: (S)-Pyrimidin-5-carbonsäure (3-{[3-fluor-5-(4-fluor-2-trifluormethyl-phenylamino)-pyridin-2-ylmethyl]-carbamoyl}-tetrahydrofuran-3-yl)-amid Dihydrochlorid

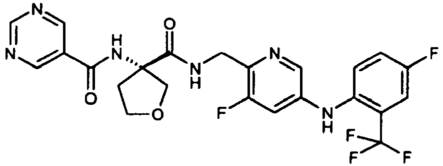

Hergestellt aus (S)-3-(Pyrimidin-5-carboxamido)tetrahydrofuran-3-carbonsäure und (6-Aminomethyl-5-fluor-pyridin-3-yl)-(4-fluor-2-trifluormethyl-phenyl)-amin analog Beispiel 150. Bei der säulenchromatographischen Reinigung wurde eine Kieselgelsäule und Dichlormethan mit 0 bis 7% Methanol als Fließmittel verwendet.
C₂₃H₁₉F₅N₆O₃ * 2 HCl (595.35)
Massenspektroskopie (ESI): [M+H]⁺ = 523

### Beispiel 154: (S)-5-Amino-N-(3-{[3-fluor-5-(4-fluor-2-trifluormethyl-phenylamino)-pyridin-2-ylmethyl]-carbamoyl}-tetrahydrofuran-3-yl)-nikotinamid

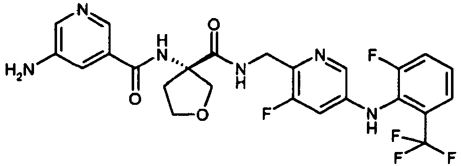

### 154a) (S)-3-[(5-Amino-pyridin-3-carbonyl)-amino]-tetrahydrofuran-3-carbonsäurebutylester

5-Amino-nikotinsäure und (S)-3-Amino-tetrahydrofuran-3-carbonsäure-butylester wurden analog Vorschrift (1d) mit TBTU gekuppelt. Zur Aufarbeitung wurde das Reaktionsgemisch eingeengt, mit Kaliumcarbonat-Lösung versetzt und mit Essigsäureethylester extrahiert. Die organischen Phasen wurden mit Wasser und Natriumchlorid-Lösung gewaschen, mit Natriumsulfat getrocknet und vom Lösungsmittel befreit. Der Rückstand wurde an einer Kieselgelsäule chromatographiert (Laufmittel: Dichlormethan mit 5 bis 10% Methanol).
C₁₅H₂₁N₃O₄ (307.35)
Massenspektroskopie (ESI): [M+H]⁺ = 308

### 154b) (S)-3-[(5-Amino-pyridin-3-carbonyl)-amino]-tetrahydrofuran-3-carbonsäure

2.45 g (7.97 mmol) (S)-3-[(5-Amino-pyridin-3-carbonyl)-amino]-tetrahydrofuran-3-carbonsäure-butylester in 50 mL Methanol wurden mit 16 mL 1 molarer Natronlauge versetzt und eine Stunde bei Raumtemperatur gerührt. Nach Zugabe von 16 mL 1 molarer Salzsäure wurden die Lösungsmittel im Vakuum abdestilliert. Der Rückstand wurde in Ethanol gelöst und die anorganischen Salze abfiltriert. Anschließend wurde das Filtrat eingeengt.
Ausbeute: 99% der Theorie
C₁₁H₁₃N₃O₄ (251.24)
Massenspektroskopie (ESI): [M+H]⁺ = 252

### 154c) (S)-5-Amino-N-(3-{[3-fluor-5-(4-fluor-2-trifluormethyl-phenylamino)-pyridin-2-yl-methyl]-carbamoyl}-tetrahydrofuran-3-yl)-nikotinamid-Dihydrochlorid

(S)-3-[(5-Amino-pyridin-3-carbonyl)-amino]-tetrahydrofuran-3-carbonsäure und (6-Aminomethyl-5-fluor-pyridin-3-yl)-(4-fluor-2-trifluormethyl-phenyl)-amin wurden wie in Vorschrift (154a) beschrieben umgesetzt und aufgereinigt. Im Anschluss an die chromatographische Reinigung wurde das Produkt in Essigsäureethylester gelöst und mit etherischer Hydrochlorid-Lösung ausgefällt.
C₂₄H₂₁F₅N₆O₃* 2 HCl (609.38)
Massenspektroskopie (ESI): [M+H]⁺ = 537

### Beispiel 155: (S)-Pyrimidin-5-carbonsäure-{3-[4-(2-trifluormethyl-phenylamino)-benzylcarbamoyl]-tetrahydrofuran-3-yl}-amid

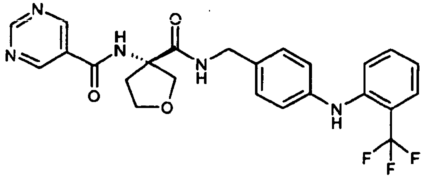

Eine Lösung von 64 mg (0.27 mmol) (S)-3-[(5-Amino-pyridin-3-carbonyl)-amino]-tetrahydrofuran-3-carbonsäure, 93 mg (0.28 mmol) O-[(Ethoxycarbonyl)cyan-methylenamino]-N,N,N',N'-tetramethyluronium-Tetrafluoroborat (TOTU) und 139 µL (0.81 mmol) DIPEA in 1 mL DMF wurde 1 Stunde bei Raumtemperatur gerührt, dann mit 144 mg (0.41 mmol) N-(4-(Aminomethyl)phenyl)-2-(trifluormethyl)anilin versetzt und über Nacht stehen gelassen. Anschließend wurde das Gemisch chromatographisch gereinigt (RP mit Gradient, Fließmittel: Acetonitril und Wasser mit 0.2% Trifluoressigsäure).
Ausbeute: 18% der Theorie
C₂₄H₂₂F₃N₅O₃ (485.46)
Massenspektroskopie (ESI): [M+H]⁺ = 486
Rₜ = 2.15 min. Methode 12

### Beispiel 156: (S)-5-Amino-N-(3-{[3-fluor-5-(4-fluor-2-trifluormethyl-phenylamino)-pyridin-2-ylmethyl]-carbamoyl}-tetrahydrofuran-3-yl)-nikotinamid Dihydrochlorid

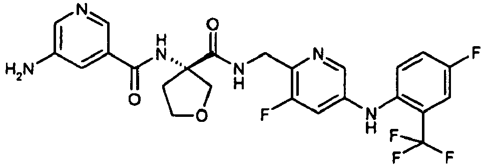

(S)-3-[(5-Amino-pyridin-3-carbonyl)-amino]-tetrahydrofuran-3-carbonsäure und (6-Aminomethyl-5-fluor-pyridin-3-yl)-(4-fluor-2-trifluormethyl-phenyl)-amin wurden wie in Vorschrift (154a) beschrieben umgesetzt und aufgereinigt. Im Anschluss an die chromatographische Reinigung wurde das Produkt in Essigsäureethylester gelöst und mit etherischer Hydrochlorid-Lösung ausgefällt.
C₂₄H₂₁F₅N₆O₃* 2 HCl (609.38)
Massenspektroskopie (ESI): [M+H]⁺ = 537

### Beispiel 157: (S)-5-Amino-N-(3-{[5-(4-fluor-2-trifluormethyl-phenylamino)-pyridin-2-yl-methyl]-carbamoyl}-tetrahydrofuran-3-yl)-nikotinamid

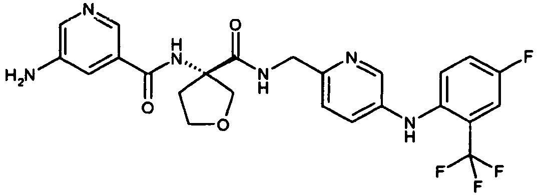

Erhalten aus (S)-3-[(5-Amino-pyridin-3-carbonyl)-amino]-tetrahydrofuran-3-carbonsäure und (6-Aminomethyl-pyridin-3-yl)-(4-fluor-2-trifluormethyl-phenyl)-amin nach Vorschrift (142c).
C₂₄H₂₂F₄N₆O₃ (518.46)
Massenspektroskopie (ESI): [M+H]⁺ = 519

### Beispiel 158: (S)-Pyrimidin-5-carbonsäure-(3-{[3-fluor-5-(2-fluor-6-trifluormethyl-phenylamino)-pyridin-2-ylmethyl]-carbamoyl}-tetrahydrofuran-3-yl)-amid Dihydrochlorid

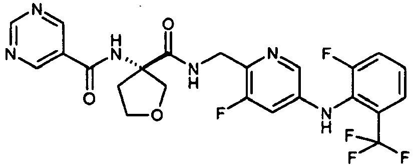

Herstellung analog Vorschrift (154a) aus (S)-3-[(Pyrimidin-5-carbonyl)-amino]-tetrahydrofuran-3-carbonsäure und (6-Aminomethyl-5-fluor-pyridin-3-yl)-(2-fluor-6-trifluormethylphenyl)-amin.
C₂₃H₁₉F₅N₆O₃* 2 HCl (595.35)
Massenspektroskopie (ESI): [M+H]⁺ = 523

### Beispiel 159: (S)-3H-Imidazo[4,5-b]pyridin-6-carbonsäure-(3-{[5-(4-fluor-2-trifluormethylphenylamino)-pyridin-2-ylmethyl]-carbamoyl}-tetrahydrofuran-3-yl)-amid

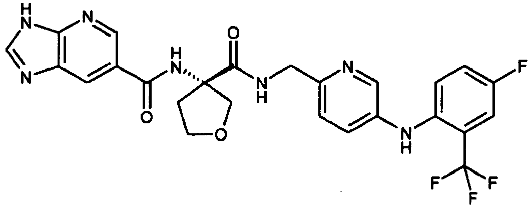

### 159a) (S)-3-[(3H-Imidazo[4,5-b]pyridin-6-carbonyl)-amino]-tetrahydrofuran-3-carbonsäure-butylester

Herstellung aus 3H-Imidazo[4,5-b]pyridin-6-carbonsäure und (S)-3-Amino-tetrahydrofuran-3-carbonsäure-butylester wie in Vorschrift (154a) beschrieben, wobei auf eine chromatographische Reinigung verzichtet wurde.
C₁₆H₂₀N₄O₄ (332.36)
R, = 1.99 min. Methode 13

### 159b) (S)-3-[(3H-Imidazo[4,5-b]pyridin-6-carbonyl)-amino]-tetrahydrofuran-3-carbonsäure

400 mg (1.20 mmol) (S)-3-[(3H-Imidazo[4,5-b]pyridin-6-carbonyl)-amino]-tetrahydrofuran-3-carbonsäure-butylester wurden in 10 mL THF und 5 mL Ethanol gelöst, mit 1.2 mL 2 molarer Lithiumhydroxid-Lösung versetzt und über Nacht bei Raumtemperatur gerührt. Anschließend destillierte man die Lösungsmittel ab und versetzte den Rückstand mit 2.4 mL 1 molarer wässriger Salzsäure. Das Gemisch wurde im Vakuum eingeengt und Wasserreste wurden durch wiederholte azeotrope Destillation mit Ethanol entfernt.
C₁₂H₁₂N₄O₄ (276.25)
Massenspektroskopie (ESI): [M-H]⁺ = 275

### 159c) (S)-3H-Imidazo[4,5-b]pyridin-6-carbonsäure (3-{[5-(4-fluor-2-trfluormethylphenylamino)-pyridin-2-ylmethyl]-carbamoyl}-tetrahydrofuran-3-yl)-amid

Herstellung aus (S)-3-[(3H-Imidazo[4,5-b]pyridin-6-carbonyl)-amino]-tetrahydrofuran-3-carbonsäure und (6-Aminomethyl-pyridin-3-yl)-(4-fluor-2-trifluormethyl-phenyl)-amin analog Vorschrift (154a).
C₂₅H₂₁F₄N₇O₃ (543.47)
Massenspektroskopie (ESI): [M+H]⁺ = 544

### Beispiel 160: Pyrimidin-5-carbonsäure-(1-{[5-(2-cyan-4-fluor-phenylamino)-3-fluorpyridin-2-ylmethyl]-carbamoyl}-cyclopropyl)-amid

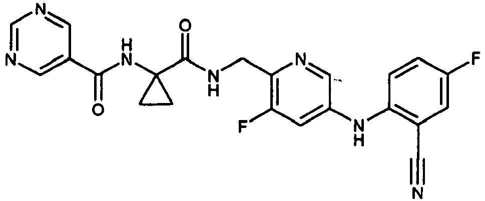

### 160a) (5-Brom-3-fluor-pyridin-2-ylmethyl)-carbaminsäure-tert-butylester

581 mg (2.41 mmol) C-(5-Brom-3-fluor-pyridin-2-yl)-methylamin wurden in 5 mL Triethylamin und 1.5 mL Wasser gelöst und unter Kühlung mit dem Eisbad mit 630 mg (2.89 mmol) Di-*tert-*butyl-dicarbonat versetzt. Anschließend ließ man die Reaktionsmischung über Nacht rühren. Das Lösungsmittel wurde abdestilliert und der Rückstand chromatographisch (RP mit Fließmittelgradient, Fließmittel: Acetonitril und Wasser mit 0.2 % Trifluoressigsäure) gereinigt. Die produkthaltigen Fraktionen wurden mit Triethylamin neutralisiert und eingeengt.
Ausbeute: 40% der Theorie
C₁₁H₁₄BrFN₂O₂ (305.14)
Rₜ = 2.29 min. Methode 12

### 160b) [5-(2-Cyan-4-fluor-phenylamino)-3-fluor-pyridin-2-ylmethyl]-carbaminsäure-tert-butylester

132 mg (0.97 mmol) 2-Amino-5-fluorbenzonitril und 295 mg (0.97 mmol) (5-Brom-3-fluorpyridin-2-ylmethyl)-carbaminsäure-*tert-*butylester wurden analog zu Vorschrift (55a) umgesetzt und aufgearbeitet.
Ausbeute: 15% der Theorie
C₁₈H₁₈F₂N₄O₂ (360.36)
Rₜ = 2.35 min. Methode 12

### 160c) 2-(6-Aminomethyl-5-fluor-pyridin-3-ylamino)-5-fluor-benzonitril-Trifluoracetat

51 mg (0.14 mmol) [5-(2-Cyan-4-fluor-phenylamino)-3-fluor-pyridin-2-ylmethyl]-carbaminsäure-*tert-*butylester wurden mit 1.5 mL Trifluoressigsäure und 2.5 mL Dichlormethan 3 Stunden bei Raumtemperatur gerührt. Anschließend wurde die Reaktionsmischung im Vakuum eingeengt und direkt weiter umgesetzt.
Ausbeute: 94% der Theorie
C₁₃H₁₀F₂N₄* C₂HF₃O₂ (374.27)
Rₜ=1.17min. Methode 12

### 160d) Pyrimidin-5-carbonsäure-(1-{[5-(2-cyan-4-fluor-phenylamino)-3-fluor-pyridin-2-yl-methyl]-carbamoyl}-cyclopropyl)-amid

28 mg (0.13 mmol) 1-[(Pyrimidin-5-carbonyl)-amino]-cyclopropancarbonsäure und 50 mg (0.13 mmol) 2-(6-Aminomethyl-5-fluor-pyridin-3-ylamino)-5-fluor-benzonitril wurden mit TBTU unter Verwendung von DMF als Lösungsmittel analog zu Vorschrift (1d) umgesetzt.
Ausbeute: 50% der Theorie
C₂₂H₁₇F₂N₇O₂ (449.13)
Massenspektroskopie (ESI): [M+H]⁺ = 450
Rₜ = 1.67 min. Methode 12

### Beispiel 161: Pyrimidin-5-carbonsäure {1-[4-(2-cyan-4-trifluormethoxy-phenylamino)-benzylcarbamoyl]-cyclopropyl}-amid

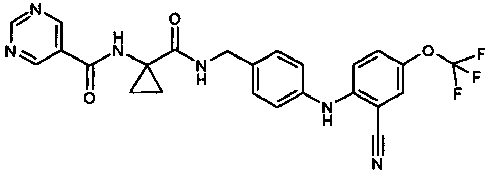

### 161a) (4-Brom-benzyl)-carbaminsäure-tert-butylester

Zu einer Lösung aus 2.00 g (8.99 mmol) 4-Brombenzylamin Hydrochlorid und 6.26 mL Triethylamin in 30 mL Dichlormethan wurden unter Kühlung mit dem Eisbad 2.35 g (11 mmol) Di-*tert-*butyl-dicarbonat in 20 mL Dichlormethan zugetropft. Anschließend wurde über Nacht gerührt und dann eingeengt. Der Rückstand wurde in Essigsäureethylester gelöst, mit Zitronensäure sauer gestellt und dann mit Wasser und Natriumhydrogencarbonat-Lösung gewaschen. Die organische Phase wurde mit Natriumsulfat getrocknet und vom Lösungsmittel befreit.
Ausbeute: 96% der Theorie
C₁₂H₁₆BrNO₂ (286.17)
Massenspektroskopie (ESI): [M+H]⁺ = 286

### 161b) [4-(2-Cyan-4-trifluormethoxy-phenylamino)-benzyl]-carbaminsäure-tert-butylester

(4-Brom-benzyl)-carbaminsäure-*tert-*butylester und 2-Amino-5-trifluormethoxy-benzonitril wurden analog Vorschrift (55a) umgesetzt.
C₂₀H₂₀F₃N₃O₃ (407.39)
Massenspektroskopie (ESI): [M+H]⁺ = 408
Rₜ = 4.63 min. Methode 13

### 161c) 2-(4-Aminomethyl-phenylamino)-5-trrifluormethoxy-benzonitril-Trifluoracetat

Zur Abspaltung der Schutzgruppe wurde [4-(2-Cyan-4-trifluormethoxy-phenylamino)-benzyl]-carbaminsäure-*tert-*butylester mit Trifluoressigsäure in Dichlormethan behandelt. Anschließend wurde das Lösungsmittel abdestilliert und der Rückstand chromatographiert (RP mit Fließmittelgradient, Fließmittel: Acetonitril und Wasser mit 0.2 % Trifluoressigsäure).
C₁₅H₁₂F₃N₃O* C₂HF₃O₂ (421.29)
Massenspektroskopie (ESI): [M+H]⁺ = 308
Rₜ = 2.45 min. Methode 13

### 161d) Pyrimidin-5-carbonsäure-{1-[4-(2-cyan-4-trifluormethoxy-phenylamino)-benzylcarbamoyl]-cyclopropyl}-amid

166 mg (0.39 mmol) 2-(4-Aminomethyl-phenylamino)-5-trifluormethoxy-benzonitril-Trifluoracetat und 82 mg (0.39 mmol) 1-[(Pyrimidin-5-carbonyl)-amino]-cyclopropancarbonsäure wurden analog Vorschrift (1d) gekuppelt.
Ausbeute: 33% der Theorie
C₂₄H₁₉F₃N₆O₃ (496.44)
Massenspektroskopie (ESI): [M+H]⁺ = 497
Rₜ = 2.24 min. Methode 13

### Beispiel 162: Pyrimidin-5-carbonsäure {1-[4-(4-chlor-2-cyan-phenylamino)-benzyl-carbamoyl]-cyclopropyl}-amid

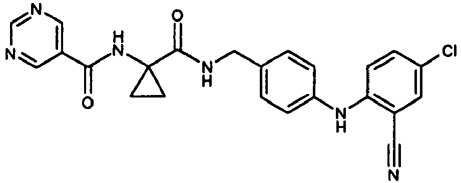

Herstellung nach der gleichen Reaktionssequenz (Buchwald-Reaktion, Schutzgruppenabspaltung, Amidknüpfung) wie bei Beispiel 161 ausgehend von (4-Brombenzyl)-carbaminsäure-*tert-*butylester.
C₂₃H₁₉ClN₆O₂ (446.89)
Massenspektroskopie (ESI): [M+H]⁺ = 447
Rₜ = 2.10 min. Methode 13

### Beispiel 163: (S)-Pyrimidin-5-carbonsäure (3-{1-[5-(4-chlor-2-trifluormethyl-phenylamino)-pyridin-2-yl]-ethylcarbamoyl}-tetrahydrofuran-3-yl)-amid

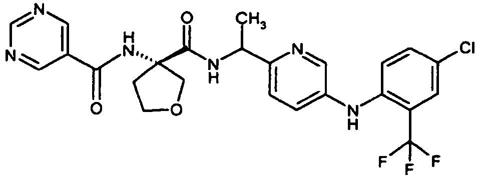

### 163a) 1-[5-(4-Chlor-2-trifluormethyl-phenylamino)-pyridin-2-yl]-ethanon

Zu einer Lösung von 2.2 mL 3 molarem Methylmagnesiumbromid in Diethylether wurden bei -5°C 500 mg (1.68 mmol) 5-(4-Chlor-2-trifluormethyl-phenylamino)-pyridin-2-carbonitril in 5 mL Diethylether getropft. Das Reaktionsgemisch wurde mit Ammoniumchlorid-Lösung hydrolysiert und mit 1 molarer Salzsäure und *tert-*Butylmethylether versetzt. Die organische Phase wurde abgetrennt, mit Natriumsulfat getrocknet und eingeengt
Ausbeute: 50% der Theorie
C₁₄H₁₀ClF₃N₂O (314.69)
Massenspektroskopie (ESI): [M+H]⁺ = 315

### 163b) 1-[5-(4-Chlor-2-trifluormethyl-phenylamino)-pyridin-2-yl]-ethanon-oxim

266 mg (0.85 mmol) 1-[5-(4-Chlor-2-trifluormethyl-phenylamino)-pyridin-2-yl]-ethanon, 73 mg (1.04 mmol) Hydroxylamin-Hydrochlorid und 238 µL (1.69 mmol) Triethylamin wurden in 15 mL Acetonitril über Nacht unter Rückfluss gerührt. Anschließend wurde das Reaktionsgemisch mit Wasser versetzt und mit Dichlormethan extrahiert. Die organische Phase wurde mit Natriumsulfat getrocknet und eingeengt. Der Rückstand wurde ohne weitere Reinigung in der nachstehenden Reaktion eingesetzt.
Ausbeute: 79% der Theorie
C₁₄H₁₁ClF₃N₃O (329.71)
Rₜ = 2.27 min. Methode 12

### 163c) [6-(1-Amino-ethyl)-pyridin-3-yl]-(4-chlor-2-trifluormethyl-phenyl)-amin

220 mg (0.67 mmol) 1-[5-(4-Chlor-2-trifluormethyl-phenylamino)-pyridin-2-yl]-ethanonoxim in 1 mL Methanol wurden portionsweise mit 50 mg Zinstaub und 1.1 mL 4 molarer Salzsäure in Methanol versetzt und dann 3 Stunden unter Rückfluss gerührt. Anschließend gab man zu dem Gemisch Wasser und extrahierte mit Dichlormethan. Die organischen Phasen wurden mit Natriumsulfat getrocknet und eingeengt.
Ausbeute: 62% der Theorie
C₁₄H₁₃ClF₃N₃ (315.72)
Rₜ = 1.76 min. Methode 12

### 163d) (S)-Pyrimidin-5-carbonsäure-(3-{1-[5-(4-chlor-2-trifluormethyl-phenylamino)-pyridin-2-yl]-ethylcarbamoyl}-tetrahydrofuran-3-yl)-amid

Die Verbindung wurde aus (S)-3-[(Pyrimidin-5-carbonyl)-amino]-tetrahydrofuran-3-carbonsäure und [6-(1-Amino-ethyl)-pyridin-3-yl]-(4-chlor-2-trifluormethyl-phenyl)-amin analog Vorschrift (51 b) erhalten.
C₂₄H₂₂ClF₃N₆O₃ (534.93)
Massenspektroskopie (ESI): [M+H]⁺ = 535
Rₜ = 1.86 min. Methode 12

### Beispiel 164: Pyrimidin-5-carbonsäure (1-{1-[5-(4-chlor-2-trifluormethyl-phenylamino)-pyridin-2-yl]-ethylcarbamoyl}-cyclopropyl)-amid Trifluoracetat

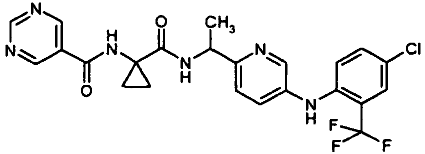

Erhalten analog Vorschrift (1d) aus [6-(1-Amino-ethyl)-pyridin-3-yl]-(4-chlor-2-trifluormethyl-phenyl)-amin und 1-[(Pyrimidin-5-carbonyl)-amino]-cyclopropancarbonsäure.
C₂₃H₂₀ClF₃N₆O₂* C₂HF₃O₂ (618.92)
Massenspektroskopie (ESI): [M+H]⁺ = 505
Rₜ = 1.84 min. Methode 12

### Beispiel 165: [5-(4-Chlor-2-trifluormethyl-phenylamino)-3-fluor-pyridin-2-ylmethyl]-carbaminsäure-tert-butylester

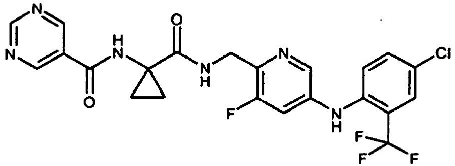

### 165a) [5-(4-Chlor-2-trifluormethyl-phenylamino)-3-fluor-pyridin-2-ylmethyl]-carbaminsäure-tert-butylester

Herstellung analog Vorschrift (55a) aus (5-Brom-3-fluor-pyridin-2-ylmethyl)-carbaminsäure-*tert-*butylester und 4-Chlor-2-trifluormethyl-phenylamin.
C₁₈H₁₈ClF₄N₃O₂ (419.80)
Massenspektroskopie (ESI): [M+H]⁺ = 420

### 165b) (6-Aminomethyl-5-fluor-pyridin-3-yl)-(4-chlor-2-trifluormethyl-phenyl)-amin Hydrochlorid

50 mg (0.12 mmol) [5-(4-Chlor-2-trifluormethyl-phenylamino)-3-fluor-pyridin-2-ylmethyl]-carbaminsäure-*tert-*butylester in 3 mL Dioxan wurden mit 2 mL halbkonzentrierter Salzsäure versetzt und zwei Stunden bei 60°C gerührt. Nach dem Einengen des Reaktionsgemisches wurden Wasserreste durch azeotrope Destillation mit Toluol entfernt.
Rₜ = 1.73 min. Methode 12

### 165c) [5-(4-Chlor-2-trifluormethyl-phenylamino)-3-fluor-pyridin-2-ylmethyl]-carbaminsäure-tert-butylester

Aus (6-Aminomethyl-5-fluor-pyridin-3-yl)-(4-chlor-2-trifluormethyl-phenyl)-amin Hydrochlorid und 1-[(Pyrimidin-5-carbonyl)-amino]-cyclopropancarbonsäure analog Vorschrift (1d) hergestellt.
C₂₂H₁₇ClF₄N₆O₂ (508.86)
Massenspektroskopie (ESI): [M+H]⁺ = 509
R₁ = 2.12 min. Methode 12

### Beispiel 166: Pyrimidin-5-carbonsäure-(1-{[5-(2,4-dichlor-phenylamino)-pyridin-2-yl-methyl]-carbamoyl}-cyclopropyl)-amid

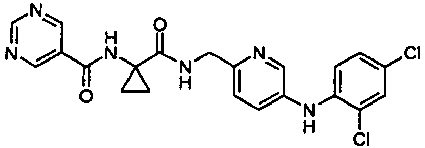

### 166a) 5-(2,4-Dichlor-phenylamino)-pyridin-2-carbonitril

1.33 g (8.2 mmol) 2,4-Dichloranilin in 30 mL DMSO wurden mit 1.38 g (12.3 mmol) Kalium-*tert-*butylat versetzt und eine Stunde bei Raumtemperatur gerührt. Anschließend gab man 1.00 g (8.2 mmol) 2-Cyan-5-fluorpyridin in 20 mL DMSO zu und ließ weitere sechs Stunden rühren. Es wurde mit Dichlormethan verdünnt, mit Natriumchlorid-Lösung gewaschen, mit Natriumsulfat getrocknet und eingeengt. Der Rückstand wurde an einer Kieselgelsäule (Laufmittel: Petrolether/Essigsäureethylester = 4:1) chromatographiert.
Ausbeute: 44% der Theorie
C₁₂H₇Cl₂N₃ (264.11)
Rₜ = 2.46 min. Methode 12

### 166b) (6-Aminomethyl-pyridin-3-yl)-(2,4-dichlor-phenyl)-amin

3.56 mL einer 2 molaren Lösung von Lithiumaluminiumhydrid in THF wurden bei -10°C zu 0.94 g (3.6 mmol) 5-(2,4-Dichlor-phenylamino)-pyridin-2-carbonitril in 60 mL THF gegeben. Das Gemisch wurde 30 Minuten bei Raumtemperatur gerührt, dann mit Wasser versetzt und filtriert. Der Feststoff wurde mit THF gewaschen und das Filtrat bis zur Trockne eingedampft.
C₁₂H₁₁Cl₂N₃ (268.14)
Massenspektroskopie (ESI): [M+H]⁺ = 268

### 166c) Pyrimidin-5-carbonsäure-(1-{[5-(2,4-dichlor-phenylamino)-pyridin-2-ylmethyl]-carbamoyl}-cyclopropyl)-amid

Erhalten aus (6-Aminomethyl-pyridin-3-yl)-(2,4-dichlor-phenyl)-amin und 1-[(Pyrimidin-5-carbonyl)-amino]-cyclopropancarbonsäure analog Vorschrift (1d).
C₂₁H₁₈C₁₂N₆O₂ (457.31)
Massenspektroskopie (ESI): [M+H]⁺ = 457

### Beispiel 167: Pyrimidin-5-carbonsäure (1-{[5-(2-brom-4-chlor-phenylamino)-pyridin-2-ylmethyl]-carbamoyl}-cyclopropyl)-amid

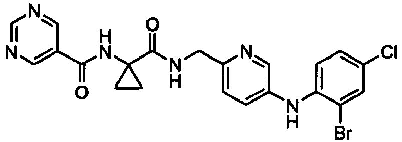

### 167a) 5-(2-Brom-4-chlor-phenylamino)-pyridin-2-carbonitril

Herstellung aus 2-Brom-4-chloranilin und 2-Cyan-5-fluorpyridin analog (166a).
C₁₂H₇BrClN₃ (308.56)
Massenspektroskopie (ESI): [M+H]⁺ = 308

### 167b) (6-Aminomethyl-pyridin-3-yl)-(2-brom-4-chlor-phenyl)-amin-Trifluoracetat

5-(2-Brom-4-chlor-phenylamino)-pyridin-2-carbonitril wurde analog Vorschrift (166b) mit Lithiumaluminiumhydrid reduziert. Abweichend erfolgte die abschließende Reinigung mittels Chromatographie (RP mit Fließmittelgradient, Fließmittel: Acetonitril und Wasser mit 0. 1 % Trifluoressigsäure).
C₁₂H₁₁BrClN₃ (312.59)
Massenspektroskopie (ESI): [M+H]⁺ = 312

### 167c) Pyrimidin-5-carbonsäure-(1-{[5-(2-brom-4-chlor-phenylamino)-pyridin-2-ylmethyl]-carbamoyl}-cyclopropyl)-amid

Analog Vorschrift (1 d) aus (6-Aminomethyl-pyridin-3-yl)-(2-brom-4-chlor-phenyl)-amin-Trifluoracetat und 1-[(Pyrimidin-5-carbonyl)-amino]-cyclopropancarbonsäure.
C₂₁H₁₈BrClN₆O₂ (501.76)
Massenspektroskopie (ESI): [M+H]⁺ = 501

### Beispiel 168: 6-Methylamino-pyrazin-2-carbonsäure-(1-{[5-(4-fluor-2-trifluormethylphenylamino)-pyridin-2-ylmethyl]-carbamoyl}-cyclopropyl)-amid

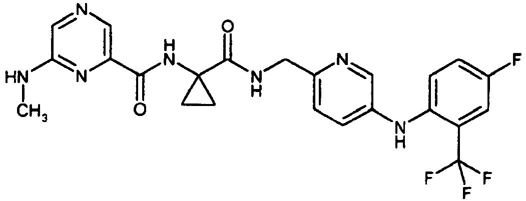

61 mg (0.40 mmol) 6-Methylamino-pyrazin-2-carbonsäure, 162 mg (0.40 mmol) 1-Amino-cyclopropancarbonsäure [5-(4-fluor-2-trifluormethyl-phenylamino)-pyridin-2-ylmethyl]-amid Hydrochlorid und 167 µL (1.20 mmol) Triethylamin wurden in 7 mL THF und 1 mL DMF vorgelegt, mit 154 mg (0.48 mmol) TBTU versetzt und dann 4 Tage bei Raumtemperatur gerührt. Das THF wurde abdestilliert und der Rückstand chromatographisch gereinigt (RP mit Fließmittelgradient, Fließmittel: Wasser und Acetonitril mit Ameisensäure). Anschließend wurden die produkthaltigen Fraktionen mit Kaliumcarbonat-Lösung alkalisch gestellt. Man destillierte das Acetonitril ab und extrahierte mit Essigsäureethylester. Die organischen Phasen wurden mit Natriumsulfat getrocknet, vom Lösungsmittel befreit und mit Diisopropylether verrieben.
Ausbeute: 34% der Theorie
C₂₃H₂₁F₄N₇O₂ (503.45)
Massenspektroskopie (ESI): [M+H]⁺ = 504

### Beispiel 169: 2-Amino-oxazol-5-carbonsäure (1-{[5-(4-fluor-2-trifluormethyl-phenylamino)-pyridin-2-ylmethyl]-carbamoyl}-cyclopropyl)-amid

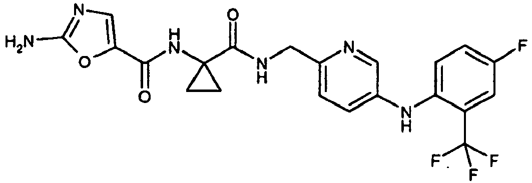

### 169a) [5-(1-{[5-(4-Fluor-2-trifluormethyl-phenylamino)-pyridin-2-ylmethyl]-carbamoyl}-cyclopropylcarbamoyl)-oxazol-2-yl]-carbaminsäure-tert-butylester

Erhalten aus 2-*tert-*Butoxycarbonylamino-oxazol-5-carbonsäure und 1-Amino-cyclopropancarbonsäure [5-(4-fluor-2-trifluormethyl-phenylamino)-pyridin-2-ylmethyl]-amid Hydrochlorid analog der Vorschrift für Beispiel 168. Abweichend wurde bei der Aufarbeitung auf eine chromatographische Reinigung verzichtet.
C₂₆H₂₆F₄N₆O₅ (578.52)
Rₜ = 3.19 min. Methode 14

### 169b) 2-Amino-oxazol-5-carbonsäure (1-{[5-(4-fluor-2-trifluormethyl-phenylamino)-pyridin-2-ylmethyl]-carbamoyl}-cyclopropyl)-amid

Die Schutzgruppe der Verbindung [5-(1-{[5-(4-Fluor-2-trifluormethyl-phenylamino)-pyridin-2-ylmethyl]-carbamoyl}-cyclopropylcarbamoyl)-oxazol-2-yl]-carbaminsäure-*tert-*butylester wurde nach dem für die Zwischenstufe (144b) beschriebenen Verfahren abgespalten.
C₂₁H₁₈F₄N₆O₃ (478.40)
Massenspektroskopie (ESI): [M+H]⁺ = 479
Rₜ =2.71 min. Methode 7

### Beispiel 170: 1-(2-Cyan-2-methyl-acetylamino)-cyclopropancarbonsäure [5-(4-fluor-2-trifluormethyl-phenylamino)-pyridin-2-ylmethyl]-amid

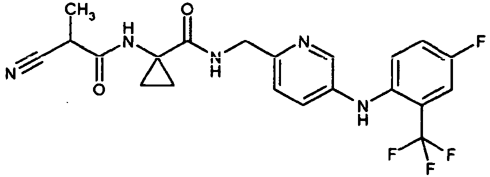

Umsetzung von 40 mg (0.40 mmol) Cyanmethyl-essigsäure und 162 mg (0.40 mmol) 1-Amino-cyclopropancarbonsäure [5-(4-fluor-2-trifluormethyl-phenylamino)-pyridin-2-yl-methyl]-amid Hydrochlorid analog zur Vorschrift für Beispiel 168.
Ausbeute: 28% der Theorie
C₂₁H₁₉F₄N₅O₂ (449.40)
Massenspektroskopie (ESI): [M+H]⁺ = 450
Rₜ = 3.03 min. Methode 7

### Beispiel 171: N-(1-{[5-(4-Brom-2-trifluormethyl-phenylamino)-pyridin-2-ylmethyl]-carbamoyl}-cyclopropyl)-2-hydroxy-isonikotinamid

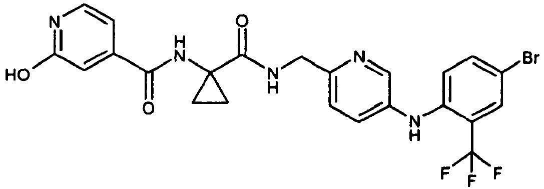

Zu einer Lösung von 23 mg (0.165 mmol) 2-Hydroxyisonikotinsäure, 56 mg (0.174 mmol) TBTU und 114 µL (0.661 mol) DIPEA in 0.5 mL DMF wurden nach 5 Minuten Rühren bei Raumtemperatur 150 mg (0.165 mmol) 1-Amino-cyclopropancarbonsäure [5-(4-brom-2-trifluormethyl-phenylamino)-pyridin-2-ylmethyl]-amid Tri-trifluoracetat gegeben. Anschließend wurde das Reaktionsgemisch über Nacht stehengelassen und dann chromatographiert (RP mit Gradient, Fließmittel: Acetonitril und Wasser mit 0.2% Trifluoressigsäure).
Ausbeute: 69% der Theorie
C₂₃H₁₉BrF₃N₅O₃ (550.33)
Massenspektroskopie (ESI): [M+H]⁺ = 550
Rₜ = 1.73 min. Methode 12

Analog wurden die nachfolgenden Beispielen 172 bis 179 aus 1-Amino-cyclopropancarbonsäure-[5-(4-brom-2-trifluormethyl-phenylamino)-pyridin-2-ylmethyl]-amid Tri-trifluoracetat und den entsprechenden Säuren hergestellt.

### Beispiel 172: Thiazol-5-carbonsäure-(1-{[5-(4-brom-2-trifluormethyl-phenylamino)-pyridin-2-ylmethyl]-carbamoyl}-cyclopropyl)-amid

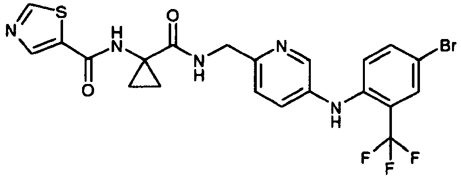

Ausbeute: 94% der Theorie
C₂₁H₁₇BrF₃N₅O₂S (540.36)
Massenspektroskopie (ESI): [M+H]⁺ = 540
Rₜ = 1.88 min. Methode 12

### Beispiel 173: 6-Amino-N-(1-{[5-(4-brom-2-trifluormethyl-phenylamino)-pyridin-2-ylmethyl]-carbamoyl}-cyclopropyl)-nikotinamid Trifluoracetat

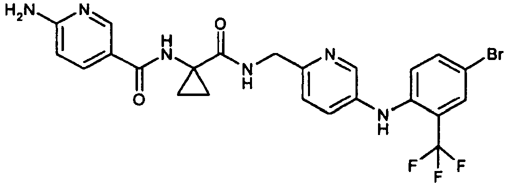

Ausbeute: 89% der Theorie
C₂₃H₂₀BrF₃N₆O₂* C₂HF₃O₂ (663.37)
Massenspektroskopie (ESI): [M+H]⁺ = 549

### Beispiel 174: Pyridazin-4-carbonsäure-(1-{[5-(4-brom-2-trifluormethyl-phenylamino)-pyridin-2-ylmethyl]-carbamoyl}-cyclopropyl)-amid

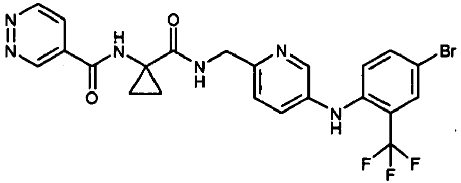

Ausbeute: 71% der Theorie
C₂₂H₁₈BrF₃N₆O₂ (535.32)
Massenspektroskopie (ESI): [M+H]⁺ = 535
Rₜ = 1.80 min. Methode 12

### Beispiel 175: 2-Dimethylamino-pyrimidin-5-carbonsäure-(1-{[5-(4-brom-2-trifluormethylphenylamino)-pyridin-2-ylmethyl]-carbamoyl}-cyclopropyl)-amid

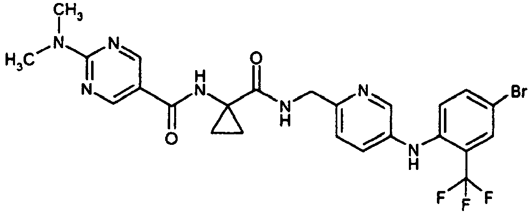

Ausbeute: 67% der Theorie
C₂₄H₂₃BrF₃N₇O₂ (578.39)
Massenspektroskopie (ESI): [M+H]⁺ = 578
R₁ = 1.99 min. Methode 12

### Beispiel 176: 2,6-Dihydroxy-pyrimidin-4-carbonsäure-(1-{[5-(4-brom-2-trifluormethylphenylamino)-pyridin-2-ylmethyl]-carbamoyl}-cyclopropyl)-amid

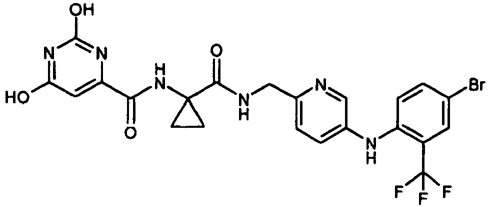

Ausbeute: 46% der Theorie
C₂₂H₁₈BrF₃N₆O₄ (567.32)
Massenspektroskopie [M+H]⁺ = 567
Rₜ = 1.74 min. Methode 12

### Beispiel 177: 1-Methyl-2-oxo-1,2-dihydro-pyridin-4-carbonsäure-(1-{[5-(4-brom-2-trifluormethyl-phenylamino)-pyridin-2-ylmethyl]-carbamoyl}-cyclopropyl)-amid

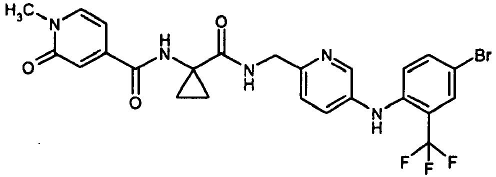

Ausbeute: 71 % der Theorie
C₂₄H₂₁BrF₃N₅O₃ (564.36)
Massenspektroskopie (ESI): [M+H]⁺ = 564
Rₜ = 1.78 min. Methode 12

### Beispiel 178: 5-Amino-N-(1-{[5-(4-brom-2-trifluormethyl-phenylamino)-pyridin-2-ylmethyl]-carbamoyl}-cyclopropyl)-nikotinamid

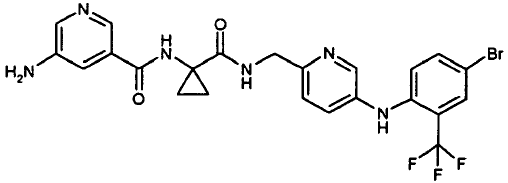

Ausbeute: 99% der Theorie
C₂₃H₂₀BrF₃N₆O₂* C₂HF₃O₂ (663.37)
Massenspektroskopie (ESI): [M+H]⁺ = 549
Rₜ = 1.68 min. Methode 12

### Beispiel 179: Pyrimidin-5-carbonsäure (1-{[5-(4-brom-2-trifluormethyl-phenylamino)-3-fluor-pyridin-2-ylmethyl]-carbamoyl}-cyclopropyl)-amid

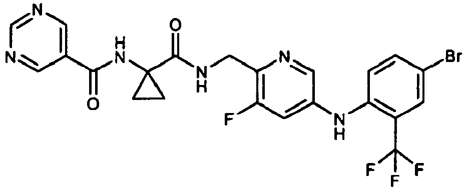

### 179a) 5-(4-Brom-2-trifluormethyl-phenylamino)-3-fluor-pyridin-2-carbonitril

Erhalten aus 2-Cyan-3,5-difluorpyridin und 4-Brom-2-trifluormethyl-phenylamin analog Vorschrift (40a).
C₁₃H₆BrF₄N₃ (360.11)
Massenspektroskopie (ESI): [M+H]⁺ = 360
Rₜ = 2.68 min. Methode 12

### 179b) (6-Aminomethyl-5-fluor-pyridin-3-yl)-(4-brom-2-trifluormethyl-phenyl)-amin

171 mg (0.48 mmol) 5-(4-Brom-2-trifluormethyl-phenylamino)-3-fluor-pyridin-2-carbonitril wurden in 3 mL Pyridin, 1.5 mL Eisessig und 1.5 mL Wasser gelöst und mit 459 mg (5.22 mmol) Natriumhypophosphit und Raney-Nickel versetzt. Anschließend wurde über drei Stunden bei 55°C und 3 bar Wasserstoffdruck hydriert. Der Katalysator wurde abfiltriert, das Filtrat bis zur Trockne eingedampft und der Rückstand chromatographisch (RP mit Gradient, Fließmittel: Acetonitril und Wasser mit 0.2 % Trifluoressigsäure) gereinigt.
C₁₃H₁₀BrF₄N₃ (364.14)
Massenspektroskopie (ESI): [M+H]⁺ = 364
Rₜ = 1.79 min. Methode 13

### 179c) Pyrimidin-5-carbonsäure (1-{[5-(4-brom-2-trifluormethyl-phenylamino)-3-fluorpyridin-2-ylmethyl]-carbamoyl}-cyclopropyl)-amid

54 mg (0.26 mmol) 1-[(Pyrimidin-5-carbonyl)-amino]-cyclopropancarbonsäure, 36 µL Triethylamin und 105 mg (0.31 mmol) TBTU in 4 mL DMF wurden 5 Minuten bei Raumtemperatur gerührt und dann mit weiteren 144 µL Triethylamin und 95 mg (0.26 mmol) (6-Aminomethyl-5-fluor-pyridin-3-yl)-(4-brom-2-trifluormethyl-phenyl)-amin versetzt. Das Reaktionsgemisch wurde über Nacht gerührt und dann bis zur Trockne eingedampft.

Den Rückstand reinigte man mittels Chromatographie (RP mit Gradient, Fließmittel: Acetonitril und Wasser mit 0.2 % Trifluoressigsäure).
C₂₂H₁₇BrF₄N₆O₂ (553.31)
Massenspektroskopie (ESI): [M+H]⁺ = 553
Rₜ = 2.20 min. Methode 13

### Beispiel 180: Pyrimidin-5-carbonsäure-(1-{1-[5-(4-brom-2-trifluormethyl-phenylamino)-pyridin-2-yl]-ethylcarbamoyl}-cyclopropyl)-amid

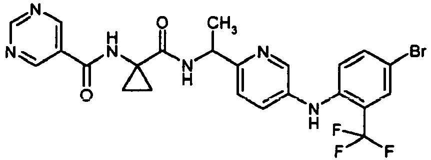

### 180a) 1-[5-(4-Brom-2-trifluormethyl-phenylamino)-pyridin-2-yl]-ethanon

Eine Lösung von 3.27 g (9.56 mmol) 5-(4-Brom-2-trifluormethyl-phenylamino)-pyridin-2-carbonitril in 100 mL Diethylether wurde unter Kühlung mit dem Eisbad zu 5.42 mL 3 molarem Methylmagnesiumbromid in Diethylether getropft. Anschließend ließ man das Reaktionsgemisch auf Raumtemperatur kommen und noch eine Stunde rühren. Es wurde mit 2.5 mL 1 molarer Salzsäure versetzt und dann bis zur Tockne eingedampft. Der Rückstand wurde chromatographisch (RP mit Gradient, Fließmittel: Acetonitril und Wasser mit 0.2%Trifluoressigsäure) gereinigt.
C₁₄H₁₀BrF₃N₂O (359.14)
Rₜ = 2.57 min. Methode 12

### 180b) 1-[5-(4-Brom-2-trifluormethyl-phenylamino)-pyridin-2-yl]-ethanon-oxim

702 mg (1.96 mmol) 1-[5-(4-Brom-2-trifluormethyl-phenylamino)-pyridin-2-yl]-ethanon, 182 mg Hydroxylamin-Hydrochlorid und 549 µl (3.91 mmol) Triethylamin in 25 mL Acetonitril wurden 1.5 Stunden unter Rückfluss erwärmt. Das Lösungsmittel wurde abdestilliert und der Rückstand mit Dichlormethan und Triethylamin versetzt und über Kieselgel filtriert. Das Filtrat wurde vom Lösungsmittel befreit und in der nachstehenden Reaktion direkt weiter eingesetzt. 870 mg Produkt.

### 180c) [6-(1-Amino-ethyl)-pyridin-3-yl]-(4-brom-2-trifluormethyl-phenyl)-amin Trifluoracetat

Eine Lösung von 870 mg (ca. 85%ig, 2.0 mmol) 1-[5-(4-Brom-2-trifluormethyl-phenylamino)-pyridin-2-yl]-ethanon-oxim in 20 mL Methanol wurde mit 6 mL 10 molarer Salzsäure in Methanol und 567 mg Zink versetzt und 3 Stunden unter Rückfluss gerührt. Anschließend wurde filtriert und das Filtrat vom Lösungsmittel befreit. Der Rückstand wurde chromatographisch gereinigt (RP mit Gradient, Fließmittel: Acetonitril und Wasser mit 0.2% Trifluoressigsäure).
Ausbeute: 78% über zwei Stufen
C₁₄H₁₃BrF₃N₃ (360.17)
Massenspektroskopie (ESI): [M+H]⁺ = 360
Rₜ= 1.83 min. Methode 12

### 180d) Pyrimidin-5-carbonsäure-(1-{1-[5-(4-brom-2-trifluormethyl-phenylamino)-pyridin-2-yl]-ethylcarbamoyl}-cyclopropyl)-amid

Analog Vorschrift (179c) aus 190 mg (0.92 mmol) 1-[(Pyrimidin-5-carbonyl)-amino]-cyclopropancarbonsäure und 330 mg (0.69 mmol) [6-(1-Amino-ethyl)-pyridin-3-yl]-(4-brom-2-trifluormethyl-phenyl)-amin Trifluoracetat.
Ausbeute: 55% der Theorie
C₂₃H₂₀BrF₃N₆O₂·C₂HF₃O₂ (663.37)
Massenspektroskopie (ESI): [M+H]⁺ = 549
Rₜ = 1.92 min. Methode 12

Das (R)- und (S)-Enantiomer von Beispiel 180 wurde mittels chiraler HPLC (SFC) aus der racemischen Verbindung erhalten (Säule: Daicel ASH, 250 mm x 10 mm, Flussrate: 10 mL/min, Fließmittel: 70% überkritisches Kohlendioxid und 30% Isopropanol mit 0.2% Triethylamin).

### Beispiel 181: 2-Methyl-pyrimidin-5-carbonsäure {1-[4-(2-cyan-4-fluor-phenylamino)-benzylcarbamoyl]-cyclopropyl}-amid

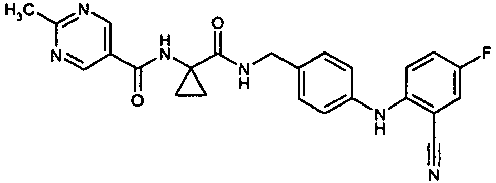

### 181a) {1-[4-(2-Cyan-4-fluor-phenylamino)-benzylcarbamoyl]-cyclopropyl}-carbaminsäure-tert-butylester

877 mg (4.36 mmol) 1-*tert-*Butoxycarbonylamino-cyclopropancarbonsäure und 2.58 (60%ig, 4.36 mmol) 2-(4-Aminomethyl-phenylamino)-5-fluor-benzonitril Trifluoracetat wurden analog Vorschrift (179c) gekuppelt.
Ausbeute: 30% der Theorie
C₂₃H₂₅FN₄O₃ (424.47)
Massenspektroskopie (ESI): [M-H]⁺ = 423
Rₜ = 2.39 min. Methode 13

### 181b) 1-Amino-cyclopropancarbonsäure 4-(2-cyan-4-fluor-phenylamino)-benzylamid Trifluoracetat

560 mg (1.32 mmol) 2-(4-Aminomethyl-phenylamino)-5-fluor-benzonitril Trifluoracetat in 15 mL Dichlormethan wurden mit 15 mL Trifluoressigsäure versetzt und bei Raumtemperatur gerührt. Anschließend wurde das Reaktionsgemisch bis zur Trockne eingedampft und chromatographisch gereinigt (RP mit Gradient, Fließmittel: Acetonitril und Wasser mit 0.2% Trifluoressigsäure).
C₁₈H₁₇FN₄O* C₂HF₃O₂ (438.38)
Massenspektroskopie (ESI): [M-H]⁺ = 325
Rₜ = 1.56 min. Methode 13

### 181c) 2-Methyl-pyrimidin-5-carbonsäure-{1-[4-(2-cyan-4-fluor-phenylamino)-benzylcarbamoyl]-cyclopropyl}-amid

Analog Vorschrift (179c) aus 42 mg (0.29 mmol) 2-Methyl-pyrimidin-5-carbonsäure und 167 mg (75%ig, 0.29 mmol) 1-Amino-cyclopropancarbonsäure-4-(2-cyan-4-fluor-phenylamino)-benzylamid Trifluoracetat.
Ausbeute: 83% der Theorie
C₂₄H₂₁FN₆O₂ (444.46)
Massenspektroskopie (ESI): [M+H]⁺ = 445
Rₜ = 1.92 min. Methode 13

Die Beispiele 182 und 183 wurden analog aus 1-Amino-cyclopropancarbonsäure-4-(2-cyan-4-fluor-phenylamino)-benzylamid Trifluoracetat und den entsprechenden Carbonsäuren erhalten.

### Beispiel 182: 2-Methoxy-pyrimidin-5-carbonsäure-{1-[4-(2-cyan-4-fluor-phenylamino)-benzylcarbamoyl]-cyclopropyl}-amid

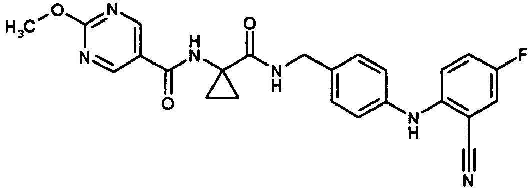

Ausbeute: 28% der Theorie
C₂₄H₂₁FN₆O₃ (460.46)
Massenspektroskopie (ESI): [M+H]⁺ = 461
Rₜ = 2.03 min. Methode 13

### Beispiel 183: 2-Methylamino-pyrimidin-5-carbonsäure-{1-[4-(2-cyan-4-fluor-phenylamino)-benzylcarbamoyl]-cyclopropyl}-amid

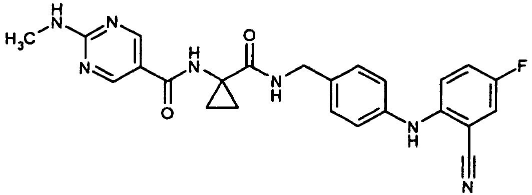

Ausbeute: 58% der Theorie
C₂₄H₂₂FN₇O₂ (459.48)
Massenspektroskopie (ESI): [M+H]⁺ = 460
Rₜ = 1.91 min. Methode 13

### Beispiel 184: 2-Amino-thiazol-5-carbonsäure-(1-{[5-(4-fluor-2-trifluormethyl-phenylamino)-pyridin-2-ylmethyl]-carbamoyl}-cyclopropyl)-amid

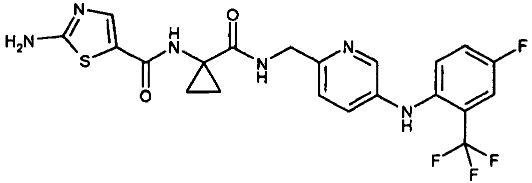

### 184a) 2-Acetylamino-thiazol-5-carbonsäure-(1-{[5-(4-fluor-2-trifluormethyl-phenylamino)-pyridin-2-ylmethyl]-carbamoyl}-cyclopropyl)-amid

Aus 1-Amino-cyclopropancarbonsäure-[5-(4-fluor-2-trifluormethyl-phenylamino)-pyridin-2-ylmethyl]-amid Hydrochlorid und 2-Acetylamino-thiazol-5-carbonsäure analog Vorschrift (142c).
Ausbeute: 51% der Theorie
C₂₃H₂₀F₄N₆O₃S (536.50)
Rₜ = 2.96 min. Methode 7

### 184b) 2-Amino-thiazol-5-carbonsäure-(1-{[5-(4-fluor-2-trifluormethyl-phenylamino)-pyridin-2-ylmethyl]-carbamoyl}-cyclopropyl)-amid

110 mg (0.21 mmol) 2-Acetylamino-thiazol-5-carbonsäure (1-{[5-(4-fluor-2-trifluormethylphenylamino)-pyridin-2-ylmethyl]-carbamoyl}-cyclopropyl)-amid wurden über Nacht bei 80°C in 5 mL 4 molarer Salzsäure gerührt. Anschließend wurde die Reaktionsmischung mit Kaliumcarbonat-Lösung alkalisch gestellt und der ausfallende Feststoff abfiltriert, mit Wasser gewaschen und getrocknet.
Ausbeute: 43% der Theorie
C₂₁H₁₈F₄N₆O₂S (494.47)
Massenspektroskopie (ESI): [M+H]⁺ = 495
Rₜ = 2.73 min. Methode 7

Die Beispiele 185 und 186 wurden analog aus 1-Amino-cyclopropancarbonsäure-[5-(4-fluor-2-trifluormethyl-phenylamino)-pyridin-2-ylmethyl]-amid Hydrochlorid und den entsprechenden Acetylamino-carbonsäure hergestellt.

### Beispiel 185: 5-Amino-2H-pyrazol-3-carbonsäure-(1-{[5-(4-fluor-2-trifluormethyl-phenylamino)-pyridin-2-ylmethyl]-carbamoyl}-cyclopropyl)-amid

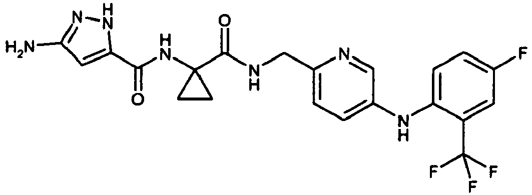

C₂₁H₁₉F₄N₇O₂ (477.42)
Massenspektroskopie (ESI): [M+H]⁺ = 478
Rₜ = 2.69 min. Methode 7

### Beispiel 186: 2-Amino-4-methyl-thiazol-5-carbonsäure-(1-{[5-(4-fluor-2-trifluormethylphenylamino)-pyridin-2-ylmethyl]-carbamoyl}-cyclopropyl)-amid

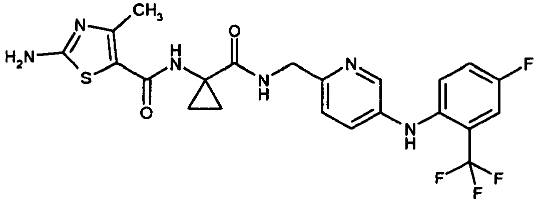

C₂₂H₂₀F₄N₆O₂S (508.49)
Massenspektroskopie (ESI): [M+H]⁺ = 509
Rₜ = 2.67 min. Methode 7

### Beispiel 187: Pyrimidin-5-carbonsäure (1-{[3-chlor-5-(4-fluor-2-trifluormethyl-phenylamino)-pyridin-2-ylmethyl]-carbamoyl}-cyclopropyl)-amid Dihydrochlorid

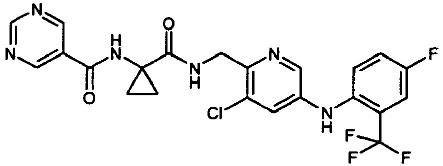

### 187a) 3-Chlor-5-(4-fluor-2-trifluormethyl-phenylamino)pyridin-2-carbonitril

Eine Lösung aus 5.00 g (28.9 mmol) 3,5-Dichlor-pyridin-2-carbonitril und 5.18 g (28.9 mmol) 4-Fluor-2-trifluormethyl-phenylamin in 75 mL DMSO wurde unter Kühlung mit 5.05 g (45.0 mmol) Kalium-*tert-*butylat versetzt und dann 30 Minuten bei Raumtemperatur gerührt. Das Reaktionsgemisch wurde in Wasser eingerührt und dann mit Diethylether extrahiert. Die organischen Phasen wurden mit Wasser und Natriumchlorid-Lösung gewaschen, mit Natriumsulfat getrocknet und eingeengt. Den Rückstand reingte man chromatographisch über eine Kieselgelsäule (Petrolether mit 5 bis 15% Essigsäureethylester).
Ausbeute: 45% der Theorie

### 187b) (6-Aminomethyl-5-chlor-pyridin-3-yl)-(4-fluor-2-trrifluormethyl-phenyl)-amin

Eine Lösung aus 100 mg (0.32 mmol) 3-Chlor-5-(4-fluor-2-trifluormethyl-phenylamino)-pyridin-2-carbonitril in 3 mL THF wurde bei Raumtemperatur mit 31 µL Boran-dimethylsulfid-Komplex versetzt und dann über Nacht gerührt. Anschließend wurde vorsichtig Methanol zugegeben und das Gemisch bis zur Trockne eingedampft. Der Rückstand wurde ohne weitere Reinigung in der nachstehenden Reaktion eingesetzt.

### 187c) Pyrimidin-5-carbonsäure (1-{[3-chlor-5-(4-fluor-2-trifluormethyl-phenylamino)-pyridin-2-ylmethyl]-carbamoyl}-cyclopropyl)-amid Dihydrochlorid

Aus (6-Aminomethyl-5-chlor-pyridin-3-yl)-(4-fluor-2-trifluormethyl-phenyl)-amin und 1-[(Pyrimidin-5-carbonyl)-amino]-cyclopropancarbonsäure analog zur Vorschrift für Beispiel 151 hergestellt..
CnH₁₇ClF₄N₆O₂* 2 HCl (581.78)
Massenspektroskopie (ESI): [M+H]⁺ = 509
Rₜ = 3.68 min. Methode 10

### Beispiel 188: Pyrimidin-5-carbonsäure-(3-{1-[5-(4-brom-2-trifluormethyl-phenylamino)-pyridin-2-yl]-ethylcarbamoyl}-(S)-tetrahydrofuran-3-yl)-amid

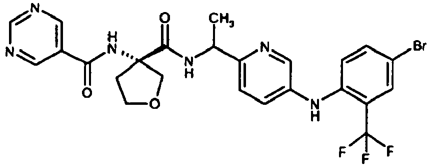

Eine Lösung von 59 mg (0.25 mmol) (S)-3-[(Pyrimidin-5-carbonyl)-amino]-tetrahydrofuran-3-carbonsäure und 48 mg (0.30 mmol) N,N'-Carbonyldiimidazol in 5 mL DMF wurde eine Stunde bei 50°C gerührt und dann mit 89 mg (0.25 mmol) [6-(1-Amino-ethyl)-pyridin-3-yl]-(4-brom-2-trifluormethyl-phenyl)-amin und 45 µL (0.26 mmol) DIPEA versetzt. Anschließend ließ man weiter eine Stunde bei Raumtemperatur rühren. Das Reaktionsgemisch wurde chromatographisch gereinigt (RP mit Fließmittelgradient, Fließmittel: Acetonitril und Wasser mit 0.2 % Trifluoressigsäure).
Ausbeute: 41 % der Theorie
C₂₄H₂₂BrF₃N₆O₃ (579.37)
Massenspektroskopie [M+H]⁺ = 579
Rₜ= 1.93 min. Methode 12

### Beispiel 188a: Pyrimidin-5-carbonsäure-(3-{1-[5-(4-brom-2-trifluormethyl-phenylamino)-pyridin-2-yl]-ethylcarbamoyl}-tetrahydrofuran-3-yl)-amid

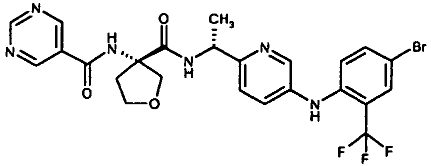

Diastereomerentrennung mittels chiraler HPLC (Säule: Daicel ASH; 250 x 4.6 mm; 5 µm; 25°C; Eluent CO₂ / (Isopropanol + 0.2% Diethylamin) 80:20; Fluß: 10 mL/min)
Rₜ = 5.32 - 7.15 Minuten

### Beispiel 188b: Pyrimidin-5-carbonsäure (3-{1-[5-(4-brom-2-trifluormethyl-phenylamino)-pyridin-2-yl]-ethylcarbamoyl}-tetrahydrofuran-3-yl)amid

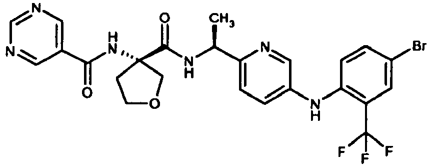

Diastereomerentrennung mittels chiraler HPLC (Säule: Daicel ASH; 250 x 4.6 mm; 5 µm; 25°C; Eluent CO₂ / (Isopropanol + 0.2% Diethylamin) 80:20; Fluß: 10 mL/min)
Rₜ = 8.23 - 10.51 Minuten

### Beispiel 189: Pyrimidin-5-carbonsäure (1-{[5-(2-chlor-4-methyl-phenylamino)-pyridin-2-yl-methyl]-carbamoyl}-cyclopropyl)-amid

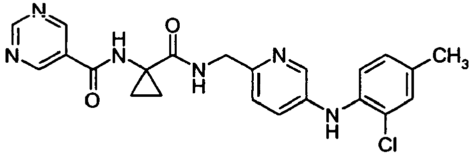

Analog Beispiel 1d) wurde das Produkt durch Amidkupplung aus 1-[(Pyrimidin-5-carbonyl)-amino]-cyclopropan carbonsäure und (6-Aminomethyl-pyridin-3-yl)-(2-chlor-4-methylphenyl)-amin unter Verwendung von TBTU als Kupplungsreagenz und Diisoproylethylamin als Base hergestellt.
C₂₂H₂₁ClN₆O₂ (436.90)
Massenspektrum (ESI): [M+H]⁺ = 437
[M-H]⁻ = 435
Rₜ = 1.59 min (Methode 2)

### Beispiel 190: Pyrimidin-5-carbonsäure (1-{[5-(2-chlor-4-fluor-phenylamino)-pyridin-2-y)-methyl]-carbamoyl}-cyclopropyl)-amid

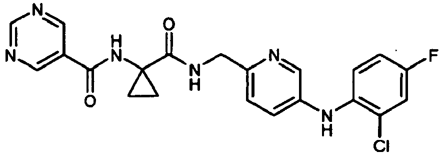

Analog Beispiel 1d) wurde das Produkt durch Amidkupplung aus 1-[(Pyrimidin-5-carbonylyamino]-cyclopropan-carbonsäure und (6-Aminomethyl-pyridin-3-yl)-(2-chlor-4-fluorphenyl)-amin hergestellt, wobei TBTU als Kupplungsreagenz und Diisoproylethylamin als Base eingesetzt wurden.
C₂₁H₁₆CIFN₆O₂ (440.86)
Massenspektrum (ESI): [M+H]⁺ = 441
Rₜ = 1.50 min (Methode 2)

### Beispiel 191: Pyrimidin-5-carbonsäure (1-{[5-(2-trifluormethyl-phenylamino)-pyridin-2-yl-methyl]-carbamoyl}-cyclohexyl)-amid

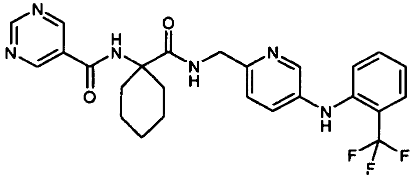

### 191 a) 1-Amino-cyclohexancarbonsäure[5-(2-trifluormethyl-phenviamino)-pyridin-2-yl-methyl]-amid

61 mg (0.25 mmol) 1-*tert*-Butoxycarbonylamino-cyclohexancarbonsäure, 80 mg (0.25 mmol) TBTU und 53 µL (0.38 mmol) Triethylamin in 2 mL DMF wurden 5 Minuten bei Raumtemperatur gerührt und dann mit 67 mg (0.25 mmol) (6-Aminomethyl-pyridin-3-yl)-(2-trifluormethyl-phenyl)-amin versetzt. Das Reaktionsgemisch wurde anschließend weiter über Nacht gerührt und dann chromatographisch gereinigt (RP mit Fließmittelgradient, Fließmittel: Acetonitril und Wasser mit 0.1% Trifluoressigsäure). 93 mg des isolierten Bocgeschützten Amins wurden 2 Stunden bei Raumtemperatur in 5 mL eines 1:1 Gemisches aus Dichlormethan und Trifluoressigsäure gerührt. Das Reaktionsgemisch wurde zur Trockne eingedampft und dann mittels Chromatographie gereinigt (RP mit Fließmittelgradient, Fließmittel: Acetonitril und Wasser mit 0.1 % Trifluoressigsäure).
Ausbeute: 68% der Theorie (als Trifluoracetat)
Massenspektroskopie [M+H]⁺ = 393
Rₜ = 1.70 min Methode 6

### 191b) Pyrimidin-5-carbonsäure-(1-{[5-(2-trifluormethyl-phenylamino)-pyridin-2-ylmethyl]-carbamoyl}-cyclohexyl)-amid

Herstellung aus 1-Amino-cyclohexancarbonsäure [5-(2-trifluormethyl-phenylamino)-pyridin-2-ylmethyl]-amid und Pyrimidin-5-carbonsäure analog Vorschrift 191a).
Ausbeute: 37% der Theorie
C₂₅H₂₅F₃N₆O₂ (498.51)
Massenspektrum (ESI): [M+H]⁺ = 499
[M-H]⁻ = 497
Rₜ = 1.84 min (Methode 5)

### Beispiel 192: Pyrimidin-5-carbonsäure (3-hydroxy-1-{[5-(2-trifluormethyl-phenylamino)-pyridin-2-ylmethyl]-carbamoyl}cyclopentyl)-amid

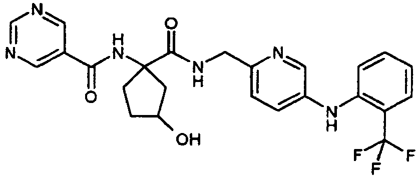

### 192a) 1-Amino-3-hydroxy-cyclopentancarbonsäure [5-(2-trifluormethyl-phenylamino)-pyridin-2-ylmethyl]-amid

1-*tert*-Butoxycarbonylamino-3-hydroxy-cyclopentancarbonsäure und (6-Aminomethylpyridin-3-yl)-(2-trifluormethyl-phenylramin wurden analog Vorschrift 191a) umgesetzt.
Ausbeute: 49% der Theorie (als Trifluoracetat)
Massenspektroskopie [M+H]⁺ = 395
Rₜ= 1.62 min Methode 6

### 192b) Pyrimidin-5-carbonsäure (3-hydroxy-1-{[5-(2-trifluormethyl-phenylamino)-pyridin-2-ylmethyl]-carbamoyl}-cyclopentyl)-amid

Die Zielverbindung wurde aus 1-Amino-3-hydroxy-cyclopentancarbonsäure [5-(2-trifluormethyl-phenylamino)-pyridin-2-ylmethyl]-amid und Pyrimidin-5-carbonsäure analog zur Vorschrift 191a) hergestellt.
Ausbeute: 55% der Theorie
C₂₄H₂₃F₃N₆O₃ (500.48)
Massenspektrum (ESI): [M+H]⁺ = 501
Rₜ = 1.66 min (Methode 5)

### Beispiel 193: Pyrimidin-5-carbonsäure (3-oxo-1-{[5-(2-trifluormethyl-phenylamino)-pyridin-2-ylmethyl]-carbamoyl}-cyclopentyl)-amid

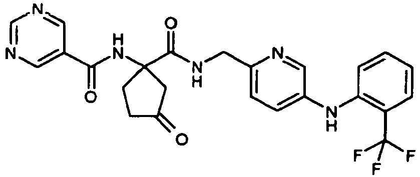

10 mg Pyrimidin-5-carbonsäure (3-hydroxy-1-{[5-(2-trifluormethyl-phenylamino)-pyridin-2-ylmethyl]-carbamoyl}-cyclopentyl)-amid in 1 mL Acetonitril wurden mit 8 mg Dess-Martin-Periodinan Reagenz versetzt und 1 Stunden bei Raumtemperatur gerührt. Anschließend wurde das Reaktionsgemisch chromatographisch gereinigt (RP mit Fließmittelgradient, Fließmittel: Acetonitril und Wasser mit 0. 1 % Trifluoressigsäure).
Ausbeute: 90% der Theorie
C₂₄H₂₁F₃N₆O₃ (498.46)
Massenspektrum (ESI): [M+H]⁺ = 499
[M-H]⁻ = 497
Rₜ = 1.73 min (Methode 5)

### Beispiel 194: Pyrimidin-5-carbonsäure (1-oxo-3-{[5-(2-trifluormethyl-phenylamino}-pyridin-2-ylmethyl]-carbamoyl}-tetrahydro-1lambda*4*-thiophen-3-yl)-amid

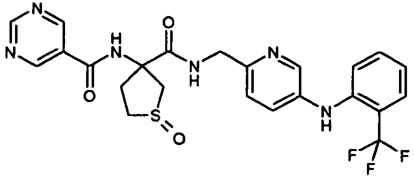

### 194a) 3-Amino-1-oxo-tetrahvdrothiophen-3-carbonsäure [5-(2-trifluormethyl-phenylamino)-pyridin-2-ylmethyl]-amid

Das Produkt wurde aus 3-*tert*-Butoxycarbonylamino-1-oxo-tetrahydro-thiophen-3-carbonsäure und (6-Aminomethyl-pyridin-3-yl)-(2-trifluormethyl-phenyl)-amin in Analogie zur Vorschrift 191a) erhalten.
Ausbeute: 96% der Theorie (als Trifluoracetat)
Massenspektroskopie [M+H]⁺ = 413
Rₜ = 1.62 min Methode 6

### 194b) Pyrimidin-5-carbonsäure (1-oxo-3-{[5-(2-trifluormethyl-phenylamino)-pyridin-2-yl-methyl]-carbamoyl}-tetrahydro-thiophen-3-yl)-amid

Hergestellt aus 3-Amino-1-oxo-tetrahydro-thiophen-3-carbonsäure-[5-(2-trifluormethylphenylamino)-pyridin-2-ylmethyl]-amid und Pyrimidin-5-carbonsäure analog zur Vorschrift 191 a).
Ausbeute: 58% der Theorie
C₂₃H₂₁F₃N₆O₃S (518.52)
Massenspektrum (ESI): [M+H]⁺ = 519
[M+H]⁺ = 517
Rₜ = 1.66 min (Methode 5)

### Beispiel 195: 4-{6-[({1-[(Pyrimidin-5-carbonyl)-amino]-cyclopropancarbonyl}-amino)-methyl]-pyridin-3-ylamino)-3-trifluormethyl-benzoesäure

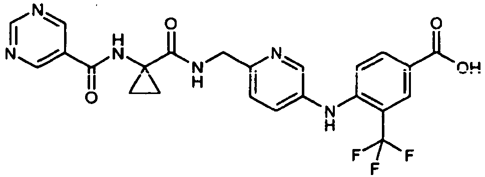

469 (0.91 mmol) 4-{6-[({1-[(Pyrimidin-5-carbonyl)-amino]-cyclopropancarbonyl}-amino)-methyl]-pyridin-3-ylamino}-3-trifluormethyl-benzoesäure-methylester wurden über Nacht in 5 mL 1 N wässriger Natriumhydroxid-Lösung und 20 mL Ethanol bei Raumtemperatur gerührt. Anschließend wurde die Reaktionsmischung mit 1 N wässriger Salzsäure neutralisiert und zur Trockne eingedampft. Der Rückstand wurde in Methanol und DMF gelöst, filtriert und dann chromatographiert (RP mit Fließmittelgradient, Fließmittel: Acetonitril und Wasser mit 0.2 % Trifluoressigsäure).
Ausbeute: 69% der Theorie
C₂₃H₁₉F₃N₆O₄ (500.44)
Massenspektrum (ESI): [M+H]⁺ = 501
[M-H]⁻ = 499

### Beispiel 196: 4-{6-[({1-[(Pyrimidin-5-carbonyl)-amino]-cyclopropanecarbonyl}-amino)-methyl]-pyridin-3-ylamino}-3-trifluormethyl-benzoesäure-methylester

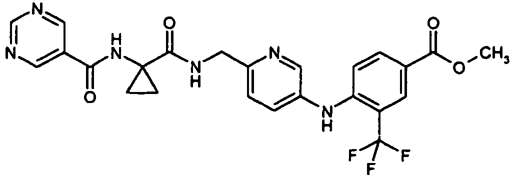

### 196a) 4-(6-Cyano-pyridin-3-ylamino)-3-trifluormethyl-benzoesäure-methylester

1190 mg (3.48 mmol) 5-(4-Brom-2-trifluormethyl-phenylamino)-pyridin-2-carbonitril, 221 µL (1.6 mmol) Triethylamin und 97 mg (0.13 mmol) Pd(ddpf)Cl₂ in 10 mL Methanol und 2 mL DMF wurden in einem Autoklaven bei einem Kohlenmonoxiddruck von 5 bar über 60 Stunden auf 50°C erhitzt. Nach dem Abdestillieren der Lösungsmittel wurde der Rückstand in Acetonitril und Methanol gelöst und filtriert. Das Filtrat wurde anschließend eingeengt und chromatographisch gereinigt (1.Säule: RP mit Fließmittelgradient, Fließmittel: Acetonitril und Wasser mit 0.1% Trifluoressigsäure; 2. Säule: Kieselgel, Fließmittel: Dichlormethan).
Ausbeute: 78% der Theorie
Massenspektroskopie [M+H]⁺ = 322

### 196b) 4-(6-Aminomethyl-pyridin-3-ylamino)-3-trifluormethyl-benzoesäure-methylester

860 mg (2.7 mmol) 4-(6-Cyano-pyridin-3-ylamino)-3-trifluormethyl-benzoesäure-methylester in 30 mL methanolischem Ammoniak wurden bei Raumtemperatur und einem Wasserstoffdruck von 50 psi in Gegenwart von 100 mg Raney-Nickel hydriert. Der Katalysator wurde abfiltriert und das Filtrat von Lösungsmittel befreit.
Ausbeute: 76% der Theorie
Massenspektroskopie [M+H]⁺ = 326

### 196c) 4-{6-[({1-[(Pyrimidin-5-carbonyl)-amino]-cyclopropancarbonyl}-amino)-methyl]-pyridin-3-ylamino}-3-trifluormethyl-benzoesäure-methylester

191 mg (0.92 mmmol) 1-[(Pyrimidin-5-carbonyl)-amino]-cydopropancarbonsäure, 305 mg (0.95 mmol) TBTU und 203 µL (1.85 mmol) N-Methylmorpholin in 3 mL DMF wurden 5 Minuten bei Raumtemperatur gerührt. Die Lösung wurde mit 300 mg (0.92 mmol) 4-(6-Aminomethyl-pyridin-3-ylamino)-3-trifluormethyl-benzoesäure-methylester versetzt und über das Wochenende stehen gelassen. Anschließend wurde das Reaktionsgemisch mittels Chromatographie gereinigt (RP mit Fließmittelgradient, Fließmittel: Acetonitril und Wasser mit 0.2 % Trifluoressigsäure).
Ausbeute: 62% der Theorie
C₂₄H₂₁F₃N₆O₄ (514.46)
Massenspektrum (ESI): [M+H]⁺ = 515
Rₜ = 1.73 min (Methode 12)

### Beispiel 197: Pyrimidin-5-carbonsäure (1-{[5-(4-cyano-2-trifluormethyl-phenylamino)-pyridin-2-ylmethyl]-carbamoyl}-cyclopropyl)-amid

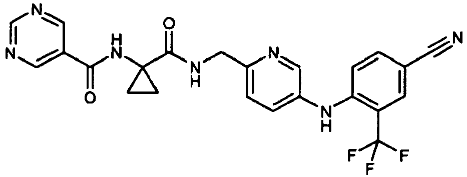

55 mg (0.18 mmol) Pyrimidin-5-carbonsäure {1-[(5-amino-pyridin-2-ylmethyl)-carbamoyl]-cyclopropyl}-amid, 33 mg (0.18 mmol) 4-Fluor-3-(trifluormethyl)benzonitril und 42 mg (0.35 mg) Kalium-*tert*-butylat in 5 mL DMSO wurden 1 h bei 50°C gerührt. Das Reaktionsgemisch wurde filtriert und das Filtrat chromatographisch gereinigt (RP mit Fließmittelgradient, Fließmittel: Acetonitril und Wasser mit 0.2 % Trifluoressigsäure).
Ausbeute: 23% der Theorie
C₂₃H₁₈F₃N₇O₂ (481.44)
Massenspektrum (ESI): [M+H]⁺ = 482
[M-H]⁻ = 480
Rₜ = 1.69 min (Methode 12)

### Beispiel 198: Pyrimidin-5-carbonsäure (1-{[5-(4-carbamoyl-2-trifluormethyl-phenylamino)-pyridin-2-ylmethyl]-carbamoyl}-cyclopropyl}-amid

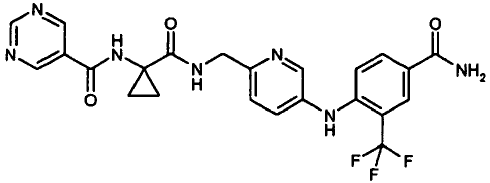

Eine Lösung von 50 mg (0.10 mmol) 4-{6-[({1-[(Pyrimidin-5-carbonyl)-amino]-cyclopropancarbonyl}amino)-methyl]-pyridin-3-ylamino}-3-trifluormethyl-benzoesäure, 33 mg (0.10 mmol) TBTU und 12 µL (0.11 mmol) N-Methylmorpholin in 0.5 mL DMF wurde 3 Minuten bei Raumtemperatur gerührt, dann mit 17 µL (0.11 mmol) 2,4-Dimethoxybenzylamin versetzt, weitere 10 Minuten gerührt und über Nacht stehen gelassen. Zur Abspaltung der Benzylgruppe wurde das Gemisch mit 10 mL Dichlormethan und 10 mL Trifluoressigsäure versetzt, über Nacht stehen gelassen und bis zur Trockne eingedampft. Der Rückstand wurde filtriert und anschließend chromatographisch gereinigt (RP mit Fließmittelgradient, Fließmittel: Acetonitril und Wasser mit 0.2% Trifluoressigsäure).
Ausbeute: 6% der Theorie
C₂₃H₂₀F₃N₇O₃ (499.45)
Massenspektrum (ESI): [M+H]⁺ = 500
[M-H]⁻ = 498

### Beispiel 199: Pyrimidin-5-carbonsäure{1-[4-(4-methoxy-phenylamino)-benzylcarbamoyl]-cyclopropyl}-amid

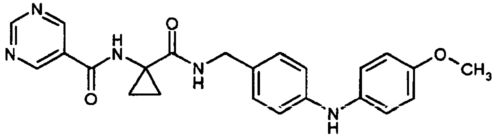

Erhalten aus 1-[(Pyrimidin-5-carbonyl)-amino]-cyclopropancarbonsäure und (4-Aminomethyl-phenyl)-(4-methoxy-phenyl)-amin analog zu Vorschrift 191a).
Ausbeute: 37% der Theorie
C₂₃H₂₃N₅O₃ (417.47)
Massenspektrum (ESI): [M+H]⁺ = 418
[M-H]⁻ = 416
Rₜ = 1.83 min (Methode 12)

### Beispiel 200: Pyrimidin-5-carbonsäure(1-{[5-(2-chlor-6-fluor-phenylamino)-pyridin-2-yl-methyl]-carbamoyl}-cyclopropyl)-amid

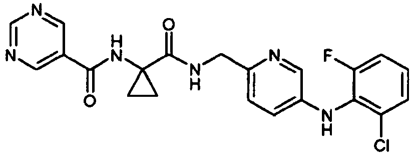

Kupplung von 1-[(Pyrimidin-5-carbonyl)-amino]-cyclopropancarbonsäure und (6-Aminomethyl-pyridin-3-yl)-(2-chlor-6-fluor-phenyl)-amin trifluoracetat mit TBTU analog zur Vorschrift 191a).
C₂₁H₁₈CIFN₆O₂ (440.86)
Massenspektrum (ESI): [M+H]⁺ = 441
[M-]⁻= 439
Rₜ = 1.38 min (Methode 2)

### Beispiel 201: Pyrimidin-5-carbonsäure {1-[4-(4-methoxy-2-methyl-phenylaminor benzylcarbamoyl]-cyclopropyl}-amid

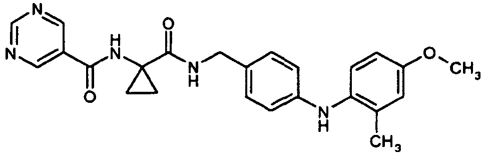

Hergestellt aus 1-[(Pyrimidin-5-carbonyl)-amino]-cyclopropancarbonsäure und (4-Aminomethyl-phenyl)-(4-methoxy-2-methyl-phenyl)-amin nach Vorschrift 191a).
C₂₄H₂₅N₅O₃ (431.49)
Massenspektrum (ESI): [M+H]⁺ = 432
[M+H]⁺ = 430
Rₜ = 1.97 min (Methode 12)

### Beispiel 202: Pyrimidin-5-carbonsäure (1-{4-[(4-methoxy-2-methyl-phenyl)-methylamino]-benzylcarbamoyl)-cyclopropyl)-amid

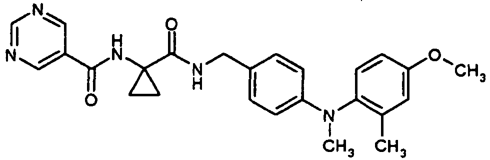

73 mg (0.35 mmol) 1-[(Pyrimidin-5-carbonyl)-amino]-cyclopropancarbonsäure und 57 mg (0.35 mmol) CDI wurden 30 Minuten bei 50°C in 5 mL DMF gerührt. 90 mg (0.35 mmol) (4-Aminomethyl-phenyl)-(4-methoxy-2-methyl-phenyl)-methyl-amin und 101 µL Diisopropylethylamin wurden bei Raumtemperatur zugegeben und man ließ weiter über Nacht rühren. Das Lösungsmittel wurde abdestilliert und der Rückstand in Methanol gelöst und chromatographisch gereinigt (RP mit Fließmittelgradient, Fließmittel: Acetonitril und Wasser mit 0.2 % Trifluoressigsäure).
Ausbeute: 19% der Theorie
C₂₅H₂₇N₅O₃ (445.52)
Massenspektrum (ESI): [M+H]⁺ = 446
Rₜ=2.16min (Methode 12)

### Beispiel 203: Pyrimidin-5-carbonsäure-(1-{1-[5-(4-chlor-2-trifluormethyl-phenylamino)-3-fluor-pyridin-2-yl]-ethylcarbamoyl}-cyclopropyl)-amid

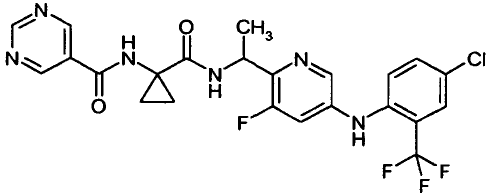

Hergestellt aus den Intermediaten A1 und B1 nach AAV1
C₂₃H₁₉ClF₄N₆O₂ (522.89)
Rₜ = 2.30 Minuten Methode 2

Das Racemat wurde mittels chiraler HPLC (Säule: Daicel AD-H 250 x 20 mm; 5 µm; 25°C; Eluent CO₂ / Isopropanol (+0.2% Diethylamin) 80:20; Fluß: 10 mL/min) in die Enantiomere getrennt.

### Beispiel 203a: (R)-Pyrimidin-5-carbonsäure-(1-{1-[5-(4-chlor-2-trifluormethyl-phenylamino)-3-fluor-pyridin-2-yl]-ethylcarbamoyl}-cyclopropyl)-amid

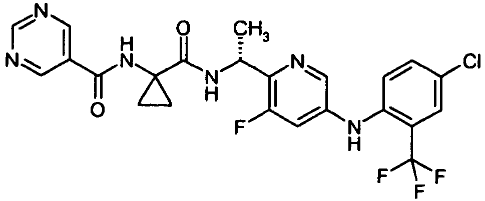

analytische chirale HPLC (Säule: Daicel AD-H; 250 x 4.6 mm; 51µm; 25°C; Eluent CO₂/ Isopropanol 80:20; Fluß: 4 mL/min)
Rₜ = 1.62 Minuten

### Beispiel 203b: (S)-Pyrimidin-5-carbonsäure-(1-{1-[5-(4-chlor-2-trifluormethyl-phenylamino)-3-fluor-pyridin-2-yl]-ethylcarbamoyl}-cyclopropyl)-amid

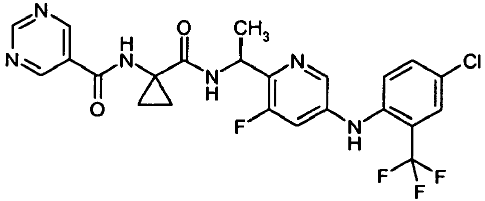

analytische chirale HPLC (Säule: Daicel AD-H; 250 x 4.6 mm; 5 µm; 25°C; Eluent CO2/ Isopropanol 80:20; Fluß: 4 mL/min)
Rₜ = 2.29 Minuten

### Beispiel 204: Pyrimidin-5-carbonsäure(1-{[5-(4-isopropyl-2-trifluormethyl-phenylamino)-pyridin-2-ylmethyl]-carbamoylrcyclopropyl)-amid

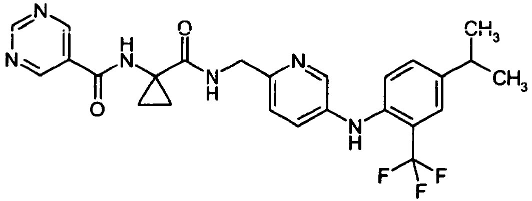

Hergestellt aus den Intermediaten A2 und B1 nach AAV1
C₂₅H₂₅F₃N₆O₂ (498.51)
Rₜ = 1.95 Minuten (Methode 2)

### Beispiel 205: N-(1-{{5-(4-Chlor-2-trifluormethyl-phenylamlno)-pyridin-2-ytmethyl]-carbamoyl}cydopropyl)-5-hydroxy-nicotinamid

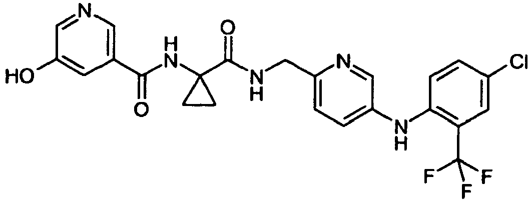

Hergestellt aus Intermediat C1 und 5-Hydroxynicotinsäure nach AAV1
C₂₃H₁₉ClF₃N5O₃ (505.88)
Rₜ = 1.74 Minuten (Methode 13)

### Beispiel 206: 6-Amino-N-(1-{[5-(4-chlor-2-trifluormethyl-phenylamino)-pyridin-2-ylmethyl]-carbamoyl}-cyclopropyl)-nicotinamid

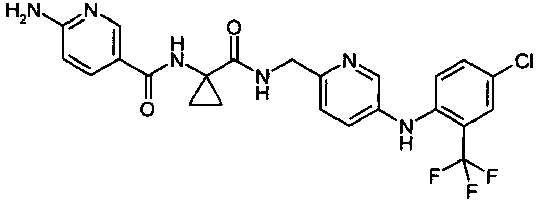

Hergestellt aus Intermediat C1 und 6-Aminonicotinsäure nach AAV1 C₂₃H₂₀ClF₃N₆O₂ (504.90)
Rₜ = 1.60 Minuten (Methode 13)

### Beispiel 207: N-(1-{[5-(4-Chlor-2-trifluormethyl-phenylamino)-pyridin-2-ylmethyl]-carbamoyl}-cyclopropyl)-2-hydroxy-isonicotinamid

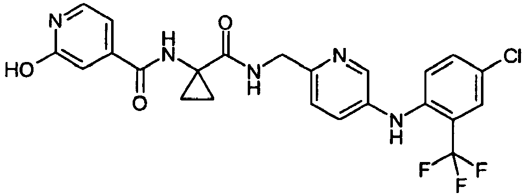

Hergestellt aus Intermediat C1 und 2-Hydroxy-isonicotinsäure nach AAV1 C₂₃H₁₉ClF₃N₅O₃ (505.88)
Rₜ = 1.69 Minuten (Methode 13)

### Beispiel 208: 2,6-Dihydroxy-pyrimidin-4-carbonsäure-(1-{[5-(4-chlor-2-trifluormethylphenylaminoypyridin-2-ylmethyl]-carbamoyl}cyclopropyl)-amid

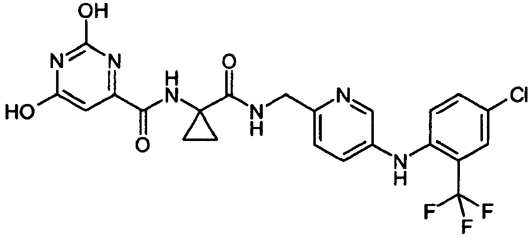

Hergestellt aus Intermediat C1 und 2,6-Dihydroxy-pyrimidin-4-carbonsäure nach AAV1 C₂₂H₁₈ClF₃N₆O₄ (522.87)
Rₜ = 1.70 Minuten (Methode 13)

### Beispiel 209: Pyridazin-4-carbonsäure (1-{[5-(4-chlor-2-trifluormethyl-phenylamino)-pyridin-2-ylmethyl]-carbamoyl}cyclopropyl)-amid

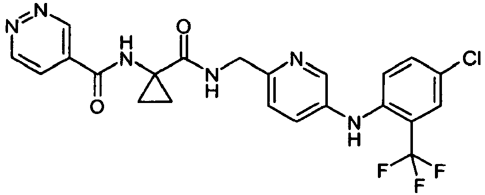

Hergestellt aus Intermediat C1 und Pyridazin-4-carbonsäure nach AAV1 C₂₂H₁₈ClF₃N₆O₂ (522.87)
Rₜ = 1.76 Minuten (Methode 13)

### Beispiele 210: 2-Dimethylamino-pyrimidin-5-carbonsäure-(1-{[5-(4-chlor-2-trifluormethylphenylaminorpyridin-2-ylmethyl]-carbamoyl}-cyclopropyl)-amid

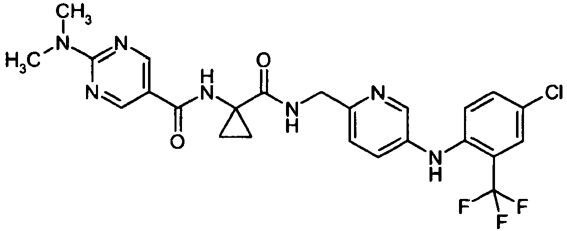

Hergestellt aus Intermediat C1 und 2-Dimethylamino-pyrimidin-5-carbonsäure nach AAV1 C₂₄H₂₃ClF₃N₇O₂ (533.94)
Rₜ = 1.94 Minuten (Methode 13)

### Beispiel 211: 6-Hydroxy-pyridin-2-carbonsäure-(1-{[5-(4-chlor-2-trifluormethyl-phenylamino)-pyridin-2-ylmethyl]-carbamoyl}-cyclopropyl)-amid

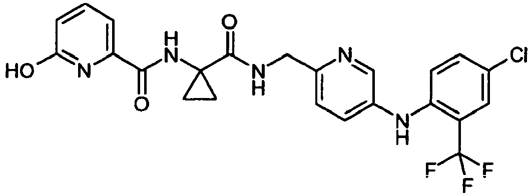

Hergestellt aus Intermediat C1 und 6-Hydroxy-pyridin-2-carbonsäure nach AAV1 C₂₃H₁₉ClF₃N₅O₃ (505.88)
Rₜ = 1.75 Minuten Methode 13

### Beispiel 212: Pyrimidin-5-carbonsäure-(1-{1-[5-(2-cyano-4-fluor-phenylamino)-3-fluorpyridin-2-yl]-ethylcarbamoyl}-cyctopropyl)-amid

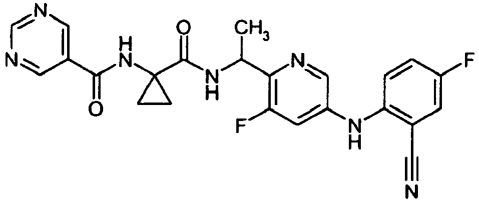

Hergestellt aus den Intermediaten A4 und B1a nach AAV1
C₂₃H₁₉F₂N₇O₂ (463.45)
Rₜ = 1.81 Minuten Methode 2

Das Racemat wurde mittels chiraler HPLC (Säule: Daicel AD-H 250 x 20mm; 5 µm; 25°C; Eluent CO₂ / Isopropanol (+0.2% Diethylamin) 80:20; Fluß: 10 mL/min) in die Enantiomere getrennt:

### Beispiel 212a: (R)-Pyrimidin-5-carbonsäure-(1-{1-[5-(2-cyano-4-fluor-phenylamino}-3-fluor-pyridin-2-yl]-ethylcarbamoyl}-cyclopropyl)-amid

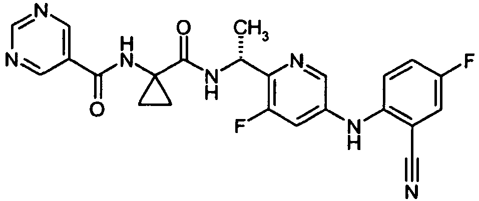

Rₜ = 2.75 Minuten

### Beispiel 212b: (S)-Pyrimidin-5-carbonsäure-(1-{1-[5-(2-cyano-4-fluor-phenylamino)-3-fluorpyridin-2-yl]-ethylcarbamoyl}-cyclopropyl)-amide

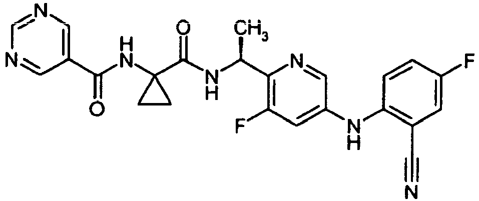

Rₜ = 5.12 Minuten

### Beispiel 213: Pyrimidin-5-carbonsäure-(1-{1-[5-(4-brom-2-trifluormethyl-phenylamino)-3-fluor-pyridin-2-yl]-ethylcarbamoyl}-cyclopropyl)-amid

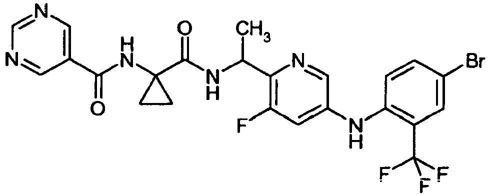

Hergestellt aus den Intermediaten A5 und B1 nach AAV1
C₂₃H₁₉BrF₄N₆O₂ (567.34)
Rₜ = 2.34 Minuten (Methode 2)

Das Racemat wurde mittels chiraler HPLC in die Enantiomere getrennt:

### Beispiel 213a: (R)-Pyrimidin-5-carbonsäure-(1-{1-[5-(4-brom-2-trifluormethyl-phenylamino)-3-fluor-pyridin-2-yl]-ethylcarbamoyl}cyclopropyl)-amid

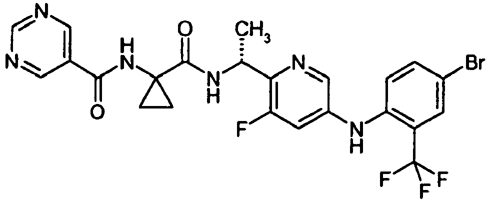

analytische chirale HPLC (Säule: Daicel AD-H; 250 x 4.6 mm; 5 µm; 25°C; Eluent CO₂ / Isopropanol 80:20; Fluß: 4mL/min)
Rₜ = 1.78 Minuten

### Beispiel 213b: (S)-Pyrimidin-5-carbonsäure-(1-{1-[5-(4-brom-2-trifluormethyl-phenylamino)-3-fluor-pyridin-2-yl]-ethylcarbamoyl}-cyclopropyl)-amid

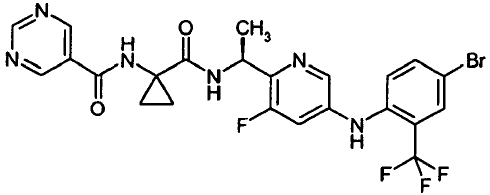

analytische chirale HPLC (Säule: Daicel AD-H; 250 x 4.6 mm; 5 µm; 25°C; Eluent CO₂ / Isopropanol 80:20; Fluß: 4mL/min)
Rₜ = 2.55 Minuten

### Beispiel 214: (S)-5-Amino-N-(3-{[5-(4-fluor-2-trifluormethyl-phenylamino)-pyridin-2-yl-methyl]-carbamoyl}-tetrahydrofuran-3-yl)-nicotinamid

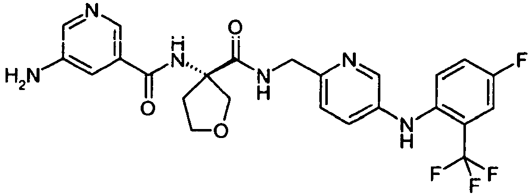

Hergestellt aus den Intermediaten A6 und B2 nach AAV1
C₂₅H₂₃F₄N₅O₃ (517.48)
Rₜ = 3.33 Minuten (Methode 7)

### Beispiel 215: 6-Oxo-5,6-dihydro-pyridazin-4-carbonsäure-(1-{[5-(4-fluor-2-trifluormethylphenylamino)-pyridin-2-ylmethyl]-carbamoyl}-cyclopropyl)-amid

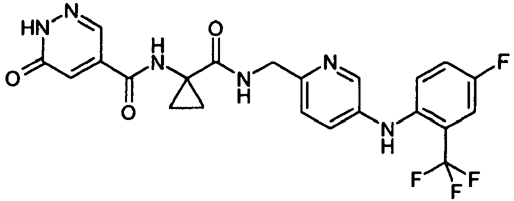

Hergestellt aus Intermediat C2 und 6-Oxo-5,6-dihydro-pyridazin-4-carbonsäure nach AAV1
C₂₂H₁₈F₄N₆O₃ (490.42)
Rₜ = 2.80 Minuten (Methode 7)

### Beispiel 216: (S)-Pyrimidin-5-carbonsäure-(3-{[3-chlor-5-(4-fluor-2-trifluormethyl-phenylamino)-pyridin-2-ylmethyl]-carbamoyl}-tetrahydrofuran-3-yl)-amid

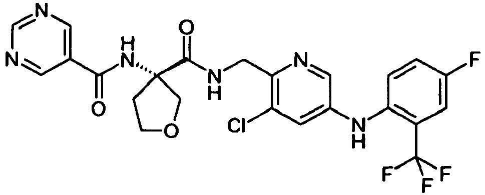

Hergestellt aus den Intermediaten A8 und B3 nach AAV1
C₂₃H₁₉ClF₄N₆O₃ (538.89)
Rₜ = 3.86 Minuten (Methode 7)

### Beispiel 217: 5-Amino-N-(1-{[5-(4-chlor-2-trifluormethyl-phenylamino)-pyridin-2-ylmethyl]-carbamoyl}-cyclopropyl)-nicotinamid

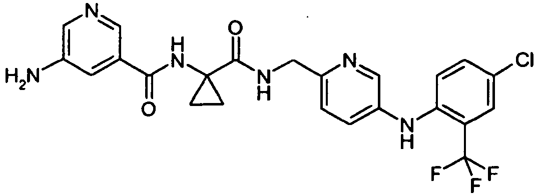

Hergestellt aus Intermediat C1 und 5-Aminonicotinsäure nach AAV1.
C₂₃H₂₀ClF₃N₆O₂ (504.90)
Rₜ = 2.01 Minuten (Methode 2)

### Beispiel 218: 5-Amino-N-(1-{[3-chlor-5-(4-fluor-2-trifluormethyl-phenylamino)-pyridin-2-yl-methyl]-carbamoyl}-cyclopropyl)-nicotinamid

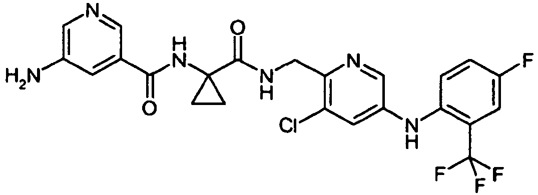

Hergestellt aus den Intermediaten A8 und B4 nach AAV1
C₂₃H₁₉ClF₄N₆O₂ (522.89)
Rₜ = 3.21 Minuten (Methode 7)

### Beispiel219: (S)-5-Amino-N-(3-{[3-chlor-5-(4-fluor-2-trifluormethyl-phenylamino)-pyridin-2-ylmethyl]-carbamoyl}-tetrahydrofuran-3-yl}-nicotinamid

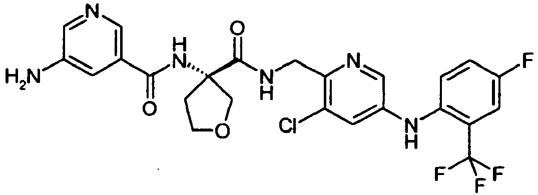

Hergestellt aus den Intermediaten A8 und B2 nach AAV1
C₂₄H₂₁ClF₄N₆O₃ (552.91)
Rₜ = 3.22 Minuten (Methode 7)

### Beispiel 220: (S)}-3H-Imidazo(4,5-b]pyridin-6-carbonsäure-(3-{[3-chlor-5-(4-fluor-2-trifluormethyl-phenylamino)-pyridin-2-ylmethyl]-carbamoyl}-tetrahydrofuran-3-yl)-amid

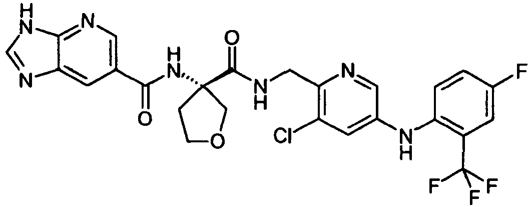

Hergestellt aus den Intermediaten A8 und B5 nach AAV1
C₂₅H₂₀ClF₄N₇O₃ (577.92)
Rₜ = 3.62 Minuten (Methode 7)

### Beispiele 221: 1-Methyl-6-oxo-1,6-dihydro-pyridazine-4-carbonsäure-(1-{[5-(4-fluor-2-trifluormethyl-phenylamino)-pyridin-2-ylmethyl]-carbamoyl}-cyclopropyl)-amid

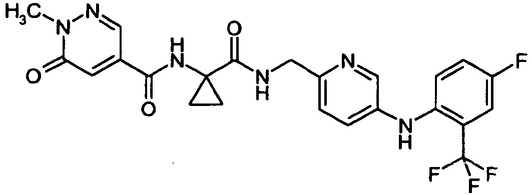

Hergestellt aus Intermediat C2 und 1-Methyl-6-oxo-1,6-dihydro-pyridazine-4-carbonsäure nach AAV1
C₂₃H₂₀F₄N₆O₃ (504.44)
Rₜ = 2.95 Minuten (Methode 7)

### Beispiel 222: (S)-5-Amino-N-(3-{[5-(4-brom-2-trifluormethyl-phenylamino)-pyridin-2-yl-methyl]-carbamoyl}-tetrahydrofuran-3-yl)-nicotinamid

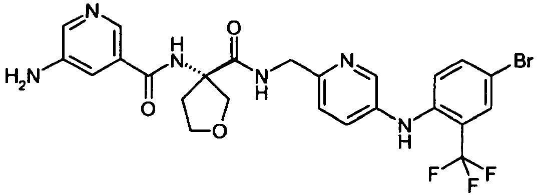

Hergestellt aus den Intermediaten A9 und B2 nach AAV1
C₂₄H₂₂BrF₃N₆O₃ (579.37)
Rₜ = 1.88 Minuten (Methode 2)

### Beispiel223: (S)-Pyrimidin-5-carbonsäure-(3-{[5-(4-brom-2-trifluormethyl-phenylamino)-pyridin-2-ylmethyl]-carbamoyl}-tetrahydrofuran-3-yl)-amid

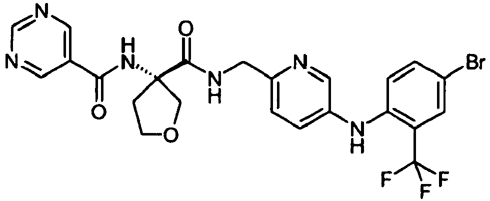

Hergestellt aus den Intermediaten A9 und B3 nach AAV1
C₂₃H₂₀BrF₃N₆O₃ (565.35)
Rₜ = 1.74 Minuten (Methode 2)

### Beispiel 224: (S)-2-Methylamino-pyrimidin-5-carbonsäure-(3-{[3-chlor-5-(4-fluor-2-trifluormethyl-phenylamino)-pyridin-2-ylmethyl]-carbamoyl}-tetrahydrofuran-3-yl)-amid

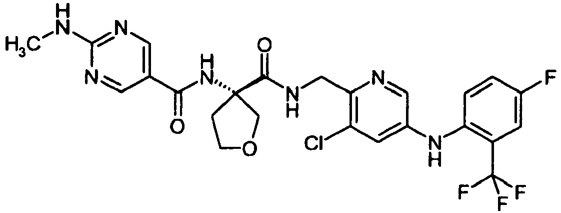

Hergestellt aus Intermediat C3 und 2-Methylamino-pyrimidin-5-carbonsäure nach AAV1 C₂₄H₂₂ClF₄N₇O₃ (567.93)
Rₜ = 3.89 Minuten (Methode 7)

### Beispiel 225: (S)-3-(3,3,3-Trifluor-propionylamino)-tetrahydrofuran-3-carbonsäure-[3-chlor-5-(4-fluor-2-trifluormethyl-phenylamio)-pyridin-2-ylmethyl]-amid

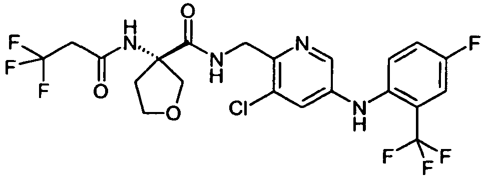

Hergestellt aus Intermediat C3 und 3,3,3-Trüluor-propionsäure nach AAV1 C₂₁H₁₈ClF₇N₄O₃ (542.84)
Rₜ = 4.18 Minuten (Methode 7)

### Beispiel 226: (S)-5-Amino-N-(3-{[5-(4-chlor-2-trifluormethyl-phenylamino)-pyridin-2-y)-methyl]-carbamoyl}-tetrahydrofuran-3-yl)-nicotinamid

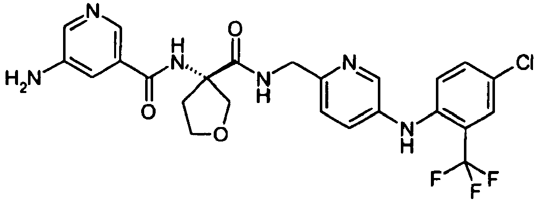

Hergestellt aus den Intermediaten A3 und B2 nach AAV1
C₂₄H₂₂ClF₃N₆O₃ (534.92)
Rₜ = 1.57 Minuten (Methode 2)

### Beispiel 227: 6-Methylamino-pyridazine-4-carbonsäure-(1-{[5-(4-fluor-2-trifluormethylphenylamino)-pyridin-2-ylmethyl]-carbamoyl}-cyclopropyl)-amid

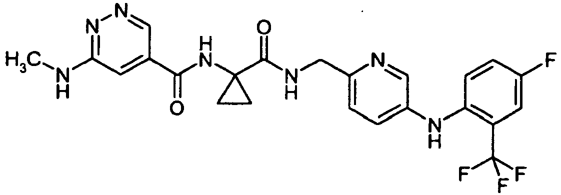

Hergestellt aus Intermediat C2 und 6-Methylamino-pyridazine-4-carbonsäure nach AAV1
C₂₃H₂₁F₄N₇O₂ (503.46)
Rₜ = 1.55 Minuten (Methode 5)

### Beispiel 228: (S)-Pyrimidin-5-carbonsäure-(3-{{5-(4-chlor-2-trifluormethyl-phenylamino)-pyridin-2-ylmethyl]-carbamoyl}-tetrahydrofuran-3-ylfamid

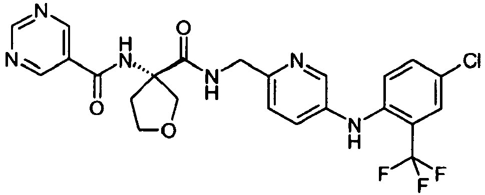

Hergestellt aus den Intermediaten A3 und B3 nach AAV1
C₂₃H₂₀Cl₃N₆O₃ (520.90)
Rₜ = 1.74 Minuten (Methode 2)

### Beispiel 229: (S)-5-Amino-N-(3-{[3-chlor-5-(2-trifluormethyl-phenylamino)-pyridin-2-yl-methyl]-carbamoy}-tetrahydrofuran-3-yl)-nicotinamid

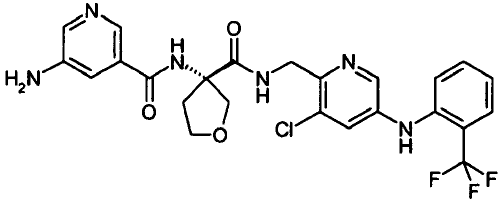

Hergestellt aus den Intermediaten A10 und B2 nach AAV1
C₂₄H₂₂ClF₃N₆O₃ (534.92)
Rₜ = 3.19 Minuten (Methode 7)

### Beispiel 230: (S)-2-Methoxy-pyrimidin-5-carbonsäure-(3-{[3-chlor-5-(4-fluor-2-trifluormethyl-phenylamino)-pyridin-2-ylmethyl]-carbamoyl}-tetrahydrofuran-3-yl)-amid

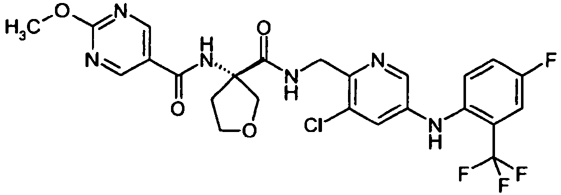

Hergestellt aus Intermediat C3 und 2-Methoxy-pyrimidin-5-carbonsäure nach AAV1
C₂₄H₂₁ClF₄N₆O₄ (568.91)
Rₜ = 4.01 Minuten (Methode 7)

### Beispiel 231: (S)-5-Amino-N-{3-[2-fluor-4-(4-fluor-2-trifluormethyl-phenylamino)-benzylcarbamoyl]-tetrahydrofuran-3-yl}-nicotinamid

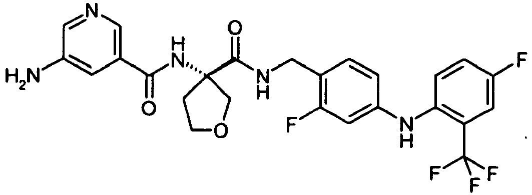

Hergestellt aus den Intermediaten A11 und B2 nach AAV1
C₂₅H₂₂F₅N₅O₃ (535.47)
Rₜ = 1.23 Minuten (Methode 2)

### Beispiel 232: 2-Methyl-pyrimidin-5-carbonsäure-(1-{[5-(4-fluor-2-trifluormethyl-phenylaminoypyridin-2-ylmethyl]-carbamoyl}-cyclopropyl)-amid

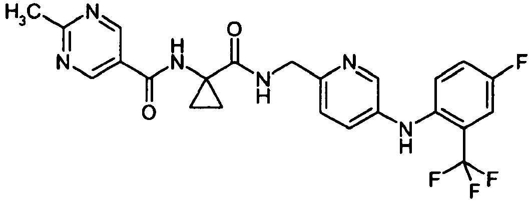

Hergestellt aus Intermediat C2 und 2-Methyl-pyrimidin-5-carbonsäure nach AAV1
C₂₃H₂₀F₄N₆O₂ (488.44)
Rₜ = 1.94 Minuten (Methode 2)

### Beispiel 233: 2-Methoxy-pyrimidin-5-carbonsäure-(1-{[5-(4-fluor-2-trifluormethyl-phenylamino)-pyridin-2-ylmethyl]-carbamoyl}-cyclopropyl)-amid

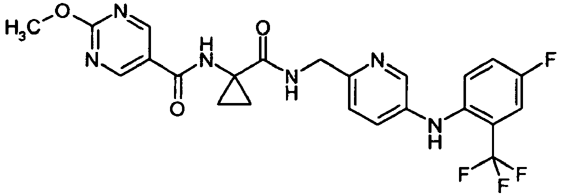

Hergestellt aus Intermediat C2 und 2-Methoxy-pyrimidin-5-carbonsäure nach AAV1
C₂₃H₂₀F₄N₆O₃ (504.44)
Rₜ = 2.00 Minuten (Methode 2)

### Beispiel 234: N-(1-{[5-(4-Brom-2-trifiuormethyi-phenylamino)-pyridin-2-ylmethyl]-carbamoyl}cyclopropyl)-6-hydroxy-nicotinamid

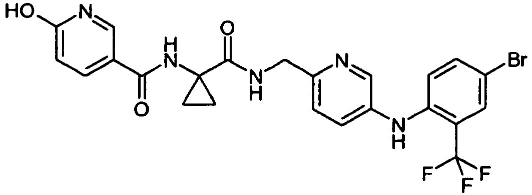

Hergestellt aus Intermediat C4 und 6-Hydroxy-nicotinsäure nach AAV1
C₂₃H₁₉BrF₃N₅O₃ (550.33)
Rₜ = 1.79 Minuten (Methode 2)

### Beispiel 235: N-(1-{[5-(4-Brom-2-trifluormethyl-phenylamino)-pyridin-2-ylmethyl]-carbamoyl}-cyclopropyl)-6-methoxy-nicotinamid

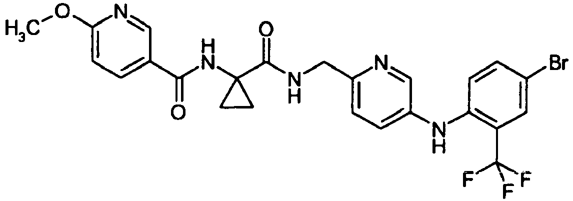

Hergestellt aus Intermediat C4 und 6-Methoxy-nicotinsäure nach AAV1
C₂₄H₂₁BrF₃N₅O₃ (564.36)
Rₜ = 1.84 Minuten (Methode 2)

### Beispiel 236: (S)-5-Amino-N-{3-[2-fluor-4-(2-trifluormethyl-phenylamino)-benzylcarbamoyl]-tetrahydrofuran-3-yl}-nicotinamid

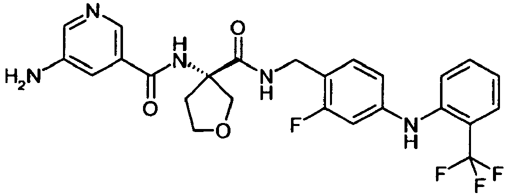

Hergestellt aus den Intermediaten A12 und B2 nach AAV1
C₂₅H₂₃F₄N₅O₃ (517.48)
Rₜ = 3.07 Minuten (Methode 3)

### Beispiel 237: (S)-5-Amino-N-(3-{[3-chlor-5-(2-fluor-6-trifluormethyl-phenylamino)-pyridin-2-ylmethyl]-carbamoyl}-tetrahydrofuran-3-yl)-nicotinamid

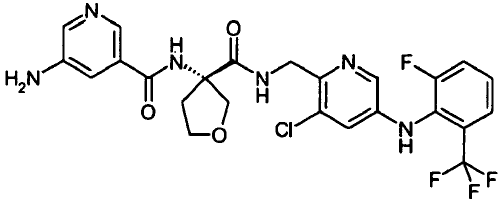

Hergestellt aus Intermediat C5 und 5-Aminonicotinsäure nach AAV1
C₂₄H₂₁ClF₄N₆O₃ (552.91)
Rₜ = 3.28 Minuten (Methode 3)

### Beispiel 238: (S)-2-Methoxy-pyrimidin-5-carbonsäure-(3-{[3-fluor-5-(4-fluor-2-trifluormethyl-phenylamino)-pyridin-2-ylmethyl]-carbamoyl}-tetrahydrofuran-3-yl)-amid

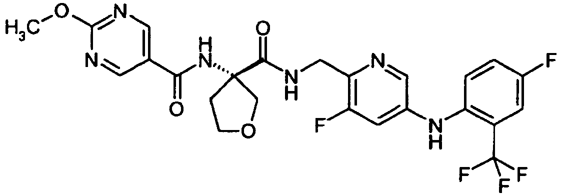

Hergestellt aus Intermediat C6 und 2-Methoxy-pyrimidin-5-carbonsäure nach AAV1
C₂₄H₂₁F₅N₆O₄ (552.46)
Rₜ = 3.62 Minuten (Methode 3)

### Beispiel 239: N-(1-{[5-(4-Chlor-2-trifluormethyl-phenylamino)-pyridin-2-ylmethyl]-carbamoyl}-cyclopropyl)-6-hydroxy-nicotinamid

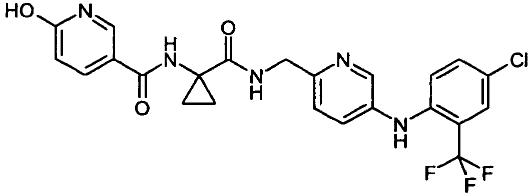

Hergestellt aus Intermediat C1 und 6-Hydroxy-nicotinsäure nach AAV1
C₂₃H₁₉ClF₃N₅O₃ (505.88)
Rₜ = 1.71 Minuten (Methode 2)

### Beispiel 240: N-(1-{[5-(4-Chlor-2-trifluormethyl-phenylamino)-pyridin-2-ylmethyl]-carbamoyl}-cyclopropyl)-6-methoxy-nicotinamid

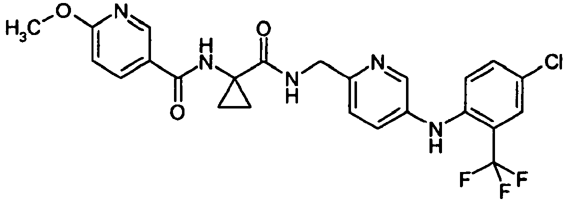

Hergestellt aus Intermediat C1 und 6-Methoxy-nicotinsäure nach AAV1
C₂₄H₂₁ClF₃N₅O₃ (519.91)
Rₜ = 1.75 Minuten (Methode 2)

### Beispiel 241: (S)-2-Methylamino-pyrimidin-5-carbonsäure-(3-{[3-chlor-5-(2-fluor-6-trifluormethyl-phenylaminohpyridin-2-ylmethyl]-carbamoyl}-tetrahydrofuran-3-yl)-amid

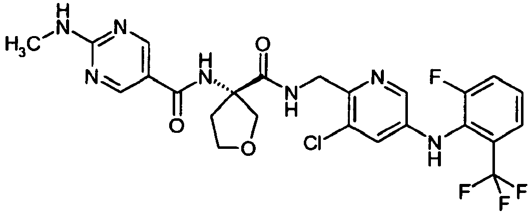

Hergestellt aus Intermediat C5 und 2-Methylamino-pyrimidin-5-carbonsäure nach AAV1
C₂₄H₂₂OF₄N₇O₃ (567.93)
Rₜ = 4.64 Minuten (Methode 3)

### Beispiel 242: (S)-Pyrimidin-5-carbonsäure-(3-{[5-(4-chlor-2-trifluormethyl-phenylamino)-3-fluor-pyridin-2-ylmethyl]-carbamoyl}tetrahydrofuran-3-yl)-amid

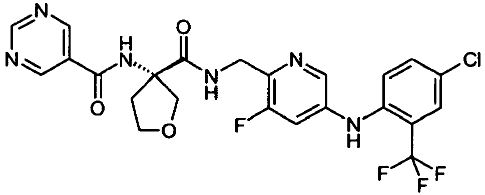

Hergestellt aus den Intermediaten A15 und B3 nach AAV1
C₂₃H₁₉ClF₄N₆O₃ (538.89)
Rₜ = 2.15 Minuten (Methode 2)

### Beispiel 243: N-(1-{[5-(4-Fluor-2-trifluormethyl-phenylamino)-pyridin-2-ylmethyl]-carbamoyl}-cyclopropyl)-5-hydroxy-nicotinamid

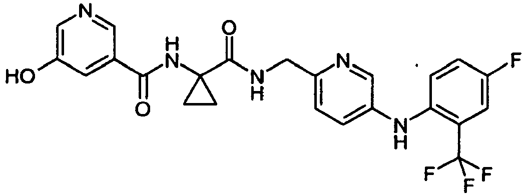

Hergestellt aus Intermediat C2 und 5-Hydroxynicotinsäure nach AAV1
C₂₃H₁₉F₄N₅O₃ (489.43)
Massenspektroskopie (ESI): [M+H]+ = 490
[M-H]- = 488

### Beispiel 244: 6-Amino-N-(1-{[5-(4-fluor-2-trifluormethyl-phenylamino)-pyridin-2-ylmethyl]-carbamoyl}-cyclopropyl)-nicotinamid

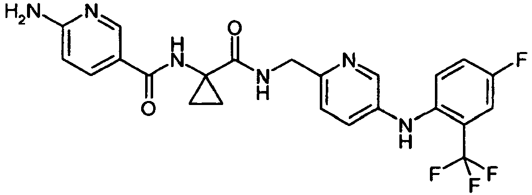

Hergestellt aus Intermediat C2 und 6-Aminonicotinsäure nach AAV1
C₂₃H₂₀F₄N₆O₂ (488.44)
Massenspektroskopie (ESI): [M+H]+ = 489
[M-H]- = 487

### Beispiel 245: 2-Isopropyl-pyrimidin-5-carbonsäure-(1-{[5-(4-fluor-2-trifluormethylphenylamino)-pyridin-2-ylmethyl]-carbamoyl}-cyclopropyl)-amid

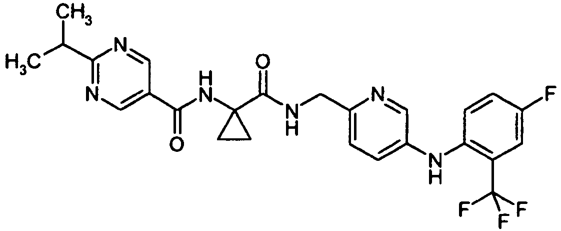

Hergestellt aus Intermediat C2 und 2-Isopropyl-pyrimidin-5-carbonsäure nach AAV1
C₂₅H₂₄F₄N₆O₂ (516.50)
Massenspektroskopie (ESI): [M+H]+ = 517

### Beispiel 246: 2-Trifluormethyl-pyrimidin-5-carbonsäure-(1-{[5-(4-fluor-2-trifluormethylphenylamino)-pyridin-2-ylmethyl]-carbamoyl}-cyclopropyl)-amid

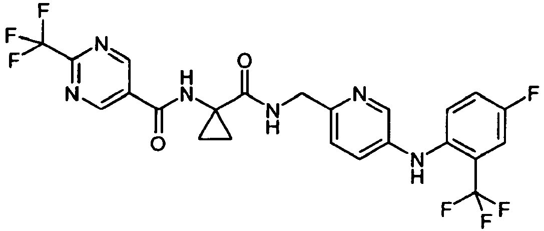

Hergestellt aus Intermediat C2 und 2-Trifluormethyl-pyrimidin-5-carbonsäure nach AAV1
C₂₃H₁₇F₇N₆O₂ (542.41)
Massenspektroskopie (ESI): [M+H]+ = 543

### Beispiel 247: 2-Ethylamino-pyrimidin-5-carbonsäure-(1-{[5-(4-fluor-2-trifluormethylphenylamino)-pyridin-2-ylmethyl]-carbamoyl)-cyclopropyl)-amid

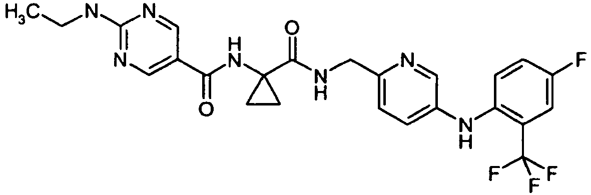

Hergestellt aus Intermediat C2 und 2-Ethylamino-pyrimidin-5-carbonsäure nach AAV1
C₂₄H₂₃F₄N₇O₂ (517.48)
Massenspektroskopie (ESI): [M+H]+ = 518
Rₜ = 1.58 Minuten (Methode 5)

### Beispiel 248: 2-Piperidin-1-yl-pyrimidin-5-carbonsäure-(1-{[5-(4-fluor-2-trifluormethylphenylamino)-pyridin-2-ylmethyl]-carbamoyl}-cyclopropyl)-amid

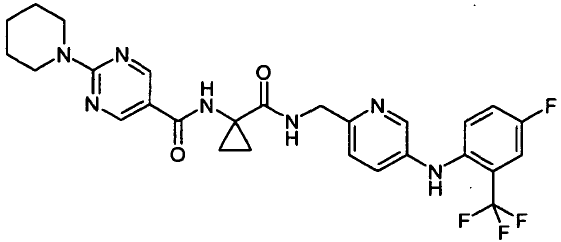

Hergestellt aus Intermediat C2 und 2-Piperidin-1-yl-pyrimidin-5-carbonsäure nach AAV1
C₂₇H₂₇F₄N₇O₂ (557.55)
Massenspektroskopie (ESI): [M+H]+ = 558

### Beispiel 249: 5-Acetylamino-N-(1-{[5-(4-fluor-2-trifluormethyl-phenylamino)-pyridin-2-yl-methyl]-carbamoyl}-cyclopropyl)-nicotinamid

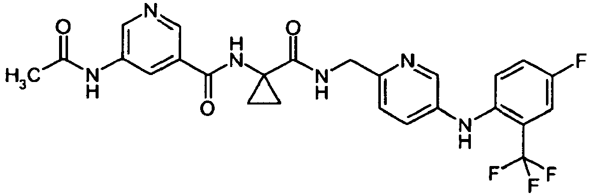

Hergestellt aus Intermediat C2 und 5-Acetylaminonicotinsäure nach AAV1
C₂₅H₂₂F₄N₆O₃ (530.48)
Massenspektroskopie (ESI): [M+H]+ = 531

### Beispiel 250: 2-Pyrrolidin-1-yl-pyrimidin-5-carbonsäure-(1-{[5-(4-fluor-2-trifluormethylphenylaminohpyridin-2-ylmethyl]-carbamoyl}-cyclopropyl)-amid

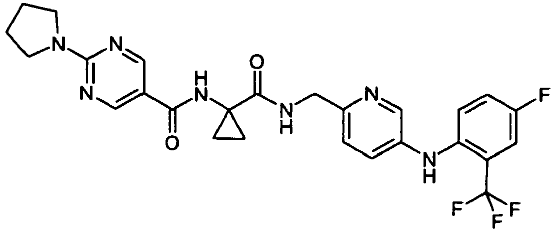

Hergestellt aus Intermediat C2 und 2-Pyrrolidin-1-yl-pyrimidin-5-carbonsäure nach AAV1
C₂₆H₂₅F₄N₇O₂ (543.52)
Massenspektroskopie (ESI): [M+H]+ = 544

### Beispiel 251: 6-Acetylamino-N-(1-{[5-(4-ftuor-2-trifluormethyl-phenylamino)-pyridin-2-yl-methyl]-carbamoyl}-cyclopropyl)nicotinamid

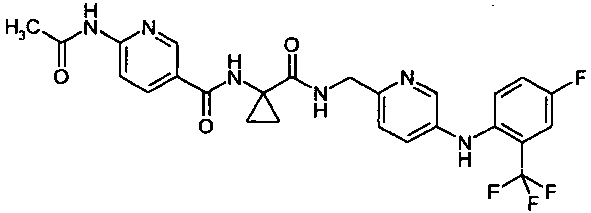

Hergestellt aus Intermediat C2 und 6-Acetylamino-nicotinsäure nach AAV1
C₂₅H₂₂F₄N₆O₃ (530.48)
Massenspektroskopie (ESI): [M+H]+ = 531

### Beispiel 252: 6-Dimethylamino-pyridazin-4-carbonsäure-(1-{[5-(4-fluor-2-trifluormethylphenylamino)-pyridin-2-ylmethyl]-carbamoyl}cyclopropyl)-amid

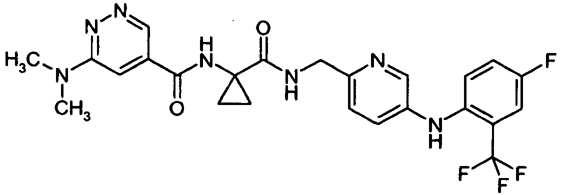

Hergestellt aus intermediat C2 und 6-Dimethylamino-pyridazin-4-carbonsäurz nach AAV1
C₂₄H_{23F4}N₇0₂ (517.48)
Massenspektroskopie (ESI): [M+H]+ = 518

### Beispiel 253: 6-Chlor-N-(1-{[5-(4-fluor-2-trifluormethyl-phenylamino)pyridin-2-ylmethyl]-carbamoyl}cyclopropyl)-nicotinamid

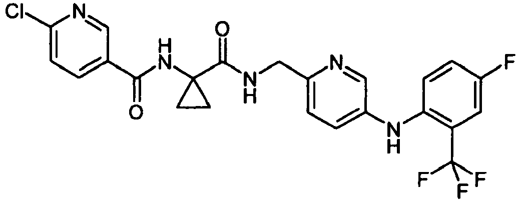

Hergestellt aus Intermediat C2 und 6-Chlor-nicotinsäure nach AAV1
C₂₃H₁₈ClF₄N₅O₂ (507.87)
Massenspektroskopie (ESI): [M+H]+ = 508

### Beispiel 254: N-(1-{[5-(4-Fluor-2-trifluormethyl-phenylamino)-pyridin-ylmethyl]-carbamoyl}-cyclopropyl)-6-trifluormethyl-nicotinamid

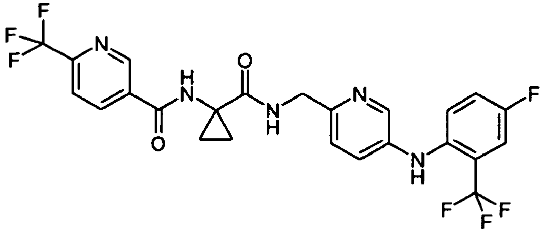

Hergestellt aus Intermediat C2 und 6-trifluormethyl-nicotinsäure nach AAV1
C₂₄H₁₈F₇N₅O₂ (541.43)
Massenspektroskopie (ESI): [M+H]+ = 542

### Beispiel 255: 1H-Pyrrolo[3,2-b]pyridin-6-carbonsäure-(1-{[5-(4-fluor-2-trifluormethylphenylamino)pyridin-2-ylmethyl]-carbamoyl}-cyclopropyl)-amid

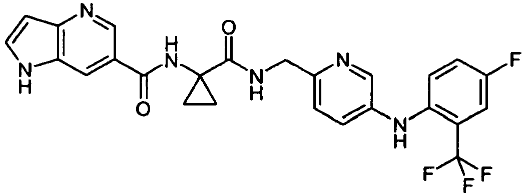

Hergestellt aus Intermediat C2 und 1H-Pyrrolo[3,2-b]pyridin-6-carbonsäure nach AAV1
C₂₅H₂₀F₄N₆O₂ (512.47)
Massenspektroskopie (ESI): [M+H]+ = 513

### Beispiel 256: 6-Cyano-N-(1-{[5-(4-fluor-2-trifluormethyl-phenylamino)-pyridin-2-ylmethyl]-carbamoyl}-cyclopropyl)-nicotinamid

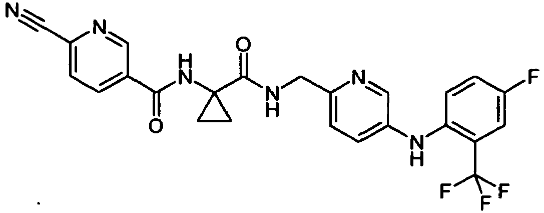

Hergestellt aus Intermediat C2 und 6-Cyano-nicotinsäure nach AAV1
C₂₄H₁₈F₄N₆O₂ (498.44)
Massenspektroskopie (ESI): [M+H]⁺ = 499

### Beispiel 257: 2-Acetylamino-thiazol-5-carbonsäure-(1-{[5-(4-fluor-2-trifluormethyl-phenylamino)-pyridin-2-ylmethyl]-carbamoyl}-cyclopropyl)-amid

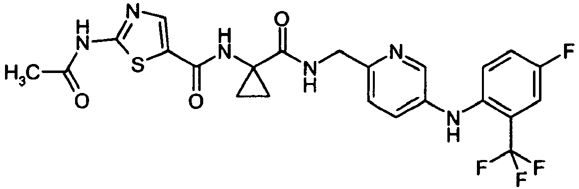

Hergestellt aus Intermediat C2 und 2-Acetylamino-thiazol-5-carbonsäure nach AAV1
C₂₃H₂₀F₄N₆O₃S (536.51)
Massenspektroskopie (ESI): [M+H]+ = 537

### Beispiel 258: (S)-2-Methoxy-pyrimidin-5-carbonsäure-(3-{[3-chlor-5-(2-fluor-6-trifluormethyl-phenylamino)-pyridin-2-ylmethyl]-carbamoy}-tetrahydrofuran-3-yl)-amid

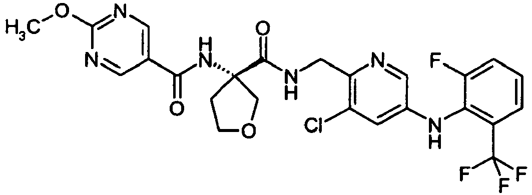

Hergestellt aus Intermediat C3 und 2-Methoxy-pyrimidin-5-carbonsäure nach AAV1
C₂₄H₂₁ClF₄N₆O₄ (568.91)
Rₜ = 3.99 Minuten (Methode 3)

### Beispiel 259: (S)-N-(3-{[3-Chlor-5-(2-fluor-6-trifluormethyl-phenylamino)-pyridin-2-yl-methyl]-carbamoyl}-tetrahydrofuran-3-yl)-5-methylamino-nicotinamid

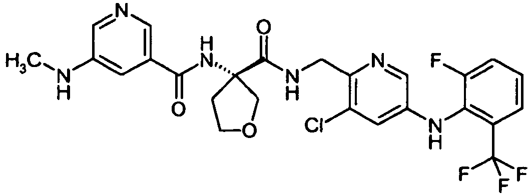

Hergestellt aus Intermediat C3 und 5-Methylamino-nicotinsäure nach AAV1
C₂₅H₂₃CIF₄N₆O₃ (566.94)
Rₜ = 3.59 Minuten (Methode 3)

### Beispiel 260: (S)-N-(3-{[3-Fluor-5-(4-fluor-2-trifluormethyl-phenylamino)-pyridin-2-yl-methyl]-carbamoyl}-tetrahydrofuran-3-yl)-5-methylamino-nicotinamid

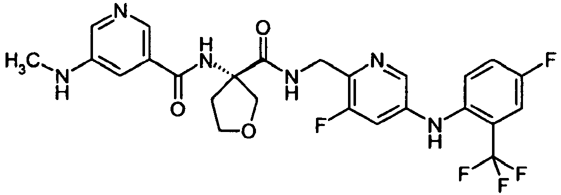

Hergestellt aus Intermediat C6 und 5-Methylamino-nicotinsäure nach AAV1
C₂₅H₂₃F₅N₆O₃ (550.49)
Rₜ = 3.81 Minuten (Methode 3)

### Beispiel 261: (S)-3-(3,3,3-Trifluor-propionylamino)-tetrahydrofuran-3-carbonsäure-[3-chlor-5-(2-fluor-6-trifluormethyl-phenylamino)-pyridin-2-ylmethyl]-amid

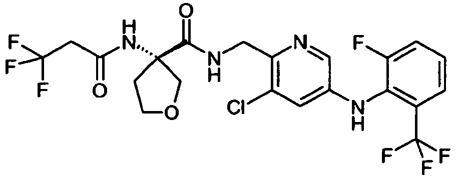

Hergestellt aus Intermediat C5 und 3,3,3-Trifluorpropionsäure nach AAV1
C₂₁H₁₈ClF₇N₄O₃ (542.84)
Rₜ = 4.11 Minuten (Methode 3)

### Beispiel 262: 6-Amino-pyridazin-4-carbonsäure-(1-{[5-(4-fluor-2-trifluormethyl-phenylamino)-pyridin-2-ylmethyl]-carbamoyl}-cyclopropyl)-amid

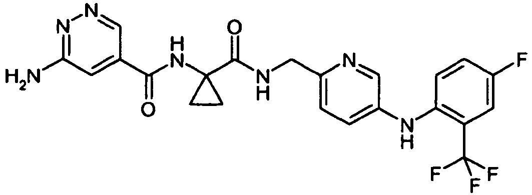

Hergestellt aus Intermediat C2 und 6-Amino-pyridazin-4-carbonsäure nach AAV1
C₂₂H₁₉F₄N₇O₂ (489.43)
Rₜ = 1.81 Minuten (Methode 6)

### Beispiel 263: N-(1-{[5-(4-Chlor-2-trifluormethyl-phenylamino)-pyridin-2-ylmethyl]-carbamoyl}-cyclopropyl}-6-dimethylamino-nicotinamid

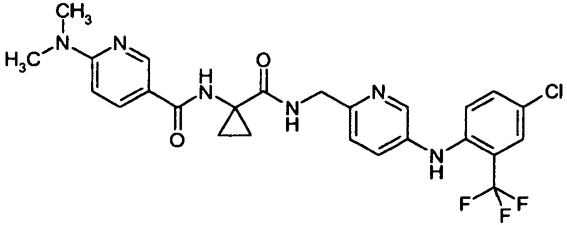

Hergestellt aus Intermediat C1 und 6-Dimethylamino-nicotinsäure nach AAV1
C₂₅H₂₄ClF₃N₆O₂ (532.95)
Rₜ = 1.90 Minuten (Methode 2)

### Beispiel 264: 1-Methyl-1H-benzimidazol-5-carbonsäure-(1-{[5-(4-chlor-2-trifluormethylphenylamino)-pyridin-2-ylmethyl]-carbamoyl}-cyclopropyl)-amid

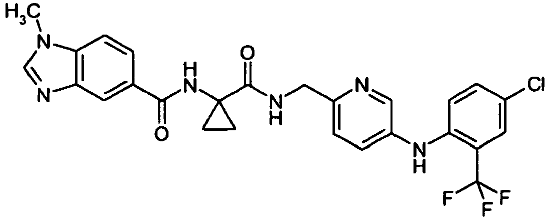

Hergestellt aus Intermediat C1 und 1-Methyl-1H-benzimidazol-5-carbonsäure nach AAV1
C₂₆H₂₂ClF₃N₆O₂ (542.95)
Rₜ = 1.72 Minuten (Methode 2)

### Beispiel 265: 2-Amino-1H-benzimidazol-5-carbonsäure-(1-{[5-(4-chlor-2-trifluormethylphenylamino}-pyridin-2-ylmethyl]-carbamoyl}-cyclopropyl)-amid

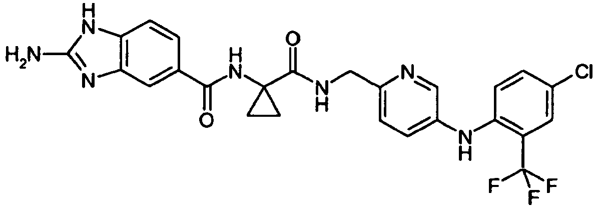

Hergestellt aus Intermediat C1 und 2-Amino-1H-benzimidazol-5-carbonsäure nach AAV1
C₂₅H₂₁ClF₃N₇O₂ (543.93)
Massenspektroskopie (ESI): [M+H]+ = 544

### Beispiel 266: 1H-Benzimidazol-5-carbonsäure-(1-{[5-(4-chlor-2-trifluormethyl-phenylamino)-pyridin-2-ylmethyl]-carbamoyl}-cyclopropyl)-amid

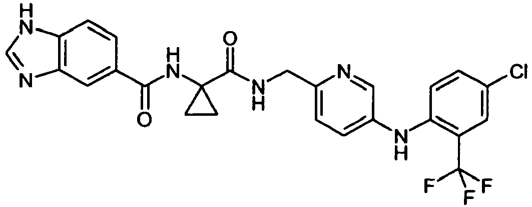

Hergestellt aus Intermediat C1 und 1 H-Benzimidazol-5-carbonsäure nach AAV1
C₂₅H₂₀ClF₃N₆O₂ (528.92)
Rₜ = 1.79 Minuten (Methode 2)

### Beispiel 267: (S)-2-Methyl-pyrimidin-5-carbonsäure-(3-{[3-fluor-5-(4-fluor-2-trifluormethylphenylamino)-pyridin-2-ylmethyl]-carbamoyl}-tetrahydrofuran-3-yl)-amid

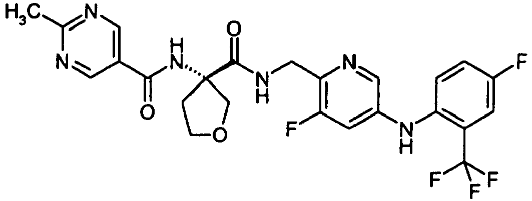

Hergestellt aus Intermediat C6 und (S)-2-Methyl-pyrimidin-5-carbonsäure nach AAV1
C₂₄H₂₁F₅N₆O₃ (536.46)
Rₜ = 3.91 Minuten (Methode 3)

### Beispiel 268: N-(1-{[5-(4-Brom-2-trifluormethyl-phenylamino)-pyridin-2-ylmethyl]-carbamoyl}-cyclopropyl)-6-dimethylamino-nicotinamid

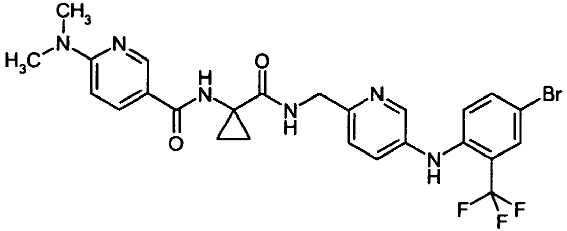

Hergestellt aus Intermediat C4 und 6-Dimethylamino-nicotinsäure nach AAV1
C₂₅H₂₄BrF₃N₆O₂ (577.40)
Rₜ = 2.09 Minuten (Methode 2)

### Beispiel 269: (S)-N-(3-{[3-Chlor-5-(4-fluor-2-trifluormethyl-phenylamino)-pyridin-2-yl-methyl]-carbamoyl}-tetrahydrofuran-3-yl)-5-methylamino-nicotinamid

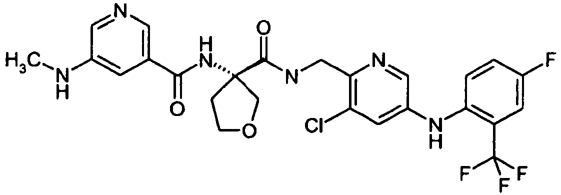

Hergestellt aus Intermediat C3 und 5-Methylaminonicotinsäure nach AAV1
C₂₅H₂₃ClF₄N₆O₃ (566.94)
Rₜ = 3.66 Minuten (Methode 3)

Beispiel 270: N-(1-{[5-(4-Ftuor-2-trifluormethyl-phenylamino)-pyridin-2-ylmethyl]-carbamoyl}-cyclopropyl)-5-methylamino-nicotinamid 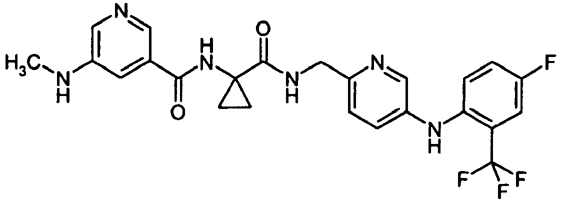 Hergestellt aus Intermediat C2 und 5-Methylaminonicotinsäure nach AAV1
C₂₄H₂₂F₄N₆O₂ (502.47)
Rₜ = 1.97 Minuten (Methode 2)

### Beispiel 271: (S)-5-Amino-N-(3-{[5-(4-chlor-2-trifluormethyl-phenylamino)-3-fluor-pyridin-2-ylmethyl]-carbamoyl}-tetrahydrofuran-3-ylnicotinamid

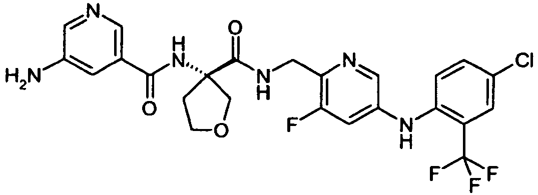

Hergestellt aus den Intermediaten A15 und B2 nach AAV1
C₂₄H₂₁ClF₄N₆O₃ (552.91)
Rₜ = 2.48 Minuten (Methode 2)

### Beispiel 272: N-(1-{[3-Fluor-5-(2-fluor-6-trifluormethyl-phenylamino)-pyridin-2-ylmethyl]-carbamoyl}-cyclopropyl)-5-methylamino-nicotinamid

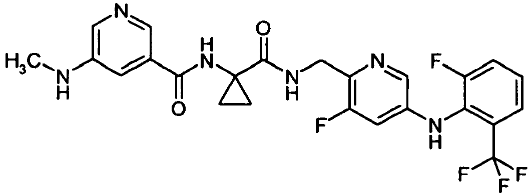

Hergestellt aus Intermediat C7 und 5-Methylaminonicotinsäure nach AAV1
C₂₄H₂₁F₅N₆O₃ (520.46)
Rₜ = 1.87 Minuten (Methode 2)

### Beispiel 273: 5-Amino-N-{3-[4-(2-trifiuormethyl-phenylamino)-benzylcarbamoyl]-oxetan-3-yl}-nicotinamid

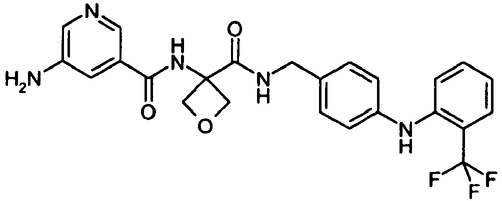

Hergestellt aus Intermediat C8 und 5-Aminonicotinsäure nach AAV1
C₂ₐHₙF₃N₅0₃ (485.46)
Rₜ = 2.17 Minuten (Methode 6)

### Beisoiel274: (S)-2-Methyl-pyrimidin-5-carbonsäure-{3-[2-fluor-4-(4-fluor-2-trifluormethylphenylamino)-benzylcarbamoyl]-tetrahydrofuran-3-yl}-amid

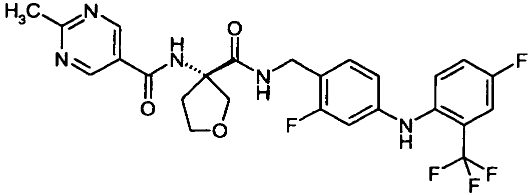

Hergestellt aus Intermediat C9 und 2-Methyl-pyrimidin-5-carbonsäure nach AAV1
C₂₅H₂₂F₅N₅O₃ (535.47)
Rₜ = 3.61 Minuten (Methode 3)

### Beispiel 275: 5-Acetylamino-N-(1-{[5-(4-chlor-2-trifluormethyl-phenylamino)-pyridin-2-yl-methyl]-carbamoyl}-cyclopropyl}-nicotinamid

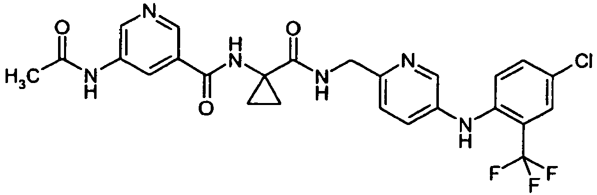

Hergestellt aus Intermediat C1 und 5-Acetylamino-nicotinsäure nach AAV1
C₂₅H₂₂ClF₃N₆O₃ (546.93)
Rₜ = 1.78 Minuten (Methode 2)

### Beispiel 276: (S)-2-Methy)amino-pyrimidin-5-carbonsäure-(3-{[5-(4-ch)or-2-trifluormethylphenylamino)-3-fluor-pyridin-2-ylmethyl]-carbamoyl}-tetrahydrofuran-3-yl)-amid

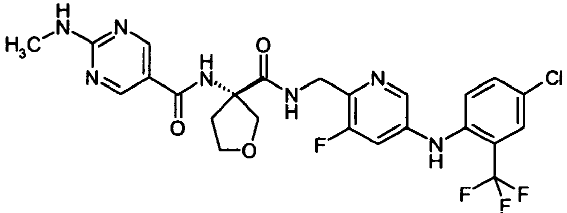

Hergestellt aus den Intermediaten A15 und B6 nach AAV1
C₂₄H₂₂ClF₄N₇O₃ (567.93)
Rₜ = 2.16 Minuten (Methode 2)

### Beispiel 277: (S)-2-Methyl-pyrimidin-5-carbonsäure-(3-{[5-(4-chlor-2-trifluormethylphenylamino)-3-fluor-pyridin-2-ylmethyl]-carbamoyl}-tetrahydrofuran-3-yl)-amid

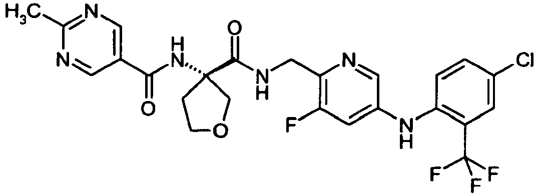

Hergestellt aus den Intermediaten A15 und B7 nach AAV1
C₂₄H₂₁ClF₄N₆O₃ (552.91)
Rₜ = 2.50 Minuten (Methode 2)

### Beispiel 278: (S)-Pyrimidin-5-carbonsäure-(3-{[3-chlor-5-(4-chlor-2-trifluormethylphenylamino)-pyridin-2-ylmethyl]-carbamoyl}-tetrahydrofuran-3-yl)-amid

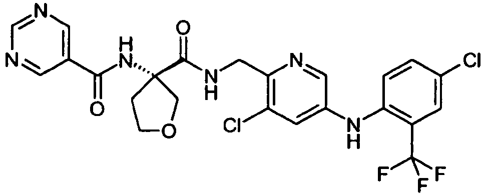

Hergestellt aus den Intermediaten A18 und B3 nach AAV1
C₂₃H₁₉Cl₂F₃N₆O₃ (555.34)
Rₜ = 2.56 Minuten (Methode 2)

### Beispiel 279: (S)-2-Methyl-pyrimidin-5-carbonsäure-{3-(2-fluor-4-(2-trifluormethyl-phenylamino)-benzylcarbamoyl]-tetrahydrofuran-3-yl}-amid

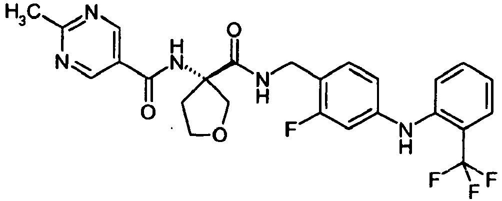

Hergestellt aus Intermediat C22 und 2-Methyl-pyrimidin-5-carbonsäure nach AAV1
C₂₅H₂₃F₄N₅O₃ (517.48)
Rₜ = 1.97 Minuten (Methode 7)

### Beispiel 280: N-(1-{[5-(4-Chlor-2-trifluormethyl-phenylamino)-pyridin-2-ylmethyl]-carbamoyl}-cyclopropyl)-5-methylamino-nicotinamid

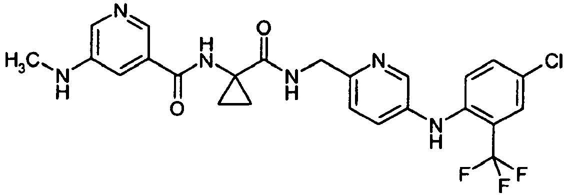

Hergestellt aus Intermediat C1 und 5-Methylaminonicotinsäure nach AAV1
C₂₄H₂₂ClF₃N₆O₂ (518.92)
Rₜ = 1.96 Minuten (Methode 2)

### Beispiel 281: (S)-2-Methylamino-pyrimidin-5-carbonsäure-(3-{[3-chlor-5-(4-chlor-2-trifluormethyl-phenylamino)-pyridin-2-ylmethyl]-carbamoyl}-tetrahydrofuran-3-yl)-amid

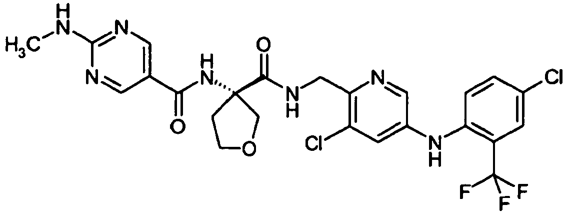

Hergestellt aus den Intermediaten A18 und B6 nach AAV1
C₂₄H₂₂Cl₂F₃N₇O₃ (584.38)
Rₜ = 2.56 Minuten (Methode 2)

### Beispiel 282: (S)-2-Methyl-pyrimidin-5-carbonsäure-(3-{[3-chlor-5-(4-chlor-2-trifluormethyl-phenylamino)-pyridin-2-ylmethyl]-carbamoyl}-tetrahydrofuran-3-yl)-amid

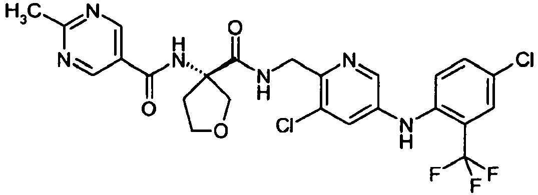

Hergestellt aus den Intermediaten A18 und B7 nach AAV1
C₂₄H₂₁Cl₂F₃N₆O₃ (569.37)
Rₜ = 2.59 Minuten (Methode 2)

### Beispiel 283: 2-Methyl-pyrimidin-5-carbonsäure-{1-[2-fluor-4-(4-fluor-2-trifluormethylphenylamino)-benzylcarbamoyl]-cyclopropyl}-amid

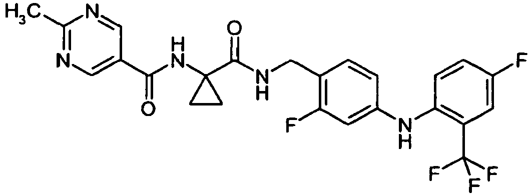

Hergestellt aus Intermediat C10 und 2-Methyl-pyrimidin-5-carbonsäure nach AAV1
C₂₄H₂₀F₅N₅O₂ (505.44)
Rₜ = 1.33 Minuten (Methode 7)

### Beispiel 284 6-Oxo-1,6-dihydro-pyridazin-4-carbonsäure-(1-{[5-(4-chlor-2-trifluormethylphenylamino)-pyridin-2-ylmethyl]-carbamoyl}-cyclopropyl)-amid

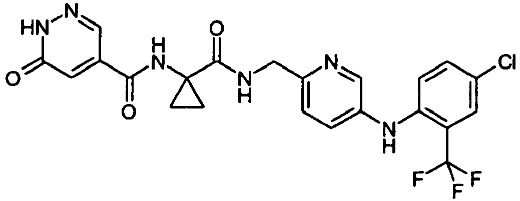

Hergestellt aus Intermediat C1 und 6-Oxo-1,6-dihydro-pyridazin-4-carbonsäure nach AAV1
C₂₂H₁₈ClF₃N₆O₃ (506.87)
Rₜ = 2.13 Minuten (Methode 2)

### Beispiel 285: (S)-5-Amino-N-(3-{[3-chlor-5-(4-chlor-2-trifluormethyl-phenylamino)-pyridin-2-ylmethyl]-carbamoyl}-tetrahydrofuran-3-yl)-nicotinamid

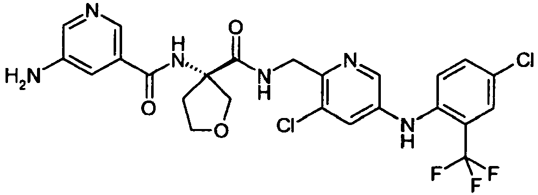

Hergestellt aus den intermediaten A18 und B2 nach AAV1
C₂₄H₂₁Cl₂F₃N₆O₃ (569.37)
Rₜ = 2.34 Minuten (Methode 2)

### Beispiel 286: (S)-N-(3-{[3-Chlor-5-(4-chlor-2-trifluormethyl-phenylamino)-pyridin-2-yl-methyl]-carbamoyl}-tetrahydrofuran-3-yl)-5-hydroxy-nicotinamid

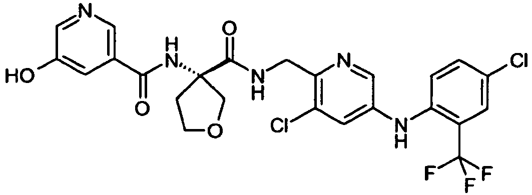

Hergestellt aus den Intermediaten A18 und B8 nach AAV 1
C₂₄H₂₀Cl₂F₃N₅O₄ (570.35)
Rₜ = 2.52 Minuten (Methode 2)
Massenspektroskopie (ESI): [M+H]+ = 570; [M-H]- = 568

### Beispiel 287: (S)-Pyrimidin-5-carbonsäure-{3-[4-(4-fluor-2-trifluormethyl-phenylamino)-benzylcarbamoyl]-tetrahydrofuran-3-yl}-amid

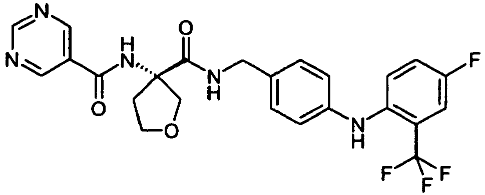

Hergestellt aus den Intermediaten A6 und B3 nach AAV1
C₂₄E₂₁F₄N₅O₃ (503.45)
Rₜ = 2.43 Minuten (Methode 2)

### Beispiel 288: (S)-2-Methyl-pyrimidin-5-carbonsäure-{3-[4-(4-fluor-2-trifluormethyl-phenylamino)-benzylcarbamoyl]-tetrahydrofuran-3-yl}-amid

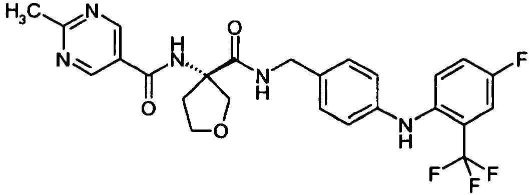

Hergestellt aus den Intermediaten A6 und B7 nach AAV1
C₂₅H₂₃F₅N₅O₃ (517.48)
Rₜ = 2.64 Minuten (Methode 2)

### Beispiel 289: (S)-2-Methylamino-pyrimidin-5-carbonsäure-{3-[4-(4-fluor-2-trifluormethylphenylamino)-benzylcarbamoyl]-tetrahydrofuran-3-yl}-amid

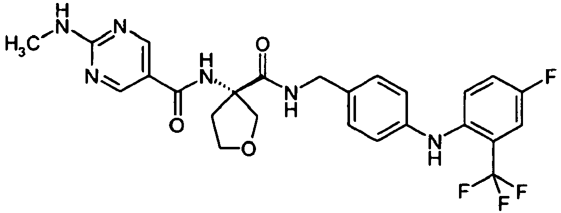

Hergestellt aus den Intermediaten A6 und B6 nach AAV1
C₂₅H₂₄F₄N₆O₃ (532.50)
Rₜ = 2.44 Minuten (Methode 2)

### Beispiel 290: (S)-5-Amino-N-{3-{2-fluor-4-(2-fluor-6-trifluormethyl-phenylamino)-benzylcarbamoyl]-tetrahydrofuran-3-yl}-nicotinamid

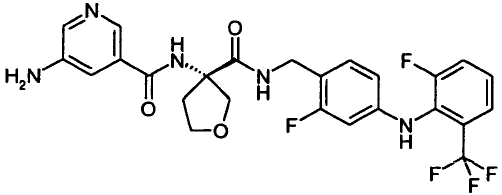

Hergestellt aus Intermediat C11 und 5-Aminonicotinsäure nach AAV1
C₂₅H₂₂F₅N₅O₃ (535.47)
Rₜ = 1.63 Minuten (Methode 7)

### Beispiel 291: (S)-2-Methyl-pyrimidin-5-carbonsäure-{3-[2-fluor-4-(2-fluor-6-trifluormethylphenylamino)-benzylcarbamoyl]-tetrahydrofuran-3-yl}-amid

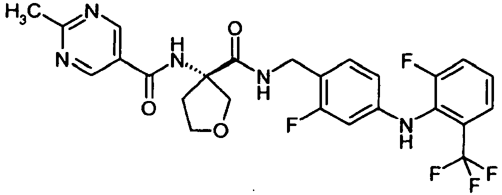

Hergestellt aus Intermediat C11 und 2-Methyl-pyrimidin-5-carbonsäure nach AAV1
C₂₅H₂₂F₅N₅O₃ (535.47)
Rₜ = 1.83 Minuten (Methode 7)

### Beispiel 292: (S)-2-Methyl-pyrimidin-5-carbonsäure-(3-{[3-chlor-5-(2-trifluormethylphenylamino)-pyridin-2-ylmethyl]-carbamoyl}-tetrahydrofuran-3-yl)-amid

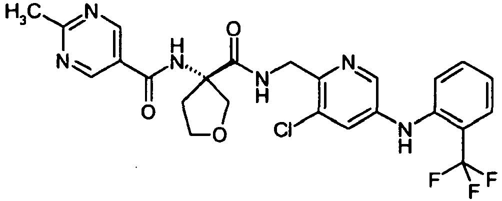

Hergestellt aus den Intermediaten A10 und B7 nach AAV1
C₂₄H₂₂ClF₃N₆O₃ (534.92)
Rₜ = 1.78 Minuten (Methode 7)

### Beispiel 293: (S)-6-Amino-N-(3-{[5-(4-ch)or-2-trifluormethyl-phenylamino)-3-fluor-pyridin-2-ylmethyl]-carbamoyl}-tetrahydrofuran-3-yl)-nicotinamid

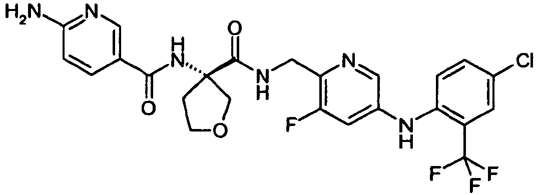

Hergestellt aus den Intermediaten A15 und B9 nach AAV1
C₂₄H₂₁ClF₄N₆O₃ (552.91)
Rₜ = 2.14 Minuten (Methode 2)

### Beispiel 294: 2-Methyl-pyrimidin-5-carbonsäure-{1-[2-fluor-4-(2-trifluormethyl-phenylamino)-benzylcarbamoyl]-cyclopropyl}-amid

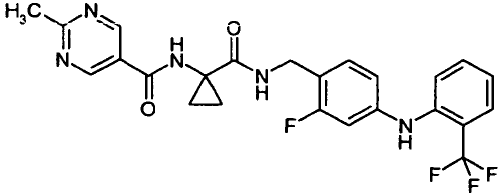

Hergestellt aus Intermediat C12 und 2-Methylpyrimidin-5-carbonsäure nach AAV1
C₂₄H₂₁F₄N₅O₂ (487.45)
Rₜ = 1.91 Minuten (Methode 7)

### Beispiel 295: Pyrimidin-5-carbonsäure-(1-{[5-(2-cyano-phenylamino}-3-fluor-pyridin-2-yl-methyl]-carbamoyl}-cyclopropyl)-amid

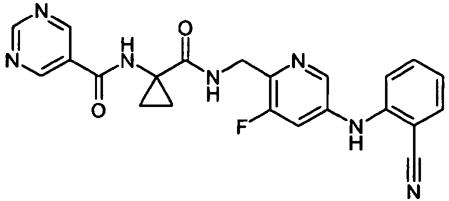

Hergestellt aus den Intermediaten A20 und B1 nach AAV1
C₂₂H₁₈FN₇O₂ (431.43)
Rₜ = 1.62 Minuten (Methode 2)

### Beispiel 296: Pyrimidin-5-carbonsäure-{1-[4-(2-cyano-phenylamino)-2-fluor-benzylcarbamoyl]-cyclopropyl}-amid

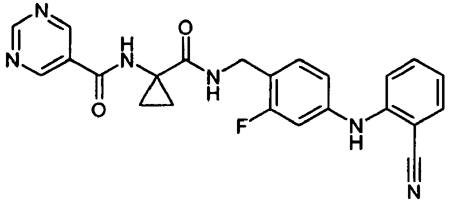

Hergestellt aus den Intermediaten A30 und B1 nach AAV1
C₂₃H₁₉FN₆O₂ (430.44)
Rₜ = 1.88 Minuten (Methode 2)

### Beispiel 297: (S)-6-Amino-N-(3-{[3-chlor-5-(4-chlor-2-trifluormethyl-phenylamino)-pyridin-2-ylmethyl]-carbamoyl}-tetrahydrofuran-3-yl)-nicotinamid

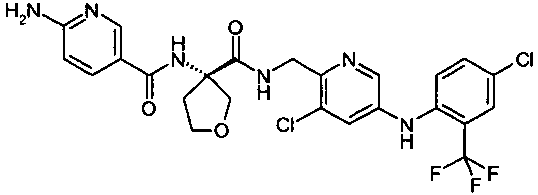

Hergestellt aus den Intermediaten A18 und B9 nach AAV1
C₂₄H₂₁Cl₂F₃N₆O₃ (569.37)
Rₜ = 2.46 Minuten (Methode 2)

### Beispiel 298: 2-Methyl-pyrimidin-5-carbonsäure-(1-{[3-fluor-5-(4-fluor-2-trifluormethylphenylamino)-pyridin-2-ylmethyl]-carbamoyl}-cyclopropyl)-amid

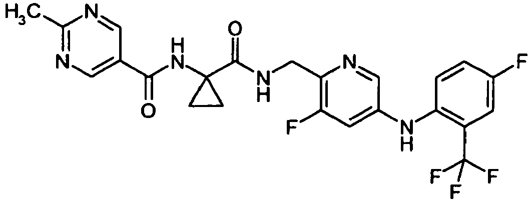

Hergestellt aus Intermediat C13 und 2-Methyl-pyrimidin-5-carbonsäure nach AAV1
C₂₃H₁₉F₅N₆O₂ (506.43)
Rₜ = 1.74 Minuten (Methode 7)

### Beispiel 299: (S)-Thiazol-5-carbonsäure-{3-(4-(4-fluor-2-trifluormethyl-phenylamino)-benzylcarbamoyl]-tetrahydrofuran-3-yl}-amid

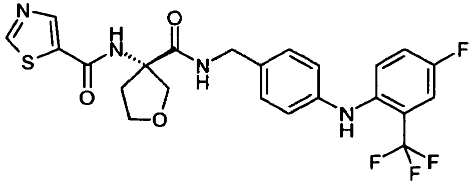

Hergestellt aus Intermediat C14 und Thiazol-5-carbonsäure nach AAV1
C₂₃H₂₀F₄N₄O₃S (508.49)
Rₜ = 2.43 Minuten (Methode 2)

### Beispiel 300: (S)-2-Methoxy-pyrimidin-5-carbonsäure-{3-[4-(4-fluor-2-trifluormethy)-phenylamino)-benzylcarbamoyl]-tetrahydrofuran-3-yl}-amid

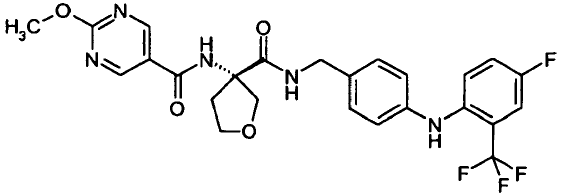

Hergestellt aus Intermediat C14 und 2-Methoxy-pyrimidin-5-carbonsäure nach AAV1
C₂₅H₂₃F₄N₅O₄ (533.48)
Rₜ = 2.61 Minuten (Methode 2)

### Beispiel 301: 2-Methyl-pyrimidin-5-carbonsäure-(1-{[3-chlor-5-(2-trifluormethyl-phenylamino)-pyridin-2-ylmethyl]-carbamoyl}-cyclopropyl)-amid

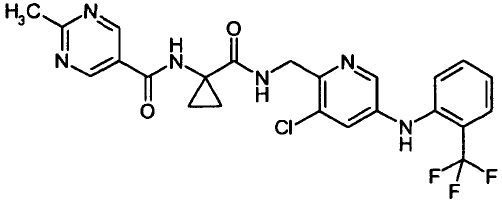

Hergestellt aus Intermediat C15 und 2-Methyl-pyrimidin-5-carbonsäure nach AAV1
C₂₃H₂₀ClF₃N₆O₂ (504.90)
Rₜ = 1.79 Minuten (Methode 7)

### Beispiel 302: N-(1-([5-(4-Fluor-2-trifluormethyl-phenylamino)-pyridin-2-ylmethyl]-carbamoyl)-cyclopropyl)-5-methyl-nicotinamid

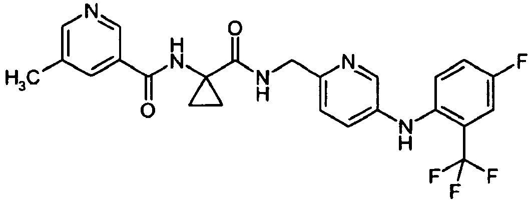

Hergestellt aus Intermediat C2 und 5-Methyl-nicotinsäure nach AAV1
C₂₄H₂₁F₄N₅O₂ (487.45)
Rₜ = 1.96 Minuten (Methode 2)

### Beispiel 303: 2-Methyl-thiazol-5-carbonsäure-(1-{[5-(4-fluor-2-trifluormethyl-phenylamino)-pyridin-2-ylmethyl]-carbamoyl}-cyclopropyl)-amid

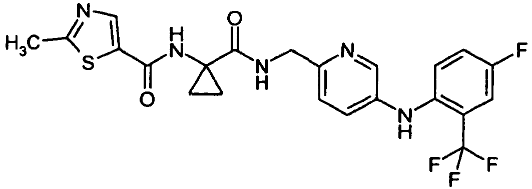

Hergestellt aus Intermediat C2 und 2-Methyl-thiazol-5-carbonsäure nach AAV1
C₂₂H₁₉F₄N₅O₂S (493.48)
Rₜ = 2.07 Minuten (Methode 2)

### Beispiel 304: (S)-2-Methylamino-pyrimidin-5-carbonsäure-(3-{[5-(4-fluor-2-trifluormethylphenylamino)-pyridin-2-ylmethyl]-carbamoyl}-tetrahydrofuran-3-yl)-amid

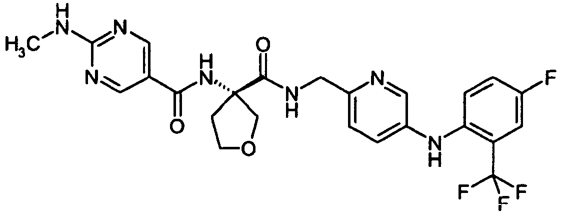

Hergestellt aus den Intermediaten A7 und B6 nach AAV1
C₂₄H₂₃F₄N₇O₃ (533.48)
Rₜ = 1.88 Minuten (Methode 2)

### Beispiel 305: (S)-N-{3-[4-(4-Fluor-2-trifluormethyl-phenylamino)-benzylcarbamoyl]-tetrahydrofuran-3-yl}-5-methylamino-nicotinamid

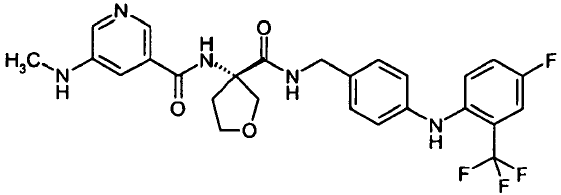

Hergestellt aus Intermediat C14 und 5-Methylaminonicotinsäure nach AAV1
C₂₆H₂₅F₄N₅O₃ (531.51)
Rₜ = 1.83 Minuten (Methode 7)

### Beispiel 306: Pyrimidin-5-carbonsäure-(1-{[5-(4-fluor-2-trifluormethyl-phenylamino)-3-methyl-pyridin-2-ylmethyl]-carbamoyl}-cyclopropyl)-amid

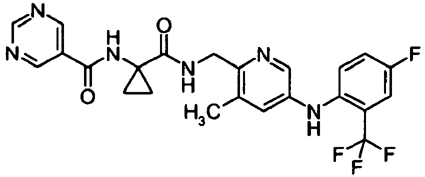

Hergestellt aus den Intermediaten A21 und B1 nach AAV1
C₂₃H₂₀F₄N₆O₂ (488.44)
Rₜ = 1.81 Minuten (Methode 2)

### Beispiel 307: (S)-2-Methyl-pyrimidin-5-carbonsäure-(3-{[5-(4-fluor-2-trifluormethylphenylamino)-pyridin-2-ylmethyl]-carbamoyl}-tetrahydrofuran-3-yl)-amid

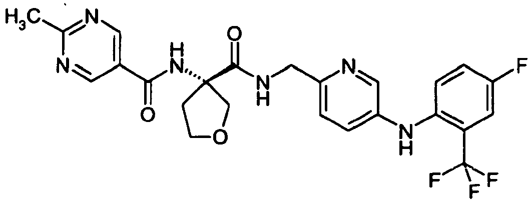

Hergestellt aus den Intermediaten A7 und B7 nach AAV1
C₂₄H₂₂F₄N₆O₃ (518.47)
Rₜ = 2.32 Minuten (Methode 2)

### Beispiel 308: (S)-N-{3-[4-(4-Fluor-2-trifluormethyl-phenylamino)-benzylcarbamoyl]-tetrahydrofuran-3-yl}-5-methyl-nicotinamid

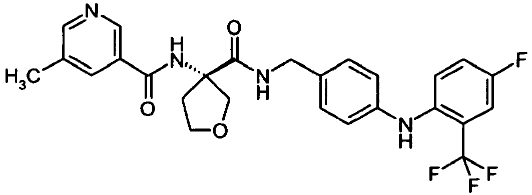

Hergestellt aus den Intermediaten C14 und 5-Methylnicotinsäure nach AAV1
C₂₆H₂₄F₄N₄O₃ (516.49)
Rₜ = 2.45 Minuten (Methode 2)

### Beispiel 309: (S)-6-Amino-N-{3-[4-(4-fluor-2-trifluormethyl-phenylamino)-benzylcarbamoyl]-tetrahydrofuran-3-yl}-nicotinamid

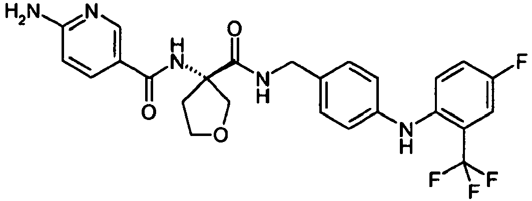

Hergestellt aus den Intermediaten C14 und 6-Aminonicotinsäure nach AAV1
C₂₅H₂₃F₄N₅O₃ (517.48)
Rₜ = 2.09 Minuten (Methode 2)

### Beispiel 310: (S)-2-Isopropyl-pyrimidin-5-carbonsäure-(3-[4-(4-fluor-2-trifluormethylphenylaminohbenzylcarbamoyl]-tetrahydrofuran-3-yl}-amid

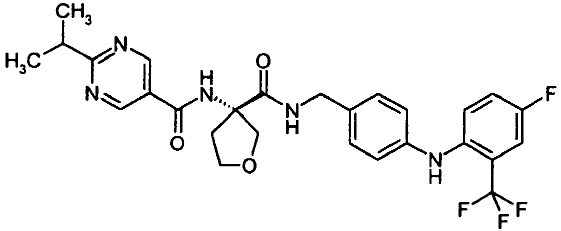

Hergestellt aus den Intermediaten C14 und 2-Isopropyl-pyrimidin-5-carbonsäure nach AAV1
C₂₇H₂₇F₄N₅O₃ (545.53)
Rₜ = 2.60 Minuten (Methode 2)

### Beispiel 311: (S)-N-(3-{[5-(4-Fluor-2-trifluormethyl-phenylamino)-pyridin-2-ylmethyl]-carbamoyl}-tetrahydrofuran-3-yl)-5-methylamino-nicotinamid

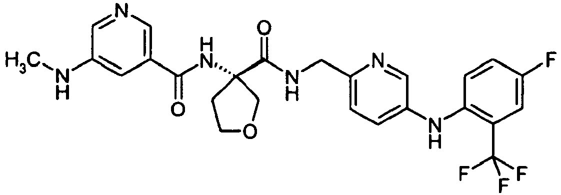

Hergestellt aus den Intermediaten C16 und 5-Methylamino-nicotinsäure nach AAV1
C₂₅H₂₄F₄N₆O₃ (532.50)
Rₜ = 2.22 Minuten (Methode 2)

### Beispiel 312: N-(1-{[5-(4-Chlor-2-trifluormethyl-phenylamino)-pyridin-2-ylmethyl]-carbamoyl}-cyclopropyl)-6-methyl-nicotinamid

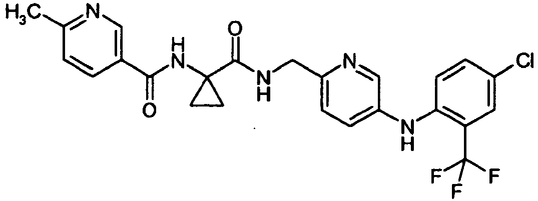

Hergestellt aus den Intermediaten C1 und 6-Methyl-nicotinsäure nach AAV1
C₂₄H₂₁ClF₃NsO₂ (503.91)
Rₜ = 2.17 Minuten (Methode 2)

### Beispiel 313: N-(1-{5-(4-Chlor-2-trifluormethyl-phenylamino)-pyridin-2-ylmethyl]-carbamoyl}-cyclopropyl)-5-methoxy-nicotinamid

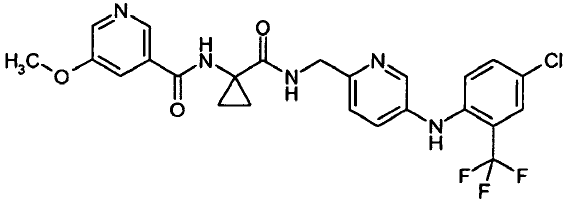

Hergestellt aus den Intermediaten C1 und 5-Methoxy-nicotinsäure nach AAV1
C₂₄H₂₁ClF₃N₅O₃ (519.91)
Rₜ = 2.29 Minuten (Methode 2)

### Beispiel 314: (S)-2-Methoxy-pyrimidin-5-carbonsäure-(3-{[5-(4-chlor-2-trifluormethylphenylamino)-3-fluor-pyridin-2-ylmethyl]-carbamoyl}-tetrahydrofuran-3-yl)-amid

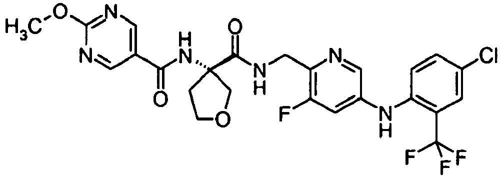

Hergestellt aus den Intermediaten C17 und 2-Methoxy-pyrimidin-5-carbonsäure nach AAV1
C₂₄H₂₁ClF₄N₆O₄ (568.91)
Rₜ = 2.53 Minuten (Methode 2)

### Beispiel 315: N-(1-{[5-(4-Brom-2-triftuormethyl-phenylamino)-pyridin-2-ylmethyl]-carbamoyl}-cyclopropyl)-6-methyl-nicotinamid

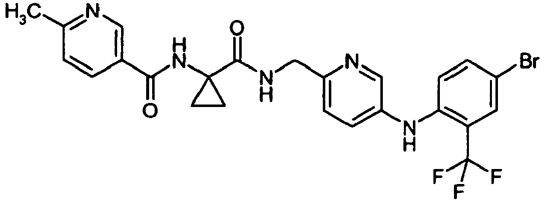

Hergestellt aus den Intermediaten C4 und 6-Methyl-nicotinsäure nach AAV1
C₂₄H₂₁BrF₃N₅O₂ (548.36)
Rₜ = 2.24 Minuten (Methode 2)

### Beispiel 316: N-(1-{[5-(4-Brom-2-trifluormethyl-phenylamino)-pyridin-2-ylmethyl]-carbamoyl}-cyclopropyl)-5-methoxy-nicotinamid

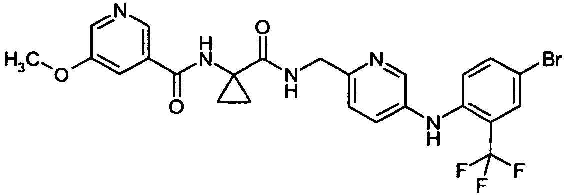

Hergestellt aus den Intermediaten C4 und 5-Methoxy-nicotinsäure nach AAV1
C₂₄H₂₁BrF₃N₅O₃ (564.36)
Rₜ = 2.34 Minuten (Methode 2)

### Beispiel 317: (S)-2-Methyl-pyrimidin-5-carbonsäure-{3-[4-(4-chlor-2-trifluormethyl-phenylamino)-benzylcarbamoyl]-tetrahydrofuran-3-yl}-amid

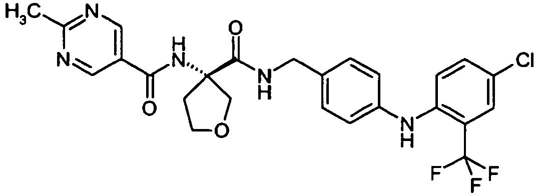

Hergestellt aus den Intermediaten A22 und B7 nach AAV1
C₂₅H₂₃ClF₃N₅O₃ (533.94)
Rₜ = 2.60 Minuten (Methode 2)

### Beispiel 318: (S)-6-Amino-N-{3-[4-(4-chlor-2-trifluormethyl-phenylamino)-benzylcarbamoyl]-tetrahydrofuran-3-yl}-nicotinamid

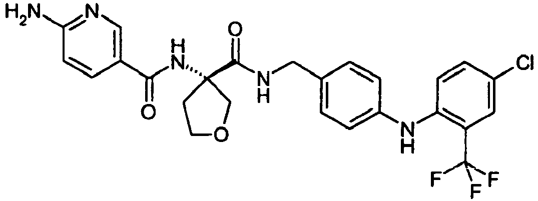

Hergestellt aus den Intermediaten A22 und B9 nach AAV1
C₂₅H₂₃ClF₃N₅O₃ (533.94)
Rₜ = 2.25 Minuten (Methode 2)

### Beispiel 319: (S)-N-(3-{[5-(4-Chlor-2-triftuormethyl-phenylamino)-3-fluor-pyridin-2-yl-methyl]-carbamoyl}-tetrahydrofuran-3-yl)-5-methylamino-nicotinamid

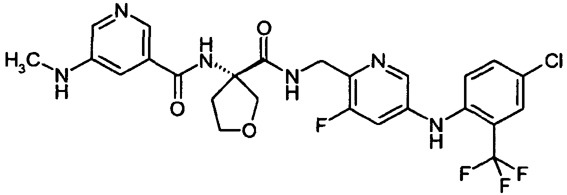

Hergestellt aus den Intermediaten C17 und 5-Methylamino-nicotinsäure nach AAV1
C₂₅H₂₃ClF₄N₆O₃ (566.94)
Rₜ = 2.21 Minuten (Methode 2)

### Beispiel 320: (S)-5-Amino-N-(3-{[3-fluor-5-(2-trifluormethyl-phenylamino)-pyridin-2-yl-methyl]-carbamoyl}-tetrahydrofuran-3-yl)-nicotinamid

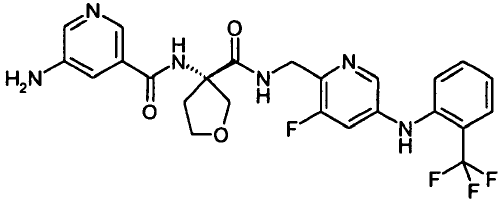

Hergestellt aus den Intermediaten A23 und B2 nach AAV1
C₂₄H₂₂F₄N₆O₃ (518.47)
Rₜ = 1.52 Minuten (Methode 7)

### Beispiel 321: (S)-5-Amino-N-{3-[4-(4-chlor-2-trifluormethyl-phenylamino)-benzylcarbamoyl]-tetrahydrofuran-3-yl}-nicotinamid

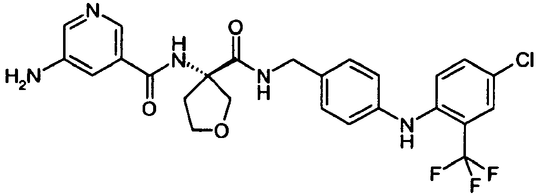

Hergestellt aus den Intermediaten A22 und B2 nach AAV1
C₂₅H₂₃ClF₃N₅O₃ (533.94)
Rₜ = 2.36 Minuten (Methode 2)

### Beispiel 322: (S)-6-Oxo-1,6-dihydro-pyridazin-4-carbonsäure-(3-[4-(4-fluor-2-trifluormethyl-phenylamino)-benzylcarbamoyl]-tetrahydrofuran-3-yl}-amid

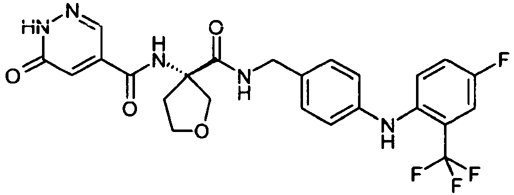

Hergestellt aus den Intermediaten C14 und 6-Oxo-1,6-dihydro-pyridazin-4-carbonsäure nach AAV1
C₂₄H₂₁F₄N₅O₄ (519.45)
Rₜ = 2.39 Minuten (Methode 2)

### Beispiel 323: (S)-2-Methylamino-pyrimidin-5-carbonsäure-{3-[4-(4-chlor-2-trifluormethylphenylamino)-benzylcarbamoyl]-tetrahydrofuran-3-yl}-amid

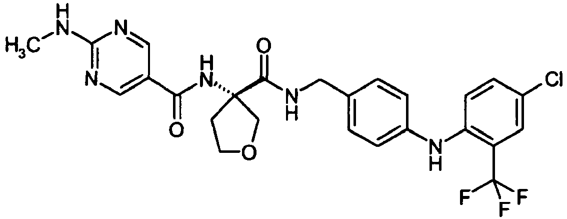

Hergestellt aus den Intermediaten A22 und B6 nach AAV1
C₂₅H₂₄ClF₃N₆O₃ (548.95)
Rₜ = 2.57 Minuten (Methode 2)

### Beispiel 324: (S)-Pyrimidin-5-carbonsäure-(3-([5-(4-fluor-2-trifluormethyl-phenylamino)-3-methyl-pyridin-2-ylmethyl]-carbamoyl}-tetrahydrofuran-3-yl)-amid

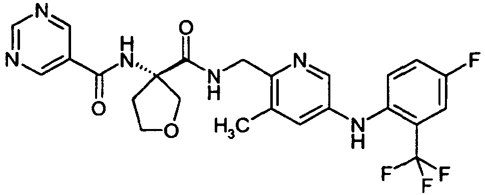

Hergestellt aus den Intermediaten A21 und B3 nach AAV1.
C₂₄H₂₂F₄N₆O₃ (518.47)
Rₜ = 1.80 Minuten (Methode 2)
Massenspektroskopie (ESI): [M+H]+ = 519
[M-H]-=517

### Beispiel 325: Pyrimidin-5-carbonsäure-(1-{[5-(4-brom-2-chlor-6-fluor-phenylamino)-pyridin-2-ylmethyl]-carbamoyl}-cyclopropyl)-amid

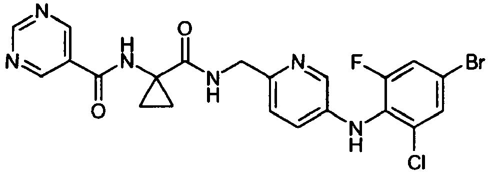

Hergestellt aus den Intermediaten A24 und B1 nach AAV1.
C₂₁H₁₇BrClFN₆O₂ (519.76)
Rₜ = 2.01 Minuten (Methode 2)

### Beispiel 326: Pyrimidin-5-carbonsäure-(1-{[5-(2-brom-6-fluor-phenylamino)-pyridin-2-yl-methyl)-carbamoyl)-cyclopropyl)-amid

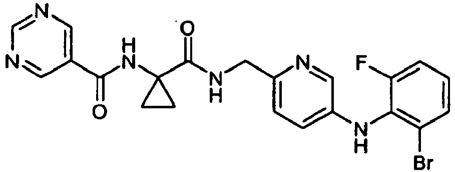

Hergestellt aus den Intermediaten A25 und B1 nach AAV1.
C₂₁H₁₈BrFN₆O₂ (486.32)
Rₜ = 1.64 Minuten (Methode 2)

### Beispiel 327: (S)-2-Methoxy-pyrimidin-5-carbonsäure-(3-{[5-(4-fluor-2-trifluormethylphenylamino)-pyridin-2-ylmethyl]-carbamoyl}-tetrahydrofuran-3-yl)-amid

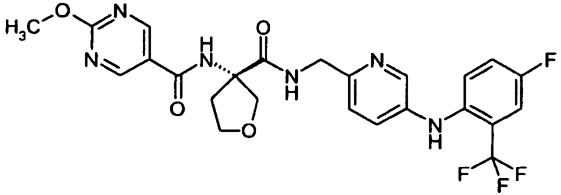

Hergestellt aus den Intermediaten C16 und 2-Methoxy-pyrimidin-5-carbonsäure nach AAV1.
C₂₄H₂₂F₄N₆O₄ (534.47)
Rₜ = 1.98 Minuten (Methode 2)

### Beispiel 328: (S)-2-Ethylamino-pyrimidin-5-carbonsäure-{3-[4-(4-fluor-2-trifluormethy)-phenylamino)-benzylcarbamoyl]-tetrahydrofuran-3-y}-amid

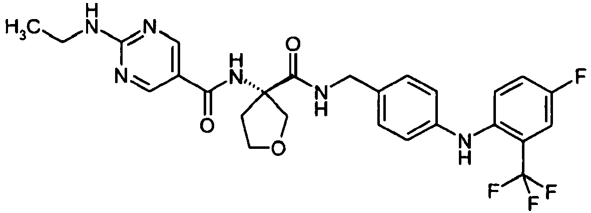

Hergestellt aus den Intermediaten C14 und 2-Ethylamino-pyrimidin-5-carbonsäure nach AAV1.
C₂₆H₂₆F₄N₆O₃ (546.52)
Rₜ = 2.62 Minuten (Methode 2)

### Beispiel 329: N-(1-{[5-(4-Chlor-2-trifluormethyl-phenylamino)-pyridin-2-ylmethyl]-carbamoyl}-cyclopropyl)-6-ethylamino-nicotinamid

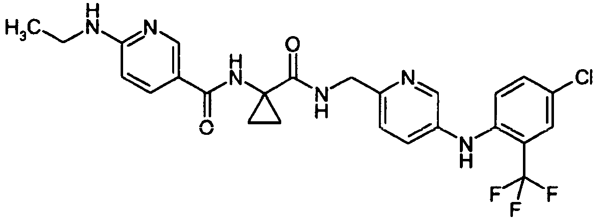

Hergestellt aus den Intermediaten C1 und 6-Ethylamino-nicotinsäure nach AAV1.
C₂₅H₂₄ClF₃N₆O₂ (532.95)
Rₜ = 2.32 Minuten (Methode 2)

### Beispiel 330: N-(1-{[5-(4-Chlor-2-trifluormethyl-phenylamino}-pyridin-2-ylmethyl]-carbamoyl}-cyclopropyl)-6-propylamino-nicotinamid

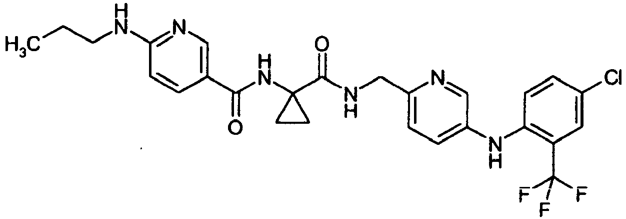

Hergestellt aus den Intermediaten C1 und 6-Propylamino-nicotinsäure nach AAV1.
C₂₆H₂₆ClF₃N₆O₂ (546.98)
Rₜ = 2.04 Minuten (Methode 2)

### Beispiel 331: (S)-N-(3-{[5-(4-Chlor-2-trifluormethyl-phenylamino)-3-fluor-pyridin-2-yl-methyl]-carbamoyl}-tetrahydrofuran-3-yl)-5-hydroxy-nicotinamid

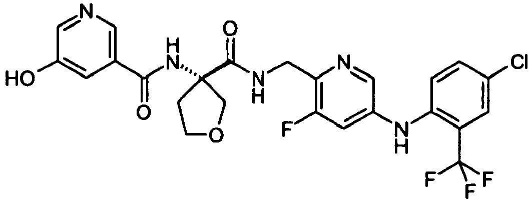

Hergestellt aus den Intermediaten A15 und B8 nach AAV1.
C₂₄H₂₀ClF₄N₅O₄ (553.90)
Rₜ = 2.48 Minuten (Methode 2)

### Beispiel 332: (S)-Pyrimidin-5-carbonsäure-(3-[4-(4-chlor-2-trifluormethyl-phenylamino)-benzylcarbamoyl]-tetrahydrofuran-3-yl}-amid

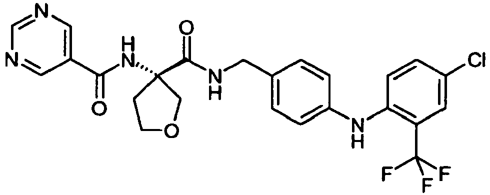

Hergestellt aus den Intermediaten C18 und Pyrimidin-5-carbonsäure nach AAV1.
C₂₄H₂₁ClF₃N₅O₃ (519.91)
Rₜ = 2.57 Minuten (Methode 2)

### Beispiel 333: (S)-2-Methoxy-pyrimidin-5-carbonsäure-{3-[4-(4-chlor-2-trifluormethylphenylamino)-benzylcarbamoyl]-tetrahydrofuran-3-yl}-amid

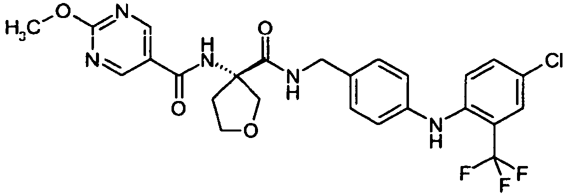

Hergestellt aus den Intermediaten C18 und 2-Methoxy-pyrimidin-5-carbonsäure nach AAV1.
C₂₅H₂₃ClF₃N₅O₄ (549.93)
Rₜ = 2.72 Minuten (Methode 2)

### Beispiel 334: N-(1-{[5-(4-Brom-2-trifluormethyl-phenylamino)-pyridin-2-ylmethyl]-carbamoyl}-cyclopropyl)-6-ethylamino-nicotinamid

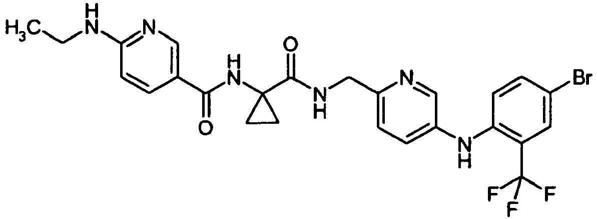

Hergestellt aus den Intermediaten C4 und 6-Ethylamino-nicotinsäure nach AAV1.
C₂₅H₂₄CBrF₃N₆O₂ (577.40)
Rₜ = 2.01 Minuten (Methode 2)

### Beispiele 335: N-(1-{(5-(4-Brom-2-trifluormethyl-phenylamino)-pyridin-2-ylmethyl]-carbamoyl}-cyclopropyl)-6-propylamino-nicotinamid

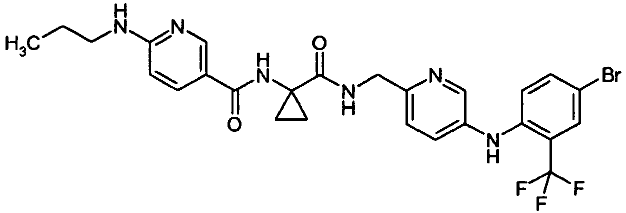

Hergestellt aus den Intermediaten C4 und 6-Propylamino-nicotinsäure nach AAV1.
C₂₆H₂₆BrF₃N₆O₂ (591.43)
Rₜ = 2.12 Minuten (Methode 2)

### Beispiel 336: (S)-Thiazol-5-carbonsäure-{3-[4-(4-chlor-2-trifluormethyl-phenylamino)-benzylcarbamoyl]-tetrahydrofuran-3-yl}-amid

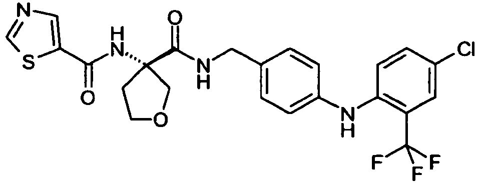

Hergestellt aus den Intermediaten C18 und Thiazol-5-carbonsäure nach AAV1.
C₂₃H₂₀ClF₃N₄O₃S (524.95) Rₜ = 2.58 Minuten (Methode 2)

### Beispiel 337: (S)-N-{3-[4-(4-Chlor-2-trifluormethyl-phenylamino)-benzylcarbamoyl]-tetrahydrofuran-3-yl}-5-methoxy-nicotinamid

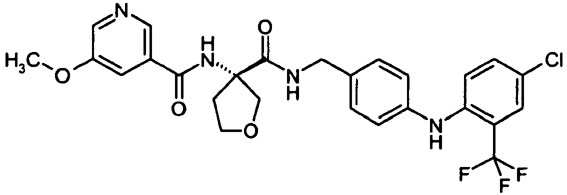

Hergestellt aus den Intermediaten C18 und 5-Methoxy-nicotinsäure nach AAV1.
C₂₆H₂₄ClF₃N₄O₄ (548.95)
Rₜ = 2.62 Minuten (Methode 2)

### Beispiel 338: (S)-6-Oxo-1,6-dihydro-pyridazin-4-carbonsäure-{3-[4-(4-chlor-2-trifluormethyl-phenylamino)-benzylcarbamoyl]-tetrahydrofuran-3-yl}-amid

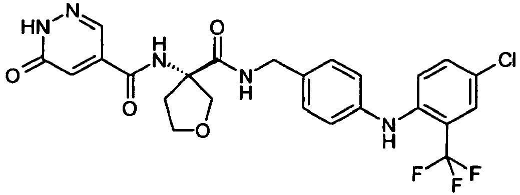

Hergestellt aus den Intermediaten A22 und B10 nach AAV1.
C₂₄H₂₁ClF₃N₅O₄ (535.91)
Rₜ = 2.28 Minuten (Methode 2)

### Beispiel 339: 5-Chlor-N-(1-{[5-(4-fluor-2-trifluormethyl-phenylamino}-pyridin-2-ylmethyl]-carbamoyl}-cyclopropyl)-nicotinamid

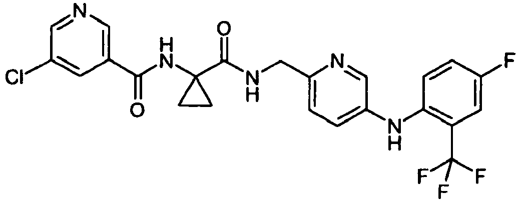

Hergestellt aus den Intermediaten C2 und 5-Chlor-nicotinsäure nach AAV1.
C₂₃H₁₈ClF₄N₅O₂ (507.87)
Rₜ = 2.18 Minuten (Methode 2)

### Beispiel 340: N-(1-{[5-(4-Fluor-2-trifluormethyl-phenylamino)-pyridin-2-ylmethyl]-carbamoyl}-cyclopropyl)-5-trifluormethyl-nicotinamid

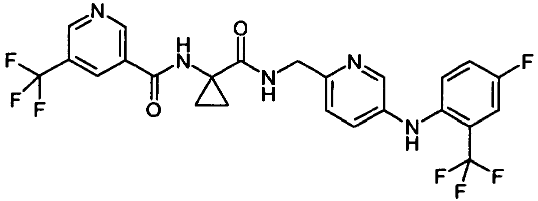

Hergestellt aus den Intermediaten C2 und 5-Trifluormethyl-nicotinsäure nach AAV1.
C₂₄H₁₈F₇N₅O₂ (541.43)
Rₜ = 2.32 Minuten (Methode 2)

### Beispiel 341: N-(1-{[5-(4-Fluor-2-trifluormethyl-phenylamino)-pyridin-2-ylmethyl]-carbamoyl}-cyclopropyl)-6-methyl-nicotinamid

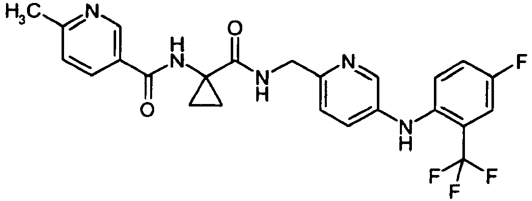

Hergestellt aus den Intermediaten C2 und 6-Methyl-nicotinsäure nach AAV1.
C₂₄H₂₁F₄N₅O₂ (487.45)
Rₜ = 1.85 Minuten (Methode 2)

### Beispiel 342: (S)-5-Amino-N-(3-{[5-(4-fluor-2-trifluormethyl-phenylamino)-3-methylpyridin-2-ylmethyl]-carbamoyl}-tetrahydrofuran-3-yl}-nicotinamid

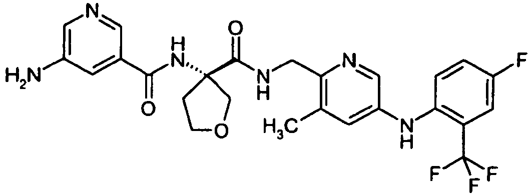

Hergestellt aus den Intermediaten A21 und B2 nach AAV1.
C₂₅H₂₄F₄N₆O₃ (487.45)
Rₜ = 1.85 Minuten (Methode 2)

### Beispiel 343: N-(1-{[5-(4-Brom-2-trifluormethyl-phenylamino)-pyridin-2-ylmethyl]-carbamoyl}-cyclopropyl)-6-methylamino-nicotinamid

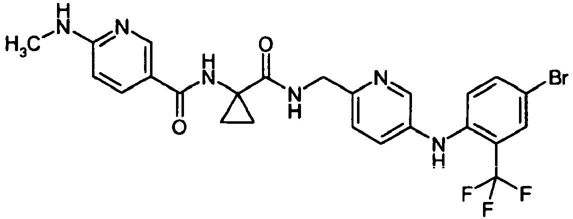

Hergestellt aus den Intermediaten C4 und 6-Methylamino-nicotinsäure nach AAV1.
C₂₄H₂₂BrF₃N₆O₂ (563.38)
Rₜ = 2.32 Minuten (Methode 2)

### Beispiel 344: 2-Methyl-pyrimidin-5-carbonsäure-(1-{[5-(4-fluor-2-trifluormethyl-phenylamino)-3-methyl-pyridin-2-ylmethyl]-carbamoyl}-cyclopropyl)-amid

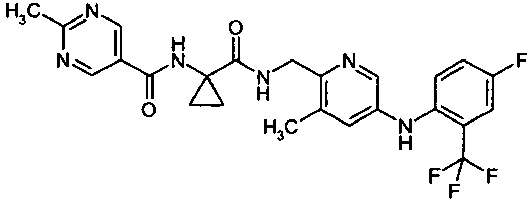

Hergestellt aus den intermediaten C19 und 2-Methyl-pyrimidin-5-carbonsäure nach AAV1.
C₂₄H₂₂F₄N₆O₂ (502.47)
Rₜ = 1.84 Minuten (Methode 2)

### Beispiel 345: (S)-2-Methyl-pyrimidin-5-carbonsäure-(3-{[5-(4-chlor-2-trifluormethy)-phenyl amino)-3-methyl-pyridin-2-ylmethyl]-carbamoyl}-tetrahydrofuran-3-yl)-amid

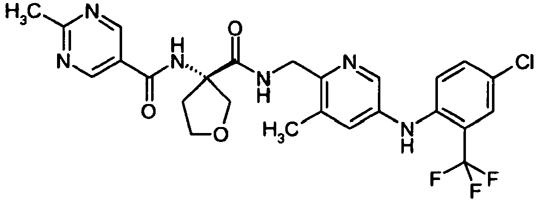

Hergestellt aus den Intermediaten A26 und B7 nach AAV1.
C₂₅H₂₄ClF₃N₆O₃ (548.95)
Rₜ = 2.16 Minuten (Methode 2)

### Beispiel 346: (S)-5-Amino-N-(3-([5-(4-chlor-2-trifluormethyl-phenylamino)-3-methylpyridin-2-ylmethyl]-carbamoyl}-tetrahydrofuran-3-yl)-nicotinamid

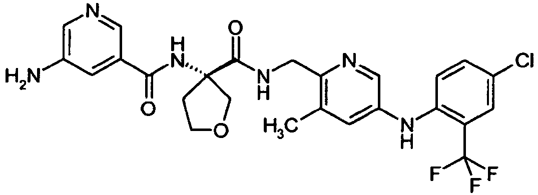

Hergestellt aus den Intermediaten A26 und B2 nach AAV1.
C₂₅H₂₄ClF₃N₆O₃ (548.95)
Rₜ = 2.23 Minuten (Methode 2)

### Beispiel 347: N-(1-{[5-(4-Chlor-2-trifluormethyl-phenylamino)-pyridin-2-ylmethyl]-carbamoyl}-cyclopropyl)-6-methylamino-nicotinamid

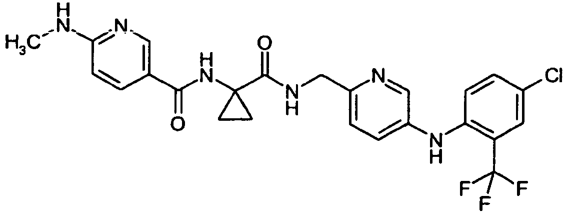

Hergestellt aus den Intermediaten C1 und 6-Methylamino-nicotinsäure nach AAV1.
C₂₄H₂₂ClF₃N₆O₂ (518.92)
Rₜ = 1.85 Minuten (Methode 2)

### Beispiel 348: (S)-6-Amino-N-(3-{[3-fluor-5-(4-fluor-2-trifluormethyl-phenylamino)-pyridin-2-ylmethyl]-carbamoyl]-tetrahydrofuran-3-yl)-nicotinamid

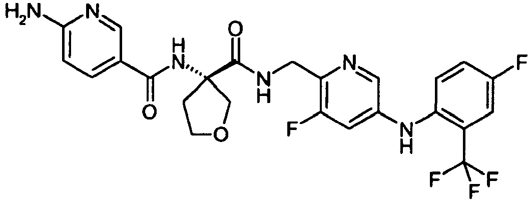

Hergestellt aus den Intermediaten A14 und B9 nach AAV1.
C₂₄H₂₁F₅N₆O₃ (536.46)
Rₜ = 1.96 Minuten (Methode 2)
Massenspektroskopie (ESI): [M+H]+ = 537
[M-H]- = 535

### Beispiel 349: (S)-6-Amino-N-(3-{[5-(4-fluor-2-trifluormethyl-phenylamino)-pyridin-2-yl-methyl]-carbamoyl}-tetrahydrofuran-3-yl)-nicotinamid

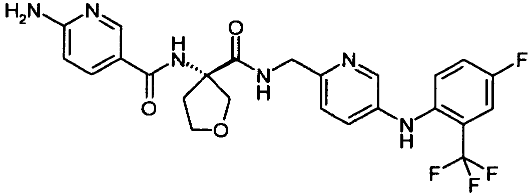

Hergestellt aus den Intermediaten A7 und B9 nach AAV1.
C₂₄H₂₂F₄N₆O₃ (518.47)
Massenspektroskopie (ESI): [M+H]+ = 519
[M-H]- = 517

### Beispiel 350: 2-Hydroxy-pyrimidin-5-carbonsäure-(1-{[5-(4-chlor-2-trifluormethyl-phenylamino)-pyridin-2-ylmethyl]-carbamoyl}-cyclopropyl)-amid

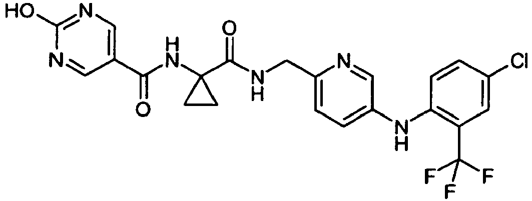

Hergestellt aus den Intermediaten C1 und 2-Hydroxy-pyrimidin-5-carbonsäure nach AAV1.
C₂₂H₁₈ClF₃N₆O₃ (506.87)
Rₜ = 2.42 Minuten (Methode 2)

### Beispiel 351: N-(1-{[5-(4-Chlor-2-trifluormethyl-phenylamino)-pyridin-2-ylmethyl]-carbamoyl}-cyclopropyl)-5-fluor-nicotinamid

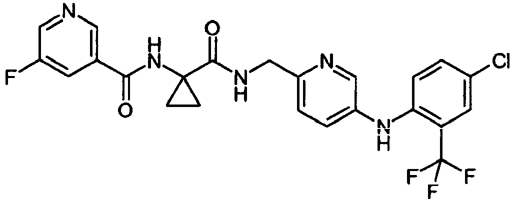

Hergestellt aus den Intermediaten C1 und 5-Fluor-nicotinsäure nach AAV1.
C₂₃H₁₈ClF₄N₅O₂ (507.87)
Rₜ = 1.96 Minuten (Methode 2)

### Beispiel 352: 5-Chlor-N-(1-{[5-(4-chlor-2-trifluormethyl-phenylamino)-pyridin-2-ylmethyl]-carbamoyl}-cyclopropyl)-nicotinamid

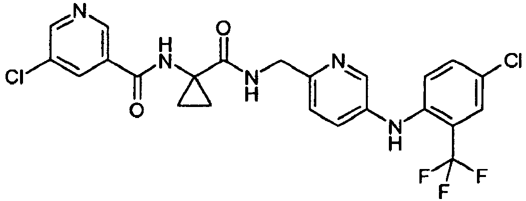

Hergestellt aus den Intermediaten C1 und 5-Chlor-nicotinsäure nach AAV1.
C₂₃H₁₈Cl₂F₃N₅O₂ (524.33)
Rₜ = 2.10 Minuten (Methode 2)

### Beispiel 353: N-(1-{[5-(4-Chlor-2-trifluormethyl-phenylamino)-pyridin-2-ylmethyl]-carbamoyl}-cyclopropyl)-5-methyl-nicotinamid

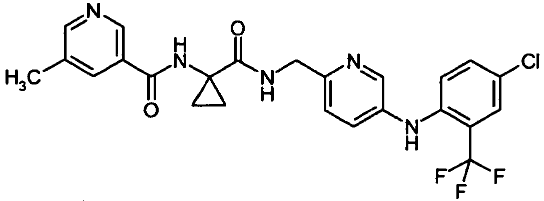

Hergestellt aus den Intermediaten C1 und 5-Methyl-nicotinsäure nach AAV1.
C₂₄H₂₁ClF₃N₅O₂ (503.91)
Rₜ = 1.86 Minuten (Methode 2)

### Beispiel 354: N-(1-{(5-(4-Chlor-2-trifluormethyl-phenylamino)-pyridin-2-ylmethyl}-carbamoyl}-cyclopropyl)-5-trifluormethyl-nicotinamid

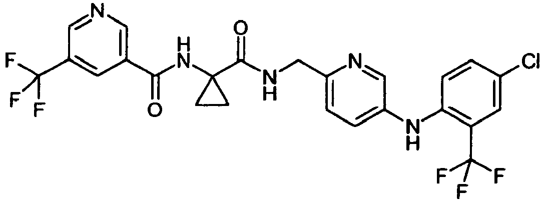

Hergestellt aus den Intermediaten C1 und 5-Trifluormethyl-isonicotinsäure nach AAV1.
C₂₄H₁₈ClF₆N₅O₂ (557.88)
Rₜ = 2.22 Minuten (Methode 2)

### Beispiel 355: N-(1-{[5-(4-Chlor-2-trifluormethyl-phenylamino)-pyridin-2-ylmethyl]-carbamoyl)-cyclopropyl)-2-fluor-isonicotinamid

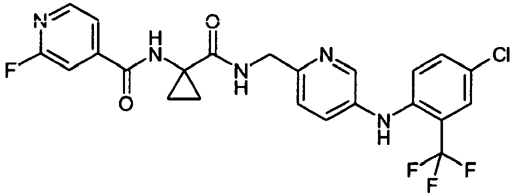

Hergestellt aus den Intermediaten C1 und 2-Fluor-isonicotinsäure nach AAV1.
C₂₃H₁₈ClF₄N₅O₂ (507.87)
Rₜ = 2.02 Minuten (Methode 2)

### Beispiel 356: 3-Methoxy-isoxazol-5-carbonsäure-(1-{[5-(4-chlor-2-trifluormethyl-phenylamino)-pyridin-2-ylmethyl]-carbamoyl}-cyclopropyl)-amid

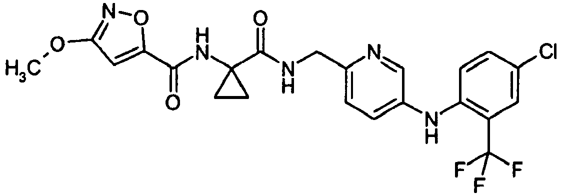

Hergestellt aus den Intermediaten C1 und 3-Methoxy-isoxazol-5-carbonsäure nach AAV1.
C₂₂H₁₉ClF₃N₅O₄ (509.87)
Rₜ = 1.96 Minuten (Methode 2)

### Beispiel 357: Isothiazol-5-carbonsäure-(1-{[5-(4-chlor-2-trifluormethyl-phenytamino)-pyridin-2-ylmethyl]-carbamoyl}-cyclopropyl)-amid

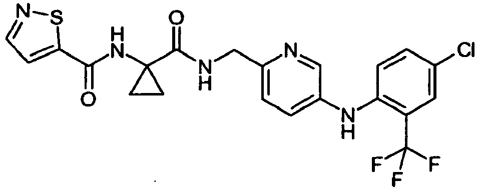

Hergestellt aus den Intermediaten C1 und Isothiazol-5-carbonsäure nach AAV1.
C₂₁H₁₇CF₃N₅O₂S (495.91)
Rₜ = 1.88 Minuten (Methode 2)

### Beispiel 358: isothiazol-4-carbonsäure-(1-{[5-(4-chlor-2-trifluormethyl-phenylamino)-pyridin-2-ylmethyl]-carbamoy}-cyclopropyl)-amid

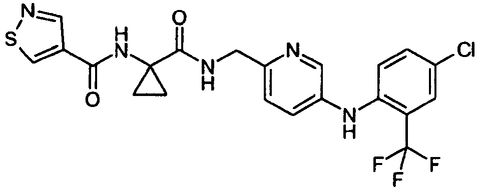

Hergestellt aus den Intermediaten C1 und Isothiazol-4-carbonsäure nach AAV1.
C₂₁H₁₇ClF₃N₅O₂S (495.91)
Rₜ = 1.84 Minuten (Methode 2)

### Beispiel 359: (S)-5-Amino-N-(3-{[3-methyl-5-(2-trifluormethyl-phenylamino)-pyridin-2-yl-methyl]-carbamoyl}-tetrahydrofuran-3-yl)-nicotinamid

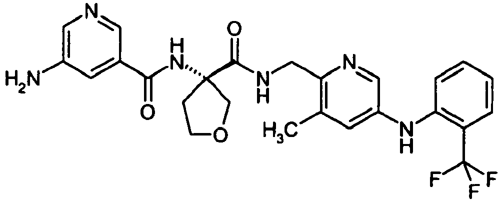

Hergestellt aus den Intermediaten A27 und B2 nach AAV1.
C₂₅H₂₅F₃N₆O₃ (514.51)
Rₜ = 1.51 Minuten (Methode 2)

### Beispiel 360: 2-Methyl-pyrimidin-5-carbonsäure-(1-{[5-(4-chlor-2-trifluormethyl-phenylamino)-3-methyl-pyridin-2-ylmethyl]-carbamoyl}-cyclopropyl)-amid

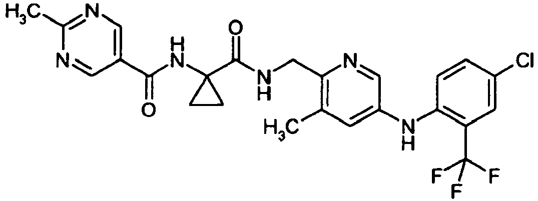

Hergestellt aus den Intermediaten A26 und B11 nach AAV1.
C₂₄H₂₂ClF₃N₆O₂ (518.92)
Rₜ = 2.11 Minuten (Methode 2)

### Beispiel 361: 5-Amino-N-(1-{[5-(4-chlor-2-trifluormethyl-phenylamino)-3-methyl-pyridin-2-ylmethyl]-carbamoyl)-cyclopropyl)-nicotinamid

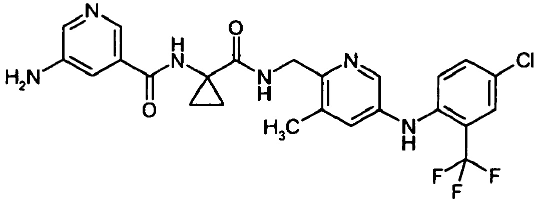

Hergestellt aus den Intermediaten A26 und B4 nach AAV1.
C₂₄H₂₂ClF₃N₆O₂ (518.92)
Rₜ = 1.83 Minuten (Methode 2)

### Beispiel 362: 1-(2,2,2-Trifluor-acetylamino)-cyclopropancarbonsäure-[5-(4-chlor-2-trifluormethyl-phenylamino)-pyridin-2-ylmethyl]-amid

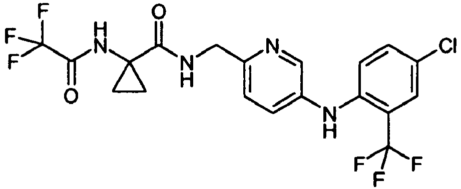

Hergestellt aus den Intermediaten C1 und Trifluoressigsäure nach AAV1
C₁₉H₁₅ClF₆N₄O₂ (480.79)
Rₜ = 2.15 Minuten (Methode 2)

### Beispiel 363: 1-(3,3,3-Trifluor-propionylamino)-cyclopropanecarbonsäure-[5-(4-chlor-2-trifluormethyl-phenylamino)-pyridin-2-ylmethyl]-amid

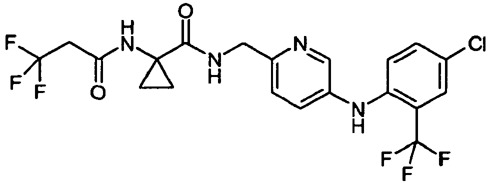

Hergestellt aus den Intermediaten C1 und 3,3,3-Trifluor-propionsäure nach AAV1
C₂₀H₁₇ClF₆N₄O₂ (494.82)
Rₜ = 2.07 Minuten (Methode 2)

### Beispiel 364: 1-(2-Cyano-acetylamino)-cyclopropancarbonsäure-[5-(4-chlor-2-trifluormethyl-phenylamino)-pyridin-2-ylmethyl]-amid

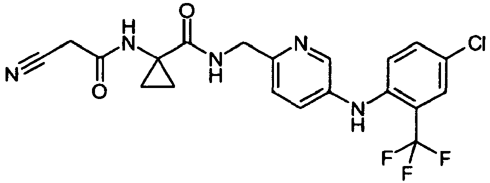

Hergestellt aus den Intermediaten C1 und 2-Cyano-essigsäure nach AAV1.
C₂₀H₁₇ClF₃N₅O₂ (451.83)
Rₜ = 1.85 Minuten (Methode 2)

### Beispiel 365: (S)-1H-Pyrrolo[3,2-b]pyridin-6-carbonsäure-{3-[4-(4-fluor-2-trifluormethylphenylamino)-benzylcarbamoyl]-tetrahydrofuran-3-yl}-amid

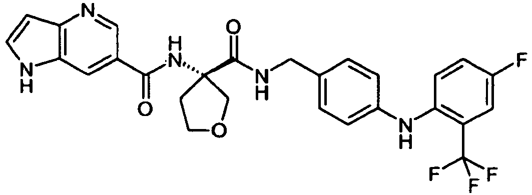

Hergestellt aus den Intermediaten C14 und 1H-Pyrrolo[3,2-b]pyridin-6-carbonsäure nach AAV1.
C₂₇H₂₃F₄N₅O₃ (541.50)
Rₜ = 2.14 Minuten (Methode 2)

### Beispiel 366: (S)-2-Methy)-pyrimidin-5-carbonsäure-(3-{[3-methy)-5-(2-trifluormethylphenylamino)-pyridin-2-ylmethyl]-carbamoyl}-tetrahydrofuran-3-yl)-amid

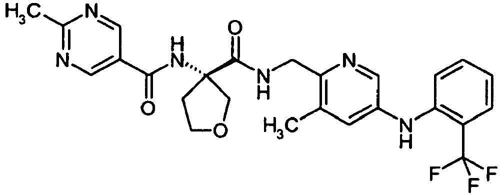

Hergestellt aus den Intermediaten A27 und B7 nach AAV1
C₂₅H₂₅F₃N₆O₃ (514.51)
Rₜ = 1.84 Minuten (Methode 2)

### Beispiel 367: 1-Cyano-1-(cyclopropancarbonyl-amino)-cyclopropancarbonsäure-[5-(4-chlor-2-trifluormethyl-phenylamino)-pyridin-2-ylmethyl]-amid

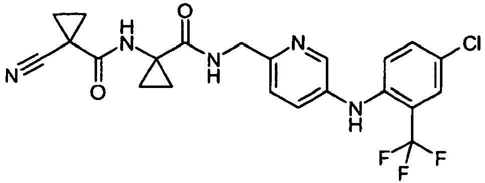

Hergestellt aus den Intermediaten C1 und 1-Cyano-1-cyclopropancarbonsäure nach AAV1.
C₂₂H₁₉ClF₃N₅O₂ (477.87)
Rₜ = 1.96 Minuten (Methode 2)

### Beispiel 368: 1-(2-Cyano-2-methyl-acetylamino)-cyclopropancarbonsäure-[5-(4-chlor-2-trifluormethyl-phenylamino)-pyridin-2-ylmethyl]-amid

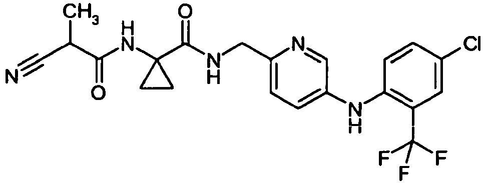

Hergestellt aus den Intermediaten C1 und 2-Cyano-2-methyl-essigsäure nach AAV1.
C₂₁H₁₉ClF₃N₅O₂ (465.86)
Rₜ = 1.92 Minuten (Methode 2)

### Beispiel 369: Isoxazol-5-carbonsäure-(1-{[5-(4-chlor-2-trifluormethyl-phenylamino)-pyridin-2-ylmethyl]-carbamoyl}-cyclopropyl)-amid

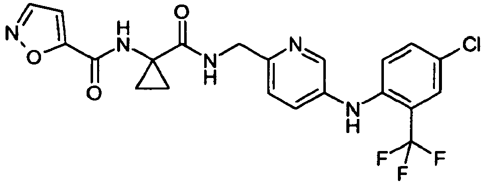

Hergestellt aus den intermediaten C1 und isoxazol-5-carbonsäure nach AAV1.
C₂₁H₁₇ClF₃N₅O₃ (479.84)
Rₜ = 1.89 Minuten (Methode 2)

### Beispiel 370: 2-Amino-pyrimidin-5-carbonsäure-(1-{[5-(4-chlor-2-trifluormethyl-phenylamino)-pyridin-2-ylmethyl]-carbamoyl}-cyclopropyl)-amid

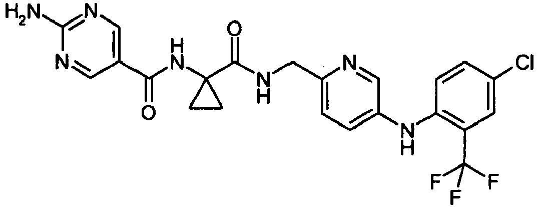

Hergestellt aus den Intermediaten C1 und 2-Amino-pyrimidin-5-carbonsäure nach AAV1.
C₂₂H₁₉ClF₃N₇O₂ (505.89)
Rₜ = 2.11 Minuten (Methode 2)

### Beispiel 371: 2-Ethyl-pyrimidin-5-carbonsäure-(1-{[5-(4-chlor-2-trifluormethyl-phenylamino)-pyridin-2-ylmethyl]-carbamoyl)-cyclopropyl)-amid

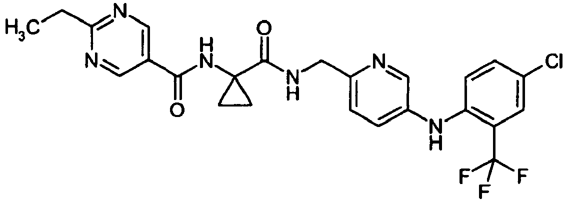

Hergestellt aus den Intermediaten C1 und 2-Ethyl-pyrimidin-5-carbonsäure nach AAV1.
C₂₄H₂₂ClF₃N₆O₂ (518.92)
Rₜ = 2.38 Minuten (Methode 2)

### Beispiel 372: 1H-Pyrazol-4-carbonsäure-(1-{[5-(4-chlor-2-trifluormethyl-phenylamino)-pyridin-2-ylmethyl]-carbamoyl}-cyclopropyl)-amid

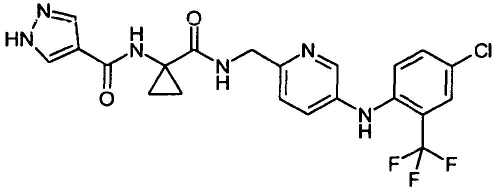

Hergestellt aus den Intermediaten C1 und 1 H-Pyrazol-4-carbonsäure nach AAV1.
C₂₁H₁₈ClF₃N₆O₂ (478.86)
Rₜ = 1.69 Minuten (Methode 2)

### Beispiel 373: 1-Methyl-1H-pyrazol-4-carbonsäure-(1-{[5-(4-chlor-2-trifluormethyl-phenylamino)-pyridin-2-ylmethyl]-carbamoyl}-cyclopropyl)-amid

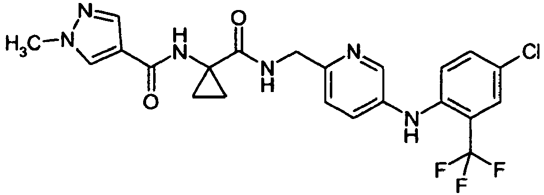

Hergestellt aus den Intermediaten C1 und 1-Methyl-1 H-pyrazol-4-carbonsäure nach AAV1.
C₂₂H₂₀ClF₃N₆O₂ (492.89)
Rₜ = 1.74 Minuten (Methode 2)

### Beispiel 374: 2-Methyl-pyrimidin-5-carbonsäure-(1-{[3-methyl-5-(2-trifluormethyl-phenylamino)-pyridin-2-ylmethyl]-carbamoyl}-cyclopropyl)-amid

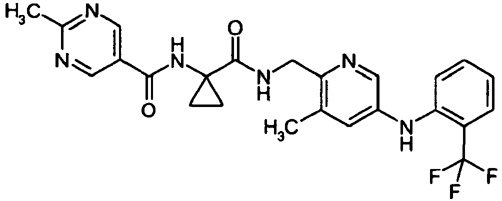

Hergestellt aus den Intermediaten C20 und 2-Methyl-pyrimidin-5-carbonsäure nach AAV1.
C₂₄H₂₃F₃N₆O₂ (484.48)
Rₜ = 1.81 Minuten (Methode 2)

### Beispiel 375: 5-Amino-N-(1-{[3-methyl-5-(2-trifluormethyl-phenylamino)-pyridin-2-yl-methyl]-carbamoyl}-cyclopropyl)-nicotinamid

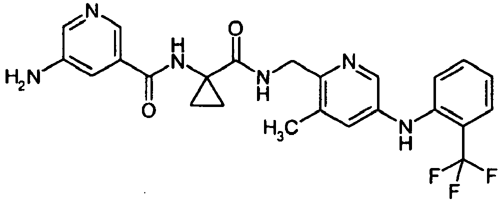

Hergestellt aus den Intermediaten C20 und 5-Amino-nicotinsäure nach AAV1.
C₂₄H₂₃F₃N₆O₂ (484.48)
Rₜ = 1.29 Minuten (Methode 2)

### Beispiel 376: N-(1-{[5-(4-Fluor-2-trifluormethyl-phenylamino)-pyridin-2-ylmethyl]-carbamoyl}-cyclopropyl)-5-methoxy-nicotinamid

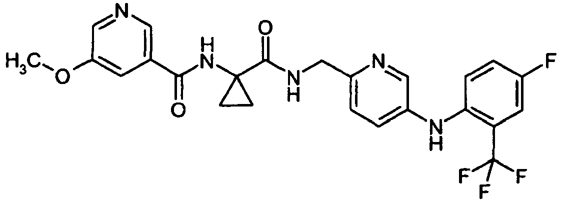

Hergestellt aus den Intermediaten C2 und 5-Methoxy-nicotinsäure nach AAV1.
C₂₄H₂₁F₄N₅O₃ (503.45)
Rₜ = 2.02 Minuten (Methode 2)

### Beispiel 377: N-(1-{[5-(4-Fluor-2-trifluormethyl-phenylamino)-pyridin-2-ylmethyl]-carbamoyl}-cyclopropyl)-6-methylamino-nicotinamid

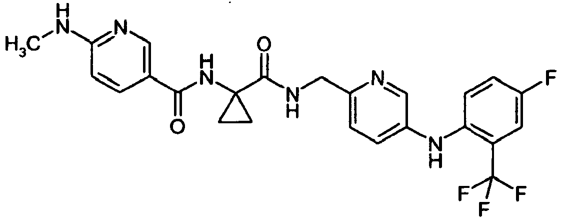

Hergestellt aus den Intermediaten C2 und 6-Methylamino-nicotinsäure nach AAV1.
C₂₄H₂₂F₄N₆O₂ (502.47)
Rₜ = 2.01 Minuten (Methode 2)

### Beispiel 378: 6-Amino-5-brom-N-(1-{[5-(4-chlor-2-trifluormethyl-phenylamino)-pyridin-2-ylmethyl]-carbamoyl}-cyclopropyl)-nicotinamid

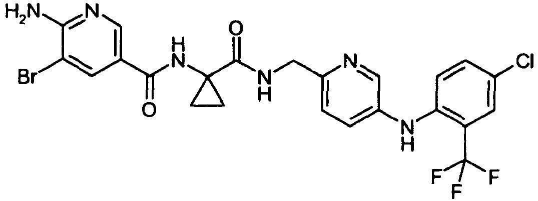

Hergestellt aus den Intermediaten C1 und 6-Amino-5-brom-nicotinsäure nach AAV1.
C₂₃H₁₉BrClF₃N₆O₂ (583.79)
Rₜ = 1.91 Minuten (Methode 2)

### Beispiel 379: 2-Cyclopropylamino-pyrimidin-5-carbonsäure-(1-{[5-(4-chlor-2-trifluormethyl-phenylamino)-pyridin-2-ylmethyl]-carbamoyl}-cyclopropyl)-amid

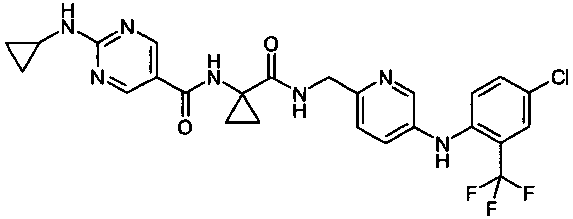

Hergestellt aus den Intermediaten C1 und 2-Cyclopropylamino-pyrimidin-5-carbonsäure nach AAV1.
C₂₅H₂₃ClF₃N₇O₂ (545.95)
Rₜ = 2.29 Minuten (Methode 2)

### Beispiel 380: 2-Propylamino-pyrimidin-5-carbonsäure-(1-{[5-(4-chlor-2-trifluormethylphenylamino)-pyridin-2-ylmethyl]-carbamoyl}-cyclopropyl)-amid

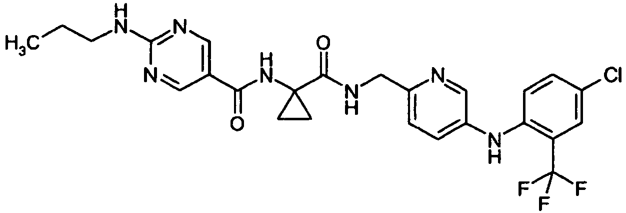

Hergestellt aus den Intermediaten C1 und 2-Propylamino-pyrimidin-5-carbonsäure nach AAV1.
C₂₅H₂₅ClF₃N₇O₂ (547.97)
Rₜ = 2.42 Minuten (Methode 2)

### Beispiel 381: (S)-N-{3-[4-(4-Fluor-2-trifluormethyl-phenylamino)-benzylcarbamoyl]-tetrahydrofuran-3-yl}-5-methoxy-nicotinamid

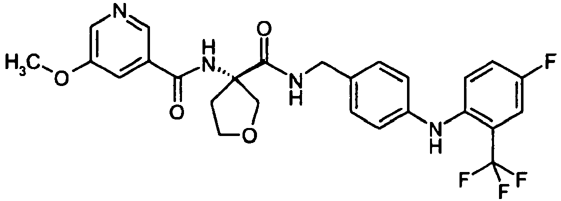

Hergestellt aus den Intermediaten C14 und 5-Methoxy-nicotinsäure nach AAV1.
C₂₆H₂₄F₄N₄O₄ (532.49)
Rₜ = 2.50 Minuten (Methode 2)

### Beispiel 382: 2-Isopropylamino-pyrimidin-5-carbonsäure-(1-{[5-(4-chlor-2-trifluormethylphenylamino)-pyridin-2-ylmethyl]-carbamoyl}-cyclopropyl)-amid

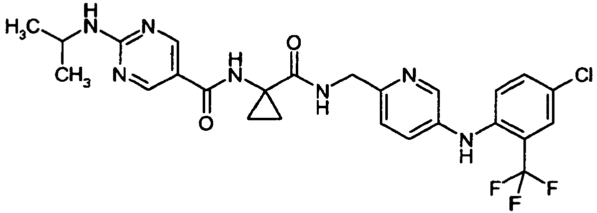

Hergestellt aus den Intermediaten C1 und 2-Isopropylamino-pyrimidin-5-carbonsäure nach AAV1.
C₂₅H₂₅ClF₃N₇O₂ (547.97)
Rₜ = 2.41 Minuten (Methode 2)

### Beispiel 383: (S)-1H-Pyrrolo[2,3-b]pyridin-5-carbonsäure-{3-[4-(4-fluor-2-trifluormethylphenylamino)-benzylcarbamoyl]-tetrahydrofuran-3-yl}-amid

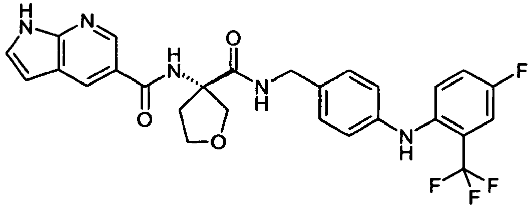

Hergestellt aus den Intermediaten C14 und 1H-Pyrrolo[2,3-b]pyridin-5-carbonsäure nach AAV1.
C₂₇H₂₃F₄N₅O₃ (541.50)
Rₜ = 2.48 Minuten (Methode 2)

### Beispiel 384: 1H-Pyrrolo[2,3-b]pyridin-5-carbonsäure-(1-{[5-(4-fluor-2-trifluormethylphenylamino)-pyridin-2-ylmethyl]-carbamoyl}-cyclopropyl)-amid

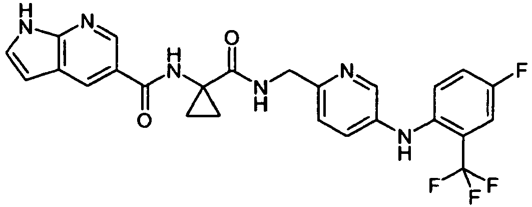

Hergestellt aus den intermediaten C2 und 1H-Pyrrolo[2,3-b]pyridin-5-carbonsäure nach AAV1.
C₂₅H₂₀F₄N₆O₂ (512.47)
Rₜ = 2.27 Minuten (Methode 2)

### Beispiel 385: 2-Cyano-pyrimidin-5-carbonsäure-(1-{[5-(4-chlor-2-trifluormethyl-phenylamino)-pyridin-2-ylmethyl]-carbamoyl}-cyclopropyl)-amid

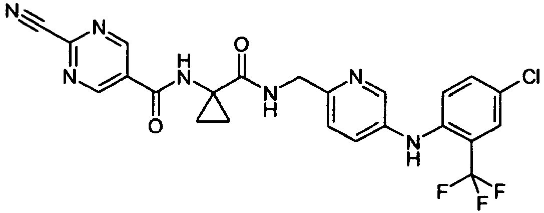

Hergestellt aus den Intermediaten C1 und 2-Cyano-pyrimidin-5-carbonsäure nach AAV1.
C₂₃H₁₇ClF₃N₇O₂ (515.88)
Rₜ = 2.39 Minuten (Methode 2)

### Beispiel 386: 2-Methyl-pyrimidin-5-carbonsäure-(1-{(5-(4-chlor-2-difluormethyl-phenylamino)-pyridin-2-ylmethyl]-carbamoyl}-cyclopropyl)-amid

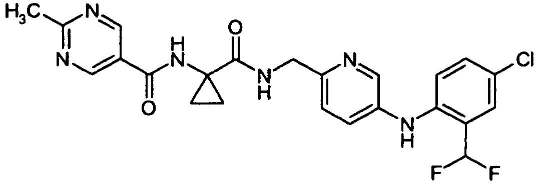

Hergestellt aus den Intermediaten C21 und 2-Methyl-pyrimidin-5-carbonsäure nach AAV1.
C₂₃H₂₁ClF₂N₆O₂ (486.91)
Rₜ = 1.68 Minuten (Methode 2)

### Beispiel 387: (S)-2-Methyl-pyrimidin-5-carbonsäure-(3-{[5-(4-fluor-2-trifluormethyl-phenylamino)-3-methyl-pyridin-2-ylmethyl]-carbamoyl}-tetrahydrofuran-3-yl)-amid

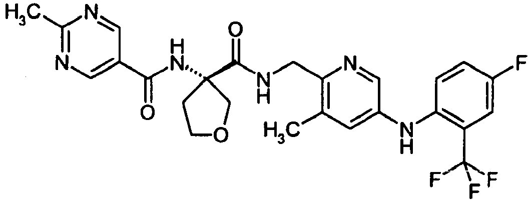

Hergestellt aus den Intermediaten A21 und B7 nach AAV1.
C₂₅H₂₄F₄N₆O₃ (532.50)
Rₜ = 1.86 Minuten (Methode 2)

### Beispiel 388: (S)-1H-Pyrrolo[2,3-b]pyridin-5-carbonsäure-(3-[4-(4-chlor-2-trifluormethylphenylamino)-benzylcarbamoyl]-tetrahydrofuran-3-yl}-amid

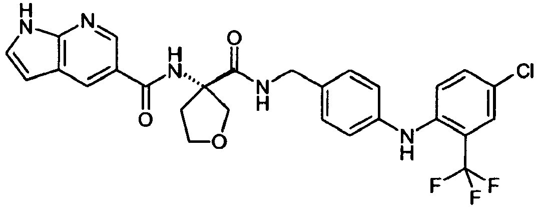

Hergestellt aus den intermediaten C18 und 1H-Pyrrolo[2,3-b]pyridin-5-carbonsäure nach AAV1.
C₂₇H₂₃ClF₃N₅O₃ (557.96)
Massenspektroskopie (ESI): [M+H]+ = 558
[M-H]- = 556

### Beispiel 389: N-(1-{[5-(4-Chlor-2-trifluormethyl-phenylamino)-pyridin-2-ylmethyl]-carbamoyl}-cyclopropyl)-6-cyclopropylamino-nicotinamid

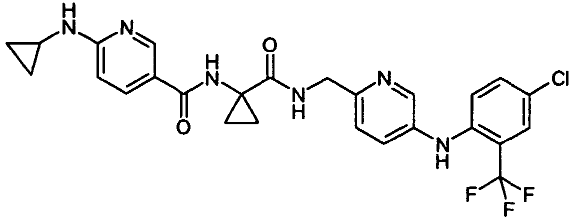

Hergestellt aus den Intermediaten C1 und 6-Cyclopropylamino-nicotinsäure nach AAV1.
C₂₆H₂₄ClF₃N₆O₂ (544.96)
Rₜ = 1.67 Minuten (Methode 2)

### Beispiele 390: 2-Ethoxy-pyrimidin-5-carbonsäure-(1-{[5-(4-chlor-2-trifluormethyl-phenylamino)-pyridin-2-ylmethyl]-carbamoyl}-cyclopropyl)-amid

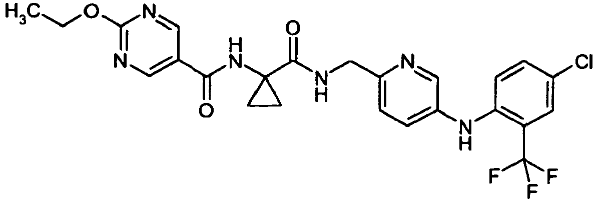

Hergestellt aus den Intermediaten C1 und 2-Ethoxy-pyrimidin-5-carbonsäure nach AAV1.
C₂₄H₂₂ClF₃N₆O₃ (534.92)
Rₜ = 1.99 Minuten (Methode 2)

### Beispiel 391: 6-Amino-N-(1-([5-(4-fluor-2-trifluormethyl-phenylamino)-pyridin-2-ylmethyl]-carbamoyl}-cyclopropyl)-5-methyl-nicotinamid

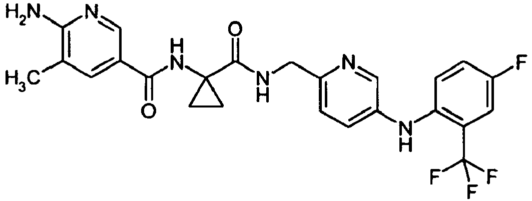

Hergestellt aus den Intermediaten C2 und 6-Amino-5-methyl-nicotinsäure nach AAV1.
C₂₄H₂₂F₄N₆O₂ (502.47)
Rₜ = 1.38 Minuten (Methode 2)

### Beispiel 392: (S)-6-Oxo-1,6-dihydro-pyridazin-4-carbonsäure-{3-[4-(2-trifluormethylphenylamino)-benzylcarbamoyl]-tetrahydrofuran-3-yl}-amid

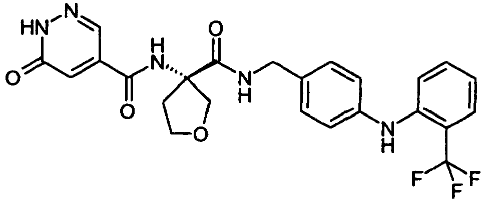

Hergestellt aus den Intermediaten A17 und B10 nach AAV1.
C₂₄H₂₂F₃N₅O₄ (501.46)
Rₜ = 2.09 Minuten (Methode 2)

### Beispiel 393: (S)-6-Oxo-1,6-dihydro-pyridazin-4-carbonsäure-{3-[2-fluor-4-(4-fluor-2-trifluormethyl-phenylamino)-benzylcarbamoyl]-tetrahydrofuran-3-yl}-amid

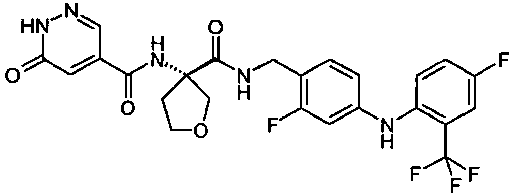

Hergestellt aus den Intermediaten A11 und B10 nach AAV1.
C₂₄H₂₀F₅N₅O₄ (537.44)
Rₜ = 2.15 Minuten (Methode 2)

### Beispiel 394: (S)-6-Oxo-1,6-dihydro-pyridazin-4-carbonsäure-{3-[4-(4-chlor-2-trifluormethyl-phenylamino)-2-fluor-benzylcarbamoyl]-tetrahydrofuran-3-yl}-amid

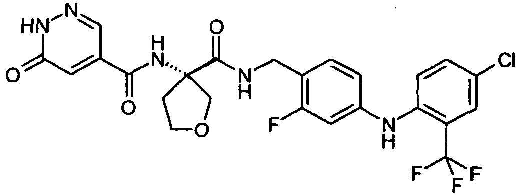

Hergestellt aus den Intermediaten A29 und B10 nach AAV1.
C₂₄H₂₀ClF₄N₅O₄ (553.90)
Rₜ = 2.31 Minuten (Methode 2)

### Beispiel 395: (S)-6-Oxo-1,6-dihydro-pyridazin-4-carbonsäure-{3-[4-(4-brom-2-trifluormethyl-phenylamino)-benzylcarbamoyl]-tetrahydrofuran-3-yl}-amid

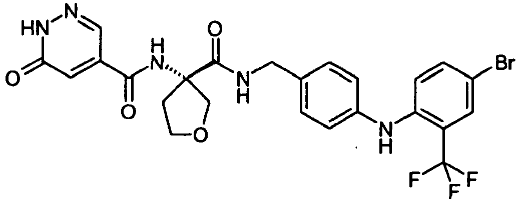

Hergestellt aus den Intermediaten A31 und B10 nach AAV1.
C₂₄H₂₁BrF₃N₅O₄ (580.35)
Rₜ = 2.32 Minuten (Methode 2)

### Beispiel 396: (S)-6-Oxo-1,6-dihydro-pyridazin-4-carbonsäure{3-[2-fluor-4-(2-trifluormethyl-phenylamino)-benzylcarbamoyl]-tetrahydro-furan-3-yl}-amid

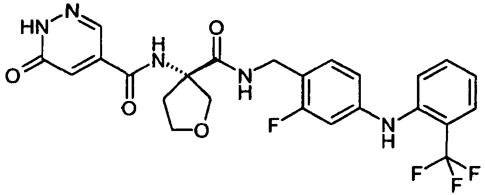

Hergestellt aus den Intermediaten A12 und B10 nach AAV1.
C₂₄H₂₁F₄N₅O₄ (519.45)
Rₜ = 1.35 Minuten (Methode 7)
Massenspektroskopie (ESI): [M+H]⁺ = 520
[M-H]⁻ = 518

Die nachfolgenden Beispiele beschreiben pharmazeutische Darreichungsformen, die als Wirkstoff eine beliebige Verbindung der allgemeinen Formel I enthalten, ohne jedoch den Umfang der vorliegenden Erfindung darauf einzuschränken:

### Beispiel I

Trockenampulle mit 75 mg Wirkstoff pro 10 ml

**Zusammensetzung:**

| | |
|---|---|
| Wirkstoff | 75.0 mg |
| Mannitol | 500 mg |
| Wasser für Injektionszwecke ad | 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

Tablette mit 50 mg Wirkstoff

**Zusammensetzung:**

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 9 mm.

### Beispiel III

Tablette mit 350 mg Wirkstoff

**Zusammensetzung:**

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tablette gepresst, biplan mit beidseitiger Facette und einseitiger Teilkerbe.
Durchmesser der Tabletten: 12 mm.

### Beispiel IV

Kapseln mit 50 mg Wirkstoff

**Zusammensetzung:**

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 3 abgefüllt.

### Beispiel V

Kapseln mit 350 mg Wirkstoff

**Zusammensetzung:**

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Gr6Be 0 abgefüllt.

### Beispiel VI

Suppositorien mit 100 mg Wirkstoff

| 1 Zäpfchen enthält: | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

## Patentansprüche

1. Verbindungen der allgemeinen Formel I 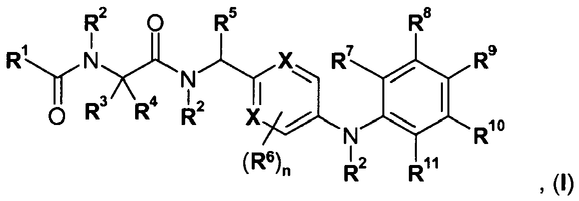 in der
**n** eine der Ziffern 0, 1 oder 2,
**R¹** (a) eine gegebenenfalls mit einem Rest **R^{1.1}** substituierte C₁₋₆-Alkylgruppe,
eine C₁₋₃-Alkylgruppe, in der jede Methylengruppe mit 1 oder 2 Fluoratomen und jede Methylgruppe mit 1, 2 oder 3 Fluoratomen substituiert sein kann,
eine gegebenenfalls mit einem Rest **R^{1.2}** substituierte C₃₋₆-Cycloalkylgruppe, in der eine -CH₂-Einheit durch eine -C(O)-Gruppe ersetzt sein kann,
eine gegebenenfalls mit 1, 2 oder 3 Resten **R^{1.3}** substituierte Aryl-C₀₋₂-alkylengruppe, ein gegebenenfalls mit 1, 2 oder 3 Resten **R^{1.4}** substituierter fünfgliedriger Heteroaryl-C₀₋₂-alkylen-Rest, der mindestens ein N-, O- oder S-Atom enthält und der gegebenenfalls zusätzlich ein, zwei oder drei weitere N-Atome enthält und der zusätzlich benzo-kondensiert sein kann,
ein gegebenenfalls mit 1 oder 2 Resten **R^{1.4}** substituierter sechsgliedriger Heteroaryl-C₀₋₂-alkylen-Rest, der ein, zwei oder drei N-Atome enthält und der zusätzlich benzo-kondensiert sein kann,
ein gegebenenfalls mit 1 oder 2 Resten **R^{1.4}** substituierter neun- oder zehngliedriger Heteroaryl-Rest, der ein, zwei oder drei N-Atome enthält,
ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
-**O**-**R^{1.1.1}**,
**-NR^{1.1.3}R^{1.1.4}** oder
**-C(=NR^{1.5})-CN,**
**R^{1.1}** Halogen, -NO₂, -CN, C₃₋₆-Cycloalkyl, -**OR^{1.1.1}, -SR^{1.1.1}, -C(O)R^{1.1.1}, -**S(O)₂**-R^{1.1.2}, -**O-S(O)₂**-R^{1.1.1}, -**CO₂**R^{1.1.1}, -**O-C(O)**-R^{1.1.1}, -NR^{1.1.3}R^{1.1.4}, -NR^{1.1.3}-**C(O)**-R^{1.1.1}, -NR^{1.1.3}-**C(O)**-R^{1.1.1}, -NR^{1.1.3}-**CO₂**-R^{1.1.1} oder -**C(O)**-NR^{1.1.3}R^{1.1.4},**
**R^{1.1.1}** (a) H,
C₁₋₄-Alkyl,
eine C₁₋₃-Alkylgruppe, in der jede Methylengruppe mit 1 oder 2 Fluoratomen und jede Methylgruppe mit 1, 2 oder 3 Fluoratomen substituiert sein kann,
eine gegebenenfalls mit 1, 2 oder 3 Resten **R^{1.1.1.1}** substituierte Phenylgruppe, C₃₋₆-Cycloalkyl oder
eine gegebenenfalls mit 1, 2 oder 3 Resten **R^{1.1.1.2}** substituierte Pyridylgruppe,
**R^{1.1.1.1}** unabhängig voneinander
Halogen, -NO₂, -CN, -OH, -O-C₁₋₄-Alkyl, C₃₋₆-Cycloalkyl, C₁₋₄-Alkyl oder
eine C₁₋₃-Alkylgruppe, in der jede Methylengruppe mit 1 oder 2 Fluoratomen und jede Methylgruppe mit 1, 2 oder 3 Fluoratomen substituiert sein kann,
**R^{1.1.1.2}** unabhängig voneinander Halogen oder C₁-₄-Alkyl,
**R^{1.1.2}** (a) C₁₋₄-Alky), eine C₁₋₃-Alkylgruppe, in der jede Methylengruppe mit 1 oder 2 Fluoratomen und jede Methylgruppe mit 1, 2 oder 3 Fluoratomen substituiert sein kann, -O-C₁₋₄-Alkyl oder
eine gegebenenfalls mit 1, 2 oder 3 Resten **R^{1.1.1.1}** substituierte Phenylgruppe,
**R^{1.1.3}**,
**R^{1.1.4}** unabhängig voneinander
H,
eine gegebenenfalls mit 1, 2 oder 3 Resten **R^{1.1.4.1}** substituierte C₁₋₄-Alkylgruppe, eine gegebenenfalls mit 1, 2 oder 3 Resten **R^{1.1.1.1}** substituierte Phenylgruppe, C₃₋₆-Cycloalkyl, oder
**R^{1.1.3}** und **R^{1.1.4}** zusammen mit dem N-Atom, an das sie gebunden sind, einen 5- oder 6-gliedrigen heterocyclischen Ring bilden, der zusätzlich ein weiteres Heteroatom ausgewählt aus N, O und S enthalten kann, oder
**R^{1.1.3}** und **R^{1.1.4}** zusammen mit dem N-Atom, an das sie gebunden sind, ein cyclisches Imid bilden,
**R^{1.1.4.1}** unabhängig voneinander Halogen, -NH₂, -NH(C₁₋₄-Alkyl), -N(C₁₋₄-Alkyl)₂ oder **-**SO₂**-R^{1.1.2},**
**R^{1.2}** Halogen, -NO₂, -CN, OH, -O-CH₃ oder Phenyl,
**R^{1.3}** (a) Halogen, -NO₂, -CN, **-**O**R^{1.1.1}, -SR^{1.1.1},** -CO₂**R^{1.1.1}**, C₁₋₆-Alkyl oder
eine C₁₋₃-Alkylgruppe, in der jede Methylengruppe mit 1 oder 2 Fluoratomen und jede Methylgruppe mit 1, 2 oder 3 Fluoratomen substituiert sein kann,
**R^{1.4}** unabhängig voneinander
Halogen, -NO₂, -CN, -O**R^{1.1.1}**, -S**R^{1.1.1}**, -S(O)-**R^{1.1.2}**, -S(O)₂-**R^{1.1.2}**, ₋N**R^{1.1.3}R^{1.1.4}**, -N(**R^{1.4.1}**)-C(O)-C₁₋₄-Alkyl, C₁₋₆-Alkyl,
eine C₁₋₃-Alkylgruppe, in der jede Methylengruppe mit 1 oder 2 Fluoratomen und jede Methylgruppe mit 1, 2 oder 3 Fluoratomen substituiert sein kann, oder
eine Oxo-Gruppe,
**R^{1.4.1}** H oder C₁₋4-Alkyl,
**R^{1.5}** -OH oder -O-C₁₋₃-Alkyl,
**R²** (a) H,
C₁₋₄-Alkyl,
C₁₋₄-Alkyl-C(O)-,
**R³** und **R⁴** zusammen mit dem Kohlenstoffatom, an dem sie gebunden sind, eine gegebenenfalls durch einen Rest **R^{3.1}** substituierte C₃₋₆-Cycloalkylengruppe, in der eine -CH₂-Einheit durch ein Heteroatom O, N, S oder durch eine Gruppe CO, SO oder SO₂ ersetzt sein kann,
**R^{3.1}** H, -OH,
**R⁵** (a) H,
C₁₋₄-Alkyl,
eine C₁₋₃-Alkylgruppe, in der jede Methylengruppe mit 1 oder 2 Fluoratomen und jede Methylgruppe mit 1, 2 oder 3 Fluoratomen substituiert sein kann,
**R⁶** unabhängig voneinander
H, Halogen, -CN, -OH, C₁₋₆-Alkyl, C₃₋₇-Cycloalkyl, -O-C₁₋₄-Alkyl, -O-CF₃, -O-C₃₋₆-Cycloalkyl, -N(C₁₋₃-Alkyl)₂, -C(O)-NH₂, -(SO₂)NH₂, -SO₂-C₁₋₃-Alkyl, oder
eine C₁₋₃-Alkylgruppe, in der jede Methylengruppe mit 1 oder 2 Fluoratomen und jede Methylgruppe mit 1, 2 oder 3 Fluoratomen substituiert sein kann,
**R⁷** (a) H, Halogen, -CN, -OH,
C₁₋₆-Alkyl,
C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
C₃₋₇-Cycloalkyl,
-O-C₁₋₆-Alkyl,
-O-C₃₋₇-Cycloalkyl,
-NH₂, -NH(C₁₋₃-Alkyl), -N(C₁₋₃-Alkyl)₂,
-C(O)-**R^{7.1}**,
-S-C₁₋₄-Alkyl, -SO₂-**R^{7.2}**,
eine gegebenenfalls mit einer oder zwei C₁₋₃-Alkylgruppen substituierte fünfgliedrige Heteroarylgruppe, die ausgewählt ist aus der Gruppe bestehend aus Pyrrolyl, Oxazolyl, Isoxazolyl, Oxadiazolyl, Thiazolyl, Isothiazolyl, Thiadiazolyl, Imidazolyl, Pyrazolyl, Triazolyl und Tetrazolyl, oder
eine gegebenenfalls mit einer oder zwei C₁₋₃-Alkylgruppen substituierte sechsgliedrige Heteroarylgruppe, die ausgewählt ist aus der Gruppe bestehend aus Pyridyl, Pyrimidinyl, Pyrazinyl, Pyridazinyl und Triazinyl,
**R^{7.1}** -NH₂, -NH(C₁₋₆-Alkyl),-N(C₁₋₆-Alkyl)₂, *N*-Acetidinyl, *N*-Pyrrolidinyl, *N*-Piperidinyl, *N*-Morpholinyl, -OH, -O-C₁₋₈-Alkyl oder -O-C₃₋₇-Cycloalkyl,
**R^{7.2}** -NH₂, -NH(C₁₋₆-Alkyl),-N(C₁₋₆-Alkyl)₂, *N*-Acetidinyl, *N*-Pyrrolidinyl, *N*-Piperidinyl oder *N*-Morpholinyl und
**R⁸** H, Halogen, C₁₋₄-Alkyl,
**R⁹** (a) H, Halogen, -CN, -OH,
C₁₋₆-Alkyl,
C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
C₃₋₇-Cycloalkyl,
C₂₋₄-Alkinyl,
-O-C₁₋₆-Alkyl,
-O-C₃₋₇-Cycloalkyl,
-NH₂, -NH(C₁₋₃-Alkyl), -N(C₁₋₃-Alkyl)₂,
-C(O)-**R^{9.1}**,
-S-C₁₋₄-Alkyl, -SO-C₁₋₄-Alkyl, -SO₂-C₁₋₄-Alkyl,
**R^{9.1}** -NH₂, -NH(C₁₋₆-Alkyl),-N(C₁₋₆-Alkyl)₂, *N*-Acetidinyl, *N*-Pyrrolidinyl, *N*-Piperidinyl, ***N***-Morpholinyl, -OH, -O-C₁₋₈-Alkyl oder -O-C₃₋₇-Cycloalkyl,
**R¹⁰** H, Halogen, C₁₋₄-Alkyl,
**R¹¹** (a) H, Halogen, -CN, -OH,
C₁₋₆-Alkyl,
C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
C₃₋₇-Cycloalkyl,
-O-C₁₋₆-Alkyl,
-O-C₃₋₇-Cycloalkyl,
-NH₂, -NH(C₁₋₃-Alkyl), -N(C₁₋₃-Alkyl)₂,
-C(O)-**R^{11.1}**,
-S-C₁₋₃-Alkyl, -SO₂-**R^{11.2}**,
eine gegebenenfalls mit einer oder zwei C₁₋₃-Alkylgruppen substituierte fünfgliedrige Heteroarylgruppe, die ausgewählt ist aus der Gruppe bestehend aus Pyrrolyl, Oxazolyl, Isoxazolyl, Oxadiazolyl, Thiazolyl, Isothiazolyl, Thiadiazolyl, Imidazolyl, Pyrazolyl, Triazolyl und Tetrazolyl, oder
eine gegebenenfalls mit einer oder zwei C₁₋₃-Alkylgruppen substituierte sechsgliedrige Heteroarylgruppe, die ausgewählt ist aus der Gruppe bestehend aus Pyridyl, Pyrimidinyl, Pyrazinyl, Pyridazinyl und Triazinyl,
**R^{11.1}** -NH₂, -NH(C₁₋₆-Alkyl),-N(C₁₋₆-Alkyl)₂, N-Acetidinyl, N-Pyrrolidinyl, N-Piperidinyl, N-Morpholinyl, -OH, -O-C₁₋₈-Alkyl oder -O-C₃₋₇-Cycloalkyl,
**R^{11.2}** -NH₂, -NH(C₁₋₆-Alkyl),-N(C₁₋₆-Alkyl)₂, *N*-Acetidinyl, *N*-Pyrrolidinyl, *N*-Piperidinyl oder *N*-Morpholinyl und
**X** unabhängig voneinander C-**R⁶** oder N, bedeuten,
deren Enantiomere, deren Diastereomere, deren Gemische und deren Salze.

2. Verbindungen der allgemeinen Formel **I** gemäß Anspruch 1, in denen **R², R³, R⁴, R⁵, R⁶, R⁷, R⁸, R⁹, R¹⁰, R¹¹,** n und **X** wie in Anspruch 1 definiert sind und
**R¹** (a) eine gegebenenfalls mit einem Rest **R^{1.1}** substituierte C₁₋₆-Alkylgruppe,
eine C₁₋₃-Alkylgruppe, in der jede Methylengruppe mit 1 oder 2 Fluoratomen und jede Methylgruppe mit 1, 2 oder 3 Fluoratomen substituiert sein kann,
eine gegebenenfalls mit einem Rest **R^{1.2}** substituierte C₃₋₆-Cycloalkylgruppe, in der eine -CH₂-Einheit durch eine -C(O)-Gruppe ersetzt sein kann,
eine gegebenenfalls mit 1, 2 oder 3 Resten **R^{1.3}** substituierte Phenylgruppe,
ein gegebenenfalls mit 1, 2 oder 3 Resten **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält,
ein gegebenenfalls mit 1 oder 2 Resten **R^{1.4}** substituierter sechsgliedriger Heteroaryl-Rest, der ein, zwei oder drei N-Atome enthält,
ein gegebenenfalls mit 1 oder 2 Resten **R^{1.4}** substituierter neun- oder zehngliedriger Heteroaryl-Rest, der ein, zwei oder drei N-Atome enthält,
ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
-O-**R^{1.1.1}** oder
-N**R^{1.1.3}R^{1.1.4}**,
**R¹.¹** -CN, C₃₋₆-Cycloalkyl, -O**R^{1.1.1}, -NR^{1.1.3}R**^{**1**.}**^{1.4},**
**R^{1.1.1}** (a) H,
C₁₋₄-Alkyl,
eine C₁₋₃-Alkylgruppe, in der jede Methylengruppe mit 1 oder 2 Fluoratomen und jede Methylgruppe mit 1, 2 oder 3 Fluoratomen substituiert sein kann,
**R^{1.1.3},**
**R^{1.1.4}** unabhängig voneinander
H,
C₁₋₄-Alkyl,
C₃₋₆-Cycloalkyl, oder
**R^{1.1.3}** und **R^{1.1.4}** zusammen mit dem N-Atom, an das sie gebunden sind, einen 5- oder 6-gliedrigen heterocyclischen Ring bilden, der zusätzlich ein weiteres Heteroatom ausgewählt aus N, O und S enthalten kann, oder
**R^{1.2}** Halogen, -NO₂, -CN, -OH, -O-CH₃ oder Phenyl,
**R^{1.3}** unabhängig voneinander
Halogen, -NO₂, -CN, -O**R^{1.1.1}**, C₁₋₆-Alkyl oder
eine C₁₋₃-Alkylgruppe, in der jede Methylengruppe mit 1 oder 2 Fluoratomen und jede Methylgruppe mit 1, 2 oder 3 Fluoratomen substituiert sein kann,
**R^{1.4}** unabhängig voneinander
Halogen, -NO₂, -CN, -O**R^{1.1.1}**, -N**R^{1.1.3}R^{1.1.4}**, -N(**R^{1.4.1}**)-C(O)-C₁₋₄-Alkyl, C₁₋₆-Alkyl, oder
eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4.1}** H oder C₁₋₄-Alkyl bedeuten,
deren Enantiomere, deren Diastereomere, deren Gemische und deren Salze.

3. Verbindungen der allgemeinen Formel **I** gemäß Anspruch 1, in denen **R², R³, R⁴, R⁵, R⁶, R⁷, R⁸, R⁹, R¹⁰, R¹¹**, n und **X** wie in Anspruch 1 definiert sind und
**R¹** (a) eine gegebenenfalls mit einem Rest **R^{1.1}** substituierte C₁₋₆-Alkylgruppe,
eine gegebenenfalls mit 1, 2 oder 3 Resten **R^{1.3}** substituierte Phenylgruppe,
ein gegebenenfalls mit 1, 2 oder 3 Resten **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält,
ein gegebenenfalls mit 1 oder 2 Resten **R^{1.4}** substituierter sechsgliedriger Heteroaryl-Rest, der ein, zwei oder drei N-Atome enthält,
ein gegebenenfalls mit 1 oder 2 Resten **R^{1.4}** substituierter neun- oder zehngliedriger Heteroaryl-Rest, der ein, zwei oder drei N-Atome enthält,
ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
**R^{1.1}** -CN, C₃₋₆-Cycloalkyl, -OH, -OCH₃, -NH₂, -NHCH₃, -N(CH₃)₂,
**R^{1.3}** unabhängig voneinander
F, Cl, Br, -OH, -OCH₃, C₁₋₆-Alkyl oder
eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
F, Cl, Br, -OH, -OCH₃, -NH₂, -NHCH₃, -N(CH₃)₂, -NHC₂₋₃-Alkyl, -N(C₂₋₃-Alkyl)₂, -NH-C(O)-C₁₋₄-Alkyl, C₁₋₆-Alkyl, oder
eine C₁₋₃-Alkylgruppe, in der jede Methylengruppe mit 1 oder 2 Fluoratomen und jede Methylgruppe mit 1, 2 oder 3 Fluoratomen substituiert sein kann, bedeuten,
deren Enantiomere, deren Diastereomere, deren Gemische und deren Salze, insbesondere deren physiologisch verträgliche Salze mit organischen oder anorganischen Säuren oder Basen.

4. Verbindungen der allgemeinen Formel **I** gemäß Anspruch 1, in denen **R², R³, R⁴, R⁵, R⁶, R⁷, R⁸, R⁹, R¹⁰, R¹¹**, n und **X** wie in Anspruch 1 definiert sind und
**R¹** (a) eine gegebenenfalls mit einem Rest **R^{1.1}** substituierte C₁₋₆-Alkylgruppe,
eine gegebenenfalls mit 1, 2 oder 3 Resten **R^{1.3}** substituierte Phenylgruppe,
ein gegebenenfalls mit 1, 2 oder 3 Resten **R^{1.4}** substituierter fünfgliedriger Heteroaryl-Rest, der ausgewählt ist aus der Gruppe bestehend aus 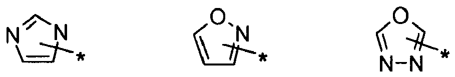 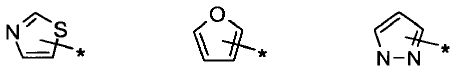 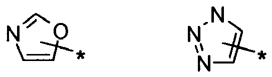 ein gegebenenfalls mit 1 oder 2 Resten **R^{1.4}** substituierter sechsgliedriger Heteroaryl-Rest, der ausgewählt ist aus der Gruppe bestehend aus 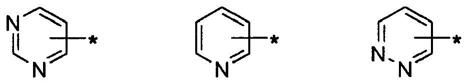 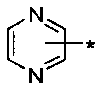 ein gegebenenfalls mit 1 oder 2 Resten **R^{1.4}** substituierter neungliedriger Heteroaryl-Rest, der ausgewählt ist aus der Gruppe bestehend aus 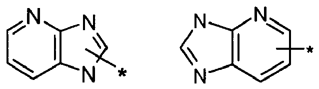 ein gegebenenfalls mit 1 oder 2 Resten **R**^{**1.**4} substituierter 5- oder 6-gliedriger
Heterocyclus, der ausgewählt ist aus der Gruppe bestehend aus 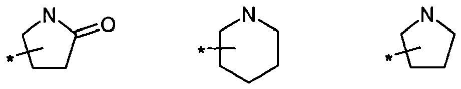 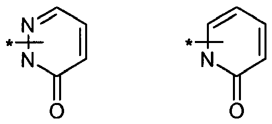 **R^{1.1}** -CN, Cyclopropyl, -OH, -OCH₃, -NH₂, -NHCH₃, -N(CH₃)₂,
**R^{1.3}** unabhängig voneinander
F, Cl, Br, -OH, -OCH₃, -OCF₃, C₁₋₄-Alkyl oder
eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
F, Cl, Br, -OH, -OCH₃, -OCF₃, -NH₂, -NH-C₁₋₄-Alkyl, -N(C₁₋₄-Alkyl)₂, -NH-C(O)-C₁₋₄-Alkyl, C₁₋₆-Alkyl, oder
eine C₁₋₃-Alkylgruppe, in der jede Methylengruppe mit 1 oder 2 Fluoratomen und jede Methylgruppe mit 1, 2 oder 3 Fluoratomen substituiert sein kann, bedeuten,
deren Enantiomere, deren Diastereomere, deren Gemische und deren Salze.

5. Verbindungen der allgemeinen Formel **I** gemäß Anspruch 1, in denen **R², R³, R⁴, R⁵, R⁶, R⁷, R⁸, R⁹, R¹⁰, R¹¹, n** und **X** wie in Anspruch 1 definiert sind und
**R¹** ausgewählt ist aus der Gruppe bestehend aus 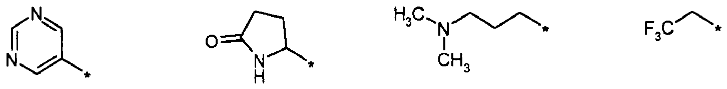 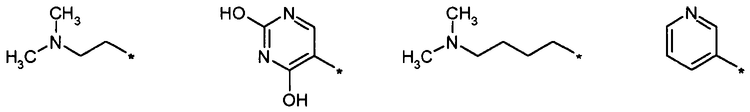 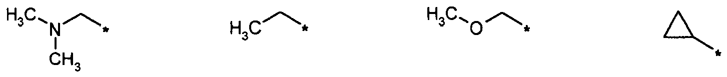 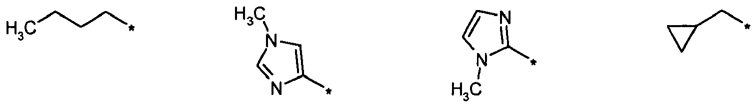 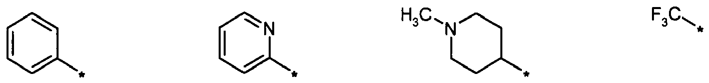 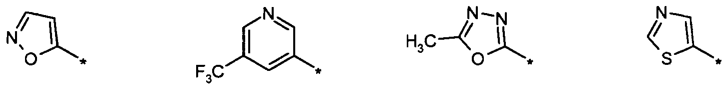 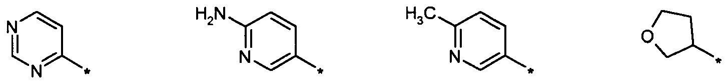 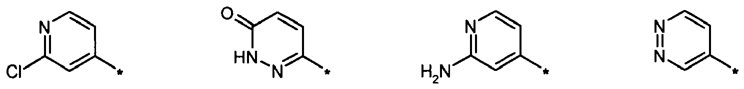 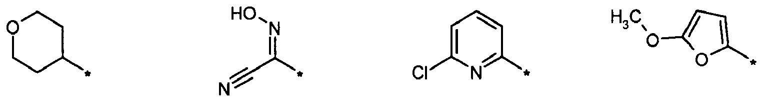 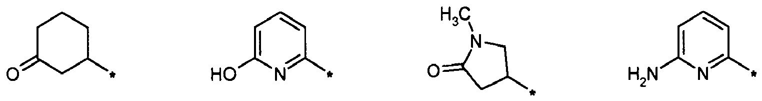 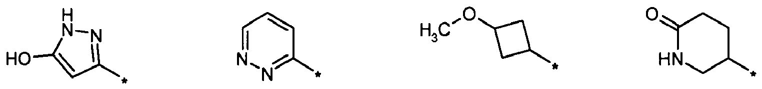 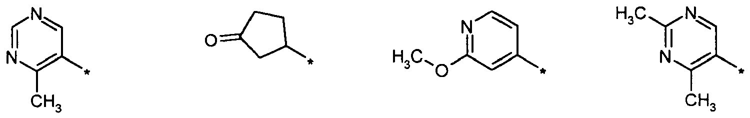 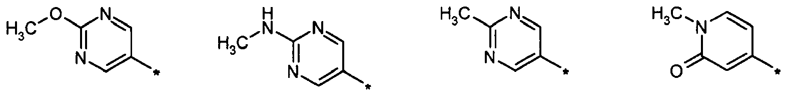 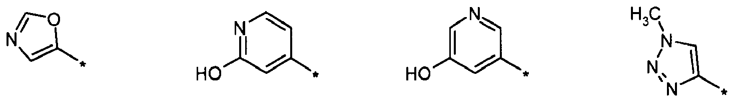 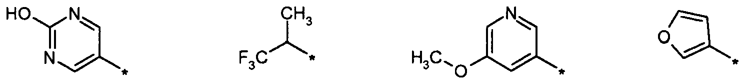 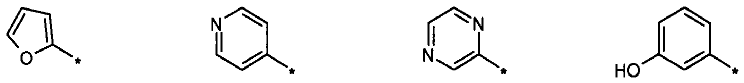 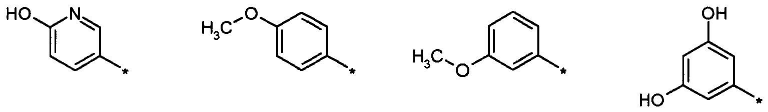 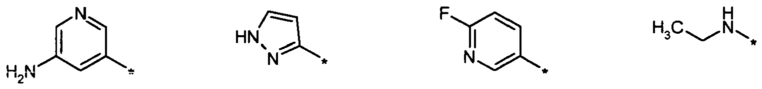 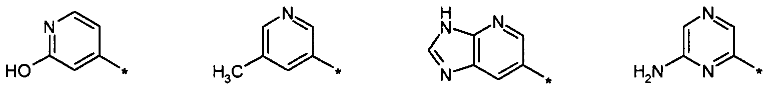 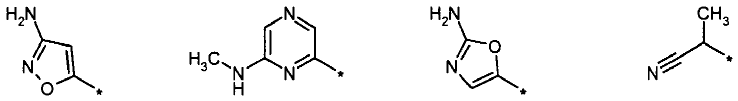 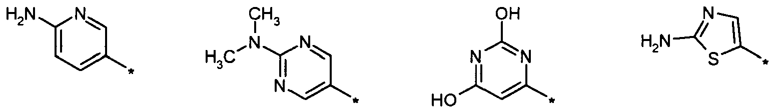 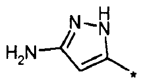 deren Enantiomere, deren Diastereomere, deren Gemische und deren Salze.

6. Verbindungen der allgemeinen Formel **I** gemäß Anspruch 1, in denen
**R², R³, R⁴, R⁵, R⁶, R⁷, R⁸, R⁹, R¹⁰, R¹¹, n** und **X** wie in Anspruch 1 erwähnt definiert sind und
**R¹** ausgewählt ist aus der Gruppe bestehend aus 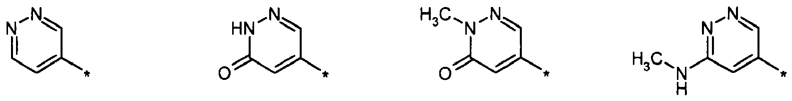 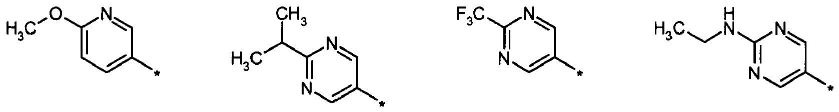 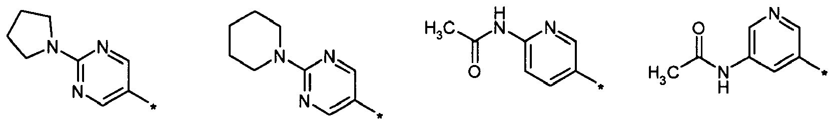 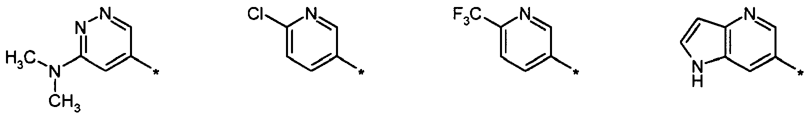 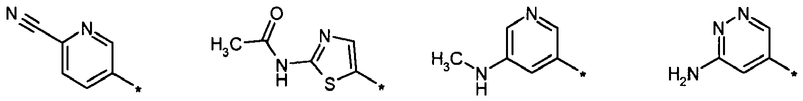 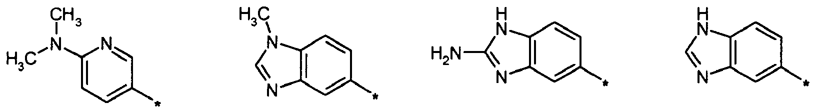 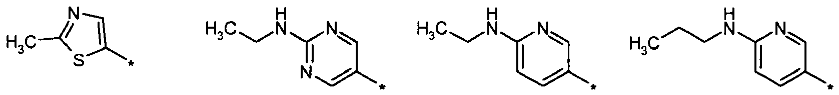 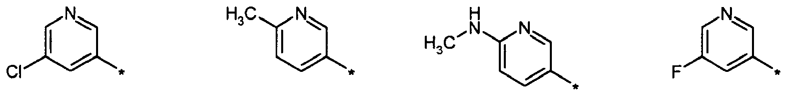 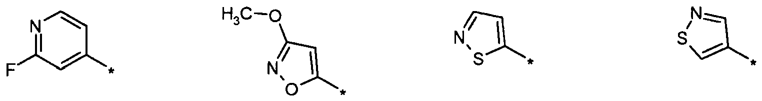 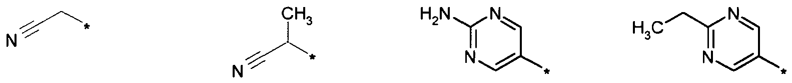 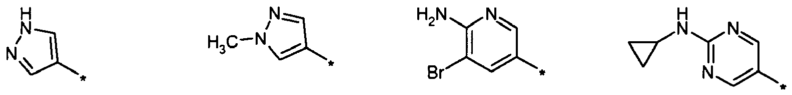 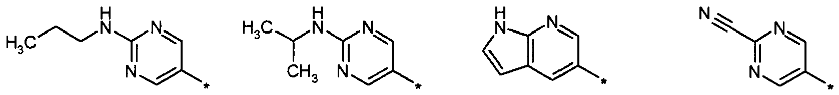 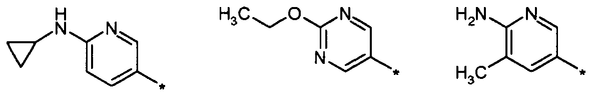 deren Enantiomere, deren Diastereomere, deren Gemische und deren Salze, insbesondere deren physiologisch verträgliche Salze mit organischen oder anorganischen Säuren oder Basen.

7. Verbindungen der allgemeinen Formel **I g**emäß Anspruch 1, in denen **R¹** wie in Anspruch 1, 2, 3, 4, 5 oder 6 definiert ist und
**n** eine der Ziffern 0, 1 oder 2,
**R²** (a) H,
C₁₋₄-Alkyl,
**R**³ und **R⁴** zusammen mit dem Kohlenstoffatom, an dem sie gebunden sind, eine gegebenenfalls durch einen Rest **R^{3.1}** substituierte C₃₋₆-Cycloalkylengruppe, in der eine -CH₂-Einheit durch ein Heteroatom O, N, S oder durch eine Gruppe CO, SO oder SO₂ ersetzt sein kann,
**R^{3.1}** H, -OH,
**R⁵** (a) H,
C₁₋₄-Alkyl,
eine C₁₋₃-Alkylgruppe, in der jede Methylengruppe mit 1 oder 2 Fluoratomen und jede Methylgruppe mit 1, 2 oder 3 Fluoratomen substituiert sein kann,
**R⁶** unabhängig voneinander
H, Halogen, -CN, -OH, C₁₋₆-Alkyl, C₃₋₇-Cycloalkyl, -O-C₁₋₄-Alkyl, -O-CF₃, -O-C₃₋₆-Cycloalkyl, -N(C₁₋₃-Alkyl)₂, -C(O)-NH₂, -(SO₂)NH₂, -SO₂-C₁₋₃-Alkyl, oder
eine C₁₋₃-Alkylgruppe, in der jede Methylengruppe mit 1 oder 2 Fluoratomen und jede Methylgruppe mit 1, 2 oder 3 Fluoratomen substituiert sein kann,
**R⁷** (a) H, Halogen, -CN, -OH,
C₁₋₆-Alkyl,
C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
C₃₋₇-Cycloalkyl,
-O-C₁₋₆-Alkyl,
-O-C₃₋₇-Cycloalkyl,
-NH₂, -NH(C₁₋₃-Alkyl), -N(C₁₋₃-Alkyl)₂,
-C(O)-**R^{7.1}**,
-S-C₁₋₄-Alkyl,
**R^{7.1}** -NH₂, , -OH, -O-C₁₋₈-Alkyl,
**R⁸** H, Halogen, C₁₋₄-Alkyl,
**''edR⁹** (a) H, Halogen, -CN, -OH,
C₁₋₆-Alkyl,
C₁₋₃-Alkyl oder -O-C₁₋₃-Alkyl, in der jede Methylengruppe mit 1 oder 2 Fluoratomen und jede Methylgruppe mit 1, 2 oder 3 Fluoratomen substituiert sein kann,
C₃₋₇-Cycloalkyl,
C₂₋₄-Alkinyl,
-O-C₁₋₆-Alkyl,
-O-C₃₋₄-Cycloalkyl,
-NH₂, -NH(C₁₋₃-Alkyl), -N(C₁₋₃-Alkyl)₂,
-C(O)-**R^{9.1}**,
-S-C₁₋₄-Alkyl, -SO-C₁₋₄-Alkyl, -SO₂-C₁₋₄-Alkyl,
**R^{9.1}** -NH₂, -OH, -O-C₁₋₈-Alkyl,
**R¹⁰** H, Halogen, C₁₋₄-Alkyl,
**R¹¹** (a) H, Halogen, -CN, -OH,
C₁₋₆-Alkyl,
**C₁₋₃-Alkyl** oder -O-C₁₋₃-Alkyl, in der jede Methylengruppe mit 1 oder 2 Fluoratomen und jede Methylgruppe mit 1, 2 oder 3 Fluoratomen substituiert sein kann,
C₃₋₇-Cycloalkyl,
-O-C₁₋₆-Alkyl,
-O-C₃₋₇-Cycloalkyl,
-NH₂, -NH(C₁₋₃-Alkyl), -N(C₁₋₃-Alkyl)₂,
-C(O)-**R^{11.1}**,
-S-C₁₋₃-Alkyl,
**R^{11.1}** -NH₂, , -OH, -O-C₁₋₈-Alkyl, und
**X** unabhängig voneinander C-**R⁶** oder N, bedeuten,
deren Enantiomere, deren Diastereomere, deren Gemische und deren Salze.

8. Verbindungen der allgemeinen Formel **I** gemäß Anspruch 1, in denen **R¹, R³, R⁴, R⁵, R⁶, R⁷, R⁸, R⁹, R¹⁰, R¹¹, n** und **X** wie in Anspruch 1, 2, 3, 4, 5, 6 oder 7 definiert sind und
**R²** H oder CH₃ bedeutet,
deren Enantiomere, deren Diastereomere, deren Gemische und deren Salze.

9. Verbindungen der allgemeinen Formel **I** gemäß Anspruch 1, in denen **R¹, R³, R⁴, R⁵, R⁶, R⁷, R⁸, R⁹, R¹⁰, R¹¹, n** und **X** wie in Anspruch 1, 2, 3, 4, 5, 6 oder 7 definiert sind und
**R²** H bedeutet,
deren Enantiomere, deren Diastereomere, deren Gemische und deren Salze.

10. Verbindungen der allgemeinen Formel **I** gemäß Anspruch 1, in denen **R¹, R², R⁵, R⁶ , R⁷, R⁸, R⁹, R¹⁰, R¹¹, n** und **X** wie in Anspruch 1, 2, 3, 4, 5, 6, 7, 8 oder 9 definiert sind und
**R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann, bedeuten,
deren Enantiomere, deren Diastereomere, deren Gemische und deren Salze.

11. Verbindungen der allgemeinen Formel **I** gemäß Anspruch 1, in denen **R¹, R², R⁵, R⁶, R⁷, R⁸, R⁹, R¹⁰, R¹¹, n** und **X** wie in Anspruch 1, 2, 3, 4, 5, 6, 7, 8 oder 9 definiert sind und
**R³** und **R⁴** zusammen mit dem Kohlenstoffatom, an dem sie gebunden sind, eine Gruppe ausgewählt aus 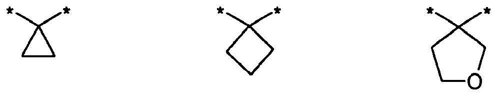 bedeuten,
deren Enantiomere, deren Diastereomere, deren Gemische und deren Salze.

12. Verbindungen der allgemeinen Formel **I** gemäß Anspruch 1, in denen **R¹, R², R³, R⁴, R⁶, R⁷, R⁸, R⁹, R¹⁰, R¹¹, n** und **X** wie in Anspruch 1, 2, 3, 4, 5, 6, 7, 8, 9, 10 oder 11 definiert sind und
**R⁵** H oder CH₃ bedeutet,
deren Enantiomere, deren Diastereomere, deren Gemische und deren Salze.

13. Verbindungen der allgemeinen Formel **I** gemäß Anspruch 1, in denen **R¹, R², R³, R⁴, R⁵, R⁷, R⁸, R⁹, R¹⁰, R¹¹, n** und **X** wie in Anspruch 1, 2, 3, 4, 5, 6, 7, 8, 9, 10, 11 oder 12 definiert sind und
**R⁶** H, F, Cl oder Methyl bedeutet,
deren Enantiomere, deren Diastereomere, deren Gemische und deren Salze.

14. Verbindungen der allgemeinen Formel **I** gemäß Anspruch 1, in denen **R¹, R², R³, R⁴, R⁵, R⁶, n** und **X** wie in Anspruch 1, 2, 3, 4, 5, 6, 7, 8, 9, 10, 11, 12 oder 13 definiert sind und
**R⁷** H, F, Cl, Br, -CN, C₁₋₄-Alkyl, CF₃, CHF₂,
**R⁸** H,
**R⁹** F, Cl, Br, C₁₋₄-Alkyl, -O-C₁₋₄-Alkyl, -S-C₁₋₄-Alkyl,
**R¹⁰** H und
**R¹¹** F, Cl, Br, -CN, C₁₋₄-Alkyl, CF₃ oder CHF₂ bedeuten,
deren Enantiomere, deren Diastereomere, deren Gemische und deren Salze.

15. Verbindungen der allgemeinen Formel **Ia**, 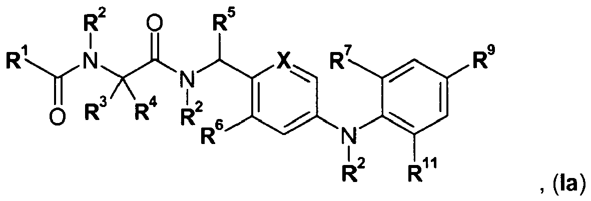 in der
**R¹** (a) eine gegebenenfalls mit einem Rest **R^{1.1}** substituierte C₁₋₆-Alkylgruppe,
eine gegebenenfalls mit 1, 2 oder 3 Resten **R^{1.3}** substituierte Phenylgruppe,
ein gegebenenfalls mit 1, 2 oder 3 Resten **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält,
ein gegebenenfalls mit 1 oder 2 Resten R^{1.4} substituierter sechsgliedriger Heteroaryl-Rest, der ein, zwei oder drei N-Atome enthält,
ein gegebenenfalls mit 1 oder 2 Resten R^{1.4} substituierter neun- oder zehngliedriger Heteroaryl-Rest, der ein, zwei oder drei N-Atome enthält,
ein gegebenenfalls mit 1 oder 2 Resten R^{1.4} substituierter 5- oder 6-gliedriger Heterocyclus, in dem eine -CH₂-Einheit durch eine -C(O)-Gruppe ersetzt sein kann,
R^{1.1} -CN, C₃₋₆-Cycloalkyl, -OH, -OCH₃, -NH₂, -NHCH₃, -N(CH₃)₂,
R^{1.3} unabhängig voneinander
F, Cl, Br, -OH, -OCH₃, C₁₋₆-Alkyl oder
eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
F, Cl, Br, -OH, -OCH₃, -NH₂, -NHCH₃, -N(CH₃)₂, -NH-C(O)-C₁₋₄-Alkyl, C₁₋₆-Alkyl, oder eine C₁₋₃-Alkylgruppe, in der jede Methylengruppe mit 1 oder 2 Fluoratomen und jede Methylgruppe mit 1, 2 oder 3 Fluoratomen substituiert sein kann,
**R²** H oder CH₃,
**R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann, bedeutet,
**R⁵** H oder C₁₋₄-Alkyl,
**R⁶** H, F, Cl, Br oder C₁₋₄-Alkyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze.

16. Verbindungen der allgemeinen Formel Ia gemäß Anspruch 14, in denen
**R¹** (a) eine gegebenenfalls mit einem Rest **R^{1.1}** substituierte C₁₋₆-Alkylgruppe,
eine gegebenenfalls mit 1, 2 oder 3 Resten **R^{1.3}** substituierte Phenylgruppe,
ein gegebenenfalls mit 1, 2 oder 3 Resten **R^{1.4}** substituierter fünfgliedriger Heteroaryl-Rest, der ausgewählt ist aus der Gruppe bestehend aus 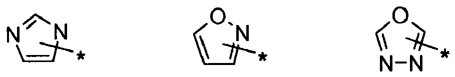 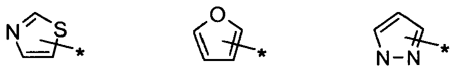 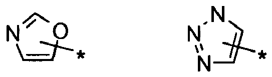 ein gegebenenfalls mit 1 oder 2 Resten **R^{1.4}** substituierter sechsgliedriger Heteroaryl-Rest, der ausgewählt ist aus der Gruppe bestehend aus 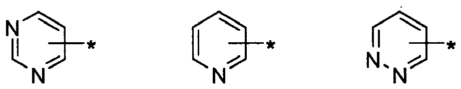 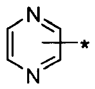 ein gegebenenfalls mit 1 oder 2 Resten **R^{1.4}** substituierter neungliedriger Heteroaryl-Rest, der ausgewählt ist aus der Gruppe bestehend aus 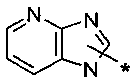 ein gegebenenfalls mit 1 oder 2 Resten **R^{1.4}** substituierter 5- oder 6-gliedriger Heterocyclus, der ausgewählt ist aus der Gruppe bestehend aus 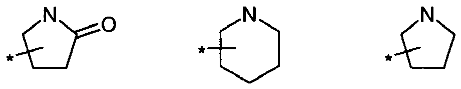 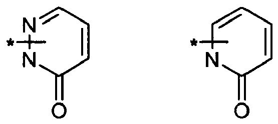 **R^{1.1}** -CN, Cyclopropyl, -OH, -OCH₃, -NH₂, -NHCH₃, -N(CH₃)₂,
**R^{1.3}** unabhängig voneinander
F, Cl, Br, -OH, -OCH₃, -OCF₃, C₁₋₄-Alkyl oder
eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
F, Cl, Br, -OH, -OCH₃, -OCF₃, -NH₂, -NH-C₁₋₄-Alkyl, -N(C₁₋₄-Alkyl)₂,
-NH-C(O)-C₁₋₄-Alkyl, C₁₋₆-Alkyl, oder
eine C₁₋₃-Alkylgruppe, in der jede Methylengruppe mit 1 oder 2 Fluoratomen und jede Methylgruppe mit 1, 2 oder 3 Fluoratomen substituiert sein kann,
**R²** H oder CH₃,
**R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann, bedeutet,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze.

17. Verbindungen der allgemeinen Formel Ia gemäß Anspruch 15, in denen
**R¹** eine Gruppe ausgewählt aus 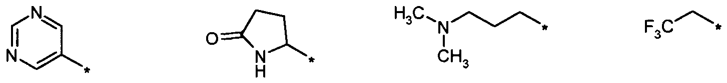 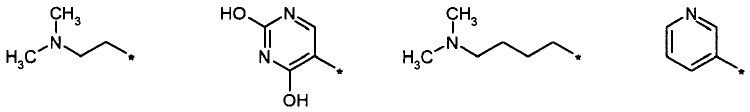 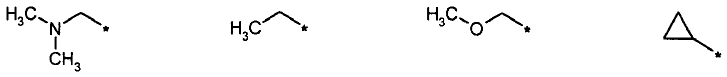 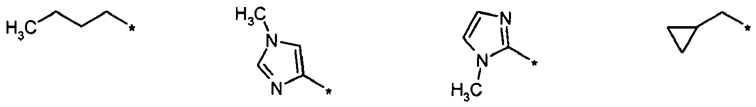 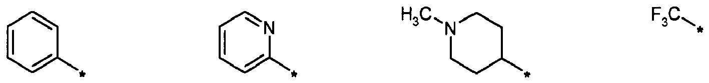 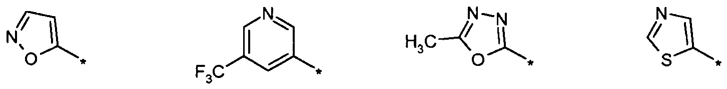 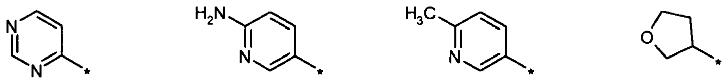 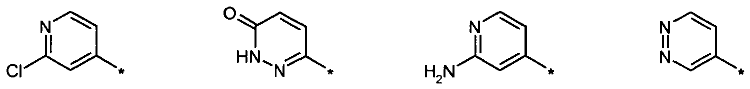 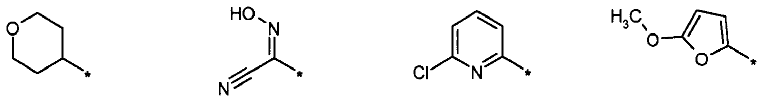 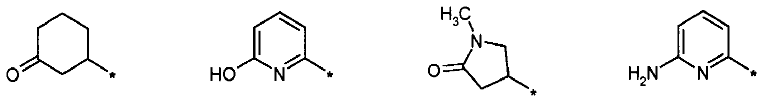 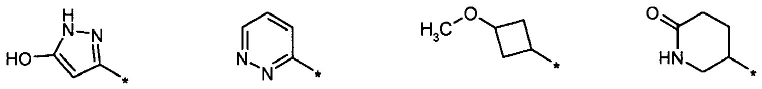 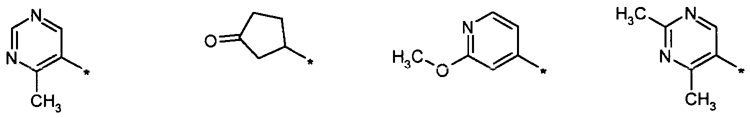 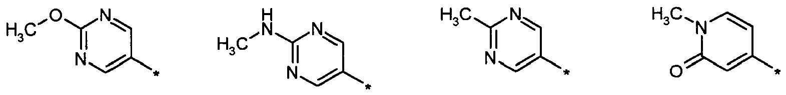 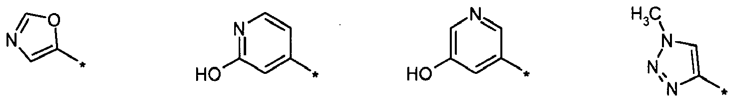 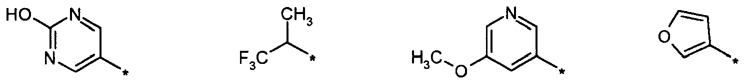 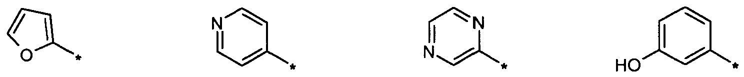 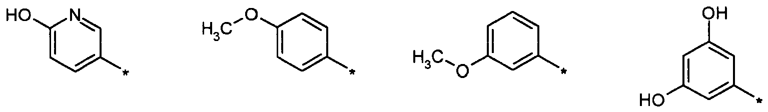 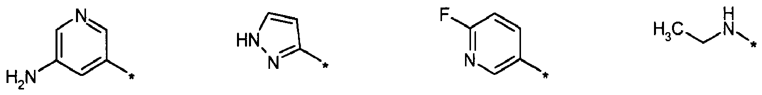 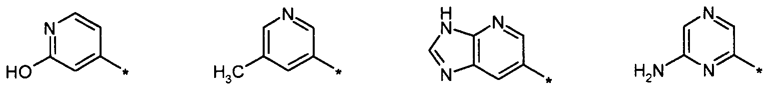 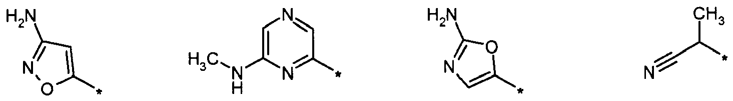 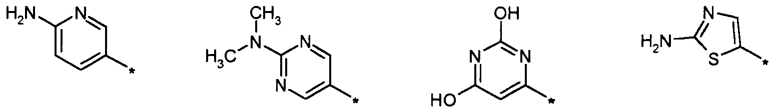 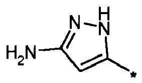 **R²** H oder CH₃,
**R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze.

18. Verbindungen der allgemeinen Formel Ia gemäß Anspruch 15, in denen
**R¹** eine Gruppe ausgewählt aus 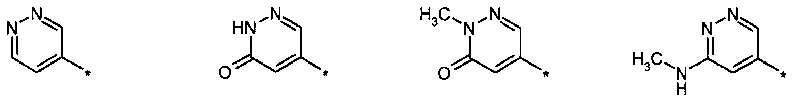 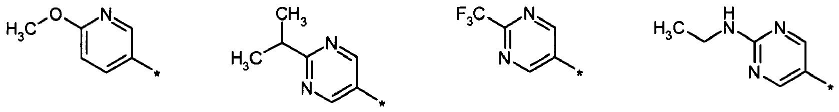 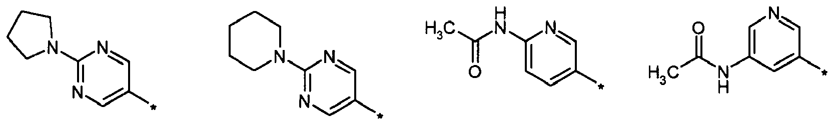 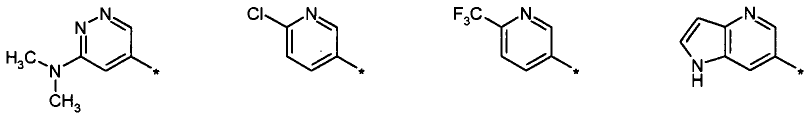 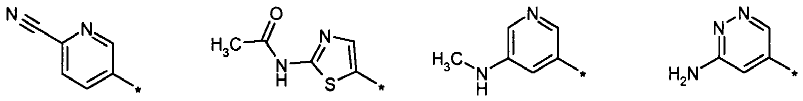 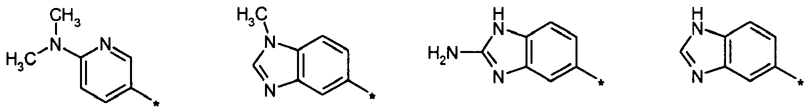 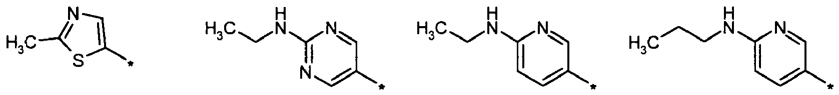 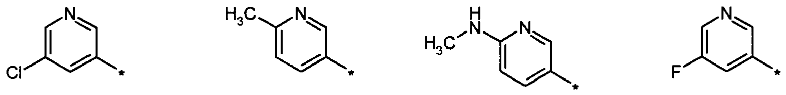 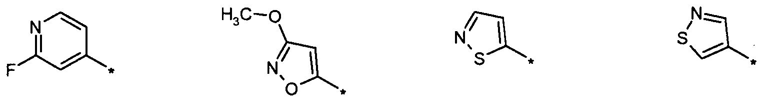 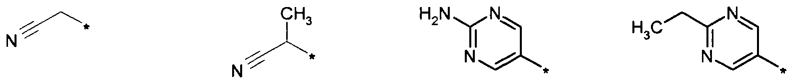 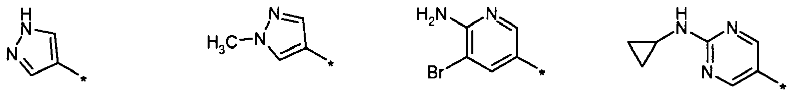 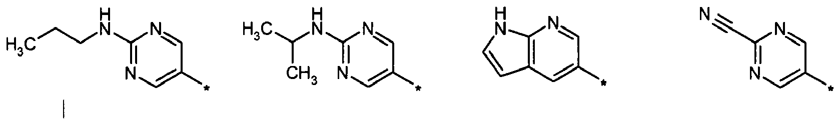 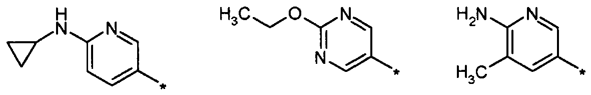 **R²** H oder CH₃,
**R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann, bedeutet,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

19. Verbindungen der allgemeinen Formel Ib, 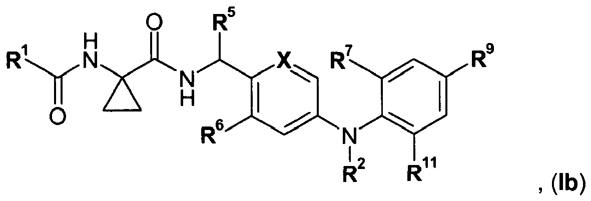 in der
**R¹** (a) eine gegebenenfalls mit einem Rest **R^{1.1}** substituierte C₁₋₆-Alkylgruppe,
eine gegebenenfalls mit 1, 2 oder 3 Resten **R^{1.3}** substituierte Phenylgruppe,
ein gegebenenfalls mit 1, 2 oder 3 Resten **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält,
ein gegebenenfalls mit 1 oder 2 Resten **R^{1.4}** substituierter sechsgliedriger Heteroaryl-Rest, der ein, zwei oder drei N-Atome enthält,
ein gegebenenfalls mit 1 oder 2 Resten **R^{1.4}** substituierter neun- oder zehngliedriger Heteroaryl-Rest, der ein, zwei oder drei N-Atome enthält,
ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
**R^{1.1}** -CN, C₃₋₆-Cycloalkyl, -OH, -OCH₃, -NH₂, -NHCH₃, -N(CH₃)₂,
**R^{1.3}** unabhängig voneinander
F, Cl, Br, -OH, -OCH₃, C₁₋₆-Alkyl oder
eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
F, Cl, Br, -OH, -OCH₃, -NH₂, -NHCH₃, -N(CH₃)₂, -NH-C(O)-C₁₋₄-Alkyl, C₁₋₆-Alkyl, oder
eine C₁₋₃-Alkylgruppe, in der jede Methylengruppe mit 1 oder 2 Fluoratomen und jede Methylgruppe mit 1, 2 oder 3 Fluoratomen substituiert sein kann,
**R²** H oder CH₃,
**R⁵** H oder C₁₋₄-Alkyl,
**R⁶** H, F, Cl, Br oder C₁₋₄-Alkyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze.

20. Verbindungen der allgemeinen Formel **Ib** gemäß Anspruch 19, in denen
**R¹** (a) eine gegebenenfalls mit einem Rest **R^{1.1}** substituierte C₁₋₆-Alkylgruppe,
eine gegebenenfalls mit 1, 2 oder 3 Resten **R^{1.3}** substituierte Phenylgruppe,
ein gegebenenfalls mit 1, 2 oder 3 Resten **R^{1.4}** substituierter fünfgliedriger Heteroaryl-Rest, der ausgewählt ist aus der Gruppe bestehend aus 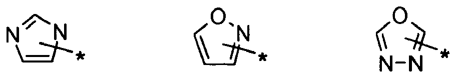 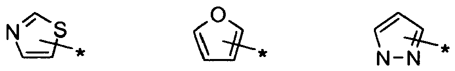 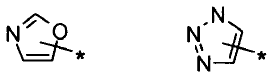 ein gegebenenfalls mit 1 oder 2 Resten **R^{1.4}** substituierter sechsgliedriger Heteroaryl-Rest, der ausgewählt ist aus der Gruppe bestehend aus 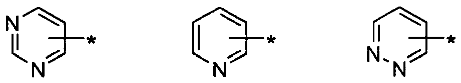 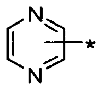 ein gegebenenfalls mit 1 oder 2 Resten **R^{1.4}** substituierter neungliedriger Heteroaryl-Rest, der ausgewählt ist aus der Gruppe bestehend aus 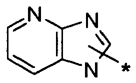 ein gegebenenfalls mit 1 oder 2 Resten **R^{1.4}** substituierter 5- oder 6-gliedriger
Heterocyclus, der ausgewählt ist aus der Gruppe bestehend aus 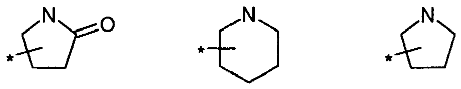 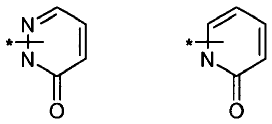 **R^{1.1}** -CN, Cyclopropyl, -OH, -OCH₃, -NH₂, -NHCH₃, -N(CH₃)₂,
**R^{1.3}** unabhängig voneinander
F, Cl, Br, -OH, -OCH₃, -OCF₃, C₁₋₄-Alkyl oder
eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
F, Cl, Br, -OH, -OCH₃, -OCF₃, -NH₂, -NH-C₁₋₄-Alkyl, -N(C₁₋₄-Alkyl)₂,
-NH-C(O)-C₁₋₄-Alkyl, C₁₋₆-Alkyl, oder
eine C₁₋₃-Alkylgruppe, in der jede Methylengruppe mit 1 oder 2 Fluoratomen und jede Methylgruppe mit 1, 2 oder 3 Fluoratomen substituiert sein kann,
**R²** H oder CH₃,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze.

21. Verbindungen der allgemeinen Formel **Ib** gemäß Anspruch 19, in denen
**R¹** eine Gruppe ausgewählt aus 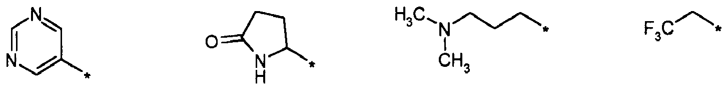 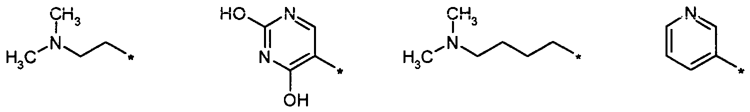 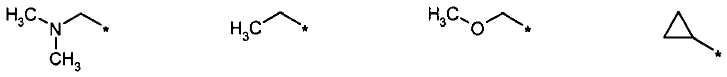 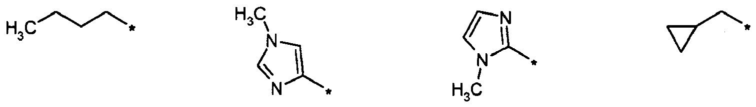 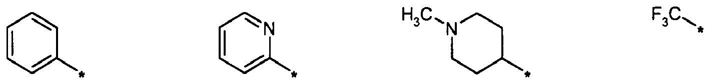 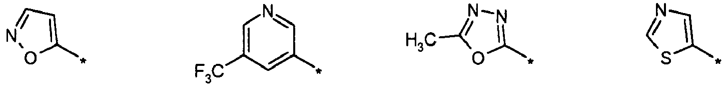 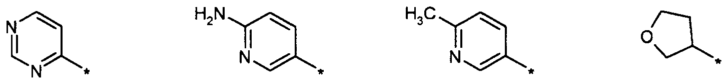 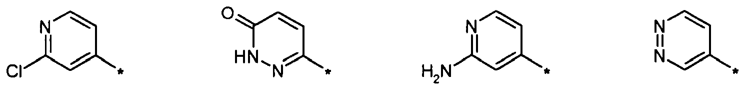 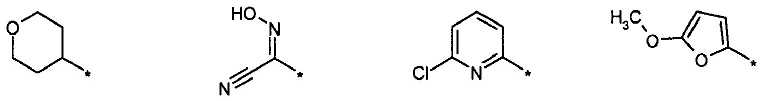 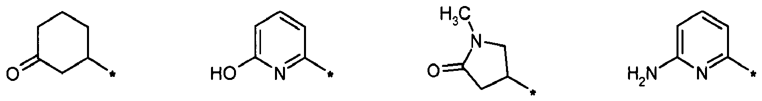 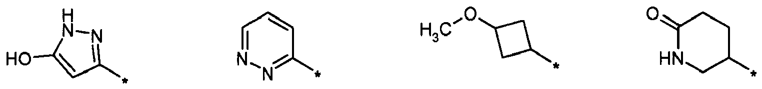 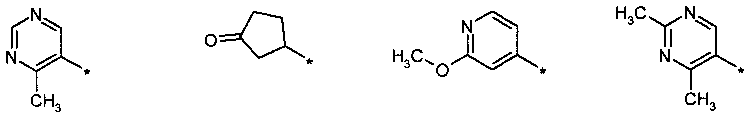 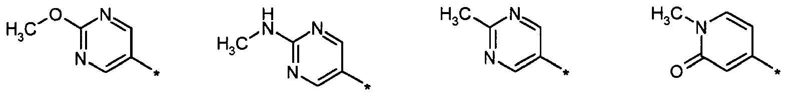 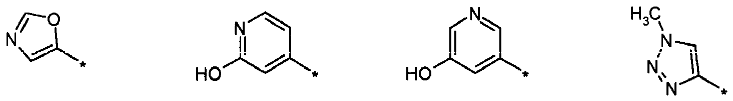 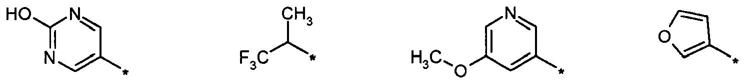 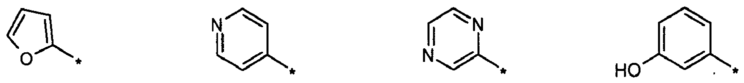 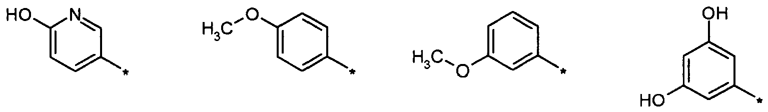 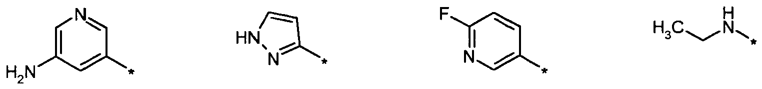 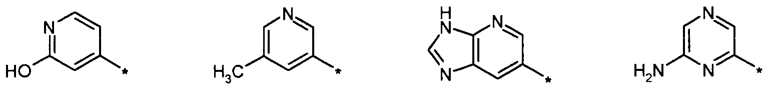 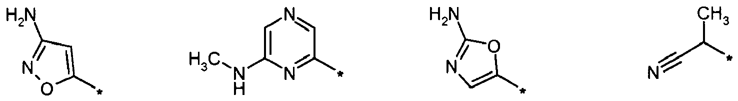 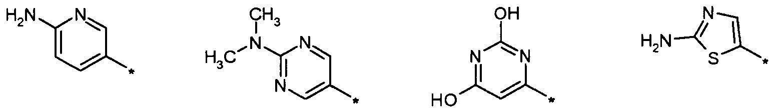 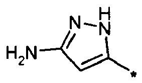 **R²** H oder CH₃,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze.

22. Verbindungen der allgemeinen Formel **Ib** gemäß Anspruch 19, in denen
**R¹** eine Gruppe ausgewählt aus 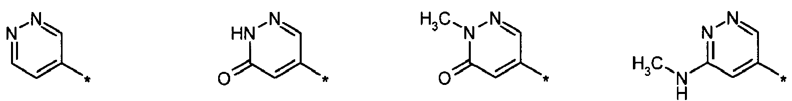 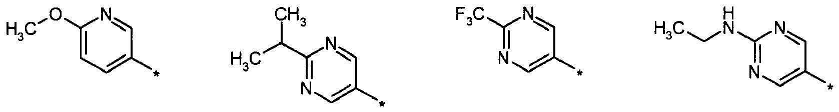 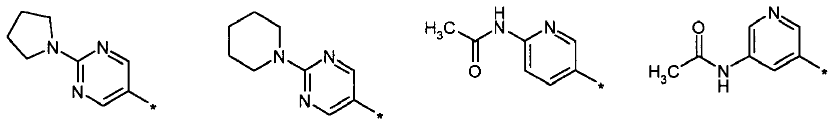 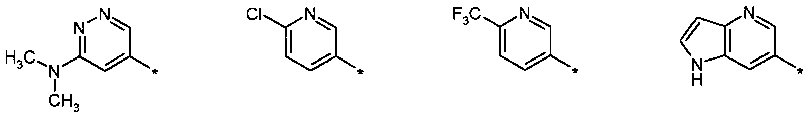 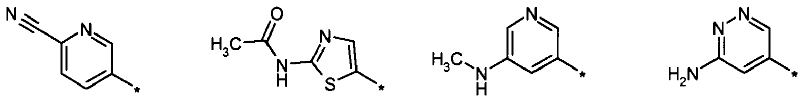 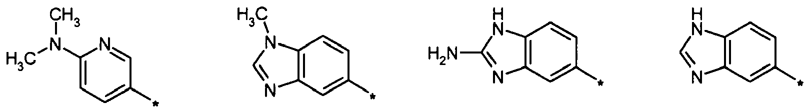 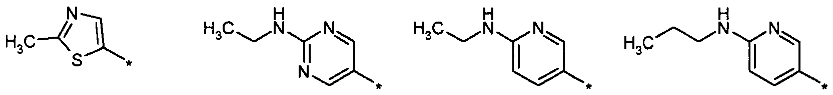 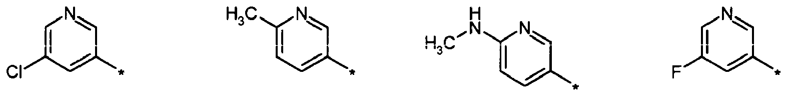 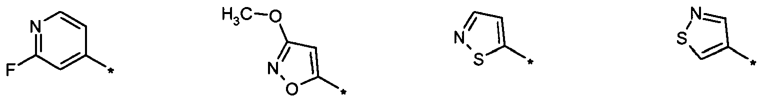 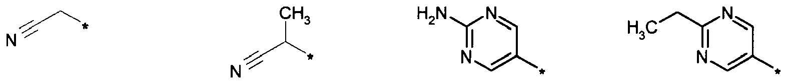 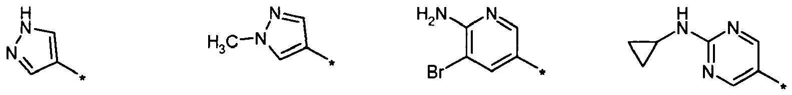 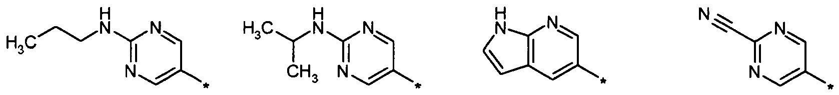 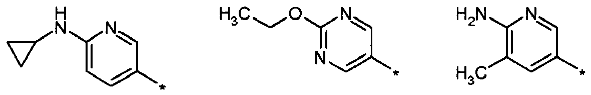 **R²** H,
**R⁵** H oder CH₃,
**R⁶** H, F, Cl oder Methyl,
**R⁷** H, F, Cl, Br, -CN, C₁₋₄-Alkyl, CF₃, CHF₂,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

23. Verbindungen der allgemeinen Formel Ic, 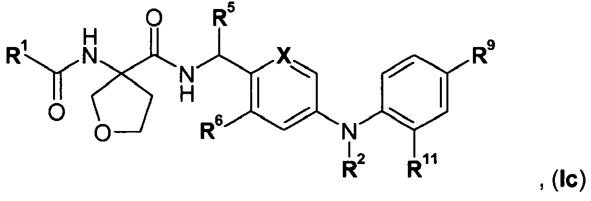 in der
**R¹** (a) eine gegebenenfalls mit einem Rest **R^{1.1}** substituierte C₁₋₆-Alkylgruppe,
eine gegebenenfalls mit 1, 2 oder 3 Resten **R^{1.3}** substituierte Phenylgruppe,
ein gegebenenfalls mit 1, 2 oder 3 Resten **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält,
ein gegebenenfalls mit 1 oder 2 Resten **R^{1.4}** substituierter sechsgliedriger Heteroaryl-Rest, der ein, zwei oder drei N-Atome enthält,
ein gegebenenfalls mit 1 oder 2 Resten **R^{1.4}** substituierter neun- oder zehngliedriger Heteroaryl-Rest, der ein, zwei oder drei N-Atome enthält,
ein gegebenenfalls mit 1 oder 2 Resten **R^{1.4}** substituierter 5- oder 6-gliedriger Heterocyclus, in dem eine -CH₂-Einheit durch eine -C(O)-Gruppe ersetzt sein kann,
**R^{1.1}** -CN, C₃₋₆-Cycloalkyl, -OH, -OCH₃, -NH₂, -NHCH₃, -N(CH₃)₂,
**R^{1.3}** unabhängig voneinander
F, Cl, Br, -OH, -OCH₃, C₁₋₆-Alkyl oder
eine C₁₋₃-Alkylgruppe, in der jede Methylengruppe mit 1 oder 2 Fluoratomen und jede Methylgruppe mit 1, 2 oder 3 Fluoratomen substituiert sein kann, und
**R^{1.4}** unabhängig voneinander
F, Cl, Br, -OH, -OCH₃, -NH₂, -NHCH₃, -N(CH₃)₂, -NH-C(O)-C₁₋₄-Alkyl, C₁₋₆-Alkyl, oder eine C₁₋₃-Alkylgruppe, in der jede Methylengruppe mit 1 oder 2 Fluoratomen und jede Methylgruppe mit 1, 2 oder 3 Fluoratomen substituiert sein kann,
**R²** H oder CH₃,
**R⁵** H oder C₁₋₄-Alkyl,
**R⁶** H, F, Cl, Br oder C₁₋₄-Alkyl,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze.

24. Verbindungen der allgemeinen Formel **Ic** gemäß Anspruch 23, in denen
**R¹** (a) eine gegebenenfalls mit einem Rest **R^{1.1}** substituierte C₁₋₆-Alkylgruppe,
eine gegebenenfalls mit 1, 2 oder 3 Resten **R^{1.3}** substituierte Phenylgruppe,
ein gegebenenfalls mit 1, 2 oder 3 Resten **R^{1.4}** substituierter fünfgliedriger Heteroaryl-Rest, der ausgewählt ist aus der Gruppe bestehend aus 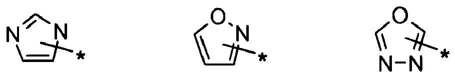 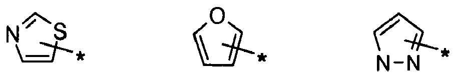 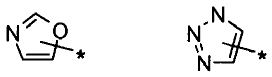 ein gegebenenfalls mit 1 oder 2 Resten **R^{1.4}** substituierter sechsgliedriger Heteroaryl-Rest, der ausgewählt ist aus der Gruppe bestehend aus 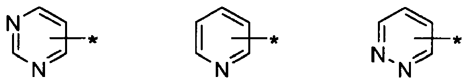 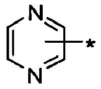 ein gegebenenfalls mit 1 oder 2 Resten **R^{1.4}** substituierter neungliedriger Heteroaryl-Rest, der ausgewählt ist aus der Gruppe bestehend aus 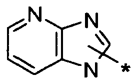 ein gegebenenfalls mit 1 oder 2 Resten **R^{1.4}** substituierter 5- oder 6-gliedriger Heterocyclus, der ausgewählt ist aus der Gruppe bestehend aus 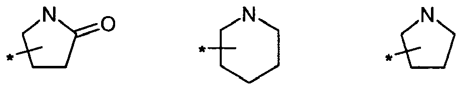 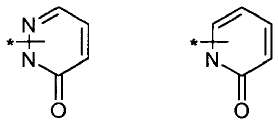 **R^{1.1}** -CN, Cyclopropyl, -OH, -OCH₃, -NH₂, -NHCH₃, -N(CH₃)₂,
**R^{1.3}** unabhängig voneinander
F, Cl, Br, -OH, -OCH₃, -OCF₃, C₁₋₄-Alkyl oder
eine C₁₋₃-Alkylgruppe, in der jede Methylengruppe mit 1 oder 2 Fluoratomen und jede Methylgruppe mit 1, 2 oder 3 Fluoratomen substituiert sein kann, und
R^{1.4} unabhängig voneinander
F, Cl, Br, -OH, -OCH₃, -OCF₃, -NH₂, -NH-C₁₋₄-Alkyl, -N(C₁₋₄-Alkyl)₂,
-NH-C(O)-C₁₋₄-Alkyl, C₁₋₆-Alkyl, oder
eine C₁₋₃-Alkylgruppe, in der jede Methylengruppe mit 1 oder 2 Fluoratomen und jede Methylgruppe mit 1, 2 oder 3 Fluoratomen substituiert sein kann,
R² H oder CH₃,
R⁵ H oder CH₃,
R⁶ H, F, Cl oder Methyl,
R⁹ F, Cl, Br, C₁₋₄-Alkyl, -O-C₁₋₄-Alkyl, -S-C₁₋₄-Alkyl,
R¹¹ F, Cl, Br, -CN, C₁₋₄-Alkyl, CF₃, CHF₂, und
X CH oder N bedeuten,
deren Enantiomere, deren Diastereomere, deren Gemische und deren Salze.

25. Verbindungen der allgemeinen Formel **Ic** gemäß Anspruch 23, in denen
R¹ eine Gruppe ausgewählt aus 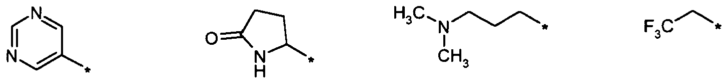 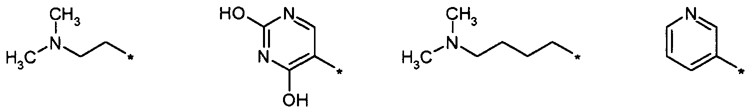 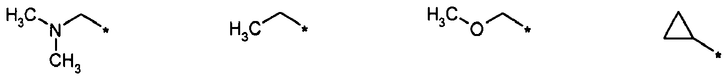 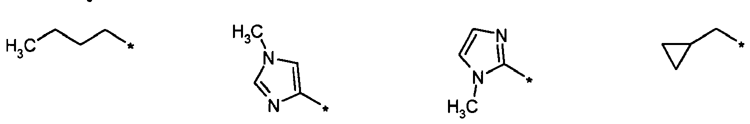 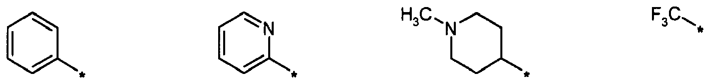 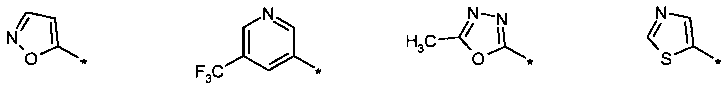 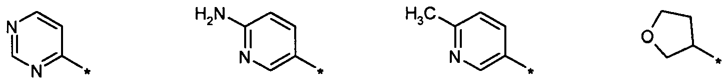 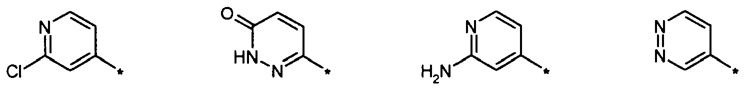 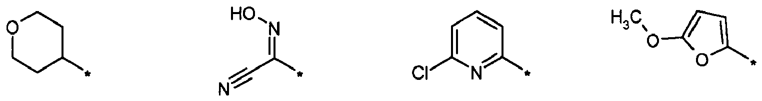 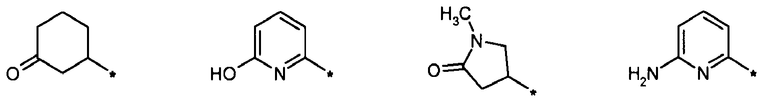 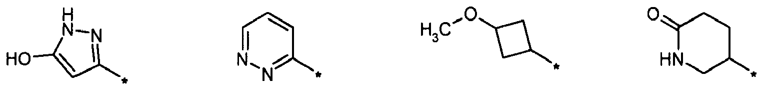 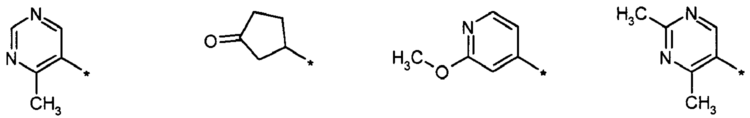 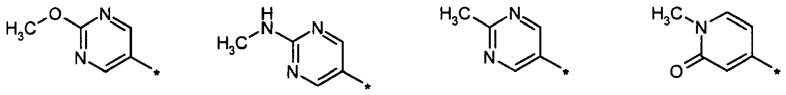 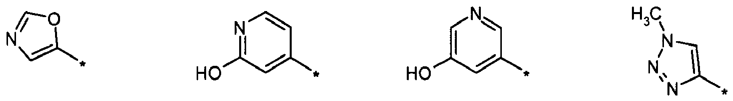 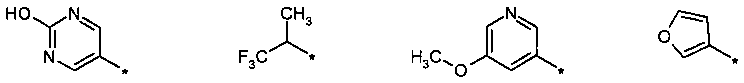 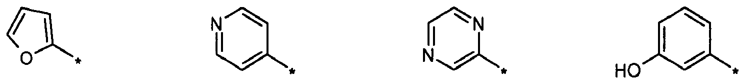 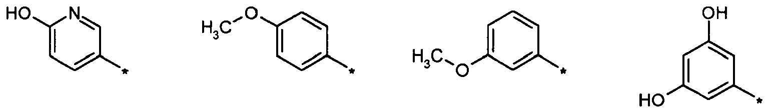 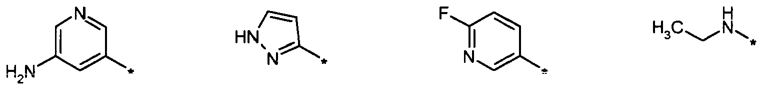 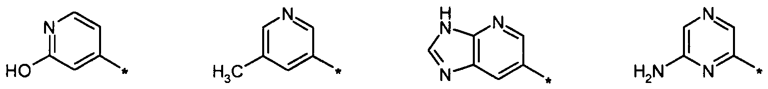 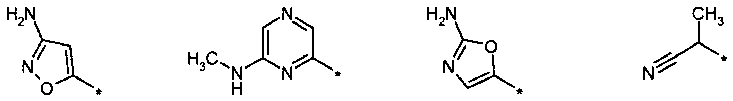 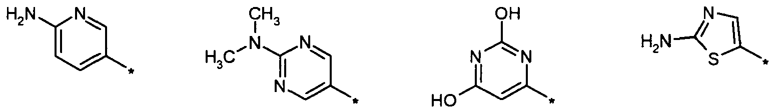 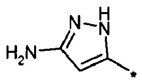 **R²** H oder CH₃,
**R⁵** H oder CH₃,
**R⁶** H, F, Cl oder Methyl,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze.

26. Verbindungen der allgemeinen Formel **Ic** gemäß Anspruch 23, in denen
**R¹** eine Gruppe ausgewählt aus 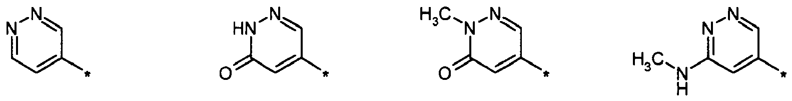 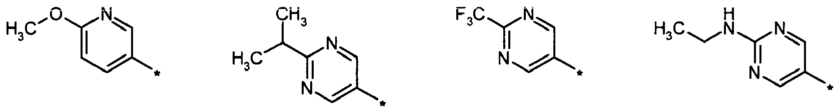 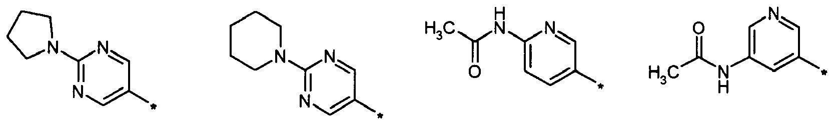 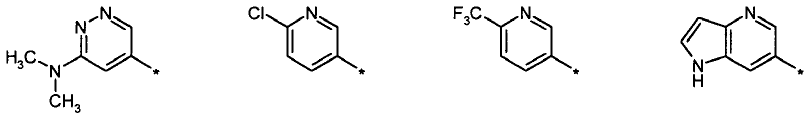 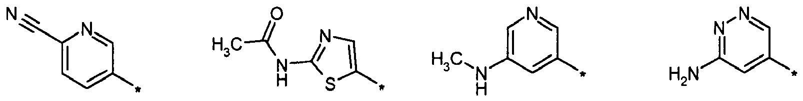 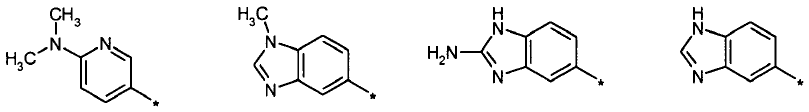 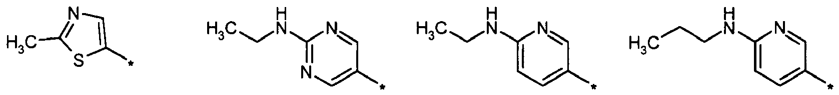 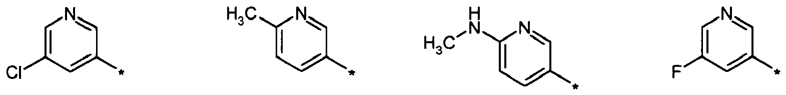 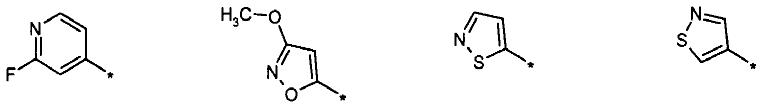 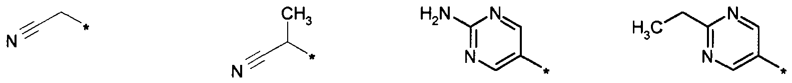 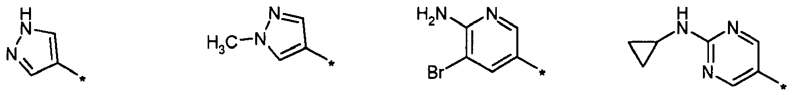 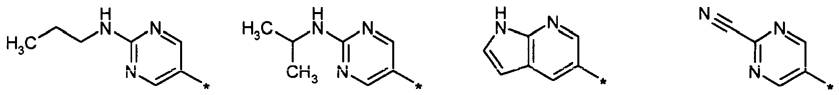 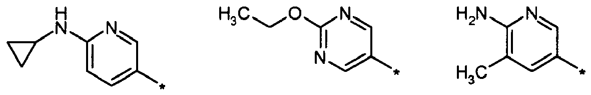 **R²** H oder CH₃,
**R⁵** H oder CH₃,
**R⁶** H, F, Cl oder Methyl,
**R⁹** F, Cl, Br, C₁₋₄-Alkyl, -O-C₁₋₄-Alkyl, -S-C₁₋₄-Alkyl,
**R¹¹** F, Cl, Br, -CN, C₁₋₄-Alkyl, CF₃, CHF₂, und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

27. Verbindungen der allgemeinen Formel **Id**, 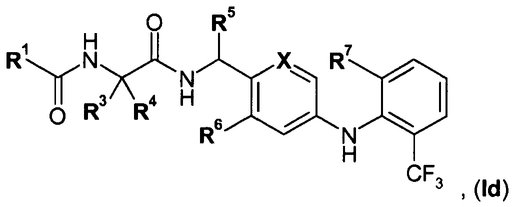 in der
**R¹** eine Gruppe ausgewählt aus 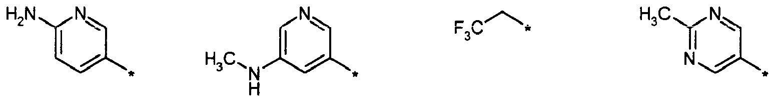 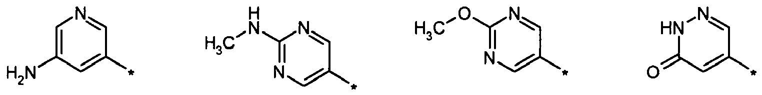 **R³** und **R⁴** zusammen mit dem Kohlenstoffatom, an dem sie gebunden sind, eine C₃₋₆-Cycloalkylengruppe, in der eine -CH₂-Einheit durch ein Sauerstoffatom ersetzt sein kann,
**R⁵** H oder CH₃,
**R⁶** Cl oder CH₃,
**R⁷** H oder F,
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

28. Folgende Verbindungen der allgemeinen Formel I gemäß Anspruch 1:
| **Nr.** | **Struktur** |
|---|---|
| (1) | 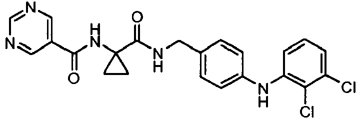 |
| (2) | 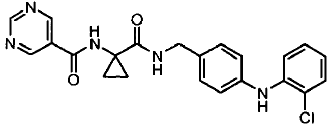 |
| (3) | 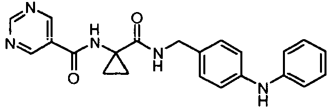 |
| (4) | 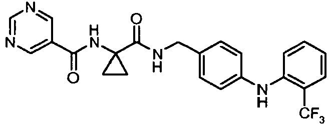 |
| (5) | 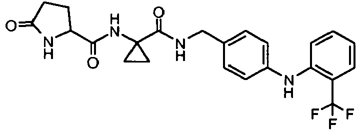 |
| (6) | 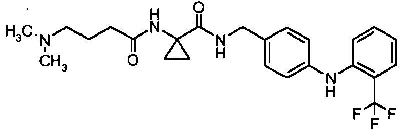 |
| (7) | 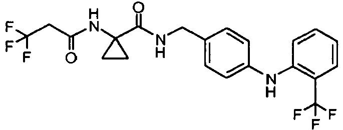 |
| (8) | 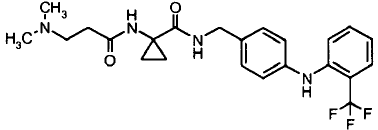 |
| (9) | 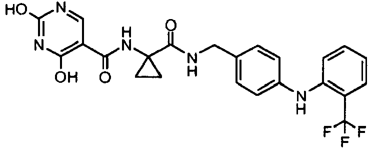 |
| (10) | 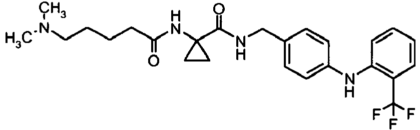 |
| (11) | 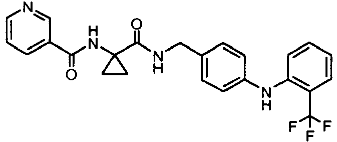 |
| (12) | 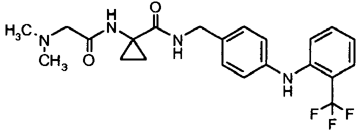 |
| (13) | 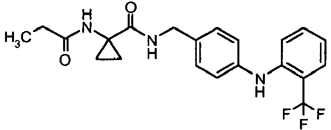 |
| (14) | 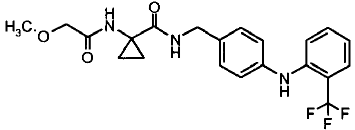 |
| (15) | 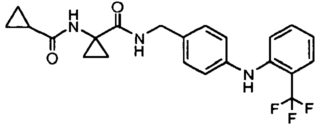 |
| (16) | 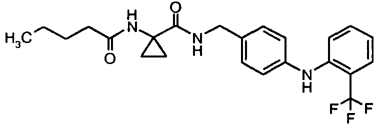 |
| (17) | 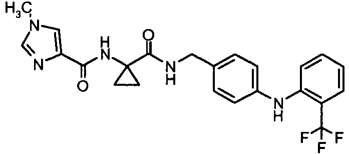 |
| (18) | 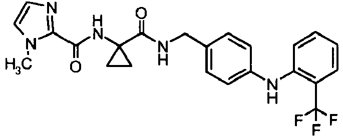 |
| (19) | 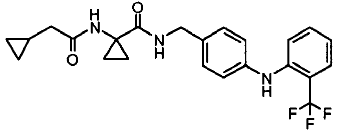 |
| (20) | 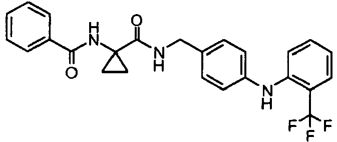 |
| (21) | 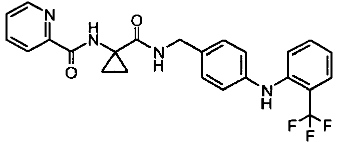 |
| (22) | 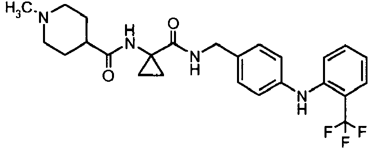 |
| (23) | 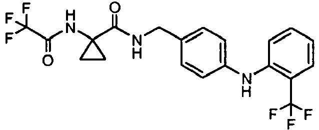 |
| (24) | 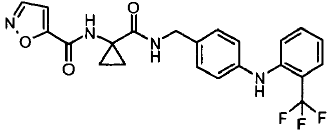 |
| (25) | 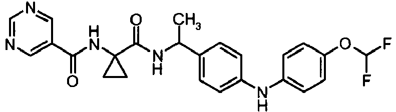 |
| (26) | 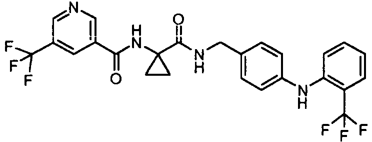 |
| (27) | 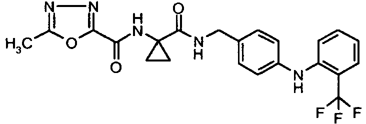 |
| (28) | 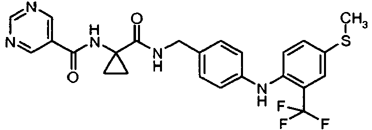 |
| (29) | 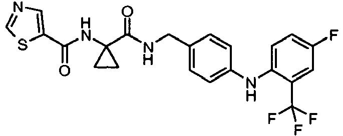 |
| (30) | 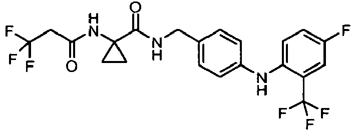 |
| (31) | 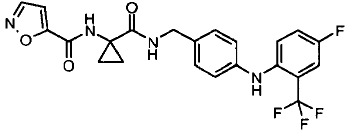 |
| (32) | 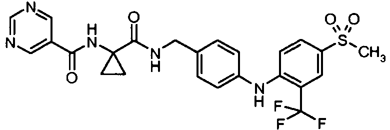 |
| (33) | 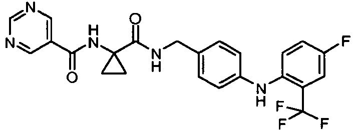 |
| (34) | 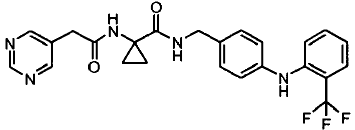 |
| (35) | 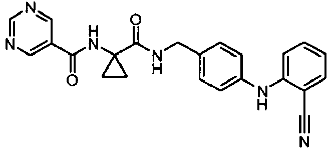 |
| (36) | 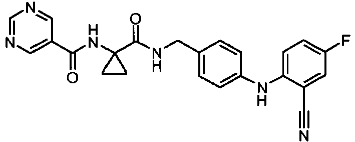 |
| (37) | 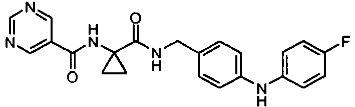 |
| (38) | 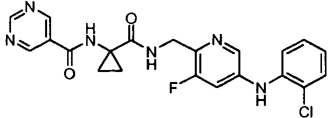 |
| (39) | 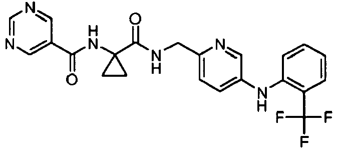 |
| (40) | 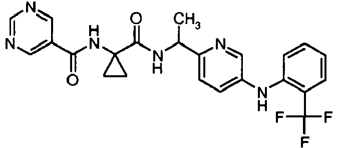 |
| (41) | 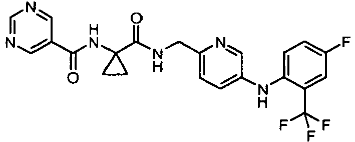 |
| (42) | 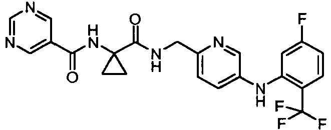 |
| (43) | 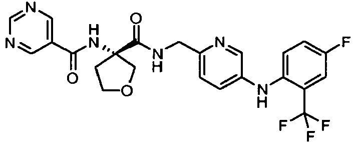 |
| (44) | 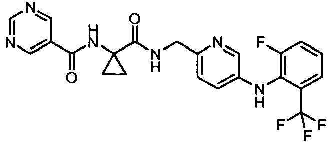 |
| (45) | 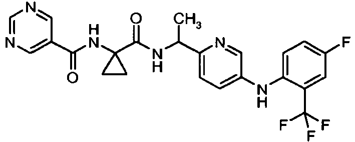 |
| (46) | 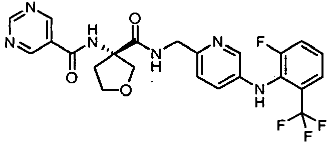 |
| (47) | 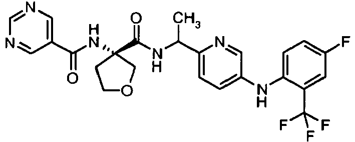 |
| (48) | 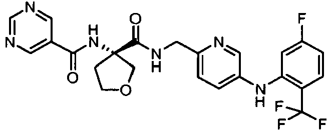 |
| (49) | 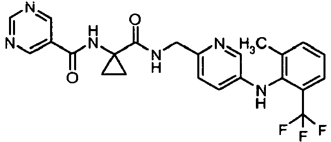 |
| (50) | 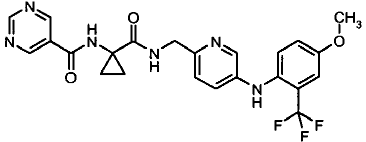 |
| (51) | 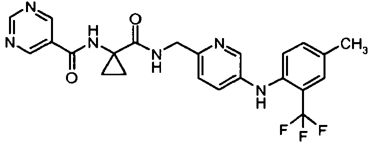 |
| (52) | 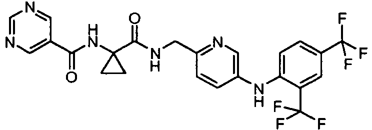 |
| (53) | 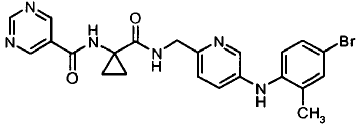 |
| (54) | 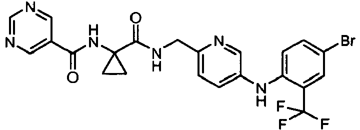 |
| (55) | 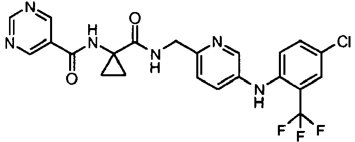 |
| (56) | 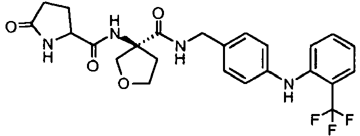 |
| (57) | 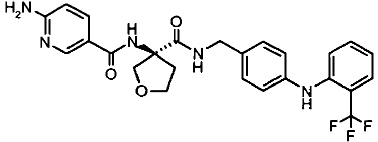 |
| (58) | 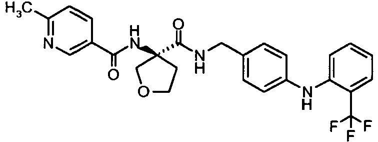 |
| (59) | 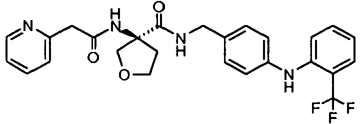 |
| (60) | 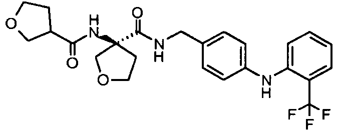 |
| (61) | 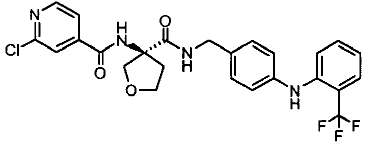 |
| (62) | 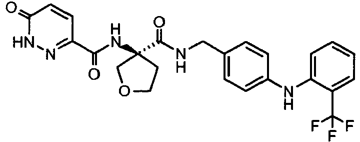 |
| (63) | 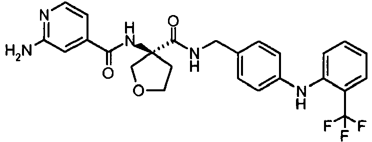 |
| (64) | 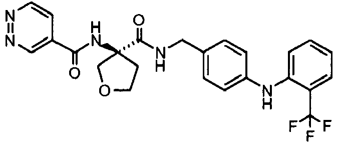 |
| (65) | 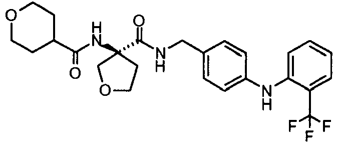 |
| (66) | 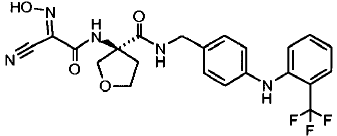 |
| (67) | 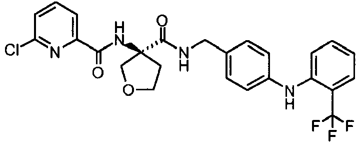 |
| (68) | 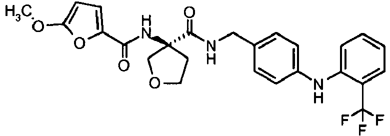 |
| (69) | 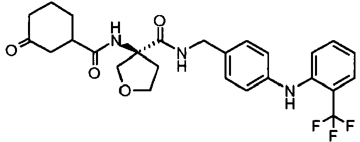 |
| (70) | 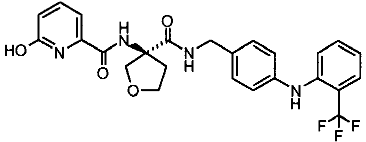 |
| (71) | 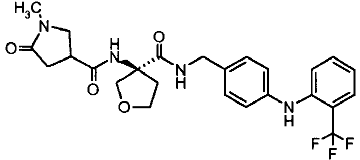 |
| (72) | 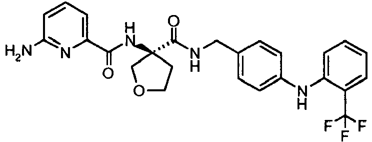 |
| (73) | 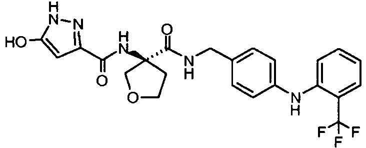 |
| (74) | 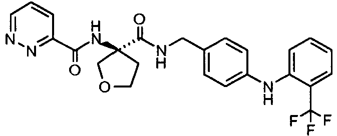 |
| (75) | 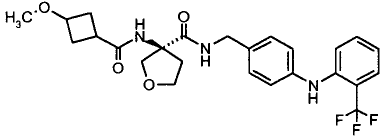 |
| (76) | 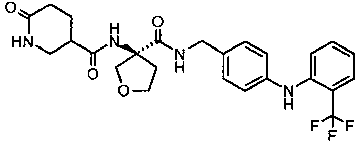 |
| (77) | 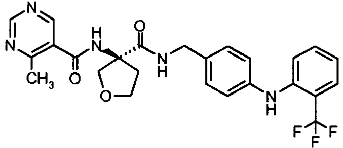 |
| (78) | 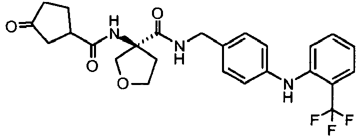 |
| (79) | 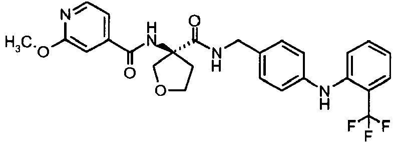 |
| (80) | 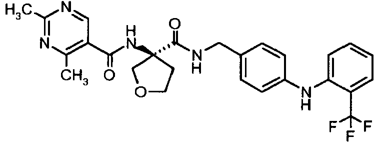 |
| (81) | 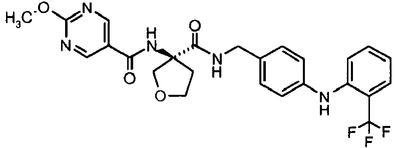 |
| (82) | 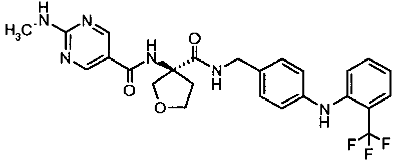 |
| (83) | 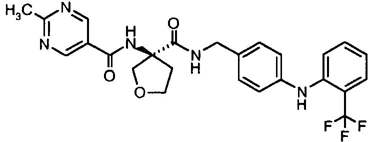 |
| (84) | 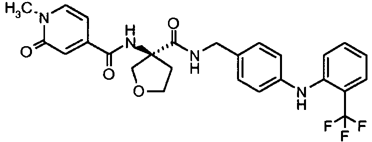 |
| (85) | 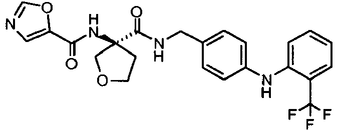 |
| (86) | 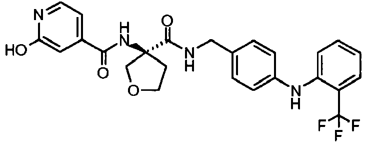 |
| (87) | 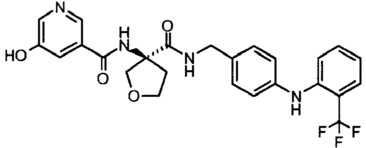 |
| (88) | 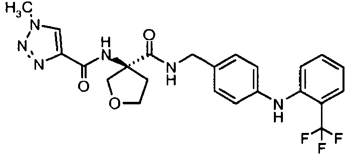 |
| (89) | 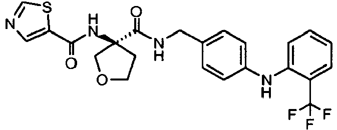 |
| (90) | 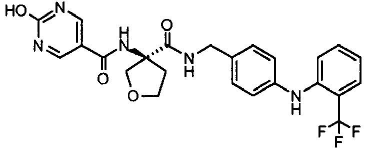 |
| (91) | 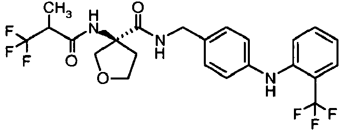 |
| (92) | 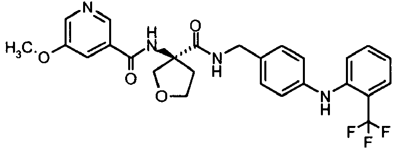 |
| (93) | 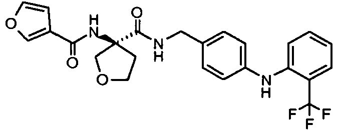 |
| (94) | 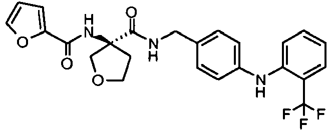 |
| (95) | 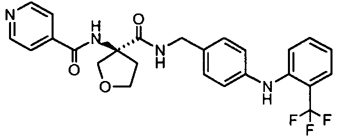 |
| (96) | 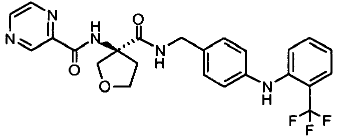 |
| (97) | 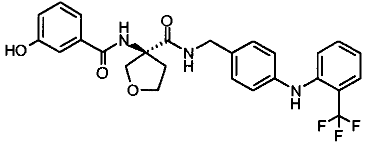 |
| (98) | 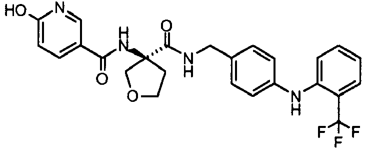 |
| (99) | 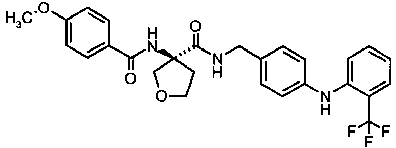 |
| (100) | 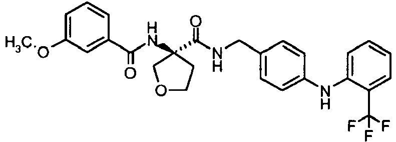 |
| (101) | 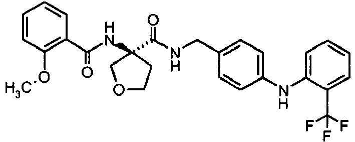 |
| (102) | 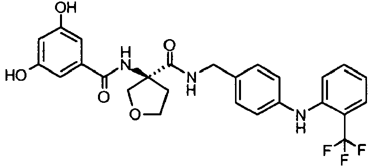 |
| (103) | 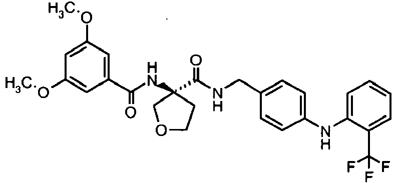 |
| (104) | 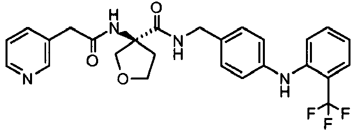 |
| (105) | 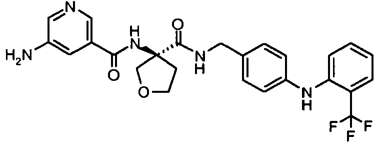 |
| (106) | 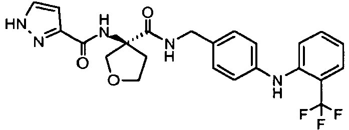 |
| (107) | 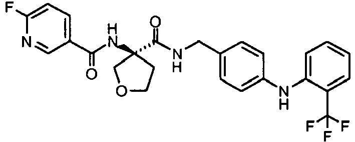 |
| (108) | 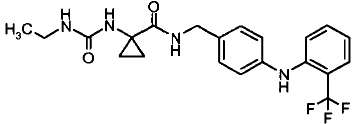 |
| (109) | 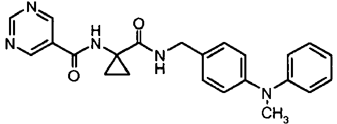 |
| (110) | 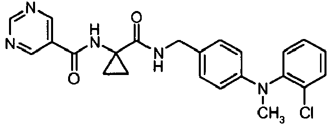 |
| (111) | 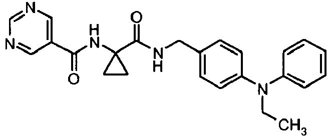 |
| (112) | 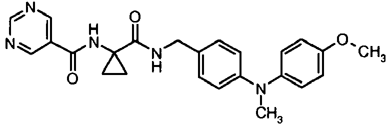 |
| (113) | 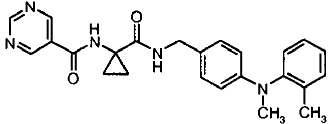 |
| (114) | 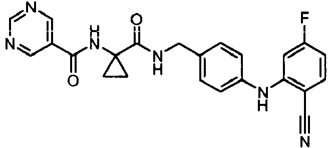 |
| (115) | 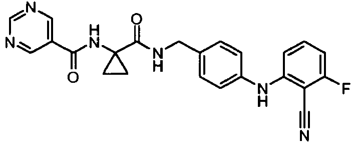 |
| (116) | 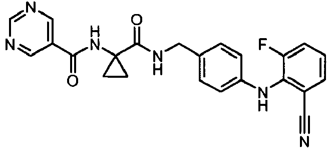 |
| (117) | 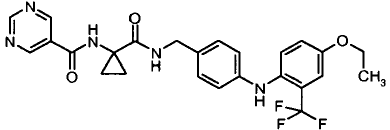 |
| (118) | 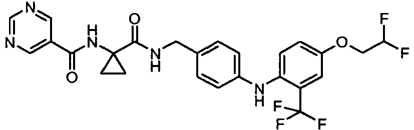 |
| (119) | 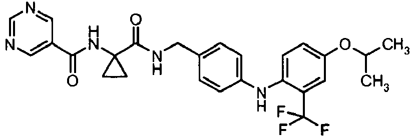 |
| (120) | 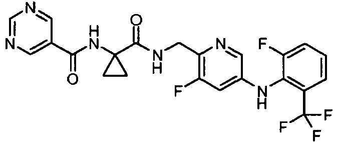 |
| (121) | 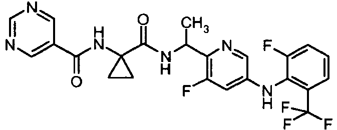 |
| (122) | 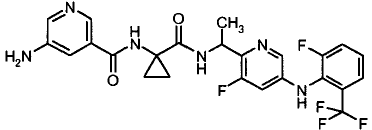 |
| (123) | 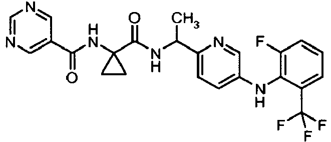 |
| (124) | 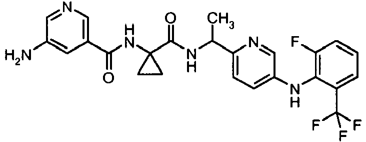 |
| (125) | 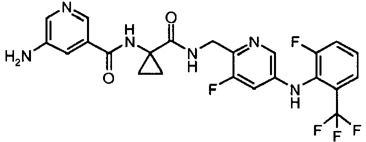 |
| (126) | 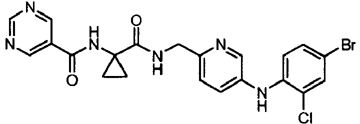 |
| (127) | 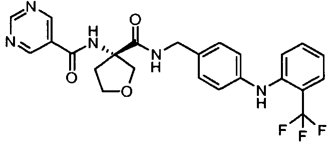 |
| (128) | 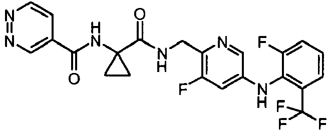 |
| (129) | 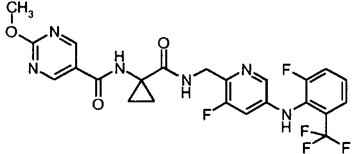 |
| (130) | 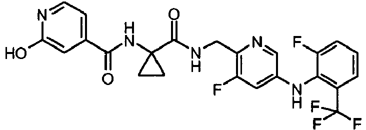 |
| (131) | 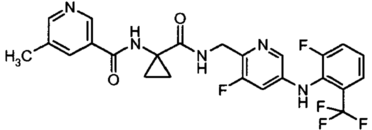 |
| (132) | 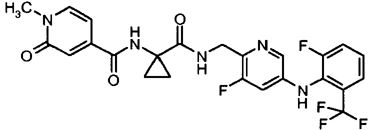 |
| (133) | 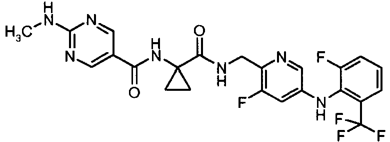 |
| (134) | 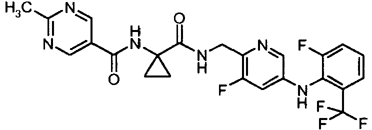 |
| (135) | 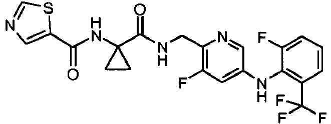 |
| (136) | 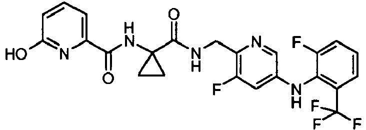 |
| (137) | 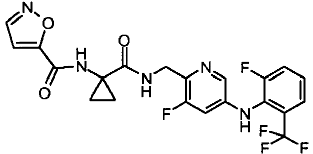 |
| (138) | 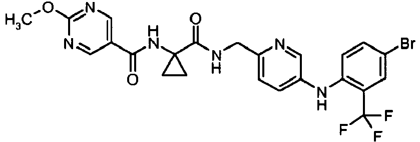 |
| (139) | 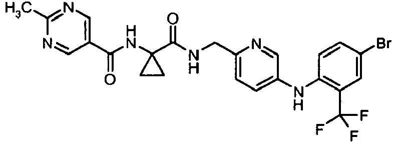 |
| (140) | 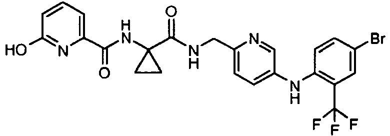 |
| (141) | 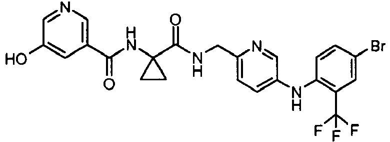 |
| (142) | 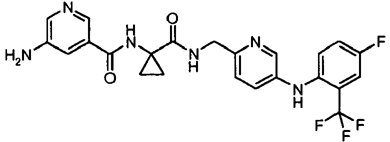 |
| (143) | 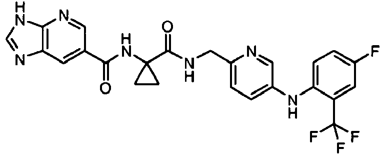 |
| (144) | 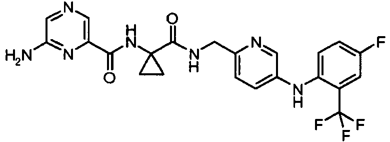 |
| (145) | 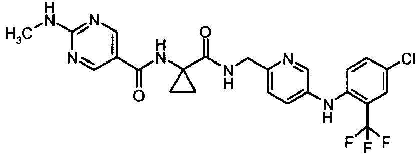 |
| (146) | 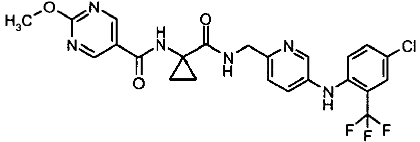 |
| (147) | 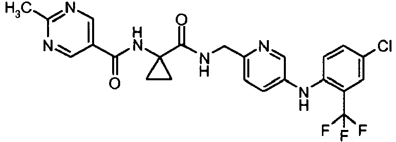 |
| (148) | 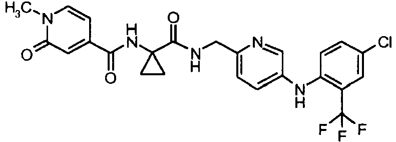 |
| (149) | 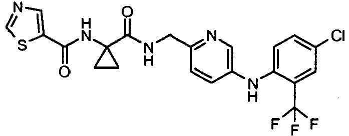 |
| (150) | 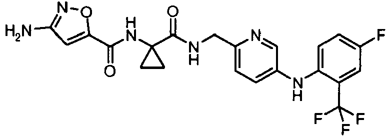 |
| (151) | 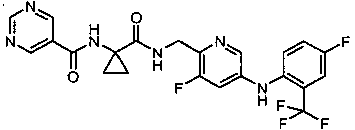 |
| (152) | 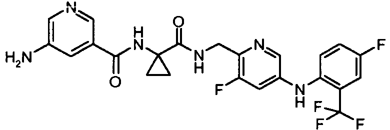 |
| (153) | 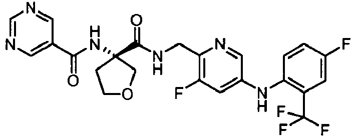 |
| (154) | 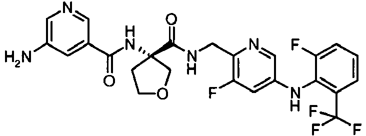 |
| (155) | 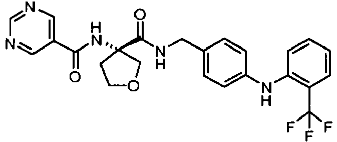 |
| (156) | 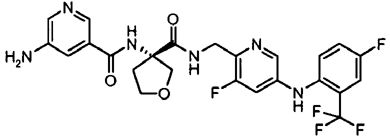 |
| (157) | 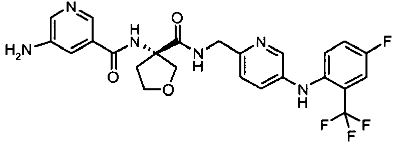 |
| (158) | 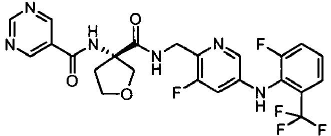 |
| (159) | 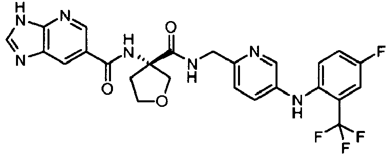 |
| (160) | 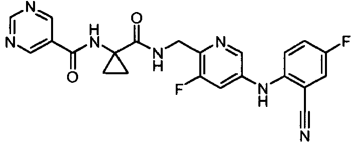 |
| (161) | 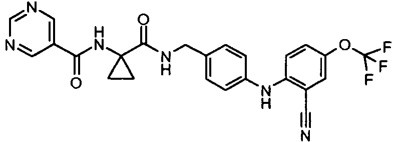 |
| (162) | 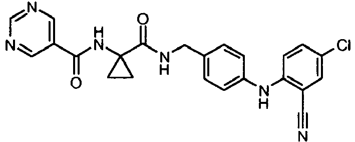 |
| (163) | 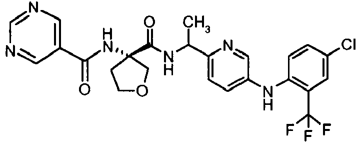 |
| (164) | 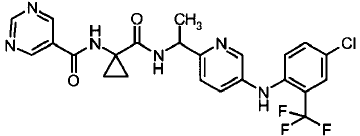 |
| (165) | 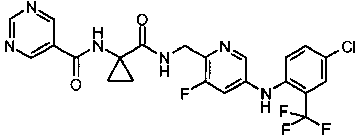 |
| (166) | 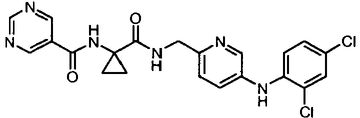 |
| (167) | 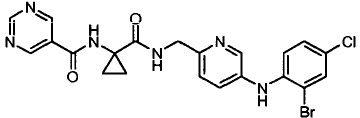 |
| (168) | 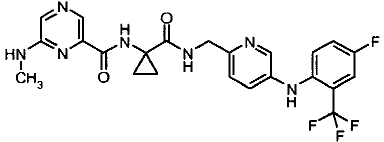 |
| (169) | 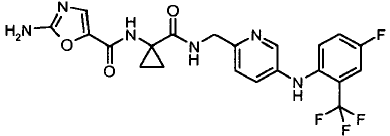 |
| (170) | 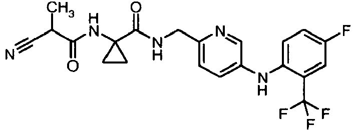 |
| (171) | 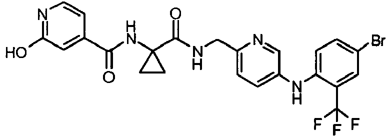 |
| (172) | 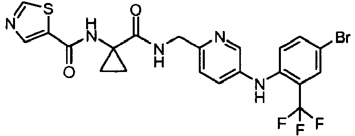 |
| (173) | 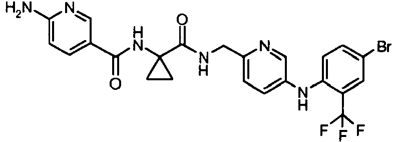 |
| (174) | 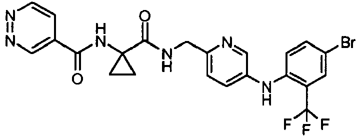 |
| (175) | 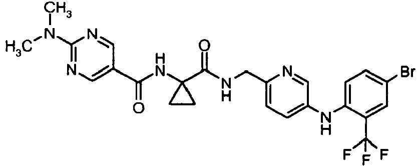 |
| (176) | 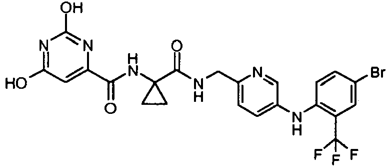 |
| (177) | 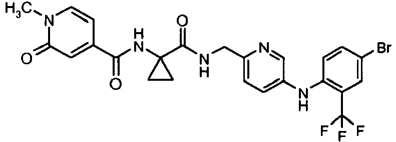 |
| (178) | 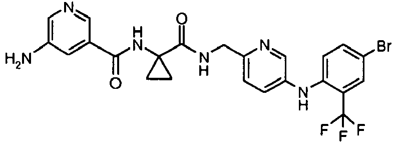 |
| (179) | 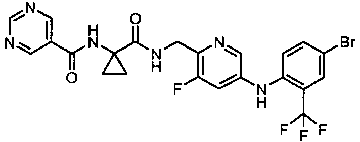 |
| (180) | 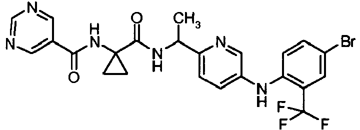 |
| (181) | 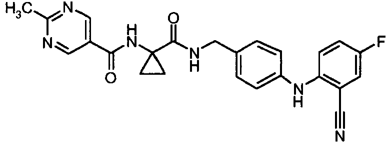 |
| (182) | 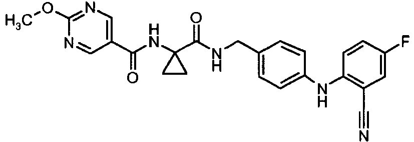 |
| (183) | 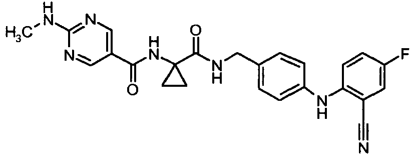 |
| (184) | 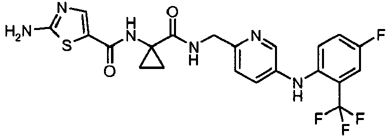 |
| (185) | 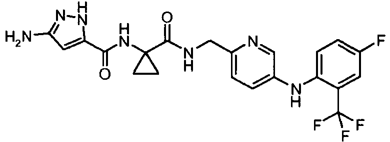 |
| (186) | 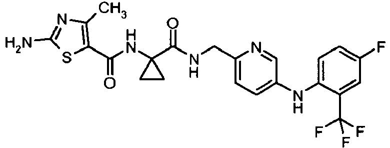 |
| (187) | 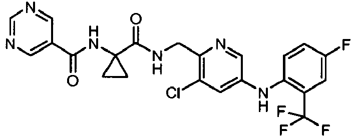 |
| (188) | 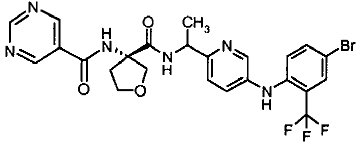 |
| (189) | 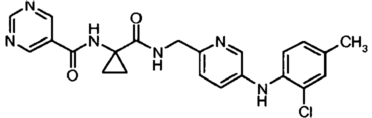 |
| (190) | 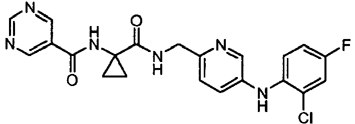 |
| (191) | 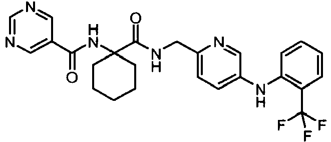 |
| (192) | 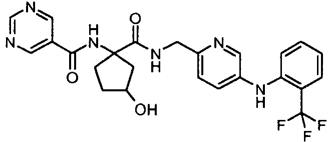 |
| (193) | 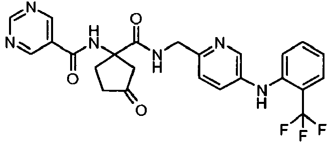 |
| (194) | 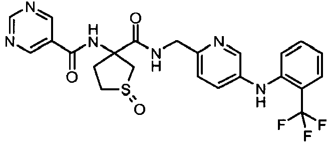 |
| (195) | 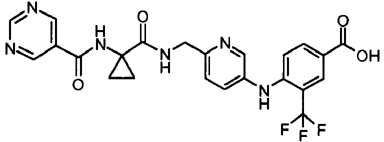 |
| (196) | 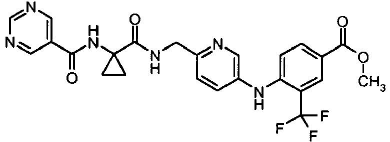 |
| (197) | 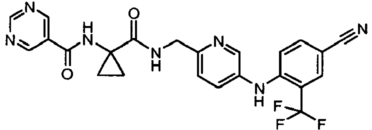 |
| (198) | 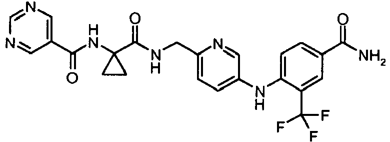 |
| (199) | 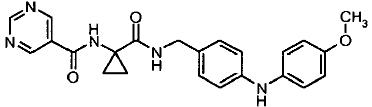 |
| (200) | 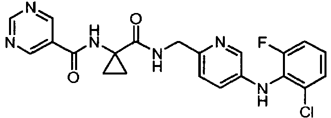 |
| (201) | 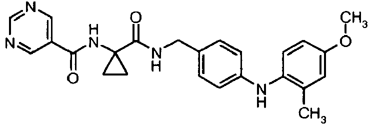 |
| (202) | 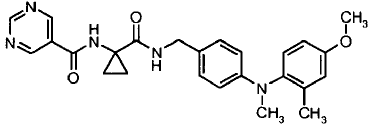 |
| (203) | 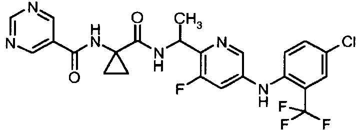 |
| (203a) | 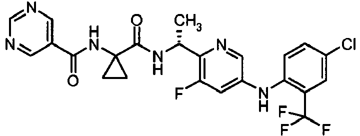 |
| (203b) | 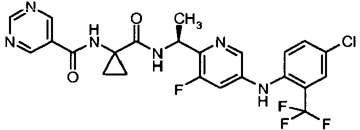 |
| (204) | 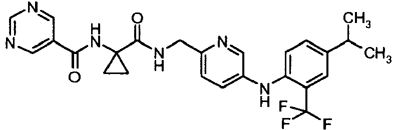 |
| (205) | 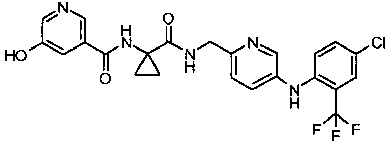 |
| (206) | 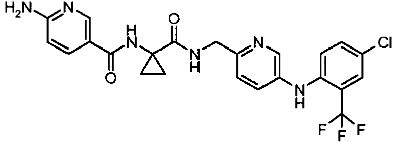 |
| (207) | 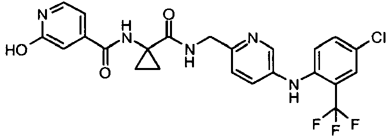 |
| (208) | 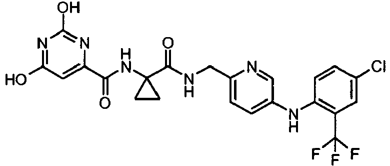 |
| (209) | 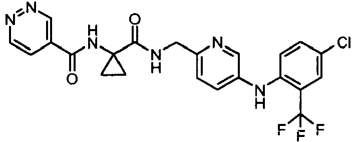 |
| (210) | 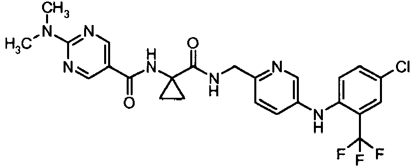 |
| (211) | 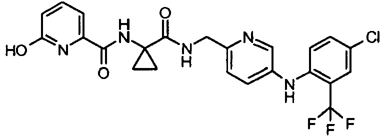 |
| (212) | 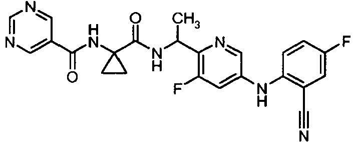 |
| (212a) | 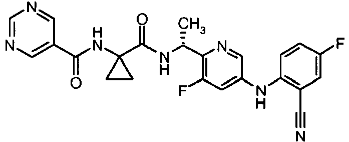 |
| (212b) | 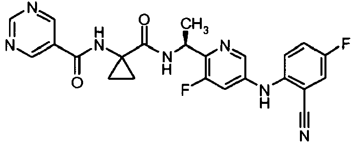 |
| (213) | 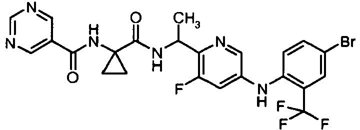 |
| (213a) | 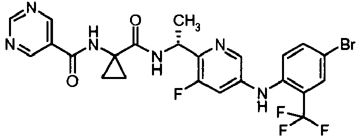 |
| (213b) | 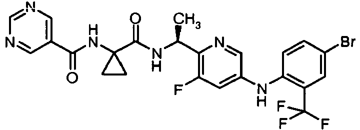 |
| (214) | 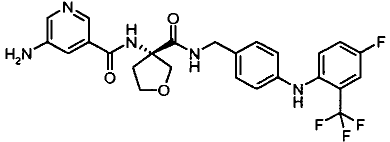 |
| (215) | 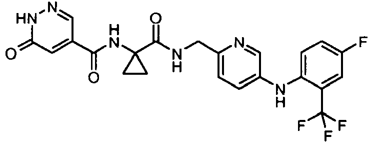 |
| (216) | 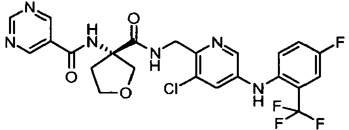 |
| (217) | 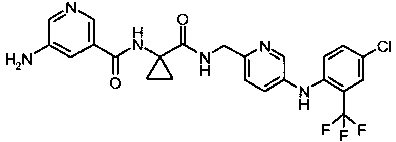 |
| (218) | 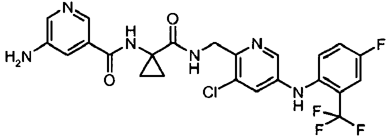 |
| (219) | 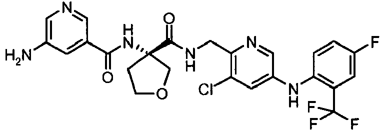 |
| (220) | 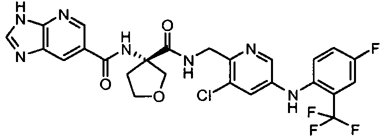 |
| (221) | 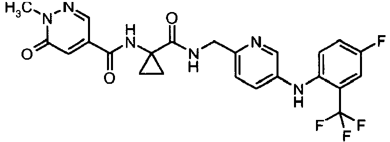 |
| (222) | 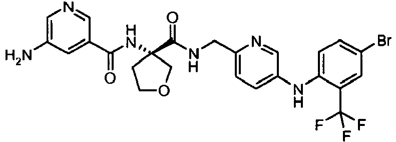 |
| (223) | 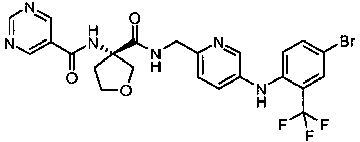 |
| (224) | 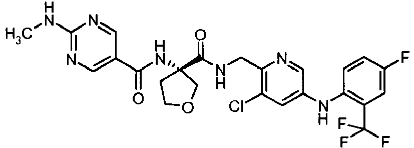 |
| (225) | 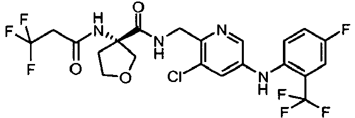 |
| (226) | 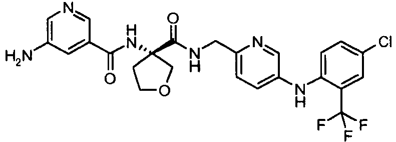 |
| (227) | 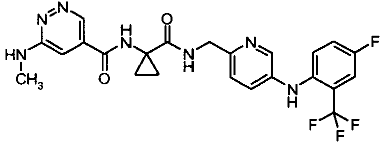 |
| (228) | 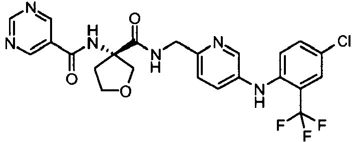 |
| (229) | 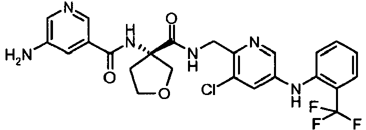 |
| (230) | 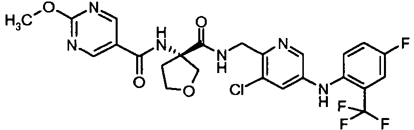 |
| (231) | 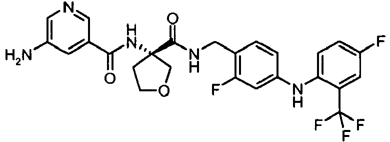 |
| (232) | 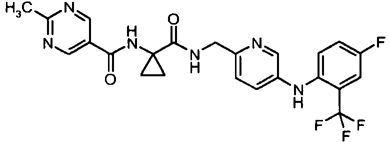 |
| (233) | 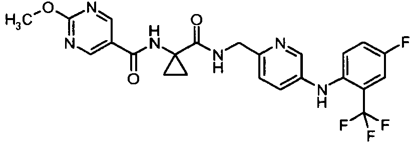 |
| (234) | 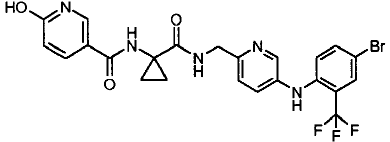 |
| (235) | 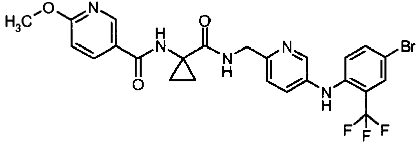 |
| (236) | 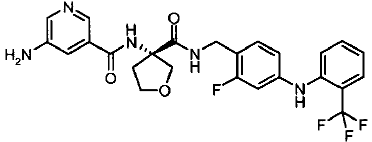 |
| (237) | 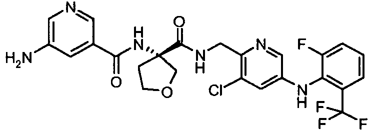 |
| (238) | 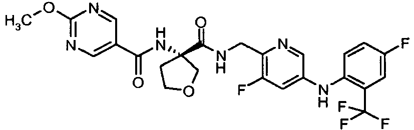 |
| (239) | 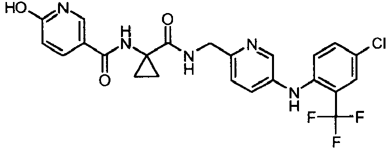 |
| (240) | 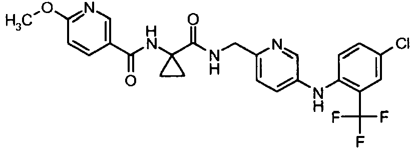 |
| (241) | 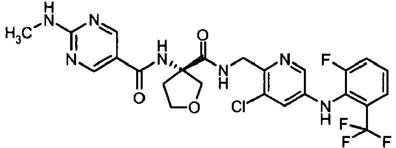 |
| (242) | 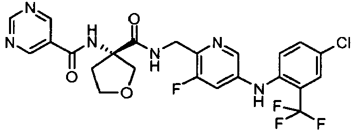 |
| (243) | 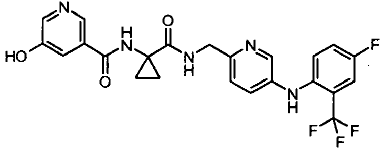 |
| (244) | 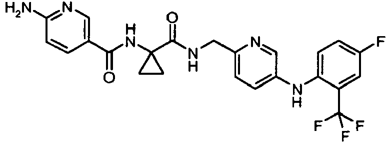 |
| (245) | 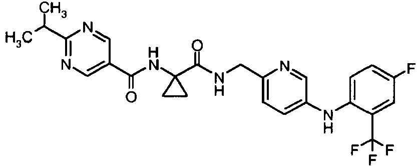 |
| (246) | 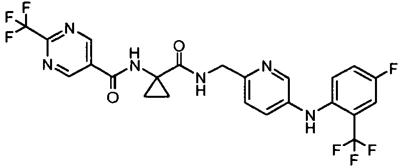 |
| (247) | 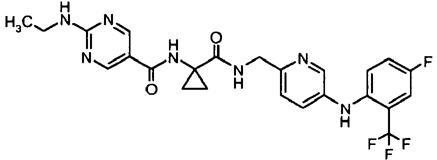 |
| (248) | 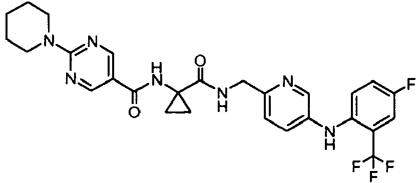 |
| (249) | 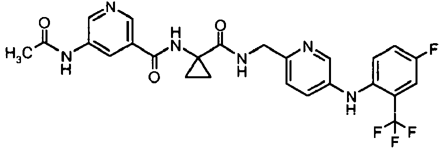 |
| (250) | 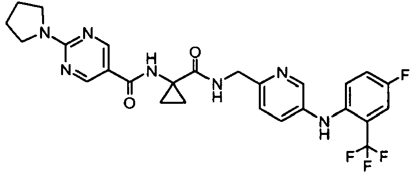 |
| (251) | 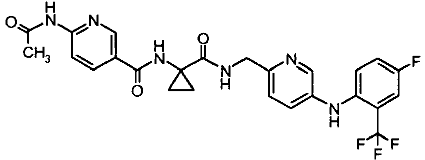 |
| (252) | 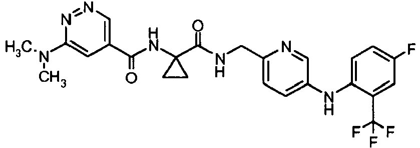 |
| (253) | 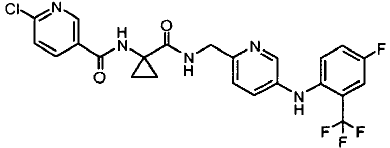 |
| (254) | 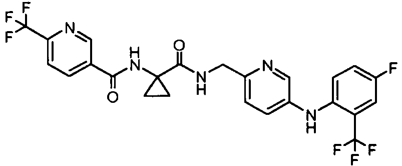 |
| (255) | 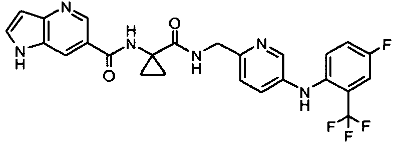 |
| (256) | 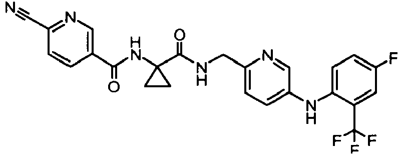 |
| (257) | 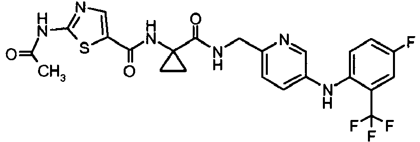 |
| (258) | 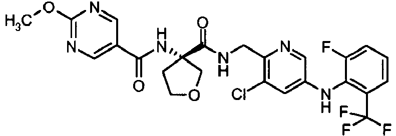 |
| (258) | 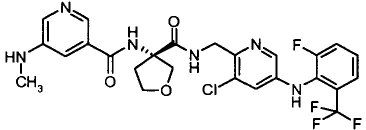 |
| (260) | 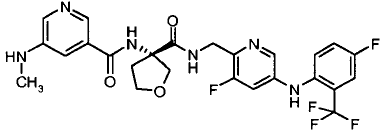 |
| (261) | 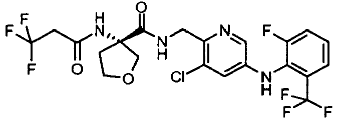 |
| (262) | 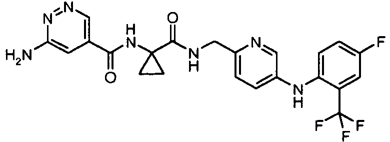 |
| (263) | 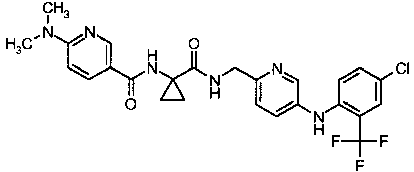 |
| (264) | 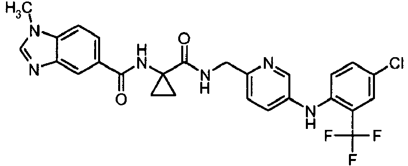 |
| (265) | 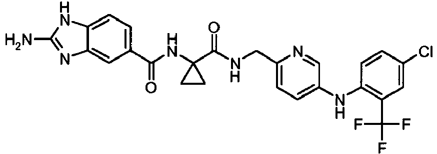 |
| (266) | 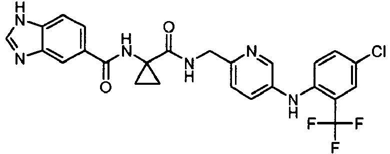 |
| (267) | 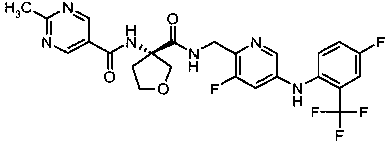 |
| (268) | 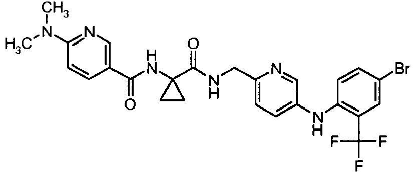 |
| (269) | 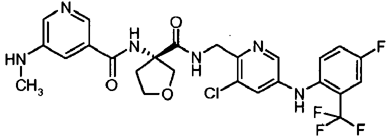 |
| (270) | 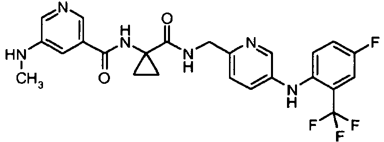 |
| (271) | 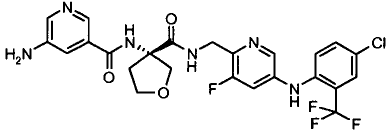 |
| (272) | 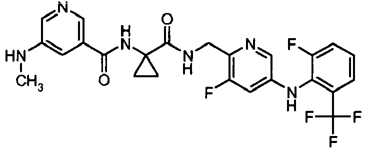 |
| (273) | 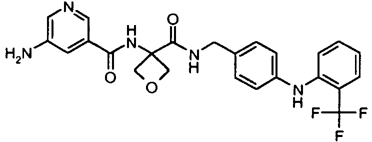 |
| (274) | 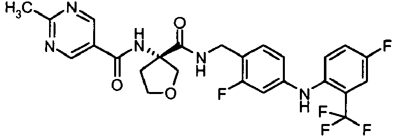 |
| (275) | 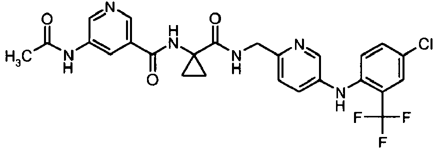 |
| (276) | 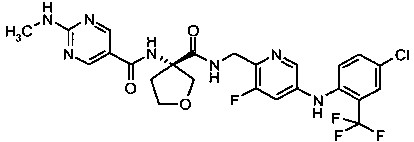 |
| (277) | 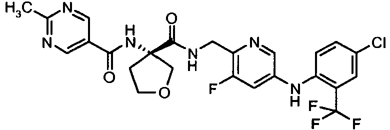 |
| (278) | 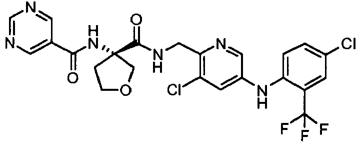 |
| (279) | 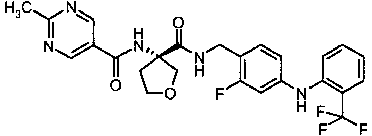 |
| (280) | 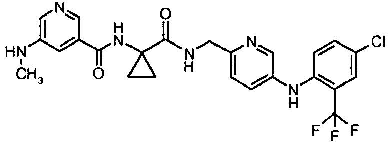 |
| (281) | 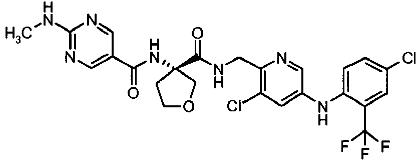 |
| (282) | 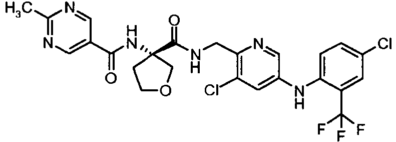 |
| (283) | 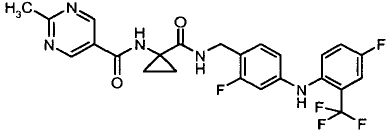 |
| (284) | 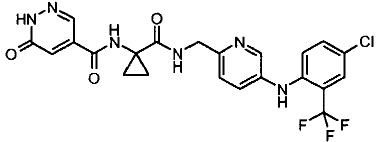 |
| (285) | 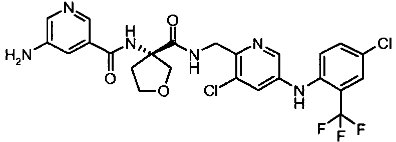 |
| (286) | 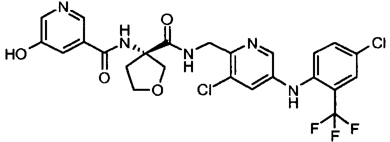 |
| (287) | 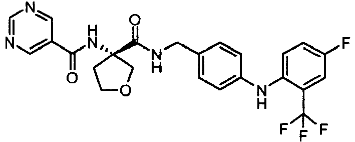 |
| (288) | 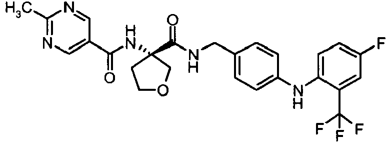 |
| (289) | 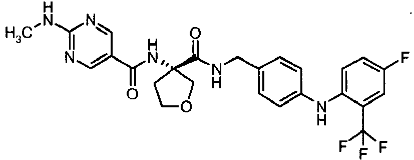 |
| (290) | 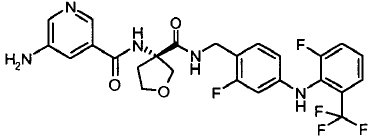 |
| (291) | 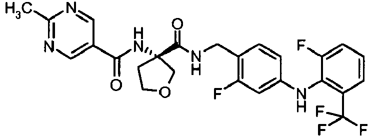 |
| (292) | 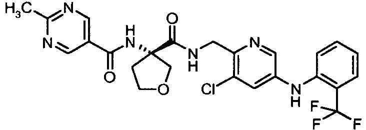 |
| (293) | 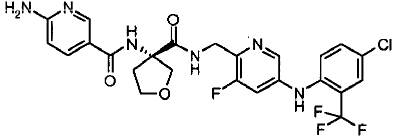 |
| (294) | 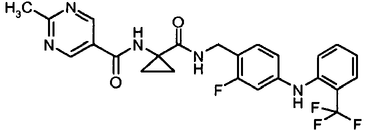 |
| (295) | 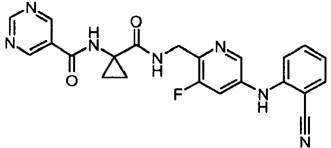 |
| (296) | 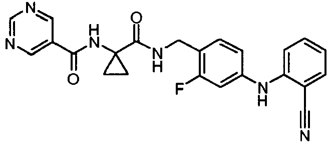 |
| (297) | 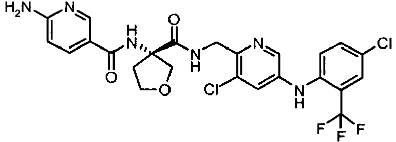 |
| (298) | 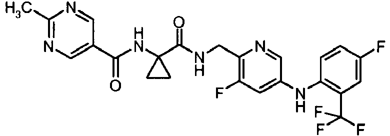 |
| (299) | 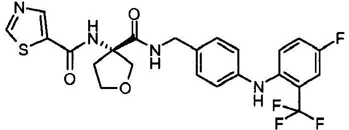 |
| (300) | 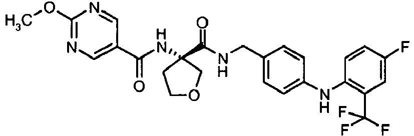 |
| (301) | 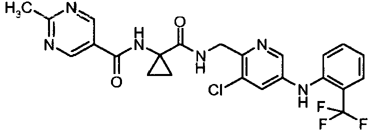 |
| (302) | 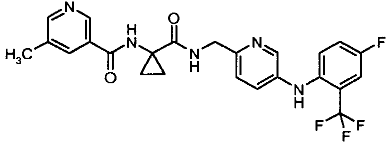 |
| (303) | 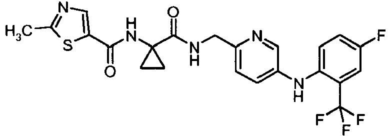 |
| (304) | 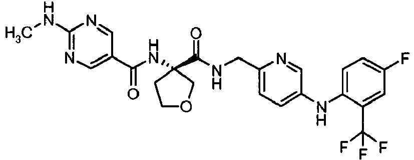 |
| (305) | 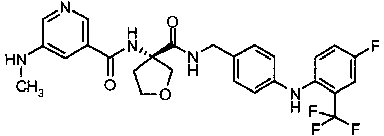 |
| (306) | 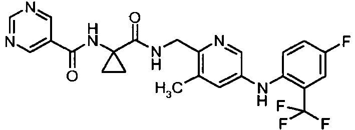 |
| (307) | 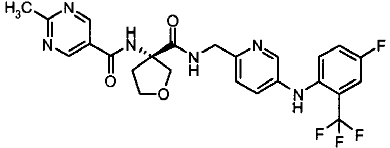 |
| (308) | 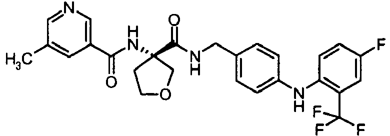 |
| (309) | 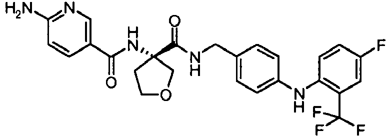 |
| (310) | 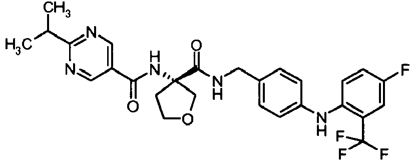 |
| (311) | 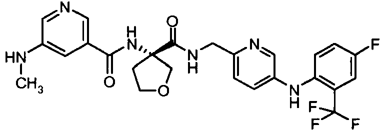 |
| (312) | 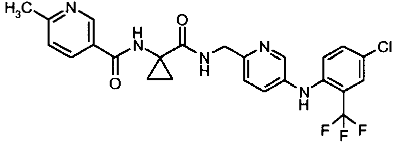 |
| (313) | 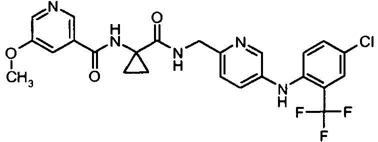 |
| (314) | 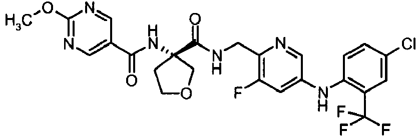 |
| (315) | 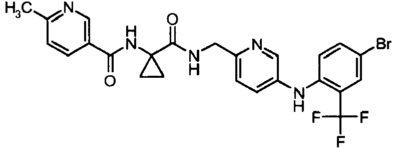 |
| (316) | 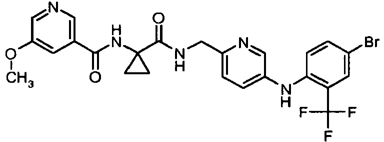 |
| (317) | 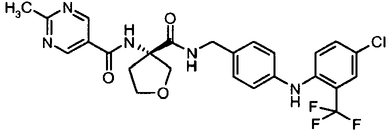 |
| (318) | 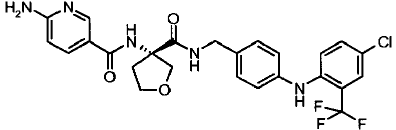 |
| (319) | 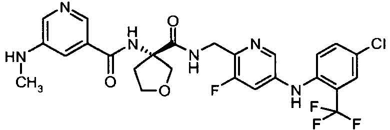 |
| (320) | 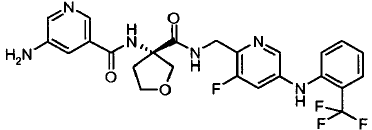 |
| (321) | 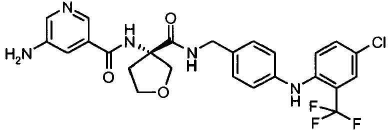 |
| (322) | 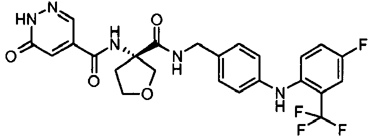 |
| (323) | 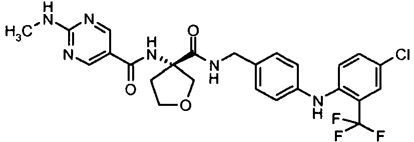 |
| (324) | 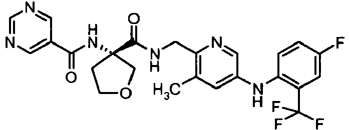 |
| (325) | 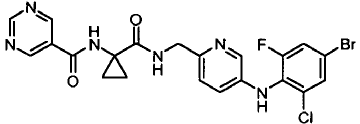 |
| (326) | 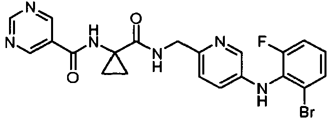 |
| (327) | 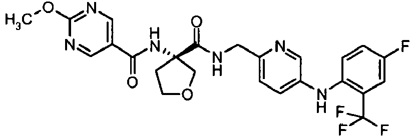 |
| (328) | 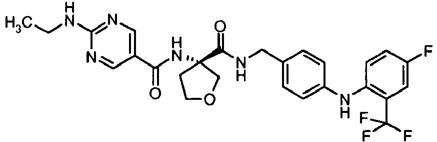 |
| (329) | 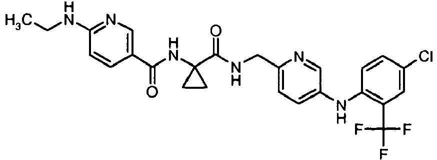 |
| (330) | 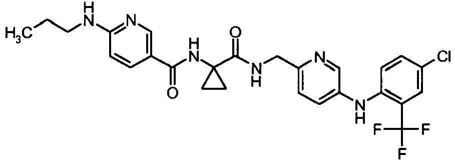 |
| (331) | 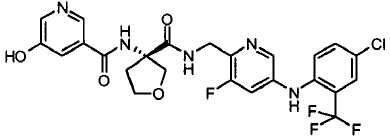 |
| (332) | 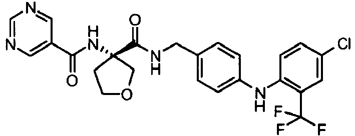 |
| (333) | 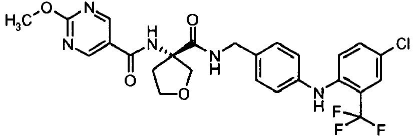 |
| (334) | 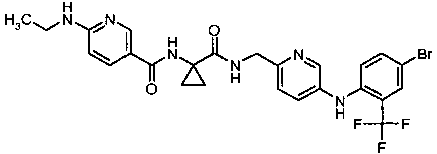 |
| (335) | 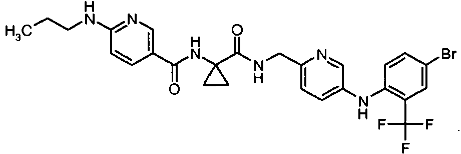 |
| (336) | 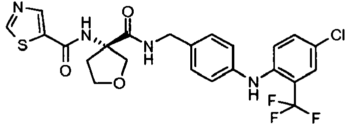 |
| (337) | 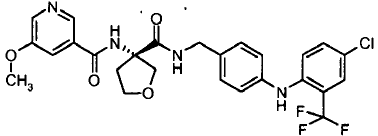 |
| (338) | 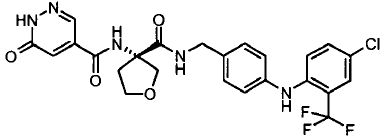 |
| (339) | 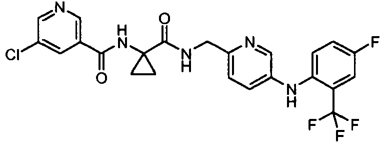 |
| (340) | 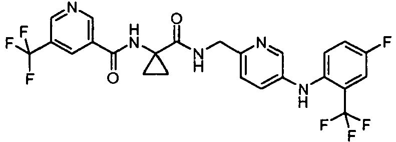 |
| (341) | 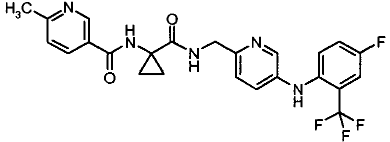 |
| (342) | 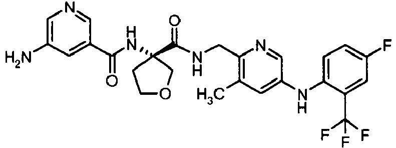 |
| (343) | 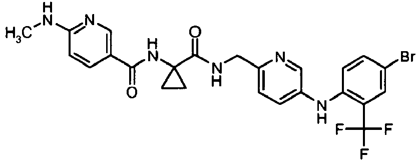 |
| (344) | 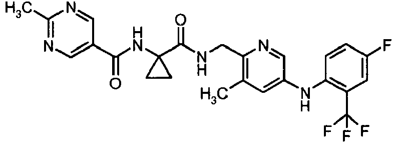 |
| (345) | 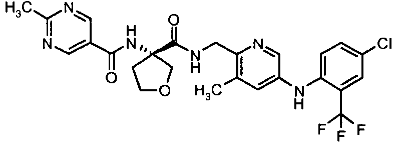 |
| (346) | 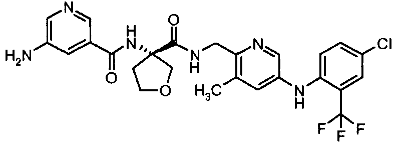 |
| (347) | 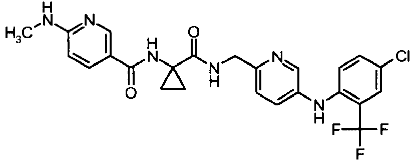 |
| (348) | 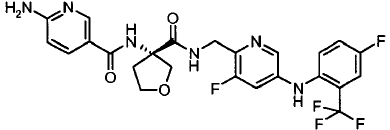 |
| (349) | 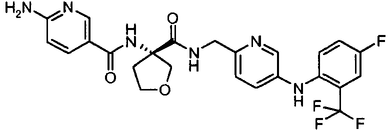 |
| (350) | 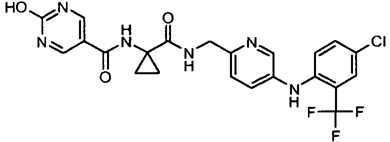 |
| (351) | 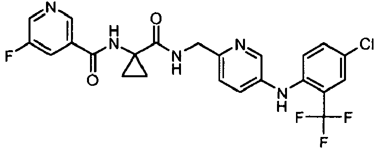 |
| (352) | 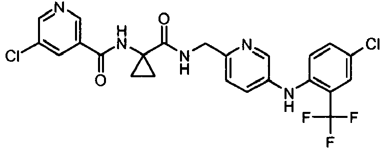 |
| (353) | 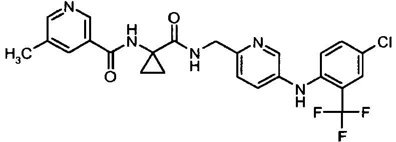 |
| (354) | 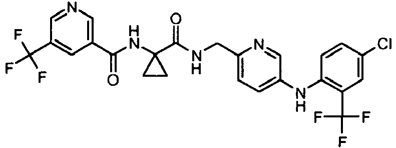 |
| (355) | 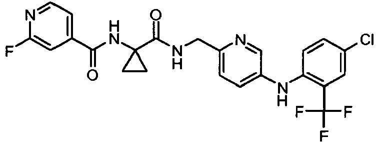 |
| (356) | 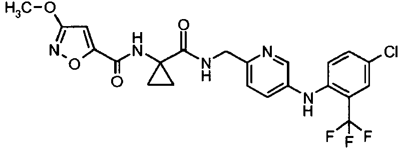 |
| (357) | 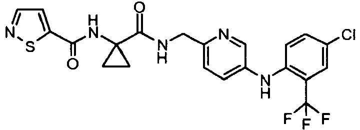 |
| (358) | 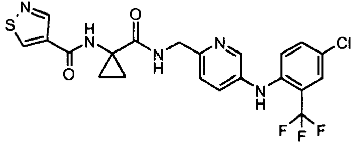 |
| (359) | 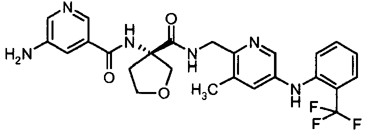 |
| (360) | 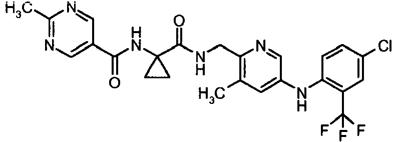 |
| (361) | 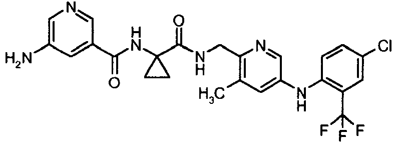 |
| (362) | 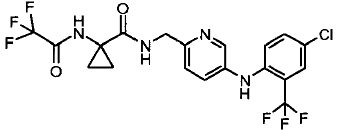 |
| (363) | 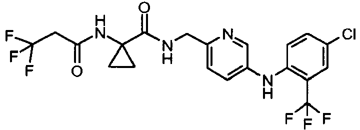 |
| (364) | 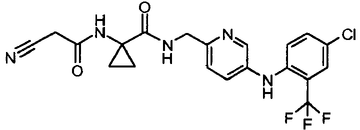 |
| (365) | 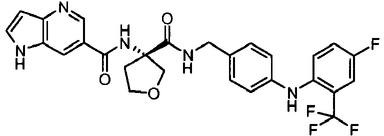 |
| (366) | 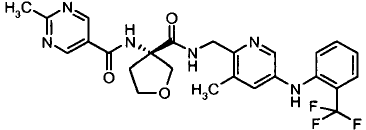 |
| (367) | 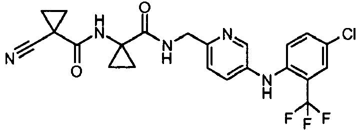 |
| (368) | 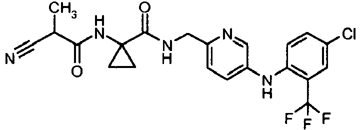 |
| (369) | 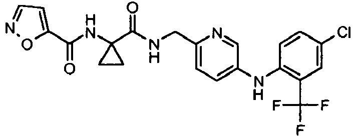 |
| (370) | 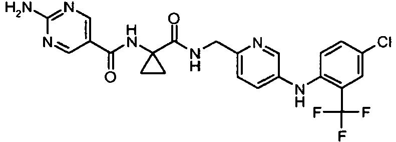 |
| (371) | 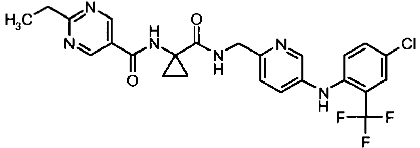 |
| (372) | 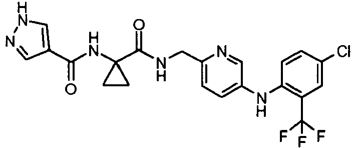 |
| (373) | 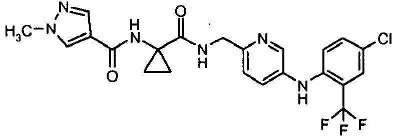 |
| (374) | 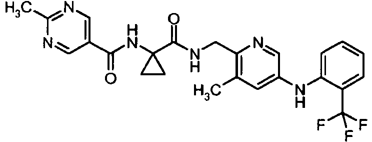 |
| (375) | 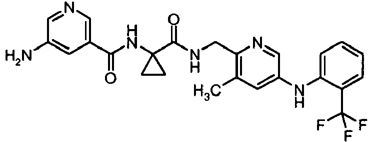 |
| (376) | 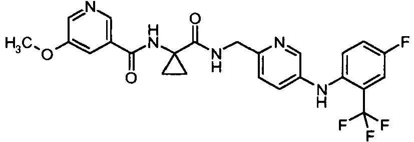 |
| (377) | 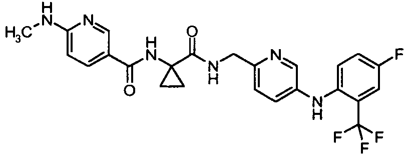 |
| (378) | 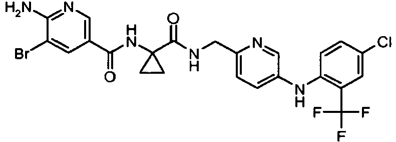 |
| (379) | 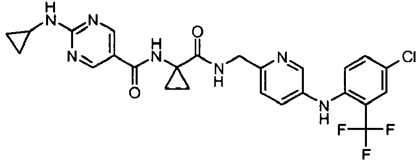 |
| (380) | 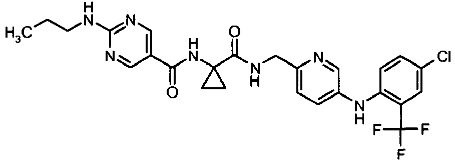 |
| (381) | 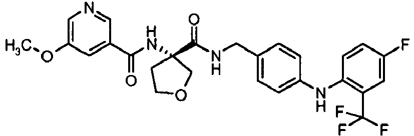 |
| (382) | 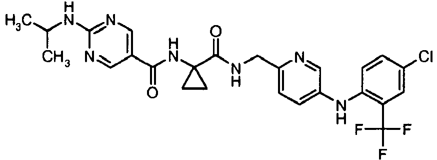 |
| (383) | 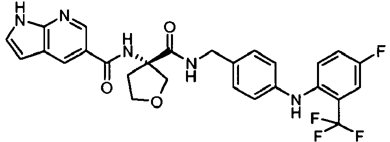 |
| (384) | 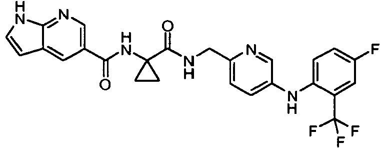 |
| (385) | 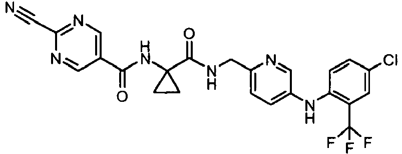 |
| (386) | 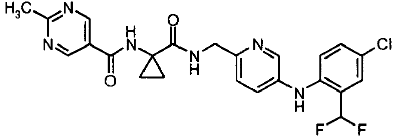 |
| (387) | 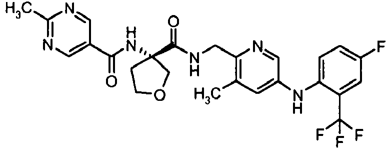 |
| (388) | 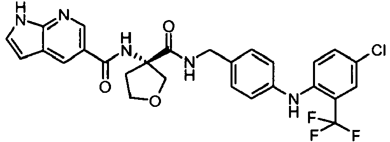 |
| (389) | 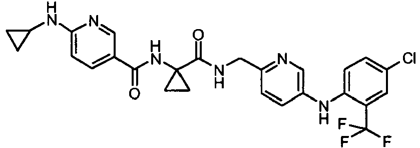 |
| (390) | 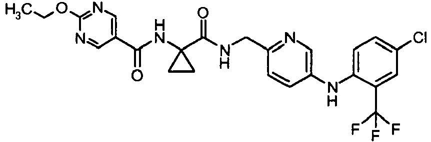 |
| (391) | 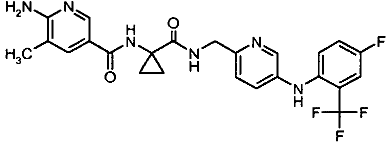 |
| (392) | 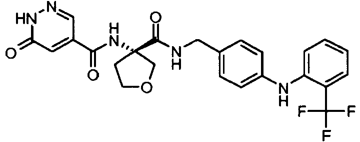 |
| (393) | 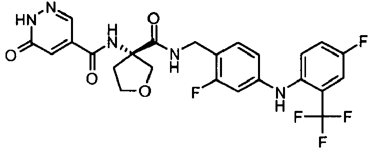 |
| (394) | 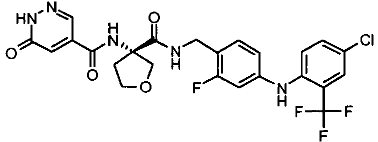 |
| (395) | 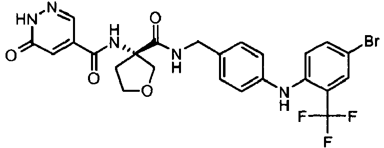 |
| (396) | 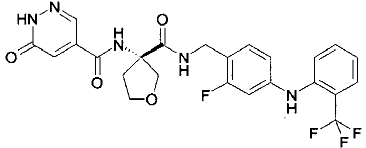 |
deren Enantiomere, deren Diastereomere, deren Gemische und deren Salze.

29. Physiologisch verträgliche Salze der Verbindungen nach einem der Ansprüche 1 bis 28 mit anorganischen oder organischen Säuren oder Basen.

30. Arzneimittel, enthaltend eine Verbindung nach mindestens einem der Ansprüche 1 bis 28 oder ein physiologisch verträgliches Salz gemäß Anspruch 29 neben gegebenenfalls einem oder mehreren inerten Trägerstoffen und/oder Verdünnungsmitteln.

31. Verbindung der allgemeinen Formel I gemäß einem der Ansprüche 1, 2, 3, 4, 5, 6, 7, 8, 9, 10, 11, 12, 13, 14, 15, 16, 17, 18, 19, 20, 21, 22, 23, 24, 25, 26, 27, 28 oder 29 zur verwendung als Arzneimittel.

32. Verbindung der allgemeinen Formel I gemäß einem der Ansprüche 1, 2, 3, 4, 5, 6, 7, 8, 9, 10, 11, 12, 13, 14, 15, 16, 17, 18, 19, 20, 21, 22, 23, 24, 25, 26, 27, 28 oder 29 zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen.

33. Verwendung einer Verbindung nach mindestens einem der Ansprüche 1 bis 24 zur Herstellung eines Arzneimittels zur akuten und prophylaktischen Behandlung von Osteoarthritis.

34. Verfahren zur Herstellung eines Arzneimittels gemäß Anspruch 30, **dadurch gekennzeichnet, dass** auf nichtchemischem Weg eine Verbindung nach mindestens einem der Ansprüche 1 bis 29 in einen oder mehrere inerte Trägerstoffe und/oder Verdünnungsmittel eingearbeitet wird.

## Claims

1. Compounds of general formula I 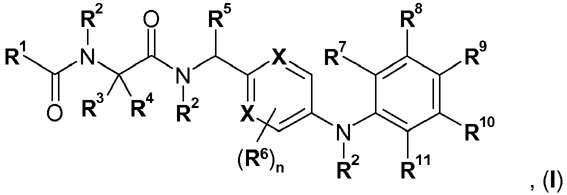 wherein
**n** denotes one of the numbers 0, 1 or 2,
**R¹** denotes
(a) a C₁₋₆-alkyl group optionally substituted by a group **R^{1.1},**
(b) a C₁₋₃-alkyl group wherein each methylene group may be substituted by 1 or 2 fluorine atoms and each methyl group may be substituted by 1, 2 or 3 fluorine atoms,
(c) a substituted C₃₋₆-cycloalkyl group optionally substituted by a group **R^{1.2}** wherein a -CH₂- unit may be replaced by a -C(O)- group,
(d) an aryl-C₀₋₂-alkylene group optionally substituted by 1, 2 or 3 groups **R^{1.3},**
(e) a five-membered heteroaryl-C₀₋₂-alkylene group optionally substituted by 1, 2 or 3 groups **R^{1.4},** which contains at least one N, O or S atom and which optionally additionally contains one, two or three further N-atoms and which may additionally be benzo-condensed,
(f) a six-membered heteroaryl-C₀₋₂-alkylene group optionally substituted by 1 or 2 groups **R^{1.4},** which contains one, two or three N-atoms and which may additionally be benzo-condensed,
(g) a nine- or ten-membered heteroaryl group optionally substituted by 1 or 2 groups **R^{1.4}** substituted, which contains one, two or three N-atoms,
(h) a 5- or 6-membered heterocyclic group optionally substituted by 1 or 2 groups **R^{1.4}**, in which a -CH₂- unit may be replaced by a -C(O)- group,
(i) -O-**R^{1.1.1}**,
(j) -N**R^{1.1.3}R^{1.1.4}** or
(k) -C(=N**R^{1.5}**)-CN,
**R^{1.1}** denotes halogen, -NO₂, -CN, C₃₋₆-cycloalkyl, -O**R^{1.1.1}**, -S**R^{1.1.1}**, -C(O)**R^{1.1.1}**, -S(O)₂-R^{1.1.2}, -O-S(O)₂-R^{1.1.1}, -CO₂R^{1.1.1}, -O-C(O)-R^{1.1.1}, -N**R^{1.1.3}R^{1.1.4}**, -N**R^{1.1,3}**-C(O)-**R^{1.1.1}**, -N**R^{1.1.3}**-C(O)-**R^{1.1.1}**, -N**R^{1.1,3}**-CO₂-**R^{1.1.1}** or -C(O)-N**R^{1.1.3}R^{1.1.4}**,
**R^{1.1.1}** denotes
(a) H,
(b) C₁₋₄-alkyl,
(c) a C₁₋₃-alkyl group wherein each methylene group may be substituted by 1 or 2 fluorine atoms and each methyl group may be substituted by 1, 2 or 3 fluorine atoms,
(d) a phenyl group optionally substituted by 1, 2 or 3 groups **R^{1.1.1.1},**
(e) C₃₋₆-cycloalkyl or
(f) a pyridyl group optionally substituted by 1, 2 or 3 groups **R^{1.1.1.2},**
**R^{1.1.1.1}** independently of one another denote
(a) halogen, -NO₂, -CN, -OH, -O-C₁₋₄-alkyl, C₃₋₆-cycloalkyl, C₁₋₄-alkyl or
(b) a C₁₋₃-alkyl group wherein each methylene group may be substituted by 1 or 2 fluorine atoms and each methyl group may be substituted by 1, 2 or 3 fluorine atoms,
**R^{1.1.1.2}** independently of one another denote halogen or C₁₋₄-alkyl,
**R^{1.1.2}** denotes
(a) C₁₋₄-alkyl,
(b) a C₁₋₃-alkyl group wherein each methylene group may be substituted by 1 or 2 fluorine atoms and each methyl group may be substituted by 1, 2 or 3 fluorine atoms,
(c) -O-C₁₋₄-alkyl or
(d) a phenyl group optionally substituted by 1, 2 or 3 groups **R^{1.1.1.1} ,**
**R^{1.1.3}** ,
**R^{1.1.4}** independently of one another denote
(a) H,
(b) a C₁₋₄-alkyl group optionally substituted by 1, 2 or 3 groups **R^{1.1.4.1},**
(c) a phenyl group optionally substituted by 1, 2 or 3 groups **R^{1.1.1.1},**
(d) C₃₋₆-cycloalkyl, or
**R^{1.1.3}** and **R^{1.1.4}** together with the N atom to which they are attached form a 5- or 6-membered heterocyclic ring, which may additionally contain a further heteroatom selected from N, O and S, or
**R^{1.1.3}** and **R^{1.1.4}** together with the N atom to which they are attached, form a cyclic imide,
**R^{1.1.4.1}** independently of one another denote halogen, -NH₂, -NH(C₁₋₄-alkyl), -N(C₁₋₄-alkyl)₂ or -SO₂-**R^{1.1.2}**,
**R^{1.2}** denotes halogen, -NO₂, -CN, OH, -O-CH₃ or phenyl,
**R^{1.3}** denotes
(a) halogen, -NO₂, -CN, -O**R^{1.1.1}**, -S**R^{1.1.1}**, -CO₂**R^{1.1.1}**, C₁₋₆-alkyl or
(b) a C₁₋₃-alkyl group wherein each methylene group may be substituted by 1 or 2 fluorine atoms and each methyl group may be substituted by 1, 2 or 3 fluorine atoms,
**R^{1.4}** independently of one another denote
(a) halogen, -NO₂, -CN, -O**R^{1.1.1}**, -S**R^{1.1.1}**, -S(O)-**R^{1.1.2}**, -S(O)₂**R^{1.1.2}**, -N**R^{1.1.3}R^{1.1.4}**, -N(**R^{1.4.1}**)-C(O)-C₁₋₄-alkyl, C₁₋₆-alkyl,
(b) a C₁₋₃-alkyl group wherein each methylene group may be substituted by 1 or 2 fluorine atoms and each methyl group may be substituted by 1, 2 or 3 fluorine atoms, or
(c) an oxo group,
**R^{1.4.1}** denotes H or C₁₋₄-alkyl,
**R^{1.5}** denotes -OH or -O-C₁₋₃-alkyl,
**R**² denotes
(a) H,
(b) C₁₋₄-alkyl,
(c) C₁₋₄-alkyl-C(O)-,
**R³** and **R⁴** together with the carbon atom to which they are bound denote a C₃₋₆-cycloalkylene group optionally substituted by a group **R^{3.1}** wherein a -CH₂- unit may be replaced by a heteroatom O, N, S or by a group CO, SO or SO₂,
**R^{3.1}** denotes H, -OH,
**R⁵** denotes
(a) H,
(b) C₁₋₄-alkyl,
(c) a C₁₋₃-alkyl group wherein each methylene group may be substituted by 1 or 2 fluorine atoms and each methyl group may be substituted by 1, 2 or 3 fluorine atoms,
**R⁶** independently of one another denote
(a) H, halogen, -CN, -OH, C₁₋₆-alkyl, C₃₋₇-cycloalkyl, -O-C₁₋₄-alkyl, -O-CF₃, -O-C₃₋₆-cycloalkyl, -N(C₁₋₃-alkyl)₂, -C(O)-NH₂, -(SO₂)NH₂, -SO₂-C₁₋₃-alkyl, or
(b) a C₁₋₃-alkyl group wherein each methylene group may be substituted by 1 or 2 fluorine atoms and each methyl group may be substituted by 1, 2 or 3 fluorine atoms,
**R**⁷ denotes
(a) H, halogen, -CN, -OH,
(b) C₁₋₆-alkyl,
(c) C₁₋₃-alkyl or -O-C₁₋₃-alkyl, wherein each methylene group may be substituted by 1 or 2 fluorine atoms and each methyl group may be substituted by 1, 2 or 3 fluorine atoms,
(d) C₃₋₇-cycloalkyl,
(e) -O-C₁₋₆-alkyl,
(f) -O-C₃₋₇-cycloalkyl,
(g) -NH₂, -NH(C₁₋₃-alkyl), -N(C₁₋₃-alkyl)₂,
(h) -C(O)-**R^{7.1}**,
(i) -S-C₁₋₄-alkyl, -SO₂-**R^{7.2}**,
(j) a five-membered heteroaryl group optionally substituted by one or two C₁₋₃-alkyl groups which is selected from among pyrrolyl, oxazolyl, isoxazolyl, oxadiazolyl, thiazolyl, isothiazolyl, thiadiazolyl, imidazolyl, pyrazolyl, triazolyl and tetrazolyl, or
(k) a six-membered heteroaryl group optionally substituted by one or two C₁₋₃-alkyl groups which is selected from among pyridyl, pyrimidinyl, pyrazinyl, pyridazinyl and triazinyl,
**R^{7.1}** denotes -NH₂, -NH(C₁₋₆-alkyl),-N(C₁₋₆-alkyl)₂, *N*-acetidinyl, *N*-pyrrolidinyl, *N*-piperidinyl, *N*-morpholinyl, -OH, -O-C₁₋₈-alkyl or -O-C₃₋₇cycloalkyl,
**R^{7.2}** denotes -NH₂, -NH(C₁₋₆-alkyl),-N(C₁₋₆-alkyl)₂, *N*-acetidinyl, *N*-pyrrolidinyl, *N*-piperidinyl or *N*-morpholinyl and
**R**⁸ denotes H, halogen, C₁₋₄-alkyl,
**R**⁹ denotes
(a) H, halogen, -CN, -OH,
(b) C₁₋₆-alkyl,
(c) C₁₋₃-alkyl or -O-C₁₋₃-alkyl, wherein each methylene group may be substituted by 1 or 2 fluorine atoms and each methyl group may be substituted by 1, 2 or 3 fluorine atoms,
(d) C₃₋₇-cycloalkyl,
(e) C₂₋₄-alkynyl,
(f) -O-C₁₋₆-alkyl,
(g) -O-C₃₋₇-cyloalkyl,
(h) -NH₂, -NH(C₁₋₃-alkyl), -N(C₁₋₃-alkyl)₂,
(i) **-C(O)-R^{9.1},**
(j) -S-C₁₋₄-alkyl, -SO-C₁₋₄-alkyl, -SO₂-C₁₋₄-alkyl,
**R^{9.1}** denotes -NH₂, -NH(C₁₋₆-alkyl),-N(C₁₋₆-alkyl)₂, *N*-acetidinyl, *N*-pyrrolidinyl, *N*-piperidinyl, *N*-morpholinyl, -OH, -O-C₁₋₈-alkyl or -O-C₃₋₇-cycloalkyl,
**R¹⁰** denotes H, halogen, C₁₋₄-alkyl,
**R¹¹** denotes
(a) H, halogen, -CN, -OH,
(b) C₁₋₆-alkyl,
(c) C₁₋₃-alkyl or -O-C₁₋₃-alkyl, wherein each methylene group may be substituted by 1 or 2 fluorine atoms and each methyl group may be substituted by 1, 2 or 3 fluorine atoms,
(d) C₃₋₇-cycloalkyl,
(e) -O-C₁₋₆-alkyl,
(f) -O-C₃₋₇-cycloalkyl,
(g) -NH₂, -NH(C₁₋₃-alkyl), -N(C₁₋₃-alkyl)₂,
(h) -C(O)-**R^{11.1}**,
(i) -S-C₁₋₃-alkyl, -SO₂-**R^{11.2}**,
(j) a five-membered heteroaryl group optionally substituted by one or two C₁₋₃-alkyl groups which is selected from among pyrrolyl, oxazolyl, isoxazolyl, oxadiazolyl, thiazolyl, isothiazolyl, thiadiazolyl, imidazolyl, pyrazolyl, triazolyl and tetrazolyl, or
(k) a six-membered heteroaryl group optionally substituted by one or two C₁₋₃-alkyl groups which is selected from among pyridyl, pyrimidinyl, pyrazinyl, pyridazinyl and triazinyl,
**R^{11.1}** denotes -NH₂, -NH(C₁₋₆-alkyl),-N(C₁₋₆-alkyl)₂, *N*-acetidinyl, *N*-pyrrolidinyl, *N*-piperidinyl, *N*-morpholinyl, -OH, -O-C₁₋₈-alkyl or -O-C₃₋₇-cycloalkyl,
**R^{11.2}** denotes -NH₂, -NH(C₁₋₆-alkyl),-N(C₁₋₆-alkyl)₂, *N*-acetidinyl, *N*-pyrrolidinyl, *N*-piperidinyl or *N*-morpholinyl and
**X** independently of one another denote C-**R⁶** or N,
the enantiomers, the diastereomers, the mixtures and the salts thereof.

2. Compounds of general formula **I** according to claim 1, wherein **R², R³, R⁴, R⁵, R⁶, R⁷ R⁸, R⁹, R¹⁰, R¹¹, n** and **X** are defined as in claim 1 and
R¹ denotes
(a) a C₁₋₆-alkyl group optionally substituted by a group **R^{1.1},**
(b) a C₁₋₃-alkyl group wherein each methylene group may be substituted by 1 or 2 fluorine atoms and each methyl group may be substituted by 1, 2 or 3 fluorine atoms,
(c) a C₃₋₆-cycloalkyl group optionally substituted by a group **R^{1.2}** wherein a -CH₂-unit may be replaced by a -C(O)- group,
(d) a phenyl group optionally substituted by 1, 2 or 3 groups **R^{1.3},**
(e) a five-membered heteroaryl group optionally substituted by 1, 2 or 3 groups **R^{1.4},** which contains at least one N, O or S atom and which optionally additionally contains one, two or three further N-atoms,
(f) a six-membered heteroaryl group optionally substituted by 1 or 2 groups **R^{1.4},** which contains one, two or three N-atoms,
(g) a nine- or ten-membered heteroaryl group optionally substituted by 1 or 2 groups **R^{1.4},** which contains one, two or three N-atoms,
(h) a 5- or 6-membered heterocyclic group optionally substituted by 1 or 2 groups **R^{1.4},** in which a -CH₂- unit may be replaced by a -C(O)- group,
(i) -O-**R^{1.1.1}** or
(j) -N**R^{1.1.3}R^{1.1.4}**,
**R^{1.1}** denotes -CN, C₃₋₆-cycloalkyl, -O**R^{1.1.1}**, -N**R^{1.1.3}R^{1.1.4}**,
**R^{1.1.1}** denotes
(a) H,
(b) C₁₋₄-alkyl,
(c) a C₁₋₃-alkyl group wherein each methylene group may be substituted by **1** or 2 fluorine atoms and each methyl group may be substituted by 1, 2 or 3 fluorine atoms,
**R^{1.1.3}** ,
**R^{1.1.4}** independently of one another denote
(a) H,
(b) C₁₋₄-alkyl,
(c) C₃₋₆-cycloalkyl, or
**R^{1.1.3}** and **R^{1.1.4}** together with the N atom to which they are attached form a 5- or 6-membered heterocyclic ring, which may additionally contain a further heteroatom selected from N, O and S, or
**R^{1.2}** denotes halogen, -NO₂, -CN, -OH, -O-CH₃ or phenyl,
**R^{1.3}** independently of one another denote
(a) halogen, -NO₂, -CN, -O**R^{1.1.1}**, C₁₋₆-alkyl or
(b) a C₁₋₃-alkyl group wherein each methylene group may be substituted by 1 or 2 fluorine atoms and each methyl group may be substituted by 1, 2 or 3 fluorine atoms,
**R**^{**1.**4} independently of one another denote
(a) halogen, -NO₂, -CN, -O**R^{1.1.1},** -N**R^{1.1.3}R^{1.1.4},** -N(**R^{1.4.1}**)-C(O)-C₁₋₄-ₐₗₖyl, C₁₋₆-alkyl, or
(b) a C₁₋₃-alkyl group wherein each methylene group may be substituted by 1 or 2 fluorine atoms and each methyl group may be substituted by 1, 2 or 3 fluorine atoms, and
**R^{1.4.1}** denotes H or C₁₋₄-alkyl,
the enantiomers, the diastereomers, the mixtures and the salts thereof.

3. Compounds of general formula **I** according to claim 1, wherein **R², R³, R⁴, R⁵, R⁶, R⁷, R⁸, R⁹, R¹⁰, R¹¹, n** and **X** are defined as in claim 1 and
**R¹** denotes
(a) a C₁₋₆-alkyl group optionally substituted by a group **R^{1.1},**
(b) a phenyl group optionally substituted by 1, 2 or 3 groups **R^{1.3},**
(c) a five-membered heteroaryl group optionally substituted by 1, 2 or 3 groups **R^{1.4},** which contains at least one N, O or S atom and which optionally additionally contains one, two or three further N-atoms,
(d) a six-membered heteroaryl group optionally substituted by 1 or 2 groups **R^{1.4},** which contains one, two or three N-atoms,
(e) a nine- or ten-membered heteroaryl group optionally substituted by 1 or 2 groups **R^{1.4},** which contains one, two or three N-atoms,
(f) a 5- or 6-membered heterocyclic group optionally substituted by 1 or 2 groups **R^{1.4},** in which a -CH₂- unit may be replaced by a -C(O)- group,
**R^{1.1}** denotes -CN, C₃₋₆-cycloalkyl, -OH, -OCH₃, -NH₂, -NHCH₃, -N(CH₃)₂,
**R^{1.3}** independently of one another denote
(a) F, Cl, Br, -OH, -OCH₃, C₁₋₆-alkyl or
(b) a C₁₋₃-alkyl group wherein each methylene group may be substituted by 1 or 2 fluorine atoms and each methyl group may be substituted by 1, 2 or 3 fluorine atoms, and
**R^{1.4}** independently of one another denote
(a) F, Cl, Br, -OH, -OCH₃, -NH₂, -NHCH₃, -N(CH₃)₂, -NHC₂₋₃-alkyl, -N(C₂₋₃-alkyl)₂, -NH-C(O)-C₁₋₄-alkyl, C₁₋₆-alkyl, or
(b) a C₁₋₃-alkyl group wherein each methylene group may be substituted by 1 or 2 fluorine atoms and each methyl group may be substituted by 1, 2 or 3 fluorine atoms,
the enantiomers, the diastereomers, the mixtures and the salts thereof, particularly the physiologically acceptable salts thereof with organic or inorganic acids or bases.

4. Compounds of general formula **I** according to claim 1, wherein **R², R³, R⁴, R⁵, R⁶, R⁷, R⁸, R⁹, R¹⁰, R¹¹, n** and **X** are defined as in claim 1 and
**R¹** denotes
(a) a C₁₋₆-alkyl group optionally substituted by a group **R^{1.1},**
(b) a phenyl group optionally substituted by 1, 2 or 3 groups **R^{1.3},**
(c) a five-membered heteroaryl group optionally substituted by 1, 2 or 3 groups **R^{1.4},** which is selected from among 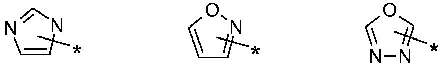 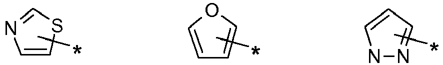 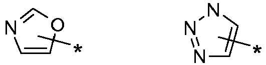
(d) a six-membered heteroaryl group optionally substituted by 1 or 2 groups **R^{1.4}** which is selected from among 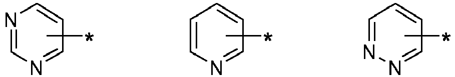 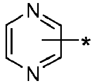
(e) a nine-membered heteroaryl group optionally substituted by 1 or 2 groups **R^{1.4}**, which is selected from among 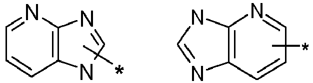
(f) a 5- or 6-membered heterocyclic group optionally substituted by 1 or 2 groups **R^{1.4},** which is selected from among 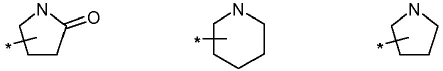 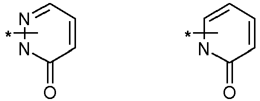
**R^{1.1}** denotes -CN, cyclopropyl, -OH, -OCH₃, -NH₂, -NHCH₃, -N(CH₃)₂, **R^{1.3}** independently of one another denotes
(a) F, Cl, Br, -OH, -OCH₃, -OCF₃, C₁₋₄-alkyl or
(b) a C₁₋₃-alkyl group wherein each methylene group may be substituted by 1 or 2 fluorine atoms and each methyl group may be substituted by 1, 2 or 3 fluorine atoms, and
**R^{1.4}** independently of one another denotes
(a) F, Cl, Br, -OH, -OCH₃, -OCF₃, -NH₂, -NH-C₁₋₄-alkyl, -N(C₁₋₄-alkyl)₂, -NH-C(O)-C₁₋₄-alkyl, C₁₋₆-alkyl, or
(b) a C₁₋₃-alkyl group wherein each methylene group may be substituted by 1 or 2 fluorine atoms and each methyl group may be substituted by 1, 2 or 3 fluorine atoms,
the enantiomers, the diastereomers, the mixtures and the salts thereof.

5. Compounds of general formula **I** according to claim 1, wherein **R², R³, R⁴, R⁵, R⁶, R⁷, R⁸, R⁹, R¹⁰, R¹¹, n** and **X** are defined as in claim 1 and
**R¹** is selected from among 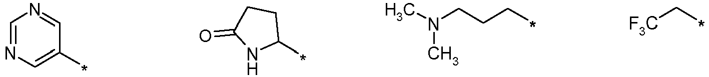 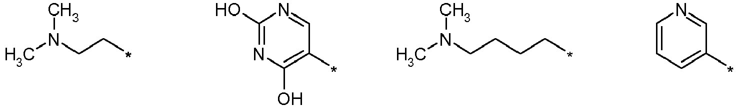 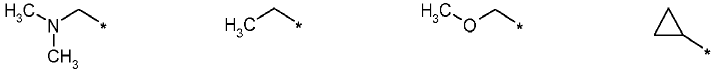 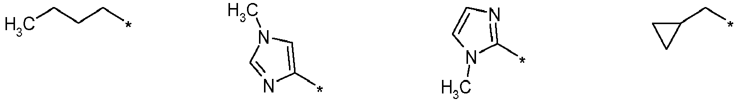 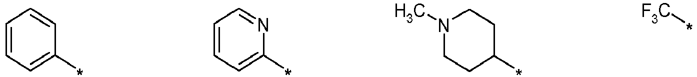 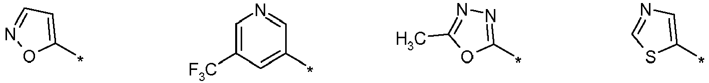 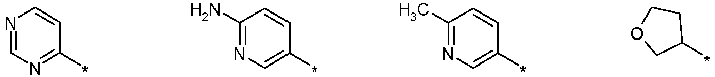 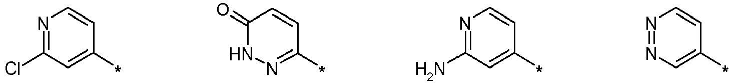 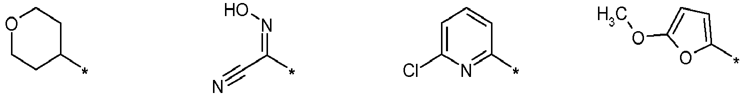 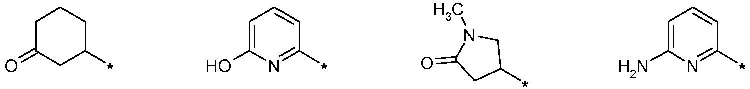 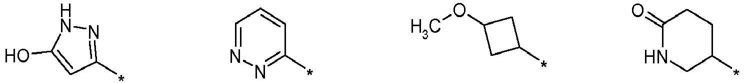 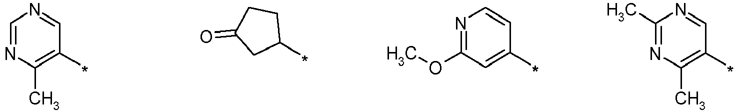 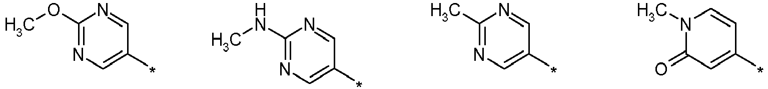 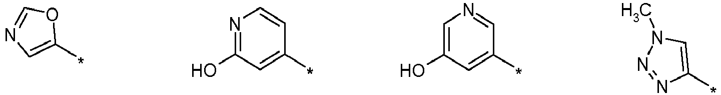 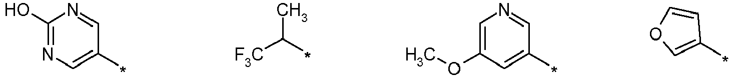 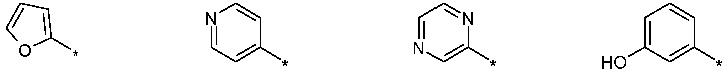 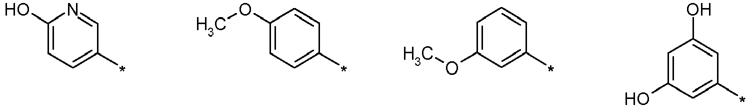 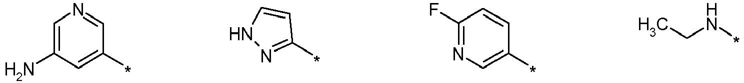 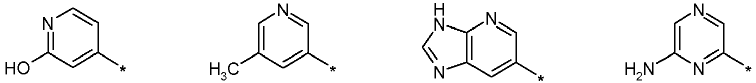 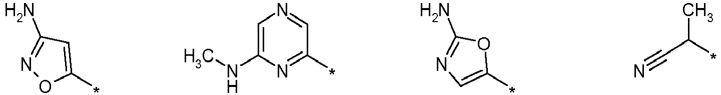 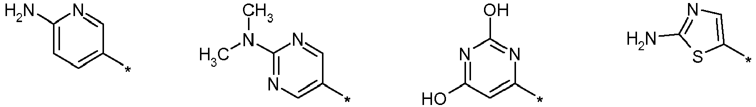 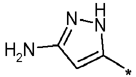 the enantiomers, the diastereomers, the mixtures and the salts thereof.

6. Compounds of general formula **I** according to claim 1, wherein R**², R³, R⁴, R⁵, R⁶, R⁷, R⁸, R⁹, R¹⁰, R¹¹, n** and **X** are defined as in claim 1 and
**R¹** is selected from among 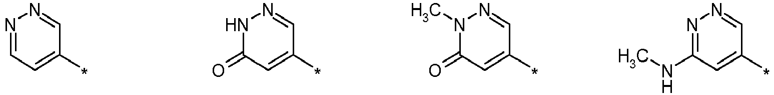 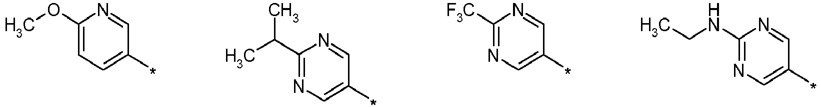 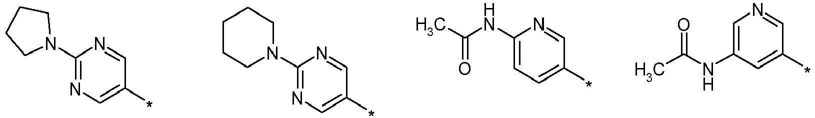 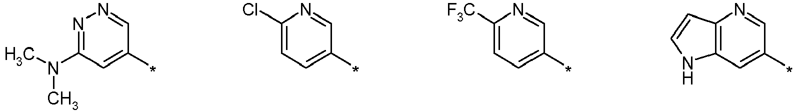 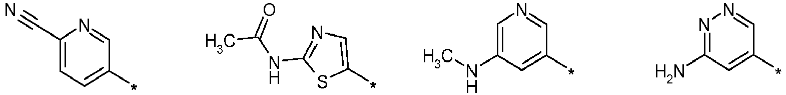 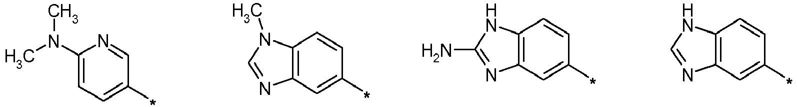 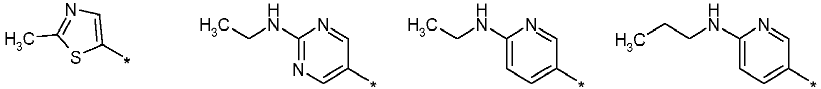 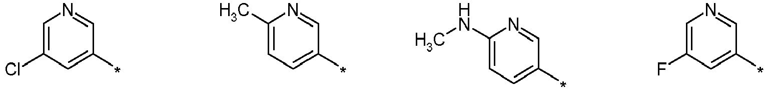 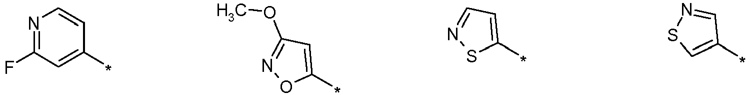 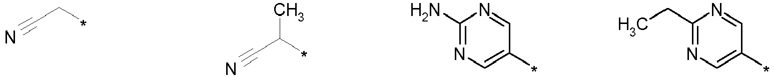 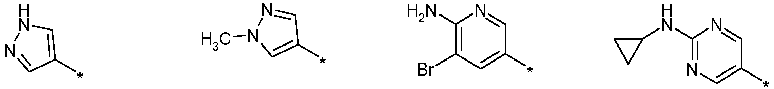 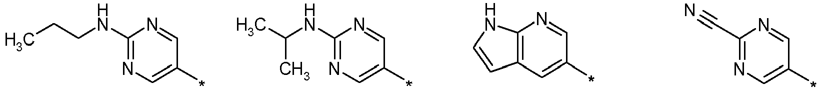 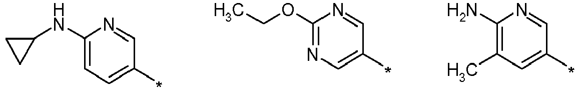 the enantiomers, the diastereomers, the mixtures and the salts thereof, particularly the physiologically acceptable salts thereof with organic or inorganic acids or bases.

7. Compounds of general formula **I** according to claim 1, wherein **R¹** is defined as in claim 1, 2, 3, 4, 5 or 6 and
**n** denotes one of the numbers 0, 1 or 2,
**R²** denotes
(a) H,
(b) C₁₋₄-alkyl,
**R³** and **R⁴** together with the carbon atom to which they are bound denote a C₃₋₆-cycloalkylene group optionally substituted by a group **R^{3.1}** wherein a -CH₂- unit may be replaced by a heteroatom O, N, S or by a group CO, SO or SO₂,
**R^{3.1}** denotes H, -OH,
**R⁵** denotes
(a) H,
(b) C₁₋₄-alkyl,
(c) a C₁₋₃-alkyl group wherein each methylene group may be substituted by 1 or 2 fluorine atoms and each methyl group may be substituted by 1, 2 or 3 fluorine atoms,
**R⁶** independently of one another denotes
(a) H, halogen, -CN, -OH, C₁₋₆-alkyl, C₃₋₇-cycloalkyl, -O-C₁₋₄-alkyl, -O-CF₃, -O-C₃₋₆-cycloalkyl, -N(C₁₋₃-alkyl)₂, -C(O)-NH₂, -(SO₂)NH₂, -SO₂-C₁₋₃-alkyl, or
(b) a C₁₋₃-alkyl group wherein each methylene group may be substituted by 1 or 2 fluorine atoms and each methyl group may be substituted by 1, 2 or 3 fluorine atoms,
**R**⁷ denotes
(a) H, halogen, -CN, -OH,
(b) C₁₋₆-alkyl,
(c) C₁₋₃-alkyl or -O-C₁₋₃-alkyl, wherein each methylene group may be substituted by 1 or 2 fluorine atoms and each methyl group may be substituted by 1, 2 or 3 fluorine atoms,
(d) C₃₋₇-cycloalkyl,
(e) -O-C₁₋₆-alkyl,
(f) -O-C₃₋₇-cycloalkyl,
(g) -NH₂, -NH(C₁₋₃-alkyl), -N(C₁₋₃-alkyl)₂,
(h) -C(O)-**R^{7.1}**,
(i) -S-C₁₋₄-alkyl,
**R^{7.1}** denotes -NH₂, , -OH, -O-C₁₋₈-alkyl,
**R**⁸ denotes H, halogen, C₁₋₄-alkyl,
**R⁹** denotes
(a) H, halogen, -CN, -OH,
(b) C₁₋₆-alkyl,
(c) C₁₋₃-alkyl or -O-C₁₋₃-alkyl, wherein each methylene group may be substituted by 1 or 2 fluorine atoms and each methyl group may be substituted by 1, 2 or 3 fluorine atoms,
(d) C₃₋₇-cycloalkyl,
(e) C₂₋₄-alkynyl,
(f) -O-C₁₋₆-alkyl,
(g) -O-C₃₋₇-cycloalkyl,
(h) -NH₂, -NH(C₁₋₃-alkyl), -N(C₁₋₃-alkyl)₂,
(i) -C(O)-**R^{9.1},**
(j) -S-C₁₋₄-alkyl, -SO-C₁₋₄-alkyl, -SO₂-C₁₋₄-alkyl,
**R^{9.1}** denotes -NH₂, -OH, -O-C₁₋₈-alkyl,
**R¹⁰** denotes H, halogen, C₁₋₄-alkyl,
**R¹¹** denotes
(a) H, halogen, -CN, -OH,
(b) C₁₋₆-alkyl,
(c) C₁₋₃-alkyl or -O-C₁₋₃-alkyl, wherein each methylene group may be substituted by 1 or 2 fluorine atoms and each methyl group may be substituted by 1, 2 or 3 fluorine atoms,
(d) C₃₋₇-cycloalkyl,
(e) -O-C₁₋₆-alkyl,
(f) -O-C₃₋₇-cycloalkyl,
(g) -NH₂, -NH(C₁₋₃-alkyl), -N(C₁₋₃-alkyl)₂,
(h) -C(O)-**R^{11.1}**,
(i) -S-C₁₋₃-alkyl,
**R^{11.1}** denotes -NH₂, -OH, -O-C₁₋₈-alkyl, and
**X** independently of one another represent C-**R⁶** or N,
the enantiomers, the diastereomers, the mixtures and the salts thereof.

8. Compounds of general formula **I** according to claim 1, wherein **R¹, R³, R⁴, R⁵, R⁶, R⁷, R⁸, R⁹, R¹⁰, R¹¹, n** and **X** are defined as in claim 1, 2, 3, 4, 5, 6 or 7 and
**R**² denotes H or CH₃,
the enantiomers, the diastereomers, the mixtures and the salts thereof.

9. Compounds of general formula **I** according to claim 1, wherein **R¹, R³, R⁴, R⁵, R⁶, R⁷, R⁸, R⁹, R¹⁰, R¹¹, n** and **X** are defined as in claim 1, 2, 3, 4, 5, 6 or 7 and
**R**² denotes H,
the enantiomers, the diastereomers, the mixtures and the salts thereof.

10. Compounds of general formula **I** according to claim 1, wherein **R¹, R², R⁵, R⁶, R⁷, R⁸, R⁹, R¹⁰, R¹¹, n** and **X** are defined as in claim 1, 2, 3, 4, 5, 6, 7, 8 or 9 and
**R³** and **R⁴** together with the carbon atom to which they are bonded denote a C₃₋₆-cycloalkylene group wherein a -CH₂- unit may be replaced by an oxygen atom,
the enantiomers, the diastereomers, the mixtures and the salts thereof.

11. Compounds of general formula **I** according to claim 1, wherein **R¹, R², R⁵, R⁶, R⁷, R⁸, R⁹, R¹⁰, R¹¹, n** and **X** are defined as in claim 1, 2, 3, 4, 5, 6, 7, 8 or 9 and **R³** and **R⁴** together with the carbon atom to which they are bonded denote a group selected from 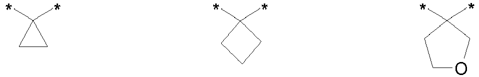 the enantiomers, the diastereomers, the mixtures and the salts thereof.

12. Compounds of general formula **I** according to claim 1, wherein **R¹, R², R³, R⁴, R⁶, R⁷, R⁸, R⁹, R¹⁰, R¹¹, n** and **X** are defined as in claim 1, 2, 3, 4, 5, 6, 7, 8, 9, 10 or 11 and
**R⁵** denotes H or CH₃,
the enantiomers, the diastereomers, the mixtures and the salts thereof.

13. Compounds of general formula **I** according to claim 1, wherein **R¹, R², R³, R⁴, R⁵, R⁷, R⁸, R⁹, R¹⁰, R¹¹, n** and **X** are defined as in claim 1, 2, 3, 4, 5, 6, 7, 8, 9, 10, 11 or 12 and
**R⁶** denotes H, F, Cl or methyl,
the enantiomers, the diastereomers, the mixtures and the salts thereof.

14. Compounds of general formula **I** according to claim 1, wherein **R¹, R², R³, R⁴, R⁵, R⁶, n** and **X** are defined as in claim 1, 2, 3, 4, 5, 6, 7, 8, 9, 10, 11, 12 or 13 and
**R⁷** denotes H, F, Cl, Br, -CN, C₁₋₄-alkyl, CF₃, CHF₂,
**R⁸** denotes H,
**R⁹** denotes F, Cl, Br, C₁₋₄-alkyl, -O-C₁₋₄-alkyl, -S-C₁₋₄-alkyl,
**R¹⁰** denotes H and
**R¹¹** denotes F, Cl, Br, -CN, C₁₋₄-alkyl, CF₃ or CHF₂,
the enantiomers, the diastereomers, the mixtures and the salts thereof.

15. Compounds of general formula **Ia,** 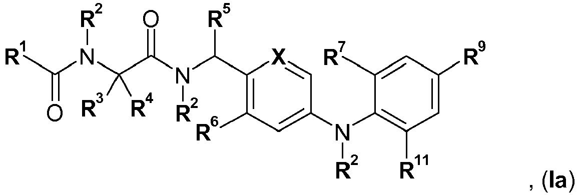 wherein
**R¹** denotes
(a) a C₁₋₆-alkyl group optionally substituted by a group **R^{1.1},**
(b) a phenyl group optionally substituted by 1, 2 or 3 groups **R^{1.3},**
(c) a five-membered heteroaryl group optionally substituted by 1, 2 or 3 groups **R^{1.4},** which contains at least one N, O or S atom and which optionally additionally contains one, two or three further N-atoms,
(d) a six-membered heteroaryl group optionally substituted by 1 or 2 groups **R^{1.4},** which contains one, two or three N-atoms,
(e) a nine- or ten-membered heteroaryl group optionally substituted by 1 or 2 groups **R^{1.4},** which contains one, two or three N-atoms,
(f) a 5- or 6-membered heterocyclic group optionally substituted by 1 or 2 groups **R^{1.4},** in which a -CH₂ unit may be replaced by a -C(O) group,
**R^{1.1}** denotes -CN, C₃₋₆-cycloalkyl, -OH, -OCH₃, -NH₂, -NHCH₃, -N(CH₃)₂,
**R^{1.3}** independently of one another denotes
(a) F, Cl, Br, -OH, -OCH₃, C₁₋₆-alkyl or
(b) a C₁₋₃-alkyl group wherein each methylene group may be substituted by 1 or 2 fluorine atoms and each methyl group may be substituted by 1, 2 or 3 fluorine atoms, and
**R^{1.4}** independently of one another denotes
(a) F, Cl, Br, -OH, -OCH₃, -NH₂, -NHCH₃, -N(CH₃)₂, -NH-C(O)-C₁₋₄-alkyl, C₁₋₆-alkyl, or
(b) a C₁₋₃-alkyl group wherein each methylene group may be substituted by 1 or 2 fluorine atoms and each methyl group may be substituted by 1, 2 or 3 fluorine atoms,
**R**² denotes H or CH₃,
**R³** and **R⁴** together with the carbon atom to which they are bonded denote a C₃₋₆-cycloalkylene group wherein a -CH₂- unit may be replaced by an oxygen atom,
**R⁵** denotes H or C₁₋₄-alkyl,
**R⁶** denotes H, F, Cl, Br or C₁₋₄-alkyl,
**R**⁷ denotes H, F, Cl, Br, -CN, C₁₋₄-alkyl, CF₃, CHF₂,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof.

16. Compounds of general formula **Ia** according to claim 14, wherein
**R¹** denotes
(a) a C₁₋₆-alkyl group optionally substituted by a group **R^{1.1},**
(b) a phenyl group optionally substituted by 1, 2 or 3 groups **R^{1.3},**
(c) a five-membered heteroaryl group optionally substituted by 1, 2 or 3 groups **R^{1.4}**, which is selected from among 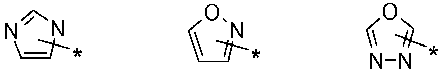 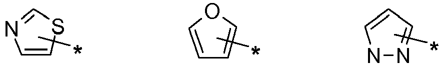 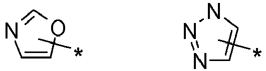
(d) a six-membered heteroaryl group optionally substituted by 1 or 2 groups **R^{1.4}**, which is selected from among 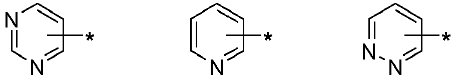 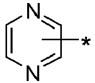
(e) a nine-membered heteroaryl group optionally substituted by 1 or 2 groups **R^{1.4}**, which is selected from among 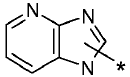
(f) a 5- or 6-membered heterocyclic group optionally substituted by 1 or 2 groups **R^{1.4},** which is selected from among 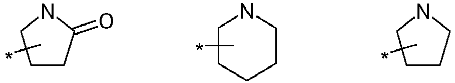 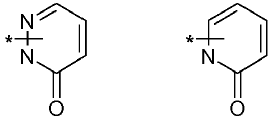
**R^{1.1}** denotes -CN, cyclopropyl, -OH, -OCH₃, -NH₂, -NHCH₃, -N(CH₃)₂,
**R^{1.3}** denotes independently of one another
(a) F, Cl, Br, -OH, -OCH₃, -OCF₃, C₁₋₄-alkyl or
(b) a C₁₋₃-alkyl group wherein each methylene group may be substituted by 1 or 2 fluorine atoms and each methyl group may be substituted by 1, 2 or 3 fluorine atoms, and
**R**^{**1.**4} denotes independently of one another
(a) F, Cl, Br, -OH, -OCH₃, -OCF₃, -NH₂, -NH-C₁₋₄-alkyl, -N(C₁₋₄-alkyl)₂, -NH-C(O)-C₁₋₄-alkyl, C₁₋₆-alkyl, or
(a) a C₁₋₃-alkyl group wherein each methylene group may be substituted by 1 or 2 fluorine atoms and each methyl group may be substituted by 1, 2 or 3 fluorine atoms,
**R**² denotes H or CH₃,
**R³** and **R⁴** together with the carbon atom to which they are bonded denote a C₃₋₆-cycloalkylene group wherein a -CH₂ unit may be replaced by an oxygen atom,
**R⁵** denotes H or CH₃,
**R⁶** denotes H, F, Cl or methyl,
**R**⁷ denotes H, F, Cl, Br, -CN, C₁₋₄-alkyl, CF₃, CHF₂,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof.

17. Compounds of general formula **Ia** according to claim 15, wherein
**R¹** denotes a group selected from 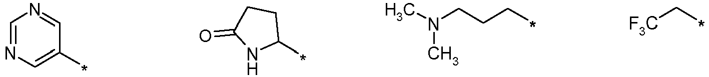 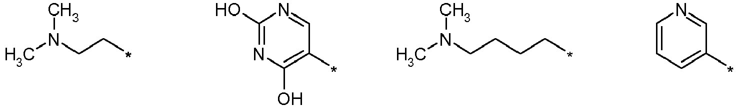 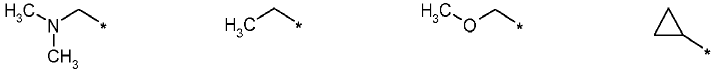 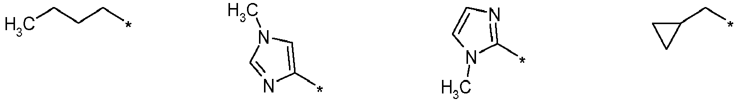 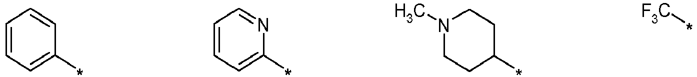 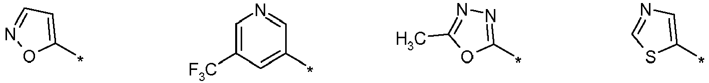 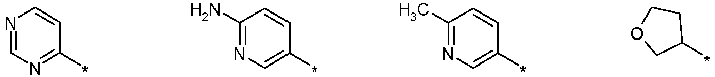 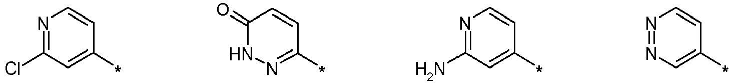 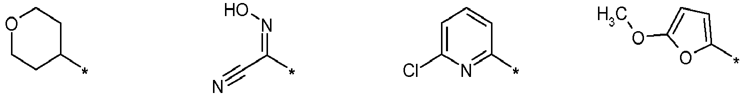 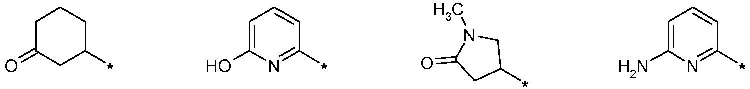 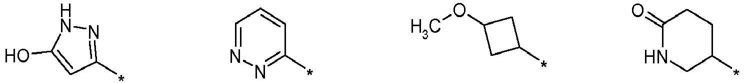 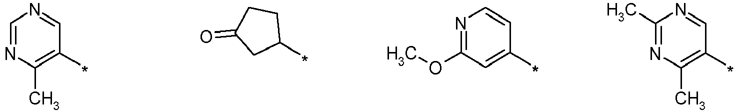 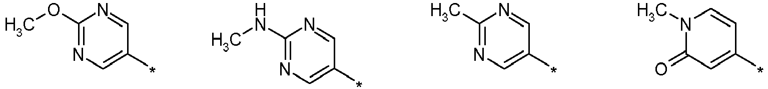 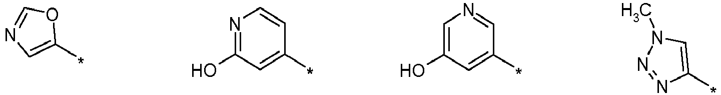 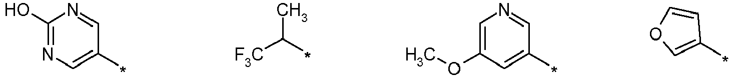 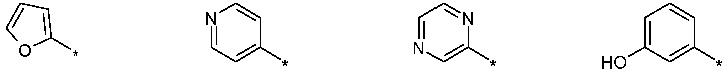 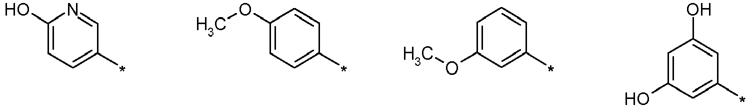 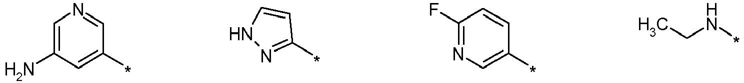 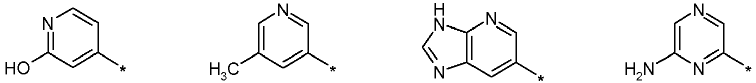 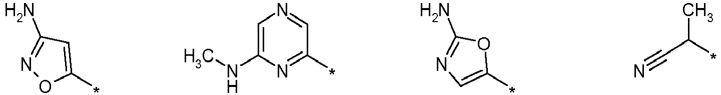 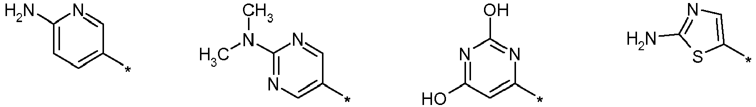 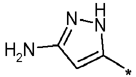 **R²** denotes H or CH₃,
**R³** and **R⁴** together with the carbon atom to which they are bonded denote a C₃₋₆-cycloalkylene group wherein a -CH₂ unit may be replaced by an oxygen atom,
**R⁵** denotes H or CH₃,
**R⁶** denotes H, F, Cl or methyl,
**R**⁷ denotes H, F, Cl, Br, -CN, C₁₋₄-alkyl, CF₃, CHF₂,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof.

18. Compounds of general formula **Ia** according to claim 15, wherein
**R¹** denotes a group selected from 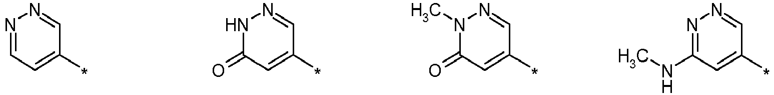 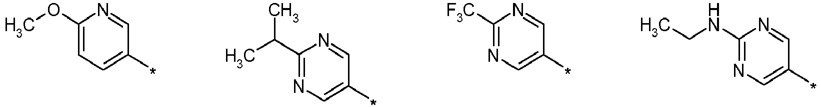 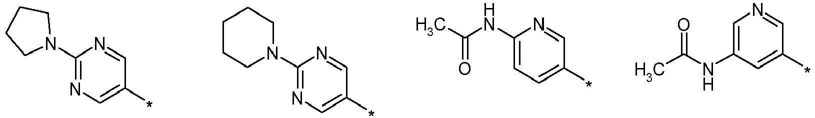 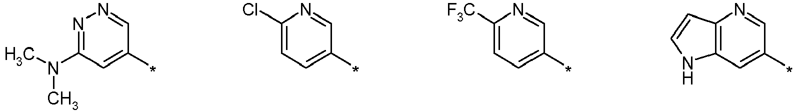 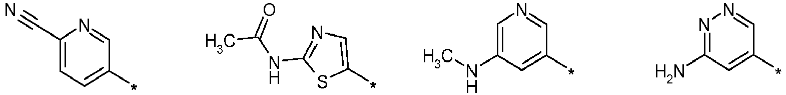 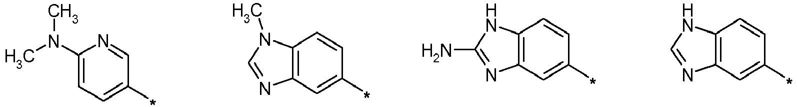 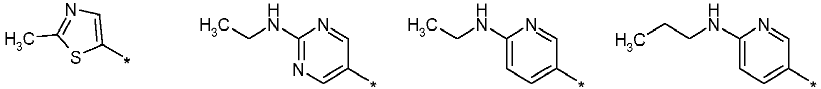 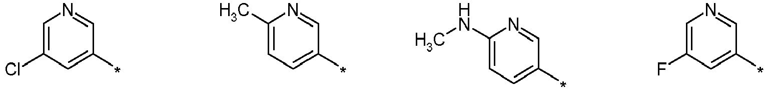 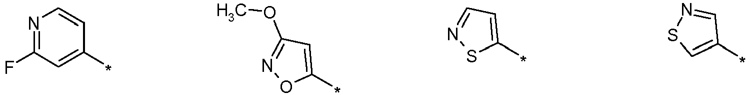 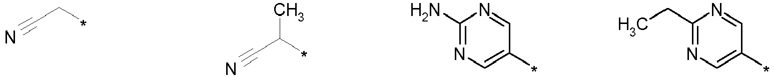 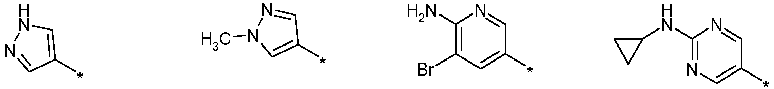 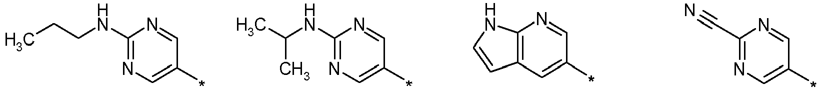 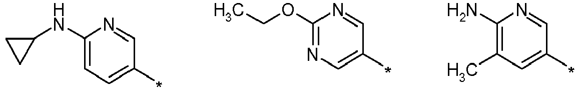 **R**² denotes H or CH₃,
**R³** and **R⁴** together with the carbon atom to which they are attached denote a C₃₋₆-cycloalkylene group wherein a -CH₂- unit may be replaced by an oxygen atom,
**R⁵** denotes H or CH₃,
**R⁶** denotes H, F, Cl or methyl,
**R**⁷ denotes H, F, Cl, Br, -CN, C₁₋₄-alkyl, CF₃, CHF₂,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof, particularly the physiologically acceptable salts thereof with organic or inorganic acids or bases.

19. Compounds of general formula **Ib** 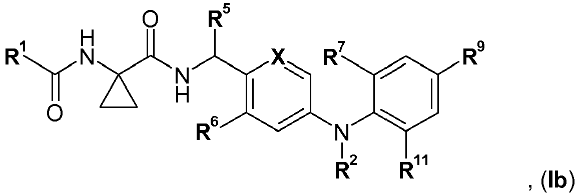 **R¹** denotes
(a) a C₁₋₆-alkyl group optionally substituted by a group **R^{1.1},**
(b) a phenyl group optionally substituted by 1, 2 or 3 groups **R^{1.3},**
(c) a five-membered heteroaryl group optionally substituted by 1, 2 or 3 groups **R^{1.4},** which contains at least one N, O or S atom and which optionally additionally contains one, two or three further N-atoms,
(d) a six-membered heteroaryl group optionally substituted by 1 or 2 groups **R^{1.4},** which contains one, two or three N-atoms,
(e) a nine- or ten-membered heteroaryl group optionally substituted by 1 or 2 groups **R^{1.4},** which contains one, two or three N-atoms,
(f) a 5- or 6-membered heterocyclic group optionally substituted by 1 or 2 groups **R^{1.4},** in which a -CH₂- unit may be replaced by a -C(O)- group,
**R^{1.1}** denotes -CN, C₃₋₆-cycloalkyl, -OH, -OCH₃, -NH₂, -NHCH₃, -N(CH₃)₂,
**R^{1.3}** denotes independently of one another
(a) F, Cl, Br, -OH, -OCH₃, C₁₋₆-alkyl or
(b) a C₁₋₃-alkyl group wherein each methylene group may be substituted by 1 or 2 fluorine atoms and each methyl group may be substituted by 1, 2 or 3 fluorine atoms, and
**R^{1.4}** denotes independently of one another
(a) F, Cl, Br, -OH, -OCH₃, -NH₂, -NHCH₃, -N(CH₃)₂, -NH-C(O)-C₁₋₄-alkyl, C₁₋₆-alkyl, or
(b) a C₁₋₃-alkyl group wherein each methylene group may be substituted by 1 or 2 fluorine atoms and each methyl group may be substituted by 1, 2 or 3 fluorine atoms,
**R**² denotes H or CH₃,
**R⁵** denotes H or C₁₋₄-alkyl,
**R⁶** denotes H, F, Cl, Br or C₁₋₄-alkyl,
**R**⁷ denotes H, F, Cl, Br, -CN, C₁₋₄-alkyl, CF₃, CHF₂,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof.

20. Compounds of general formula **Ib** according to claim 19, wherein
**R¹** denotes
(a) a C₁₋₆-alkyl group optionally substituted by a group **R^{1.1},**
(b) a phenyl group optionally substituted by 1, 2 or 3 groups **R^{1.3},**
(c) a five-membered heteroaryl group optionally substituted by 1, 2 or 3 groups **R^{1.4},** which is selected from among 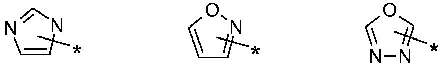 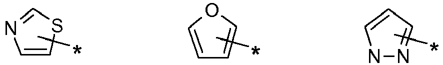 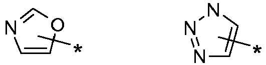
(d) a six-membered heteroaryl group optionally substituted by 1 or 2 groups **R^{1.4}**, which is selected from among 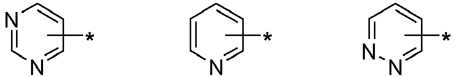 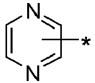
(e) a nine-membered heteroaryl group optionally substituted by 1 or 2 groups **R^{1.4}**, which is selected from among 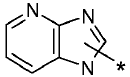
(f) a 5- or 6-membered heterocyclic group optionally substituted by 1 or 2 groups **R^{1.4},** which is selected from among 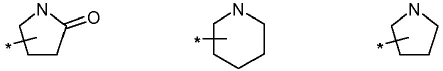 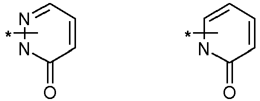
**R^{1.1}** denotes -CN, cyclopropyl, -OH, -OCH₃, -NH₂, -NHCH₃, -N(CH₃)₂,
**R^{1.3}** denotes independently of one another
(a) F, Cl, Br, -OH, -OCH₃, -OCF₃, C₁₋₄-alkyl or
(b) a C₁₋₃-alkyl group wherein each methylene group may be substituted by 1 or 2 fluorine atoms and each methyl group may be substituted by 1, 2 or 3 fluorine atoms, and
**R^{1.4}** denotes independently of one another
(a) F, Cl, Br, -OH, -OCH₃, -OCF₃, -NH₂, -NH-C₁₋₄-alkyl, -N(C₁₋₄-alkyl)₂, -NH-C(O)-C₁₋₄-alkyl, C₁₋₆-alkyl, or
(b) a C₁₋₃-alkyl group wherein each methylene group may be substituted by 1 or 2 fluorine atoms and each methyl group may be substituted by 1, 2 or 3 fluorine atoms,
**R²** denotes H or CH₃,
**R⁵** denotes H or CH₃,
**R⁶** denotes H, F, Cl or methyl,
**R**⁷ denotes H, F, Cl, Br, -CN, C₁₋₄-alkyl, CF₃, CHF₂,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof.

21. Compounds of general formula **Ib** according to claim 19, wherein
**R¹** denotes a group selected from 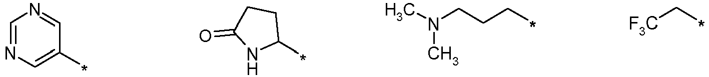 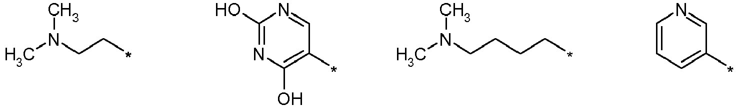 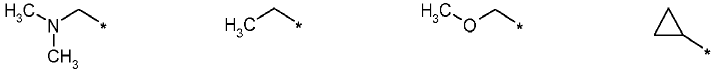 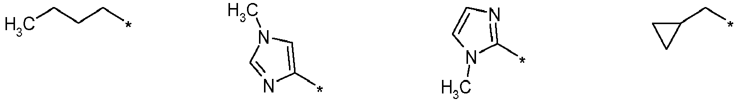 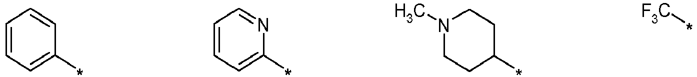 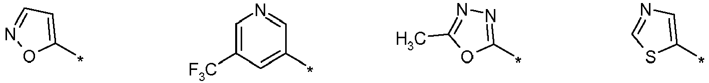 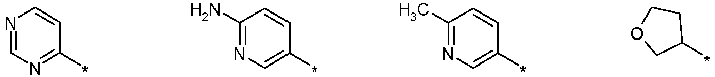 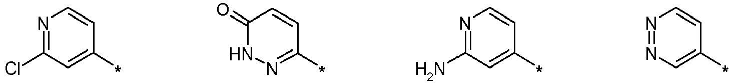 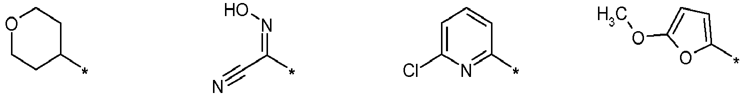 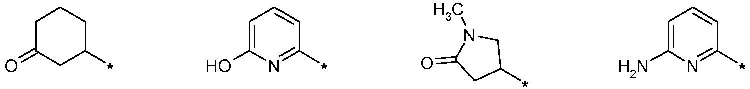 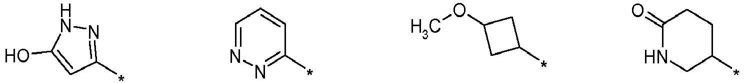 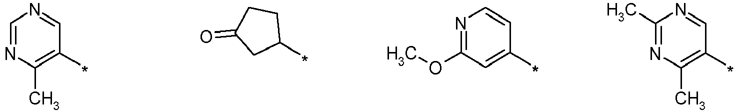 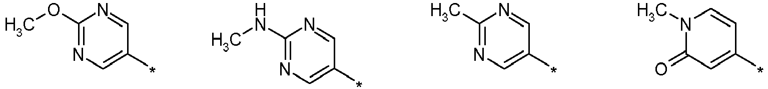 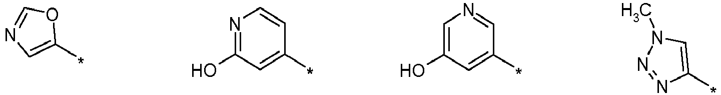 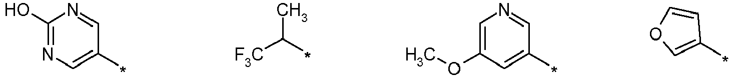 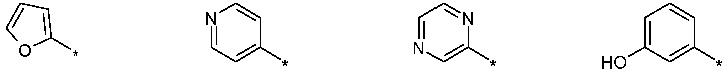 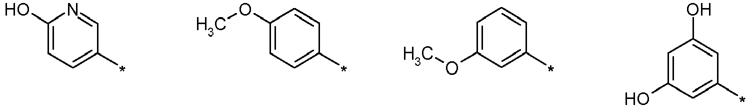 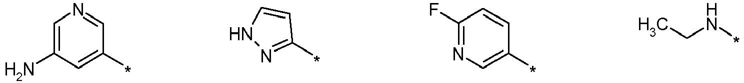 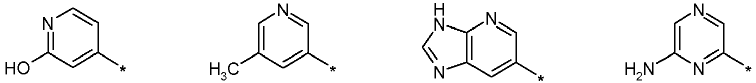 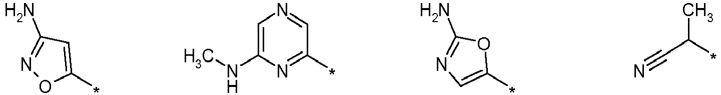 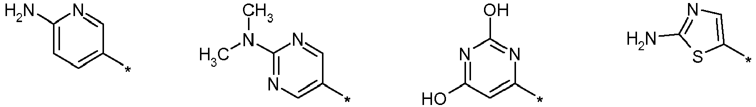 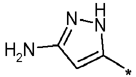 **R**² denotes H or CH₃,
**R⁵** denotes H or CH₃,
**R⁶** denotes H, F, Cl or methyl,
**R**⁷ denotes H, F, Cl, Br, -CN, C₁₋₄-alkyl, CF₃, CHF₂,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof.

22. Compounds of general formula **Ib** according to claim 19, wherein
**R¹** denotes a group selected from 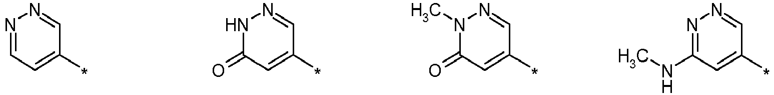 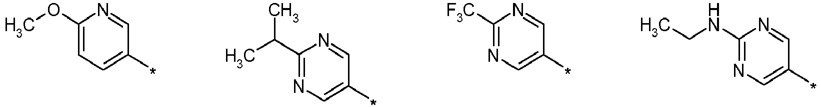 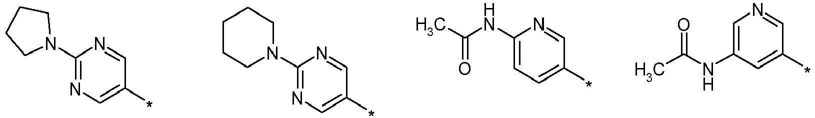 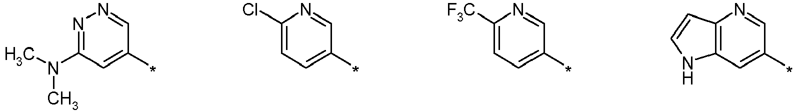 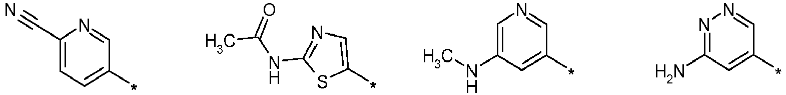 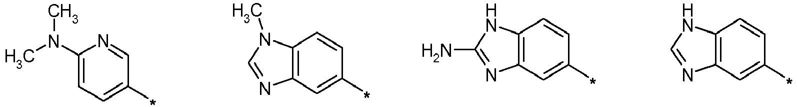 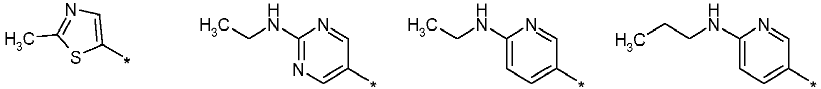 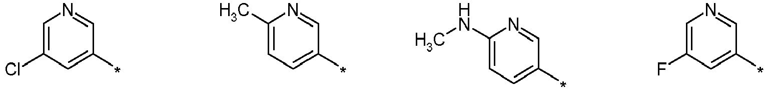 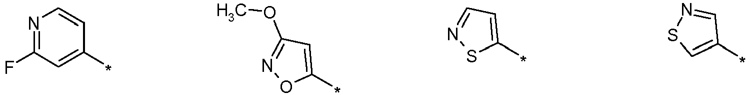 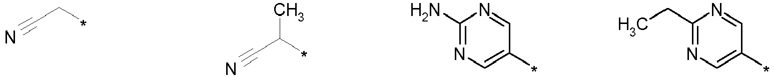 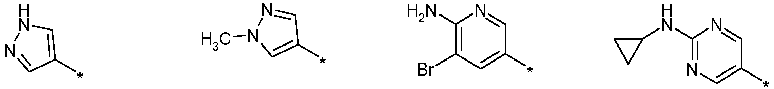 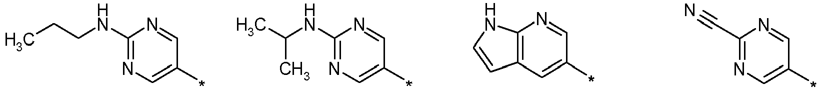 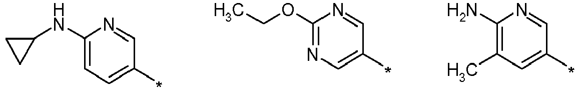 **R**² denotes H,
**R⁵** denotes H or CH₃,
**R⁶** denotes H, F, Cl or methyl,
**R⁷** denotes H, F, Cl, Br, -CN, C₁₋₄-alkyl, CF₃, CHF₂,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof, particularly the physiologically acceptable salts thereof with organic or inorganic acids or bases.

23. Compounds of general formula **Ic**, 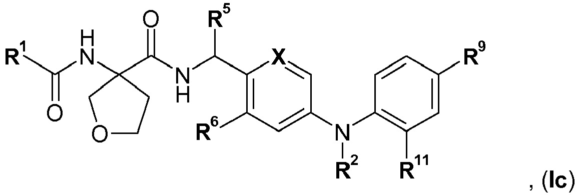 wherein
**R¹** denotes
(a) a C₁₋₆-alkyl group optionally substituted by a group **R^{1.1}**,
(b) a phenyl group optionally substituted by 1, 2 or 3 groups **R^{1.3}**,
(c) a five-membered heteroaryl group optionally substituted by 1, 2 or 3 groups **R^{1.4}**, which contains at least one N, O or S atom and which optionally additionally contains one, two or three further N-atoms,
(d) a six-membered heteroaryl group optionally substituted by 1 or 2 groups **R^{1.4}**, which contains one, two or three N-atoms,
(e) a nine- or ten-membered heteroaryl group optionally substituted by 1 or 2 groups **R^{1.4},** which contains one, two or three N-atoms,
(f) a 5- or 6-membered heterocyclic group optionally substituted by 1 or 2 groups **R^{1.4}**, in which a -CH₂- unit may be replaced by a -C(O)- group,
**R^{1.1}** denotes -CN, C₃₋₆-cycloalkyl, -OH, -OCH₃, -NH₂, -NHCH₃, -N(CH₃)₂,
**R^{1.3}** denotes independently of one another
(a) F, Cl, Br, -OH, -OCH₃, C₁₋₆-alkyl or
(b) a C₁₋₃-alkyl group wherein each methylene group may be substituted by 1 or 2 fluorine atoms and each methyl group may be substituted by 1, 2 or 3 fluorine atoms, and
**R^{1.4}** denotes independently of one another
(a) F, Cl, Br, -OH, -OCH₃, -NH₂, -NHCH₃, -N(CH₃)₂, -NH-C(O)-C₁-₄-alkyl, C₁₋₆-alkyl, or
(a) a C₁₋₃-alkyl group wherein each methylene group may be substituted by 1 or 2 fluorine atoms and each methyl group may be substituted by 1, 2 or 3 fluorine atoms,
**R²** denotes H or CH₃,
**R⁵** denotes H or C₁₋₄-alkyl,
**R⁶** denotes H, F, Cl, Br or C₁₋₄-alkyl,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof.

24. Compounds of general formula **Ic** according to claim 23, wherein **R¹** denotes
(a) a C₁₋₆-alkyl group optionally substituted by a group **R^{1.1}**,
(b) a phenyl group optionally substituted by 1, 2 or 3 groups **R^{1.3}**,
(c) a five-membered heteroaryl group optionally substituted by 1, 2 or 3 groups **R^{1.4}**, which is selected from among 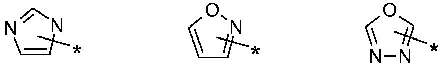 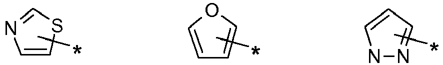 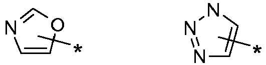
(d) a six-membered heteroaryl group optionally substituted by 1 or 2 groups **R^{1.4}**, which is selected from among 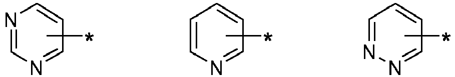 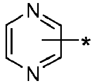
(e) a nine-membered heteroaryl group optionally substituted by 1 or 2 groups **R^{1.4}**, which is selected from among 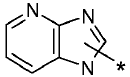
(f) a 5- or 6-membered heterocyclic group optionally substituted by 1 or 2 groups **R^{1.4},** which is selected from among 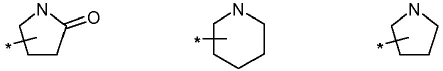 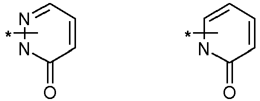
**R^{1.1}** denotes -CN, cyclopropyl, -OH, -OCH₃, -NH₂, -NHCH₃, -N(CH₃)₂,
**R^{1.3}** denotes independently of one another
(a) F, Cl, Br, -OH, -OCH₃, -OCF₃, C₁₋₄-alkyl or
(b) a C₁₋₃-alkyl group wherein each methylene group may be substituted by 1 or 2 fluorine atoms and each methyl group may be substituted by 1, 2 or 3 fluorine atoms, and
**R^{1.4}** denotes independently of one another
(a) F, Cl, Br, -OH, -OCH₃, -OCF₃, -NH₂, -NH-C₁₋₄-alkyl, -N(C₁₋₄-alkyl)₂, -NH-C(O)-C₁₋₄-alkyl, C₁₋₆-alkyl, or
(a) a C₁₋₃-alkyl group wherein each methylene group may be substituted by 1 or 2 fluorine atoms and each methyl group may be substituted by 1, 2 or 3 fluorine atoms,
**R²** denotes H or CH₃,
**R⁵** denotes H or CH₃,
**R⁶** denotes H, F, Cl or methyl,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof.

25. Compounds of general formula **Ic** according to claim 23, wherein
**R¹** denotes a group selected from 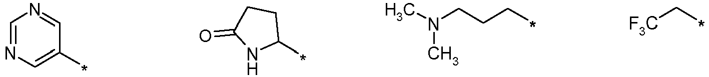 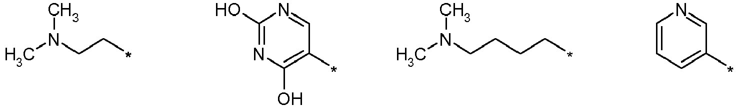 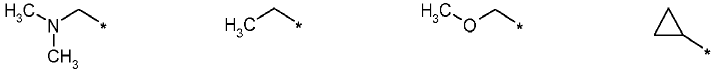 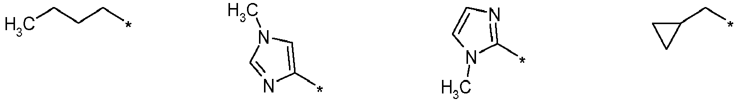 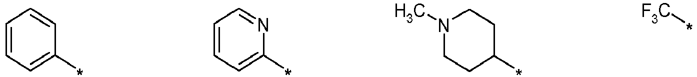 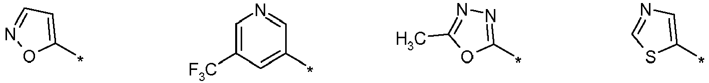 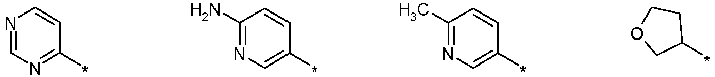 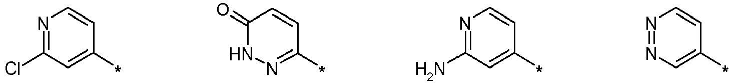 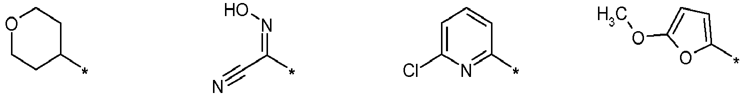 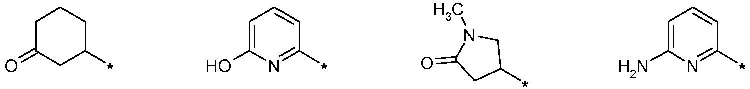 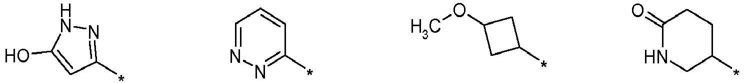 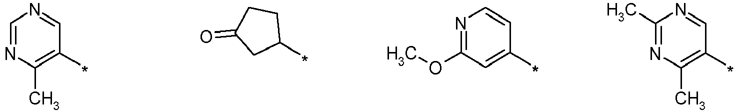 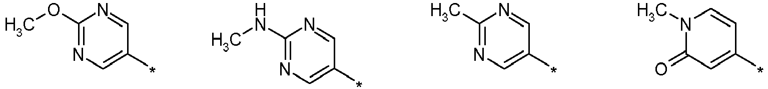 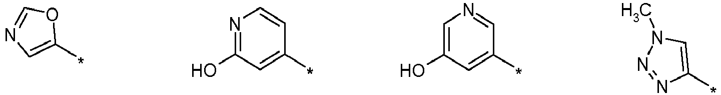 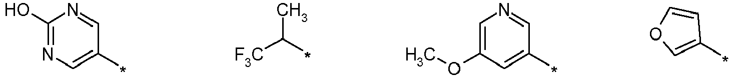 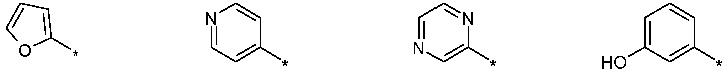 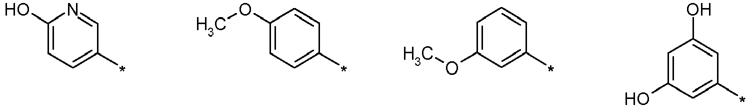 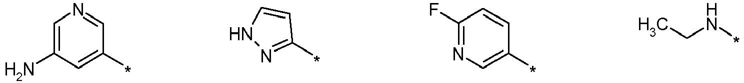 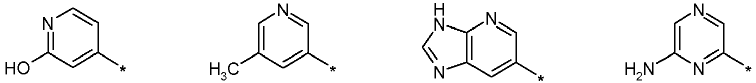 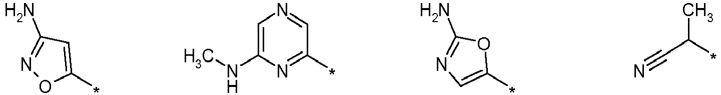 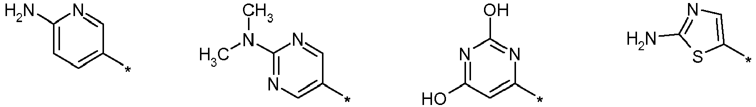 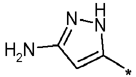 **R²** denotes H or CH₃,
**R⁵** denotes H or CH₃,
**R⁶** denotes H, F, Cl or methyl,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof.

26. Compounds of general formula Ic according to claim 23, wherein
**R¹** denotes a group selected from 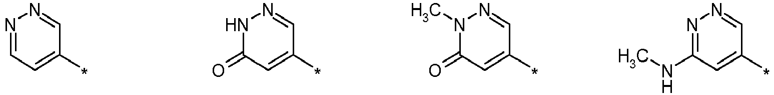 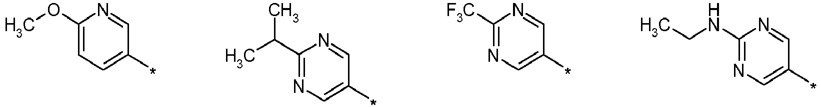 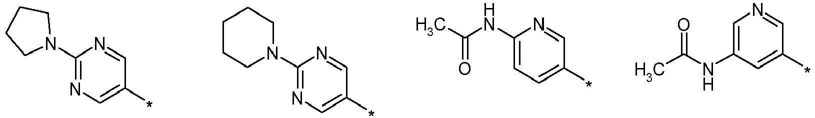 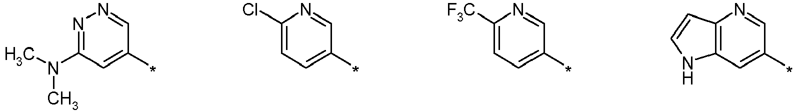 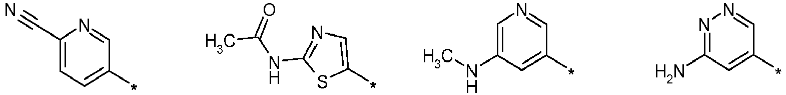 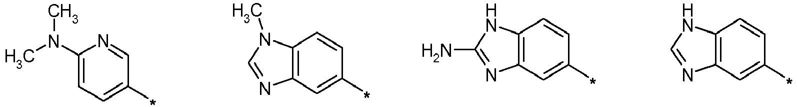 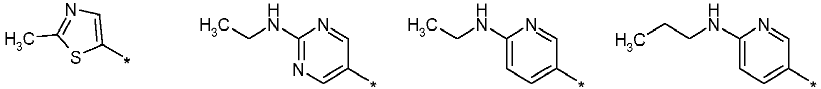 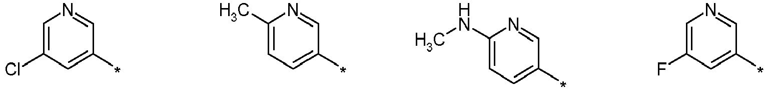 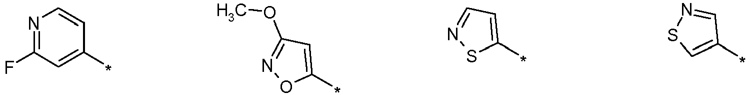 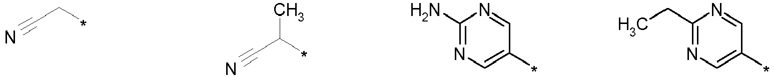 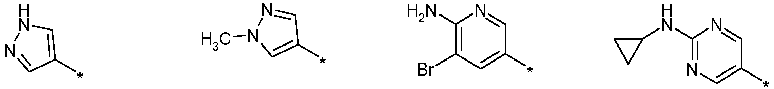 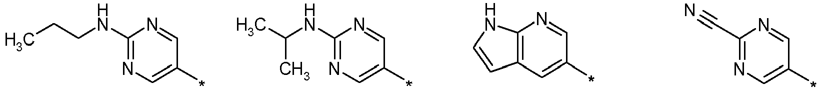 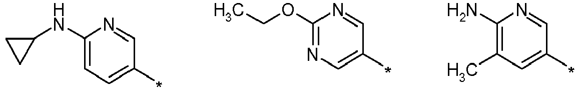 **R²** denotes H or CH₃,
**R⁵** denotes H or CH₃,
**R⁶** denotes H, F, Cl or methyl,
**R⁹** denotes F, Cl, Br, C₁₋₄-alkyl, -O-C₁₋₄-alkyl, -S-C₁₋₄-alkyl,
**R¹¹** denotes F, Cl, Br, -CN, C₁₋₄-alkyl, CF₃, CHF₂, and
**X** denotes CH or N,
the enantiomers, the diastereomers, the mixtures and the salts thereof, particularly the physiologically acceptable salts thereof with organic or inorganic acids or bases.

27. Compounds of general formula **Id,** 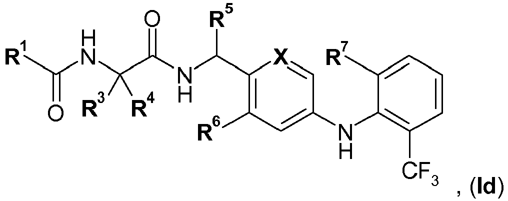 wherein
R¹ denotes a group selected from 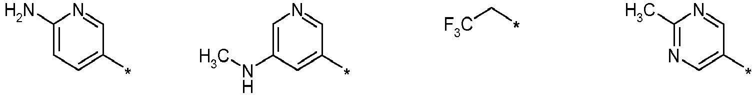 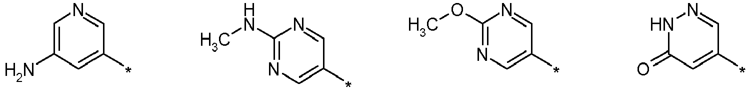 **R³** and **R⁴** together with the carbon atom to which they are attached denote a C₃₋₆-cycloalkylene group wherein a -CH₂ unit may be replaced by an oxygen atom,
**R⁵** denotes H or CH₃,
**R⁶** denotes Cl or CH₃,
**R⁷** denotes H or F,
**X** denotes CH or N,
the enantiomers, the diastereomers, the mixtures and the salts thereof, particularly the physiologically acceptable salts thereof with organic or inorganic acids or bases.

28. The following compounds of general formula I according to claim 1:
| **No.** | **Structure** |
|---|---|
| (1) | 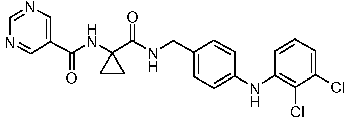 |
| (2) | 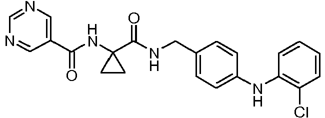 |
| (3) | 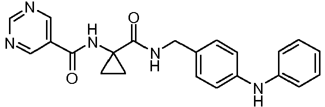 |
| (4) | 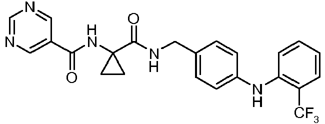 |
| (5) | 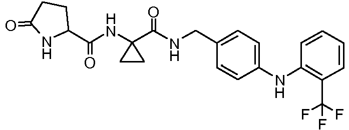 |
| (6) | 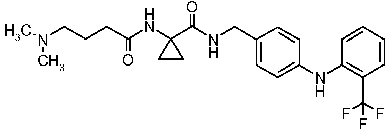 |
| (7) | 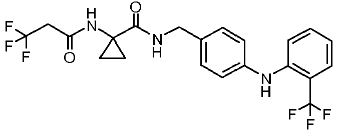 |
| (8) | 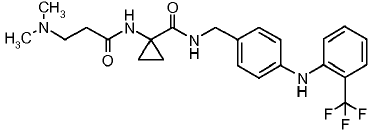 |
| (9) | 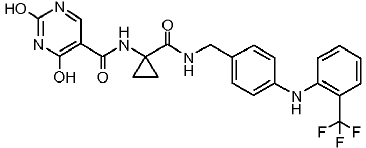 |
| (10) | 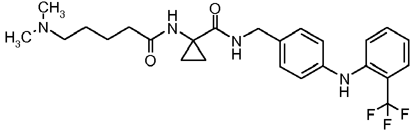 |
| (11) | 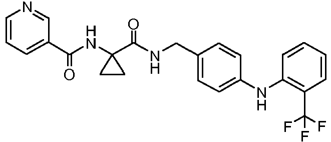 |
| (12) | 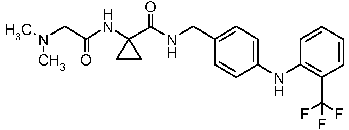 |
| (13) | 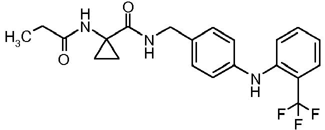 |
| (14) | 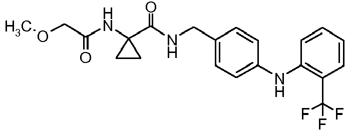 |
| (15) | 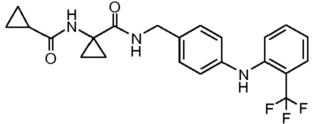 |
| (16) | 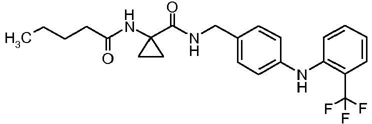 |
| (17) | 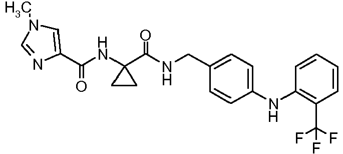 |
| (18) | 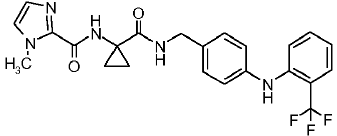 |
| (19) | 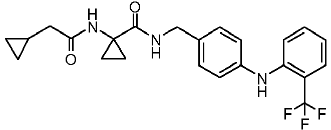 |
| (20) | 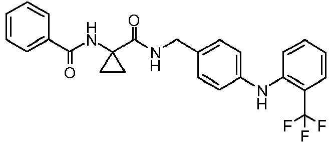 |
| (21) | 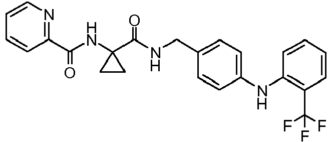 |
| (22) | 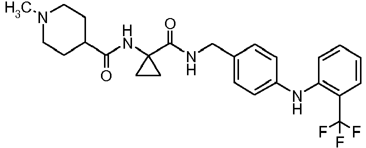 |
| (23) | 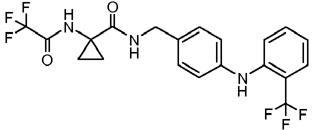 |
| (24) | 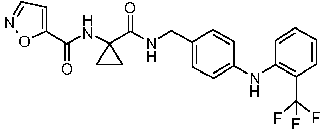 |
| (25) | 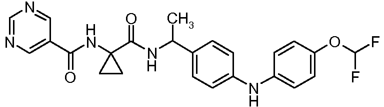 |
| (26) | 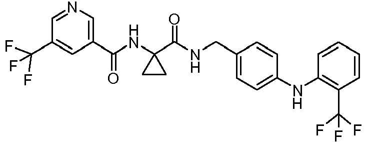 |
| (27) | 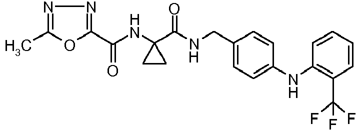 |
| (28) | 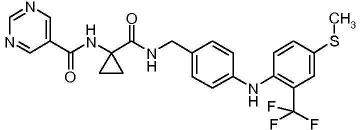 |
| (29) | 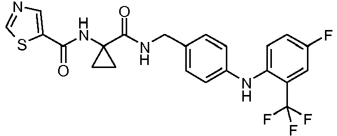 |
| (30) | 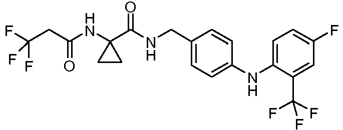 |
| (31) | 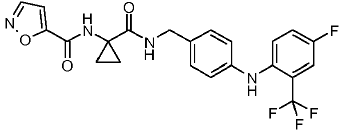 |
| (32) | 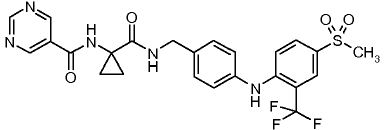 |
| (33) | 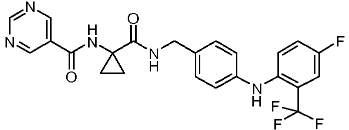 |
| (34) | 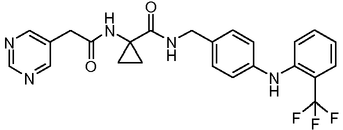 |
| (35) | 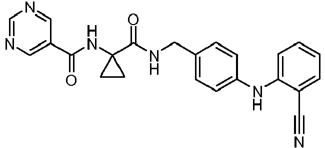 |
| (36) | 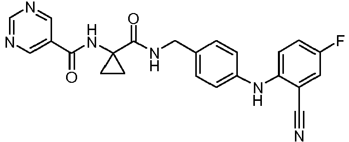 |
| (37) | 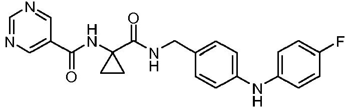 |
| (38) | 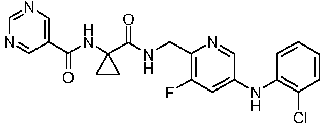 |
| (39) | 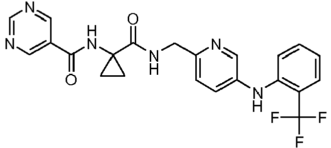 |
| (40) | 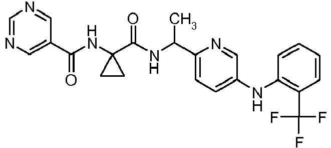 |
| (41) | 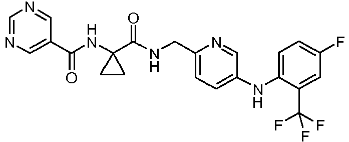 |
| (42) | 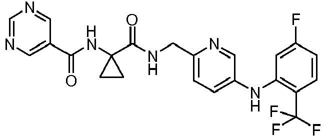 |
| (43) | 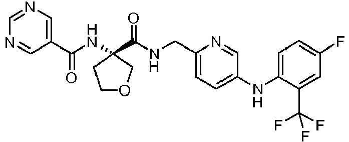 |
| (44) | 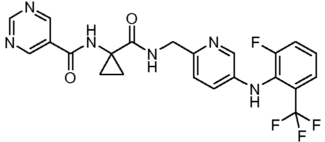 |
| (45) | 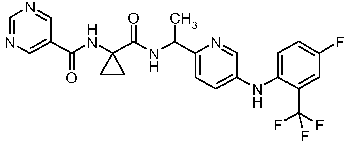 |
| (46) | 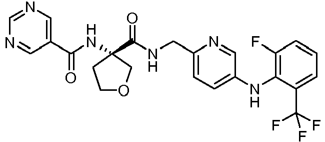 |
| (47) | 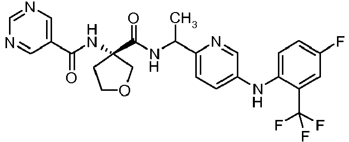 |
| (48) | 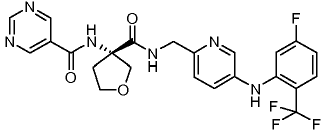 |
| (49) | 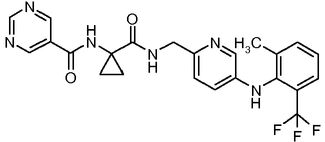 |
| (50) | 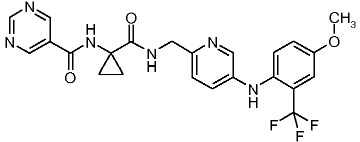 |
| (51) | 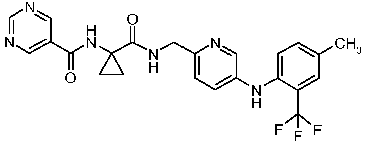 |
| (52) | 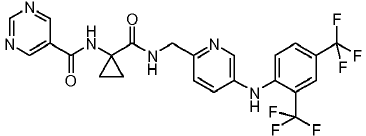 |
| (53) | 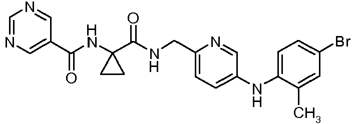 |
| (54) | 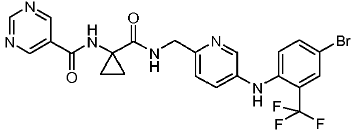 |
| (55) | 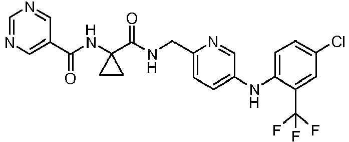 |
| (56) | 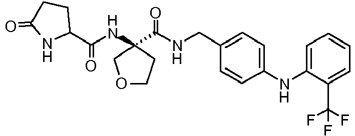 |
| (57) | 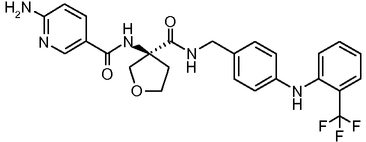 |
| (58) | 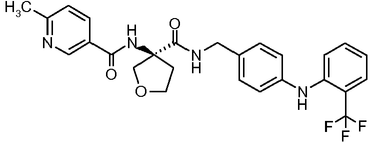 |
| (59) | 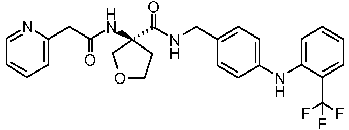 |
| (60) | 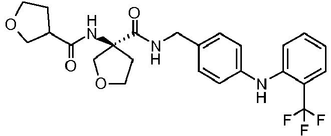 |
| (61) | 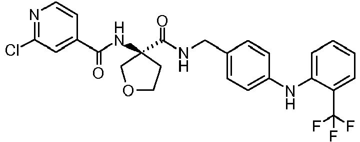 |
| (62) | 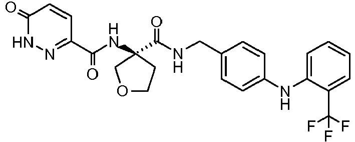 |
| (63) | 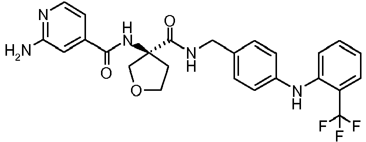 |
| (64) | 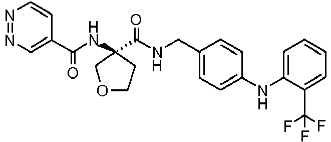 |
| (65) | 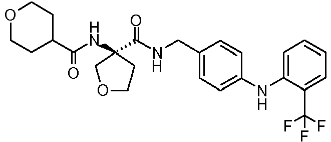 |
| (66) | 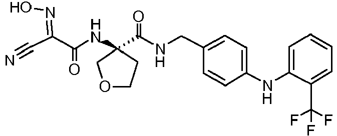 |
| (67) | 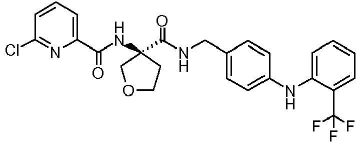 |
| (68) | 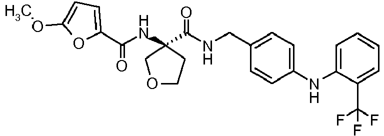 |
| (69) | 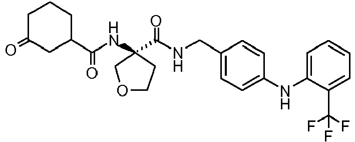 |
| (70) | 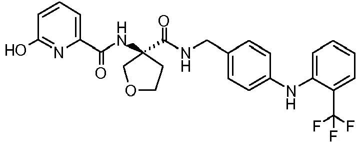 |
| (71) | 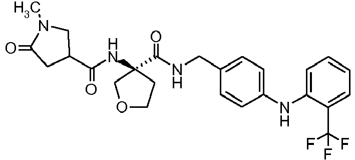 |
| (72) | 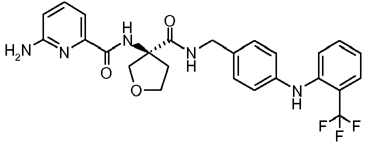 |
| (73) | 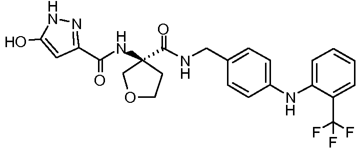 |
| (74) | 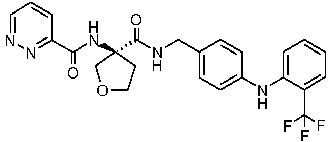 |
| (75) | 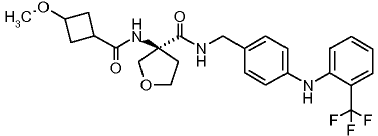 |
| (76) | 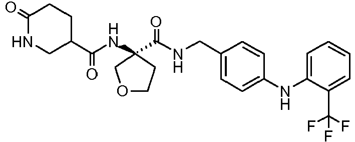 |
| (77) | 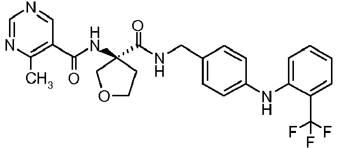 |
| (78) | 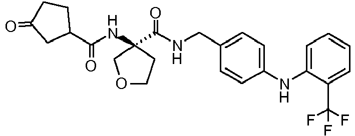 |
| (79) | 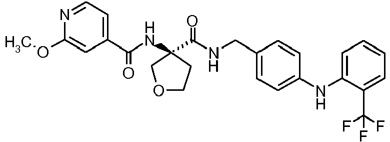 |
| (80) | 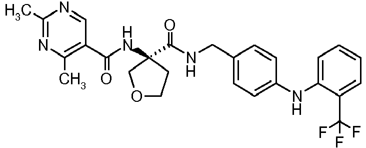 |
| (81) | 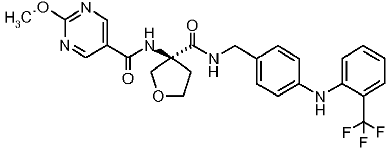 |
| (82) | 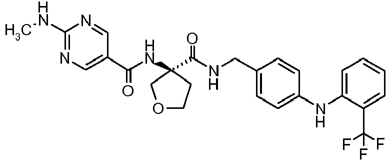 |
| (83) | 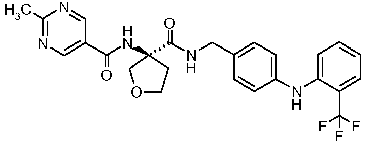 |
| (84) | 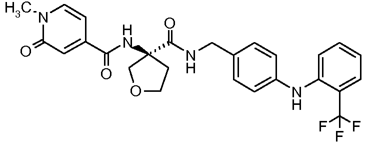 |
| (85) | 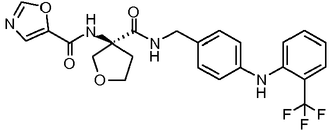 |
| (86) | 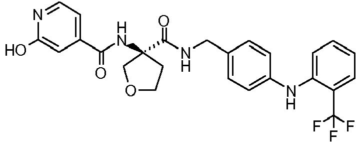 |
| (87) | 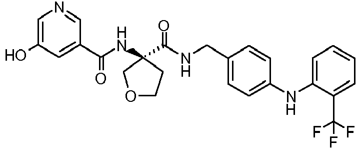 |
| (88) | 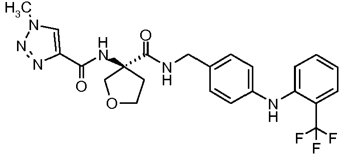 |
| (89) | 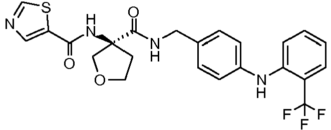 |
| (90) | 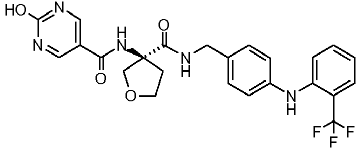 |
| (91) | 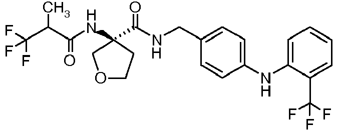 |
| (92) | 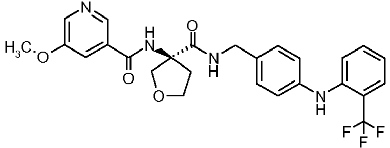 |
| (93) | 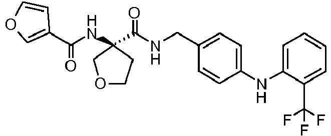 |
| (94) | 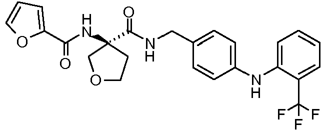 |
| (95) | 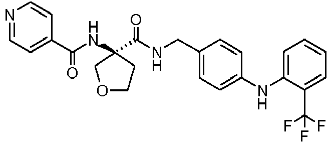 |
| (96) | 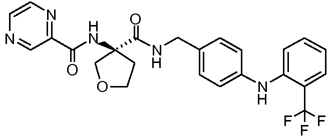 |
| (97) | 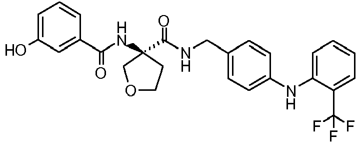 |
| (98) | 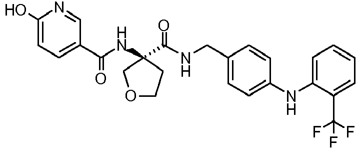 |
| (99) | 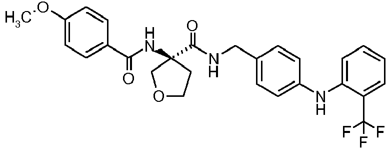 |
| (100) | 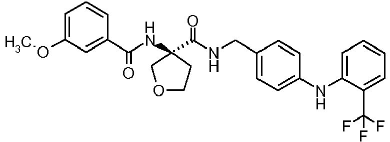 |
| (101) | 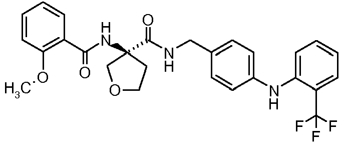 |
| (102) | 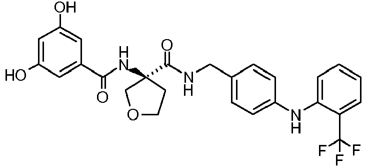 |
| (103) | 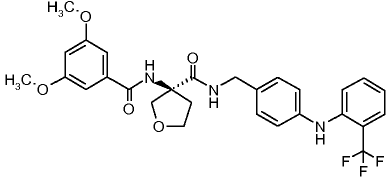 |
| (104) | 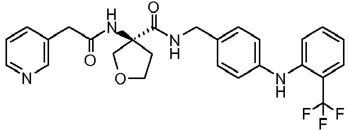 |
| (105) | 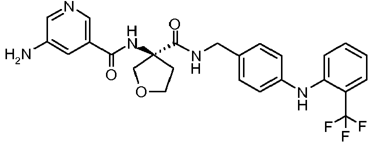 |
| (106) | 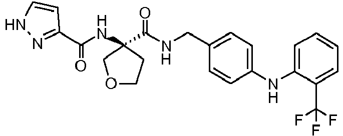 |
| (107) | 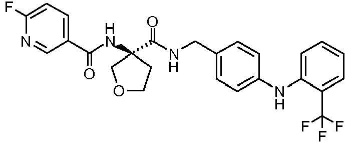 |
| (108) | 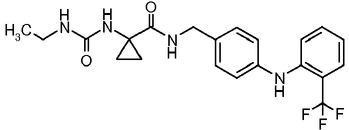 |
| (109) | 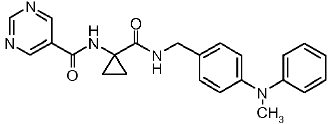 |
| (110) | 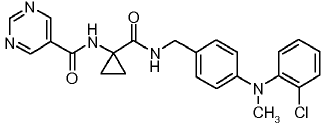 |
| (111) | 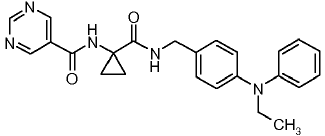 |
| (112) | 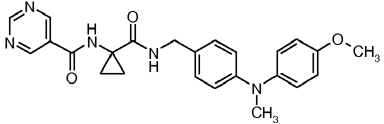 |
| (113) | 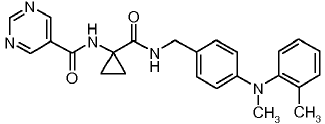 |
| (114) | 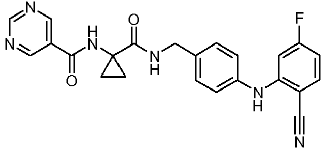 |
| (115) | 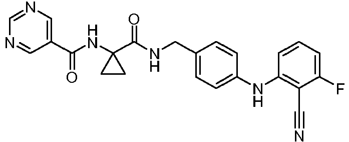 |
| (116) | 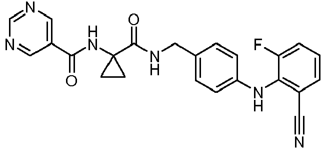 |
| (117) | 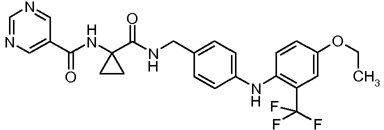 |
| (118) | 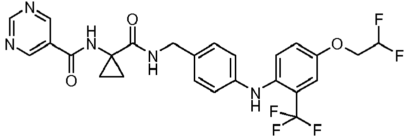 |
| (119) | 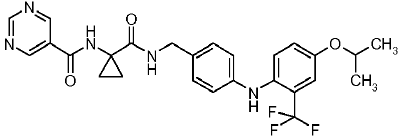 |
| (120) | 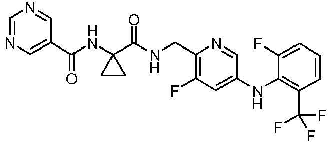 |
| (121) | 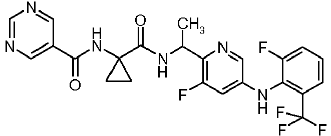 |
| (122) | 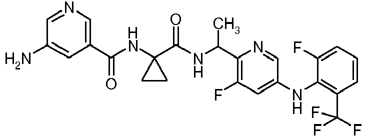 |
| (123) | 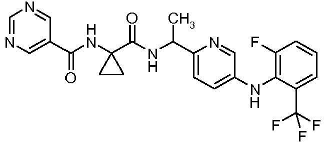 |
| (124) | 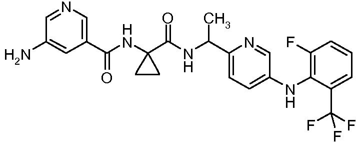 |
| (125) | 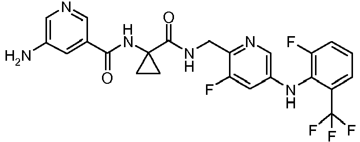 |
| (126) | 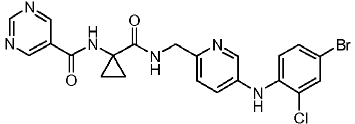 |
| (127) | 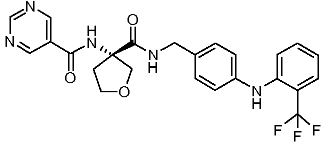 |
| (128) | 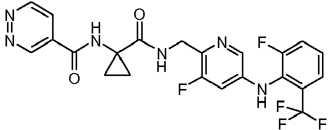 |
| (129) | 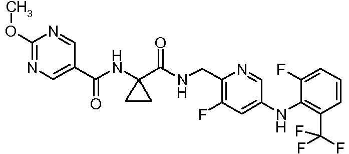 |
| (130) | 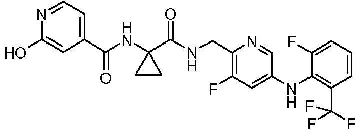 |
| (131) | 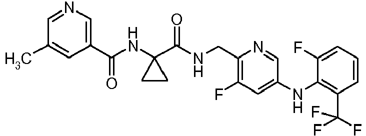 |
| (132) | 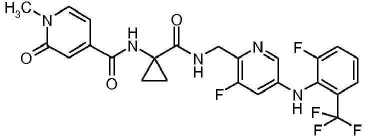 |
| (133) | 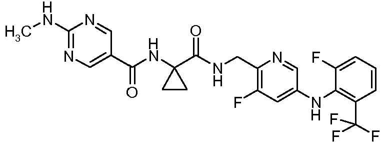 |
| (134) | 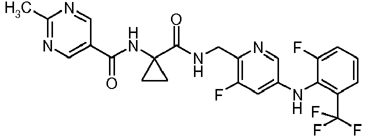 |
| (135) | 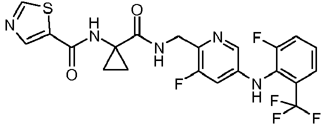 |
| (136) | 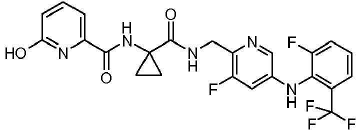 |
| (137) | 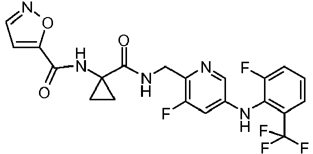 |
| (138) | 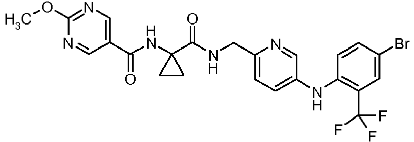 |
| (139) | 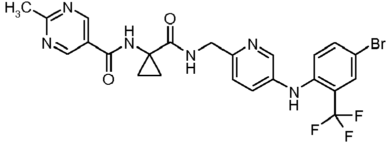 |
| (140) | 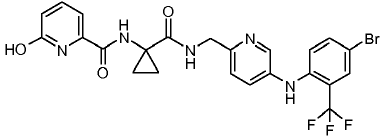 |
| (141) | 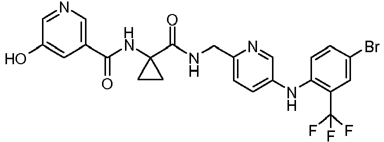 |
| (142) | 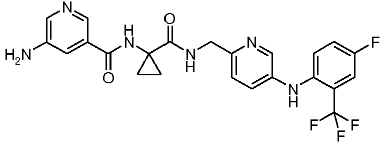 |
| (143) | 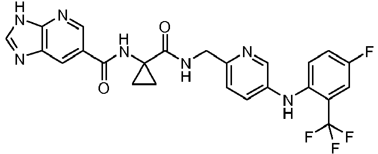 |
| (144) | 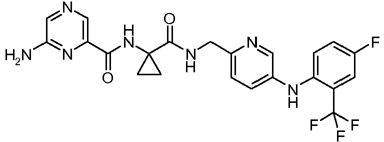 |
| (145) | 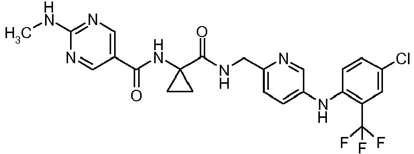 |
| (146) | 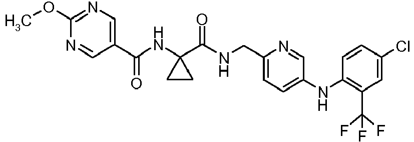 |
| (147) | 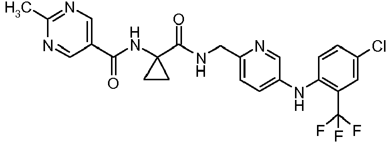 |
| (148) | 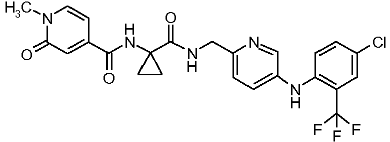 |
| (149) | 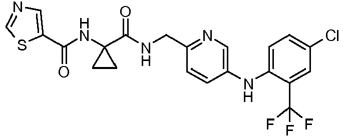 |
| (150) | 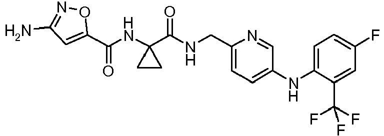 |
| (151) | 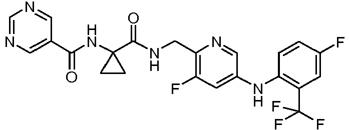 |
| (152) | 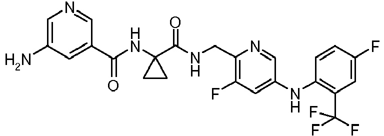 |
| (153) | 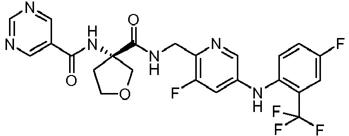 |
| (154) | 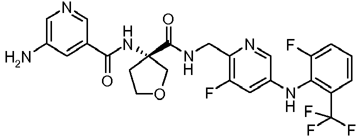 |
| (155) | 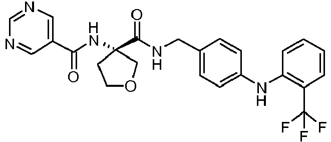 |
| (156) | 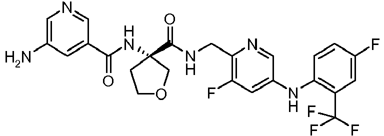 |
| (157) | 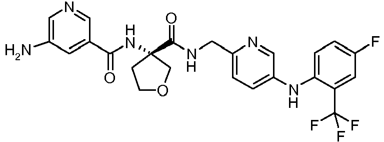 |
| (158) | 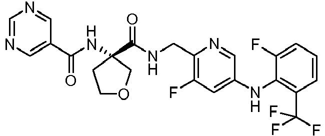 |
| (159) | 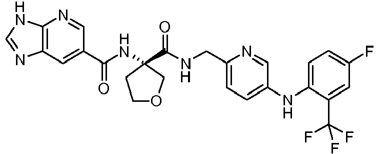 |
| (160) | 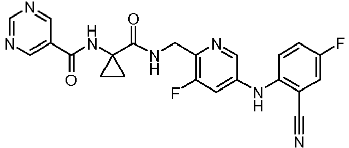 |
| (161) | 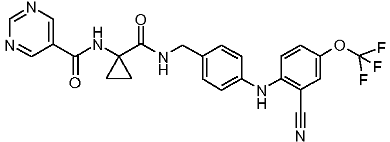 |
| (162) | 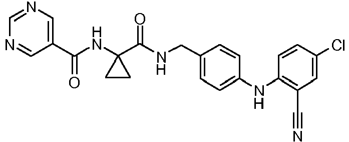 |
| (163) | 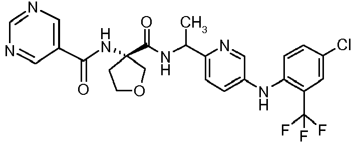 |
| (164) | 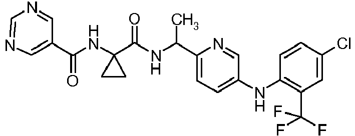 |
| (165) | 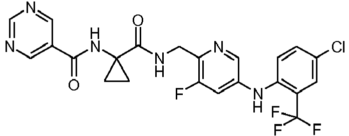 |
| (166) | 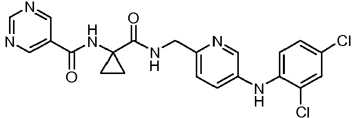 |
| (167) | 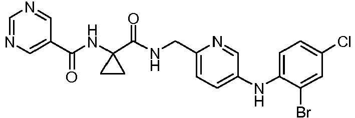 |
| (168) | 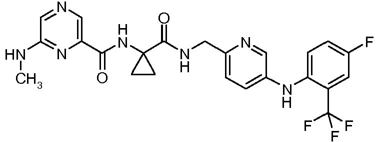 |
| (169) | 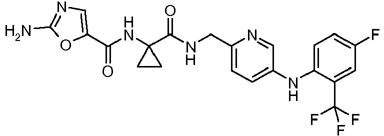 |
| (170) | 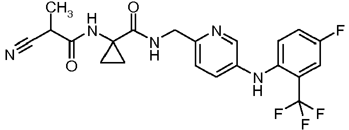 |
| (171) | 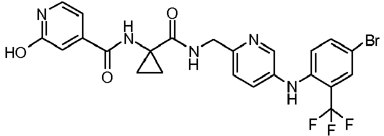 |
| (172) | 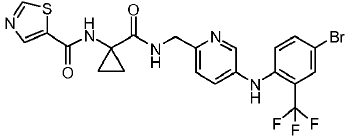 |
| (173) | 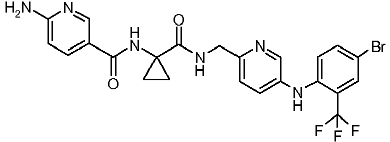 |
| (174) | 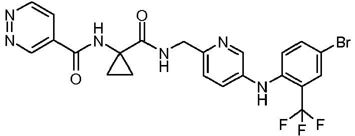 |
| (175) | 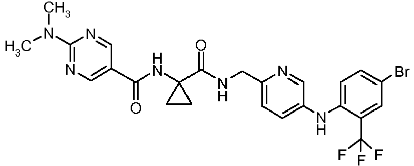 |
| (176) | 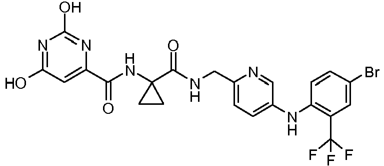 |
| (177) | 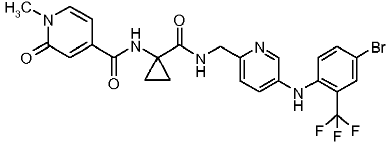 |
| (178) | 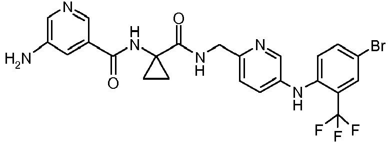 |
| (179) | 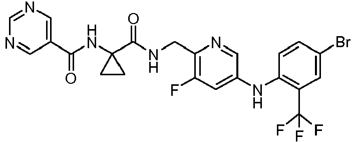 |
| (180) | 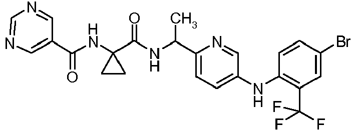 |
| (181) | 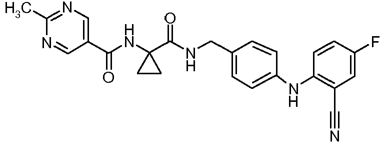 |
| (182) | 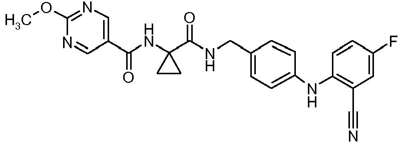 |
| (183) | 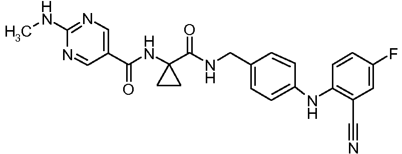 |
| (184) | 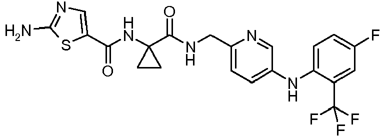 |
| (185) | 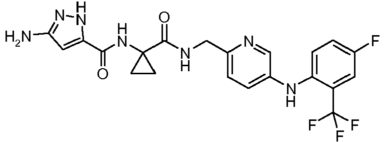 |
| (186) | 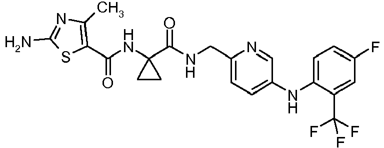 |
| (187) | 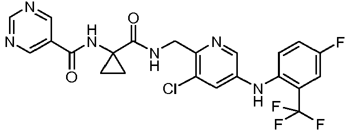 |
| (188) | 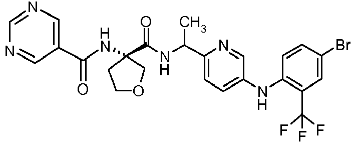 |
| (189) | 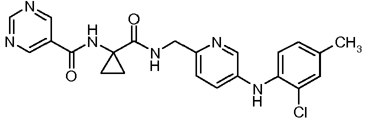 |
| (190) | 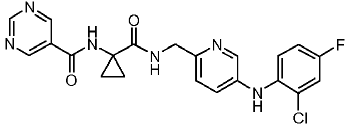 |
| (191) | 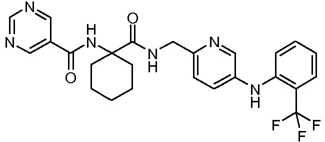 |
| (192) | 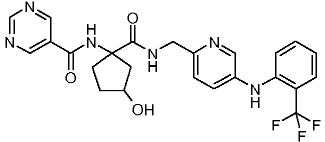 |
| (193) | 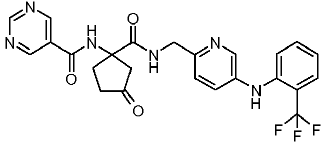 |
| (194) | 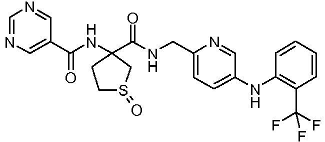 |
| (195) | 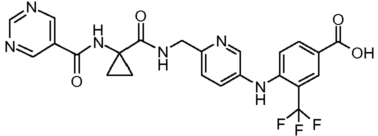 |
| (196) | 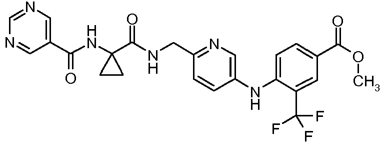 |
| (197) | 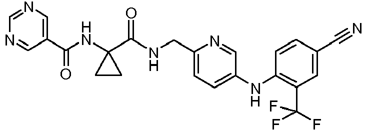 |
| (198) | 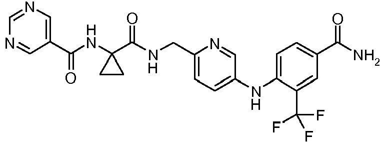 |
| (199) | 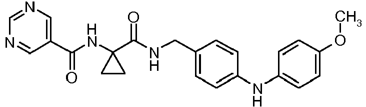 |
| (200) | 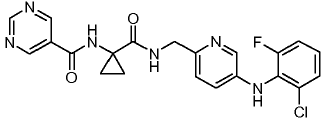 |
| (201) | 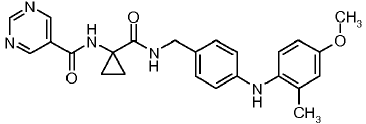 |
| (202) | 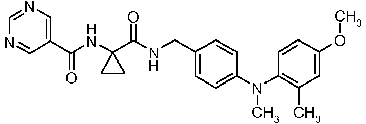 |
| (203) | 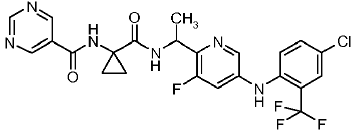 |
| (203a) | 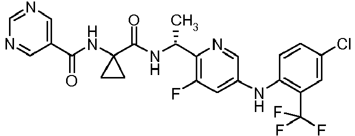 |
| (203b) | 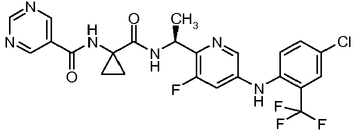 |
| (204) | 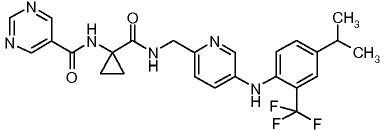 |
| (205) | 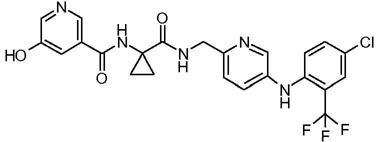 |
| (206) | 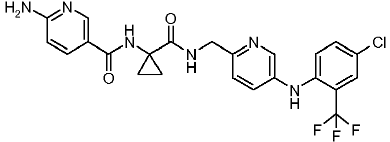 |
| (207) | 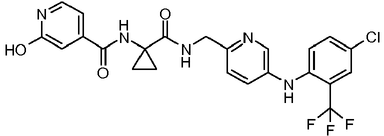 |
| (208) | 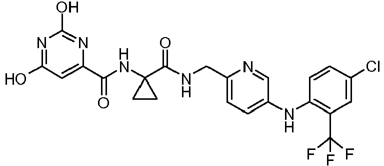 |
| (209) | 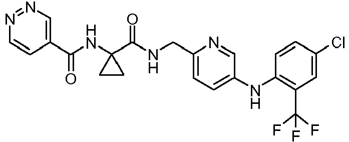 |
| (210) | 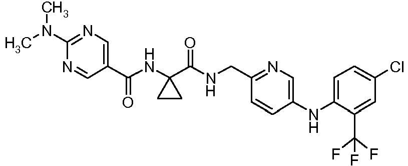 |
| (211) | 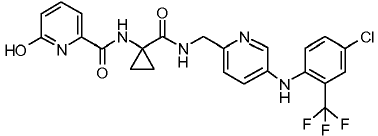 |
| (212) | 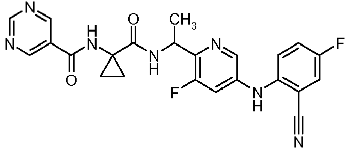 |
| (212a) | 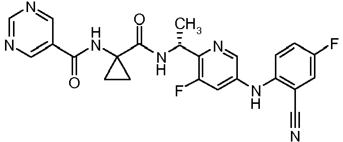 |
| (212b) | 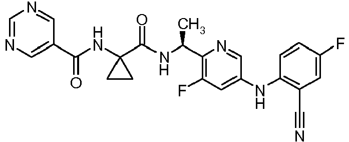 |
| (213) | 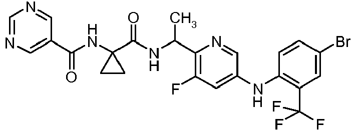 |
| (213a) | 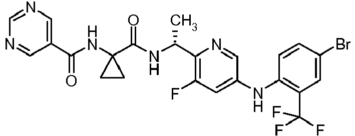 |
| (213b) | 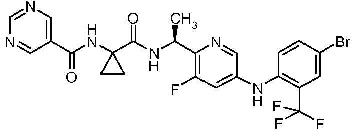 |
| (214) | 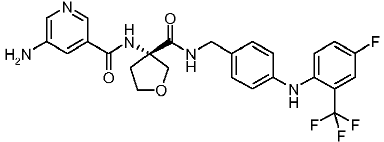 |
| (215) | 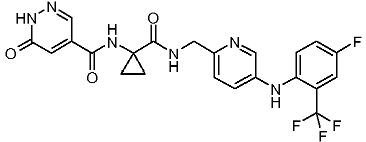 |
| (216) | 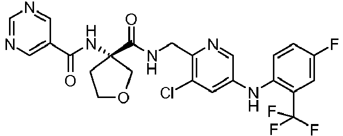 |
| (217) | 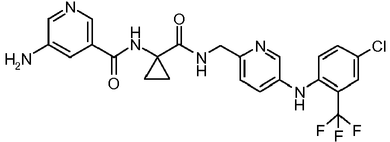 |
| (218) | 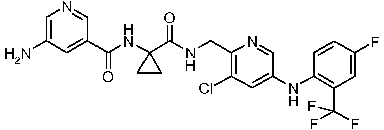 |
| (219) | 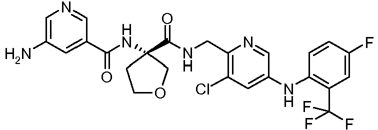 |
| (220) | 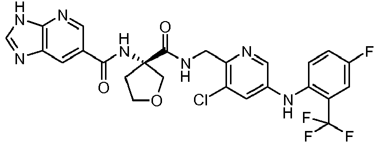 |
| (221) | 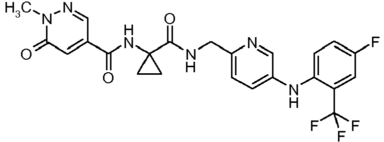 |
| (222) | 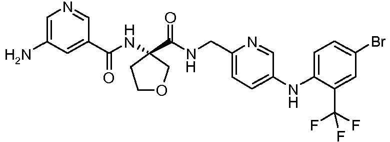 |
| (223) | 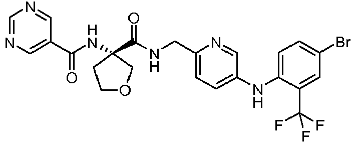 |
| (224) | 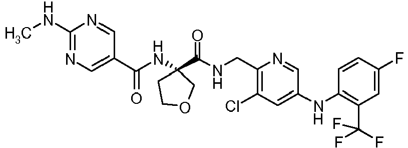 |
| (225) | 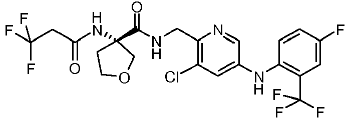 |
| (226) | 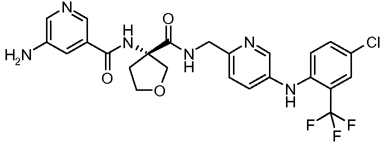 |
| (227) | 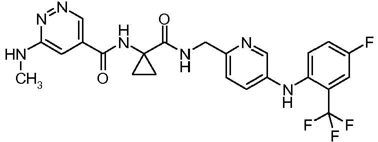 |
| (228) | 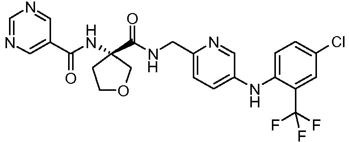 |
| (229) | 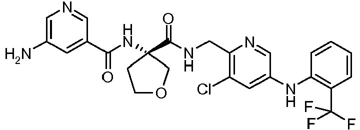 |
| (230) | 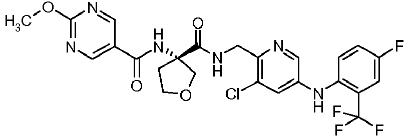 |
| (231) | 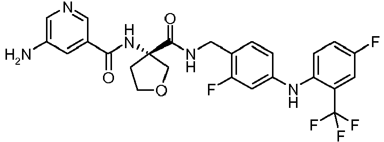 |
| (232) | 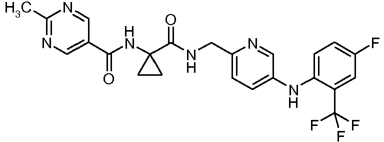 |
| (233) | 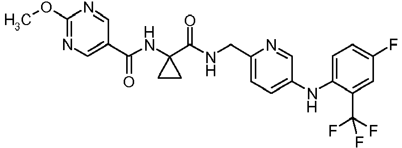 |
| (234) | 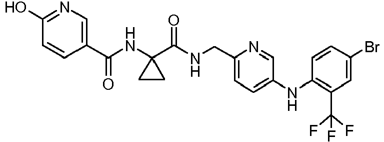 |
| (235) | 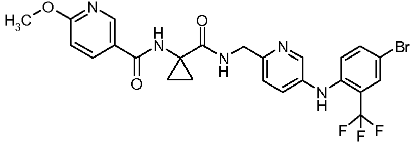 |
| (236) | 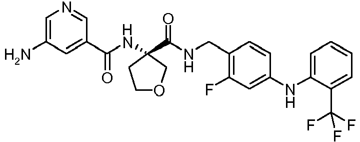 |
| (237) | 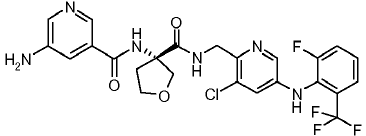 |
| (238) | 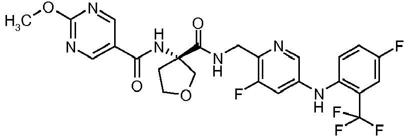 |
| (239) | 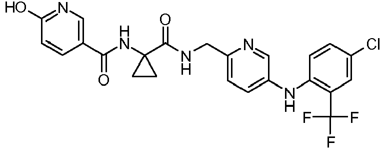 |
| (240) | 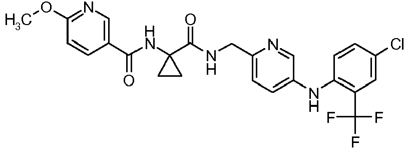 |
| (241) | 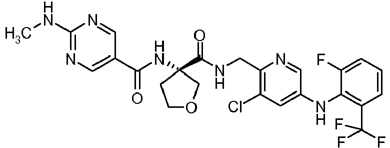 |
| (242) | 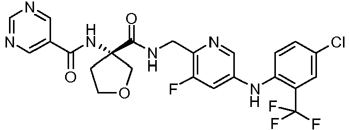 |
| (243) | 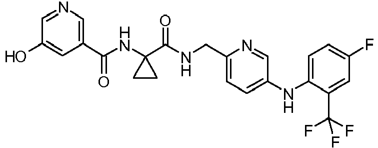 |
| (244) | 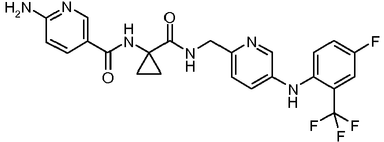 |
| (245) | 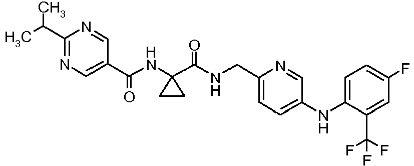 |
| (246) | 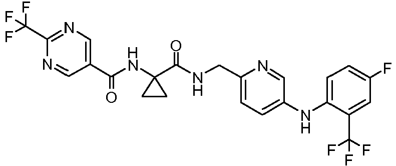 |
| (247) | 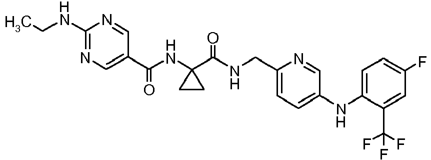 |
| (248) | 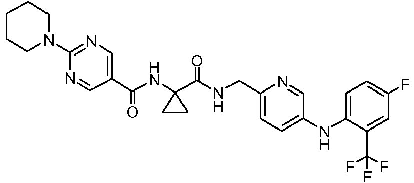 |
| (249) | 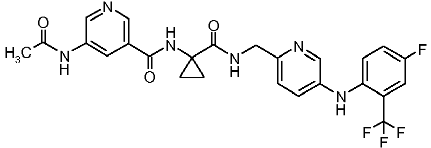 |
| (250) | 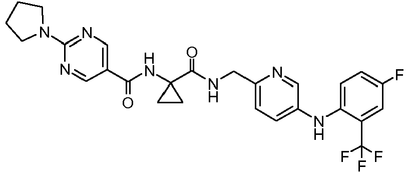 |
| (251) | 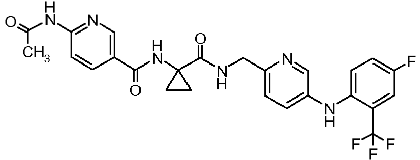 |
| (252) | 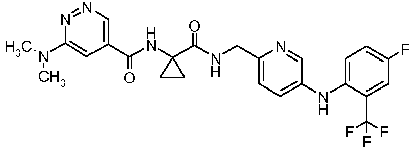 |
| (253) | 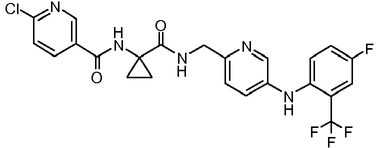 |
| (254) | 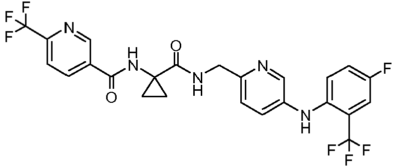 |
| (255) | 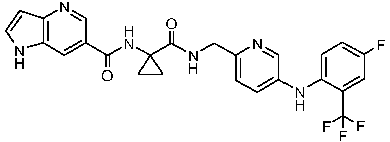 |
| (256) | 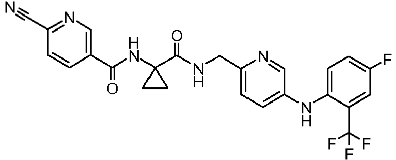 |
| (257) | 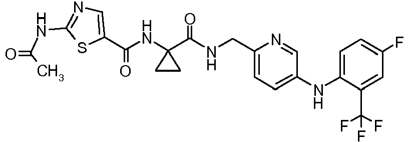 |
| (258) | 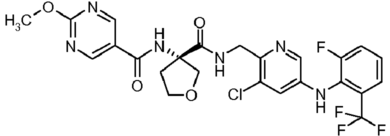 |
| (259) | 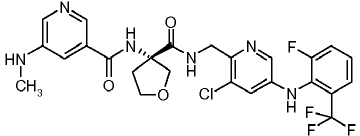 |
| (260) | 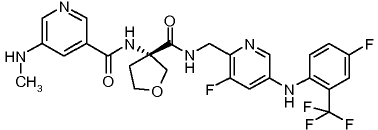 |
| (261) | 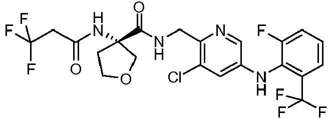 |
| (262) | 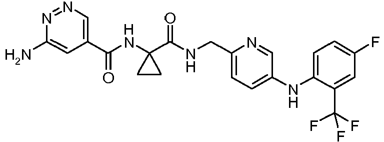 |
| (263) | 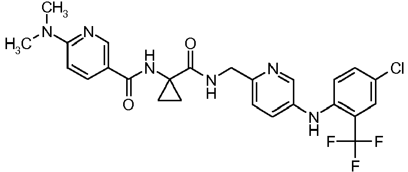 |
| (264) | 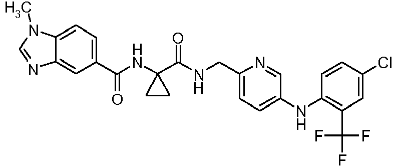 |
| (265) | 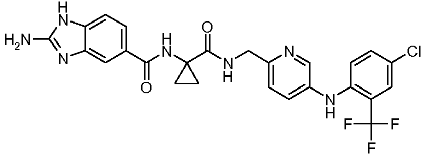 |
| (266) | 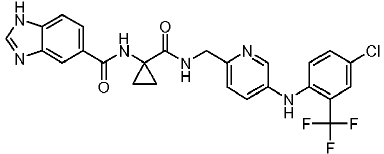 |
| (267) | 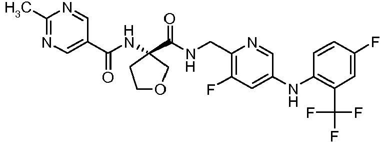 |
| (268) | 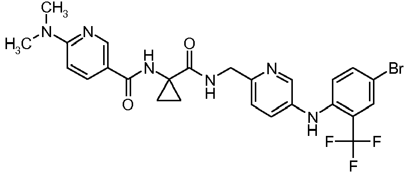 |
| (269) | 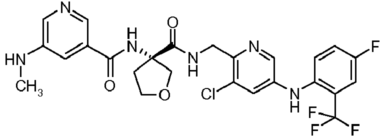 |
| (270) | 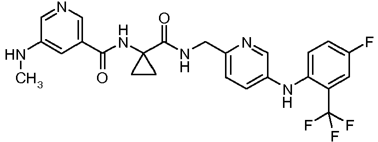 |
| (271) | 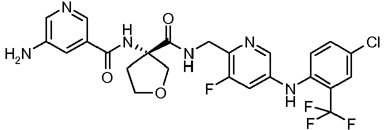 |
| (272) | 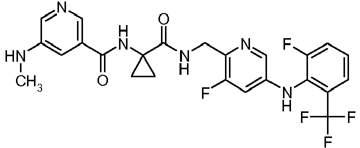 |
| (273) | 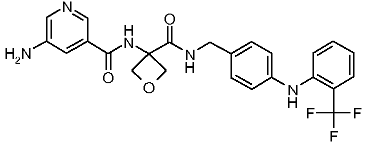 |
| (274) | 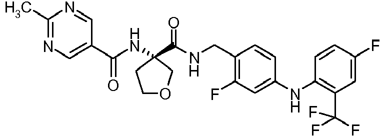 |
| (275) | 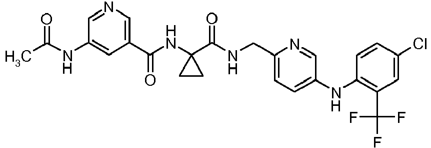 |
| (276) | 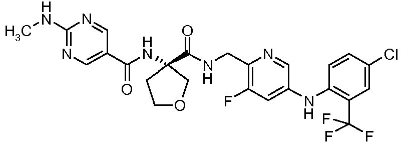 |
| (277) | 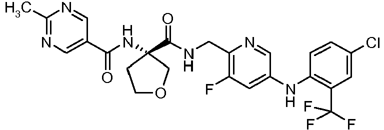 |
| (278) | 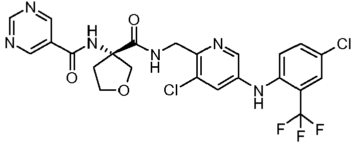 |
| (279) | 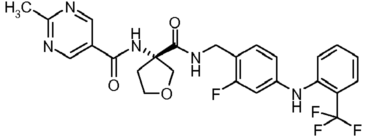 |
| (280) | 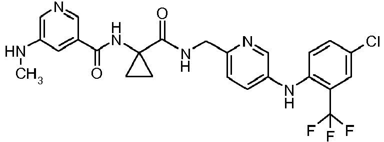 |
| (281) | 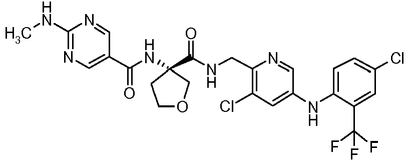 |
| (282) | 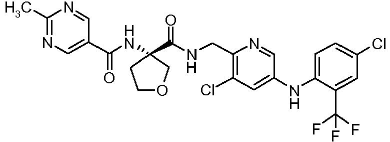 |
| (283) | 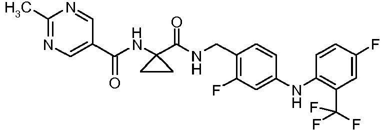 |
| (284) | 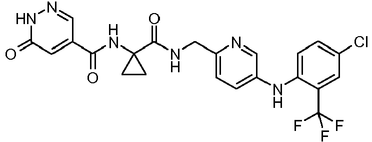 |
| (285) | 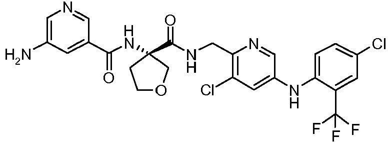 |
| (286) | 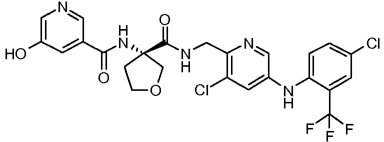 |
| (287) | 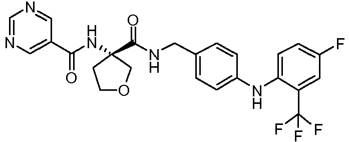 |
| (288) | 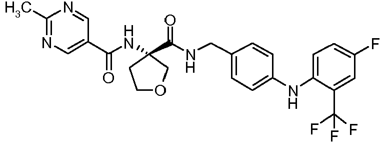 |
| (289) | 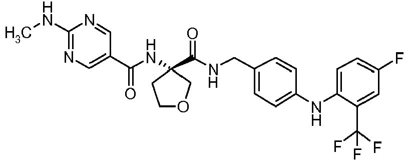 |
| (290) | 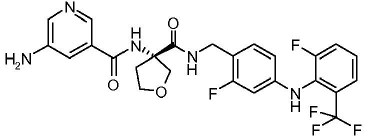 |
| (291) | 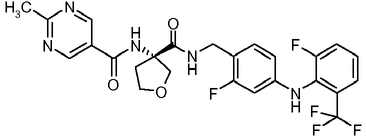 |
| (292) | 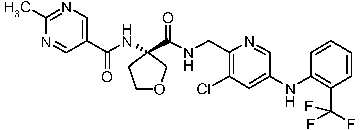 |
| (293) | 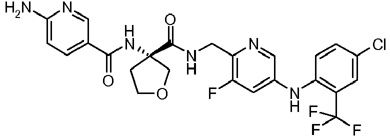 |
| (294) | 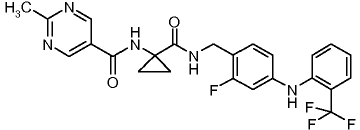 |
| (295) | 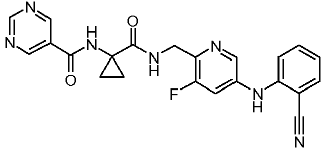 |
| (296) | 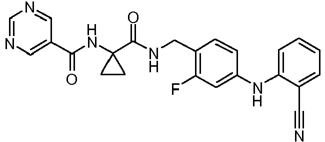 |
| (297) | 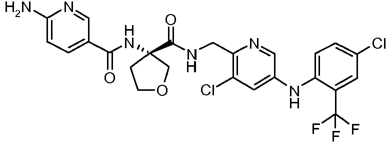 |
| (298) | 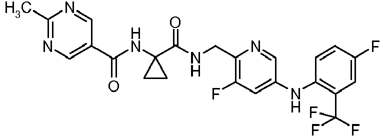 |
| (299) | 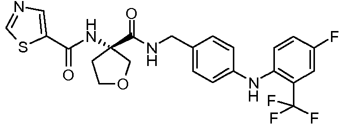 |
| (300) | 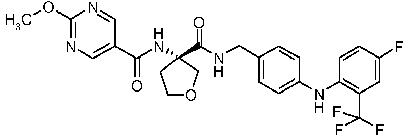 |
| (301) | 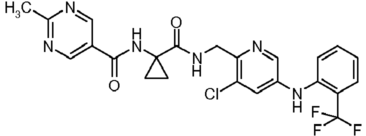 |
| (302) | 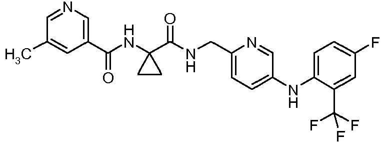 |
| (303) | 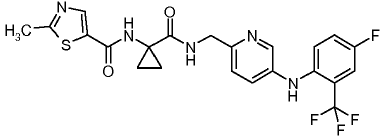 |
| (304) | 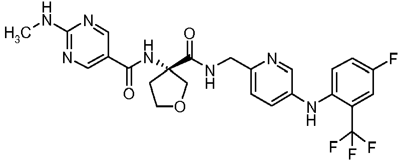 |
| (305) | 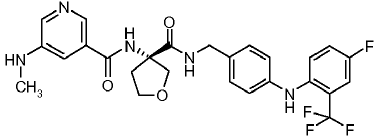 |
| (306) | 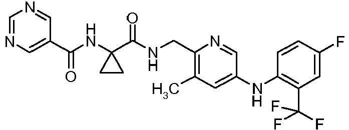 |
| (307) | 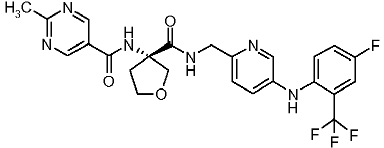 |
| (308) | 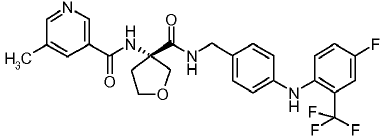 |
| (309) | 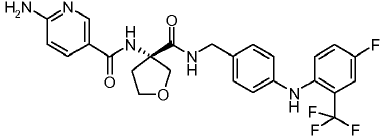 |
| (310) | 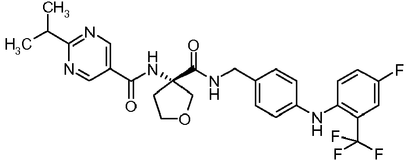 |
| (311) | 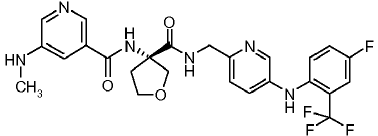 |
| (312) | 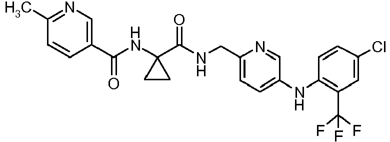 |
| (313) | 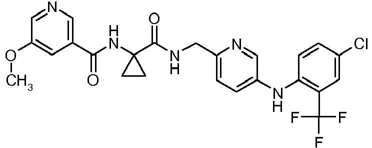 |
| (314) | 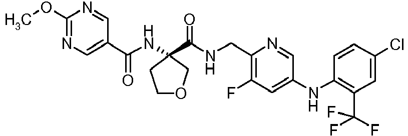 |
| (315) | 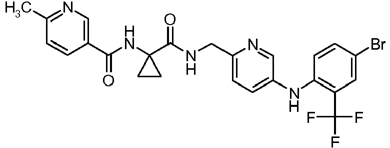 |
| (316) | 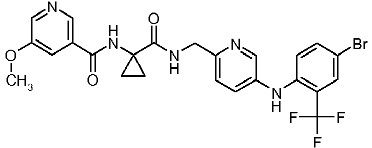 |
| (317) | 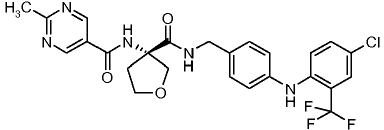 |
| (318) | 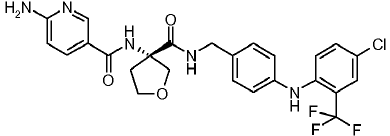 |
| (319) | 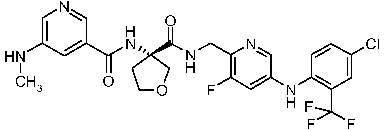 |
| (320) | 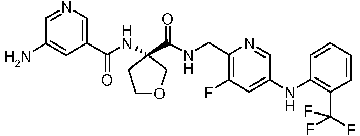 |
| (321) | 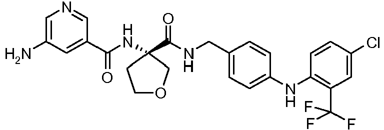 |
| (322) | 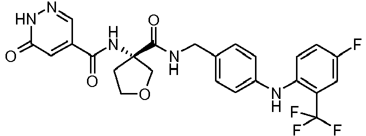 |
| (323) | 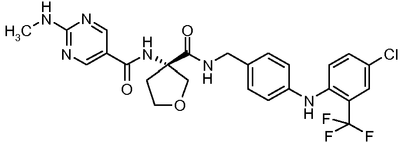 |
| (324) | 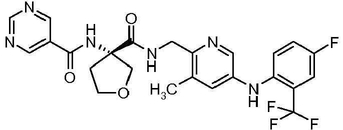 |
| (325) | 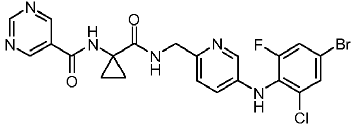 |
| (326) | 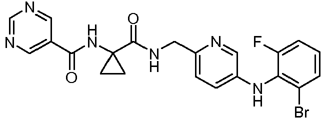 |
| (327) | 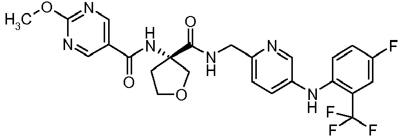 |
| (328) | 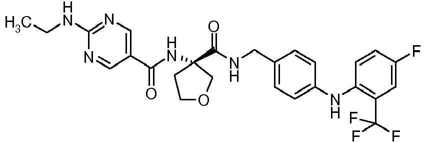 |
| (329) | 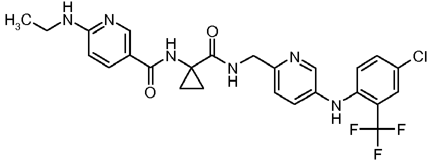 |
| (330) | 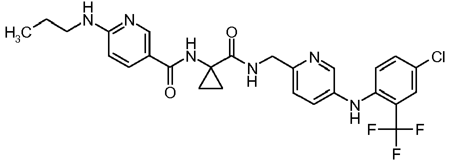 |
| (331) | 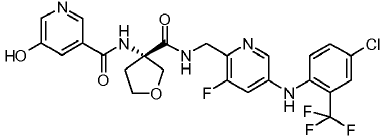 |
| (332) | 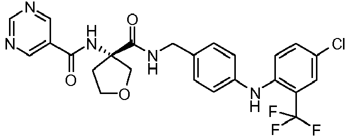 |
| (333) | 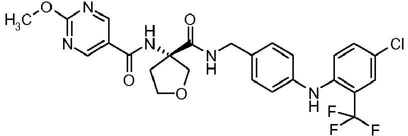 |
| (334) | 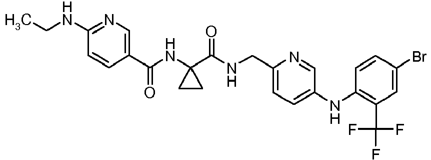 |
| (335) | 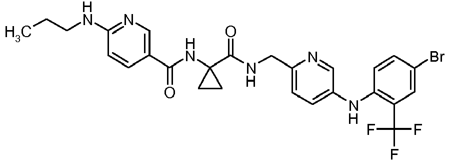 |
| (336) | 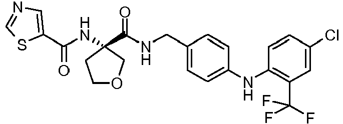 |
| (337) | 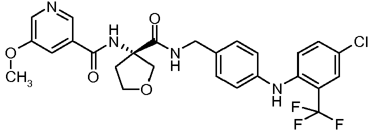 |
| (338) | 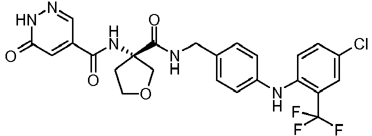 |
| (339) | 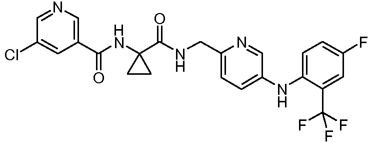 |
| (340) | 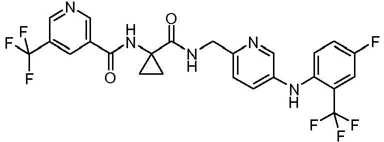 |
| (341) | 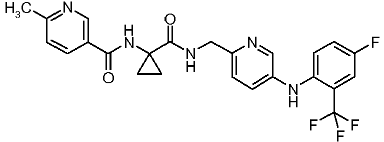 |
| (342) | 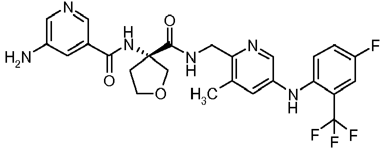 |
| (343) | 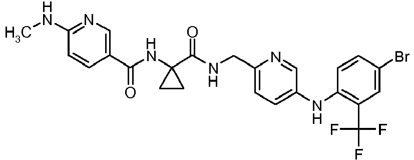 |
| (344) | 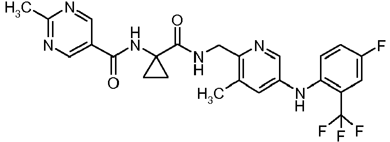 |
| (345) | 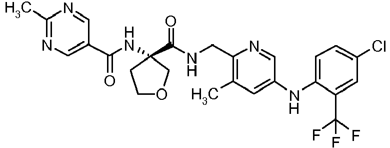 |
| (346) | 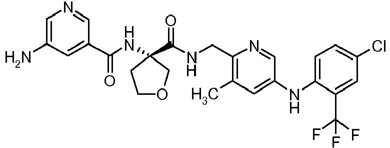 |
| (347) | 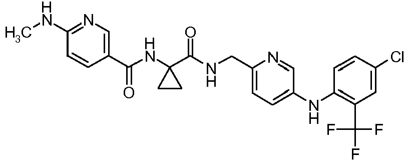 |
| (348) | 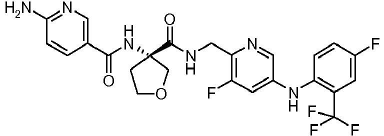 |
| (349) | 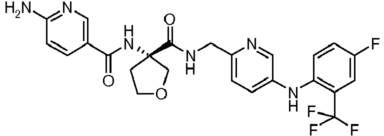 |
| (350) | 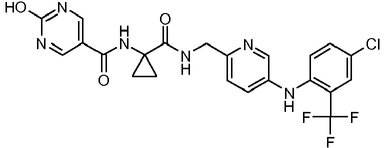 |
| (351) | 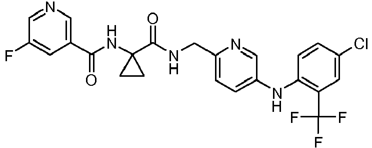 |
| (352) | 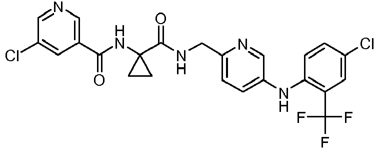 |
| (353) | 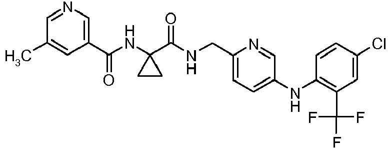 |
| (354) | 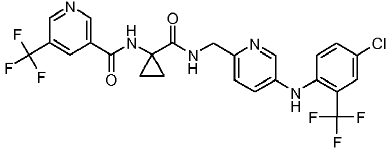 |
| (355) | 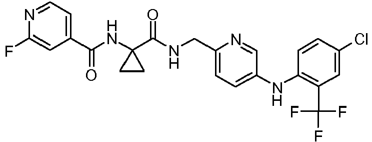 |
| (356) | 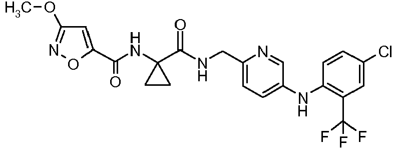 |
| (357) | 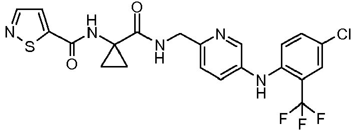 |
| (358) | 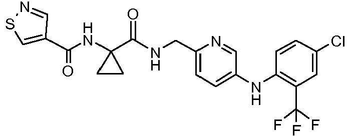 |
| (359) | 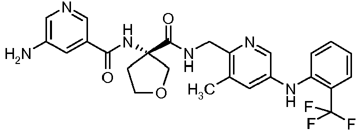 |
| (360) | 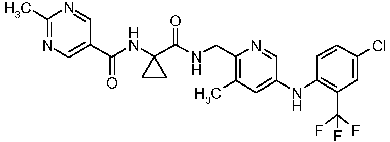 |
| (361) | 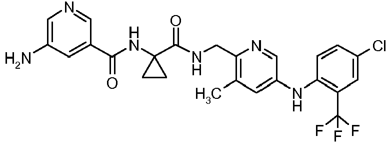 |
| (362) | 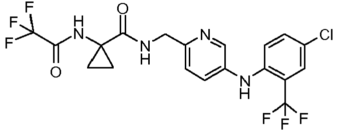 |
| (363) | 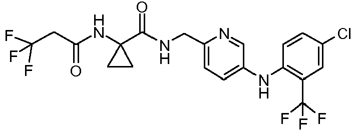 |
| (364) | 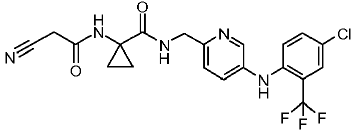 |
| (365) | 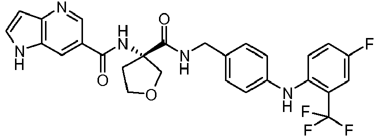 |
| (366) | 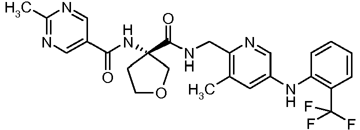 |
| (367) | 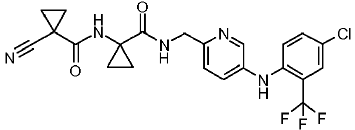 |
| (368) | 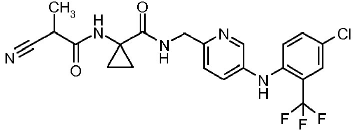 |
| (369) | 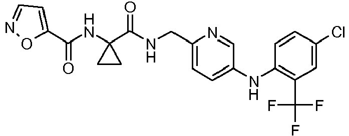 |
| (370) | 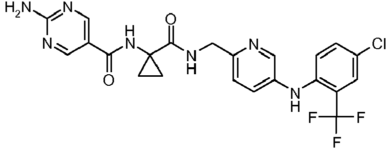 |
| (371) | 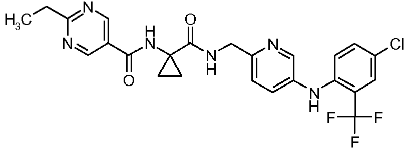 |
| (372) | 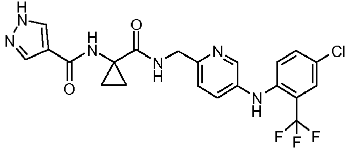 |
| (373) | 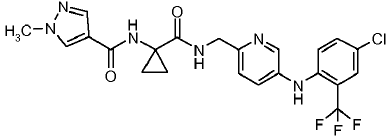 |
| (374) | 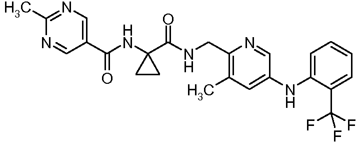 |
| (375) | 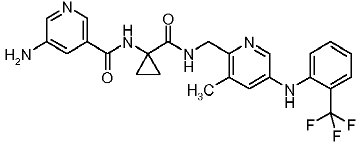 |
| (376) | 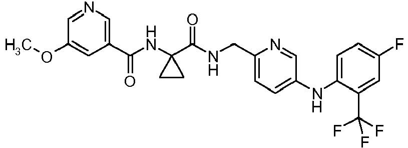 |
| (377) | 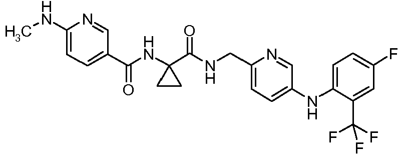 |
| (378) | 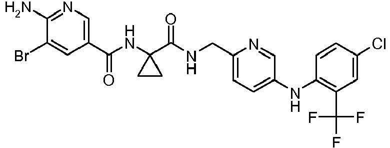 |
| (379) | 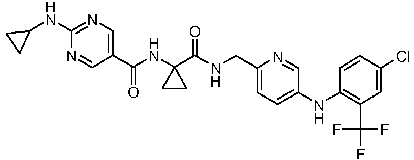 |
| (380) | 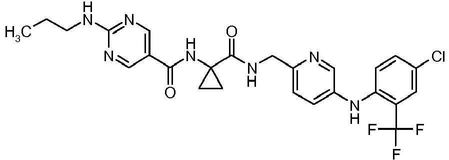 |
| (381) | 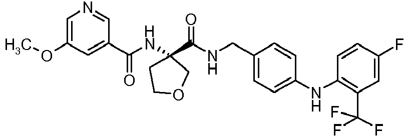 |
| (382) | 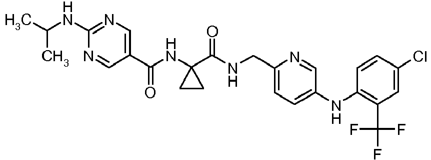 |
| (383) | 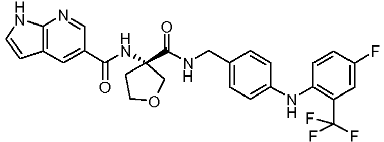 |
| (384) | 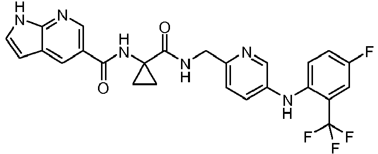 |
| (385) | 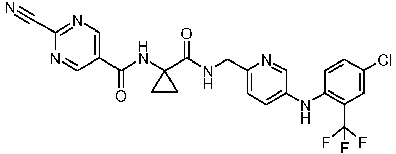 |
| (386) | 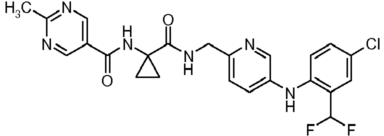 |
| (387) | 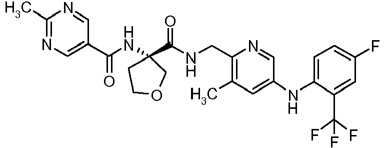 |
| (388) | 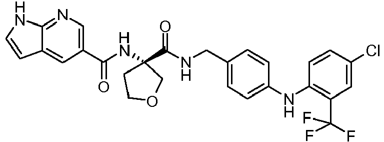 |
| (389) | 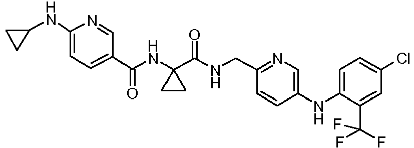 |
| (390) | 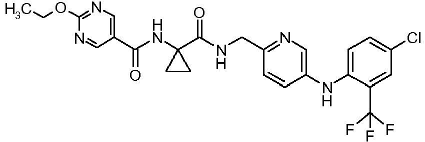 |
| (391) | 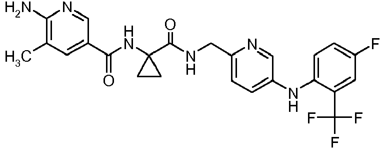 |
| (392) | 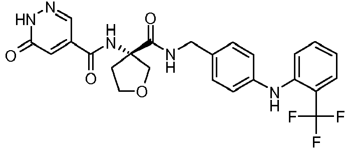 |
| (393) | 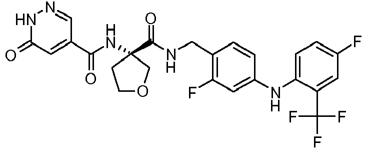 |
| (394) | 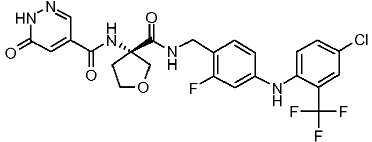 |
| (395) | 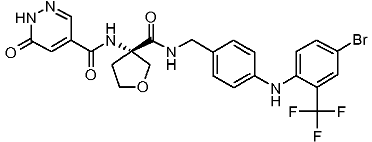 |
| (396) | 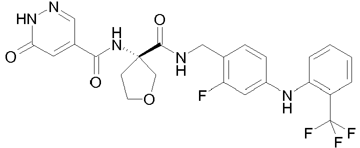 |
the enantiomers, the diastereomers, the mixtures and the salts thereof.

29. Physiologically acceptable salts of the compounds according to one of claims 1 to 28 with inorganic or organic acids or bases.

30. Medicaments, containing a compound according to at least one of claims 1 to 28 or a physiologically acceptable salt according to claim 29 optionally together with one or more inert carriers and/or diluents.

31. Compound of general formula I according to one of claims 1, 2, 3, 4, 5, 6, 7, 8, 9, 10, 11, 12, 13, 14, 15, 16, 17, 18, 19, 20, 21, 22, 23, 24, 25, 26, 27, 28 or 29 for use as medicaments.

32. Compound of general formula I according to one of claims 1, 2, 3, 4, 5, 6, 7, 8, 9, 10, 11, 12, 13, 14, 15, 16, 17, 18, 19, 20, 21, 22, 23, 24, 25, 26, 27, 28 or 29 for preparing a medicament for the acute and prophylactic treatment of acute pain, visceral pain, neuropathic pain, inflammatory / pain receptor-mediated pain, tumour pain and headache diseases.

33. Use of a compound according to at least one of claims 1 to 24 for preparing a medicament for the acute and prophylactic treatment of osteoarthritis.

34. Process for preparing a medicament according to claim 30, **characterised in that** a compound according to at least one of claims 1 to 29 is incorporated by a non-chemical method in one or more inert carriers and/or diluents.

## Revendications

1. Composés de formule générale I 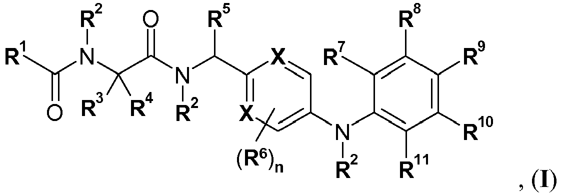 dans laquelle
**n** représente un des chiffres 0, 1 ou 2,
**R¹** représente
(a) un groupe alkyle en C₁₋₆ éventuellement substitué par un radical **R^{1.1}**,
(b) un groupe alkyle en C₁₋₃ dans lequel chaque groupe méthylène peut être substitué par 1 ou 2 atomes de fluor et chaque groupe méthyle par 1, 2 ou 3 atomes de fluor,
(c) un groupe cycloalkyle en C₃₋₆ éventuellement substitué par un radical **R**^{1.2}, dans lequel un motif -CH₂- peut être remplacé par un groupe -C(O)-
(d) un groupe aryl (alkylène en C₀₋₂) éventuellement substitué par 1, 2 ou 3 radicaux **R**^{1.3},
(e) un radical hétéroaryl (alkylène en C₀₋₂) à cinq membres, éventuellement substitué par 1, 2 ou 3 radicaux **R**^{1.4}, comprenant au moins un atome de N, 0 ou S et éventuellement en plus un, deux ou trois autres atomes de N et pouvant être en plus benzo-condensé,
(f) un radical hétéroaryl(alkylène en C₀₋₂) à six membres, éventuellement substitué par 1 ou 2 radicaux **R^{1.4}**, comprenant un, deux ou trois atomes de N et pouvant être en plus benzo-condensé,
(g) un radical hétéroaryle à neuf ou dix membres, éventuellement substitué par 1 ou 2 radicaux **R^{1.4},** comprenant un, deux ou trois atomes de N,
(h) un hétérocycle à 5 ou 6 membres, éventuellement substitué par 1 ou 2 radicaux **R^{1.4}**, dans lequel un motif -CH₂-peut être remplacé par un groupe -C(O)-
(i) -O-**R^{1.1.1}**,
(j) N**R^{1.1.3}**R**^{1.1.4}** ou
(k) C(=N**R**^{1.5})-CN,
**R^{1.1}** représente un atome d'halogène, un groupe -NO₂, -CN, cycloalkyle en C₃₋₆, -O**R^{1.1.1}**, -S**R^{1.1.1}**, -C(O)**R^{1.1.1}**, -S(O)₂-**R^{1.1.2}**, -O-S(O)₂-**R^{1.1.1}**, -CO₂**R^{1.1.1}**, -O-C(O)-**R^{1.1.1}**, -N**R^{1.1.3}R^{1.1.4}**, -N**R^{1.1.3}**-C(O)-**R^{1.1.1}**, -N**R^{1.1.3}**-C(O)-**R^{1.1.1}**, -N**R^{1.1.1}**-CO₂-**R^{1.1.1}** ou -C(O)-N**R^{1.1.3}R^{1.1.4}**,
**R^{1.1.1}** représente
(a) H,
(b) un groupe alkyle en C₁₋₄,
(c) un groupe alkyle en C₁₋₃, dans lequel chaque groupe méthylène peut être substitué par 1 ou 2 atomes de fluor et chaque groupe méthyle par 1, 2 ou 3 atomes de fluor,
(d) un groupe phényle éventuellement substitué par 1, 2 ou 3 radicaux **R^{1.1.1.1}**,
(e) un groupe cycloalkyle en C₃₋₆, ou
(f) un groupe pyridyle éventuellement substitué par 1, 2 ou 3 radicaux **R^{1.1.1.2}**,
**R^{1.1.1.1}** représente, indépendamment les uns des autres,
(a) un atome d'halogène, -NO₂, -CN, -OH, -O-alkyle en C₁₋₄, cycloalkyle en C₃₋₆, alkyle en C₁₋₄, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
(c) un groupe -O-alkyle en C₁₋₄, ou
(d) un groupe phényle éventuellement substitué par 1, 2 ou 3 radicaux **R**^{1.1.1.1},
**R**^{1.1.1.2} représente, indépendamment les uns des autres, un atome d'halogène ou un groupe alkyle en C₁₋₄,
**R**^{1.1.2} représente
(a) un groupe alkyle en C₁₋₄,
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
(c) un groupe -O-alkyle en C₁₋₄, ou
(d) un groupe phényle éventuellement substitué par 1, 2 ou 3 radicaux **R**^{1.1.1.1}, **R^{1.1.1.1}**,
**R^{1.1.4}** représentent, indépendamment les uns des autres
(a) H,
(b) un groupe alkyle en C₁₋₄, éventuellement substitué par 1, 2 ou 3 radicaux **R^{1.1.4.1}**,
(c) un groupe phényle éventuellement substitué par 1, 2 ou 3 radicaux **R^{1.1.1.1}**,
(d) un groupe cycloalkyle en C₃₋₆, ou
**R^{1.1.3}** et **R^{1.1.4}** représentent, conjointement avec l'atome de N auquel ils sont reliés, un cycle hétérocyclique à 5 ou 6 membres, qui peut comprendre en plus un autre hétéroatome choisi parmi N, 0 et S, ou
**R^{1.1.3}** et **R^{1.1.4}** forment, conjointement avec l'atome de N auquel ils sont reliés, un imide cyclique,
**R^{1.1.4.1}** représente, indépendamment les uns des autres, un atome d'halogène, un groupe -NH₂, -NH(alkyle en C₁₋₄), -N(alkyle en C₁₋₄)₂ ou -SO₂-**R^{1.1.2}**,
**R^{1.2}** représente un atome d'halogène, un groupe -NO₂, -CN, OH, -O-CH₃ ou phényle,
R**^{1.3}** représente
(a) un atome d'halogène, -NO₂, -CN, -O**R^{1.1.1}**, -S**R^{1.1.1}**, -CO₂**R^{1.1.1}**, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
R^{1.4} représente, indépendamment les uns des autres,
(a) un atome d'halogène, -NO₂, -CN, -O**R^{1.1.1}**, -S**R^{1.1.1}**, -S(O)-**R^{1.1.2}**, -S(O)₂-R^{1.1.2}, -N**R^{1.1.3}R^{1.1.4}**, -N(**R^{1.4.1}**)-C(O)-alkyle en C₁₋₄, alkyle en C₁₋₆,
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
(c) un groupe oxo,
**R^{1.4.1}** représente H ou alkyle en C₁₋₄,
**R^{1.5}** représente -OH ou -O-alkyle en C₁₋₃,
**R²** représente
(a) H,
(b) alkyle en C₁₋₄,
(c) alkyle en C₁₋₄-C(O)-,
**R³** et **R⁴** représentent, conjointement avec l'atome de carbone auquel ils sont reliés, un groupe cycloalkylène en C₃₋₆, éventuellement substitué par un radical **R^{3.1}**, dans lequel un motif -CH₂- peut être remplacé par un hétéroatome 0, N, S ou par un groupe CO, SO ou SO₂,
**R^{3.1}** représente H, -OH,
**R⁵** représente
(a) H,
(b) un groupe alkyle en C₁₋₄,
(c) un groupe alkyle en C₁₋₃, dans lequel chaque groupe méthylène peut être substitué par 1 ou 2 atomes de fluor et chaque groupe méthyle par 1, 2 ou 3 atomes de fluor,
**R⁶** représente, indépendamment les uns des autres,
(a) H, un atome d'halogène, -CN, -OH, alkyle en C₁₋₆, cycloalkyle en C₃₋₇, -O-alkyle en C₁₋₄, -O-CF₃, -O-cycloalkyle en C₃₋₆, -N (alkyle en C₁₋₃)₂, -C(O)-NH₂, -(SO₂)NH₂, -SO₂- alkyle en C₁₋₃, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
**R⁷** représente
(a) H, un atome d'halogène, -CN, -OH,
(b) un groupe alkyle en C₁₋₆,
(c) un groupe alkyle en C₁₋₃ ou O-alkyle en C₁₋₃, dans lequel chaque groupe méthylène peut être substitué par 1 ou 2 atomes de fluor et chaque groupe méthyle par 1, 2 ou 3 atomes de fluor,
(d) cycloalkyle en C₃₋₇,
(e) -O-alkyle en C₁₋₆,
(f) -O-cycloalkyle en C₃₋₇,
(g) -NH₂, -NH(alkyle en C₁₋₃), -N(alkyle en C₁₋₃)₂,
(h) -C(O)-**R^{7.1}**,
(i) -S-alkyle en C₁₋₄, -SO₂-**R^{7.2}**,
(j) un groupe hétéroaryle à cinq membres éventuellement substitué par un ou deux groupes alkyle en C₁₋₃, choisi dans le groupe constitué par le pyrrolyle, l'oxazolyle, l'isoxazolyle, l'oxadiazolyle, le thiazolyle, l'isothiazolyle, le thiadiazolyle, l'imidazolyle, le pyrazolyle, le triazolyle et le tétrazolyle, ou
(k) un groupe hétéroaryle à six membres éventuellement substitué par un ou deux groupes alkyle en C₁₋₃, choisi dans le groupe constitué par le pyridyle, le pyrimidinyle, le pyrazinyle, le pyridazinyle et le triazinyle,
**R^{7.1}** représente -NH₂, -NH(alkyle en C₁₋₆), -N(alkyle en C₁₋₆)₂, *N*-acétidinyle, *N*-pyrrolidinyle, *N*-pipéridinyle, *N*-morpholinyle, -OH, -O-alkyle en C₁₋₈ ou -O-cycloalkyle en C₃₋₇,
**R^{7.2}** représente -NH₂, -NH(alkyle en C₁₋₆), -N(alkyle e n C₁₋₆)₂, *N*-acétidinyle, *N*-pyrrolidinyle, *N*-pipéridinyle ou *N*-morpholinyle, et
**R⁸** représente H, un atome d'halogène, alkyle en C₁₋₄,
**R⁹** représente
(a) H, un atome d'halogène, -CN, -OH,
(b) un groupe alkyle en C₁₋₆,
(c) un groupe alkyle en C₁₋₃ ou O-alkyle en C₁₋₃, dans lequel chaque groupe méthylène peut être substitué par 1 ou 2 atomes de fluor et chaque groupe méthyle par 1, 2 ou 3 atomes de fluor,
(d) cycloalkyle en C₃₋₇,
(e) alcinyle en C₂₋₄,
(f) -O-alkyle en C₁₋₆,
(g) -O-cycloalkyle en C₃₋₇,
(h) -NH₂, -NH(alkyle en C₁₋₃), -N(alkyle en C₁₋₃)₂,
(i) -C(O)-**R^{9.1}**,
(j) -S-alkyle en C₁₋₄, -SO-alkyle en C₁₋₄, - SO₂-alkyle en C₁₋₄,
**R^{9.1}** représente -NH₂, -NH(alkyle en C₁₋₆), -N(alkyle e n C₁₋₆)₂, *N*-acétidinyle, *N*-pyrrolidinyle, *N*-pipéridinyle, *N*-morpholinyle, -OH, -O-alkyle en C₁₋₈ ou -O-cycloalkyle en C₃₋₇,
**R¹⁰** représente H, un atome d'halogène, alkyle en C₁₋₄,
**R¹¹** représente
(a) H, un atome d'halogène, -CN, -OH,
(b) un groupe alkyle en C₁₋₆,
(c) un groupe alkyle en C₁₋₃ ou O-alkyle en C₁₋₃, dans lequel chaque groupe méthylène peut être substitué par 1 ou 2 atomes de fluor et chaque groupe méthyle par 1, 2 ou 3 atomes de fluor,
(d) cycloalkyle en C₃₋₇,
(e) -O-alkyle en C₁₋₆,
(f) -O-cycloalkyle en C₃₋₇,
(g) -NH₂, -NH(alkyle en C₁₋₃), -N(alkyle en C₁₋₃)₂,
(h) -C(O)-**R^{11.1}**,
(i) -S-alkyle en C₁₋₄, -SO₂-**R^{11.2}**,
(j) un groupe hétéroaryle à cinq membres éventuellement substitué par un ou deux groupes alkyle en C₁₋₃, choisi dans le groupe constitué par le pyrrolyle, l'oxazolyle, l'isoxazolyle, l'oxadiazolyle, le thiazolyle, l'isothiazolyle, le thiadiazolyle, l'imidazolyle, le pyrazolyle, le triazolyle et le tétrazolyle, ou
(k) un groupe hétéroaryle à six membres éventuellement substitué par un ou deux groupes alkyle en C₁₋₃, choisi dans le groupe constitué par le pyridyle, le pyrimidinyle, le pyrazinyle, le pyridazinyle et le triazinyle,
**R^{11.1}** représente -NH₂, -NH(alkyle en C₁₋₆), -N (alkyle en C₁₋₆)₂, *N*-acétidinyle, *N*-pyrrolidinyle, *N*-pipéridinyle, *N*-morpholinyle, -OH, -O-alkyle en C₁₋₈ ou -O-cycloalkyle en C₃₋₇,
**R^{11.2}** représente -NH₂, -NH(alkyle en C₁₋₆), -N(alkyle en C₁₋₆)₂, N-acétidinyle, N-pyrrolidinyle, N-pipéridinyle ou N-morpholinyle, et
**X** représente, indépendamment les uns des autres, C-**R⁶** ou N,
leurs énantiomères, leurs diastéréomères, leurs mélanges et leurs sels.

2. Composés de formule générale **I** selon la revendication 1, dans lesquels **R², R³, R⁴, R⁵, R⁶, R⁷, R⁸, R⁹, R¹⁰, R¹¹, n** et **X** sont définis tels que dans la revendication 1 et
**R¹** représente
(a) un groupe alkyle en C₁₋₆ éventuellement substitué par un radical **R^{1.1}**,
(b) un groupe alkyle en C₁₋₃ dans lequel chaque groupe méthylène peut être substitué par 1 ou 2 atomes de fluor et chaque groupe méthyle par 1, 2 ou 3 atomes de fluor,
(c) un groupe cycloalkyle en C₃₋₆ éventuellement substitué par un radical **R^{1.2}**, dans lequel un motif -CH₂- peut être remplacé par un groupe -C(O)-
(d) un groupe phényle éventuellement substitué par 1, 2 ou 3 radicaux **R^{1.3},**
(e) un radical hétéroaryle à cinq membres, éventuellement substitué par 1, 2 ou 3 radicaux **R^{1.4},** comprenant au moins un atome de N, 0 ou S et éventuellement en plus un, deux ou trois autres atomes de N,
(f) un radical hétéroaryle à six membres, éventuellement substitué par 1 ou 2 radicaux **R^{1.4}** comprenant un, deux ou trois atomes de N,
(g) un radical hétéroaryle à neuf ou dix membres, éventuellement substitué par 1 ou 2 radicaux **R^{1.4}** comprenant un, deux ou trois atomes de N,
(h) un hétérocycle à 5 ou 6 membres, éventuellement substitué par 1 ou 2 radicaux **R^{1.4},** dans lequel un motif -CH₂-peut être remplacé par un groupe -C(O)-
(i) **-**O**-R^{1.1.1},** ou
(j) N**R^{1.1.3}R^{1.1.4},**
**R^{1.1}** représente -CN, cycloalkyle en C₃₋₆, -O**R^{1.1.1}**, -N**R^{1.1.3}R^{1.1.4}**,
**R^{1.1.1}** représente
(a) H,
(b) un groupe alkyle en C₁₋₄,
(c) un groupe alkyle en C₁₋₃, dans lequel chaque groupe méthylène peut être substitué par 1 ou 2 atomes de fluor et chaque groupe méthyle par 1, 2 ou 3 atomes de fluor,
**R^{1.1.3}**,
**R^{1.1.4}** représentent, indépendamment les uns des autres
(a) H,
(b) un groupe alkyle en C₁₋₄,
(c) un groupe cycloalkyle en C₃₋₆, ou
**R^{1.1.3}** et **R^{1.1.4}** représentent, conjointement avec l'atome de N auquel ils sont reliés, un cycle hétérocyclique à 5 ou 6 membres, qui peut comprendre en plus un autre hétéroatome choisi parmi N, 0 et S, ou
**R^{1.2}** représente un atome d'halogène, un groupe -NO₂, -CN, OH, -O-CH₃ ou phényle,
**R^{1.3}** représente, indépendamment les uns des autres,
(a) un atome d'halogène, -NO₂, -CN, -O**R^{1.1.1}**, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
**R^{1.4}** représente, indépendamment les uns des autres,
(a) un atome d'halogène, -NO₂, -CN, -O**R^{1.1.1}**, -N**R^{1.1.3}R^{1.1.4}**, -N(**R^{1.4.1}**)-C(O)-alkyle en C₁₋₄, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
**R^{1.4.1}** représente H ou alkyle en C₁₋₄,
leurs énantiomères, leurs diastéréomères, leurs mélanges et leurs sels.

3. Composés de formule générale **I** selon la revendication 1, dans lesquels **R², R³, R⁴, R⁵, R⁶, R⁷, R⁸, R⁹, R¹⁰, R¹¹, n** et **X** sont définis tels que dans la revendication 1 et
R¹ représente
(a) un groupe alkyle en C₁₋₆ éventuellement substitué par un radical **R^{1.1},**
(b) un groupe phényle éventuellement substitué par 1, 2 ou 3 radicaux **R^{1.3},**
(c) un radical hétéroaryle à cinq membres, éventuellement substitué par 1, 2 ou 3 radicaux **R^{1.4},** comprenant au moins un atome de N, 0 ou S et éventuellement en plus un, deux ou trois autres atomes de N,
(d) un radical hétéroaryle à six membres, éventuellement substitué par 1 ou 2 radicaux **R^{1.4},** comprenant un, deux ou trois atomes de N,
(e) un radical hétéroaryle à neuf ou dix membres, éventuellement substitué par 1 ou 2 radicaux **R^{1.4},** comprenant un, deux ou trois atomes de N,
(f) un hétérocycle à 5 ou 6 membres, éventuellement substitué par 1 ou 2 radicaux **R^{1.4},** dans lequel un motif -CH₂-peut être remplacé par un groupe -C(O)-,
**R^{1.1}** représente -CN, cycloalkyle en C₃₋₆, -OH, -OCH₃, -NH₂, -NHCH₃, -N(CH₃)₂,
**R^{1.3}** représente, indépendamment les uns des autres,
(a) F, Cl, Br, -OH, -OCH₃, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor, et
**R^{1.4}** représente, indépendamment les uns des autres,
(a) F, Cl, Br, -OH, -OCH₃, -NH₂, -NHCH₃, -N(CH₃)₂, -NH (alkyle en C₂₋₃)-, -N (alkyle en C₂₋₃)₂, -NH-C(O)-alkyle en C₁₋₄, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
leurs énantiomères, leurs diastéréomères, leurs mélanges et leurs sels, en particulier leurs sels physiologiquement acceptables avec des acides ou des bases, organiques ou minéraux.

4. Composés de formule générale **I** selon la revendication 1, dans lesquels **R², R³, R⁴, R⁵, R⁶, R⁷, R⁸, R⁹, R¹⁰, R¹¹, n** et **X** sont définis tels que dans la revendication 1 et
**R¹** représente
(a) un groupe alkyle en C₁₋₆ éventuellement substitué par un radical **R^{1.1},**
(b) un groupe phényle éventuellement substitué par 1, 2 ou 3 radicaux **R^{1.3},**
(c) un radical hétéroaryle à cinq membres, éventuellement substitué par 1, 2 ou 3 radicaux **R^{1.4},** choisi dans le groupe constitué par : 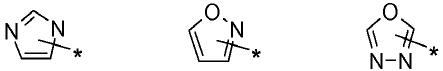 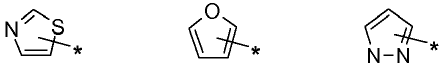 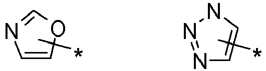
(d) un radical hétéroaryle à six membres, éventuellement substitué par 1 ou 2 radicaux **R^{1.4},** choisi dans le groupe constitué par : 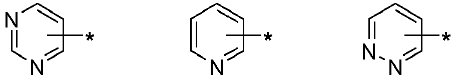 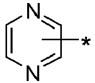
(e) un radical hétéroaryle à neuf membres, éventuellement substitué par 1 ou 2 radicaux **R^{1.4},** choisi dans le groupe constitué par : 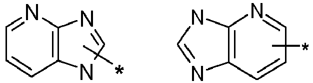
(f) un hétérocycle à 5 ou 6 membres, éventuellement substitué par 1 ou 2 radicaux **R^{1.4},** choisi dans le groupe constitué par : 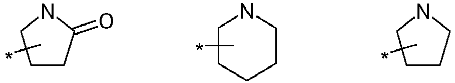 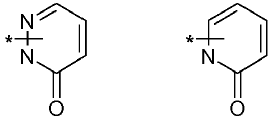
R^{1.1} représente -CN, cyclopropyle, -OH, -OCH₃, -NH₂, -NHCH₃, -N(CH₃)₂,
**R^{1.3}** représente, indépendamment les uns des autres,
(a) F, Cl, Br, -OH, -OCH₃, -OCF₃, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor, et
**R^{1.4}** représente, indépendamment les uns des autres,
(a) F, Cl, Br, -OH, -OCH₃, -OCF₃, -NH₂, -NH(alkyle en C₁₋₄)-, -N(alkyle en C₁₋₄)₂, -NH-C(O)-alkyle en C₁₋₄, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
leurs énantiomères, leurs diastéréomères, leurs mélanges et leurs sels.

5. Composés de formule générale **I** selon la revendication 1, dans lesquels **R², R³, R⁴, R⁵, R⁶, R⁷, R⁸, R⁹, R¹⁰, R¹¹, n** et **X** sont définis tels que dans la revendication 1 et
**R¹** est choisi dans le groupe constitué par : 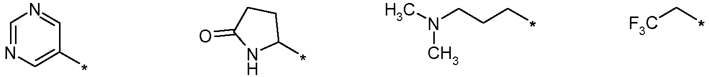 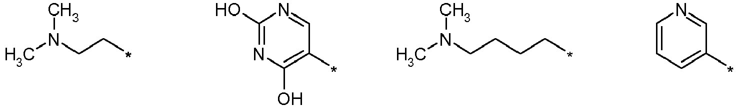 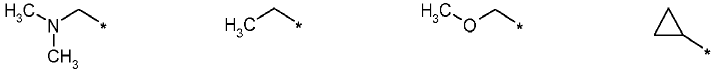 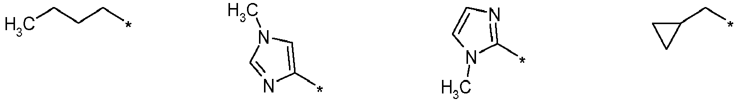 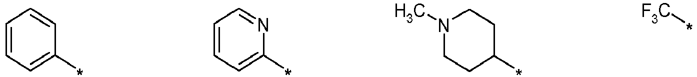 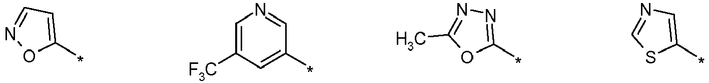 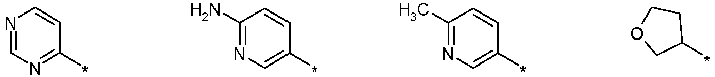 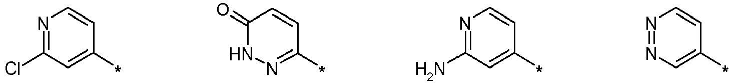 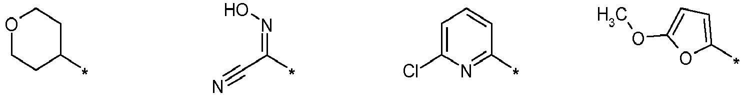 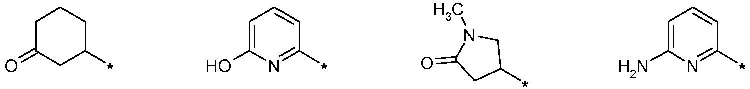 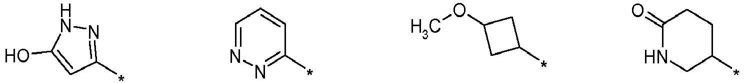 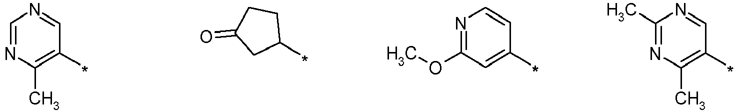 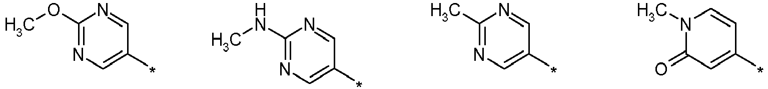 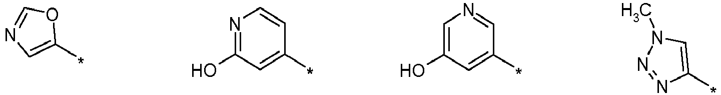 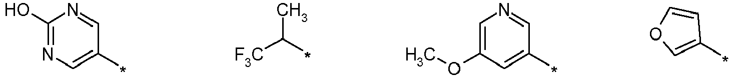 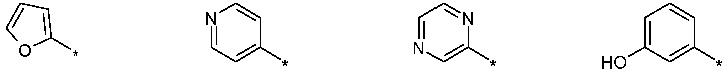 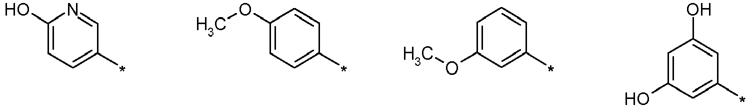 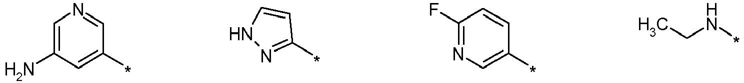 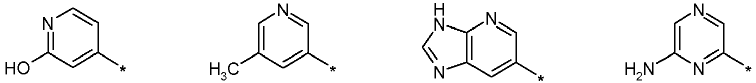 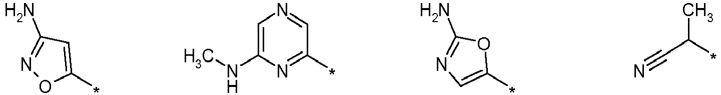 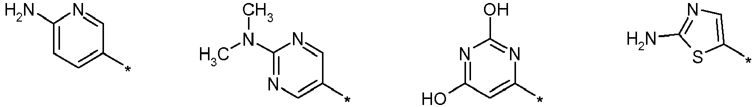 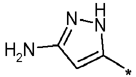 leurs énantiomères, leurs diastéréomères, leurs mélanges et leurs sels.

6. Composés de formule générale **I** selon la revendication 1, dans lesquels **R², R³, R⁴, R⁵, R⁶, R⁷, R⁸, R⁹, R¹⁰, R¹¹, n** et **X** sont définis tels que dans la revendication 1 et
**R¹** est choisi dans le groupe constitué par : 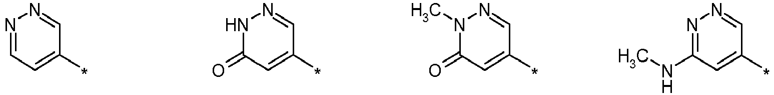 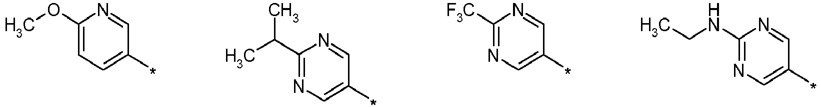 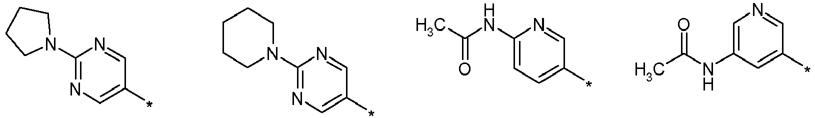 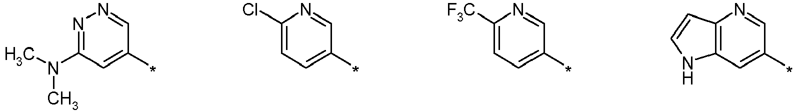 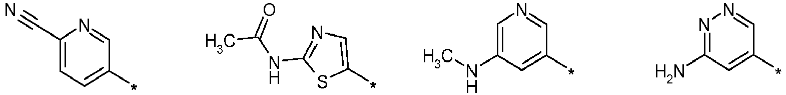 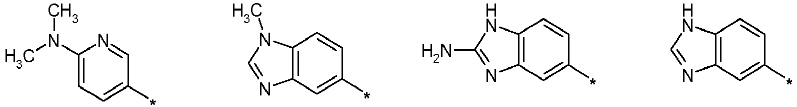 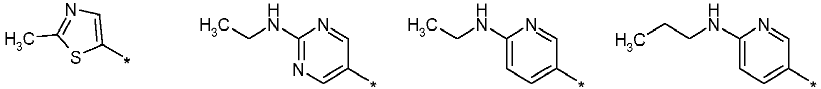 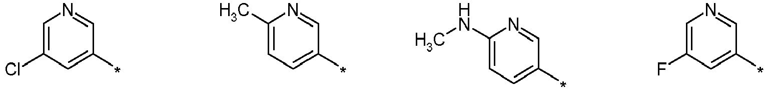 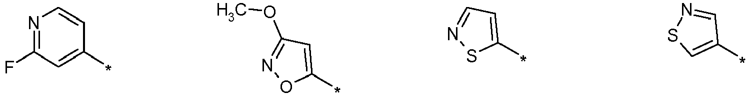 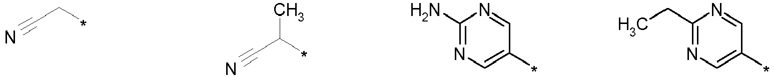 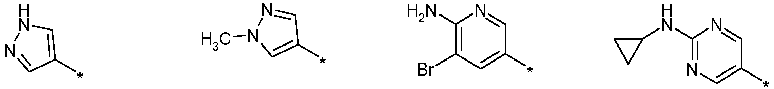 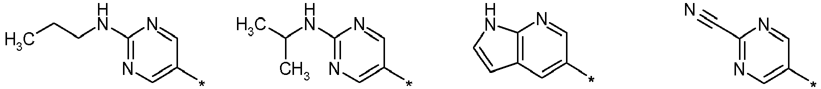 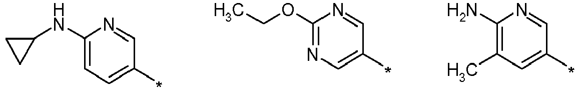 leurs énantiomères, leurs diastéréomères, leurs mélanges et leurs sels, en particulier leurs sels physiologiquement acceptables avec des acides ou des bases, organiques ou minéraux.

7. Composés de formule générale **I** selon la revendication 1, dans lesquels **R¹** est défini tel que mentionné dans la revendication 1, 2, 3, 4, 5 ou 6 et
**n** représente un des chiffres 0, 1 ou 2,
**R²** représente
(a) H,
(b) alkyle en C₁₋₄,
**R³** et **R⁴** représentent, conjointement avec l'atome de carbone auquel ils sont reliés, un groupe cycloalkylène en C₃₋₆, éventuellement substitué par un radical **R^{3.1},** dans lequel un motif -CH₂- peut être remplacé par un hétéroatome 0, N, S ou par un groupe CO, SO ou SO₂,
**R^{3.1}** représente H, -OH,
**R⁵** représente
(a) H,
(b) un groupe alkyle en C₁₋₄,
(c) un groupe alkyle en C₁₋₃, dans lequel chaque groupe méthylène peut être substitué par 1 ou 2 atomes de fluor et chaque groupe méthyle par 1, 2 ou 3 atomes de fluor,
**R⁶** représente, indépendamment les uns des autres,
(a) H, un atome d'halogène, -CN, -OH, alkyle en C₁₋₆, cycloalkyle en C₃₋₇, -O-alkyle en C₁₋₄, -O-CF₃, -O-cycloalkyle en C₃₋₆, -N(alkyle en C₁₋₃)₂, -C(O)-NH₂, -(SO₂)NH₂, -SO₂-alkyle en C₁₋₃, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
**R⁷** représente
(a) H, un atome d'halogène, -CN, -OH,
(b) un groupe alkyle en C₁₋₆,
(c) un groupe alkyle en C₁₋₃ ou O-alkyle en C₁₋₃, dans lequel chaque groupe méthylène peut être substitué par 1 ou 2 atomes de fluor et chaque groupe méthyle par 1, 2 ou 3 atomes de fluor,
(d) cycloalkyle en C₃₋₇,
(e) -O-alkyle en C₁₋₆,
(f) -O-cycloalkyle en C₃₋₇,
(g) -NH₂, -NH(alkyle en C₁₋₃), -N(alkyle en C₁₋₃)₂,
(h) -C(O)-**R^{7.1}**,
(i) -S-alkyle en C₁₋₄,
**R^{7.1}** représente -NH₂, -OH, -O-alkyle en C₁₋₈,
**R⁸** représente H, un atome d'halogène, alkyle en C₁₋₄,
**R⁹** représente
(a) H, un atome d'halogène, -CN, -OH,
(b) un groupe alkyle en C₁₋₆,
(c) un groupe alkyle en C₁₋₃ ou O-alkyle en C₁₋₃, dans lequel chaque groupe méthylène peut être substitué par 1 ou 2 atomes de fluor et chaque groupe méthyle par 1, 2 ou 3 atomes de fluor,
(d) cycloalkyle en C₃₋₇,
(e) alcinyle en C₂₋₄,
(f) -O-alkyle en C₁₋₆,
(g) -O-cycloalkyle en C₃₋₇,
(h) -NH₂, -NH(alkyle en C₁₋₃), -N(alkyle en C₁₋₃)₂,
(i) -C(O) **-R^{9.1},**
(j) -S-alkyle en C₁₋₄, -SO-alkyle en C₁₋₄, - SO₂-alkyle en C₁₋₄,
**R^{9.1}** représente -NH₂, -OH, -O-alkyle en C₁₋₈, **R¹⁰** représente H, un atome d'halogène, alkyle en C₁₋₄,
**R¹¹** représente
(a) H, un atome d'halogène, -CN, -OH,
(b) un groupe alkyle en C₁₋₆,
(c) un groupe alkyle en C₁₋₃ ou -O-alkyle en C₁₋₃, dans lequel chaque groupe méthylène peut être substitué par 1 ou 2 atomes de fluor et chaque groupe méthyle par 1, 2 ou 3 atomes de fluor,
(d) cycloalkyle en C₃₋₇,
(e) -O-alkyle en C₁₋₆,
(f) -O-cycloalkyle en C₃₋₇,
(g) -NH₂, -NH(alkyle en C₁₋₃), -N(alkyle en **C₁₋₃)₂,**
(h) -C(O)-**R^{11.1}**,
(i) -S-alkyle en C₁₋₃,
**R^{11.1}** représente -NH₂, -OH, -O-alkyle en C₁₋₈, et
**X** représente, indépendamment les uns des autres, C-**R⁶** ou N,
leurs énantiomères, leurs diastéréomères, leurs mélanges et leurs sels.

8. Composés de formule générale **I** selon la revendication 1, dans lesquels **R¹, R³, R⁴, R⁵, R⁶, R^{?}, R⁸, R⁹, R¹⁰, R¹¹, n** et **X** sont définis tels que mentionnés dans la revendication 1, 2, 3, 4, 5, 6 ou 7 et
**R²** représente H ou CH₃,
leurs énantiomères, leurs diastéréomères, leurs mélanges et leurs sels.

9. Composés de formule générale **I** selon la revendication 1, dans lesquels **R¹, R³, R⁴, R⁵, R⁶, R⁷, R⁸, R⁹, R¹⁰, R¹¹, n** et **X** sont définis tels que mentionnés dans la revendication 1, 2, 3, 4, 5, 6 ou 7 et
**R²** représente H,
leurs énantiomères, leurs diastéréomères, leurs mélanges et leurs sels.

10. Composés de formule générale **I** selon la revendication 1, dans lesquels **R¹, R², R⁵, R⁶, R⁷, R⁸, R⁹, R¹⁰, R¹¹, n** et **X** sont définis tels que mentionnés dans la revendication 1, 2, 3, 4, 5, 6, 7, 8 ou 9 et
**R³ et R⁴** représentent, conjointement avec l'atome de carbone auquel ils sont reliés, un groupe cycloalkylène en C₃₋₆, dans lequel un motif -CH₂- peut être remplacé par un atome d'oxygène,
leurs énantiomères, leurs diastéréomères, leurs mélanges et leurs sels.

11. Composés de formule générale **I** selon la revendication 1, dans lesquels **R¹, R², R⁵, R⁶, R⁷, R⁸, R⁹, R¹⁰, R¹¹, n** et **X** sont définis tels que mentionnés dans la revendication 1, 2, 3, 4, 5, 6, 7, 8 ou 9 et
**R³ et R⁴** représentent, conjointement avec l'atome de carbone auquel ils sont reliés, un groupe choisi parmi : 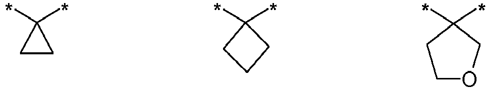 leurs énantiomères, leurs diastéréomères, leurs mélanges et leurs sels.

12. Composés de formule générale **I** selon la revendication 1, dans lesquels **R¹, R², R³, R⁴, R⁶, R⁷, R⁸, R⁹, R¹⁰, R¹¹, n** et **X** sont définis tels que mentionnés dans la revendication 1, 2, 3, 4, 5, 6, 7, 8, 9, 10 ou 11 et
**R⁵** représente H ou CH₃,
leurs énantiomères, leurs diastéréomères, leurs mélanges et leurs sels.

13. Composés de formule générale **I** selon la revendication 1, dans lesquels **R¹, R², R³, R⁴, R⁵, R⁷, R⁸, R⁹, R¹⁰, R¹¹, n** et **X** sont définis tels que mentionnés dans la revendication 1, 2, 3, 4, 5, 6, 7, 8, 9, 10, 11 ou 12 et
**R⁶** représente H, F, Cl ou méthyle,
leurs énantiomères, leurs diastéréomères, leurs mélanges et leurs sels.

14. Composés de formule générale I selon la revendication 1, dans lesquels R¹, R², R³, R⁴, R⁵, R⁶, n et X sont définis tels que mentionnés dans la revendication 1, 2, 3, 4, 5, 6, 7, 8, 9, 10, 11, 12 ou 13 et
R⁷ représente H, F, Cl, Br, -CN, alkyle en C₁₋₄, CF₃, CHF₂,
R⁸ représente H,
R⁹ représente F, Cl, Br, alkyle en C₁₋₄, -O-alkyle en C₁₋₄, -S-alkyle en C₁₋₄,
**R¹⁰** représente H, et
**R¹¹** représente F, Cl, Br, -CN, alkyle en C₁₋₄, CF₃, CHF₂,
leurs énantiomères, leurs diastéréomères, leurs mélanges et leurs sels.

15. Composés de formule générale Ia 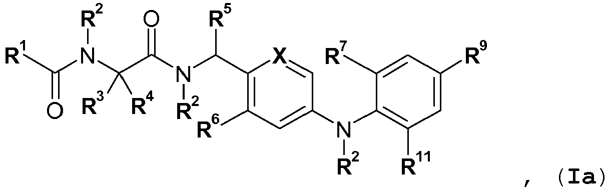 dans laquelle
**R¹** représente
(a) un groupe alkyle en C₁₋₆ éventuellement substitué par un radical **R^{1.1},**
(b) un groupe phényle éventuellement substitué par 1, 2 ou 3 radicaux **R^{1.3},**
(c) un radical hétéroaryle à cinq membres, éventuellement substitué par 1, 2 ou 3 radicaux **R^{1.4},** comprenant au moins un atome de N, 0 ou S et éventuellement en plus un, deux ou trois autres atomes de N,
(d) un radical hétéroaryle à six membres, éventuellement substitué par 1 ou 2 radicaux **R^{1.4}** comprenant un, deux ou trois atomes de N,
(e) un radical hétéroaryle à neuf ou dix membres, éventuellement substitué par 1 ou 2 radicaux **R^{1.4}** comprenant un, deux ou trois atomes de N,
(f) un hétérocycle à 5 ou 6 membres, éventuellement substitué par 1 ou 2 radicaux **R^{1.4},** dans lequel un motif -CH₂-peut être remplacé par un groupe -C(O)-,
**R^{1.1}** représente -CN_{,} cycloalkyle en C₃₋₆, -OH, -OCH₃, -NH₂, -NHCH₃, -N(CH₃)₂,
**R^{1.3}** représente, indépendamment les uns des autres,
(a) F, Cl, Br, -OH, -OCH₃, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor, et
**R^{1.4}** représente, indépendamment les uns des autres,
(a) F, Cl, Br, -OH, -OCH₃, -NH₂, -NHCH₃, -N(CH₃)₂, -NH-C(O)-alkyle en C₁₋₄, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
R² représente H ou CH₃,
R³ et R⁴ représentent, conjointement avec l'atome de carbone auquel ils sont reliés, un groupe cycloalkylène en C₃₋₆, dans lequel un motif -CH₂- peut être remplacé par un atome d'oxygène,
**R⁵** représente H ou alkyle en C₁₋₄,
**R⁶** représente H, F, Cl, Br ou alkyle en C₁₋₄,
**R⁷** représente H, F, Cl, Br, -CN, alkyle en C₁₋₄, CF₃, CHF₂,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs mélanges et leurs sels.

16. Composés de formule générale Ia selon la revendication 14, dans lesquels
**R¹** représente
(a) un groupe alkyle en C₁₋₆ éventuellement substitué par un radical **R^{1.1},**
(b) un groupe phényle éventuellement substitué par 1, 2 ou 3 radicaux **R^{1.3},**
(c) un radical hétéroaryle à cinq membres, éventuellement substitué par 1, 2 ou 3 radicaux **R^{1.4},** choisi dans le groupe constitué par : 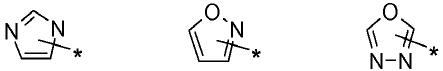 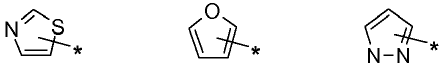 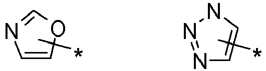
(d) un radical hétéroaryle à six membres, éventuellement substitué par 1 ou 2 radicaux **R^{1.4},** choisi dans le groupe constitué par : 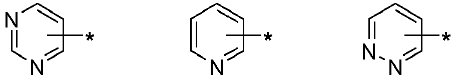 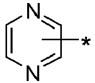
(e) un radical hétéroaryle à neuf membres, éventuellement substitué par 1 ou 2 radicaux **R^{1.4},** choisi dans le groupe constitué par : 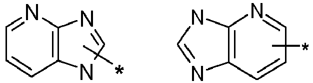
(f) un hétérocycle à 5 ou 6 membres, éventuellement substitué par 1 ou 2 radicaux **R^{1.4},** choisi dans le groupe constitué par : 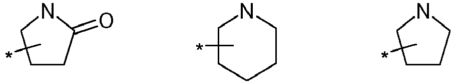 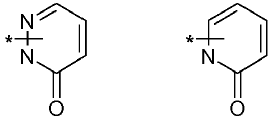
**R^{1.1}** représente -CN, cyclopropyle, -OH, -OCH₃, -NH₂, -NHCH₃, -N(CH₃)₂,
**R^{1.3}** représente, indépendamment les uns des autres,
(a) F, Cl, Br, -OH, -OCH₃, -OCF₃, alkyle en C₁₋₄, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor, et
**R^{1.4}** représente, indépendamment les uns des autres,
(a) F, Cl, Br, -OH, -OCH₃, -OCF₃, -NH₂, -NH(alkyle en C₁₋₄)-, -N(alkyle en C₁₋₄)₂, -NH-C(O)-alkyle en C₁₋₄, alkyle en C₁₋₆, ou
**(b)** un groupe alkyle en C₁₋₃, dans lequel chaque groupe méthylène peut être substitué par 1 ou 2 atomes de fluor et chaque groupe méthyle par 1, 2 ou 3 atomes de fluor,
**R²** représente H ou CH₃,
**R³ et R⁴** représentent, conjointement avec l'atome de carbone auquel ils sont reliés, un groupe cycloalkylène en C₃₋₆, dans lequel un motif -CH₂-peut être remplacé par un atome d'oxygène,
**R⁵** représente H ou CH₃,
**R⁶** représente H, F, Cl, ou méthyle,
**R⁷** représente H, F, Cl, Br, -CN, alkyle en C₁₋₄, CF₃, CHF₂,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs **mélanges** et leurs sels.

17. Composés de formule générale Ia selon la revendication 15, dans lesquels
**R¹** représente un groupe choisi parmi : 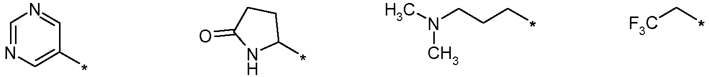 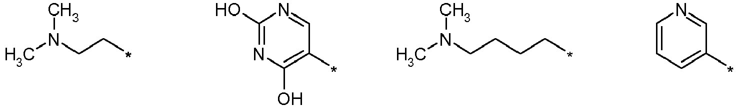 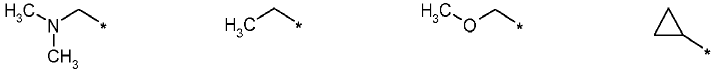 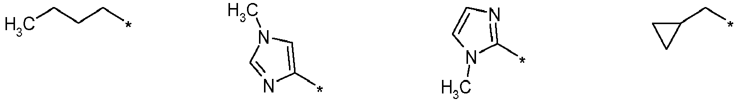 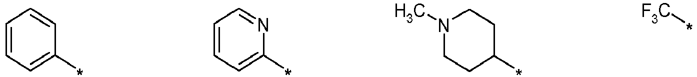 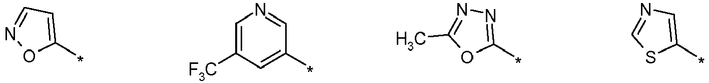 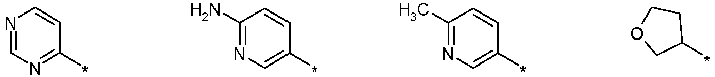 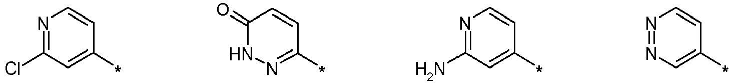 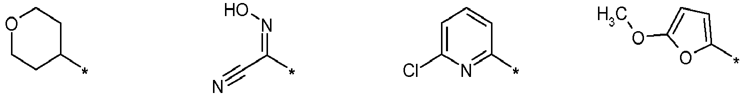 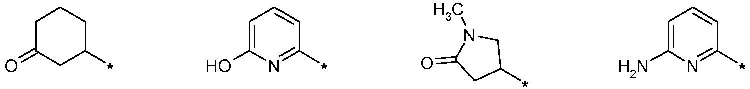 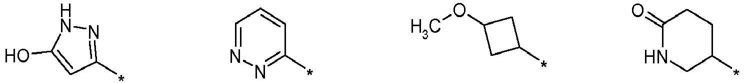 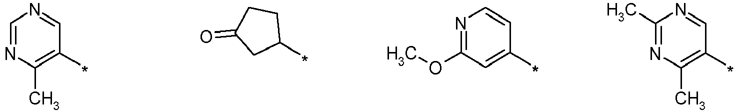 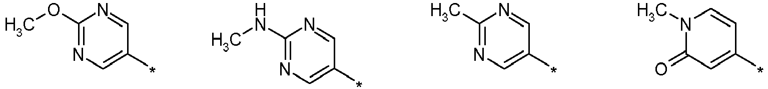 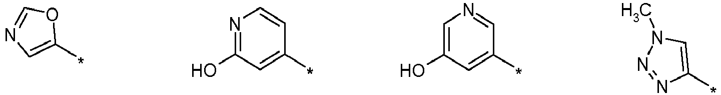 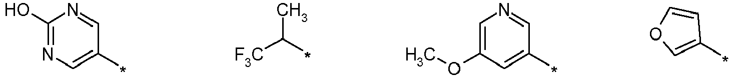 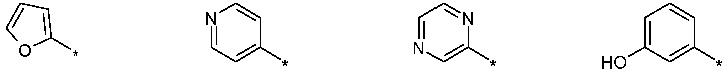 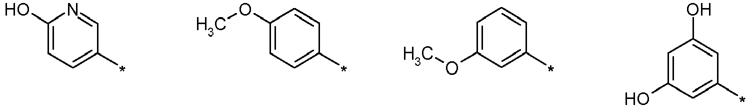 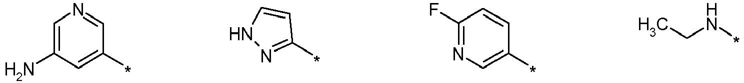 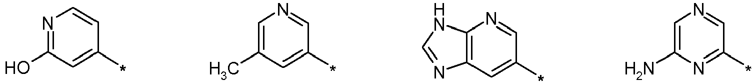 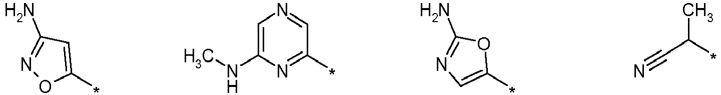 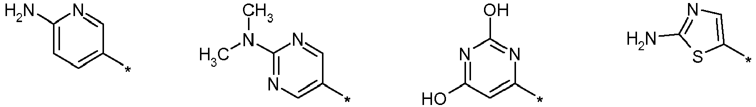 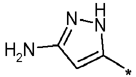 **R²** représente H ou CH₃,
**R³ et R⁴** représentent, conjointement avec l'atome de carbone auquel ils sont reliés, un groupe cycloalkylène en C₃-₆, dans lequel un motif -CH₂-peut être remplacé par un atome d'oxygène,
**R⁵** représente H ou CH₃,
**R⁶** représente H, F, Cl, ou méthyle,
**R⁷** représente H, F, Cl, Br, -CN, alkyle en C₁₋₄, CF₃, CHF₂,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs mélanges et leurs sels.

18. Composés de formule générale Ia selon la revendication 15, dans lesquels
**R¹** représente un groupe choisi parmi : 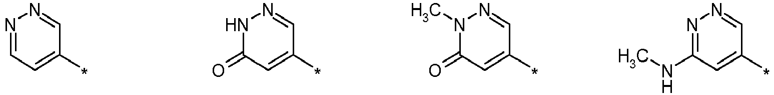 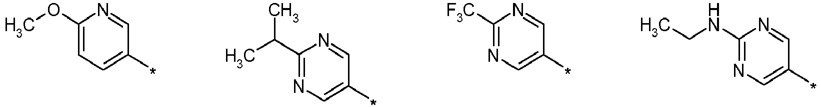 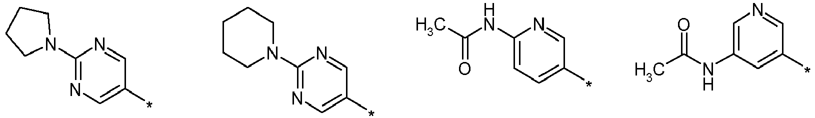 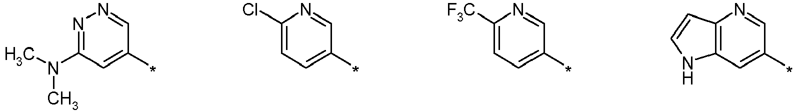 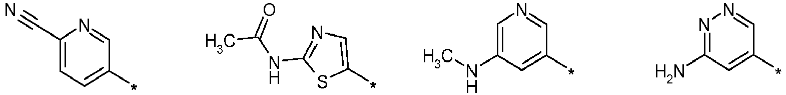 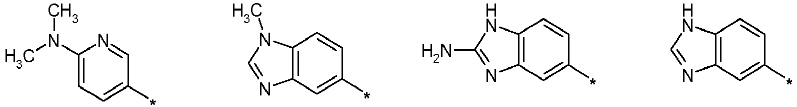 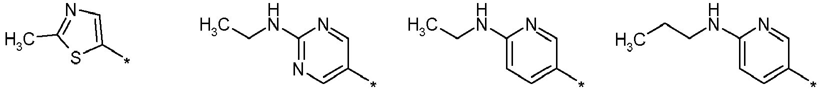 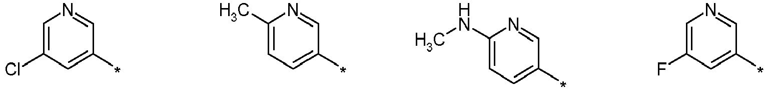 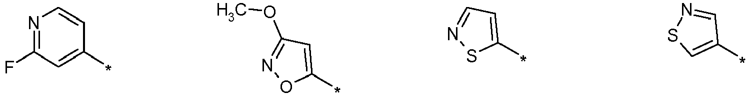 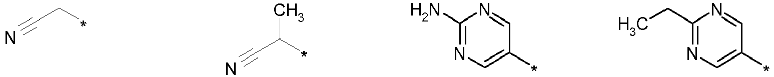 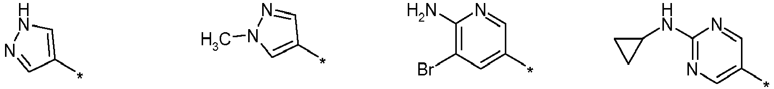 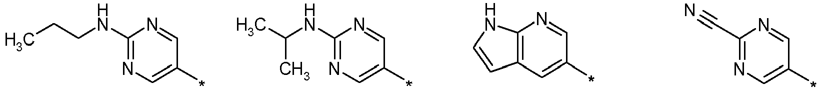 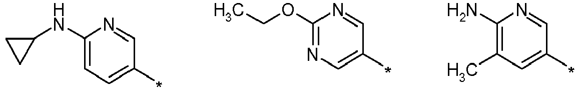 **R²** représente H ou CH₃,
**R³ et R⁴** représentent, conjointement avec l'atome de carbone auquel ils sont reliés, un groupe cycloalkylène en C₃₋₆, dans lequel un motif -CH₂-peut être remplacé par un atome d'oxygène,
**R⁵** représente H ou CH₃,
**R⁶** représente H, F, Cl, ou méthyle,
**R⁷** représente H, F, Cl, Br, -CN, alkyle en C₁₋₄, CF₃, CHF₂,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs mélanges et leurs sels, en particulier leurs sels physiologiquement acceptables avec des acides ou des bases, organiques ou minéraux.

19. Composés de formule générale **Ib** 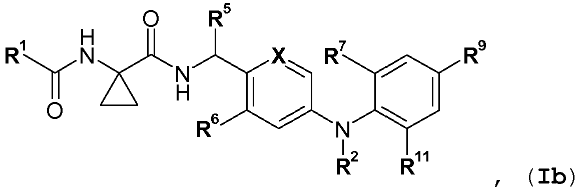 dans laquelle
**R¹** représente
(a) un groupe alkyle en C₁₋₆ éventuellement substitué par un radical **R^{1.1}**,
(b) un groupe phényle éventuellement substitué par 1, 2 ou 3 radicaux **R^{1.3}**,
(c) un radical hétéroaryle à cinq membres, éventuellement substitué par 1, 2 ou 3 radicaux **R^{1.4}**, comprenant au moins un atome de N, 0 ou S et éventuellement en plus un, deux ou trois autres atomes de N,
(d) un radical hétéroaryle à six membres, éventuellement substitué par 1 ou 2 radicaux **R^{1.4}**, comprenant un, deux ou trois atomes de N,
(e) un radical hétéroaryle à neuf ou dix membres, éventuellement substitué par 1 ou 2 radicaux **R^{1.4},** comprenant un, deux ou trois atomes de N,
(f) un hétérocycle à 5 ou 6 membres, éventuellement substitué par 1 ou 2 radicaux **R^{1.4}**, dans lequel un motif -CH₂-peut être remplacé par un groupe -C(0)-,
**R^{1.1}** représente -CN, cycloalkyle en C₃₋₆, -OH, -OCH₃, -NH₂, -NHCH₃, -N (CH₃)₂,
**R^{1.3}** représente, indépendamment les uns des autres,
(a) F, Cl, Br, -OH, -OCH₃, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor, et
**R^{1.4}** représente, indépendamment les uns des autres,
(a) F, Cl, Br, -OH, -OCH₃, -NH₂, -NHCH₃, -N(CH₃)₂, -NH-C(O)-alkyle en C₁₋₄, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
**R²** représente H ou CH₃,
**R⁵** représente H ou alkyle en C₁₋₄,
**R⁶** représente H, F, Cl, Br ou alkyle en C₁₋₄,
**R⁷** représente H, F, Cl, Br, -CN, alkyle en C₁₋₄, CF₃, CHF₂,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs mélanges et leurs sels.

20. Composés de formule générale **Ib** selon la revendication 19, dans lesquels
**R¹** représente
(a) un groupe alkyle en C₁₋₆ éventuellement substitué par un radical **R^{1.1}**,
(b) un groupe phényle éventuellement substitué par 1, 2 ou 3 radicaux **R^{1.3}**_{,}
(c) un radical hétéroaryle à cinq membres, éventuellement substitué par 1, 2 ou 3 radicaux **R^{1.4}**, choisi dans le groupe constitué par : 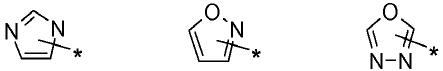 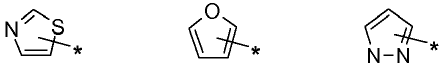 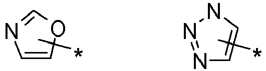
(d) un radical hétéroaryle à six membres, éventuellement substitué par 1 ou 2 radicaux **R^{1.4}**, choisi dans le groupe constitué par : 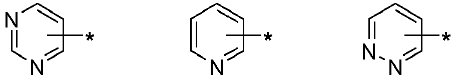 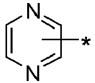
(e) un radical hétéroaryle à neuf membres, éventuellement substitué par 1 ou 2 radicaux **R^{1.4},** choisi dans le groupe constitué par : 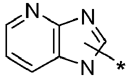
(f) un hétérocycle à 5 ou 6 membres, éventuellement substitué par 1 ou 2 radicaux **R^{1.4}**, choisi dans le groupe constitué par : 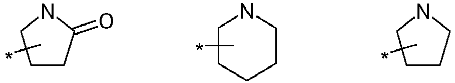 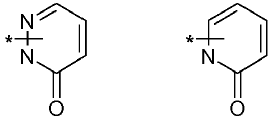
**R^{1.1}** représente -CN, cyclopropyle, -OH, -OCH₃, -NH₂, -NHCH₃, -N(CH₃)₂,
**R^{1.3}** représente, indépendamment les uns des autres,
(a) F, Cl, Br, -OH, -OCH₃, -OCF₃, alkyle en C₁₋₄, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor, et
**R^{1.4}** représente, indépendamment les uns des autres,
(a) F, Cl, Br, -OH, -OCH₃, -OCF₃, -NH₂, -NH(alkyle en C₁₋₄) -, -N (alkyle en C₁₋₄)₂, -NH-C(O)-alkyle en C₁₋₄, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
**R²** représente H ou CH₃,
**R⁵** représente H ou CH₃,
**R⁶** représente H, F, Cl, ou méthyle,
**R⁷** représente H, F, Cl, Br, -CN, alkyle en C₁₋₄, CF₃, CHF₂,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs mélanges et leurs sels.

21. Composés de formule générale **Ib** selon la revendication 19, dans lesquels
**R¹** représente 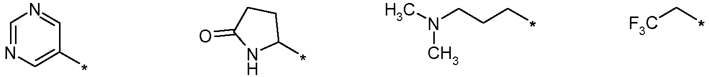 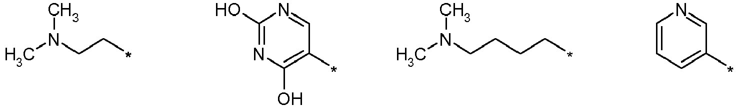 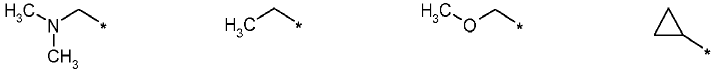 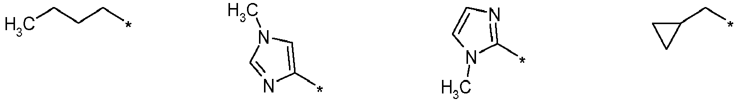 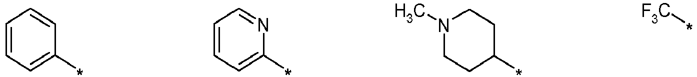 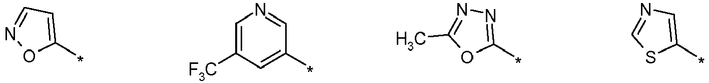 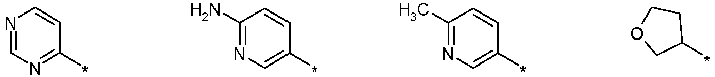 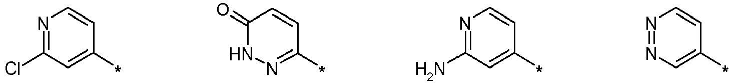 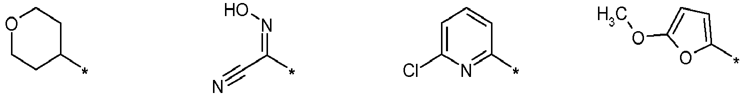 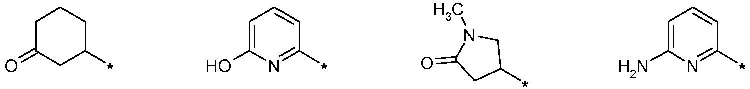 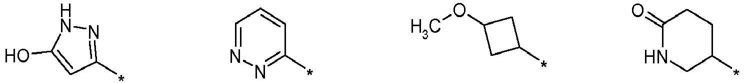 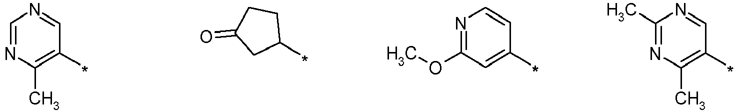 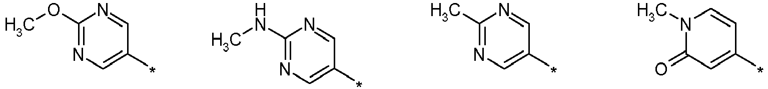 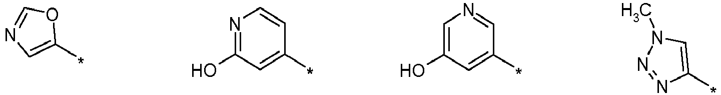 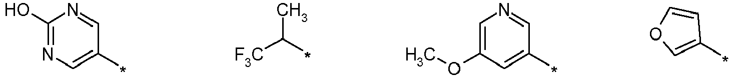 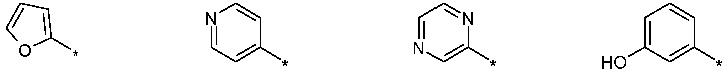 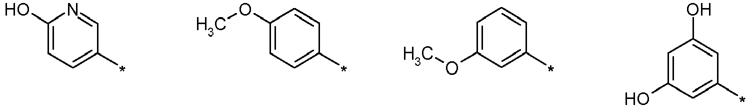 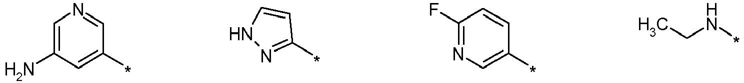 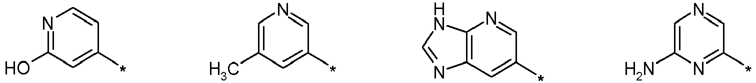 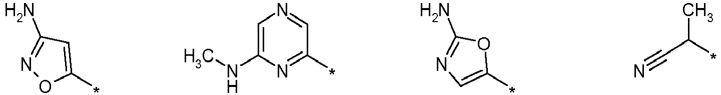 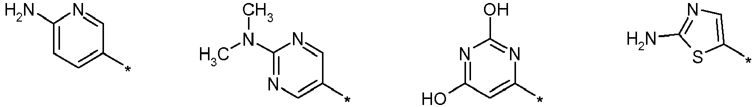 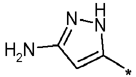 **R²** représente H ou CH₃,
**R⁵** représente H ou CH₃,
**R⁶** représente H, F, Cl, ou méthyle,
**R⁷** représente H, F, Cl, Br, -CN, alkyle en C₁₋₄, CF₃, CHF₂,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs mélanges et leurs sels.

22. Composés de formule générale **Ib** selon la revendication 19, dans lesquels
**R¹** représente 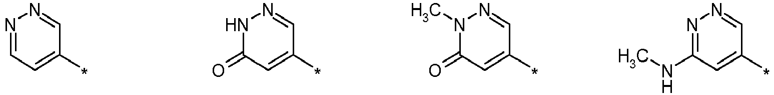 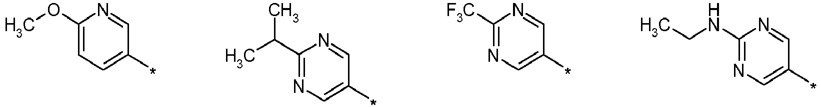 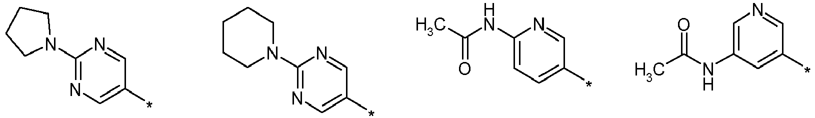 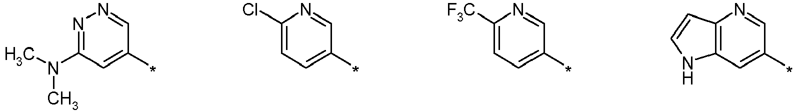 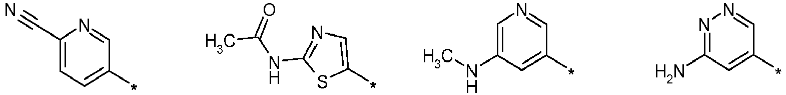 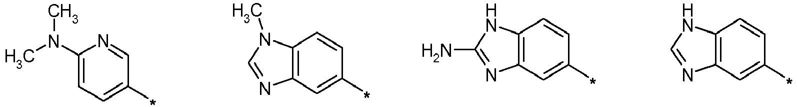 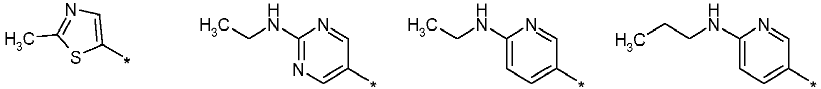 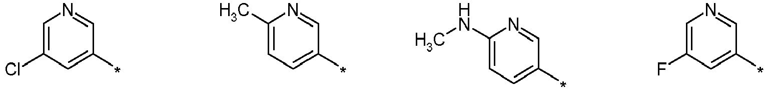 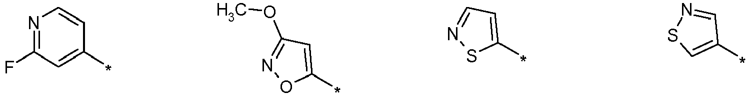 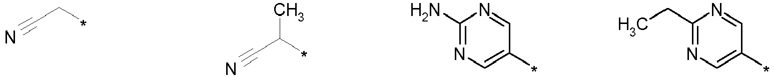 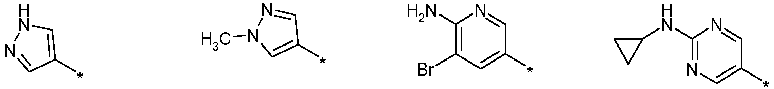 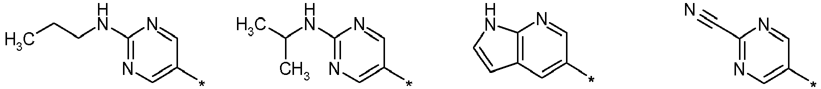 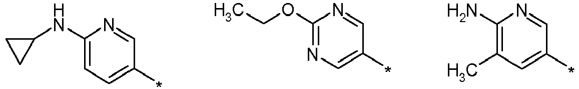 **R²** représente H,
**R⁵** représente H ou CH₃,
**R⁶** représente H, F, Cl, ou méthyle,
**R⁷** représente H, F, Cl, Br, -CN, alkyle en C₁₋₄, CF₃, CHF₂,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs mélanges et leurs sels, en particulier leurs sels physiologiquement acceptables avec des acides ou des bases, organiques ou minéraux.

23. Composés de formule générale **Ic** 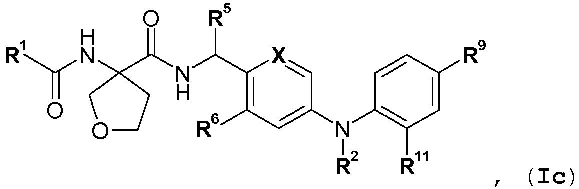 dans laquelle
**R¹** représente
(a) un groupe alkyle en C₁₋₆ éventuellement substitué par un radical **R^{1.1}**,
(b) un groupe phényle éventuellement substitué par 1, 2 ou 3 radicaux **R^{1.3}**,
(c) un radical hétéroaryle à cinq membres, éventuellement substitué par 1, 2 ou 3 radicaux **R^{1.4}**, comprenant au moins un atome de N, 0 ou S et éventuellement en plus un, deux ou trois autres atomes de N,
(d) un radical hétéroaryle à six membres, éventuellement substitué par 1 ou 2 radicaux **R^{1.4}**, comprenant un, deux ou trois atomes de N,
(e) un radical hétéroaryle à neuf ou dix membres, éventuellement substitué par 1 ou 2 radicaux **R^{1.4}**, comprenant un, deux ou trois atomes de N,
(f) un hétérocycle à 5 ou 6 membres, éventuellement substitué par 1 ou 2 radicaux **R^{1.4}**, dans lequel un motif -CH₂-peut être remplacé par un groupe -C(O)-,
**R^{1.1}** représente -CN, cycloalkyle en C₃₋₆, -OH, -OCH₃, -NH₂, -NHCH₃, -N (CH₃) ₂,
**R^{1.3}** représente, indépendamment les uns des autres,
(a) F, Cl, Br, -OH, -OCH₃, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor, et
**R^{1.4}** représente, indépendamment les uns des autres,
(a) F, Cl, Br, -OH, -OCH₃, -NH₂, -NHCH₃, -N (CH₃) ₂, -NH-C(O)-alkyle en C₁₋₄, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
**R²** représente H ou CH₃,
**R⁵** représente H ou alkyle en C₁₋₄,
**R⁶** représente H, F, Cl, Br ou alkyle en C₁₋₄,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs mélanges et leurs sels.

24. Composés de formule générale **Ic** selon la revendication 23, dans lesquels
**R¹** représente
(a) un groupe alkyle en C₁₋₆ éventuellement substitué par un radical **R^{1.1}**,
(b) un groupe phényle éventuellement substitué par 1, 2 ou 3 radicaux **R^{1.3}**,
(c) un radical hétéroaryle à cinq membres, éventuellement substitué par 1, 2 ou 3 radicaux **R^{1.4}**, choisi dans le groupe constitué par : 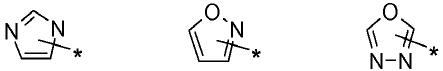 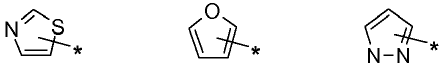 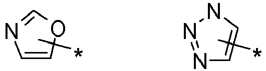
(d) un radical hétéroaryle à six membres, éventuellement substitué par 1 ou 2 radicaux **R^{1.4}**, choisi dans le groupe constitué par : 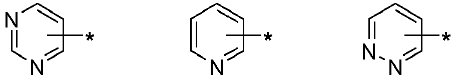 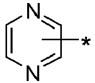
(e) un radical hétéroaryle à neuf membres, éventuellement substitué par 1 ou 2 radicaux **R^{1.4}**, choisi dans le groupe constitué par : 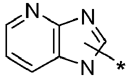
(f) un hétérocycle à 5 ou 6 membres, éventuellement substitué par 1 ou 2 radicaux **R^{1.4},** choisi dans le groupe constitué par : 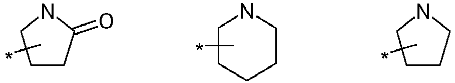 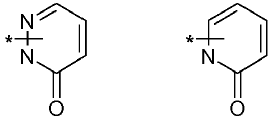
**R^{1.1}** représente -CN, cyclopropyle, -OH, -OCH₃, -NH₂, -NHCH₃, -N (CH₃) ₂,
**R^{1.3}** représente, indépendamment les uns des autres,
(a) F, Cl, Br, -OH, -OCH₃, -OCF₃, alkyle en C₁₋₄, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor, et
**R^{1.4}** représente, indépendamment les uns des autres,
(a) F, Cl, Br, -OH, -OCH₃, -OCF₃, -NH₂, -NH (alkyle en C₁₋₄) -, -N (alkyle en C₁₋₄)₂, -NH-C(O)-alkyle en C₁₋₄, alkyle en C₁₋₆, ou
(b) un groupe alkyle en C₁₋₃, dans lequel chaque groupe méthylène peut être substitué par 1 ou 2 atomes de fluor et chaque groupe méthyle par 1, 2 ou 3 atomes de fluor,
**R²** représente H ou CH₃,
**R⁵** représente H ou CH₃,
**R⁶** représente H, F, Cl, ou méthyle,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs mélanges et leurs sels.

25. Composés de formule générale **Ic** selon la revendication 23, dans lesquels
**R¹** représente 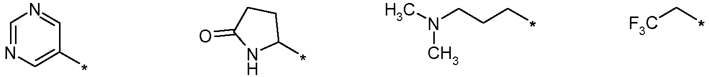 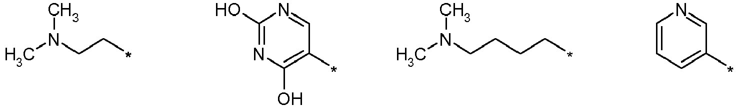 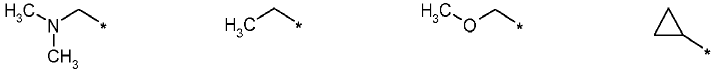 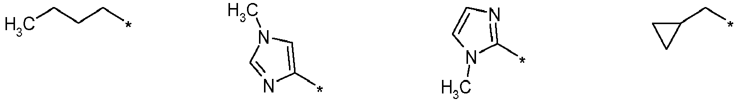 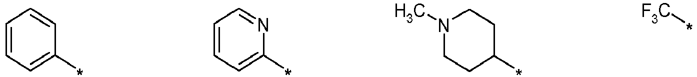 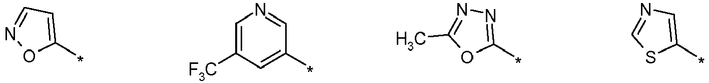 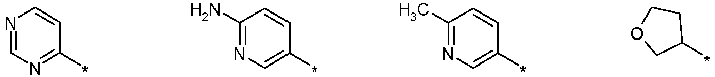 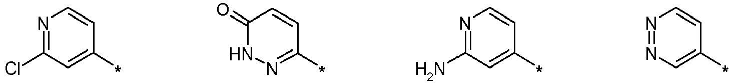 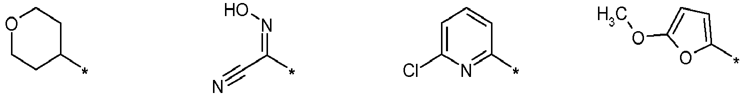 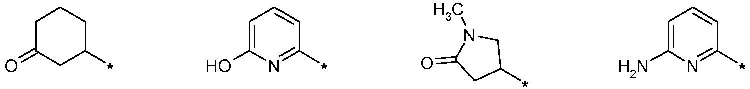 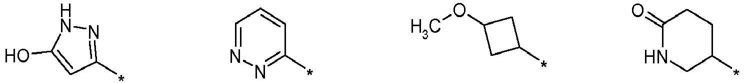 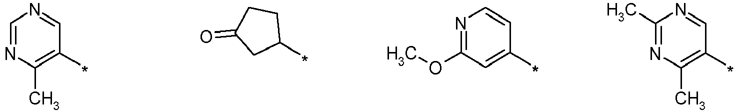 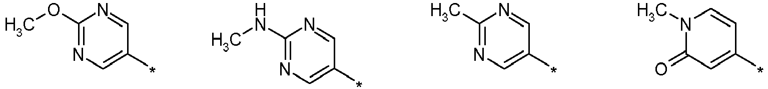 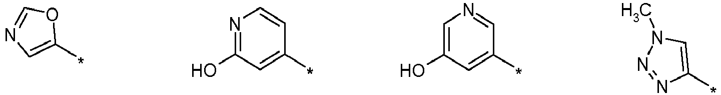 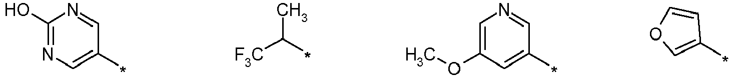 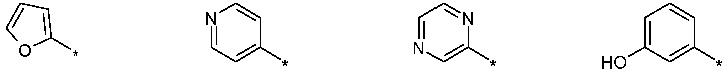 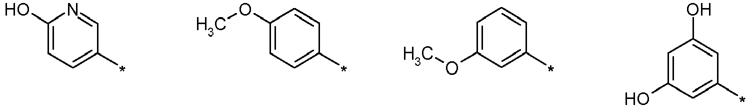 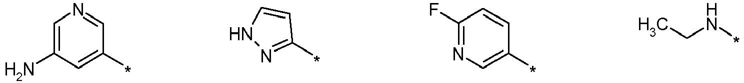 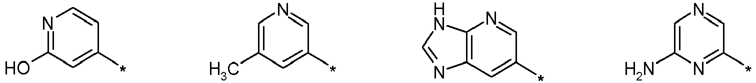 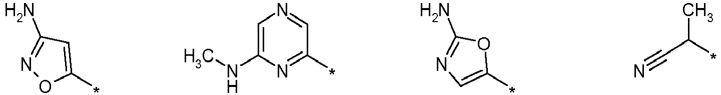 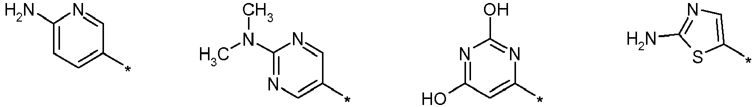 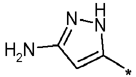 **R²** représente H ou CH₃,
**R⁵** représente H ou CH₃,
**R⁶** représente H, F, Cl, ou méthyle,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs mélanges et leurs sels.

26. Composés de formule générale **Ic** selon la revendication 23, dans lesquels
**R¹** représente 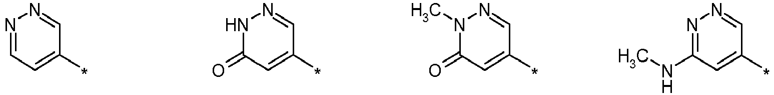 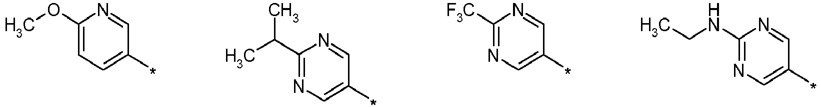 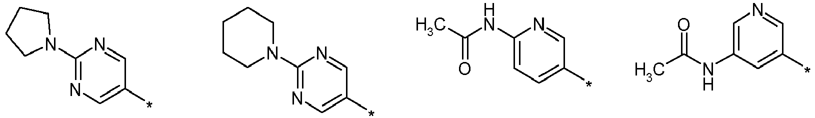 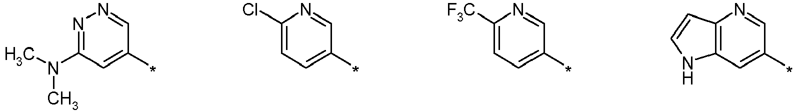 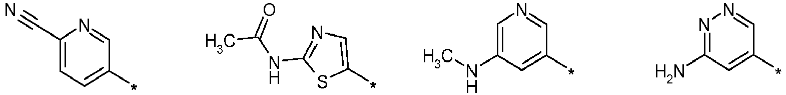 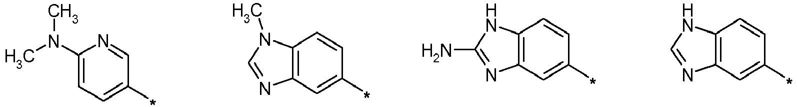 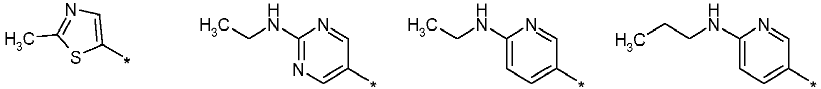 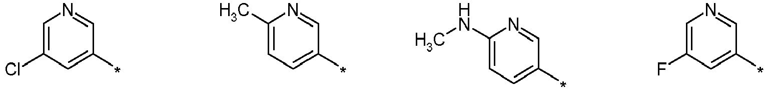 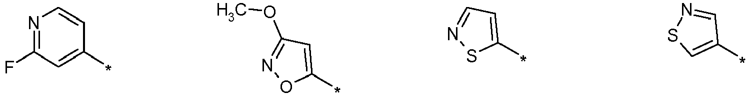 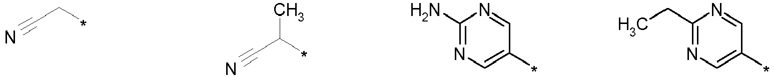 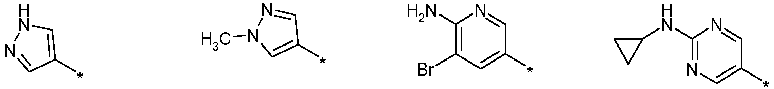 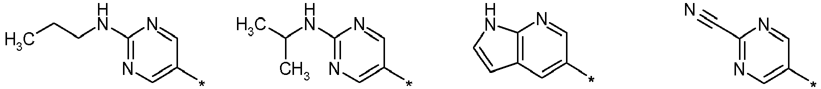 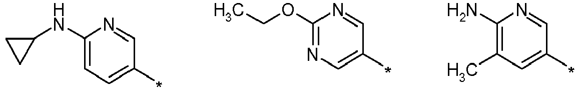 **R²** représente H ou CH₃,
**R⁵** représente H ou CH₃,
**R⁶** représente H, F, Cl, ou méthyle,
**R⁹** représente F, Cl, Br, alkyle en C₁₋₄, -0-alkyle en C₁₋₄, -S-alkyle en C₁₋₄,
**R¹¹** représente F, Cl, Br, -CN, alkyle en C₁₋₄, CF₃, CHF₂, et
**X** représente CH ou N,
leurs énantiomères, leurs diastéréomères, leurs mélanges et leurs sels, en particulier leurs sels physiologiquement acceptables avec des acides ou des bases, organiques ou minéraux.

27. Composés de formule générale **Id** 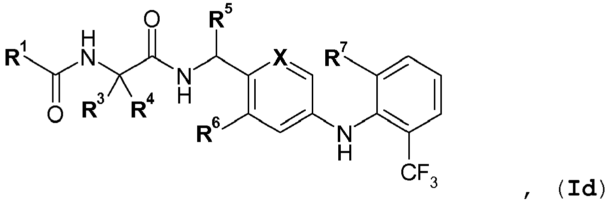 dans laquelle
**R¹** représente un groupe choisi parmi : 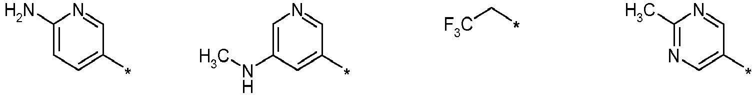 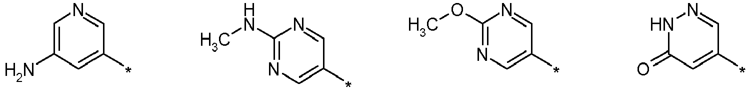 **R³ et R⁴** représentent, conjointement avec l'atome de carbone auquel ils sont reliés, un groupe cycloalkylène en C₃₋₆, dans lequel un motif -CH₂-peut être remplacé par un atome d'oxygène,
**R⁵** représente H ou CH₃,
**R⁶** représente Cl ou CH₃,
**R⁷** représente H ou F,
**X** représente CH ou N,
leurs énantiomères, leurs diastéréomères, leurs mélanges et leurs sels, en particulier leurs sels physiologiquement acceptables avec des acides ou des bases, organiques ou minéraux.

28. Composés de formule générale I selon la revendication 1, qui sont les suivantes :
| **N°** | **Structure** |
|---|---|
| (1) | 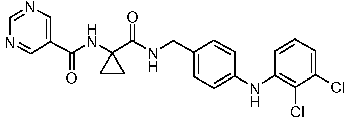 |
| (2) | 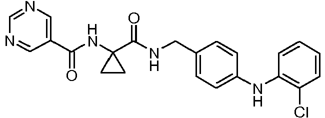 |
| (3) | 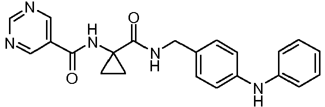 |
| (4) | 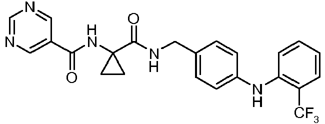 |
| (5) | 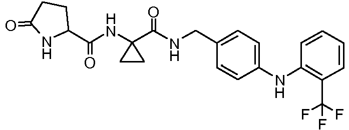 |
| (6) | 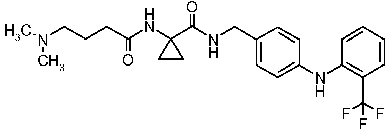 |
| (7) | 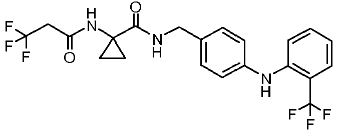 |
| (8) | 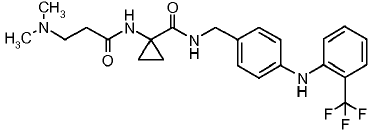 |
| (9) | 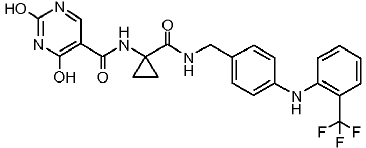 |
| (10) | 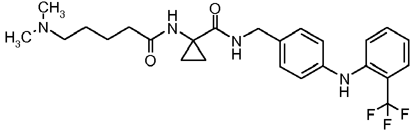 |
| (11) | 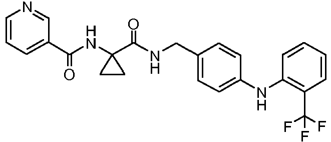 |
| (12) | 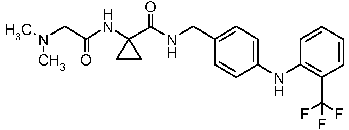 |
| (13) | 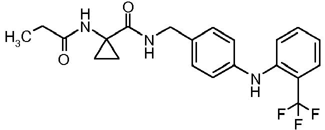 |
| (14) | 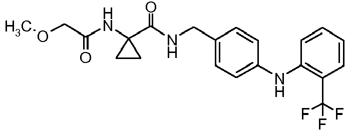 |
| (15) | 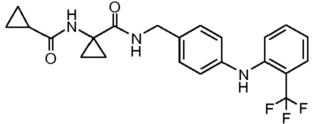 |
| (16) | 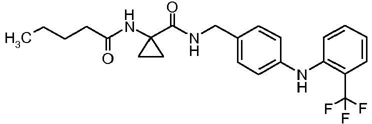 |
| (17) | 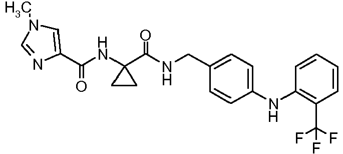 |
| (18) | 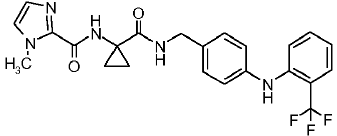 |
| (19) | 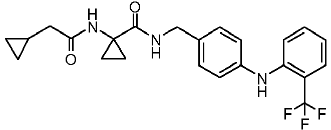 |
| (20) | 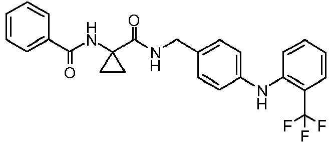 |
| (21) | 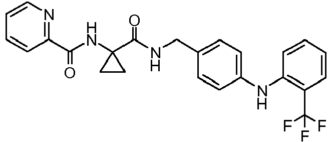 |
| (22) | 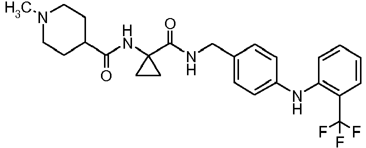 |
| (23) | 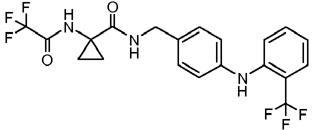 |
| (24) | 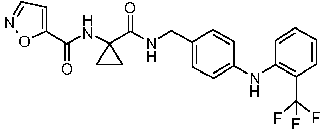 |
| (25) | 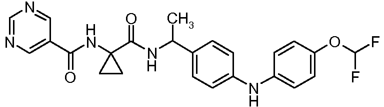 |
| (26) | 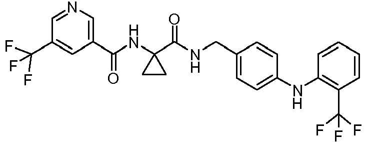 |
| (27) | 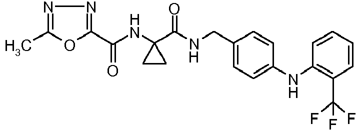 |
| (28) | 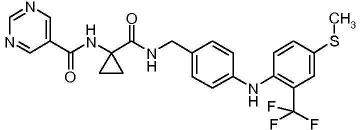 |
| (29) | 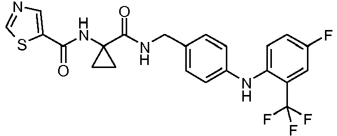 |
| (30) | 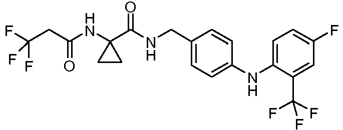 |
| (31) | 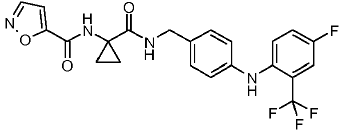 |
| (32) | 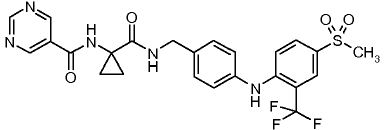 |
| (33) | 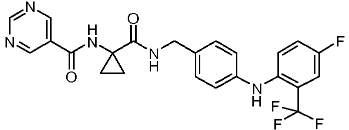 |
| (34) | 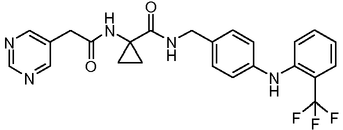 |
| (35) | 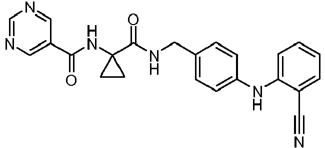 |
| (36) | 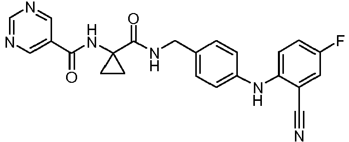 |
| (37) | 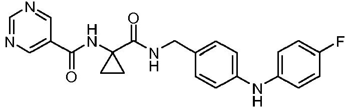 |
| (38) | 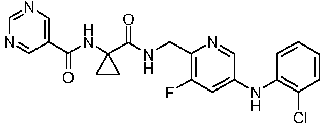 |
| (39) | 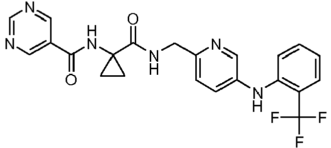 |
| (40) | 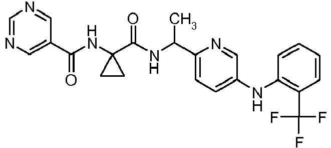 |
| (41) | 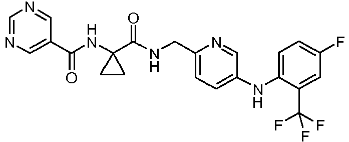 |
| (42) | 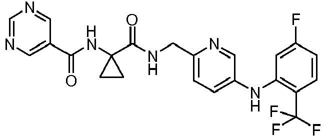 |
| (43) | 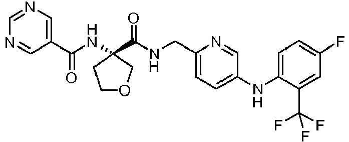 |
| (44) | 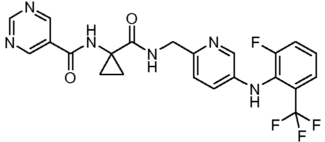 |
| (45) | 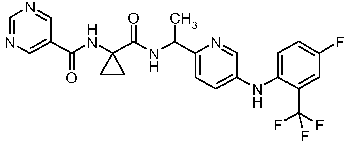 |
| (46) | 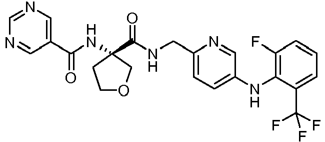 |
| (47) | 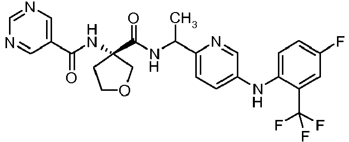 |
| (48) | 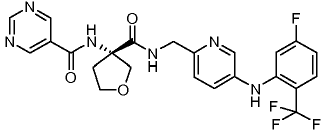 |
| (49) | 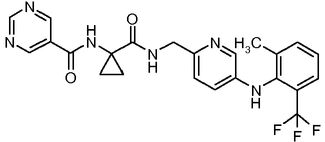 |
| (50) | 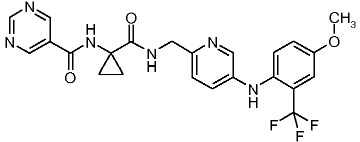 |
| (51) | 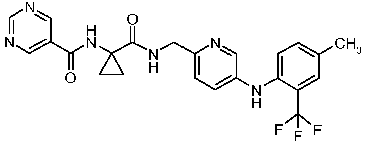 |
| (52) | 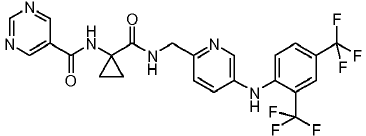 |
| (53) | 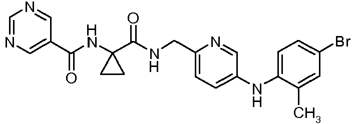 |
| (54) | 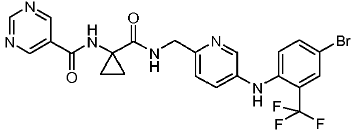 |
| (55) | 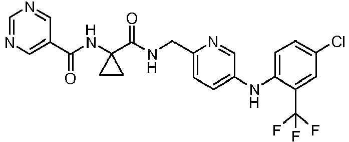 |
| (56) | 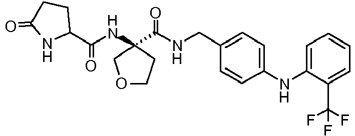 |
| (57) | 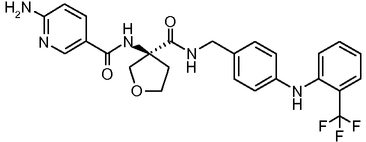 |
| (58) | 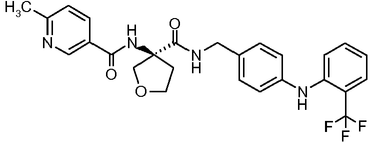 |
| (59) | 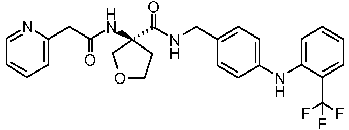 |
| (60) | 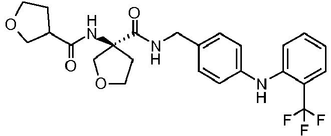 |
| (61) | 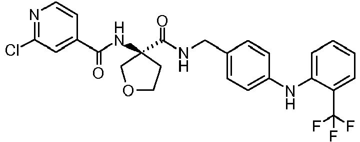 |
| (62) | 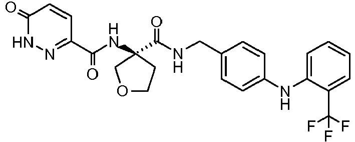 |
| (63) | 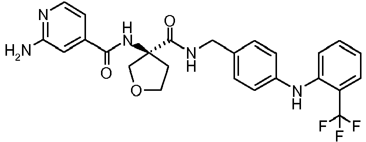 |
| (64) | 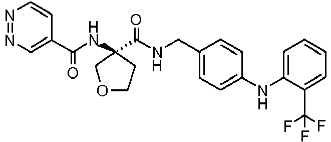 |
| (65) | 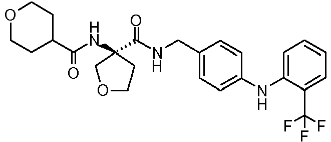 |
| (66) | 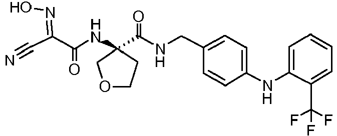 |
| (67) | 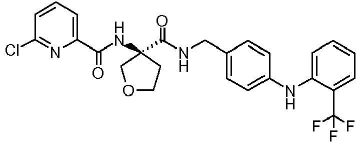 |
| (68) | 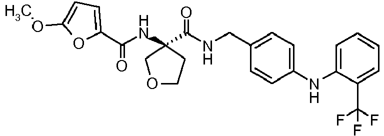 |
| (69) | 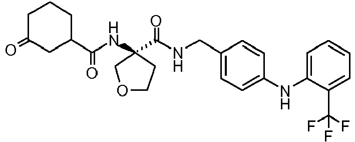 |
| (70) | 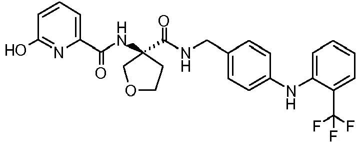 |
| (71) | 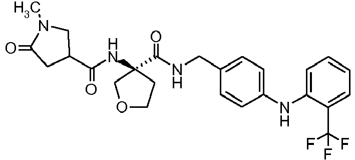 |
| (72) | 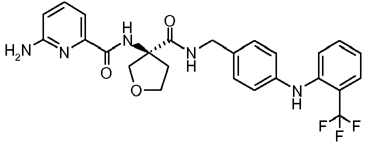 |
| (73) | 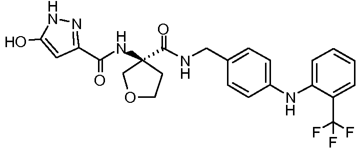 |
| (74) | 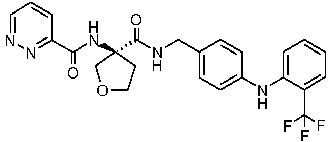 |
| (75) | 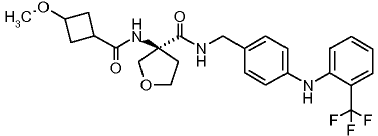 |
| (76) | 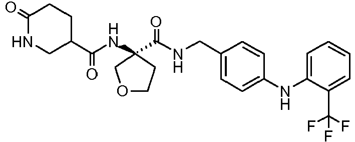 |
| (77) | 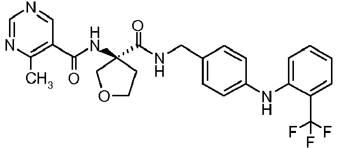 |
| (78) | 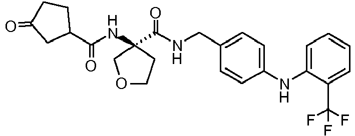 |
| (79) | 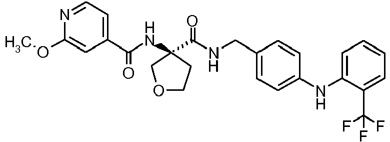 |
| (80) | 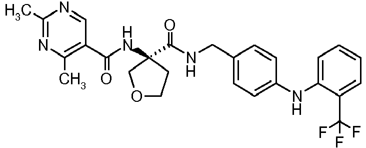 |
| (81) | 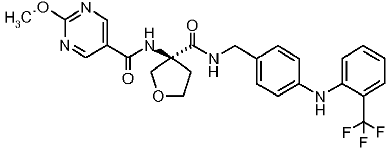 |
| (82) | 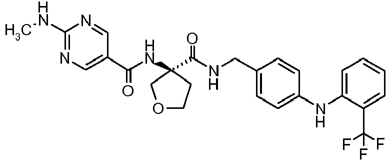 |
| (83) | 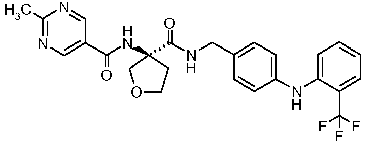 |
| (84) | 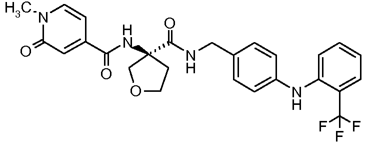 |
| (85) | 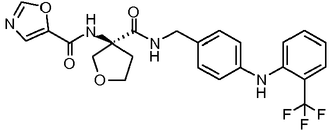 |
| (86) | 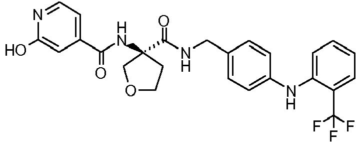 |
| (87) | 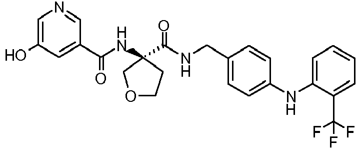 |
| (88) | 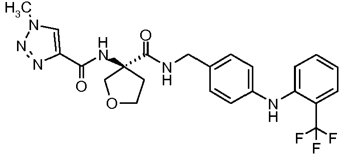 |
| (89) | 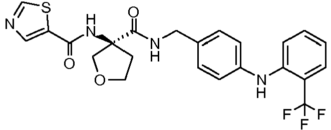 |
| (90) | 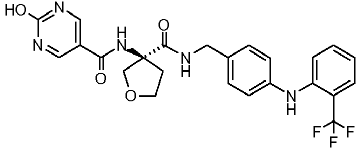 |
| (91) | 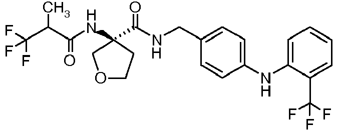 |
| (92) | 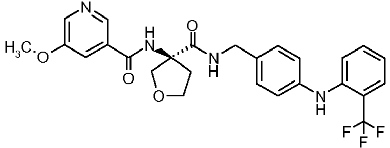 |
| (93) | 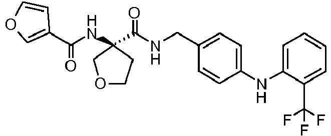 |
| (94) | 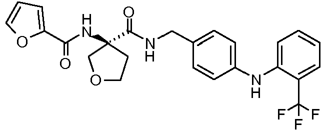 |
| (95) | 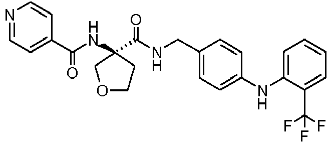 |
| (96) | 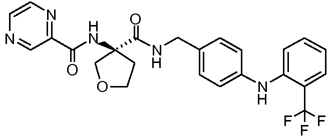 |
| (97) | 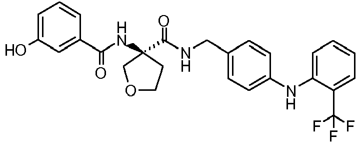 |
| (98) | 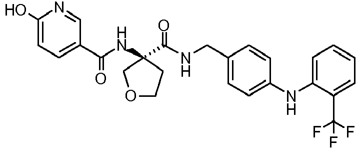 |
| (99) | 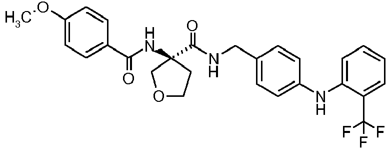 |
| (100) | 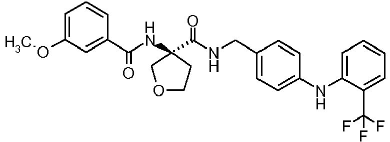 |
| (101) | 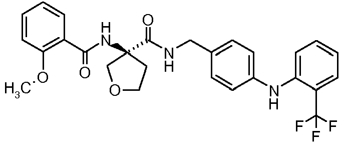 |
| (102) | 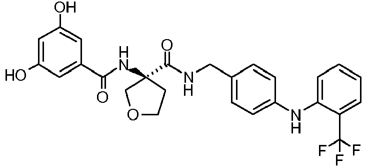 |
| (103) | 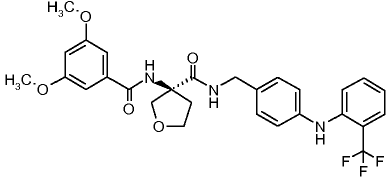 |
| (104) | 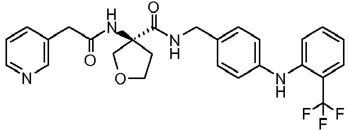 |
| (105) | 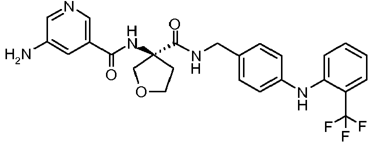 |
| (106) | 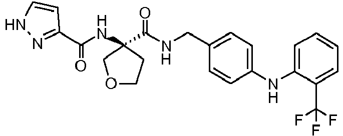 |
| (107) | 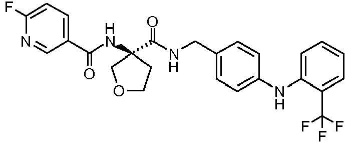 |
| (108) | 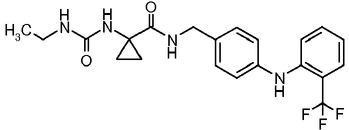 |
| (109) | 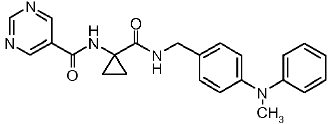 |
| (110) | 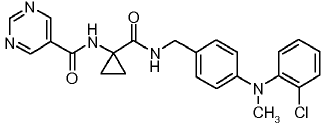 |
| (111) | 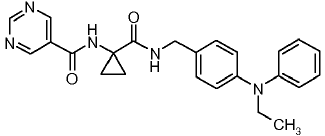 |
| (112) | 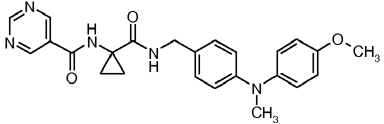 |
| (113) | 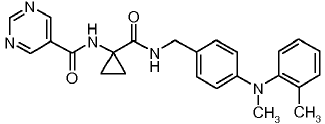 |
| (114) | 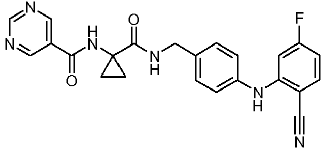 |
| (115) | 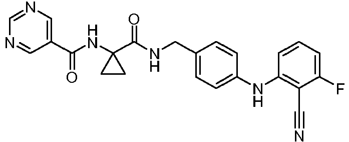 |
| (116) | 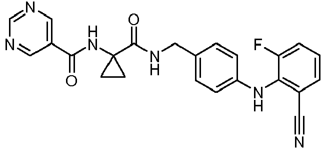 |
| (117) | 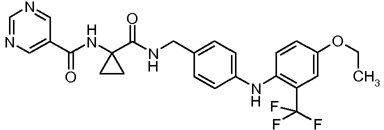 |
| (118) | 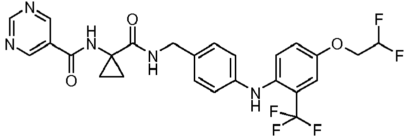 |
| (119) | 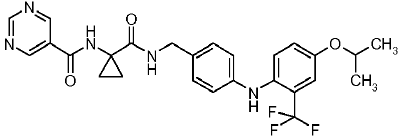 |
| (120) | 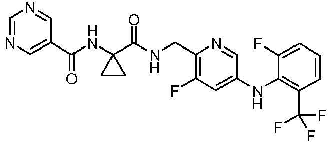 |
| (121) | 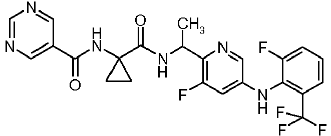 |
| (122) | 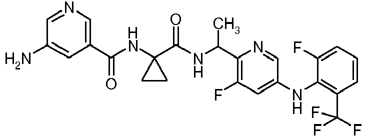 |
| (123) | 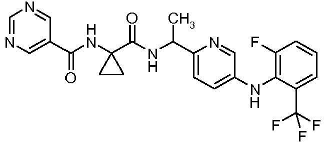 |
| (124) | 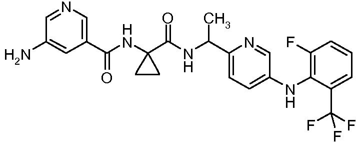 |
| (125) | 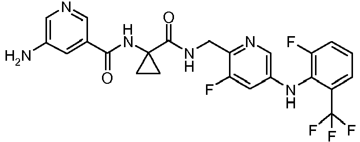 |
| (126) | 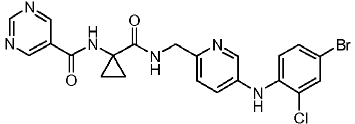 |
| (127) | 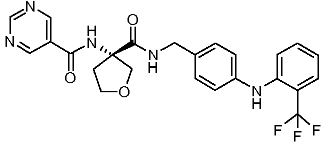 |
| (128) | 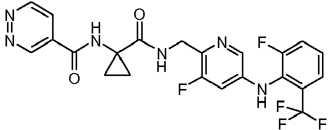 |
| (129) | 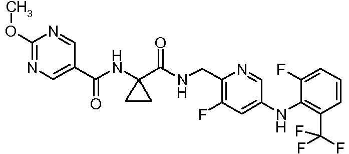 |
| (130) | 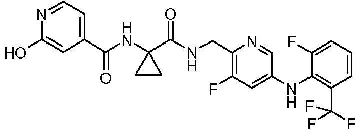 |
| (131) | 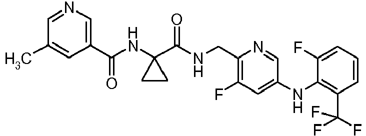 |
| (132) | 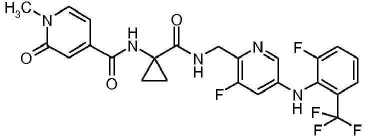 |
| (133) | 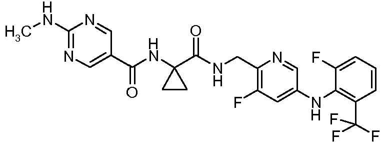 |
| (134) | 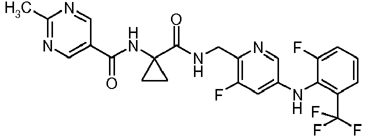 |
| (135) | 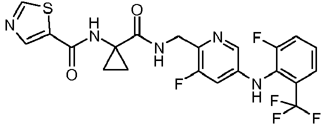 |
| (136) | 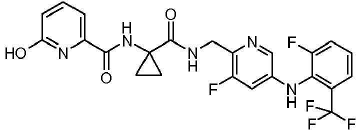 |
| (137) | 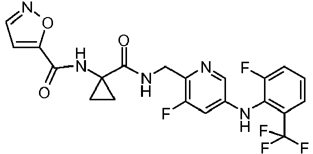 |
| (138) | 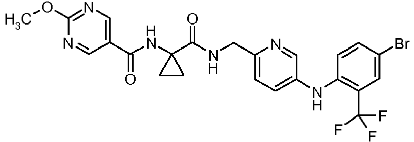 |
| (139) | 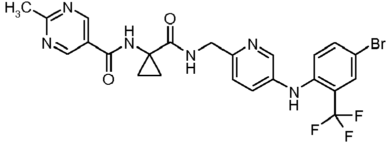 |
| (140) | 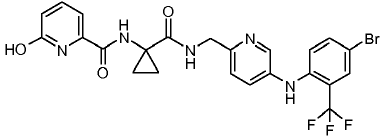 |
| (141) | 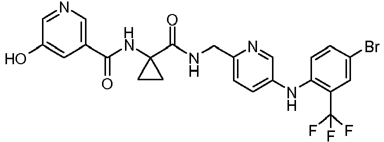 |
| (142) | 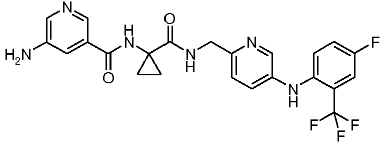 |
| (143) | 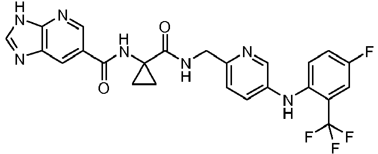 |
| (144) | 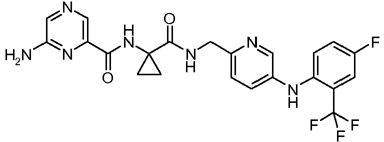 |
| (145) | 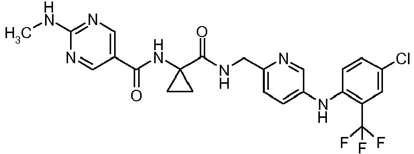 |
| (146) | 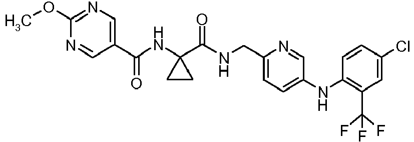 |
| (147) | 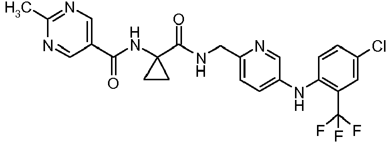 |
| (148) | 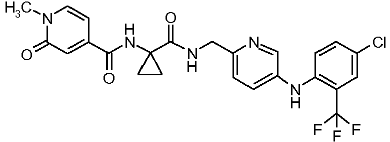 |
| (149) | 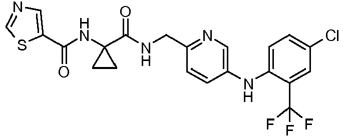 |
| (150) | 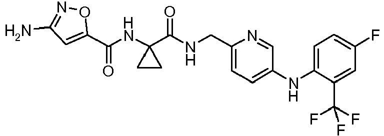 |
| (151) | 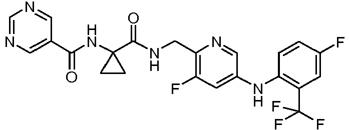 |
| (152) | 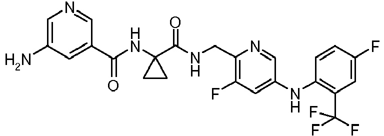 |
| (153) | 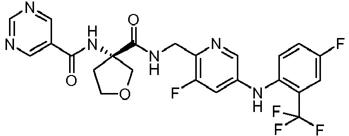 |
| (154) | 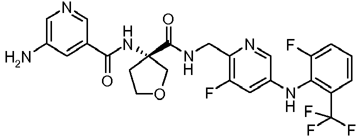 |
| (155) | 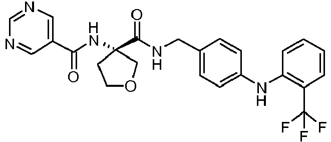 |
| (156) | 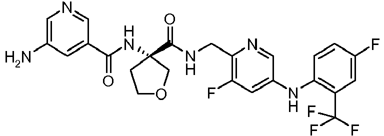 |
| (157) | 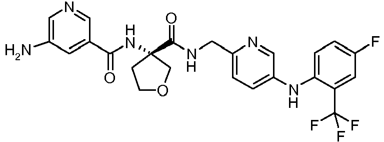 |
| (158) | 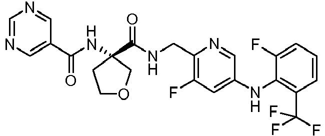 |
| (159) | 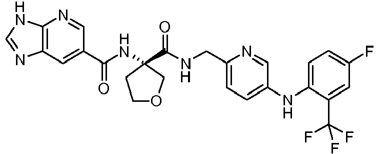 |
| (160) | 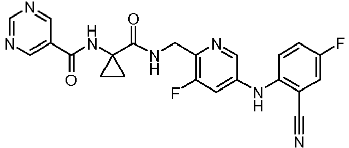 |
| (161) | 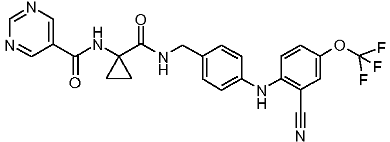 |
| (162) | 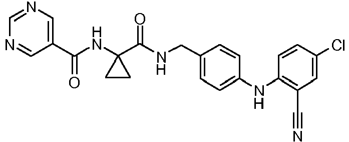 |
| (163) | 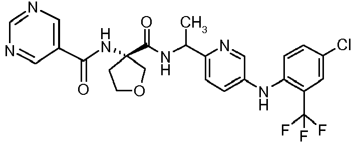 |
| (164) | 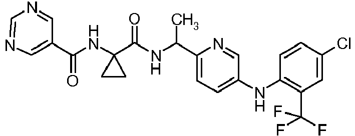 |
| (165) | 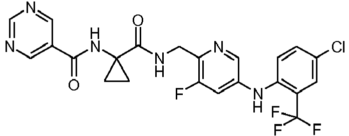 |
| (166) | 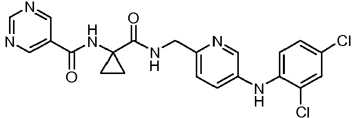 |
| (167) | 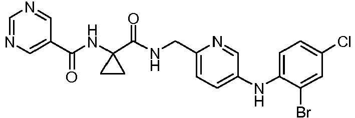 |
| (168) | 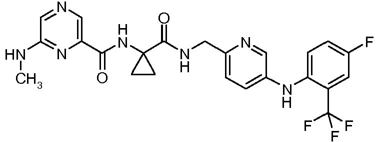 |
| (169) | 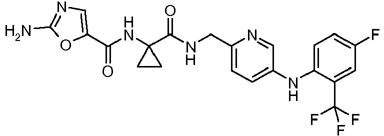 |
| (170) | 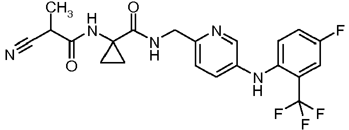 |
| (171) | 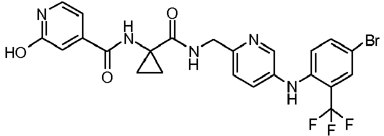 |
| (172) | 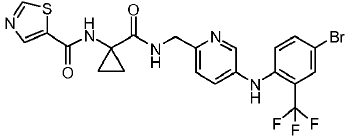 |
| (173) | 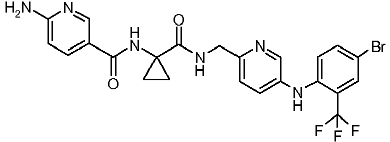 |
| (174) | 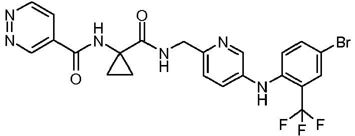 |
| (175) | 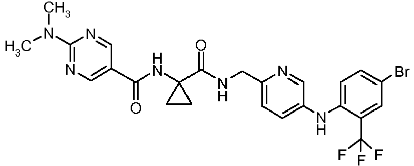 |
| (176) | 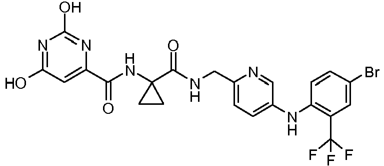 |
| (177) | 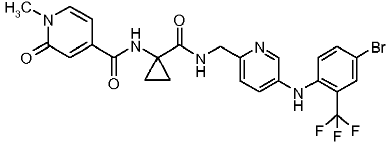 |
| (178) | 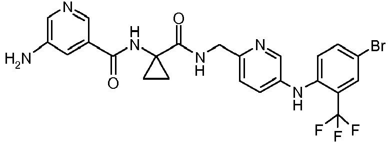 |
| (179) | 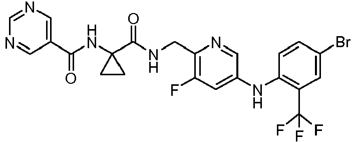 |
| (180) | 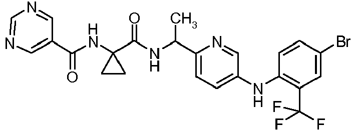 |
| (181) | 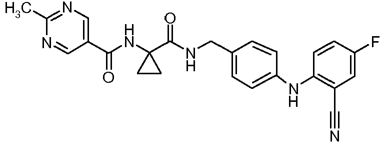 |
| (182) | 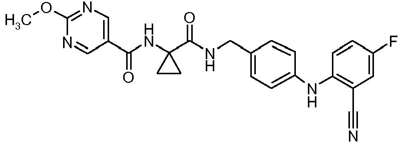 |
| (183) | 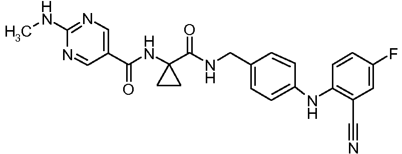 |
| (184) | 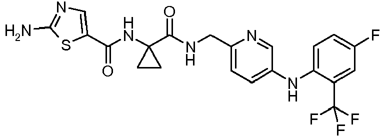 |
| (185) | 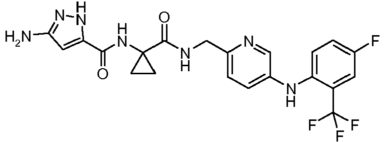 |
| (186) | 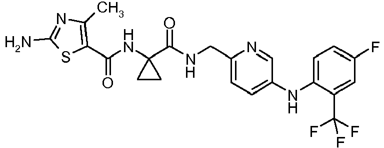 |
| (187) | 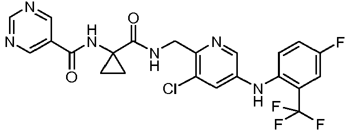 |
| (188) | 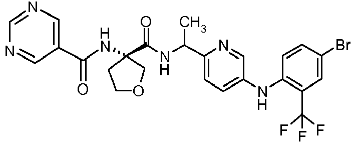 |
| (189) | 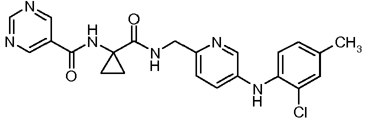 |
| (190) | 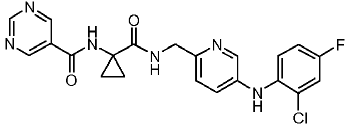 |
| (191) | 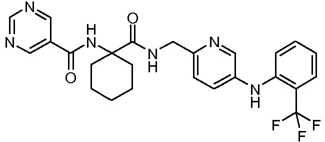 |
| (192) | 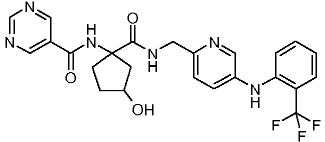 |
| (193) | 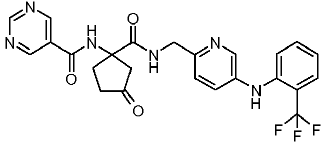 |
| (194) | 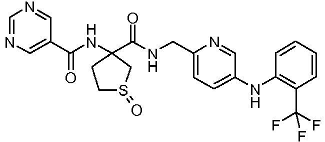 |
| (195) | 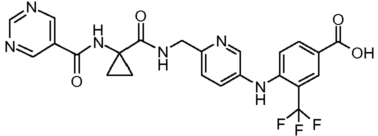 |
| (196) | 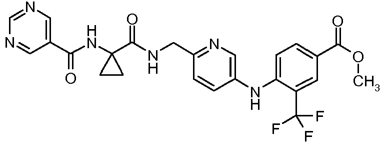 |
| (197) | 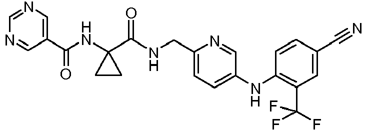 |
| (198) | 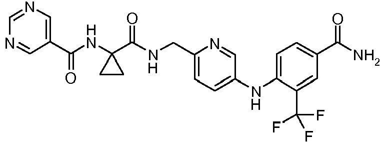 |
| (199) | 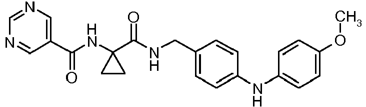 |
| (200) | 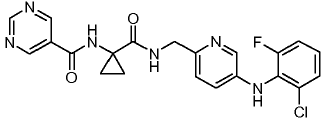 |
| (201) | 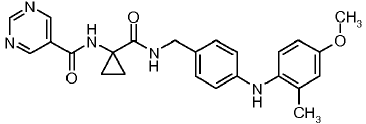 |
| (202) | 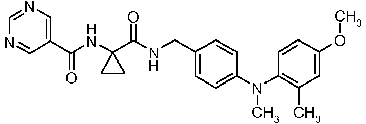 |
| (203) | 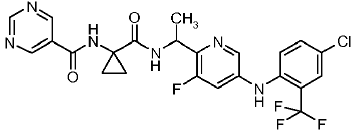 |
| (203a) | 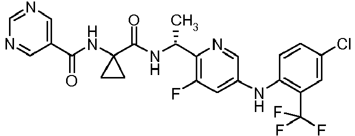 |
| (203b) | 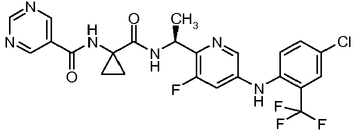 |
| (204) | 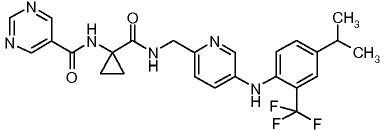 |
| (205) | 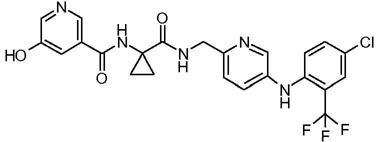 |
| (206) | 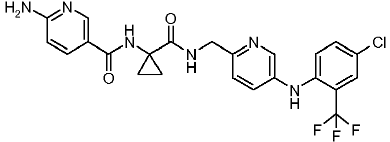 |
| (207) | 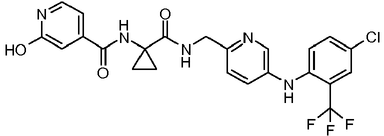 |
| (208) | 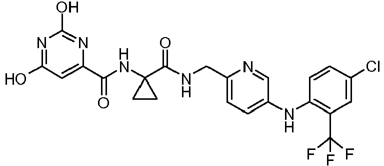 |
| (209) | 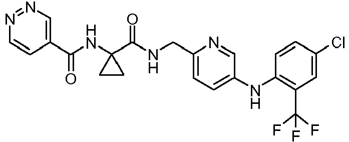 |
| (210) | 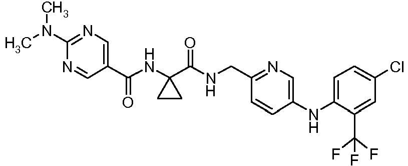 |
| (211) | 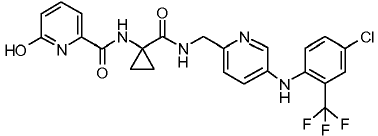 |
| (212) | 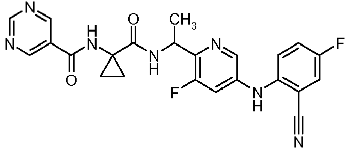 |
| (212a) | 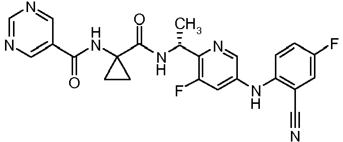 |
| (212b) | 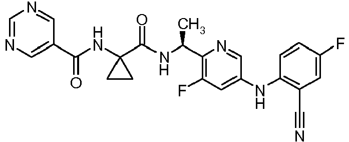 |
| (213) | 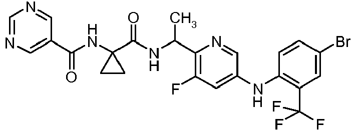 |
| (213a) | 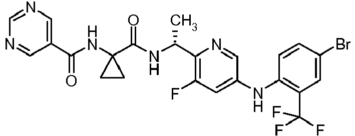 |
| (213b) | 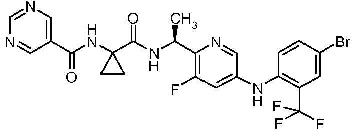 |
| (214) | 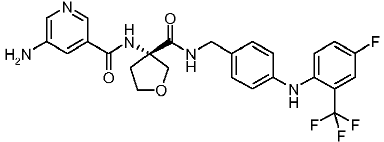 |
| (215) | 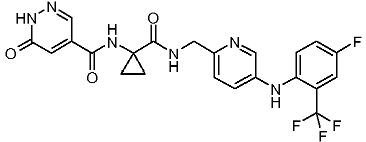 |
| (216) | 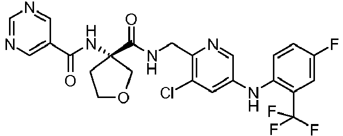 |
| (217) | 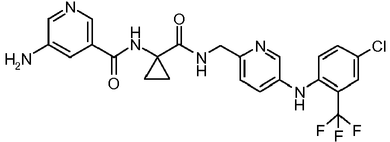 |
| (218) | 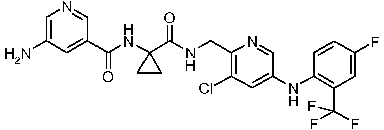 |
| (219) | 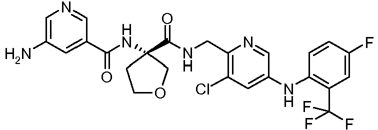 |
| (220) | 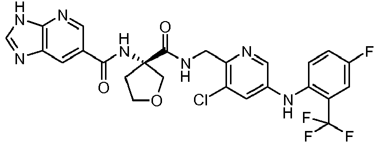 |
| (221) | 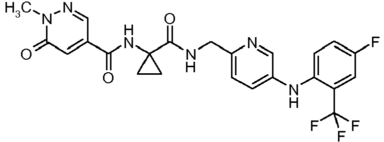 |
| (222) | 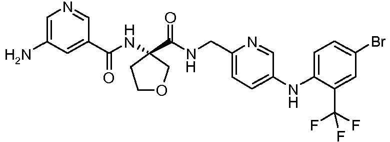 |
| (223) | 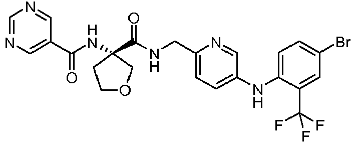 |
| (224) | 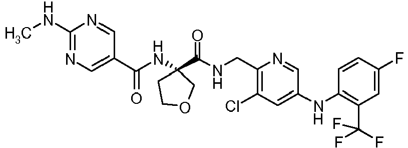 |
| (225) | 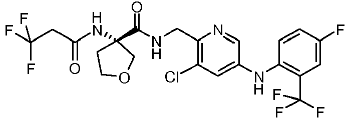 |
| (226) | 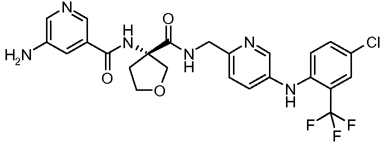 |
| (227) | 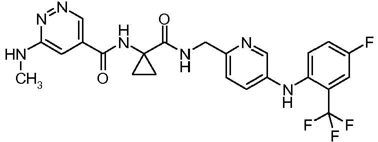 |
| (228) | 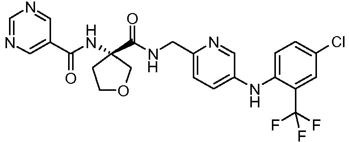 |
| (229) | 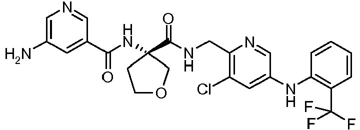 |
| (230) | 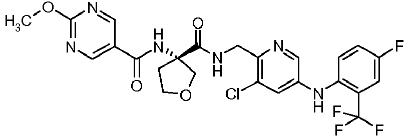 |
| (231) | 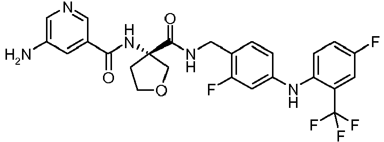 |
| (232) | 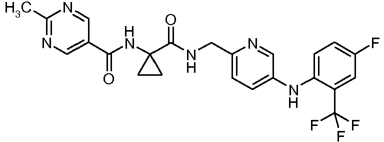 |
| (233) | 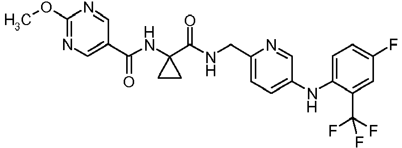 |
| (234) | 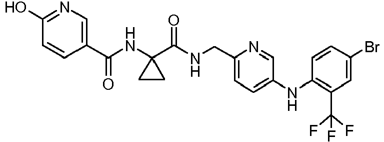 |
| (235) | 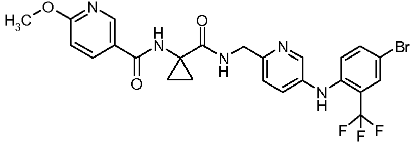 |
| (236) | 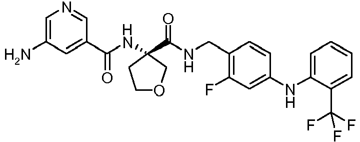 |
| (237) | 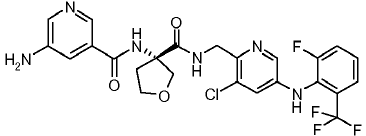 |
| (238) | 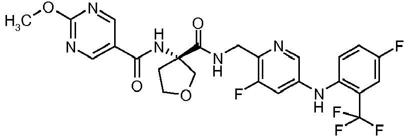 |
| (239) | 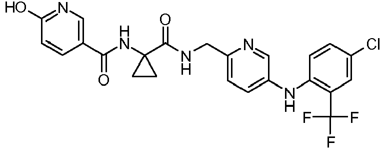 |
| (240) | 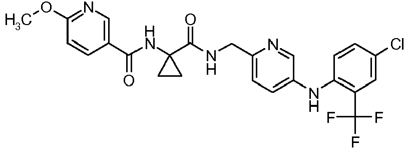 |
| (241) | 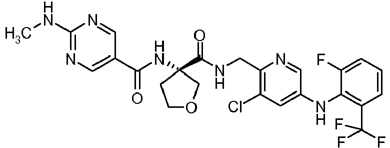 |
| (242) | 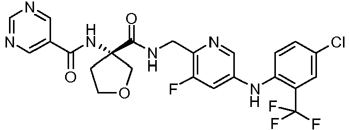 |
| (243) | 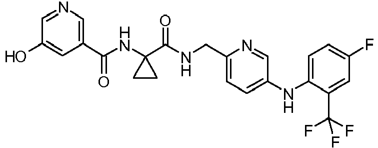 |
| (244) | 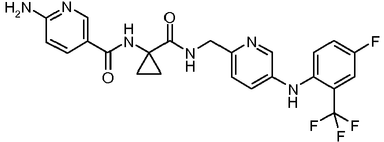 |
| (245) | 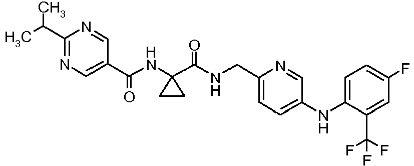 |
| (246) | 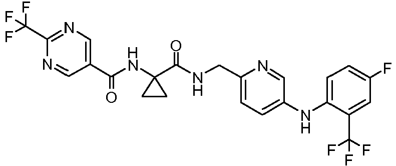 |
| (247) | 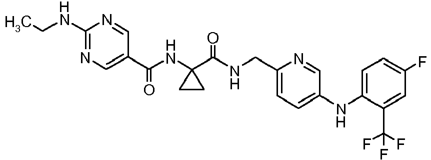 |
| (248) | 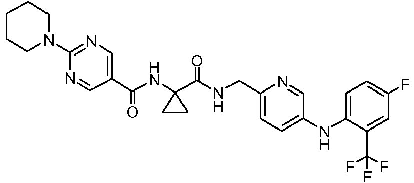 |
| (249) | 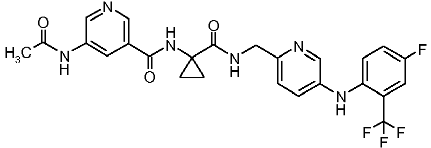 |
| (250) | 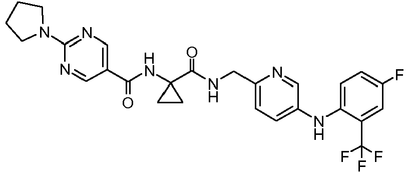 |
| (251) | 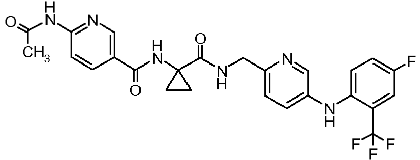 |
| (252) | 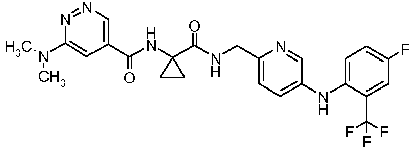 |
| (253) | 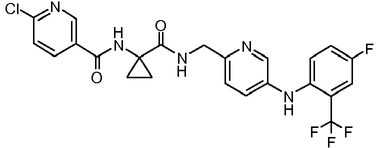 |
| (254) | 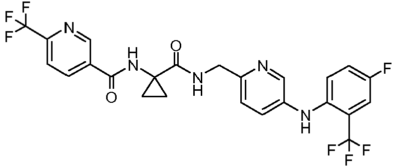 |
| (255) | 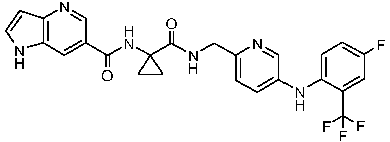 |
| (256) | 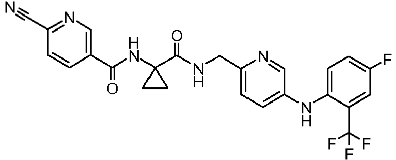 |
| (257) | 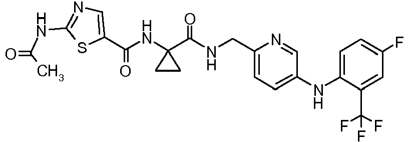 |
| (258) | 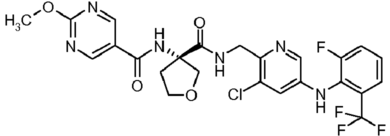 |
| (259) | 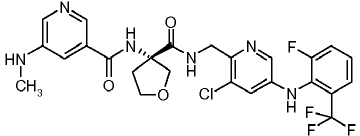 |
| (260) | 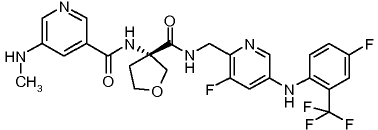 |
| (261) | 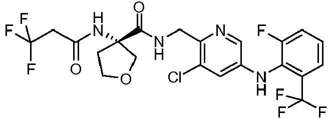 |
| (262) | 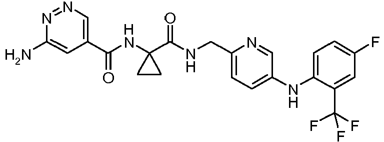 |
| (263) | 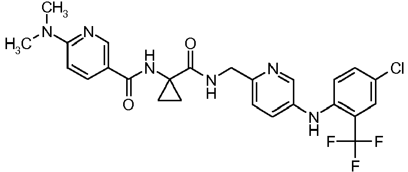 |
| (264) | 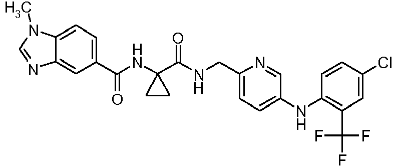 |
| (265) | 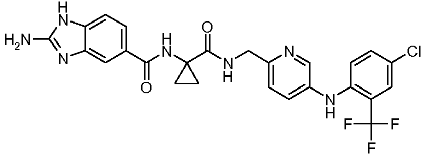 |
| (266) | 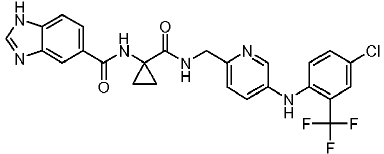 |
| (267) | 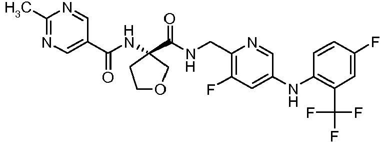 |
| (268) | 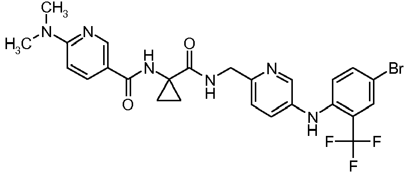 |
| (269) | 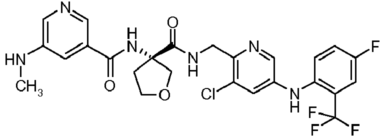 |
| (270) | 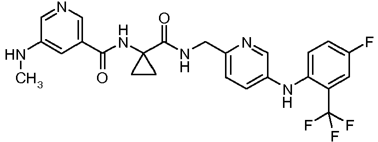 |
| (271) | 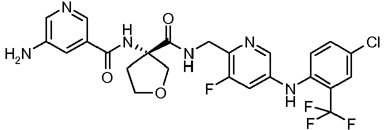 |
| (272) | 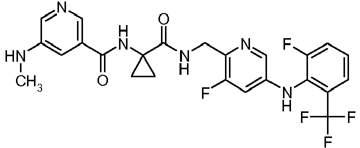 |
| (273) | 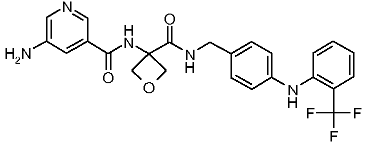 |
| (274) | 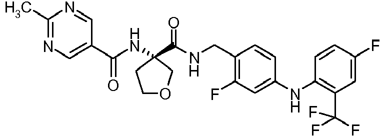 |
| (275) | 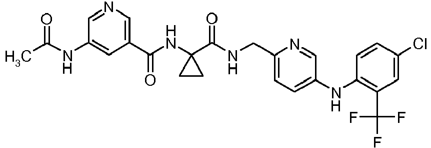 |
| (276) | 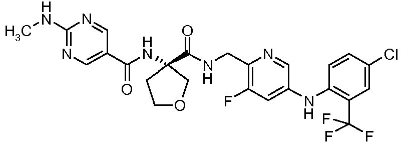 |
| (277) | 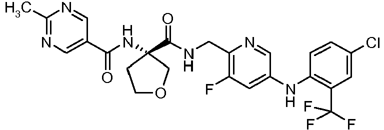 |
| (278) | 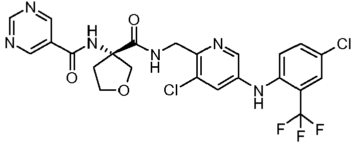 |
| (279) | 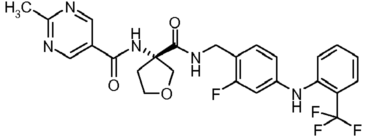 |
| (280) | 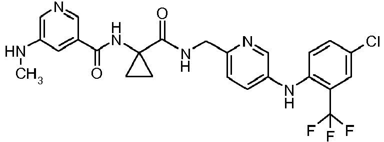 |
| (281) | 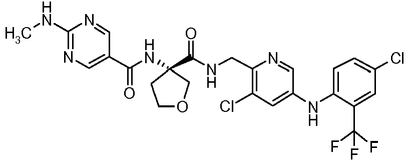 |
| (282) | 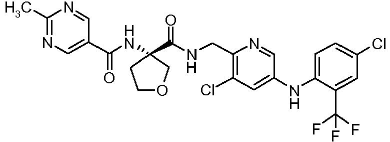 |
| (283) | 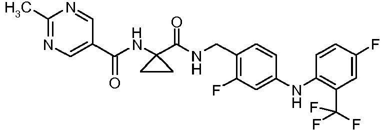 |
| (284) | 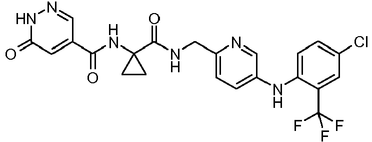 |
| (285) | 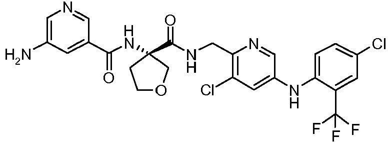 |
| (286) | 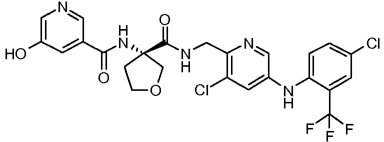 |
| (287) | 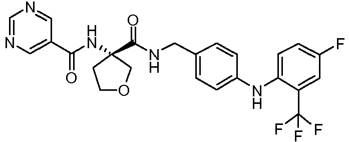 |
| (288) | 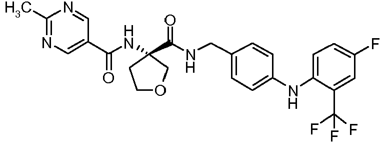 |
| (289) | 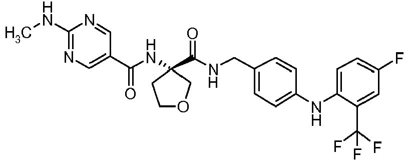 |
| (290) | 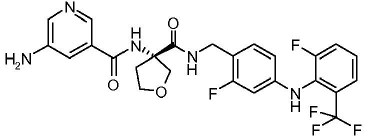 |
| (291) | 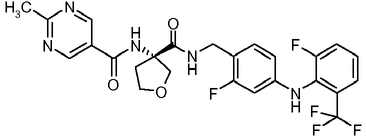 |
| (292) | 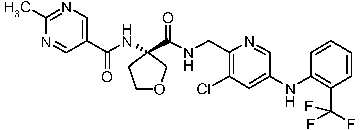 |
| (293) | 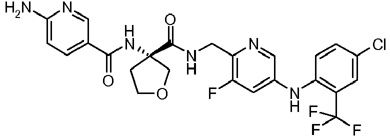 |
| (294) | 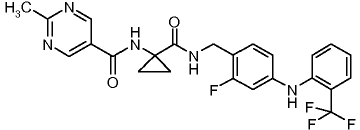 |
| (295) | 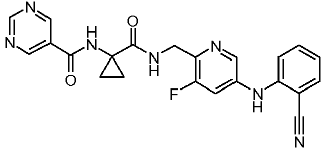 |
| (296) | 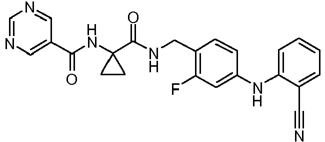 |
| (297) | 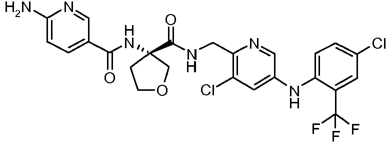 |
| (298) | 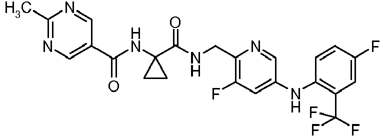 |
| (299) | 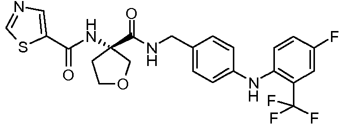 |
| (300) | 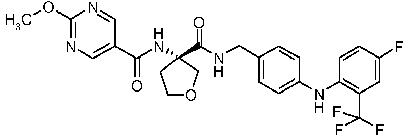 |
| (301) | 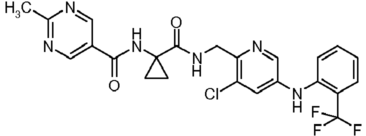 |
| (302) | 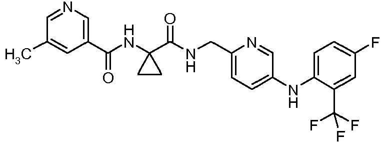 |
| (303) | 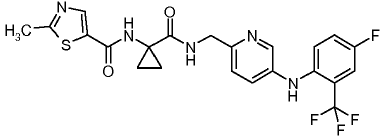 |
| (304) | 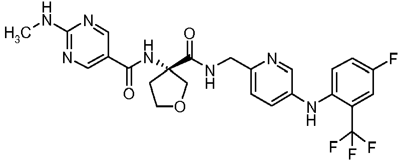 |
| (305) | 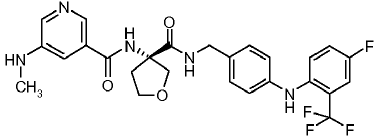 |
| (306) | 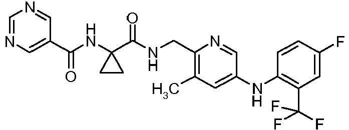 |
| (307) | 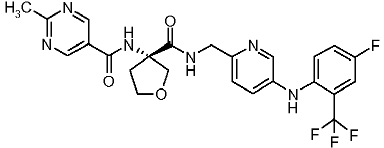 |
| (308) | 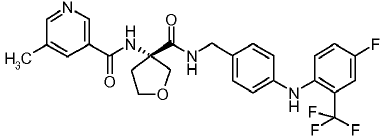 |
| (309) | 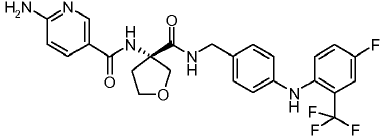 |
| (310) | 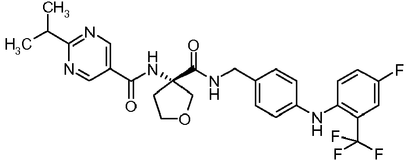 |
| (311) | 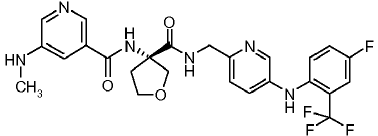 |
| (312) | 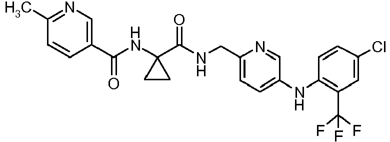 |
| (313) | 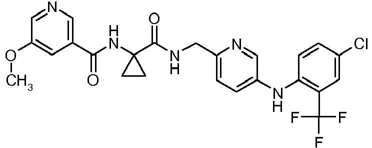 |
| (314) | 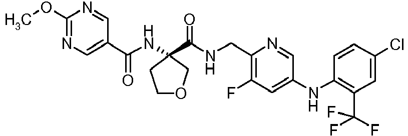 |
| (315) | 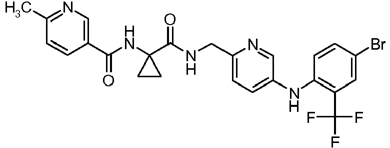 |
| (316) | 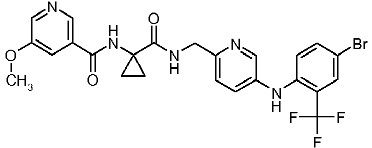 |
| (317) | 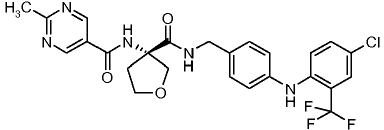 |
| (318) | 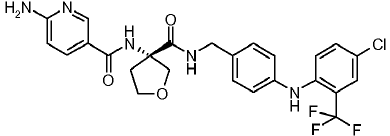 |
| (319) | 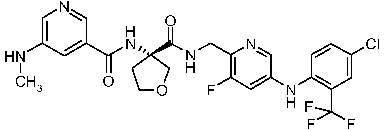 |
| (320) | 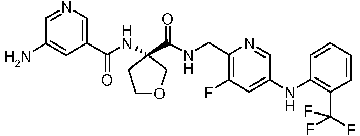 |
| (321) | 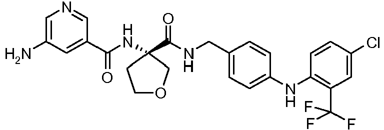 |
| (322) | 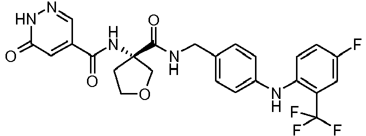 |
| (323) | 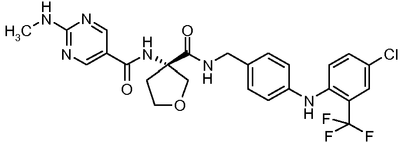 |
| (324) | 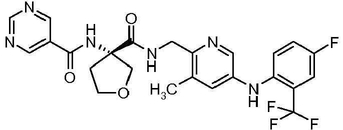 |
| (325) | 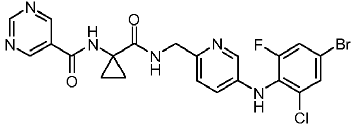 |
| (326) | 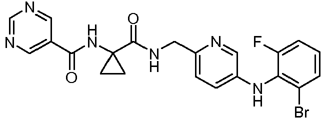 |
| (327) | 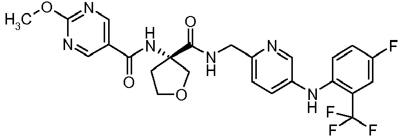 |
| (328) | 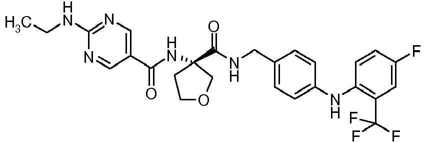 |
| (329) | 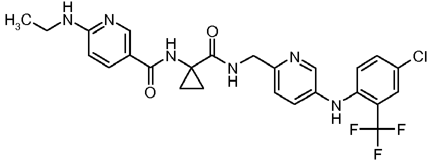 |
| (330) | 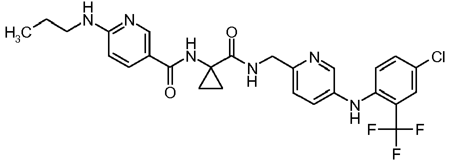 |
| (331) | 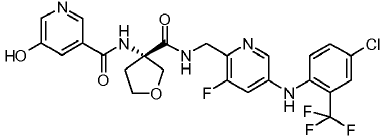 |
| (332) | 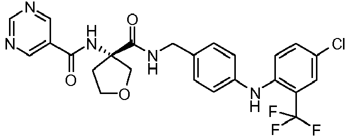 |
| (333) | 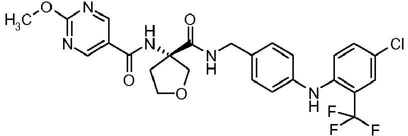 |
| (334) | 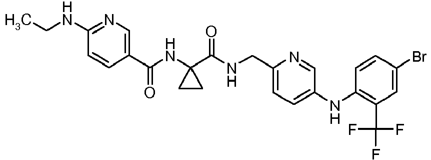 |
| (335) | 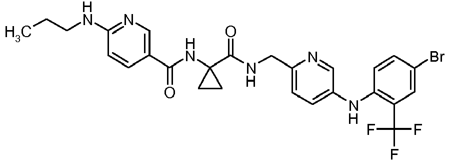 |
| (336) | 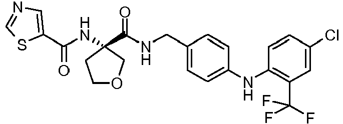 |
| (337) | 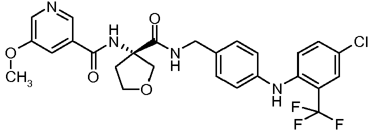 |
| (338) | 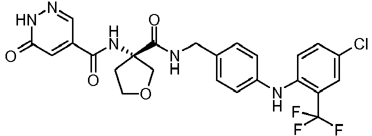 |
| (339) | 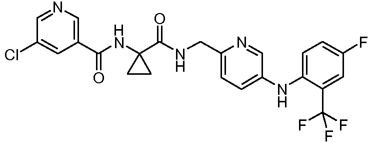 |
| (340) | 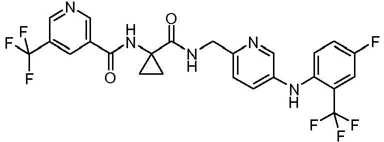 |
| (341) | 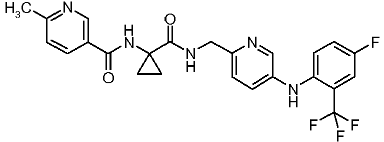 |
| (342) | 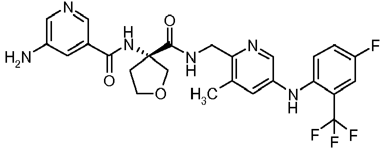 |
| (343) | 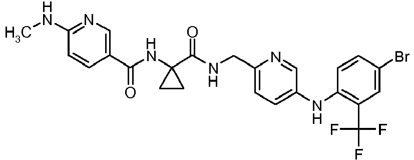 |
| (344) | 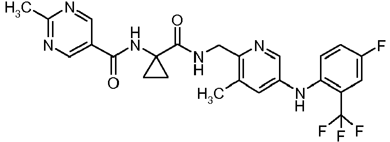 |
| (345) | 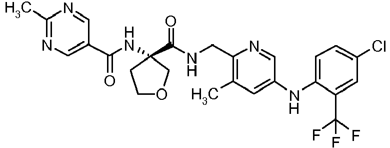 |
| (346) | 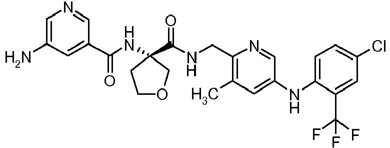 |
| (347) | 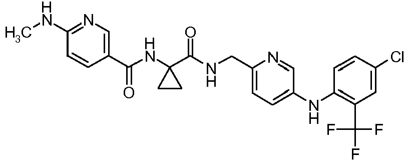 |
| (348) | 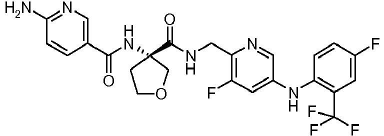 |
| (349) | 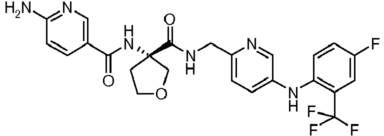 |
| (350) | 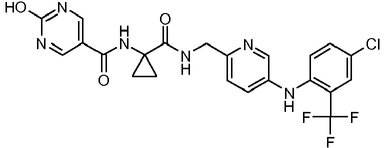 |
| (351) | 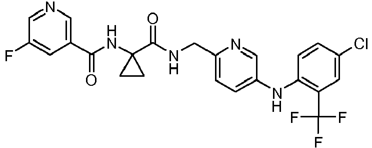 |
| (352) | 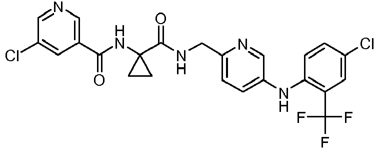 |
| (353) | 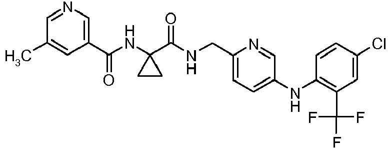 |
| (354) | 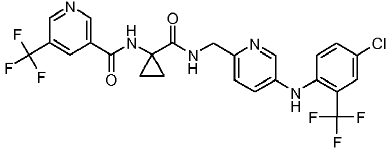 |
| (355) | 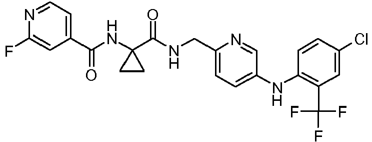 |
| (356) | 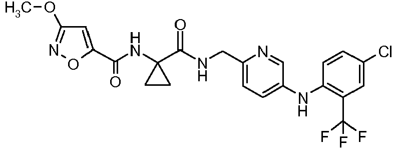 |
| (357) | 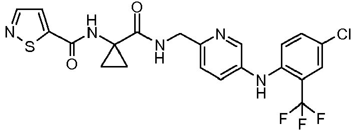 |
| (358) | 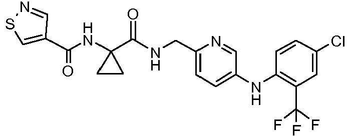 |
| (359) | 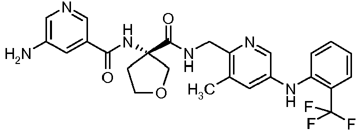 |
| (360) | 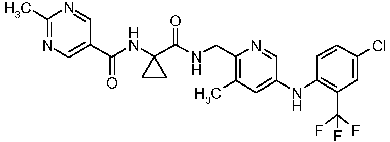 |
| (361) | 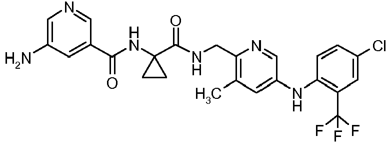 |
| (362) | 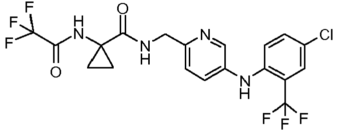 |
| (363) | 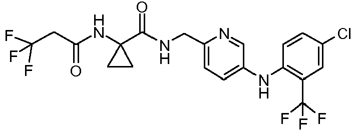 |
| (364) | 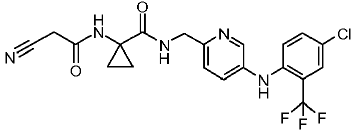 |
| (365) | 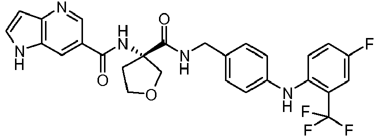 |
| (366) | 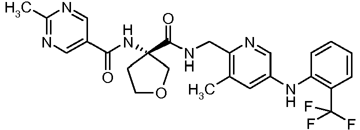 |
| (367) | 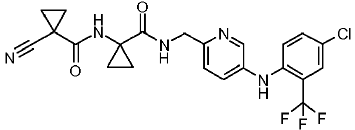 |
| (368) | 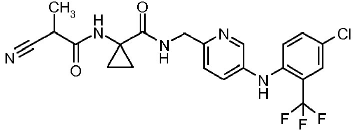 |
| (369) | 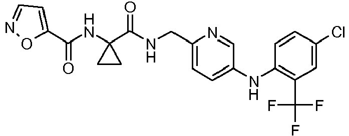 |
| (370) | 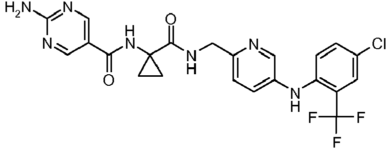 |
| (371) | 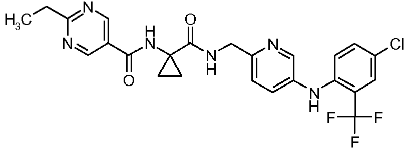 |
| (372) | 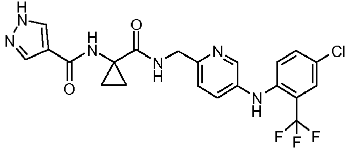 |
| (373) | 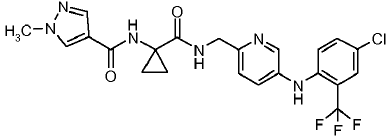 |
| (374) | 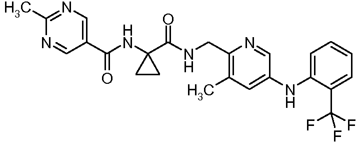 |
| (375) | 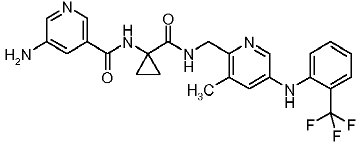 |
| (376) | 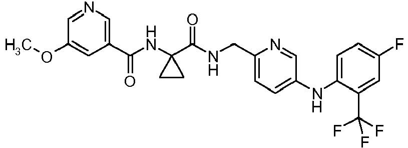 |
| (377) | 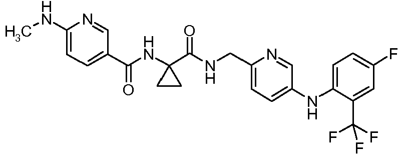 |
| (378) | 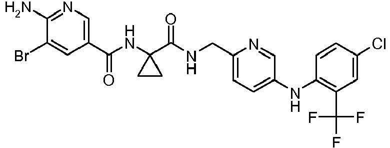 |
| (379) | 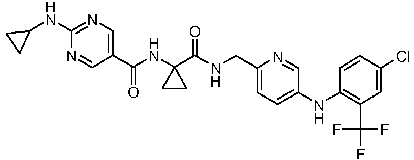 |
| (380) | 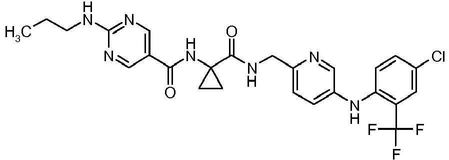 |
| (381) | 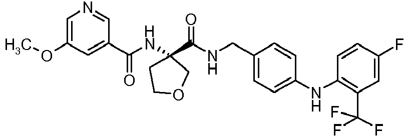 |
| (382) | 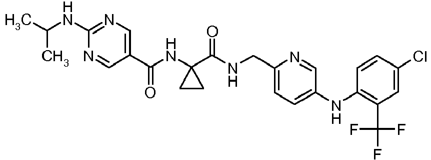 |
| (383) | 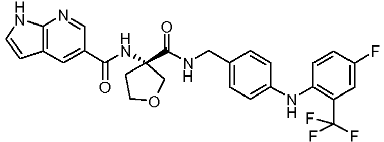 |
| (384) | 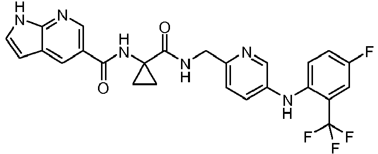 |
| (385) | 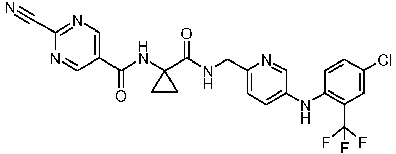 |
| (386) | 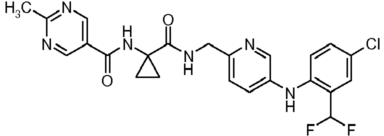 |
| (387) | 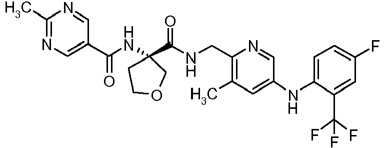 |
| (388) | 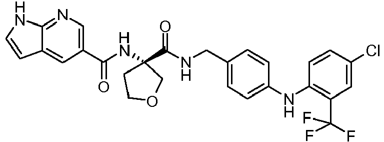 |
| (389) | 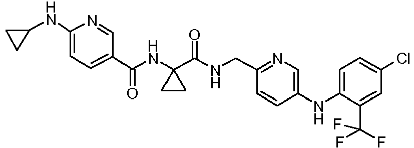 |
| (390) | 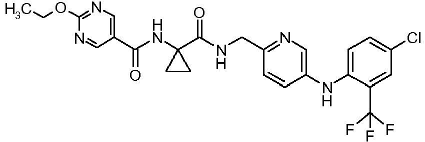 |
| (391) | 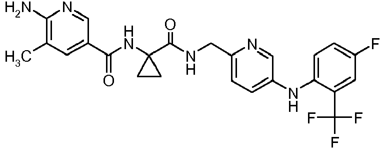 |
| (392) | 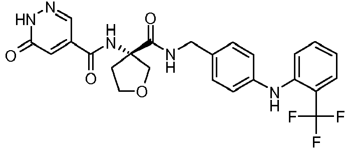 |
| (393) | 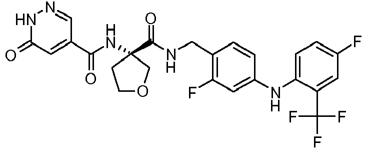 |
| (394) | 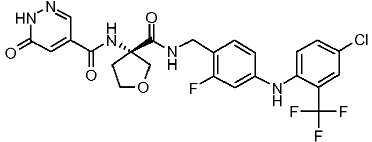 |
| (395) | 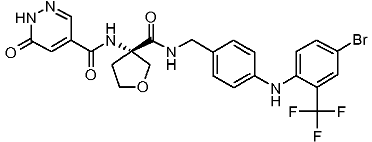 |
| (396) | 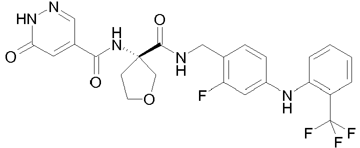 |
leurs énantiomères, leurs diastéréomères, leurs mélanges et leurs sels.

29. Sels physiologiquement acceptables des composés selon l'une des revendications 1 à 28 avec des acides ou des bases, minéraux ou organiques.

30. Médicaments contenant un composé selon au moins une des revendications 1 à 28 ou un sel physiologiquement acceptable selon la revendication 29, en plus éventuellement d'un ou plusieurs excipients et/ou diluants inertes.

31. Composé de formule générale I selon l'une des revendications 1, 2, 3, 4, 5, 6, 7, 8, 9, 10, 11, 12, 13, 14, 15, 16, 17, 18, 19, 20, 21, 22, 23, 24, 25, 26, 27, 28 ou 29 destiné à l'utilisation comme médicaments.

32. Composé de formule générale I selon l'une des revendications 1, 2, 3, 4, 5, 6, 7, 8, 9, 10, 11, 12, 13, 14, 15, 16, 17, 18, 19, 20, 21, 22, 23, 24, 25, 26, 27, 28 ou 29 pour la fabrication d'un médicament destiné au traitement aigu et prophylactique des douleurs aiguës, des douleurs des entrailles, des douleurs neuropathiques, des douleurs inflammatoires/ induites par les récepteurs de la douleur, des douleurs dues à des tumeurs et des céphalées.

33. Utilisation d'un composé selon au moins une des revendications 1 à 24 pour la fabrication d'un médicament destiné au traitement aigu et prophylactique de l'ostéoarthrite.

34. Procédé de fabrication d'un médicament selon la revendication 30, **caractérisé en ce que** l'on incorpore par voie non chimique un composé selon au moins une des revendications 1 à 29 dans un ou plusieurs excipients et/ou diluants inertes.
